# EUROPÄISCHE PATENTSCHRIFT

(11) **EP 3 066 080 B1**
(45) Veröffentlichungstag und Bekanntmachung des Hinweises auf die Patenterteilung: **09.05.2018**
(21) Anmeldenummer: 14793179.4
(22) Anmeldetag: 05.11.2014
(51) Int. Cl.: C07D 231/12, A01N 43/56, A01N 43/72, C07D 261/08, A01P 7/00, C07D 401/04, C07D 403/04, C07D 207/337, A01N 43/80

(54) **SUBSTITUIERTE BENZAMIDE ZUR BEHANDLUNG VON ARTHROPODEN**
NOVEL COMPOUNDS FOR COMBATING ARTHROPODS
COMPOSÉS NOUVEAUX POUR COMBATRE DES ARTHROPODES

(30) Priorität: 05.11.2013 EP 13191610; 15.08.2014 EP 14181149
(43) Veröffentlichungstag der Anmeldung: 14.09.2016
(73) Patentinhaber: Bayer CropScience Aktiengesellschaft, 40789 Monheim am Rhein (DE)
(72) Erfinder: HALLENBACH, Werner, 40789 Monheim (DE); SCHWARZ, Hans-Georg, 46282 Dorsten (DE); ILG, Kerstin, 50670 Köln (DE); GÖRGENS, Ulrich, 40882 Ratingen (DE); KÖBBERLING, Johannes, 41466 Neuss (DE); TURBERG, Andreas, 42781 Haan (DE); BÖHNKE, Niels, 10713 Berlin (DE); MAUE, Michael, 40764 Langenfeld (DE); VELTEN, Robert, 40764 Langenfeld (DE); HARSCHNECK, Tobias, 40225 Düsseldorf (DE); HAHN, Julia Johanna, 40598 Düsseldorf (DE); HORSTMANN, Sebastian, 51381 Leverkusen (DE)
(74) Vertreter: BIP Patents
(86) Internationale Anmeldenummer: PCT/EP2014/073795
(87) Internationale Veröffentlichungsnummer: WO 2015/067647

(56) Entgegenhaltungen:
- WO-A1-03/087061
- WO-A1-2011/009484
- WO-A1-2012/175474
- WO-A1-2013/041602
- GABRIEL G GAMBER ET AL: "3,5-Diarylazoles as novel and selective inhibitors of protein kinase D", BIOORGANIC & MEDICINAL CHEMISTRY LETTERS, PERGAMON, GB, Bd. 21, Nr. 5, 6. Januar 2011 (2011-01-06) , Seiten 1447-1451, XP028144502, ISSN: 0960-894X, DOI: 10.1016/J.BMCL.2011.01.014 [gefunden am 2011-01-11]
- LOUNKINE EUGEN ET AL: "Chemotography for multi-target SAR analysis in the context of biological pathways", BIOORGANIC & MEDICINAL CHEMISTRY, Bd. 20, Nr. 18, 20. Februar 2012 (2012-02-20), Seiten 5416-5427, XP028938241, ISSN: 0968-0896, DOI: 10.1016/J.BMC.2012.02.034
- DATABASE CA [Online] CHEMICAL ABSTRACTS SERVICE, COLUMBUS, OHIO, US; BALLELL, LLUIS ET AL: "Fueling Open-Source Drug Discovery: 177 Small-Molecule Leads against Tuberculosis", XP002732971, gefunden im STN Database accession no. 2013:56758

## Beschreibung

Die vorliegende Anmeldung betrifft neue Verbindungen, Verfahren zu ihrer Herstellung und ihre Verwendung zur Bekämpfung von tierischen Schädlingen, vor allem von Arthropoden und insbesondere von Insekten, Spinnentieren und Nematoden.

Es ist bekannt, dass bestimmte Halogen-substituierte Verbindungen insektizid wirksam sind (EP 1 911 751, WO2012/069366, WO2012/080376, WO2012/107434 und WO2012/175474).

WO 2011/113756 offenbart Triazol Derivate, die insektizidale Wirkung aufweisen.

Ferner ist bekannt, dass bestimmte Halogen-substituierte Verbindungen Cytokin-inhibitorische Aktivitäten aufweisen (WO 2000/07980).

Moderne Pflanzenschutzmittel müssen vielen Anforderungen genügen, beispielsweise in Bezug auf Höhe, Dauer und Breite ihrer Wirkung und möglichen Verwendung. Es spielen Fragen der Toxizität, der Kombinierbarkeit mit anderen Wirkstoffen oder Formulierhilfsmitteln eine Rolle sowie die Frage des Aufwands, der für die Synthese eines Wirkstoffs betrieben werden muss. Ferner können Resistenzen auftreten. Aus all diesen Gründen kann die Suche nach neuen Pflanzenschutzmitteln nie als abgeschlossen betrachtet werden und es besteht ständig Bedarf an neuen Verbindungen mit gegenüber den bekannten Verbindungen zumindest in Bezug auf einzelne Aspekte verbesserten Eigenschaften.

Aufgabe der vorliegenden Erfindung war es, Verbindungen bereitzustellen, durch die das Spektrum der Schädlingsbekämpfungsmittel unter verschiedenen Aspekten verbreitert und/oder ihre Aktivität verbessert wird.

Es wurde nun überraschenderweise gefunden, dass bestimmte Halogen-substituierte Verbindungen sowie deren Salze biologische Eigenschaften aufweisen und sich insbesondere zur Bekämpfung von tierischen Schädlingen eignen, und deshalb besonders gut im agrochemischen Bereich und im Bereich der Tiergesundheit einsetzbar sind.

Ähnliche Verbindungen sind bereits aus WO 2010/051926 bekannt geworden.

### Zusammenfassung

Es wurden nun neue insektizid, akarizid und/oder parasitizid wirksame Halogen-substituierten Verbindungen der allgemeine Formel (Ia") gefunden: 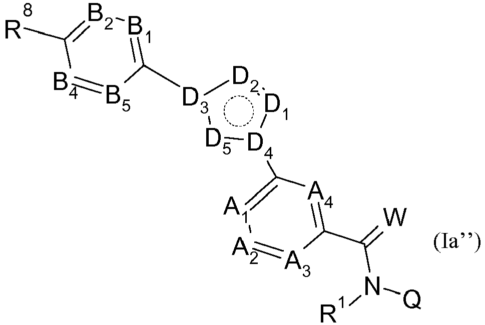 worin
- D₁: für C-R¹¹ oder ein Heteroatom ausgewählt aus N oder O steht;
- D₂: für C-R¹¹ oder ein Heteroatom ausgewählt aus N oder O steht;
- D₃: für C oder N steht;
- D₄: für C, oder N steht;
- D₅: für C-R¹¹ oder N steht;
wobei maximal ein (1) oder zwei Gruppierungen ausgewählt aus D₁, D₂, D₃, D₄ und D₅ für ein Heteroatom stehen; 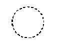 für ein aromatisches System steht; und
- R¹: für H, jeweils gegebenenfalls substituiertes C₂-C₆-Alkenyl, C₂-C₆-Alkinyl, C₃-C₇-Cycloalkyl, C₁-C₆-Alkylcarbonyl, C₁-C₆-Alkoxycarbonyl, Aryl(C₁-C₃)-alkyl, oder Heteroaryl(C₁-C₃)-alkyl steht oder für gegebenenfalls substituiertes C₁-C₆-Alkyl steht, besonders bevorzugt für H steht;
die Gruppierungen
- A₁: C-H,
- A₂: CR³ oder N,
- A₃: CR⁴,
- A₄: C-H,
- B₁: CR⁶ oder N,
- B₂: C-H,
- B₄: C-H und
- B₅: CR¹⁰ oder N stehen;R³, R⁴, R⁶, und R¹⁰ unabhängig voneinander für H, Halogen, Cyano, Nitro, jeweils gegebenenfalls substituiertes C₁-C₆-Alkyl, C₃-C₆-Cycloalkyl, C₁-C₆-Alkoxy, *N*-C₁-C₆-Alkoxy-imino-C₁-C₃-alkyl, C₁-C₆-Alkylsulfanyl, C₁-C₆-Alkylsulfinyl, C₁-C₆-Alkylsulfonyl, *N*-C₁-C₆-Alkylamino oder *N,N*-Di-C₁-C₆-alkylamino, stehen;
wenn keine der Gruppierungen A₂ und A₃ für N steht, können R³ und R⁴ gemeinsam mit dem Kohlenstoffatom, an das sie gebunden sind, einen 5- oder 6-gliedrigen Ring bilden, der 0, 1 oder 2 N-Atome und/oder 0 oder 1 O-Atom und/oder 0 oder 1 S-Atom enthält, oder
wenn keine der Gruppierungen A₁ und A₂ für N steht, können R² und R³ gemeinsam mit dem Kohlenstoffatom, an das sie gebunden sind, einen 6-gliedrigen Ring bilden, der 0, 1 oder 2 N-Atome enthält;
- R⁸: für mit Fluor substituiertes C₁-C₄-Alkoxy oder mit Fluor substituiertes C₁-C₄-Alkyl steht;
- R¹¹: unabhängig voneinander für H, Halogen, Cyano, Nitro, Amino oder ein gegebenenfalls substituiertes C₁-C₆-Alkyl, C₁-C₆-Alkyloxy, C₁-C₆-Alkylcarbonyl, C₁-C₆-Alkylsulfanyl, C₁-C₆-Alkylsulfinyl, C₁-C₆-Alkylsulfonyl, bevorzugt für H, steht;
- W: für O oder S steht;
- Q: für H, Formyl, Hydroxy, Amino oder jeweils gegebenenfalls substituiertes C₁-C₆-Alkyl, C₂-C₆-Alkenyl, C₂-C₆-Alkinyl, C₃-C₆-Cycloalkyl, C₁-C₅-Heterocycloalkyl, C₁-C₄-Alkoxy, C₁-C₆-Alkyl-C₃-C₆-cycloalkyl, C₃-C₆-Cycloalkyl-C₁-C₆-alkyl, C₆-,C₁₀-,C₁₄-Aryl, C₁-C₅-Heteroaryl, C₆-,C₁₀-,C₁₄-Aryl-(C₁-C₃)-alkyl, C₁-C₅-Heteroaryl-(C₁-C₃)-alkyl, *N*-C₁-C₄-Alkylamino, *N*-C₁-C₄-Alkylcarbonylamino, oder *N,N*-Di-C₁-C₄-alkylamino steht; oder
für einen gegebenenfalls mehrfach mit V substituierten, ungesättigten 6-gliedrigen Carbozyklus steht; oder
für einen gegebenenfalls mehrfach mit V substituierten, ungesättigten 4-, 5- bzw. 6-gliedrigen, heterozyklischen Ring steht, wobei
- V: unabhängig voneinander für Halogen, Cyano, Nitro, jeweils gegebenenfalls substituiertes C₁-C₆-Alkyl, C₁-C₄-Alkenyl, C₁-C₄-Alkinyl, C₃-C₆-Cycloalkyl, C₁-C₆-Alkoxy, *N*-C₁-C₆-Alkoxy-imino-C₁-C₃-alkyl, C₁-C₆-Alkylsulfanyl, C₁-C₆-Alkylsulfinyl, C₁-C₆-Alkylsulfonyl, oder *N,N*-Di-(C₁-C₆-alkyl)amino steht,
wobei für den Fall, dass die Gruppen R³, R⁴, R⁶, R¹⁰, R¹¹ Q und V jeweils unabhängig voneinaner substituiert sind, die Substituenten ein (1) Substituent oder mehrere Substituenten ausgewählt sind aus einer Gruppe bestehend aus Amino, Hydroxy, Halogen, Nitro, Cyano, Isocyano, Mercapto, Isothiocyanato, C₁-C₄-Carboxy, Carbonamid, SF₅, Aminosulfonyl, C₁-C₄-Alkyl, C₃-C₄-Cycloalkyl, C₂-C₄-Alkenyl, C₃-C₄-Cycloalkenyl, C₂-C₄-Alkinyl, *N*-Mono-C₁-C₄-alkyl-amino, *N,N*-Di-C₁-C₄-alkylamino, *N*-C₁-C₄-Alkanoylamino, C₁-C₄-Alkoxy, C₂-C₄-Alkenyloxy, C₂-C₄-Alkinyloxy, C₃-C₄-Cycloalkoxy, C₃-C₄-Cycloalkenyloxy, C₁-C₄-Alkoxycarbonyl, C₂-C₄- C₂-C₄-Alkenyloxycarbonyl, C₂-C₄-Alkinyloxycarbonyl, C₆-,C₁₀-,C₁₄-Aryloxycarbonyl, C₁-C₄-Alkanoyl, C₂-C₄-Alkenylcarbonyl, C₂-C₄-Alkinylcarbonyl, C₆-,C₁₀-,C₁₄-Arylcarbonyl, C₁-C₄-Alkylsulfanyl, C₃-C₄-Cycloalkylsulfanyl, C₁-C₄-Alkylthio, C₂-C₄-Alkenylthio, C₃-C₄-Cycloalkenylthio, C₂-C₄-Alkinylthio, C₁-C₄-Alkylsulfenyl und C₁-C₄-Alkylsulfinyl, wobei beide Enantiomere der C₁-C₄-Alkylsulfinylgruppe umfasst sind, C₁-C₄-Alkylsulfonyl, *N*-Mono-C₁-C₄-alkyl-aminosulfonyl, *N,N*-Di-C₁-C₄-alkyl-aminosulfonyl, C₁-C₄-Alkylphosphinyl, C₁-C₄-Alkylphosphonyl, wobei für C₁-C₄-Alkylphosphinyl bzw. C₁-C₄-Alkylphosphonyl beide Enantiomere umfasst sind, *N*-C₁-C₄-Alkyl-aminocarbonyl, *N,N*-Di-C₁-C₄-alkyl-amino-carbonyl, N-C₁-C₄-Alkanoyl-amino-carbonyl, N-C₁-C₄-Alkanoyl-N-C₁-C₄-alkyl-aminocarbonyl, C₆-,C₁₀-,C₁₄-Aryl, C₆-,C₁₀-,C₁₄-Aryloxy, Benzyl, Benzyloxy, Benzylthio, C₆-,C₁₀-,C₁₄-Arylthio, C₆-,C₁₀-,C₁₄-Arylamino, Benzylamino, Heterocyclyl und Trialkylsilyl, mit einer Doppelbindung verbundene Substituenten wie C₁-C₄-Alkyliden (z. B. Methyliden oder Ethyliden), eine Oxogruppe, eine Thioxogruppe, eine Iminogruppe sowie einer substituierten Iminogruppe,
sowie Salze, N-Oxide und tautomere Formen der Verbindungen der Formel (Ia").

Eine weitere Ausführungsform der vorliegenden Erfindung betrifft Verbindungen der Formel (Ia"), wobei die Verbindungen der Formel (Ia") Verbindungen der Formel (I-T3) darstellen 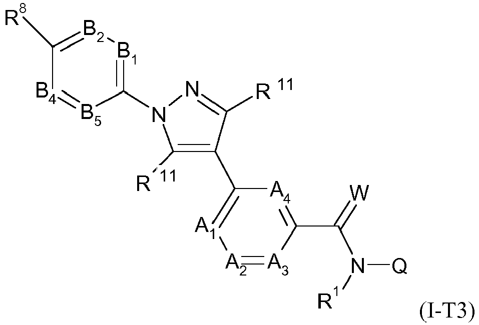 worin R¹, A₁, A₂, A₃, A₄, R¹¹, B₁, B₂, B₄, B₅, R⁸, Q und W wie hierin beschrieben definiert sind.

Eine weitere Ausführungsform der vorliegenden Erfindung betrifft Verbindungen der Formel (Ia"), wobei die Verbindungen der Formel (Ia") Verbindungen der Formel (I-T2) darstellen 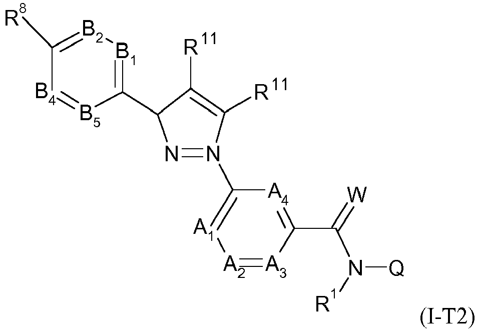 worin R¹, A₁, A₂, A₃, A₄, R¹¹, B₁, B₂, B₄, B₅, R⁸, Q und W wie hierin beschrieben definiert sind.

Eine weitere Ausführungsform der vorliegenden Erfindung betrifft Verbindungen der Formel (Ia"), wobei die Verbindungen der Formel (Ia") Verbindungen der Formel (I-T4) darstellen 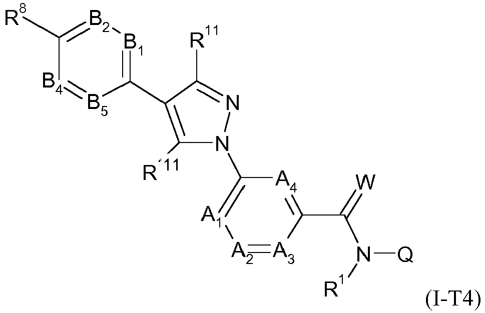 worin R¹, A₁, A₂, A₃, A₄, R¹¹, B₁, B₂, B₄, B₅, R⁸, Q und W wie hierin beschrieben definiert.

Eine weitere Ausführungsform der vorliegenden Erfindung betrifft Verbindungen der Formel (Ia"), wobei die Verbindungen der Formel (Ia") Verbindungen der Formel (I-T22) darstellen 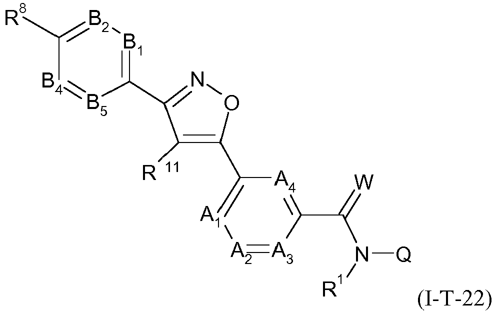 worin R¹, A₁, A₂, A₃, A₄, R¹¹, B₁, B₂, B₄, B₅, R⁸, Q und W wie hierin beschrieben definiert sind.

Eine weitere Ausführungsform der vorliegenden Erfindung betrifft Verbindungen der Formel (Ia"), wobei die Verbindungen der Formel (Ia") Verbindungen der Formel (I-T23) darstellen 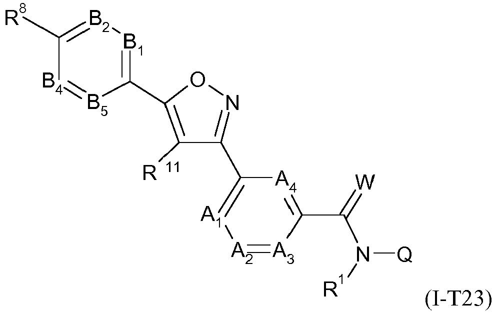 worin R¹, A₁, A₂, A₃, A₄, R¹¹, B₁, B₂, B₄, B₅, R⁸, Q und W wie hierin beschrieben definiert sind.

Eine weitere Ausführungsform der vorliegenden Erfindung betrifft Verbindungen gemäß den hierin beschriebenen Formeln und Ausführungsformen, wobei R¹¹ unabhängig voneinander für H steht und W für O steht.

Eine weitere Ausführungsform der vorliegenden Erfindung betrifft Verbindungen gemäß den hierin beschriebenen Formeln und Ausführungsformen, wobei R¹¹ unabhängig voneinander für H steht und W für O steht und R⁸ für perfluoriertes C₁-C₃-Alkyl oder perfluoriertes C₁-C₃-Alkoxy steht.

Eine weitere Ausführungsform der vorliegenden Erfindung betrifft Verbindungen gemäß den hierin beschriebenen Formeln und Ausführungsformen, wobei R¹ für H steht.

Eine weitere Ausführungsform der vorliegenden Erfindung betrifft Verbindungen gemäß den hierin beschriebenen Formeln und Ausführungsformen, wobei Q für mit Fluor substituiertes C₁-C₄-Alkyl, C₃-C₄-Cycloalkyl, gegebenenfalls mit Cyano oder Fluor substituiertes C₃-C₄-Cycloalkyl, C₄-C₆-Heterocycloalkyl, 1-Oxido-thietan-3-yl, 1,1-Dioxido-thietan-3-yl, Benzyl, Pyridin-2-ylmethyl, Methylsulfonyl, oder 2-oxo-2-(2,2,2-trifluoroethylamino)ethyl steht.

Noch eine weitere Ausführungsform der vorliegenden Erfindung betrifft Verbindungen gemäß den hierin beschriebenen Formeln, wobei R¹¹ unabhängig voneinander für H steht.

Noch eine weitere Ausführungsform der vorliegenden Erfindung betrifft Verbindungen gemäß den hierin beschriebenen Formeln, wobei R⁶ und R¹⁰ unabhängig voneinander für H, Halogen, Cyano, Nitro, jeweils gegebenenfalls substituiertes C₁-C₄-Alkyl, C₃-C₄-Cycloalkyl, C₁-C₄-Alkoxy, *N-*Alkoxyiminoalkyl, C₁-C₄-Alkylsulfanyl, C₁-C₄-Alkylsulfinyl, C₁-C₄-Alkylsulfonyl, *N*-C₁-C₄-Alkylamino, *N,N*-Di-C₁-C₄-alkylamino stehen.

Noch eine weitere Ausführungsform der vorliegenden Erfindung betrifft Verbindungen gemäß den hierin beschriebenen Formeln, wobei R³ und R⁴ unabhängig voneinander für H, Halogen, Cyano, Nitro, jeweils gegebenenfalls substituiertes C₁-C₄-Alkyl, C₃-C₄-Cycloalkyl, C₁-C₄-Alkoxy, *N*-C₁-C₄-Alkoxyimino-C₁-C₄-alkyl, C₁-C₄-Alkylsulfanyl, C₁-C₄-Alkylsulfinyl, C₁-C₄-Alkylsulfonyl, *N*-C₁-C₄-Alkylamino, oder *N,N*-Di-C₁-C₄-alkylamino stehen.

Noch eine weitere Ausführungsform der vorliegenden Erfindung betrifft Verbindungen gemäß den hierin beschriebenen Formeln, wobei Q für mit Fluor oder mit Carbonamid (-C(=O)N(R)₂, wobei R unabhängig voneinander für H, C₁-C₃-Alkyl oder mit Halogen substituiertes C₁-C₃-Alkyl steht) substituiertes C₁-C₄-Alkyl, gegebenenfalls mit Cyano oder Fluor substituiertes C₃-C₄-Cycloalkyl, C₄-C₆-Heterocycloalkyl, 1-Oxido-thietan-3-yl, 1,1-Dioxido-thietan-3-yl, Benzyl, Pyridin-2-ylmethyl, Methylsulfonyl, oder 2-oxo-2-(2,2,2-trifluoroethylamino)ethyl steht.

Noch eine weitere Ausführungsform der vorliegenden Erfindung betrifft Verbindungen gemäß den hierin beschriebenen Formeln, wobei Q für 2,2,2-Trifluorethyl, 2,2-Difluorethyl, 3,3,3-Trifluorpropyl, Cyclopropyl, Cyclobutyl, Cyclopropyl, Cyclobutyl, 1-Cyano-cyclopropyl, trans-2-Fluorcyclopropyl, oder cis-2-Fluorcyclopropyl, Oxetan-3-yl, Thietan-3-yl, 1-Oxido-thietan-3-yl, 1,1-Dioxido-thietan-3-yl, Benzyl, Pyridin-2-ylmethyl, Methylsulfonyl, oder 2-oxo-2-(2,2,2-trifluoroethylamino)ethyl steht.

Ein weiterer Aspekt betrifft insektizide Mittel, gekennzeichnet durch einen Gehalt von mindestens einer Verbindung der Formel (Ia") wie hierin beschrieben und einem Streckmittel und/oder einer oberflächenaktiven Substanz.

Ein weiterer Aspekt betrifft ein Verfahren zum Schutz von transgenem oder konventionellem Saatgut und der daraus entstehenden Pflanze vor dem Befall von Schädlingen, dadurch gekennzeichnet, dass das Saatgut mit mindestens einer Verbindung der Formel (Ia") wie hierin beschrieben behandelt wird.

Noch ein weitere Aspekt betrifft Verbindungen der Formel (Ia") wie hierin beschrieben oder von einem insektiziden Mittel wie hierin beschrieben zum Bekämpfen von Schädlingen.

Ein weiterer Aspekt betrifft Verbindungen der Formel (Ia") wie hierin beschrieben in der Vektorkontrolle.

Noch ein weiterer Aspekt betrifft Saatgut, bei dem eine Verbindung der Formel (Ia") wie hierin beschrieben als Bestandteil einer Umhüllung oder als weitere Schicht oder weitere Schichten zusätzlich zu einer Umhüllung auf das Saatgut aufgebracht ist.

Entsprechend betrifft ein weiterer Aspekt ein Verfahren zum Aufbringen einer Umhüllung umfassend mindestens eine Verbindung der Formel (Ia") wie hierin beschrieben oder zum Aufbringen einer Verbindung der Formel (Ia") wie hierin beschrieben, die als Schicht oder weitere Schichten zusätzlich zu einer Umhüllung auf Saatgut aufgebracht wird, umfassend die Schritte, a) mischen von Samen mit einem Überzugsmaterial bestehend aus oder umfassend eine Verbindung der Formel (Ia") wie hierin beschrieben, b) anreichern der erhaltenen übergezogenen Samenmasse, c) trocknen der erhaltenen angereicherten Samenmasse, d) entballen (dis- oder entagglomerieren) der erhaltenen getrockneten Samenmasse.

Die hier beschriebenen Verbindungen der Formel (Ia") können gegebenenfalls in Abhängigkeit von der Art der Substituenten als geometrische und/oder als optisch aktive Isomere oder entsprechende Isomerengemische in unterschiedlicher Zusammensetzung vorliegen. Die Erfindung betrifft sowohl die reinen Isomere als auch die Isomerengemische.

Die erfindungsgemäßen Verbindungen können auch als Metallkomplexe vorliegen.

### Definitionen

Der Fachmann ist sich bewusst, dass, wenn nicht ausdrücklich angegeben, die Ausdrücke "ein", "eine" oder "eines" wie in dieser Anmeldung genutzt je nach Situation "ein/eine/eines (1)", "ein/eine/eines (1) oder mehr" oder "mindestens ein/eine/eines (1)" bedeuten kann.

Für alle hierin beschriebenen Strukturen wie cyclischen Systeme und Gruppen gilt, dass benachbarte Atome nicht -O-O- oder -O-S- sein dürfen.

Strukturen mit einer variablen Anzahl an möglichen Kohlenstoffatomen (C-Atomen) können in der vorliegenden Anmeldung als C_{untere Grenze C-Atome}-C_{obere Grenze C-Atome}-Strukturen (C_{uG}-C_{oG}-Strukturen) bezeichnet werden, umso näher bestimmt zu werden. Beispiel: eine Alkylgruppe kann aus 3 bis 10 C-Atomen bestehen und entspricht dann C₃-C₁₀-Alkyl. Ringstrukturen aus C-Atomen und Heteroatomen können als "uG bis oG-gliedrige" Strukturen bezeichnet werden. Ein Beispiel einer 6-gliedrigen Ringstruktur ist Toluol (eine 6-gliedriges Ringstruktur, die mit einer Methylgruppe substituiert ist).

Steht ein Sammelbegriff für einen Substituenten, z. B. C_{uG}-C_{oG}-Alkyl, am Ende eines zusammengesetzten Substituenten wie z.B. bei C_{uG}-C_{oG}-Cycloalkyl-C_{uG}-C_{oG}-Alkyl, so kann der am Anfang stehende Bestandteil des zusammengesetzten Substituenten, z.B. das C_{uG}-C_{oG}-Cycloalkyl, ein-bzw. mehrfach, gleich oder verschieden und unabhängig voneinander mit dem letzten Substituenten, z. B. C_{uG}-C_{oG}-Alkyl, substituiert sein. Alle in dieser Anmeldung verwendeten Sammelbegriffe für chemische Gruppen, cyclische Systeme und cyclische Gruppen können durch den Zusatz "C_{uG}-C_{oG}" oder "uG bis oG-gliedrig(e)" näher bestimmt werden.

Die Definition für Sammelbegriffe solange nicht anders definiert gilt auch für diese Sammelbegriffe in zusammengesetzten Substituenten. Beispiel: Die Definition für C_{uG}-C_{oG}-Alkyl gilt auch für C_{uG}-C_{oG}-Alkyl als Bestandteil eines zusammengesetzten Substituenten wie z.B. C_{uG}-C_{oG}-Cycloalkyl-C_{uG}-C_{oG}-Alkyl.

Dem Fachmann ist klar, dass in dieser Anmeldung genannte Beispiele nicht als beschränkend anzusehen sind sondern lediglich einige Ausführungsformen näher beschreiben.

Bei den in den vorstehenden Formeln angegebenen Definitionen der Symbole wurden Sammelbegriffe verwendet, die allgemein repräsentativ für die folgenden Substituenten stehen:

Halogen bezieht sich auf die Elemente der 7. Hauptgruppe, bevorzugt Fluor, Chlor, Brom und Iod, mehr bevorzugt Fluor, Chlor und Brom und noch bevorzugter Fluor und Chlor.

Beispiele für Heteroatom sind N, O, S, P, B, Si. Bevorzugt bezieht sich der Begriff Heteroatom auf N, S und O.

Erfindungsgemäß steht "Alkyl" - in Alleinstellung oder als Bestandteil einer chemischen Gruppe - für geradkettige oder verzweigte Kohlenwasserstoffe, vorzugsweise mit 1 bis 6 Kohlenstoffatomen, wie beispielsweise Methyl, Ethyl, n-Propyl, Isopropyl, n-Butyl, Isobutyl, s-Butyl, t-Butyl, Pentyl, 1-Methylbutyl, 2-Methylbutyl, 3-Methylbutyl, 1,2-Dimethylpropyl, 1,1-Dimethylpropyl, 2,2-Dimethylpropyl, 1-Ethylpropyl, Hexyl, 1-Methylpentyl, 2-Methylpentyl, 3-Methylpentyl, 4-Methylpentyl, 1,2-Dimethylpropyl, 1,3-Dimethylbutyl, 1,4-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl und 2-Ethylbutyl. Ferner bevorzugt sind Alkyle mit 1 bis 4 Kohlenstoffatomen, wie unter anderem Methyl, Ethyl, n-Propyl, Isopropyl, n-Butyl, Isobutyl, s-Butyl oder t-Butyl. Die erfindungsgemäßen Alkyle können mit einem oder mehreren, gleichen oder verschiedenen Resten substituiert sein.

Erfindungsgemäß steht "Alkenyl" - in Alleinstellung oder als Bestandteil einer chemischen Gruppe - für geradkettige oder verzweigte Kohlenwasserstoffe, vorzugsweise mit 2 bis 6 Kohlenstoffatomen und mindestens einer Doppelbindung, wie beispielsweise Vinyl, 2-Propenyl, 2-Butenyl, 3-Butenyl, 1-Methyl-2-propenyl, 2-Methyl-2-propenyl, 2-Pentenyl, 3-Pentenyl, 4-Pentenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-2-propenyl, 1-Ethyl-2-propenyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-dimethyl-3-butenyl, 1,2-Dimethyl-2-butenyl, 1,2-Dimethyl-3-butenyl, 1,3-Dimethyl-2-butenyl, 2,2-Dimethyl-3-butenyl, 2,3-Dimethyl-2-butenyl, 2,3-Dimethyl-3-butenyl, 1-Ethyl-2-butenyl, 1-Ethyl-3-butenyl, 2-Ethyl-2-butenyl, 2-Ethyl-3-butenyl, 1,1,2-Trimethyl-2-propenyl, 1-Ethyl-1-methyl-2-propenyl und 1-Ethyl-2-methyl-2-propenyl. Ferner bevorzugt sind Alkenyle mit 2 bis 4 Kohlenstoffatomen, wie unter anderem 2-Propenyl, 2-Butenyl oder 1-Methyl-2-propenyl. Die erfindungsgemäßen Alkenyle können mit einem oder mehreren, gleichen oder verschiedenen Resten substituiert sein.

Erfindungsgemäß steht "Alkinyl" - in Alleinstellung oder als Bestandteil einer chemischen Gruppe - für geradkettige oder verzweigte Kohlenwasserstoffe, vorzugsweise mit 2 bis 6 Kohlenstoffatomen und mindestens einer Dreifachbindung wie beispielsweise 2-Propinyl, 2-Butinyl, 3-Butinyl, 1-Methyl-2-propinyl, 2-Pentinyl, 3-Pentinyl, 4-Pentinyl, 1-Methyl-3-butinyl, 2-Methyl-3-butinyl, 1-Methyl-2-butinyl, 1,1-Dimethyl-2-propinyl, 1-Ethyl-2-propinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-4-pentinyl, 4-Methyl-2-pentinyl, 1,1-Dimethyl-3-butinyl, 1,2-Dimethyl-3-butinyl, 2,2-Dimethyl-3-butinyl, 1-Ethyl-3-butinyl, 2-Ethyl-3-butinyl, 1-Ethyl-1-methyl-2-propinyl und 2,5-Hexadiynyl. Ferner bevorzugt sind Alkinyle mit 2 bis 4 Kohlenstoffatomen wie unter anderem Ethinyl, 2-Propinyl oder 2-Butinyl-2-propenyl. Die erfindungsgemäßen Alkinyle können mit einem oder mehreren, gleichen oder verschiedenen Resten substituiert sein.

Erfindungsgemäß steht "Cycloalkyl" - in Alleinstellung oder als Bestandteil einer chemischen Gruppe - für mono-, bi- oder tricyclische Kohlenwasserstoffe, vorzugsweise mit 3 bis 10 Kohlenstoffen wie beispielsweise Cyclopropyl, Cyclobutyl, Cyclopentyl, Cyclohexyl, Cycloheptyl, Cyclooctyl, Bicyclo[2.2.1]heptyl, Bicyclo[2.2.2]octyl oder Adamantyl. Ferner bevorzugt sind Cycloalkyle mit 3, 4, 5, 6 oder 7 Kohlenstoffatomen, wie unter anderem Cyclopropyl oder Cyclobutyl. Die erfindungsgemäßen Cycloalkyle können mit einem oder mehreren, gleichen oder verschiedenen Resten substituiert sein.

Erfindungsgemäß steht "Alkylcycloalkyl" für mono-, bi- oder tricyclisches Alkylcycloalkyl, vorzugsweise mit 4 bis 10 oder 4 bis 7 Kohlenstoffatomen, wie beispielsweise Methylcyclopropyl, Ethylcyclopropyl, Isopropylcyclobutyl, 3-Methylcyclopentyl und 4-Methyl-cyclohexyl. Ferner bevorzugt sind Alkylcycloalkyle mit 4, 5 oder 7 Kohlenstoffatomen wie unter anderen Ethylcyclopropyl oder 4-Methyl-cyclohexyl. Die erfindungsgemäßen Alkylcycloalkyle können mit einem oder mehreren, gleichen oder verschiedenen Resten substituiert sein.

Erfindungsgemäß steht "Cycloalkylalkyl" für mono, bi- oder tricyclisches Cycloalkylalkyl, vorzugsweise mit 4 bis 10 oder 4 bis 7 Kohlenstoffatomen, wie beispielsweise Cyclopropylmethyl, Cyclobutylmethyl, Cyclopentylmethyl, Cyclohexylmethyl und Cyclopentylethyl. Ferner bevorzugt sind Cycloalkylalkyle mit 4, 5 oder 7 Kohlenstoffatomen wie unter anderen Cyclopropylmethyl oder Cyclobutylmethyl. Die erfindungsgemäßen Cycloalkylalkyle können mit einem oder mehreren, gleichen oder verschiedenen Resten substituiert sein.

Erfindungsgemäß steht "Hydroxyalkyl" für geradkettigen oder verzweigten Alkohol, vorzugsweise mit 1 bis 6 Kohlenstoffatomen, wie beispielsweise Methanol, Ethanol, n-Propanol, Isopropanol, n-Butanol, Isobutanol, s-Butanol und t-Butanol. Ferner bevorzugt sind Hydroxyalkylgruppen mit 1 bis 4 Kohlenstoffatomen. Die erfindungsgemäßen Hydroxyalkylgruppen können mit einem oder mehreren, gleichen oder verschiedenen Resten substituiert sein

Erfindungsgemäß steht "Alkoxy" für geradkettiges oder verzweigtes O-Alkyl, vorzugsweise mit 1 bis 6 Kohlenstoffatomen, wie beispielsweise Methoxy, Ethoxy, n-Propoxy, Isopropoxy, n-Butoxy, Isobutoxy, s-Butoxy und t-Butoxy. Ferner bevorzugt sind Alkoxygruppen mit 1 bis 4 Kohlenstoffatomen. Die erfindungsgemäßen Alkoxygruppen können mit einem oder mehreren, gleichen oder verschiedenen Resten substituiert sein.

Erfindungsgemäß steht "Alkylsulfanyl" für geradkettiges oder verzweigtes S-Alkyl, vorzugsweise mit 1 bis 6 Kohlenstoffatomen, wie beispielsweise Methylthio, Ethylthio, n-Propylthio, Isopropylthio, n-Butylthio, Isobutylthio, s-Butylthio und t-Butylthio. Ferner bevorzugt sind Alkylsulfanylgruppen mit 1 bis 4 Kohlenstoffatomen. Die erfindungsgemäßen Alkylsulfanylgruppen können mit einem oder mehreren, gleichen oder verschiedenen Resten substituiert sein.

Erfindungsgemäß steht "Alkylsulfinyl" für geradkettiges oder verzweigtes Alkylsulfinyl, vorzugsweise mit 1 bis 6 Kohlenstoffatomen wie beispielsweise Methylsulfinyl, Ethylsulfinyl, n-Propylsulfinyl, Isopropylsulfinyl, n-Butylsulfinyl, Isobutylsulfinyl, s-Butylsulfinyl und t-Butylsulfinyl. Ferner bevorzugt sind Alkylsulfinylgruppen mit 1 bis 4 Kohlenstoffatomen. Die erfindungsgemäßen Alkylsulfinylgruppen können mit einem oder mehreren, gleichen oder verschiedenen Resten substituiert sein.

Erfindungsgemäß steht "Alkylsulfonyl" für geradkettiges oder verzweigtes Alkylsulfonyl, vorzugsweise mit 1 bis 6 Kohlenstoffatomen wie beispielsweise Methylsulfonyl, Ethylsulfonyl, n-Propylsulfonyl, Isopropylsulfonyl, n-Butylsulfonyl, Isobutylsulfonyl, s-Butylsulfonyl und t-Butylsulfonyl. Ferner bevorzugt sind Alkylsulfonylgruppen mit 1 bis 4 Kohlenstoffatomen. Die erfindungsgemäßen Alkylsulfonylgruppen können mit einem oder mehreren, gleichen oder verschiedenen Resten substituiert sein.

Erfindungsgemäß steht "Alkylcarbonyl" für geradkettiges oder verzweigtes Alkyl-C(=O), vorzugsweise mit 2 bis 7 Kohlenstoffatomen, wie Methylcarbonyl, Ethylcarbonyl, n-Propylcarbonyl, Isopropylcarbonyl, s-Butylcarbonyl und t-Butylcarbonyl. Ferner bevorzugt sind Alkylcarbonyle mit 1 bis 4 Kohlenstoffatomen. Die erfindungsgemäßen Alkylcarbonyle können mit einem oder mehreren, gleichen oder verschiedenen Resten substituiert sein.

Erfindungsgemäß steht "Cycloalkylcarbonyl" für geradkettiges oder verzweigtes Cycloalkylcarbonyl, vorzugsweise mit 3 bis 10 Kohlenstoffatomen im Cycloalkylteil, wie beispielsweise Cyclopropylcarbonyl, Cyclobutylcarbonyl, Cyclopentylcarbonyl, Cyclohexyl-carbonyl, Cycloheptylcarbonyl, Cyclooctylcarbonyl, Bicyclo[2.2.1]heptyl, Bycyclo[2.2.2]octylcarbonyl und Adamantylcarbonyl. Ferner bevorzugt sind Cycloalkylcarbonyl mit 3, 5 oder 7 Kohlenstoffatomen im Cycloalkylteil. Die erfindungsgemäßen Cycloalkylcarbonylgruppen können mit einem oder mehreren, gleichen oder verschiedenen Resten substituiert sein.

Erfindungsgemäß steht "Alkoxycarbonyl" - in Alleinstellung oder als Bestandteil einer chemischen Gruppe - für geradkettiges oder verzweigtes Alkoxycarbonyl, vorzugsweise mit 1 bis 6 Kohlenstoffatomen oder 1 bis 4 Kohlenstoffatomen im Alkoxyteil, wie beispielsweise Methoxycarbonyl, Ethoxycarbonyl, n-Propoxycarbonyl, Isopropoxycarbonyl, s-Butoxycarbonyl und t-Butoxycarbonyl. Die erfindungsgemäßen Alkoxycarbonylgruppen können mit einem oder mehreren, gleichen oder verschiedenen Resten substituiert sein.

Erfindungsgemäß steht "Alkylaminocarbonyl" für geradkettiges oder verzweigtes Alkylaminocarbonyl mit vorzugsweise 1 bis 6 Kohlenstoffatomen oder 1 bis 4 Kohlenstoffatomen im Alkylteil, wie beispielsweise Methylaminocarbonyl, Ethylaminocarbonyl, n-Proylaminocarbonyl, Isopropylaminocarbonyl, s-Butylaminocarbonyl und t-Butylaminocarbonyl. Die erfindungsgemäßen Alkylaminocarbonylgruppen können mit einem oder mehreren, gleichen oder verschiedenen Resten substituiert sein.

Erfindungsgemäß steht "*N,N-*Dialkylamino-carbonyl" für geradkettiges oder verzweigtes *N,N-*Dialkylaminocarbonyl mit vorzugsweise 1 bis 6 Kohlenstoffatomen oder 1 bis 4 Kohlenstoffatomen im Alkylteil, wie beispielsweise *N,N*-Dimethylamino-carbonyl, N.N-Diethylamino-carbonyl, *N,N*-Di(n-propylamino)-carbonyl, *N,N-*Di-(isopropylamino)-carbonyl und *N,N-*Di-(s-butylamino)-carbonyl. Die erfindungsgemäßen *N,N-*Dialkylamino-carbonylgruppen können mit einem oder mehreren, gleichen oder verschiedenen Resten substituiert sein.

Erfindungsgemäß steht "Aryl" für ein mono-, bi- oder polycyclisches aromatisches System mit vorzugsweise 6 bis 14, insbesondere 6 bis 10 Ring-Kohlenstoffatomen, wie beispielsweise Phenyl, Naphthyl, Anthryl, Phenanthrenyl, vorzugsweise Phenyl. Ferner steht Aryl auch für mehrcyclische Systeme, wie Tetrahydronaphtyl, Indenyl, Indanyl, Fluorenyl, Biphenyl, wobei die Bindungsstelle am aromatischen System ist. Die erfindungsgemäßen Arylgruppen können mit einem oder mehreren, gleichen oder verschiedenen Resten substituiert sein.

Beispiele substitutierter Aryle stellen die Arylalkyle dar, die gleichfalls mit einem oder mehreren, gleichen oder verschiedenen Resten im C₁-C₄-Alkyl- und/oder C₆-C₁₄-Arylteil substituiert sein können. Beispiele solcher Arylalkyle sind unter anderem Benzyl und 1-Phenylethyl.

Erfindungsgemäß steht "Heterocyclus", "heterocyclischer Ring" oder "heterocyclisches Ringsystem" für ein carbocyclisches Ringsystem mit mindestens einem Ring, in dem mindestens ein Kohlenstoffatom durch ein Heteroatom ersetzt ist, vorzugsweise durch ein Heteroatom aus der Gruppe N, O, S, P, B, Si, Se und der gesättigt, ungesättigt oder heteroaromatisch ist und dabei unsubstituiert oder substituiert sein kann, wobei die Bindungsstelle an einem Ringatom lokalisiert ist. Wenn nicht anders definiert, enthält der heterocyclische Ring vorzugsweise 3 bis 9 Ringatome, insbesondere 3 bis 6 Ringatome, und ein oder mehrere, vorzugsweise 1 bis 4, insbesondere 1, 2 oder 3 Heteroatome im heterocyclischen Ring, vorzugsweise aus der Gruppe N, O, und S, wobei jedoch nicht zwei Sauerstoffatome direkt benachbart sein sollen. Die heterocyclischen Ringe enthalten gewöhnlicherweise nicht mehr als 4 Stickstoffatome, und/oder nicht mehr als 2 Sauerstoffatome und/oder nicht mehr als 2 Schwefelatome. Ist der Heterocyclylrest oder der heterocyclische Ring gegebenenfalls substituiert, kann er mit anderen carbocyclischen oder heterocyclischen Ringen annelliert sein. Im Falle von gegebenenfalls substituiertem Heterocyclyl werden erfindungsgemäß auch mehrcyclische Systeme umfaßt, wie beispielsweise 8-Aza-bicyclo[3.2.1]octanyl oder 1-Aza-bicyclo[2.2.1]heptyl. Im Falle von gegebenenfalls substituiertem Heterocyclyl werden erfindungsgemäß auch spirocyclische Systeme umfasst, wie beispielsweise 1-Oxa-5-aza-spiro[2.3]hexyl.

Erfindungsgemäße Heterocyclylgruppen sind beispielsweise Piperidinyl, Piperazinyl, Morpholinyl, Thiomorpholinyl, Dihydropyranyl, Tetrahydropyranyl, Dioxanyl, Pyrrolinyl, Pyrrolidinyl, Imidazolinyl, Imidazolidinyl, Thiazolidinyl, Oxazolidinyl, Dioxolanyl, Dioxolyl, Pyrazolidinyl, Tetrahydrofuranyl, Dihydrofuranyl, Oxetanyl, Oxiranyl, Azetidinyl, Aziridinyl, Oxazetidinyl, Oxaziridinyl, Oxazepanyl, Oxazinanyl, Azepanyl, Oxopyrrolidinyl, Dioxopyrrolidinyl, Oxomorpholinyl, Oxopiperazinyl und Oxepanyl.

Eine besondere Bedeutung kommt Heteroarylen, also heteroaromatischen Systemen zu. Erfindungsgemäß steht der Ausdruck Heteroaryl für heteroaromatische Verbindungen, das heißt vollständig ungesättigte aromatische heterocyclische Verbindungen, die unter die vorstehende Definiton von Heterocyclen fallen. Vorzugsweise für 5- bis 7-gliedrige Ringe mit 1 bis 3, vorzugsweise 1 oder 2 gleichen oder verschiedenen Heteroatomen aus der oben genannten Gruppe. Erfindungsgemäße Heteroaryle sind beispielsweise Furyl, Thienyl, Pyrazolyl, Imidazolyl, 1,2,3- und 1,2,4-Triazolyl, Isoxazolyl, Thiazolyl, Isothiazolyl, 1,2,3-, 1,3,4-, 1,2,4- und 1,2,5-Oxadiazolyl, Azepinyl, Pyrrolyl, Pyridyl, Pyridazinyl, Pyrimidinyl, Pyrazinyl, 1,3,5-, 1,2,4- und 1,2,3-Triazinyl, 1,2,4-, 1,3,2-, 1,3,6- und 1,2,6-Oxazinyl, Oxepinyl, Thiepinyl, 1,2,4-Triazolonyl und 1,2,4-Diazepinyl. Die erfindungsgemäßen Heteroarylgruppen können ferner mit einem oder mehreren, gleichen oder verschiedenen Resten substituiert sein.

Der Begriff "(gegebenenfalls) substituierte" Gruppen/Substituenten, wie ein substituierter Alkyl-, Alkenyl-, Alkinyl-, Alkoxy-, Alkylsulfanyl-, Alkylsulfinyl-, Alkylsulfonyl-, Cycloalkyl-, Aryl-, Phenyl-, Benzyl-, Heterocyclyl- und Heteroarylrest, bedeutet beispielsweise einen vom unsubstituierten Grundkörper abgeleiteten substituierten Rest, wobei die Substituenten beispielsweise ein (1) Substituent oder mehrere Substituenten, vorzugsweise 1, 2, 3, 4, 5, 6, oder 7, ausgewählt sind aus einer Gruppe bestehend aus Amino, Hydroxy, Halogen, Nitro, Cyano, Isocyano, Mercapto, Isothiocyanato, C₁-C₄-Carboxy, Carbonamid, SF₅, Aminosulfonyl, C₁-C₄-Alkyl, C₃-C₄-Cycloalkyl, C₂-C₄-Alkenyl, C₃-C₄-Cycloalkenyl, C₂-C₄-Alkinyl, *N*-Mono-C₁-C₄-alkyl-amino, *N,N*-Di-C₁-C₄-alkylamino, *N*-C₁-C₄-Alkanoylamino, C₁-C₄-Alkoxy, C₂-C₄-Alkenyloxy, C₂-C₄-Alkinyloxy, C₃-C₄-Cycloalkoxy, C₃-C₄-Cycloalkenyloxy, C₁-C₄-Alkoxycarbonyl, C₂-C₄- C₂-C₄-Alkenyloxycarbonyl, C₂-C₄-Alkinyloxycarbonyl, C₆-,C₁₀-,C₁₄-Aryloxycarbonyl, C₁-C₄-Alkanoyl, C₂-C₄-Alkenylcarbonyl, C₂-C₄-Alkinylcarbonyl, C₆-,C₁₀-,C₁₄-Arylcarbonyl, C₁-C₄-Alkylsulfanyl, C₃-C₄-Cycloalkylsulfanyl, C₁-C₄-Alkylthio, C₂-C₄-Alkenylthio, C₃-C₄-Cycloalkenylthio, C₂-C₄-Alkinylthio, C₁-C₄-Alkylsulfenyl und C₁-C₄-Alkylsulfinyl, wobei beide Enantiomere der C₁-C₄-Alkylsulfinylgruppe umfasst sind, C₁-C₄-Alkylsulfonyl, *N*-Mono-C₁-C₄-alkyl-aminosulfonyl, *N,N*-Di-C₁-C₄-alkyl-aminosulfonyl, C₁-C₄-Alkylphosphinyl, C₁-C₄-Alkylphosphonyl, wobei für C₁-C₄-Alkylphosphinyl bzw. C₁-C₄-Alkylphosphonyl beide Enantiomere umfasst sind, *N*-C₁-C₄-Alkyl-aminocarbonyl, *N,N*-Di-C₁-C₄-alkyl-amino-carbonyl, N-C₁-C₄-Alkanoyl-amino-carbonyl, N-C₁-C₄-Alkanoyl-N-C₁-C₄-alkyl-aminocarbonyl, C₆-,C₁₀-,C₁₄-Aryl, C₆-,C₁₀-,C₁₀-Aryloxy, Benzyl, Benzyloxy, Benzylthio, C₆-,C₁₀-,C₁₄-Arylthio, C₆-,C₁₀-,C₁₄-Arylamino, Benzylamino, Heterocyclyl und Trialkylsilyl, mit einer Doppelbindung verbundene Substituenten wie C₁-C₄-Alkyliden (z. B. Methyliden oder Ethyliden), eine Oxogruppe, eine Thioxogruppe, eine Iminogruppe sowie einer substituierten Iminogruppe. Wenn zwei oder mehrere Reste einen oder mehrere Ringe bilden, so können diese carbocyclisch, heterocyclisch, gesättigt, teilgesättigt, ungesättigt, beispielsweise auch aromatisch und weiter substituiert sein.

Die beispielhaft genannten Substituenten ("erste Substituentenebene") können, sofern sie kohlenwasserstoffhaltige Anteile enthalten, dort gegebenenfalls weiter substituiert sein ("zweite Substitutentenebene"), beispielsweise durch einen oder mehreren der Substituenten jeweils unabhängig voneinander ausgewählt aus Halogen, Hydroxy, Amino, Nitro, Cyano, Isocyano, Azido, Acylamino, einer Oxogruppe und einer Iminogruppe. Vorzugsweise werden vom Begriff "(gegebenenfalls) substituierter" Gruppe nur ein oder zwei Substitutentenebenen umfasst.

Die erfindungsgemäßen mit Halogen substituierten chemischen Gruppen bzw. halogenierte Gruppen (wie z. B. Alkyl oder Alkoxy) sind einfach oder mehrfach bis zur maximal möglichen Substituentenzahl mit Halogen substituiert. Solche Gruppen werden auch als Halogruppen bezeichnet (wie, Z. B. Haloalkyl). Bei mehrfacher Substitution mit Halogen, können die Halogenatome gleich oder verschieden sein und können alle an eines oder an mehrere Kohlenstoffatome gebunden sein. Dabei steht Halogen insbesondere für Fluor, Chlor, Brom oder Iod, vorzugsweise für Fluor, Chlor oder Brom und besonders bevorzugt für Fluor. Insbesondere sind mit Halogen substituierte Gruppen Monohalocycloalkyl wie 1-Fluor-cyclopropyl, 2-Fluor-cyclopropyl oder 1-Fluor-cyclobutyl, Monohaloalkyl wie 2-Chlor-ethyl, 2-Fluor-ethyl, 1-Chlor-ethyl, 1-Fluor-ethyl, Chlormethyl, oder Fluormethyl; Perhaloalkyl wie Trichlormethyl oder Trifluormethyl oder CF₂CF₃, Polyhaloalkyl wie Difluormethyl, 2-Fluor-2-Chlor-ethyl, Dichlormethyl, 1,1,2,2-Tetraflourethyl, oder 2,2,2-Trifluorethyl. Weitere Beispiele für Halogenalkyle sind Trichlormethyl, Chlordifluormethyl, Dichlorfluormethyl, Chlormethyl, Brommethyl, 1-Fluorethyl, 2-Fluorethyl, 2,2-Difluorethyl, 2,2,2-Trifluorethyl, 2,2,2-Trichlorethyl, 2-Chlor-2,2-difluorethyl, Pentafluorethyl, 3,3,3-Trifluorpropyl und Pentafluor-t-butyl. Bevorzugt sind Halogenalkyle mit 1 bis 4 Kohlenstoffatomen und 1 bis 9, vorzugsweise 1 bis 5 gleichen oder verschiedenen Halogenatomen, die ausgewählt sind unter Fluor, Chlor oder Brom. Besonders bevorzugt sind Halogenalkyle mit 1 oder 2 Kohlenstoffatomen und mit 1 bis 5 gleichen oder verschiedenen Halogenatomen, die ausgewählt sind unter Fluor oder Chlor, wie unter anderen Difluormethyl, Trifluormethyl oder 2,2-Difluorethyl. Weitere Beispiele für mit Halogen substituierten Verbindungen sind Haloalkoxy wie OCF₃, OCHF₂, OCH₂F, OCF₂CF₃, OCH₂CF₃, OCH₂CHF₂ und OCH₂CH₂Cl, Halogenalkylsulfanyle wie Difluormethylthio, Trifluormethylthio, Trichlormethylthio, Chlordifluormethylthio, 1-Fluorethylthio, 2-Fluorethylthio, 2,2-Difluorethylthio, 1,1,2,2-Tetrafluorethylthio, 2,2,2-Trifluorethylthio oder 2-Chlor-1,1,2-trifluorethylthio, Halogenalkylsulfinyle wie Difluormethylsulfinyl, Trifluormethylsulfinyl, Trichlormethylsulfinyl, Chlordifluormethylsulfinyl, 1-Fluorethylsulfinyl, 2-Fluorethylsulfinyl, 2,2-Difluorethylsulfinyl, 1,1,2,2-Tetrafluorethylsulfinyl, 2,2,2-Trifluorethylsulfinyl und 2-Chlor-1,1,2-trifluorethylsulfinyl, Halogenalkylsulfinyle wie Difluormethylsulfinyl, Trifluormethylsulfinyl, Trichlormethylsulfinyl, Chlordifluormethylsulfinyl, 1-Fluorethylsulfinyl, 2-Fluorethylsulfinyl, 2,2-Difluorethylsulfinyl, 1,1,2,2-Tetrafluorethylsulfinyl, 2,2,2-Trifluorethylsulfinyl und 2-Chlor-1,1,2-trifluorethylsulfinyl, Halogenalkylsulfonylgrupen wie Difluormethylsulfonyl, Trifluormethylsulfonyl, Trichlormethylsulfonyl, Chlordifluormethylsulfonyl, 1-Fluorethylsulfonyl, 2-Fluorethylsulfonyl, 2,2-Difluorethylsulfonyl, 1,1,2,2-Tetrafluorethylsulfonyl, 2,2,2-Trifluorethylsulfonyl und 2-Chlor-1,1,2-trifluorethylsulfonyl.

Bei Resten mit C-Atomen sind solche mit 1 bis 4 C-Atomen, insbesondere 1 oder 2 C-Atomen bevorzugt. Bevorzugt sind in der Regel Substituenten aus der Gruppe Halogen, z.B. Fluor und Chlor, (C₁-C₄)Alkyl, vorzugsweise Methyl oder Ethyl, (C₁-C₄)Haloalkyl, vorzugsweise Trifluormethyl, (C₁-C₄)Alkoxy, vorzugsweise Methoxy oder Ethoxy, (C₁-C₄)Haloalkoxy, Nitro und Cyano. Besonders bevorzugt sind dabei die Substituenten Methyl, Methoxy, Fluor und Chlor.

Substituiertes Amino wie mono- oder disubstituiertes Amino bedeutet einen Rest aus der Gruppe der substituierten Aminoreste, welche beispielsweise durch einen bzw. zwei gleiche oder verschiedene Reste aus der Gruppe Alkyl, Hydroxy, Amino, Alkoxy, Acyl und Aryl *N*-substituiert sind; vorzugsweise *N*-Mono- und *N,N*-Dialkylamino, (z.B. Methylamino, Ethylamino, *N,N*-Dimethylamino, *N,N-*Diethylamino, *N,N*-Di-n-propylamino, *N,N*-Diisopropylamino oder *N,N*-Dibutylamino), *N*-Mono- oder *N,N-*Dialkoxyalkylaminogruppen (z.B. *N*-Methoxymethylamino, *N*-Methoxyethylamino, *N,N*-Di-(methoxymethyl)-amino oder *N,N-*Di-(methoxyethyl)-amino), *N*-Mono- und *N,N-*Diarylamino, wie gegebenenfalls substituierte Aniline, Acylamino, *N,N*-diacylamino, *N*-Alkyl-*N*-arylamino, *N*-Alkyl-*N-*acylamino sowie gesättigte *N*-Heterocyclen; dabei sind Alkylreste mit 1 bis 4 C-Atomen bevorzugt; Aryl ist dabei vorzugsweise Phenyl oder substituiertes Phenyl; für Acyl gilt dabei die weiter unten genannte Definition, vorzugsweise (C₁-C₄)Alkanoyl. Entsprechenes gilt für substituiertes Hydroxylamino oder Hydrazino.

Erfindungsgemäß umfasst der Begriff "cyclische Aminogruppen" heteroaromatische oder aliphatische Ringsysteme mit einem oder mehreren Stickstoffatomen. Die Heterocyclen sind gesättigt oder ungesättigt, bestehen aus einem oder mehreren, gegebenenfalls kondensierten Ringsystemen und beinhalten gegebenenfalls weitere Heteroatome, wie beispielsweise ein oder zwei Stickstoff-, Sauerstoff- und/oder Schwefelatome. Ferner umfasst der Begriff auch solche Gruppen, die einen Spiroring oder verbrücktes Ringsystem aufweisen. Die Anzahl der Atome, die die cyclische Aminogruppe bilden, ist beliebig und kann z.B. im Falle eines Einringsystems aus 3 bis 8 Ringatomen und im Falle eines Zweiringsystems aus 7 bis 11 Atomen bestehen.

Beispielhaft für cyclische Aminogruppen mit gesättigten und ungesättigten monocyclischen Gruppen mit einem Stickstoffatom als Heteroatom seien 1-Azetidinyl, Pyrrolidino, 2-Pyrrolidin-1-yl, 1-Pyrrolyl, Piperidino, 1,4-Dihydropyrazin-1-yl, 1,2,5,6-Tetrahydropyrazin-1-yl, 1,4-Dihydropyridin-1-yl, 1,2,5,6-Tetrahydropyridin-1-yl, Homopiperidinyl genannt; beispielhaft für cyclische Aminogruppen mit gesättigten und ungesättigten monocyclischen Gruppen mit zwei oder mehreren Stickstoffatomen als Heteroatome seien 1-Imidazolidinyl, 1-Imidazolyl, 1-Pyrazolyl, 1-Triazolyl, 1-Tetrazolyl, 1-Piperazinyl, 1-Homopiperazinyl, 1,2-Dihydro-piperazin-1-yl, 1,2-Dihydro-pyrimidin-1-yl, Perhydropyrimidin-1-yl, 1,4-Diazacycloheptan-1-yl, genannt; beispielhaft für cyclische Aminogruppen mit gesättigten und ungesättigten monocyclischen Gruppen mit einem oder zwei Sauerstoffatomen und einem bis drei Stickstoffatomen als Heteroatome, wie beispielsweise Oxazolidin-3-yl, 2,3-Dihydroisoxazol-2-yl, Isoxazol-2-yl, 1,2,3-Oxadiazin-2-yl, Morpholino, beispielhaft für cyclische Aminogruppen mit gesättigten und ungesättigten monocyclischen Gruppen mit einem bis drei Stickstoffatomen und einem bis zwei Schwefelatomen als Heteroatome seien Thiazolidin-3-yl, Isothiazolin-2-yl, Thiomorpholino, oder Dioxothiomorpholino genannt; beispielhaft für cyclische Aminogruppen mit gesättigten und ungesättigten kondensierten cyclischen Gruppen seien Indol-1-yl, 1,2-Dihydrobenzimidazol-1-yl, Perhydropyrrolo[1,2-a]pyrazin-2-yl genannt; beispielhaft für cyclische Aminogruppen mit spirocyclischen Gruppen sei das 2-Azaspiro[4,5]decan-2-yl genannt; beispielhaft für cyclische Aminogruppen mit verbrückten heterocyclischen Gruppen sei das 2-Azabicyclo[2,2,1]heptan-7-yl genannt.

Substituiertes Amino schließt auch quartäre Ammoniumverbindungen (Salze) mit vier organischen Substituenten am Stickstoffatom ein.

Gegebenenfalls substituiertes Phenyl ist vorzugsweise Phenyl, das unsubstituiert oder ein- oder mehrfach, vorzugsweise bis zu dreifach durch gleiche oder verschiedene Reste aus der Gruppe Halogen, C₁-C₄₋Alkyl, C₁-C₄₋Alkoxy, C₁-C₄₋Alkoxy-C₁-C₄₋alkoxy, C₁-C₄₋Alkoxy-C₁-C₄₋alkyl, C₁-C₄₋Halogenalkyl, C₁-C₄₋Halogenalkoxy, C₁-C₄₋Alkylsulfanyl, C₁-C₄₋Halogenalkylsulfanyl, Cyano, Isocyano und Nitro substituiert ist, z.B. o-, m- und p-Tolyl, Dimethylphenyle, 2-, 3- und 4-Chlorphenyl, 2-, 3- und 4-Fluorphenyl, 2-, 3- und 4-Trifluormethyl- und -Trichlormethylphenyl, 2,4-, 3,5-, 2,5- und 2,3-Dichlorphenyl, o-, m- und p-Methoxyphenyl, 4-Heptafluorphenyl.

Gegebenenfalls substituiertes Cycloalkyl ist vorzugsweise Cycloalkyl, das unsubstituiert oder ein- oder mehrfach, vorzugsweise bis zu dreifach durch gleiche oder verschiedene Reste aus der Gruppe Halogen, Cyano, C₁-C₄-Alkyl, C₁-C₄-Alkoxy, C₁-C₄-Alkoxy-C₁-C₄-alkoxy, C₁-C₄-Alkoxy-C₁-C₄-alkyl, C₁-C₄-Halogenalkyl und C₁-C₄-Halogenalkoxy substituiert ist, insbesondere durch einen oder zwei C₁-C₄-Alkylreste substituiert ist.

Gegebenenfalls substituiertes Heterocyclyl ist vorzugsweise Heterocyclyl, das unsubstituiert oder ein- oder mehrfach, vorzugsweise bis zu dreifach durch gleiche oder verschiedene Reste aus der Gruppe Halogen, Cyano, C₁-C₄-Alkyl, C₁-C₄-Alkoxy, C₁-C₄-Alkoxy-C₁-C₄-alkoxy, C₁-C₄-Alkoxy-C₁-C₄-alkyl, C₁-C₄-Halogenalkyl, C₁-C₄-Halogenalkoxy, Nitro und Oxo substituiert ist, insbesondere ein- oder mehrfach durch Reste aus der Gruppe Halogen, C₁-C₄-Alkyl, C₁-C₄-Alkoxy, C₁-C₄-Halogenalkyl und Oxo, ganz besonders durch einen oder zwei C₁-C₄-Alkylreste substituiert ist.

Beispiele für Alkyl substituierte Heteroaryle sind Furylmethyl, Thienylmethyl, Pyrazolylmethyl, Imidazolylmethyl, 1,2,3- und 1,2,4-Triazolylmethyl, Isoxazolylmethyl, Thiazolylmethyl, Isothiazolylmethyl, 1,2,3-, 1,3,4-, 1,2,4- und 1,2,5-Oxadiazolylmethyl, Azepinylmethyl, Pyrrolylmethyl, Pyridylmethyl,, Pyridazinylmethyl, Pyrimidinylmethyl, Pyrazinylmethyl, 1,3,5-, 1,2,4- und 1,2,3-Triazinylmethyl, 1,2,4-, 1,3,2-, 1,3,6- und 1,2,6-Oxazinylmethyl, Oxepinylmethyl, Thiepinylmethyl und 1,2,4-Diazepinylmethyl.

Erfindungsgemäße Verbindungen können in bevorzugten Ausführungsformen vorkommen. Einzelne hierin beschriebene Ausführungsformen können dabei miteinander kombiniert werden. Nicht umfasst sind solche Kombinationen, die den Naturgesetzen widersprechen und die der Fachmann daher aufgrund seines Fachwissens ausgeschlossen hätte. Beispielsweise sind Ringstrukturen mit drei oder mehreren benachbarten O-Atomen ausgeschlossen.

Dem Fachmann ist offenbar, dass alle Ausführungsformen alleine oder in Kombination vorliegen können.

Die Verbindungen der Formel (Ia"), insbesondere Verbindungen der Formeln (I-T2), (I-T3), (I-T4), (I-T22) und (I-T23) können gegebenenfalls in Abhängigkeit der Art der Substituenten als Salze, Tautomere, geometrische und/oder optisch aktive Isomere oder entsprechende Isomerengemische in unterschiedlicher Zusammensetzung vorliegen.

Die erfindungsgemäßen Verbindungen können gegebenenfalls in verschiedenen polymorphen Formen oder als Mischung verschiedener polymorpher Formen vorliegen. Sowohl die reinen Polymorphe als auch die Polymorphgemische sind Gegenstand der Erfindung und können erfindungsgemäß verwendet werden.

### Weitere Ausführungsformen (nicht-erfindungsgemäß)

Im folgenden werden Ausführungsformen der Verbindungen der Formel (I) näher beschrieben: 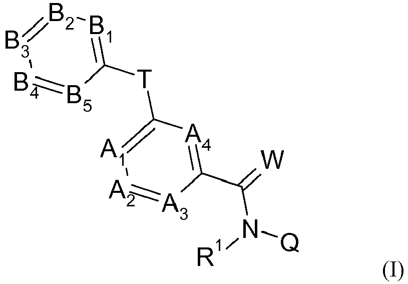 worin
- R¹: für H, jeweils gegebenenfalls substituiertes C₂-C₆-Alkenyl, C₂-C₆-Alkinyl, C₃-C₇-Cycloalkyl, C₁-C₆-Alkylcarbonyl, C₁-C₆-Alkoxycarbonyl, Aryl(C₁-C₃)-alkyl, Heteroaryl(C₁-C₃)-alkyl steht oder für gegebenenfalls substituiertes C₁-C₆-Alkyl steht, bevorzugt für H steht oder bevorzugt für C₁-C₂-Alkyl steht, ganz besonders bevorzugt für H steht oder Methyl steht,
die Gruppierungen
- A₁: für CR² oder N,
- A₂: für CR³ oder N,
- A₃: für CR⁴ oder N,
- A₄: für CR⁵ oder N,
- B₁: für CR⁶ oder N,
- B₂: für CR⁷ oder N,
- B₃: für CR⁸ oder N,
- B₄: für CR⁹ oder N, und
- B₅: für CR¹⁰ oder N stehen,
wobei aber nicht mehr als drei der Gruppierungen A₁ bis A₄ für N und nicht mehr als drei der Gruppierungen B₁ bis B₅ gleichzeitig für N stehen;
- R², R³, R⁴, R⁵, R⁶, R⁷, R⁹ und R¹⁰: unabhängig voneinander für H, Halogen, Cyano, Nitro, jeweils gegebenenfalls substituiertes C₁-C₆-Alkyl, C₃-C₆-Cycloalkyl, C₁-C₆-Alkoxy, *N*-C₁-C₆-Alkoxy-imino-C₁-C₃-alkyl, C₁-C₆-Alkylsulfanyl, C₁-C₆-Alkylsulfinyl, C₁-C₆-Alkylsulfonyl, *N*-C₁-C₆-Alkylamino, *N,N*-Di-C₁-C₆-alkylamino oder *N*-C₁-C₃-Alkoxy-C₁-C₄-Alkylamino oder 1-Pyrrolidinyl stehen;
wenn keine der Gruppierungen A₂ und A₃ für N steht, können R³ und R⁴ gemeinsam mit dem Kohlenstoffatom, an das sie gebunden sind, einen 5- oder 6-gliedrigen Ring bilden, der 0, 1 oder 2 N-Atome und/oder 0 oder 1 O-Atom und/oder 0 oder 1 S-Atom enthält, oder
wenn keine der Gruppierungen A₁ und A₂ für N steht, können R² und R³ gemeinsam mit dem Kohlenstoffatom, an das sie gebunden sind, einen 6-gliedrigen Ring bilden, der 0, 1 oder 2 N-Atome enthält;
- R⁸: für Halogen, Cyano, Nitro, jeweils gegebenenfalls substituiertes C₁-C₆-Alkyl, C₃-C₆-Cycloalkyl, C₁-C₆-Alkoxy, *N*-C₁-C₆-Alkoxy-imino-C₁-C₃-alkyl, C₁-C₆-Alkylsulfanyl, C₁-C₆-Alkylsulfinyl, C₁-C₆-Alkylsulfonyl, *N*-C₁-C₆-Alkylamino oder *N,N*-Di-C₁-C₆-alkylamino, steht;
- W: für O oder S steht;
- Q: für H, Formyl, Hydroxy, Amino oder jeweils gegebenenfalls substituiertes C₁-C₆-Alkyl, C₂-C₆-Alkenyl, C₂-C₆-Alkinyl, C₃-C₆-Cycloalkyl, C₁-C₅-Heterocycloalkyl, C₁-C₄-Alkoxy, C₁-C₆-Alkyl-C₃-C₆-cycloalkyl, C₃-C₆-Cycloalkyl-C₁-C₆-alkyl, C₆-,C₁₀-,C₁₄-Aryl, C₁-C₅-Heteroaryl, C₆-,C₁₀-,C₁₄-Aryl-C₁-C₃-alkyl, C₁-C₅-Heteroaryl-C₁-C₃-alkyl, *N*-C₁-C₄-Alkylamino, *N*-C₁-C₄-Alkylcarbonylamino, oder *N,N*-Di-C₁-C₄-alkylamino steht; oder
für einen gegebenenfalls mehrfach mit V substituierten, ungesättigten 6-gliedrigen Carbozyklus steht; oder
für einen gegebenenfalls mehrfach mit V substituierten, ungesättigten 4-, 5- bzw. 6-gliedrigen, heterozyklischen Ring steht, wobei
- V: unabhängig voneinander für Halogen, Cyano, Nitro, jeweils gegebenenfalls substituiertes C₁-C₆-Alkyl, C₁-C₄-Alkenyl, C₁-C₄-Alkinyl, C₃-C₆-Cycloalkyl, C₁-C₆-Alkoxy, *N*-C₁-C₆-Alkoxy-imino-C₁-C₃-alkyl, C₁-C₆-Alkylsulfanyl, C₁-C₆-Alkylsulfinyl, C₁-C₆-Alkylsulfonyl, oder *N,N-*Di-(C₁-C₆-alkyl)amino steht;
- T: für einen gegebenenfalls substituierten 5-gliedrigen Heteroaromaten enthaltend maximal 2 Heteroatome, wie vier C-Atome und ein (1) Heteroatom, bevorzugt ein (1) N-, ein (1) O- oder ein (1) S-Atom oder drei C-Atome und zwei Heteroatome, bevorzugt zwei N-Atome, ein (1) N-und ein (1) O-, oder ein (1) N- und ein (1) S-Atom, steht.
sowie Salze, N-Oxide und tautomere Formen der Verbindungen der Formel (I).

### R¹

In einer bevorzugten Ausführungsform steht R¹ in einer Verbindung der Formel (I) für H, jeweils gegebenenfalls substituiertes Methyl, Ethyl, n-Propyl, Isopropyl, n-Butyl, Isobutyl, s-Butyl, t-Butyl, Methoxymethyl, Ethoxymethyl, Propoxymethyl, Methylcarbonyl, Ethylcarbonyl, n-Propylcarbonyl, Isopropylcarbonyl, s-Butylcarbonyl, t-Butylcarbonyl, Methoxycarbonyl, Ethoxycarbonyl, n-Propoxycarbonyl, Isopropoxycarbonyl, s-Butoxycarbonyl, t-Butoxycarbonyl, Cyanomethyl, 2-Cyanoethyl, Benzyl, 4-Methoxybenzyl, Pyrid-2-yl-methyl, Pyrid-3-yl-methyl, Pyrid-4-yl-methyl, 4-Chlor-pyrid-3-yl-methyl.

In einer noch bevorzugteren Ausführungsform steht R¹ für H.

### W

In einer weiteren bevorzugten Ausführungsform steht W für O.

### Q

In einer weiteren bevorzugten Ausführungsform steht Q für H, jeweils gegebenenfalls substituiertes Methyl, Ethyl, n-Propyl, 1-Methylethyl, 1,1-Dimethylethyl, 1-Methylpropyl, n-Butyl, 2-Methylpropyl, 2-Methylbutyl, Hydroxymethyl, 2-Hydroxypropyl, Cyanomethyl, 2-Cyanoethyl, 2-Fluorethyl, 2,2-Difluorethyl, 2,2,2-Trifluorethyl, 1-Trifluormethylethyl, 2,2-Difluorpropyl, 3,3,3-Trifluropropyl, 2,2-Dimethyl-3-fluorpropyl, Cyclopropyl, 1-Cyano-cyclopropyl, 1-Methoxycarbonylcyclopropyl, 1-(*N*-Methylcarbamoyl)cyclopropyl, 1-(*N*-Cyclopropylcarbamoyl)cyclopropyl, 1-(Thiocarbamoyl)cyclopropyl, Cyclopropyl-methyl, Cyclobutyl, Cyclopentyl, Cyclohexyl, 1-Cyclopropylethyl, Bis(cyclopropyl)methyl, 2,2-Dimethylcyclopropyl-methyl, 2-Phenylcyclopropyl, 2,2-Dichlorcyclopropyl, trans-2-Chlorcyclopropyl, cis-2-Chlorcyclopropyl, 2,2-Difluorcyclopropyl, trans-2-Fluorcyclopropyl, cis-2-Fluorcyclopropyl, trans-4-Hydroxycyclohexyl, 4-Trifluormethylcyclohexyl, Prop-2-enyl, 2-Methylprop-2-enyl, Prop-2-inyl, 1,1-Dimethylbut-2-inyl, 3-Chlor-prop-2-enyl,, 3,3-Dichlor-prop-2-enyl, 3,3-Dichlor-1,1-dimethylprop-2-enyl, Phenyl, 2-Chlorphenyl, 3-Chlorphenyl, 4-Chlorphenyl, Oxetan-3-yl, Thietan-3-yl, 1-Oxido-thietan-3-yl, 1,1-Dioxido-thietan-3-yl, Isoxazol-3-ylmethyl, 2-oxo-2-(2,2,2-trifluoroethylamino)ethyl, 1,2,4-Triazol-3-ylmethyl, 3-Methyloxetan-3-ylmethyl, Benzyl, 2,6-Difluorphenylmethyl, 3-Fluorphenylmethyl, 2-Fluorphenylmethyl, 2,5-Difluorphenylmethyl, 1-Phenylethyl, 4-Chlorphenylethyl, 2-Trifluormethylphenylethyl, 1-Pyridin-2-ylethyl, Pyridin-2-ylmethyl, 5-Fluorpyridin-2-ylmethyl, (6-Chlor-pyridin-3-yl)methyl, Pyrimidin-2-ylmethyl, Methoxy, 2-Ethoxyethyl, 2-(Methylsulfanyl)ethyl, 1-Methyl-2-(ethylsulfanyl)ethyl, 2-Methyl-1-(methylsulfanyl)propan-2-yl, Methoxycarbonyl, Methoxycarbonylmethyl, NH₂, *N*-Ethylamino, *N-*Allylamino, *N,N*-Dimethylamino, *N,N*-Diethylamino; oder
Q steht für ein mit 0-4 Substituenten V substituiertes Phenyl, Naphthyl, Pyridazin, Pyrazin, Pyrimidin, Triazin, Pyridin, Pyrazol, Thiazol, Isothiazol, Oxazol, Isoxazol, Triazol, Imidazol, Furan, Thiophen, Pyrrol, Oxadiazol, Thiadiazol, wobei
V unabhängig voneinander für F, Cl, Br, I, Cyano, Nitro, Methyl, Ethyl, Difluormethyl, Trichlormethyl, Chlordifluormethyl, Dichlorfluormethyl, Trifluormethyl, Chlormethyl, Brommethyl, 1-Fluorethyl, 2-Fluorethyl, 2,2-Difluorethyl, 2,2,2-Trifluorethyl, 1,2,2,2-Tetrafluorethyl, 1-Chlor-1,2,2,2-tetrafluorethyl, 2,2,2-Trichlorethyl, 2-Chlor-2,2-difluorethyl, 1,1-Difluorethyl, Pentafluorethyl, Heptafluor-n-propyl, Heptafluor-isopropyl, Nonafluor-n-butyl, Cyclopropyl, Cyclobutyl, Methoxy, Ethoxy, n-Propoxy, 1-Methylethoxy, Fluormethoxy, Difluormethoxy, Chlor-difluormethoxy, Dichlor-fluormethoxy, Trifluormethoxy, 2,2,2-Trifluorethoxy, 2-Chlor-2,2-difluorethoxy, Pentafluorethoxy, *N-*Methoxyiminomethyl, 1-(*N*-Methoxyimino)-ethyl, Methylsulfanyl, Methylsulfonyl, Methylsulfinyl, Trifluormethylsulfonyl, Trifluormethylsulfinyl, Trifluormethylsulfanyl, *N,N*-Dimethylamino steht.

In einer weiteren bevorzugten Ausführungsform steht Q für gegebenenfalls substituiertes C₁-C₄-Alkyl oder gegebenenfalls substituiertes C₃-C₆-Cycloalkyl oder für einen gegebenenfalls mit ein, zwei oder drei V substituierten, ungesättigten 4-, 5- bzw. 6-gliedrigen, heterozyklischen Ring steht, wobei V unabhängig voneinander für Halogen, Cyano, Nitro, Oxo (=O), gegebenenfalls mit Halogen substituiertes C₁-C₆-Alkyl, C₁-C₄-Alkenyl, C₁-C₆-Alkoxy, C₁-C₆-Alkylsulfanyl, C₁-C₆-Alkylsulfinyl, C₁-C₆-Alkylsulfonyl steht. Bevorzugt steht Q für mit Halogen substituiertes C₁-C₃-Alkyl; mit Cyano, Hydroxy oder Carbonamid (-C(=O)N(R)₂, wobei R unabhängig voneinander für H oder C₁-C₃-Alkyl steht, substituiertes C₁-C₃-Alkyl steht; C₃-Cycloalkyl; mit Cyano, Halogen, Nitro oder halogenierten C₁-C₂-Alkyl substituierten C₃-Cycloalkyl; einem gegebenenfalls mit ein, zwei, oder drei V substituierten, ungesättigten 4-, 5- bzw. 6-gliedrigen, heterozyklischen Ring enthaltend ein oder zwei Heteroatome ausgewählt aus einer Gruppe bestehend aus N, O und S, wobei V unabhängig voneinander für Halogen, Cyano, Nitro, Oxo (=O), gegebenenfalls mit Halogen substituiertes C₁-C₆-Alkyl steht. Besonders bevorzugt steht Q für fluoriertes C₁-C₃-Alkyl wie CF₃, CH₂CF₃ oder CH₂CH₂CF₃; mit Carbonamid (-C(=O)N(R)₂, wobei R unabhängig voneinander für H, C₁-C₃-Alkyl oder mit Halogen substituiertes C₁-C₃-Alkyl steht; substituiertem C₁-C₃-Alkyl wie 2-oxo-2-(2,2,2-trifluoroethylamino)ethyl; Cyclopropyl; mit Cyano oder fluorierten C₁-C₂-Alkyl substituierte Cyclopropyl wie 1-(cyano)cyclopropyl oder 1-(trifluoromethyl)cyclopropyl); einem 4-gliedrigen heterocyclischem Ring enthaltend ein Heteroatom ausgewählt aus einer Gruppe bestehend aus N, O oder S wie Thietan-3-yl.

In einer mehr bevorzugten Ausführungsform steht Q für mit Fluor substituiertes C₁-C₄-Alkyl wie 2,2,2-Trifluorethyl, 2,2-Difluorethyl, 3,3,3-Trifluropropyl; C₃-C₄-Cycloalkyl wie Cyclopropyl oder Cyclobutyl; gegebenenfalls substituiertes C₃-C₄-Cycloalkyl wie 1-Trifluoromethyl-cyclopropyl, 1-tert.Butyl-cyclopropyl, 1-Thiocarbamoyl-cyclopropyl, 1-Cyano-cyclopropyl, trans-2-Fluorcyclopropyl, cis-2-Fluorcyclopropyl; C₄-C₆-Heterocycloalkyl wie Oxetan-3-yl, Thietan-3-yl, 1-Oxido-thietan-3-yl, oder 1,1-Dioxido-thietan-3-yl; Benzyl; Pyridin-2-ylmethyl; Methylsulfonyl; oder 2-oxo-2-(2,2,2-trifluoroethylamino)ethyl steht.

In einer besonders bevorzugten Ausführungsform steht Q für mit Fluor substituiertes C₁-C₃-Alkyl wie 2,2,2-trifluoroethyl oder 3,3,3-trifluoropropyl; Cyclopropyl; gegebenenfalls substituiertes Cyclopropyl wie 1-Cyano-cyclopropyl oder 1-TriFluoromethyl-cyclopropyl, Thietan-3-yl; oder 2-oxo-2-(2,2,2-trifluoroethyl)aminoethyl.

### A1 bis A4

In einer bevorzugten Ausführungsform steht maximal eine (1) Gruppierung A₁ bis A₄ für N (in anderen Worten: ein (1) A₁ bis A₄ (bevorzugt A₂) steht für N); oder kein (0) A₁ bis A₄ steht für N (in anderen Worten: A₁ bis A₄ stehen für CR², CR³, CR⁴, und CR⁵) ; oder eine oder zwei Gruppierungen ausgewählt aus A₁, A₂, A₃, A₄, für N stehen können und maximal eine Gruppierung ausgewählt aus B₁, B₂, B₃, B₄, und B₅ für N steht.

In einer weiteren bevorzugten Ausführungsform stehen R², R³, R⁴, und R⁵ (wenn die entsprechende Gruppierung A für CR steht) in einer Verbindung der Formel (I) unabhängig voneinander für H, Halogen, Cyano, Nitro, jeweils gegebenenfalls substituiertes C₁-C₄-Alkyl, C₃-C₄-Cycloalkyl, C₁-C₄-Alkoxy, *N*-C₁-C₄-Alkoxyimino-C₁-C₄-alkyl, C₁-C₄-Alkylsulfanyl, C₁-C₄-Alkylsulfinyl, C₁-C₄-Alkylsulfonyl, *N*-C₁-C₄-Alkylamino, *N,N*-Di-C₁-C₄-alkylamino oder *N*-C₁-C₃-Alkoxy-C₁-C₄-Alkylamino oder 1-Pyrrolidinyl.

In einer weiteren bevorzugten Ausführungsform stehen R² und R⁵ unabhängig voneinander für H, Methyl, F und Cl.

In einer weiteren bevorzugten Ausführungsform stehen R³ und R⁴ unabhängig voneinander für H, F, Cl, Br, I, Cyano, Nitro, Methyl, Ethyl, Fluormethyl, Difluormethyl, Chlordifluormethyl, Trifluormethyl, 2,2,2-Trifluorethyl, Methoxy, Ethoxy, n-Propoxy, 1-Methylethoxy, Fluormethoxy, Difluormethoxy, Chlor-difluormethoxy, Dichlor-fluormethoxy, Trifluormethoxy, 2,2,2-Trifluorethoxy, 2-Chlor-2,2-difluorethoxy, Pentafluorethoxy, N-Methoxyiminomethyl, l-(N-Methoxyimino)-ethyl, Methylsulfanyl, Trifluormethylsulfanyl, Methylsulfonyl, Methylsulfinyl, Trifluormethylsulfonyl, Trifluormethylsulfinyl.

### B1 bis B5

In einer bevorzugten Ausführungsform steht maximal eine (1) Gruppierung B₁ bis B₅ für N (in anderen Worten: ein (1) B₁ bis B₅ steht für N); oder kein (0) B₁ bis B₅ steht für N (B₁ bis B₅ stehen für CR⁶, CR⁷, CR⁸, CR⁹ und CR¹⁰).

In einer weiteren bevorzugten Ausführungsform stehen R⁶, R⁷, R⁹ und R¹⁰ (wenn die entsprechende Gruppierung B für CR steht) unabhängig voneinander für H, Halogen, Cyano, Nitro, jeweils gegebenenfalls substituiertes C₁-C₄-Alkyl, C₃-C₄-Cycloalkyl, C₁-C₄-Alkoxy, *N-*Alkoxyiminoalkyl, C₁-C₄-Alkylsulfanyl, C₁-C₄-Alkylsulfinyl, C₁-C₄-Alkylsulfonyl, *N*-C₁-C₄-Alkylamino, *N,N*-Di-C₁-C₄-alkylamino.

In einer weiteren bevorzugten Ausführungsform stehen R⁶, R⁷, R⁹ und R¹⁰ unabhängig voneinander für H, Halogen, Cyano, Nitro, Methyl, Ethyl, Fluormethyl, Difluormethyl, Chlordifluormethyl, Trifluormethyl, 2,2,2-Trifluorethyl, Methoxy, Ethoxy, n-Propoxy, 1-Methylethoxy, Fluormethoxy, Difluormethoxy, Chlor-difluormethoxy, Dichlor-fluormethoxy, Trifluormethoxy, 2,2,2-Trifluorethoxy, 2-Chlor-2,2-difluorethoxy, Pentafluorethoxy, N-Methoxyiminomethyl, 1-(N-Methoxyimino)-ethyl, Methylsulfanyl, Trifluormethylsulfanyl, Methylsulfonyl, Methylsulfinyl, Trifluormethylsulfonyl, Trifluormethylsulfinyl.

In einer weiteren bevorzugten Ausführungsform stehen R⁶ und R¹⁰ unabhängig voneinander für H, Halogen (insbesondere Chlor, Brom, Fluor), Cyano, Nitro, Methyl, Ethyl, Difluormethyl, Chlordifluormethyl, Trifluormethyl, Methoxy, Ethoxy, 1-Methylethoxy, Difluormethoxy, Chlor-difluormethoxy, Dichlor-fluormethoxy, Trifluormethoxy, 2,2,2-Trifluorethoxy, 2-Chlor-2,2-difluorethoxy, Methylsulfanyl, Trifluormethylsulfanyl, Methylsulfonyl, Methylsulfinyl, Trifluormethylsulfonyl, Trifluormethylsulfinyl.

In einer weiteren bevorzugten Ausführungsform stehen R⁶ und R¹⁰ für die hierin beschriebenen Substituenten, jedoch stehen R⁶ und R¹⁰ nicht gleichzeitig in einer Verbindung für H. Mit anderen Worten, wenn R⁶ in einer Verbindung für H steht, steht R¹⁰ für einen der anderen hierin beschriebenen Substituenten und umgekehrt.

In einer weiteren bevorzugten Ausführungsform stehen R⁶ und R¹⁰ jeweils für einen Substituenten ausgewählt aus Halogen (bevorzugt Cl, Br oder F), C₁-C₃-alkyl, mit Halogen substituiertes C₁-C₃-alkyl, C₁-C₃-alkoxy oder mit Halogen substituiertes C₁-C₃-alkoxy.

In einer weiteren bevorzugten Ausführungsform stehen R⁶ und R¹⁰ jeweils für Halogen (wie Cl, Br oder F), jeweils für C₁-C₃-alkyl, oder jeweils für mit Halogen substituiertes C₁-C₃-alkyl wie z. B. perfluoriertes C₁-C₃-alkyl (Perfluormethyl, perFluoroethyl oder Perfluorpropyl).

In einer weiteren bevorzugten Ausführungsform steht R⁶ für perfluoriertes C₁-C₃-alkyl (z. B. Perfluormethyl) und R¹⁰ für Cl, Br oder F, besonders bevorzugt für Cl oder Br.

### R8

In einer besonders bevorzugten Ausfüuhrungsform steht B₃ für C-R⁸ worin R⁸ für Halogen, Cyano, Nitro, mit Halogen substituiertes C₁-C₄-Alkyl, C₃-C₄-Cycloalkyl, C₁-C₄-Alkoxy, *N*-C₁-C₄-Alkoxyimino-C₁-C₄-alkyl, C₁-C₄-Alkylsulfanyl, C₁-C₄-Alkylsulfinyl, C₁-C₄-Alkylsulfonyl, *N*-C₁-C₄-Alkylamino, oder *N,N*-Di-C₁-C₄-alkylamino steht.

In einer weiteren bevorzugten Ausführungsform steht R⁸ für Halogen wie Fluor, Chlor, Brom, Iod oder mit Halogen substituiertes C₁-C₄-Alkyl, Cyano, Nitro, Methyl, Ethyl, Difluormethyl, Trichlormethyl, Chlordifluormethyl, Dichlorfluormethyl, Trifluormethyl, Chlormethyl, Brommethyl, 1-Fluorethyl, 2-Fluorethyl, 2,2-Difluorethyl, 2,2,2-Trifluorethyl, 1,2,2,2-Tetrafluorethyl, 1-Chlor-1,2,2,2-tetrafluorethyl, 2,2,2-Trichlorethyl, 2-Chlor-2,2-difluorethyl, 1,1-Difluorethyl, Pentafluorethyl, Pentafluor-tert-butyl, Heptafluor-n-propyl, Heptafluor-isopropyl, Nonafluor-n-butyl, Nonafluor-sec-butyl, Cyclopropyl, Cyclobutyl, Methoxy, Ethoxy, n-Propoxy, 1-Methylethoxy, Fluormethoxy, Difluormethoxy, Chlor-difluormethoxy, Dichlor-fluormethoxy, Trifluormethoxy, 2,2,2-Trifluorethoxy, 2-Chlor-2,2-difluorethoxy, Pentafluorethoxy, *N*-Methoxyiminomethyl, 1-(*N*-Methoxyimino)-ethyl, Methylsulfanyl, Methylsulfonyl, Methylsulfinyl, Trifluormethylsulfonyl, Trifluormethylsulfinyl, Trifluormethylsulfanyl, *N*,*N*-Dimethylamino.

In einer mehr bevorzugten Ausführungsform steht R⁸ für Difluormethyl, Trichlormethyl, Chlordifluormethyl, Dichlorfluormethyl, Trifluormethyl, 1-Fluorethyl, 2-Fluorethyl, 2,2-Difluorethyl, 2,2,2-Trifluorethyl, 1,2,2,2-Tetrafluorethyl, 1-Chlor-1,2,2,2-tetrafluorethyl, 2,2,2-Trichlorethyl, 2-Chlor-2,2-difluorethyl, 1,1-Difluorethyl, Pentafluorethyl, Pentafluor-tert-butyl, Heptafluor-n-propyl, Heptafluor-isopropyl, Nonafluor-n-butyl, Nonafluor-sec-butyl, Fluormethoxy, Difluormethoxy, Chlor-difluormethoxy, Dichlor-fluormethoxy, Trifluormethoxy, 2,2,2-Trifluorethoxy, 2-Chlor-2,2-difluorethoxy, Pentafluorethoxy, Trifluormethylsulfonyl, Trifluormethylsulfinyl, Trifluormethylsulfanyl, steht.

In einer weiteren mehr bevorzugten Ausführungsform steht R⁸ für mit Halogen substituiertes C₁-C₃-Alkyl (bevorzugt perfluoriertes C₁-C₃-Alkyl (CF₃, C₂F₅ oder C₃F₇)) oder mit Halogen substituiertes Alkoxy (bevorzugt perfluoriertes C₁-C₃-Alkoxy (OCF₃, OC₂F₅ oder OC₃F₇)).

In einer besonders bevorzugten Ausführungsform steht R⁸ für perfluoriertes C₁-C₃-Alkyl wie perfluoriertes n- bzw. i-Propyl (-C₃F₇), perfluoriertes Ethyl (C₂F₅) oder perfluoriertes Methyl (CF₃), besonders bevorzugt perfluoriertes n- bzw. i-Propyl (-C₃F₇) oder perfluoriertes Methyl.

### A und B

In einer weiteren bevorzugten Ausführungsform stehen die Gruppierungen A₁ bis A₄ und B₁ bis B₅ in Verbindungen der Formel (I) für
- A₁: C-H,
- A₂: CR³ oder N,
- A₃: CR⁴,
- A₄: CR⁵ oder N,
- B₁: CR⁶ oder N,
- B₂: CR⁷,
- B₃: CR⁸,
- B₄: CR⁹ und
- B₅: CR¹⁰ oder N.

In einer noch bevorzugteren Ausführungsform stehen die Gruppierungen A₁ bis A₄ und B₁ bis B₅ in Verbindungen der Formel (I) für
- A₁: C-H,
- A₂: CR³ oder N,
- A₃: CR⁴,
- A₄: C-H,
- B₁: CR⁶ oder N,
- B₂: C-H,
- B₃: CR⁸,
- B₄: C-H und
- B₅: CR¹⁰ oder N.

In einer noch bevorzugteren Ausführungsform stehen die Gruppierungen A₁ bis A₄ und B₁ bis B₅ in Verbindungen der Formel (I) für
- A₁: C-H,
- A₂: CR³ oder N,
- A₃: CR⁴,
- A₄: C-H oder N,
- B₁: CR⁶,
- B₂: C-H,
- B₃: CR⁸,
- B₄: C-H und
- B₅: CR¹⁰ oder N.

### T

In einer weiteren bevorzugten Ausführungsform steht T für einen der nachfolgend aufgeführten 5 gliedrigen Heteroaromaten, wobei die Bindung zum C-Atom des (C-B₁-B₅)-Ringsystems mit einer gestrichelten Bindung mit Sternchen und die Bindung zum C-Atom des (C-A₁-A₂-A₃-C-A₄)-Ringsystems mit einer gestrichelten Bindung gekennzeichnet ist, 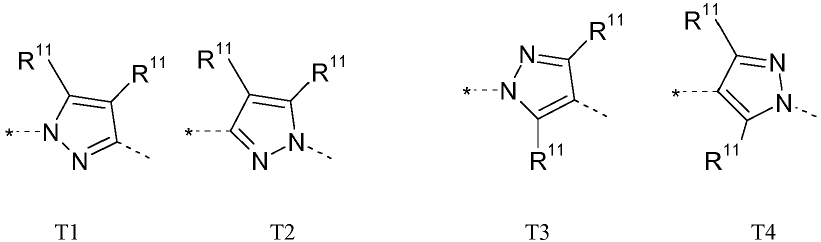 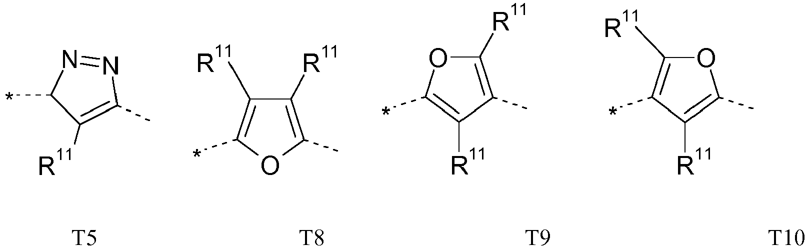 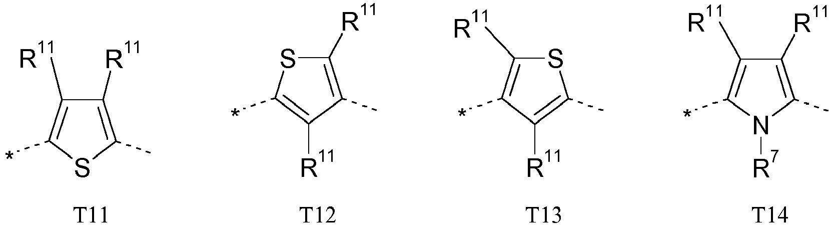 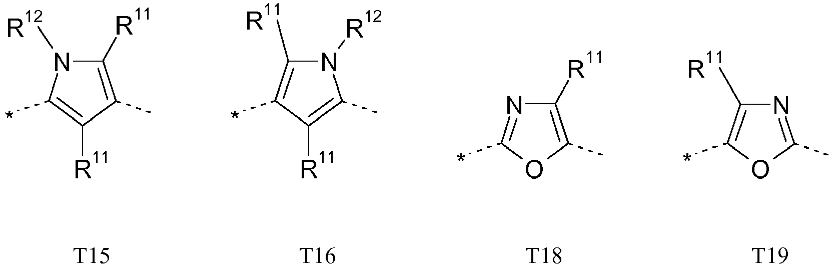 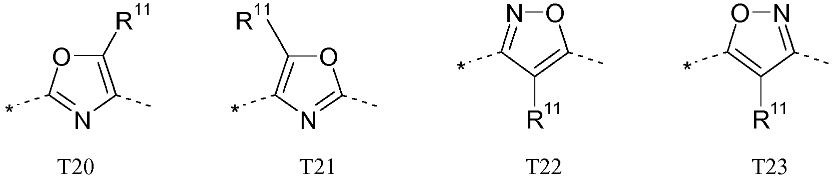 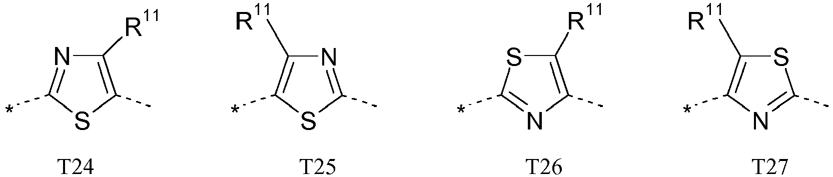 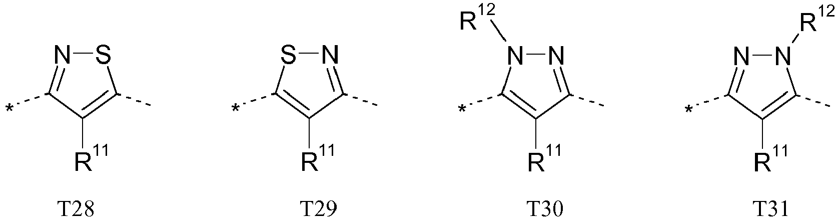 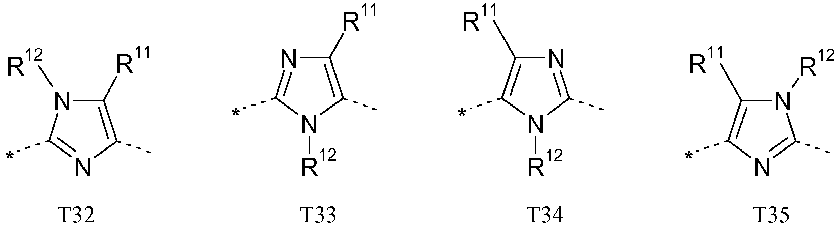 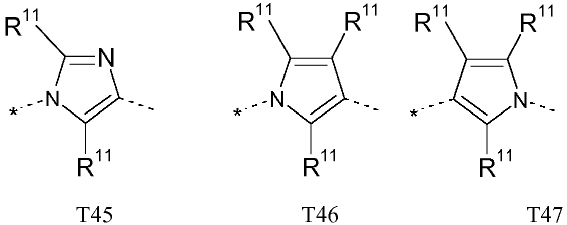 wobei
- R¹¹: unabhängig voneinander für H, Halogen, Cyano, Nitro, Amino oder ein gegebenenfalls substituiertes C₁-C₆-Alkyl, C₁-C₆-Alkyloxy, C₁-C₆-Alkylcarbonyl, C₁-C₆-Alkylsulfanyl, C₁-C₆-Alkylsulfinyl, C₁-C₆-Alkylsulfonyl, bevorzugt für H, steht; und
- R¹²: für H, Halogen, Cyano, Nitro, Amino oder ein gegebenenfalls substituiertes C₁-C₆-Alkyl, C₁-C₆-Alkyloxy, C₁-C₆-Alkylcarbonyl, C₁-C₆-Alkylsulfanyl, C₁-C₆-Alkylsulfinyl, C₁-C₆-Alkylsulfonyl, bevorzugt für H oder Methyl, steht.

In einer weiteren bevorzugten Ausführungsform steht R¹¹ unabhängig voneinander für Halogen, Cyano, Nitro, Amino, Methyl, Ethyl, 1-Methylethyl, tert-Butyl, Trifluormethyl, Difluormethyl, Methoxy, Ethoxy, Trifluormethoxy, 2,2-Difluorethoxy, 2,2,2-Trifluorethoxy, Methylcarbonyl, Ethylcarbonyl, Trifluormethylcarbonyl, Methylsulfanyl, Methylsulfinyl, Methylsulfonyl, Trifluormethylsulfonyl, Trifluormethylsulfanyl, oder Trilfuormethylsulfinyl.

In einer mehr bevorzugten Ausführungsform steht R¹¹ unabhängig voneinander für H, Methyl, Ethyl, 2-Methylethyl, 2,2-Dimethylethyl, Fluor, Chlor, Brom, Iod, Nitro, Trifluormethyl, oder Amino.

In einer weiteren bevorzugten Ausführungsform steht T für einen der nachfolgend aufgeführten 5 gliedrigen Heteroaromaten, wobei die Bindung zum C-Atom des (C-B₁-B₅)-Ringsystems mit einer gestrichelten Bindung mit Sternchen und die Bindung zum C-Atom des (C-A₁-A₂-A₃-C-A₄)-Ringsystems mit einer gestrichelten Bindung gekennzeichnet ist, 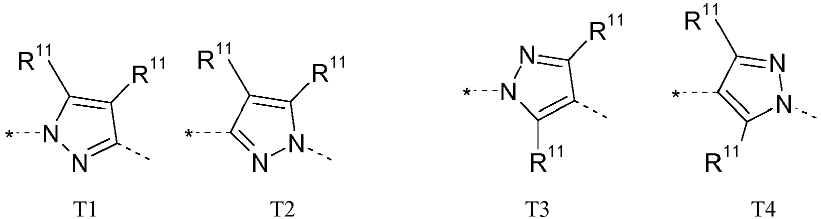 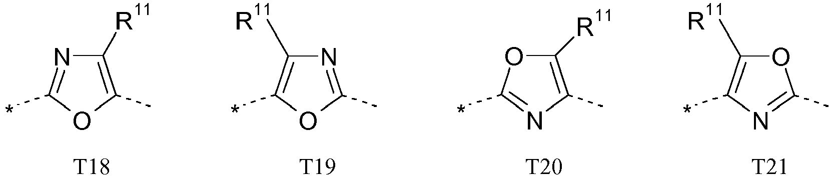 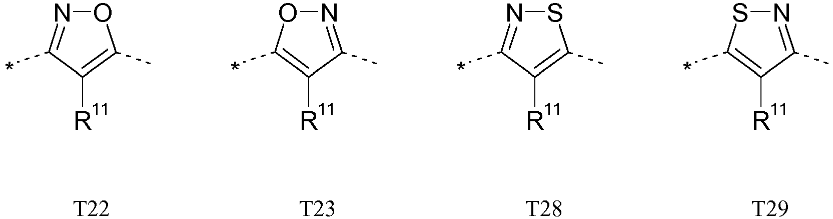 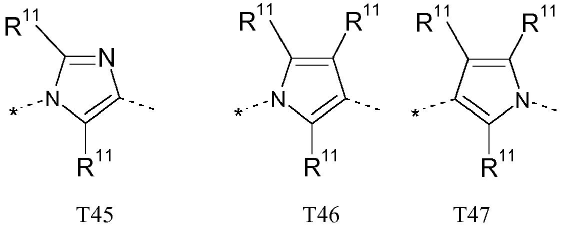 wobei R¹¹ unabhängig voneinander wie hierin beschrieben definiert ist.

In einer mehr bevorzugten Ausführungsform steht T für einen der nachfolgend aufgeführten 5 gliedrigen Heteroaromaten, wobei die Bindung zum C-Atom des (C-B₁-B₅)-Ringsystems mit einer gestrichelten Bindung mit Sternchen und die Bindung zum C-Atom des (C-A₁-A₂-A₃-C-A₄)-Ringsystems mit einer gestrichelten Bindung gekennzeichnet ist, 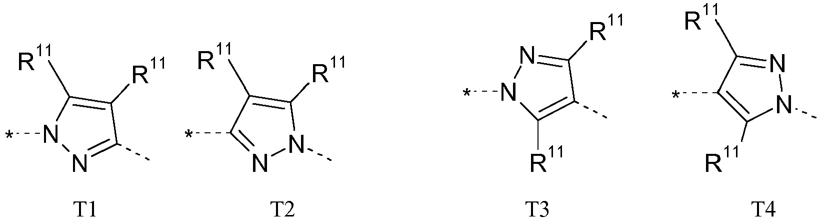 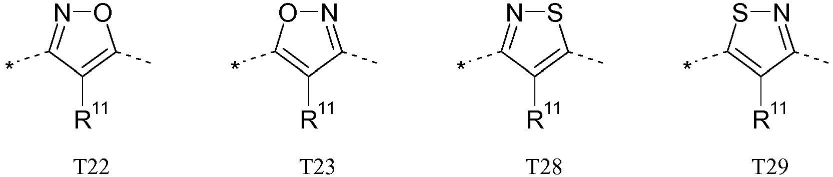 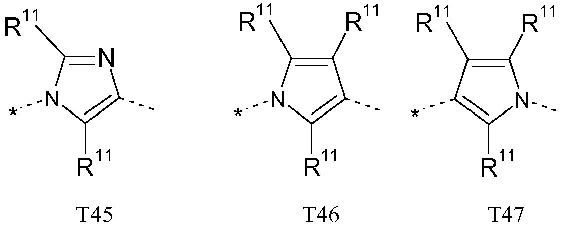 wobei R¹¹ wie hierin beschrieben definiert ist und n für die Werte 1 oder 2 steht.

In einer besonders bevorzugten Ausführungsform steht T für einen der nachfolgend aufgeführten 5 gliedrigen Heteroaromaten, wobei die Bindung zum C-Atom des (C-B₁-B₅)-Ringsystems mit einer gestrichelten Bindung mit Sternchen und die Bindung zum C-Atom des (C-A₁-A₂-A₃-C-A₄)-Ringsystems mit einer gestrichelten Bindung gekennzeichnet ist, 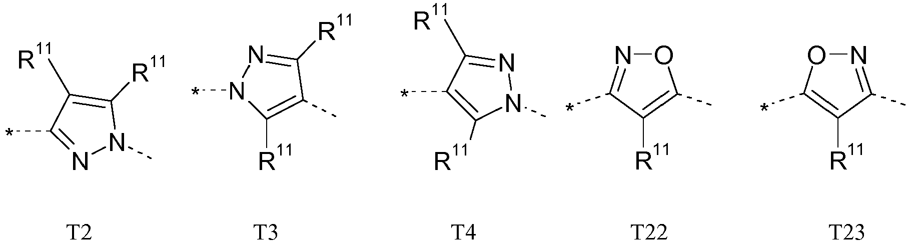 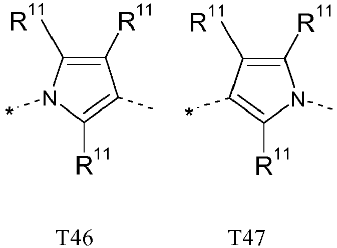 wobei R¹¹ unabhängig voneinander wie hierin beschrieben definiert ist.

In einer weiteren besonders bevorzugten Ausführungsform steht T für einen der nachfolgend aufgeführten 5 gliedrigen Heteroaromaten, wobei die Bindung zum C-Atom des (C-B₁-B₅)-Ringsystems mit einer gestrichelten Bindung mit Sternchen und die Bindung zum C-Atom des (C-A₁-A₂-A₃-C-A₄)-Ringsystems mit einer gestrichelten Bindung gekennzeichnet ist, 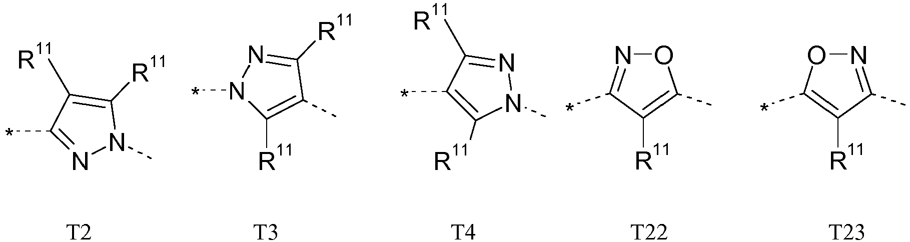 wobei R¹¹ unabhängig voneinander wie hierin beschrieben definiert ist.

In einer noch bevorzugteren Ausführungsform steht in der Formel (I) und weiteren hierin aufgeführten allegmeinen Formeln
- A₁: für C-R² oder N, bevorzugt für C- R²,
- A₂: für CR³ oder N,
- A₃: für CR⁴,
- A₄: für C-R⁵ oder N,
- B₁: für CR⁶,
- B₂: für C-H,
- B₃: für CR⁸,
- B₄: für C-H,
- B₅: für CR¹⁰ oder N,
- R¹: für Wasserstoff,
- R²: für Wasserstoff, C₁-C₃-Alkyl, Fluor oder Chlor, bevorzugt für H,
- R³: für Wasserstoff oder mit Halogen substituiertes C₁-C₃-Alkyl (bevorzugt perfluoriertes C₁-C₃-Alkyl (CF₃, C₂F₅ oder C₃F₇)),
- R⁴: für Wasserstoff, Chlor, Fluor, C₁-C₃-Alkyl (wie -CH₃), Cyclopropyl, C₁-C₃-Alkoxy (wie-O-CH₃), *N*-C₁-C₄-Alkylamino (-NH-C₁-C₃-Alkyl wie -NH-CH₃), C₃-cycloalkylamino (wie -NH-C₃H₅), *N-*C₁-C₃-Alkoxy-C₁-C₃-Alkylamino (wie -NH-C₂H₄-O-CH₃) oder 1-Pyrrolidinyl, besonders bevorzugt Chlor,
- R⁵: für Wasserstoff oder Fluor, bevorzugt H,
- R⁶ und R¹⁰: unabhängig voneinander für Wasserstoff, C₁-C₃-Alkyl (bevorzugt stehen R⁶ und R¹⁰ für C₁-C₃-Alkyl), C₁-C₃-Alkoxy, mit Halogen substituiertes C₁-C₃-Alkyl (bevorzugt perfluoriertes C₁-C₃-Alkyl (CF₃, C₂F₅ oder C₃F₇))mit Halogen substituiertes C₁-C₃-Alkoxy (bevorzugt perfluoriertes C₁-C₃-Alkoxy (OCF₃, OC₂F₅ oder OC₃F₇)), C₁-C₃-Alkylsulfanyl, C₁-C₃-Alkylsulfinyl, C₁-C₃-Alkylsulfonyl, Fluor, Brom oder Chlor (bevorzugt stehen R⁶ und R¹⁰ für Chlor),
- R⁸: für mit Halogen substituiertes C₁-C₃-Alkyl (bevorzugt perfluoriertes C₁-C₃-Alkyl (CF₃, C₂F₅ oder C₃F₇)) oder mit Halogen substituiertes C₁-C₃-Alkoxy (bevorzugt perfluoriertes C₁-C₃-Alkoxy (OCF₃, OC₂F₅ oder OC₃F₇)),
- R¹¹: für Wasserstoff, Cyano (CN) oder Amino (NH₂),
- W: für Sauerstoff oder Schwefel, bevorzugt für Sauerstoff,
- Q: für C₁-C₃-Alkyl, Cyclopropyl, 1-(Cyano)-cyclopropyl, 1-(perfluoriertes C₁-C₃-Alkyl)-cyclopropyl (wie (1-(Trifluoromethyl)-cyclopropyl), 1-(C₁-C₄-Alkyl)-cyclopropyl (wie 1-(tert.Butyl)-cyclopropyl), 1-(Thiocarbamoyl)-cyclopropyl, mit Halogen substituiertes C₁-C₃-Alkyl (z. B. CH₂CF₃, CH₂CH₂CF₃), Thietan-3-yl, N-Methyl-Pyrazol-3-yl, 2-oxo-2(2,2,2-trifluorethylamino)ethyl, und
- T: für ein T ausgewählt aus der Gruppe bestehend aus T1 bis T47, bevorzugt für T2, T3, T4, T22 oder T23 (besonders bevorzugt für T22 oder T23).

In einer weiteren noch bevorzugteren Ausführungsform stehen in der Formel (I) und weiteren hierin aufgeführten allegmeinen Formeln
- A₁: für C-R² oder N, bevorzugt für C- R²,
- A₂: für CR³ oder N,
- A₃: für CR⁴,
- A₄: für C-R⁵ oder N,
- B₁: für CR⁶,
- B₂: für C-H,
- B₃: für CR⁸,
- B₄: für C-H,
- B₅: für CR¹⁰ oder N,
- R¹: für C₁-C₂-Alkyl (Methyl oder Ethyl, besonders bevorzugt für Methyl),
- R²: für Wasserstoff, C₁-C₃-Alkyl, Fluor oder Chlor, bevorzugt für H,
- R³: für Wasserstoff oder mit Halogen substituiertes C₁-C₃-Alkyl (bevorzugt perfluoriertes C₁-C₃-Alkyl (CF₃, C₂F₅ oder C₃F₇)),
- R⁴: für Wasserstoff, Chlor, Fluor, C₁-C₃-Alkyl (wie -CH₃), Cyclopropyl, C₁-C₃-Alkoxy (wie-O-CH₃), *N*-C₁-C₄-Alkylamino (-NH-C₁-C₃-Alkyl wie -NH-CH₃), C₃-cycloalkylamino (wie -NH-C₃H₅), *N-*C₁-C₃-Alkoxy-C₁-C₃-Alkylamino (wie. -NH-C₂H₄-O-CH₃) oder 1-Pyrrolidinyl, besonders bevorzugt Chlor,
- R⁵: für Wasserstoff oder Fluor, bevorzugt H,
- R: ⁶ und R¹⁰ unabhängig voneinander für Wasserstoff, C₁-C₃-Alkyl (bevorzugt stehen R⁶ und R¹⁰ für C₁-C₃-Alkyl), C₁-C₃-Alkoxy, mit Halogen substituiertes C₁-C₃-Alkyl (bevorzugt perfluoriertes C₁-C₃-Alkyl (CF₃, C₂F₅ oder C₃F₇))mit Halogen substituiertes C₁-C₃-Alkoxy (bevorzugt perfluoriertes C₁-C₃-Alkoxy (OCF₃, OC₂F₅ oder OC₃F₇)), C₁-C₃-Alkylsulfanyl, C₁-C₃-Alkylsulfinyl, C₁-C₃-Alkylsulfonyl, Fluor, Brom oder Chlor (bevorzugt stehen R⁶ und R¹⁰ für Chlor),
- R⁸: für mit Halogen substituiertes C₁-C₃-Alkyl (bevorzugt perfluoriertes C₁-C₃-Alkyl (CF₃, C₂F₅ oder C₃F₇)) oder mit Halogen substituiertes C₁-C₃-Alkoxy (bevorzugt perfluoriertes C₁-C₃-Alkoxy (OCF₃, OC₂F₅ oder OC₃F₇)),
- R¹¹: für Wasserstoff, Cyano (CN) oder Amino (NH₂),
- W: für Sauerstoff oder Schwefel, bevorzugt für Sauerstoff,
- Q: für C₁-C₃-Alkyl, Cyclopropyl, 1-(Cyano)-cyclopropyl, 1-(perfluoriertes C₁-C₃-Alkyl)-cyclopropyl (wie (1-(Trifluoromethyl)-cyclopropyl), 1-(C₁-C₄-Alkyl)-cyclopropyl (wie 1-(tert.Butyl)-cyclopropyl), 1-(Thiocarbamoyl)-cyclopropyl, mit Halogen substituiertes C₁-C₃-Alkyl (z. B. CH₂CF₃, CH₂CH₂CF₃), Thietan-3-yl, N-Methyl-Pyrazol-3-yl, 2-oxo-2(2,2,2-trifluorethylamino)ethyl, und
- T: für ein T ausgewählt aus der Gruppe bestehend aus T1 bis T47, bevorzugt für T2, T3, T4, T22 oder T23 (besonders bevorzugt für T22 oder T23).

Eine weitere bevorzugte Ausführungsform betrifft zudem Verbindungen der Formel (Ia) 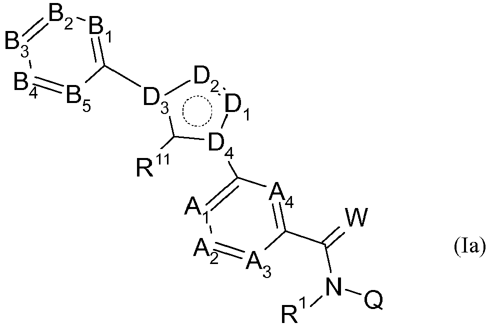 worin
R¹, R¹¹, Q, W, A₁, A₂, A₃, A₄, B₁, B₂, B₃, B₄, und B₅ wie hierin beschrieben definiert sind, wobei maximal eine Gruppierung ausgewählt aus A₁, A₂, A₃, A₄, für N steht und maximal eine Gruppierung ausgewählt aus B₁, B₂, B₃, B₄, und B₅ für N steht; oder wobei eine oder zwei Gruppierungen ausgewählt aus A₁, A₂, A₃, A₄, für N stehen können und maximal eine Gruppierung ausgewählt aus B₁, B₂, B₃, B₄, und B₅ für N steht; und
D₁ und D₂ jeweils unabhängig voneinander für C-R¹¹ oder ein Heteroatom stehen, bevorzugt für C-R¹¹ oder ein Heteroatom ausgewählt aus N, O oder S, besonders bevorzugt für C-R¹¹ oder ein Heteroatom ausgewählt aus N oder O, stehen;
die Gruppierungen D₃ und D₄ unabhängig voneinander für C oder für ein Heteroatom ausgewählt aus N stehen;
wobei ein (1) oder zwei Gruppierungen ausgewählt aus D₁, D₂, D₃ und D₄ für ein Heteroatom stehen; 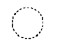 für ein aromatisches System steht.

Eine weitere bevorzugte Ausführungsform betrifft zudem Verbindungen der Formel (Ia') 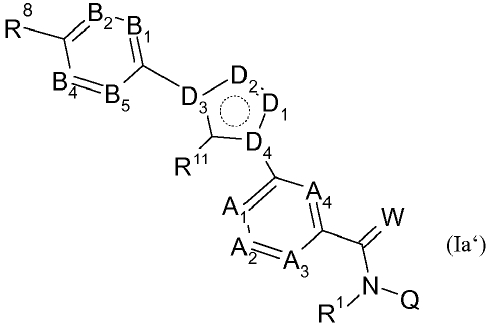 worin
R¹, R¹¹, Q, W, A₁, A₂, A₃, A₄, B₁, B₂, B₄, und B₅ wie hierin beschrieben definiert sind, wobei maximal eine Gruppierung ausgewählt aus A₁, A₂, A₃, A₄, für N steht und maximal eine Gruppierung ausgewählt aus B₁, B₂, B₃, B₄, und B₅ für N steht; oder wobei eine oder zwei Gruppierungen ausgewählt aus A₁, A₂, A₃, A₄, für N stehen können und maximal eine Gruppierung ausgewählt aus B₁, B₂, B₃, B₄, und B₅ für N steht;
D₁ und D₂ jeweils unabhängig voneinander für C-R¹¹ oder ein Heteroatom stehen, bevorzugt für C-R¹¹ oder ein Heteroatom ausgewählt aus N, O oder S, besonders bevorzugt für C-R¹¹ oder ein Heteroatom ausgewählt aus N oder O, stehen;
die Gruppierungen D₃ und D₄ unabhängig voneinander für C oder für ein Heteroatom ausgewählt aus N stehen;
wobei ein (1) oder zwei Gruppierungen ausgewählt aus D₁, D₂, D₃ und D₄ für ein Heteroatom stehen; in anderen Worten: wobei maximal ein (1) oder zwei Gruppierungen ausgewählt aus D₁, D₂, D₃ und D₄ für ein Heteroatom steht/stehen, wobei ein (1) oder zwei Gruppierungen ausgewählt aus D₁, D₂, D₃ und D₄ für ein Heteroatom ausgewählt aus N oder O im Fall von D₁ und D₂, oder N im Fall von D₃ und D₄ stehen; 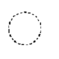 für ein aromatisches System steht
und R⁸ wie hierin definiert ist, bevorzugt für perfluoriertes C₁-C₄-Alkyl steht.

Eine weitere bevorzugte Ausführungsform betrifft Verbindungen der Formel (Ib) 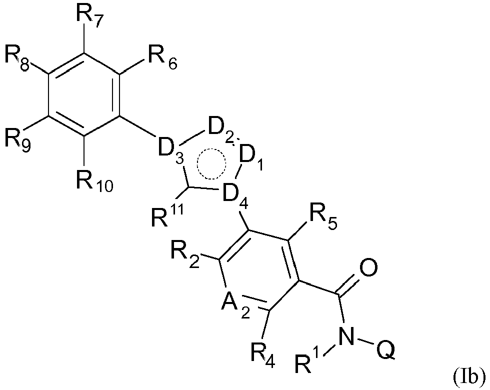 worin R₁, R₂, R₄, R₅, R₆, R₇, R₈, R₉, R₁₀, R₁₁, A₂, Q, D₁, D₂, D₃ D₄ und 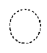 wie hierin beschrieben definiert sind und wobei ein (1) oder zwei Gruppierungen ausgewählt aus D₁, D₂, D₃ und D₄ für ein Heteroatom stehen.

Zwei besonders bevorzugte Ausführungsformen betreffen Verbindungen der Formel (Ib) und (Id) worin D₁ für N, D₂ für O und D₃ und D₄ für C stehen; oder D₁ für C-R¹³, D₂ für N und D₃ für N und D₄ für C steht wobei R₁₃ für H, Halogen, Cyano, Nitro, Amino oder ein gegebenenfalls substituiertes C₁-C₆-Alkyl, C₁-C₆-Alkyloxy, C₁-C₆-Alkylcarbonyl, C₁-C₆-Alkylsulfanyl, C₁-C₆-Alkylsulfinyl, C₁-C₆-Alkylsulfonyl, bevorzugt für H oder Halogen wie F, Cl, Br oder I, und besonders bevorzugt für H, steht; und R₁ bevorzugt für H steht oder R₁ bevorzugt für Methyl steht.

Eine weitere besonders bevorzugte Ausführungsform betrifft Verbindungen der Formel (Ib) und (Id) worin D₁ für O, D₂ für N und D₃ und D₄ für C stehen; wobei R₁₃ für H, Halogen, Cyano, Nitro, Amino oder ein gegebenenfalls substituiertes C₁-C₆-Alkyl, C₁-C₆-Alkyloxy, C₁-C₆-Alkylcarbonyl, C₁-C₆-Alkylsulfanyl, C₁-C₆-Alkylsulfinyl, C₁-C₆-Alkylsulfonyl, bevorzugt für H oder Halogen wie F, Cl, Br oder I, und besonders bevorzugt für H, steht; und R₁ bevorzugt für H steht oder R₁ bevorzugt für Methyl steht.

Eine weitere bevorzugte Ausführungsform betrifft Verbindungen der Formel (Ic) 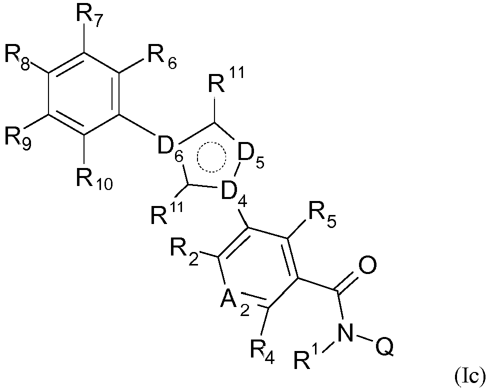 worin R₁, R₂, R₄, R₅, R₆, R₇, R₈, R₉, R₁₀, R₁₁, A₂, und Q wie hierin beschrieben definiert sind und 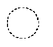 für ein aromatisches System steht; und
eine Gruppierung ausgewählt aus D₄ und D₆ für N steht, wobei die jeweils andere Gruppierung ausgewählt aus D₄ und D₆ für N oder C steht; und
D₅ für N oder C-R¹¹ steht;
unter der Bedingung, dass maximal zwei Gruppierungen ausgewählt aus D₄, D₅ und D₆ für N stehen.

Bevorzugte Ausführungsformen betreffen Verbindungen der Formel (Ic), worin D₄ für N und D₅ und D₆ jeweils für C-R¹¹ stehen; worin D₆ für N und D₅ und D₄ jeweils für C-R¹¹ stehen; oder worin D₄ und D₅ jeweils für N stehen und D₆ für C-R¹¹ steht.

Eine weitere bevorzugte Ausführungsform betrifft Verbindungen der Formel (Id) 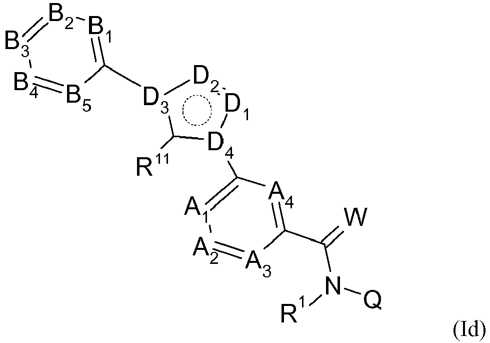 wobei R¹, R¹¹, Q, W, A₁, A₂, A₃, A₄, B₁, B₂, B₃, B₄, und B₅, D₁, D₂, D₃ und D₄ sowie wie hierin beschrieben definiert sind, wobei maximal ein (1) oder zwei Gruppierungen ausgewählt aus D₁, D₂, D₃ und D₄ für ein Heteroatom stehen und wobei maximal eine Gruppierung ausgewählt aus A₁, A₂, A₃, A₄, für N steht und maximal eine Gruppierung ausgewählt aus B₁, B₂, B₃, B₄, und B₅ für N steht.

Eine besonders bevorzugte Ausführungsform betrifft Verbindungen der Formel (Ia), (Ib), (Ic) oder (Id) worin R⁸ für C₁-C₆-Alkyl, mit Halogen substituiertes C₁-C₆-Alkyl, C₃-C₆-Cycloalkyl, mit Halogen substituiertes C₃-C₆-Cycloalkyl, C₁-C₆-Alkoxy, mit Halogen substituiertes C₁-C₆-Alkoxy, N-Alkoxyiminoalkyl, mit Halogen substituiertes C₁-C₆-Alkylsulfanyl, mit Halogen substituiertes C₁-C₆-Alkylsulfinyl, mit Halogen substituiertes C₁-C₆-Alkylsulfonyl, *N*-C₁-C₆-Alkylamino, *N,N*-Di-C₁-C₄-Alkylamino, sowie für Halogen, Cyano oder Nitro steht. Beispiele sind Fluor, Chlor, Brom, Iod, Cyano, Nitro, Methyl, Ethyl, Difluormethyl, Trichlormethyl, Chlordifluormethyl, Dichlorfluormethyl, Trifluormethyl, Chlormethyl, Brommethyl, 1-Fluorethyl, 2-Fluorethyl, 2,2-Difluorethyl, 2,2,2-Trifluorethyl, 1,2,2,2-Tetrafluorethyl, 1-Chlor-1,2,2,2-tetrafluorethyl, 2,2,2-Trichlorethyl, 2-Chlor-2,2-difluorethyl, 1,1-Difluorethyl, Pentafluorethyl Pentafluor-tert-butyl, Heptafluor-n-propyl, Heptafluor-isopropyl, Nonafluor-n-butyl, Nonafluor-sec-butyl, Cyclopropyl, Cyclobutyl, Methoxy, Ethoxy, n-Propoxy, 1-Methylethoxy, Fluormethoxy, Difluormethoxy, Chlor-difluormethoxy, Dichlor-fluormethoxy, Trifluormethoxy, 2,2,2-Trifluorethoxy, 2-Chlor-2,2-difluorethoxy, Pentafluorethoxy, N-Methoxyiminomethyl, 1-(*N*-Methoxyimino)-ethyl, Methylsulfanyl, Methylsulfonyl, Methylsulfinyl, Trifluormethylsulfonyl, Trifluormethylsulfinyl, Trifluormethylsulfanyl, *N,N*-Dimethylamino. Mehr bevorzugt steht R⁸ für mit Halogen substituiertes C₁-C₄-Alkyl wie Difluormethyl, Trichlormethyl, Chlordifluormethyl, Dichlorfluormethyl, Trifluormethyl, 1-Fluorethyl, 2-Fluorethyl, 2,2-Difluorethyl, 2,2,2-Trifluorethyl, 1,2,2,2-Tetrafluorethyl, 1-Chlor-1,2,2,2-tetrafluorethyl, 2,2,2-Trichlorethyl, 2-Chlor-2,2-difluorethyl, 1,1 -Difluorethyl, Pentafluorethyl Pentafluor-tert-butyl, Heptafluor-n-propyl, Heptafluor-isopropyl, Nonafluor-n-butyl, Nonafluor-sec-butyl; mit Halogen substituiertes C₁-C₄-Alkoxy wie Fluormethoxy, Difluormethoxy, Chlor-difluormethoxy, Dichlor-fluormethoxy, Trifluormethoxy, 2,2,2-Trifluorethoxy, 2-Chlor-2,2-difluorethoxy, Pentafluorethoxy; Trifluormethylsulfonyl; Trifluormethylsulfinyl; oder Trifluormethylsulfanyl steht. Noch mehr bevorzugt steht R⁸ für Difluormethyl, Trichlormethyl, Chlordifluormethyl, Dichlorfluormethyl, Trifluormethyl, 1-Fluorethyl, 2-Fluorethyl, 2,2-Difluorethyl, 2,2,2-Trifluorethyl, 1,2,2,2-Tetrafluorethyl, 1-Chlor-1,2,2,2-tetrafluorethyl, 2,2,2-Trichlorethyl, 2-Chlor-2,2-difluorethyl, 1,1-Difluorethyl, Pentafluorethyl Pentafluor-tert-butyl, Heptafluor-n-propyl, Heptafluor-isopropyl, Nonafluor-n-butyl, Nonafluor-sec-butyl, Fluormethoxy, Difluormethoxy, Chlor-difluormethoxy, Dichlor-fluormethoxy, Trifluormethoxy, 2,2,2-Trifluorethoxy, 2-Chlor-2,2-difluorethoxy, Pentafluorethoxy, Trifluormethylsulfonyl, Trifluormethylsulfinyl, oder Trifluormethylsulfanyl. Besonders bevorzugt steht R⁸ in Verbindungen der Formel (Ib) für perfluoriertes C₁-C₃-Alkyl wie perfluoriertes Propyl (-C₃F₇), perfluoriertes Ethyl (C₂F₅) oder perfluoriertes Methyl (CF₃), ganz besonders bevorzugt für perfluoriertes Propyl (-C₃F₇) oder perfluoriertes Methyl.

Besonders bevorzugte Verbindungen entsprechend den Verbindungen der Formel (Ia) sind Verbindungen der Formel (I-T2), (I-T3), (I-T4), (I-T22) und (I-T23).

Eine Ausführungsform der vorliegenden Erfindung bezieht sich auf Verbindungen der Formel (I-T2) und (I-T4).

Eine weitere Ausführungsform bezieht sich auf Verbindungen der Formel (I-T3).

Eine weitere Ausführungsfrom bezieht sich auf Verbindungen der Formeln (I-T22) und (I-T23).

Somit bezieht sich eine ganz besonders bevorzugte Ausführungsform auf Verbindungen der Formel (I-T2). Eine bevorzugte Ausführungsform bezieht sich dabei wiederum auf Verbindungen der Formel (I-T2) in der R¹ für H steht. Eine weitere bevorzugte Ausführungsform bezieht sich dabei wiederum auf Verbindungen der Formel (I-T2) in der R¹ für Methyl steht.

Eine weitere ganz besonders bevorzugte Ausführungsform auf Verbindungen der Formel (I-T3). Eine bevorzugte Ausführungsform bezieht sich dabei wiederum auf Verbindungen der Formel (I-T3) in der R¹ für H steht. Eine weitere bevorzugte Ausführungsform bezieht sich dabei wiederum auf Verbindungen der Formel (I-T3) in der R¹ für Methyl steht.

Eine weitere ganz besonders bevorzugte Ausführungsform auf Verbindungen der Formel (I-T4). Eine bevorzugte Ausführungsform bezieht sich dabei wiederum auf Verbindungen der Formel (I-T4) in der R¹ für H steht. Eine weitere bevorzugte Ausführungsform bezieht sich dabei wiederum auf Verbindungen der Formel (I-T4) in der R¹ für Methyl steht.

Eine weitere ganz besonders bevorzugte Ausführungsform auf Verbindungen der Formel (I-T22). Eine bevorzugte Ausführungsform bezieht sich dabei wiederum auf Verbindungen der Formel (I-T22) in der R¹ für H steht. Eine weitere bevorzugte Ausführungsform bezieht sich dabei wiederum auf Verbindungen der Formel (I-T22) in der R¹ für Methyl steht.

Eine weitere ganz besonders bevorzugte Ausführungsform auf Verbindungen der Formel (I-T23). Eine bevorzugte Ausführungsform bezieht sich dabei wiederum auf Verbindungen der Formel (I-T23) in der R¹ für H steht. Eine weitere bevorzugte Ausführungsform bezieht sich dabei wiederum auf Verbindungen der Formel (I-T23) in der R¹ für Methyl steht. 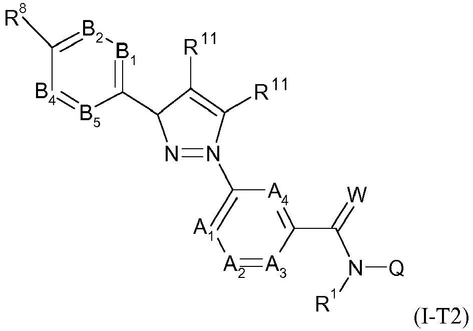 worin
R¹, A₁, A₂, A₃, A₄, R¹¹, B₁, B₂, B₄, B₅, R⁸, R¹¹ Q und W wie hierin beschrieben definiert sind wobei maximal eine Gruppierung ausgewählt aus A₁, A₂, A₃, A₄, für N steht und maximal eine Gruppierung ausgewählt aus B₁, B₂, B₃, B₄, und B₅ für N steht; oder wobei eine oder zwei Gruppierungen ausgewählt aus A₁, A₂, A₃, A₄, für N stehen können und maximal eine Gruppierung ausgewählt aus B₁, B₂, B₃, B₄, und B₅ für N steht; bzw. 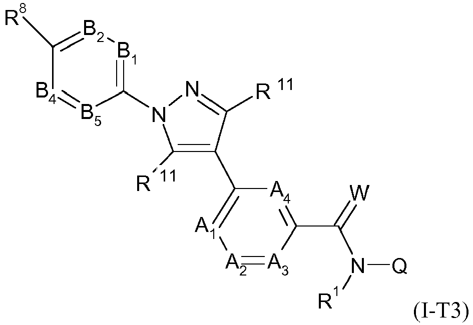 worin
R¹, A₁, A₂, A₃, A₄, R¹¹, B₁, B₂, B₄, B₅, R⁸, R¹¹ Q und W wie hierin beschrieben definiert sind wobei maximal eine Gruppierung ausgewählt aus A₁, A₂, A₃, A₄, für N steht und maximal eine Gruppierung ausgewählt aus B₁, B₂, B₃, B₄, und B₅ für N steht; oder wobei eine oder zwei Gruppierungen ausgewählt aus A₁, A₂, A₃, A₄, für N stehen können und maximal eine Gruppierung ausgewählt aus B₁, B₂, B₃, B₄, und B₅ für N steht; bzw. 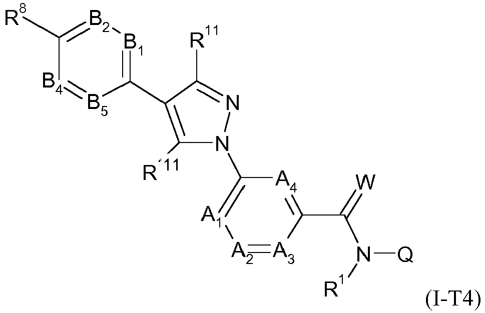 worin
R¹, A₁, A₂, A₃, A₄, R¹¹, B₁, B₂, B₄, B₅, R⁸, R¹¹ Q und W wie hierin beschrieben definiert sind wobei maximal eine Gruppierung ausgewählt aus A₁, A₂, A₃, A₄, für N steht und maximal eine Gruppierung ausgewählt aus B₁, B₂, B₃, B₄, und B₅ für N steht; oder wobei eine oder zwei Gruppierungen ausgewählt aus A₁, A₂, A₃, A₄, für N stehen können und maximal eine Gruppierung ausgewählt aus B₁, B₂, B₃, B₄, und B₅ für N steht; bzw. 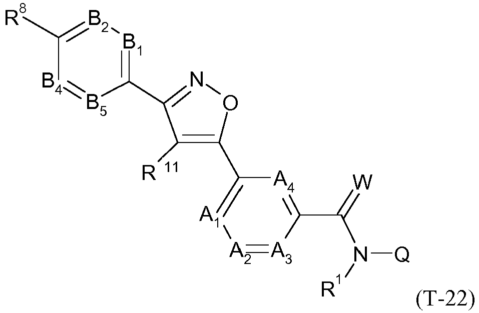 worin
R¹, A₁, A₂, A₃, A₄, R¹¹, B₁, B₂, B₄, B₅, R⁸, R¹¹ Q und W wie hierin beschrieben definiert sind wobei maximal eine Gruppierung ausgewählt aus A₁, A₂, A₃, A₄, für N steht und maximal eine Gruppierung ausgewählt aus B₁, B₂, B₃, B₄, und B₅ für N steht; oder wobei eine oder zwei Gruppierungen ausgewählt aus A₁, A₂, A₃, A₄, für N stehen können und maximal eine Gruppierung ausgewählt aus B₁, B₂, B₃, B₄, und B₅ für N steht; bzw. 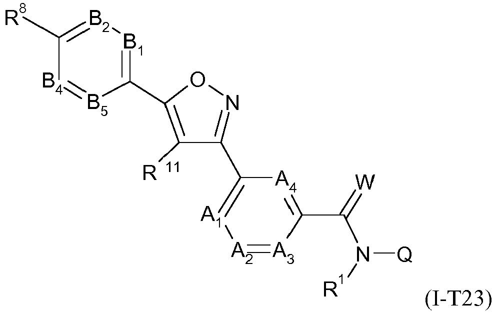 worin
R¹, A₁, A₂, A₃, A₄, R¹¹, B₁, B₂, B₄, B₅, R⁸, R¹¹ Q und W wie hierin beschrieben definiert sind wobei maximal eine Gruppierung ausgewählt aus A₁, A₂, A₃, A₄, für N steht und maximal eine Gruppierung ausgewählt aus B₁, B₂, B₃, B₄, und B₅ für N steht; oder wobei eine oder zwei Gruppierungen ausgewählt aus A₁, A₂, A₃, A₄, für N stehen können und maximal eine Gruppierung ausgewählt aus B₁, B₂, B₃, B₄, und B₅ für N steht.

Eine weitere bevorzugte Ausführungsform betrifft Verbindungen der Formel (In) (T = T2) 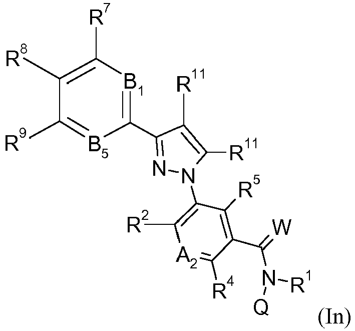 worin R¹, Q, W, A₂, B₁, B₅, R₂, R₄, R⁵, R⁶, R⁷, R⁸, R⁹ und R¹¹ wie hierin beschrieben definiert sind, bevorzugt, worin R¹ H darstellt oder worin R¹ Methyl darstellt.

Eine weiter bevorzugte Ausführungsform betrifft Verbindungen der Formel (In) worin
- W: für O steht;
- Q: für gegebenenfalls substituiertes C₁-C₄-Alkyl oder gegebenenfalls substituiertes C₃-C₆-Cycloalkyl oder für einen gegebenenfalls mit ein, zwei oder drei V substituierten, ungesättigten 4-, 5- bzw. 6-gliedrigen, heterozyklischen Ring steht, wobei V unabhängig voneinander für Halogen, Cyano, Nitro, Oxo (=O), gegebenenfalls mit Halogen substituiertes C₁-C₆-Alkyl, C₁-C₄-Alkenyl, C₁-C₆-Alkoxy, C₁-C₆-Alkylsulfanyl, C₁-C₆-Alkylsulfinyl, C₁-C₆-Alkylsulfonyl steht;
bevorzugt für mit Halogen substituiertes C¦-C₃-Alkyl; mit Cyano, Hydroxy oder Carbonamid (-C(=O)N(R)₂, wobei R unabhängig voneinander für H oder C₁-C₃-Alkyl steht, substituiertes C₁-C₃-Alkyl steht; C₃-Cycloalkyl; mit Cyano, Halogen, Nitro oder halogenierten C₁-C₂-Alkyl substituierten C₃-Cycloalkyl; einem gegebenenfalls mit ein, zwei, oder drei V substituierten, ungesättigten 4-, 5- bzw. 6-gliedrigen, heterozyklischen Ring enthaltend ein oder zwei Heteroatome ausgewählt aus einer Gruppe bestehend aus N, O und S, wobei V unabhängig voneinander für Halogen, Cyano, Nitro, Oxo (=O), gegebenenfalls mit Halogen substituiertes C₁-C₆-Alkyl steht, steht;
besonders bevorzugt für fluoriertes C₁-C₃-Alkyl wie CF₃, CH₂CF₃ oder CH₂CH₂CF₃; für mit Carbonamid (-C(=O)N(R)₂, wobei R unabhängig voneinander für H, C₁-C₃-Alkyl oder mit Halogen substituiertes C₁-C₃-Alkyl steht) substituiertes C₁-C₃-Alkyl wie 2-oxo-2-(2,2,2-trifluoroethylamino)ethyl; für Cyclopropyl; für mit Cyano oder fluorierten C₁-C₂-Alkyl substituierte Cyclopropyl wie 1 -(cyano)cyclopropyl oder 1-(trifluoromethyl)cyclopropyl); für einem 4-gliedrigen heterocyclischem Ring enthaltend ein Heteroatom ausgewählt aus einer Gruppe bestehend aus N, O oder S wie Thietan-3-yl steht;
- R⁷ und R⁹: jeweils für H stehen;
- R¹¹: jeweils für H steht;
- R¹: für H steht;
- R²: für H, Halogen oder C₁-C₄-alkyl steht, bevorzugt für H, Fluor, Chlor oder methyl steht;
- R⁴: für H oder Halogen steht, bevorzugt für H, Fluoro oder Chloro steht;
- R⁵: für H oder Halogen steht, bevorzugt für H, Fluoro oder Chloro steht;
- B₅: für N oder C-R¹⁰ steht, bevorzugt für C-R¹⁰, worin
- R¹⁰: für Wasserstoff, C₁-C₃-Alkyl, C₁-C₃-Alkoxy, mit Halogen substituiertes C₁-C₃-Alkyl (bevorzugt perfluoriertes C₁-C₃-Alkyl (CF₃, C₂F₅ oder C₃F₇)), mit Halogen substituiertes C₁-C₃-Alkoxy (bevorzugt perfluoriertes C₁-C₃-Alkoxy (OCF₃, OC₂F₅ oder OC₃F₇)), C₁-C₃-Alkylsulfanyl, C₁-C₃-Alkylsulfinyl, C₁-C₃-Alkylsulfonyl Fluor, Brom oder Chlor steht;
- A₂: für N or C-R³ steht, bevorzugt für C-R³, worin
- R³: für H, Halogen, oder gegebenenfalls substituiertes C₁-C₄-alkyl steht, bevorzugt für H, Fluor, Chlor oder gegebenenfalls mit Halogen substituiertes C₁-C₂-alkyl steht, besonders bevorzugt für H oder mit Fluoro substituiertes Methyl wie z. B. Perfluormethyl steht;
- R⁶: für Wasserstoff, C₁-C₃-Alkyl, C₁-C₃-Alkoxy, mit Halogen substituiertes C₁-C₃-Alkyl (bevorzugt perfluoriertes C₁-C₃-Alkyl (CF₃, C₂F₅ oder C₃F₇)), mit Halogen substituiertes C₁-C₃-Alkoxy (bevorzugt perfluoriertes C₁-C₃-Alkoxy (OCF₃, OC₂F₅ oder OC₃F₇)), C₁-C₃-Alkylsulfanyl, C₁-C₃-Alkylsulfinyl, C₁-C₃-Alkylsulfonyl, Fluor, Brom oder Chlor steht, bevorzugt für Fluor, Brom, Chlor, C₁-C₂-alkyl, mit Halogen substituiertes C₁-C₂-alkyl (z. B. Perfluormethyl) oder gegebenenfalls mit Halogen substituiertes C₁-C₂-alkoxy, besonders bevorzugt für Fluor, Brom, Chlor, Methyl, Ethyl, fluoriertes Methyl oder fluoriertes Ethyl (besonders bevorzugt Perfluormethyl oder Perfluorethyl), fluoriertes Methoxy oder fluoriertes Ethoxy (besonders bevorzugt Perfluormethoxy) steht;
- R⁸: für Halogen oder gegebenenfalls mit Halogen substituiertes C₁-C₄-alkyl oder gegebenenfalls mit Halogen substituiertes C₁-C₄-alkoxy steht, bevorzugt für mit Halogen substituiertes C₁-C₃-alkyl oder mit Halogen substituiertes C₁-C₃-alkoxy, mehr bevorzugt für mit Halogen substituiertes C₁-C₃-alkyl wie fluoriertes C₁-C₃-alkyl (z. B. fluoriertes C₃-alkyl wie perFluoropropyl) steht.

Eine weiter bevorzugte Ausführungsform betrifft Verbindungen der Formel (In) worin
- W: für O steht;
- Q: für gegebenenfalls substituiertes C₁-C₄-Alkyl oder gegebenenfalls substituiertes C₃-C₆-Cycloalkyl oder für einen gegebenenfalls mit ein, zwei oder drei V substituierten, ungesättigten 4-, 5- bzw. 6-gliedrigen, heterozyklischen Ring steht, wobei V unabhängig voneinander für Halogen, Cyano, Nitro, Oxo (=O), gegebenenfalls mit Halogen substituiertes C₁-C₆-Alkyl, C₁-C₄-Alkenyl, C₁-C₆-Alkoxy, C₁-C₆-Alkylsulfanyl, C₁-C₆-Alkylsulfinyl, C₁-C₆-Alkylsulfonyl steht;
bevorzugt für mit Halogen substituiertes C¦-C₃-Alkyl; mit Cyano, Hydroxy oder Carbonamid (-C(=O)N(R)₂, wobei R unabhängig voneinander für H oder C₁-C₃-Alkyl steht, substituiertes C₁-C₃-Alkyl steht; C₃-Cycloalkyl; mit Cyano, Halogen, Nitro oder halogenierten C₁-C₂-Alkyl substituierten C₃-Cycloalkyl; einem gegebenenfalls mit ein, zwei, oder drei V substituierten, ungesättigten 4-, 5- bzw. 6-gliedrigen, heterozyklischen Ring enthaltend ein oder zwei Heteroatome ausgewählt aus einer Gruppe bestehend aus N, O und S, wobei V unabhängig voneinander für Halogen, Cyano, Nitro, Oxo (=O), gegebenenfalls mit Halogen substituiertes C₁-C₆-Alkyl steht, steht;
besonders bevorzugt für fluoriertes C₁-C₃-Alkyl wie CF₃, CH₂CF₃ oder CH₂CH₂CF₃; für mit Carbonamid (-C(=O)N(R)₂, wobei R unabhängig voneinander für H, C₁-C₃-Alkyl oder mit Halogen substituiertes C₁-C₃-Alkyl steht) substituiertes C₁-C₃-Alkyl wie 2-oxo-2-(2,2,2-trifluoroethylamino)ethyl; für Cyclopropyl; für mit Cyano oder fluorierten C₁-C₂-Alkyl substituierte Cyclopropyl wie 1 -(cyano)cyclopropyl oder 1-(trifluoromethyl)cyclopropyl); für einem 4-gliedrigen heterocyclischem Ring enthaltend ein Heteroatom ausgewählt aus einer Gruppe bestehend aus N, O oder S wie Thietan-3-yl steht;
- R⁷ und R⁹: jeweils für H stehen;
- R¹¹: jeweils für H steht;
- R¹: für Methyl steht;
- R²: für H, Halogen oder C₁-C₄-alkyl steht, bevorzugt für H, Fluor, Chlor oder Methyl steht;
- R⁴: für H oder Halogen steht, bevorzugt für H, Fluoro oder Chloro steht;
- R⁵: für H oder Halogen steht, bevorzugt für H, Fluoro oder Chloro steht;
- B₅: für N or C-R¹⁰ steht, bevorzugt für C-R¹⁰, worin
- R¹⁰: für Wasserstoff, C₁-C₃-Alkyl, C₁-C₃-Alkoxy, mit Halogen substituiertes C₁-C₃-Alkyl (bevorzugt perfluoriertes C₁-C₃-Alkyl (CF₃, C₂F₅ oder C₃F₇)), mit Halogen substituiertes C₁-C₃-Alkoxy (bevorzugt perfluoriertes C₁-C₃-Alkoxy (OCF₃, OC₂F₅ oder OC₃F₇)), C₁-C₃-Alkylsulfanyl, C₁-C₃-Alkylsulfinyl, C₁-C₃-Alkylsulfonyl Fluor, Brom oder Chlor steht;
- A₂: für N or C-R³ steht, bevorzugt für C-R³, worin
- R³: für H, Halogen, oder gegebenenfalls substituiertes C₁-C₄-alkyl steht, bevorzugt für H, Fluor, Chlor oder gegebenenfalls mit Halogen substituiertes C₁-C₂-alkyl steht, besonders bevorzugt für H oder mit Fluoro substituiertes Methyl wie z. B. Perfluormethyl steht;
- R⁶: für Wasserstoff, C₁-C₃-Alkyl, C₁-C₃-Alkoxy, mit Halogen substituiertes C₁-C₃-Alkyl (bevorzugt perfluoriertes C₁-C₃-Alkyl (CF₃, C₂F₅ oder C₃F₇)), mit Halogen substituiertes C₁-C₃-Alkoxy (bevorzugt perfluoriertes C₁-C₃-Alkoxy (OCF₃, OC₂F₅ oder OC₃F₇)), C₁-C₃-Alkylsulfanyl, C₁-C₃-Alkylsulfinyl, C₁-C₃-Alkylsulfonyl Fluor, Brom oder Chlor steht, bevorzugt für Fluor, Brom, Chlor, C₁-C₂-alkyl, mit Halogen substituiertes C₁-C₂-alkyl (z. B. Perfluormethyl) oder gegebenenfalls mit Halogen substituiertes C₁-C₂-alkoxy, besonders bevorzugt für Fluor, Brom, Chlor, Methyl, Ethyl, fluoriertes Methyl oder fluoriertes Ethyl (besonders bevorzugt Perfluormethyl oder Perfluorethyl), fluoriertes Methoxy oder fluoriertes Ethoxy (besonders bevorzugt Perfluormethoxy) steht;
- R⁸: für Halogen oder gegebenenfalls mit Halogen substituiertes C₁-C₄-alkyl oder gegebenenfalls mit Halogen substituiertes C₁-C₄-alkoxy steht, bevorzugt für mit Halogen substituiertes C₁-C₃-alkyl oder mit Halogen substituiertes C₁-C₃-alkoxy, mehr bevorzugt für mit Halogen substituiertes C₁-C₃-alkyl wie fluoriertes C₁-C₃-alkyl (z. B. fluoriertes C₃-alkyl wie perFluoropropyl) steht.

Eine weitere bevorzugte Ausführungsform betrifft Verbindungen der Formel (Ie) (T = T3) 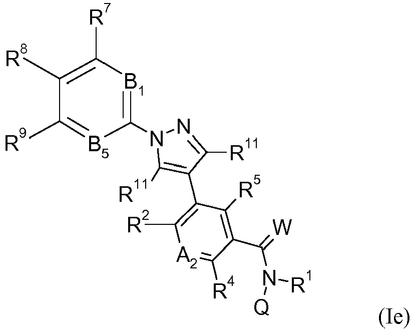 worin R¹, Q, W, A₂, B₁, B₅, R₂, R₄, R⁵, R⁶, R⁷, R⁸, R⁹ und R¹¹ wie hierin beschrieben definiert sind, bevorzugt, worin R¹ H darstellt oder worin R¹ Methyl darstellt.

Eine weiter bevorzugte Ausführungsform betrifft Verbindungen der Formel (Ie) worin
- W: für O steht;
- Q: für gegebenenfalls substituiertes C₁-C₄-Alkyl oder gegebenenfalls substituiertes C₃-C₆-Cycloalkyl oder für einen gegebenenfalls mit ein, zwei oder drei V substituierten, ungesättigten 4-, 5- bzw. 6-gliedrigen, heterozyklischen Ring steht, wobei V unabhängig voneinander für Halogen, Cyano, Nitro, Oxo (=O), gegebenenfalls mit Halogen substituiertes C₁-C₆-Alkyl, C₁-C₄-Alkenyl, C₁-C₆-Alkoxy, C₁-C₆-Alkylsulfanyl, C₁-C₆-Alkylsulfinyl, C₁-C₆-Alkylsulfonyl steht;
bevorzugt für mit Halogen substituiertes C¦-C₃-Alkyl; mit Cyano, Hydroxy oder Carbonamid (-C(=O)N(R)₂, wobei R unabhängig voneinander für H oder C₁-C₃-Alkyl steht, substituiertes C₁-C₃-Alkyl steht; C₃-Cycloalkyl; mit Cyano, Halogen, Nitro oder halogenierten C₁-C₂-Alkyl substituierten C₃-Cycloalkyl; einem gegebenenfalls mit ein, zwei, oder drei V substituierten, ungesättigten 4-, 5- bzw. 6-gliedrigen, heterozyklischen Ring enthaltend ein oder zwei Heteroatome ausgewählt aus einer Gruppe bestehend aus N, O und S, wobei V unabhängig voneinander für Halogen, Cyano, Nitro, Oxo (=O), gegebenenfalls mit Halogen substituiertes C₁-C₆-Alkyl steht, steht;
besonders bevorzugt für fluoriertes C₁-C₃-Alkyl wie CF₃, CH₂CF₃ oder CH₂CH₂CF₃; für mit Carbonamid (-C(=O)N(R)₂, wobei R unabhängig voneinander für H, C₁-C₃-Alkyl oder mit Halogen substituiertes C₁-C₃-Alkyl steht) substituiertes C₁-C₃-Alkyl wie 2-oxo-2-(2,2,2-trifluoroethylamino)ethyl; für Cyclopropyl; für mit Cyano oder fluorierten C₁-C₂-Alkyl substituierte Cyclopropyl wie 1 -(cyano)cyclopropyl oder 1-(trifluoromethyl)cyclopropyl); für einem 4-gliedrigen heterocyclischem Ring enthaltend ein Heteroatom ausgewählt aus einer Gruppe bestehend aus N, O oder S wie Thietan-3-yl steht;
- R⁷ und R⁹: jeweils für H stehen;
- R¹¹: jeweils für H steht;
- R¹: für H steht;
- R²: für H, Halogen oder C₁-C₄-alkyl steht, bevorzugt für H, Fluor, Chlor oder methyl steht;
- R⁴: für H oder Halogen steht, bevorzugt für H, Fluoro oder Chloro steht;
- R⁵: für H oder Halogen steht, bevorzugt für H, Fluoro oder Chloro steht;
- B₅: für N or C-R¹⁰ steht, bevorzugt für C-R¹⁰, worin
- R¹⁰: für Wasserstoff, C₁-C₃-Alkyl, C₁-C₃-Alkoxy, mit Halogen substituiertes C₁-C₃-Alkyl (bevorzugt perfluoriertes C₁-C₃-Alkyl (CF₃, C₂F₅ oder C₃F₇)), mit Halogen substituiertes C₁-C₃-Alkoxy (bevorzugt perfluoriertes C₁-C₃-Alkoxy (OCF₃, OC₂F₅ oder OC₃F₇)), C₁-C₃-Alkylsulfanyl, C₁-C₃-Alkylsulfinyl, C₁-C₃-Alkylsulfonyl Fluor, Brom oder Chlor steht;
- A₂: für N or C-R³ steht, bevorzugt für C-R³, worin
- R³: für H, Halogen, oder gegebenenfalls substituiertes C₁-C₄-alkyl steht, bevorzugt für H, Fluor, Chlor oder gegebenenfalls mit Halogen substituiertes C₁-C₂-alkyl steht, besonders bevorzugt für H oder mit Fluoro substituiertes Methyl wie z. B. Perfluormethyl steht;
- R⁶: für Wasserstoff, C₁-C₃-Alkyl, C₁-C₃-Alkoxy, mit Halogen substituiertes C₁-C₃-Alkyl (bevorzugt perfluoriertes C₁-C₃-Alkyl (CF₃, C₂F₅ oder C₃F₇)), mit Halogen substituiertes C₁-C₃-Alkoxy (bevorzugt perfluoriertes C₁-C₃-Alkoxy (OCF₃, OC₂F₅ oder OC₃F₇)), C₁-C₃-Alkylsulfanyl, C₁-C₃-Alkylsulfinyl, C₁-C₃-Alkylsulfonyl Fluor, Brom oder Chlor steht, bevorzugt für Fluor, Brom, Chlor, C₁-C₂-alkyl, mit Halogen substituiertes C₁-C₂-alkyl (z. B. Perfluormethyl) oder gegebenenfalls mit Halogen substituiertes C₁-C₂-alkoxy, besonders bevorzugt für Fluor, Brom, Chlor, Methyl, Ethyl, fluoriertes Methyl oder fluoriertes Ethyl (besonders bevorzugt Perfluormethyl oder Perfluorethyl), fluoriertes Methoxy oder fluoriertes Ethoxy (besonders bevorzugt Perfluormethoxy) steht;
- R⁸: für Halogen oder gegebenenfalls mit Halogen substituiertes C₁-C₄-alkyl oder gegebenenfalls mit Halogen substituiertes C₁-C₄-alkoxy steht, bevorzugt für mit Halogen substituiertes C₁-C₃-alkyl oder mit Halogen substituiertes C₁-C₃-alkoxy, mehr bevorzugt für mit Halogen substituiertes C₁-C₃-alkyl wie fluoriertes C₁-C₃-alkyl (z. B. fluoriertes C₃-alkyl wie perFluoropropyl) steht.

Eine weiter bevorzugte Ausführungsform betrifft Verbindungen der Formel (Ie) worin
- W: für O steht;
- Q: für gegebenenfalls substituiertes C₁-C₄-Alkyl oder gegebenenfalls substituiertes C₃-C₆-Cycloalkyl oder für einen gegebenenfalls mit ein, zwei oder drei V substituierten, ungesättigten 4-, 5- bzw. 6-gliedrigen, heterozyklischen Ring steht, wobei V unabhängig voneinander für Halogen, Cyano, Nitro, Oxo (=O), gegebenenfalls mit Halogen substituiertes C₁-C₆-Alkyl, C₁-C₄-Alkenyl, C₁-C₆-Alkoxy, C₁-C₆-Alkylsulfanyl, C₁-C₆-Alkylsulfinyl, C₁-C₆-Alkylsulfonyl steht;
bevorzugt für mit Halogen substituiertes C¦-C₃-Alkyl; mit Cyano, Hydroxy oder Carbonamid (-C(=O)N(R)₂, wobei R unabhängig voneinander für H oder C₁-C₃-Alkyl steht, substituiertes C₁-C₃-Alkyl steht; C₃-Cycloalkyl; mit Cyano, Halogen, Nitro oder halogenierten C₁-C₂-Alkyl substituierten C₃-Cycloalkyl; einem gegebenenfalls mit ein, zwei, oder drei V substituierten, ungesättigten 4-, 5- bzw. 6-gliedrigen, heterozyklischen Ring enthaltend ein oder zwei Heteroatome ausgewählt aus einer Gruppe bestehend aus N, O und S, wobei V unabhängig voneinander für Halogen, Cyano, Nitro, Oxo (=O), gegebenenfalls mit Halogen substituiertes C₁-C₆-Alkyl steht, steht;
besonders bevorzugt für fluoriertes C₁-C₃-Alkyl wie CF₃, CH₂CF₃ oder CH₂CH₂CF₃; für mit Carbonamid (-C(=O)N(R)₂, wobei R unabhängig voneinander für H, C₁-C₃-Alkyl oder mit Halogen substituiertes C₁-C₃-Alkyl steht) substituiertes C₁-C₃-Alkyl wie 2-oxo-2-(2,2,2-trifluoroethylamino)ethyl; für Cyclopropyl; für mit Cyano oder fluorierten C₁-C₂-Alkyl substituierte Cyclopropyl wie 1 -(cyano)cyclopropyl oder 1-(trifluoromethyl)cyclopropyl); für einem 4-gliedrigen heterocyclischem Ring enthaltend ein Heteroatom ausgewählt aus einer Gruppe bestehend aus N, O oder S wie Thietan-3-yl steht;
- R⁷ und R⁹: jeweils für H stehen;
- R¹¹: jeweils für H steht;
- R¹: für Methyl steht;
- R²: für H, Halogen oder C₁-C₄-alkyl steht, bevorzugt für H, Fluor, Chlor oder methyl steht;
- R⁴: für H oder Halogen steht, bevorzugt für H, Fluoro oder Chloro steht;
- R⁵: für H oder Halogen steht, bevorzugt für H, Fluoro oder Chloro steht;
- B₅: für N or C-R¹⁰ steht, bevorzugt für C-R¹⁰, worin
- R¹⁰: für Wasserstoff, C₁-C₃-Alkyl, C₁-C₃-Alkoxy, mit Halogen substituiertes C₁-C₃-Alkyl (bevorzugt perfluoriertes C₁-C₃-Alkyl (CF₃, C₂F₅ oder C₃F₇)), mit Halogen substituiertes C₁-C₃-Alkoxy (bevorzugt perfluoriertes C₁-C₃-Alkoxy (OCF₃, OC₂F₅ oder OC₃F₇)), C₁-C₃-Alkylsulfanyl, C₁-C₃-Alkylsulfinyl, C₁-C₃-Alkylsulfonyl Fluor, Brom oder Chlor steht;
- A₂: für N or C-R³ steht, bevorzugt für C-R³, worin
- R³: für H, Halogen, oder gegebenenfalls substituiertes C₁-C₄-alkyl steht, bevorzugt für H, Fluor, Chlor oder gegebenenfalls mit Halogen substituiertes C₁-C₂-alkyl steht, besonders bevorzugt für H oder mit Fluoro substituiertes Methyl wie z. B. Perfluormethyl steht;
- R⁶: für Wasserstoff, C₁-C₃-Alkyl, C₁-C₃-Alkoxy, mit Halogen substituiertes C₁-C₃-Alkyl (bevorzugt perfluoriertes C₁-C₃-Alkyl (CF₃, C₂F₅ oder C₃F₇)), mit Halogen substituiertes C₁-C₃-Alkoxy (bevorzugt perfluoriertes C₁-C₃-Alkoxy (OCF₃, OC₂F₅ oder OC₃F₇)), C₁-C₃-Alkylsulfanyl, C₁-C₃-Alkylsulfinyl, C₁-C₃-Alkylsulfonyl Fluor, Brom oder Chlor steht, bevorzugt für Fluor, Brom, Chlor, C₁-C₂-alkyl, mit Halogen substituiertes C₁-C₂-alkyl (z. B. Perfluormethyl) oder gegebenenfalls mit Halogen substituiertes C₁-C₂-alkoxy, besonders bevorzugt für Fluor, Brom, Chlor, Methyl, Ethyl, fluoriertes Methyl oder fluoriertes Ethyl (besonders bevorzugt Perfluormethyl oder Perfluorethyl), fluoriertes Methoxy oder fluoriertes Ethoxy (besonders bevorzugt Perfluormethoxy) steht;
- R⁸: für Halogen oder gegebenenfalls mit Halogen substituiertes C₁-C₄-alkyl oder gegebenenfalls mit Halogen substituiertes C₁-C₄-alkoxy steht, bevorzugt für mit Halogen substituiertes C₁-C₃-alkyl oder mit Halogen substituiertes C₁-C₃-alkoxy, mehr bevorzugt für mit Halogen substituiertes C₁-C₃-alkyl wie fluoriertes C₁-C₃-alkyl (z. B. fluoriertes C₃-alkyl wie perfluoropropyl) steht.

Eine weiter bevorzugte Ausführungsform betrifft Verbindungen der Formel (If) (T = T23) 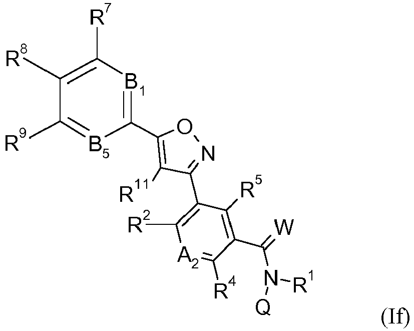 worin R¹, Q, W, A₂, B₁, B₅, R², R⁴, R⁵, R⁶, R⁷, R⁸, R⁹ und R¹¹ wie hierin beschrieben definiert sind bevorzugt, worin R¹ H darstellt oder worin R¹ Methyl darstellt.

Eine bevorzugte Ausführungsform betrifft Verbindungen der Formel (If) worin
- W: für O steht;
- Q: für gegebenenfalls substituiertes C₁-C₄-Alkyl oder gegebenenfalls substituiertes C₃-C₆-Cycloalkyl oder für einen gegebenenfalls mit ein, zwei oder drei V substituierten, ungesättigten 4-, 5- bzw. 6-gliedrigen, heterozyklischen Ring steht, wobei V unabhängig voneinander für Halogen, Cyano, Nitro, Oxo (=O), gegebenenfalls mit Halogen substituiertes C₁-C₆-Alkyl, C₁-C₄-Alkenyl, C₁-C₆-Alkoxy, C₁-C₆-Alkylsulfanyl, C₁-C₆-Alkylsulfinyl, C₁-C₆-Alkylsulfonyl steht;
bevorzugt für mit Halogen substituiertes C¦-C₃-Alkyl; mit Cyano, Hydroxy oder Carbonamid (-C(=O)N(R)₂, wobei R unabhängig voneinander für H oder C₁-C₃-Alkyl steht, substituiertes C₁-C₃-Alkyl steht; C₃-Cycloalkyl; mit Cyano, Halogen, Nitro oder halogenierten C₁-C₂-Alkyl substituierten C₃-Cycloalkyl; einem gegebenenfalls mit ein, zwei, oder drei V substituierten, ungesättigten 4-, 5- bzw. 6-gliedrigen, heterozyklischen Ring enthaltend ein oder zwei Heteroatome ausgewählt aus einer Gruppe bestehend aus N, O und S, wobei V unabhängig voneinander für Halogen, Cyano, Nitro, Oxo (=O), gegebenenfalls mit Halogen substituiertes C₁-C₆-Alkyl steht, steht;
besonders bevorzugt für fluoriertes C₁-C₃-Alkyl wie CF₃, CH₂CF₃ oder CH₂CH₂CF₃; für mit Carbonamid (-C(=O)N(R)₂, wobei R unabhängig voneinander für H, C₁-C₃-Alkyl oder mit Halogen substituiertes C₁-C₃-Alkyl steht) substituiertes C₁-C₃-Alkyl wie 2-oxo-2-(2,2,2-trifluoroethylamino)ethyl; für Cyclopropyl; für mit Cyano oder fluorierten C₁-C₂-Alkyl substituierte Cyclopropyl wie 1-(cyano)cyclopropyl oder 1-(trifluoromethyl)cyclopropyl); für einem 4-gliedrigen heterocyclischem Ring enthaltend ein Heteroatom ausgewählt aus einer Gruppe bestehend aus N, O oder S wie Thietan-3-yl steht;
- R⁷ und R⁹: jeweils für H stehen;
- R¹¹: jeweils für H steht;
- R¹: für H steht;
- R²: für H, Halogen oder C₁-C₄-alkyl steht, bevorzugt für H, Fluor, Chlor oder Methyl steht;
- R⁴: für H oder Halogen steht, bevorzugt für H, Fluor oder Chlor steht;
- R⁵: für H oder Halogen steht, bevorzugt für H, Fluor oder Chlor steht;
- B₅: für N oder C-R¹⁰ steht, bevorzugt für C-R¹⁰, worin
- R¹⁰: für H, Halogen, C₁-C₄-alkyl oder C₁-C₄-alkoxy steht, bevorzugt für H, Fluor, Brom, Chlor, C₁-C₂-alkyl oder C₁-C₂-alkoxy, besonders bevorzugt für H, Chlor, Brom Fluor, methyl oder methoxy steht;
- A₂: für N oder C-R³ steht, bevorzugt für C-R³, worin
- R³: für H, Halogen, oder gegebenenfalls substituiertes C₁-C₄-alkyl steht, bevorzugt für H, Fluor, Chlor oder gegebenenfalls mit Halogen substituiertes C₁-C₂-alkyl steht, besonders bevorzugt für H oder mit Fluor substituiertes Methyl wie z. B. Perfluormethyl steht;
- R⁶: für H, Halogen, gegebenenfalls substituiertes C₁-C₄-alkyl oder gegebenenfalls substituiertes C₁-C₄-alkoxy steht, bevorzugt für Fluor, Chlor, C₁-C₂-alkyl, mit Halogen substituiertes C₁-C₂-alkyl (z. B. Perfluormethyl) oder gegebenenfalls mit Halogen substituiertes C₁-C₂-alkoxy, besonders bevorzugt für Fluor, Brom, Chlor, Methyl, Ethyl, fluoriertes Methyl oder fluoriertes Ethyl (besonders bevorzugt Perfluormethyl oder Perfluorethyl), fluoriertes Methoxy oder fluoriertes Ethoxy (besonders bevorzugt Perfluormethoxy) steht;
- R⁸: für Halogen oder gegebenenfalls mit Halogen substituiertes C₁-C₄-alkyl oder gegebenenfalls mit Halogen substituiertes C₁-C₄-alkoxy steht, bevorzugt für mit Halogen substituiertes C₁-C₃-alkyl oder mit Halogen substituiertes C₁-C₃-alkoxy, mehr bevorzugt für mit Halogen substituiertes C₁-C₃-alkyl wie fluoriertes C₁-C₃-alkyl (z. B. fluoriertes C₃-alkyl wie Perfluorpropyl) steht.

Eine bevorzugte Ausführungsform betrifft Verbindungen der Formel (If) worin
- W: für O steht;
- Q: für gegebenenfalls substituiertes C₁-C₄-Alkyl oder gegebenenfalls substituiertes C₃-C₆-Cycloalkyl oder für einen gegebenenfalls mit ein, zwei oder drei V substituierten, ungesättigten 4-, 5- bzw. 6-gliedrigen, heterozyklischen Ring steht, wobei V unabhängig voneinander für Halogen, Cyano, Nitro, Oxo (=O), gegebenenfalls mit Halogen substituiertes C₁-C₆-Alkyl, C₁-C₄-Alkenyl, C₁-C₆-Alkoxy, C₁-C₆-Alkylsulfanyl, C₁-C₆-Alkylsulfinyl, C₁-C₆-Alkylsulfonyl steht;
bevorzugt für mit Halogen substituiertes C¦-C₃-Alkyl; mit Cyano, Hydroxy oder Carbonamid (-C(=O)N(R)₂, wobei R unabhängig voneinander für H oder C₁-C₃-Alkyl steht, substituiertes C₁-C₃-Alkyl steht; C₃-Cycloalkyl; mit Cyano, Halogen, Nitro oder halogenierten C₁-C₂-Alkyl substituierten C₃-Cycloalkyl; einem gegebenenfalls mit ein, zwei, oder drei V substituierten, ungesättigten 4-, 5- bzw. 6-gliedrigen, heterozyklischen Ring enthaltend ein oder zwei Heteroatome ausgewählt aus einer Gruppe bestehend aus N, O und S, wobei V unabhängig voneinander für Halogen, Cyano, Nitro, Oxo (=O), gegebenenfalls mit Halogen substituiertes C₁-C₆-Alkyl steht, steht;besonders bevorzugt für fluoriertes C₁-C₃-Alkyl wie CF₃, CH₂CF₃ oder CH₂CH₂CF₃; für mit Carbonamid (-C(=O)N(R)₂, wobei R unabhängig voneinander für H, C₁-C₃-Alkyl oder mit Halogen substituiertes C₁-C₃-Alkyl steht) substituiertes C₁-C₃-Alkyl wie 2-oxo-2-(2,2,2-trifluoroethylamino)ethyl; für Cyclopropyl; für mit Cyano oder fluorierten C₁-C₂-Alkyl substituierte Cyclopropyl wie 1 -(cyano)cyclopropyl oder 1-(trifluoromethyl)cyclopropyl); für einem 4-gliedrigen heterocyclischem Ring enthaltend ein Heteroatom ausgewählt aus einer Gruppe bestehend aus N, O oder S wie Thietan-3-yl steht.
- R⁷ und R⁹: jeweils für H stehen;
- R¹¹: jeweils für H steht;
- R¹: für Methyl steht;
- R²: für H, Halogen oder C₁-C₄-alkyl steht, bevorzugt für H, Fluor, Chlor oder Methyl steht;
- R⁴: für H oder Halogen steht, bevorzugt für H, Fluor oder Chlor steht;
- R⁵: für H oder Halogen steht, bevorzugt für H, Fluor oder Chlor steht;
- B₅: für N oder C-R¹⁰ steht, bevorzugt für C-R¹⁰, worin
- R¹⁰: für Wasserstoff, C₁-C₃-Alkyl, C₁-C₃-Alkoxy, mit Halogen substituiertes C₁-C₃-Alkyl (bevorzugt perfluoriertes C₁-C₃-Alkyl (CF₃, C₂F₅ oder C₃F₇)), mit Halogen substituiertes C₁-C₃-Alkoxy (bevorzugt perfluoriertes C₁-C₃-Alkoxy (OCF₃, OC₂F₅ oder OC₃F₇)), C₁-C₃-Alkylsulfanyl, C₁-C₃-Alkylsulfinyl, C₁-C₃-Alkylsulfonyl Fluor, Brom oder Chlor steht;
- A₂: für N or C-R³ steht, bevorzugt für C-R³, worin
- R³: für H, Halogen, oder gegebenenfalls substituiertes C₁-C₄-alkyl steht, bevorzugt für H, Fluor, Chlor oder gegebenenfalls mit Halogen substituiertes C₁-C₂-alkyl steht, besonders bevorzugt für H oder mit Fluoro substituiertes Methyl wie z. B. Perfluormethyl steht;
- R⁶: für Wasserstoff, C₁-C₃-Alkyl, C₁-C₃-Alkoxy, mit Halogen substituiertes C₁-C₃-Alkyl (bevorzugt perfluoriertes C₁-C₃-Alkyl (CF₃, C₂F₅ oder C₃F₇)), mit Halogen substituiertes C₁-C₃-Alkoxy (bevorzugt perfluoriertes C₁-C₃-Alkoxy (OCF₃, OC₂F₅ oder OC₃F₇)), C₁-C₃-Alkylsulfanyl, C₁-C₃-Alkylsulfinyl, C₁-C₃-Alkylsulfonyl Fluor, Brom oder Chlor steht, bevorzugt für Fluor, Brom, Chlor, C₁-C₂-alkyl, mit Halogen substituiertes C₁-C₂-alkyl (z. B. Perfluormethyl) oder gegebenenfalls mit Halogen substituiertes C₁-C₂-alkoxy, besonders bevorzugt für Fluor, Brom, Chlor, Methyl, Ethyl, fluoriertes Methyl oder fluoriertes Ethyl (besonders bevorzugt Perfluormethyl oder Perfluorethyl), fluoriertes Methoxy oder fluoriertes Ethoxy (besonders bevorzugt Perfluormethoxy) steht;
- R⁸: für Halogen oder gegebenenfalls mit Halogen substituiertes C₁-C₄-alkyl oder gegebenenfalls mit Halogen substituiertes C₁-C₄-alkoxy steht, bevorzugt für mit Halogen substituiertes C₁-C₃-alkyl oder mit Halogen substituiertes C₁-C₃-alkoxy, mehr bevorzugt für mit Halogen substituiertes C₁-C₃-alkyl wie fluoriertes C₁-C₃-alkyl (z. B. fluoriertes C₃-alkyl wie Perfluorpropyl) steht.

Eine weiter bevorzugte Ausführungsform betrifft Verbindungen der Formel (Ig) (T = T4) 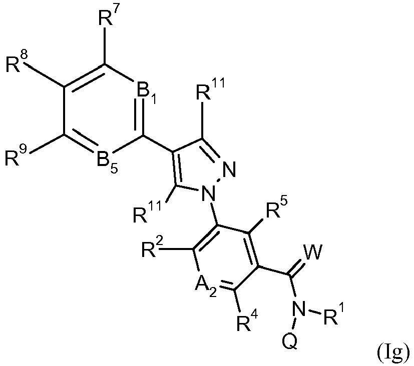 worin R¹, Q, W, A₂, B₁, B₅, R², R⁴, R⁵, R⁶, R⁷, R⁸, R⁹ und R¹¹ wie hierin beschrieben definiert sind bevorzugt, worin R¹ H darstellt oder worin R¹ Methyl darstellt.

Eine bevorzugte Ausführungsform betrifft Verbindungen der Formel (Ig) worin
- W: für O steht;
- Q: für gegebenenfalls substituiertes C₁-C₄-Alkyl oder gegebenenfalls substituiertes C₃-C₆-Cycloalkyl oder für einen gegebenenfalls mit ein, zwei oder drei V substituierten, ungesättigten 4-, 5- bzw. 6-gliedrigen, heterozyklischen Ring steht, wobei V unabhängig voneinander für Halogen, Cyano, Nitro, Oxo (=O), gegebenenfalls mit Halogen substituiertes C₁-C₆-Alkyl, C₁-C₄-Alkenyl, C₁-C₆-Alkoxy, C₁-C₆-Alkylsulfanyl, C₁-C₆-Alkylsulfinyl, C₁-C₆-Alkylsulfonyl steht;
bevorzugt für mit Halogen substituiertes C¦-C₃-Alkyl; mit Cyano, Hydroxy oder Carbonamid (-C(=O)N(R)₂, wobei R unabhängig voneinander für H oder C₁-C₃-Alkyl steht, substituiertes C₁-C₃-Alkyl steht; C₃-Cycloalkyl; mit Cyano, Halogen, Nitro oder halogenierten C₁-C₂-Alkyl substituierten C₃-Cycloalkyl; einem gegebenenfalls mit ein, zwei, oder drei V substituierten, ungesättigten 4-, 5- bzw. 6-gliedrigen, heterozyklischen Ring enthaltend ein oder zwei Heteroatome ausgewählt aus einer Gruppe bestehend aus N, O und S, wobei V unabhängig voneinander für Halogen, Cyano, Nitro, Oxo (=O), gegebenenfalls mit Halogen substituiertes C₁-C₆-Alkyl steht, steht;
besonders bevorzugt für fluoriertes C₁-C₃-Alkyl wie CF₃, CH₂CF₃ oder CH₂CH₂CF₃; für mit Carbonamid (-C(=O)N(R)₂, wobei R unabhängig voneinander für H, C₁-C₃-Alkyl oder mit Halogen substituiertes C₁-C₃-Alkyl steht) substituiertes C₁-C₃-Alkyl wie 2-oxo-2-(2,2,2-trifluoroethylamino)ethyl; für Cyclopropyl; für mit Cyano oder fluorierten C₁-C₂-Alkyl substituierte Cyclopropyl wie 1 -(cyano)cyclopropyl oder 1-(trifluoromethyl)cyclopropyl); für einem 4-gliedrigen heterocyclischem Ring enthaltend ein Heteroatom ausgewählt aus einer Gruppe bestehend aus N, O oder S wie Thietan-3-yl steht.
- R⁷ und R⁹: jeweils für H stehen;
- R¹¹: jeweils für H steht;
- R¹: für H steht;
- R²: für H, Halogen oder C₁-C₄-alkyl steht, bevorzugt für H, Fluor, Chlor oder Methyl steht;
- R⁴: für H oder Halogen steht, bevorzugt für H, Fluor oder Chlor steht;
- R⁵: für H oder Halogen steht, bevorzugt für H, Fluor oder Chlor steht;
- B₅: für N oder C-R¹⁰ steht, bevorzugt für C-R¹⁰, worin
- R¹⁰: für Wasserstoff, C₁-C₃-Alkyl, C₁-C₃-Alkoxy, mit Halogen substituiertes C₁-C₃-Alkyl (bevorzugt perfluoriertes C₁-C₃-Alkyl (CF₃, C₂F₅ oder C₃F₇)), mit Halogen substituiertes C₁-C₃-Alkoxy (bevorzugt perfluoriertes C₁-C₃-Alkoxy (OCF₃, OC₂F₅ oder OC₃F₇)), C₁-C₃-Alkylsulfanyl, C₁-C₃-Alkylsulfinyl, C₁-C₃-Alkylsulfonyl Fluor, Brom oder Chlor steht;
- A₂: für N or C-R³ steht, bevorzugt für C-R³, worin
- R³: für H, Halogen, oder gegebenenfalls substituiertes C₁-C₄-alkyl steht, bevorzugt für H, Fluor, Chlor oder gegebenenfalls mit Halogen substituiertes C₁-C₂-alkyl steht, besonders bevorzugt für H oder mit Fluoro substituiertes Methyl wie z. B. Perfluormethyl steht;
- R⁶: für Wasserstoff, C₁-C₃-Alkyl, C₁-C₃-Alkoxy, mit Halogen substituiertes C₁-C₃-Alkyl (bevorzugt perfluoriertes C₁-C₃-Alkyl (CF₃, C₂F₅ oder C₃F₇)), mit Halogen substituiertes C₁-C₃-Alkoxy (bevorzugt perfluoriertes C₁-C₃-Alkoxy (OCF₃, OC₂F₅ oder OC₃F₇)), C₁-C₃-Alkylsulfanyl, C₁-C₃-Alkylsulfinyl, C₁-C₃-Alkylsulfonyl Fluor, Brom oder Chlor steht, bevorzugt für Fluor, Brom, Chlor, C₁-C₂-alkyl, mit Halogen substituiertes C₁-C₂-alkyl (z. B. Perfluormethyl) oder gegebenenfalls mit Halogen substituiertes C₁-C₂-alkoxy, besonders bevorzugt für Fluor, Brom, Chlor, Methyl, Ethyl, fluoriertes Methyl oder fluoriertes Ethyl (besonders bevorzugt Perfluormethyl oder Perfluorethyl), fluoriertes Methoxy oder fluoriertes Ethoxy (besonders bevorzugt Perfluormethoxy) steht;
- R⁸: für Halogen oder gegebenenfalls mit Halogen substituiertes C₁-C₄-alkyl oder gegebenenfalls mit Halogen substituiertes C₁-C₄-alkoxy steht, bevorzugt für mit Halogen substituiertes C₁-C₃-alkyl oder mit Halogen substituiertes C₁-C₃-alkoxy, mehr bevorzugt für mit Halogen substituiertes C₁-C₃-alkyl wie fluoriertes C₁-C₃-alkyl (z. B. fluoriertes C₃-alkyl wie Perfluorpropyl) steht.

Eine bevorzugte Ausführungsform betrifft Verbindungen der Formel (Ig) worin
- W: für O steht;
- Q: für gegebenenfalls substituiertes C₁-C₄-Alkyl oder gegebenenfalls substituiertes C₃-C₆-Cycloalkyl oder für einen gegebenenfalls mit ein, zwei oder drei V substituierten, ungesättigten 4-, 5- bzw. 6-gliedrigen, heterozyklischen Ring steht, wobei V unabhängig voneinander für Halogen, Cyano, Nitro, Oxo (=O), gegebenenfalls mit Halogen substituiertes C₁-C₆-Alkyl, C₁-C₄-Alkenyl, C₁-C₆-Alkoxy, C₁-C₆-Alkylsulfanyl, C₁-C₆-Alkylsulfinyl, C₁-C₆-Alkylsulfonyl steht;
bevorzugt für mit Halogen substituiertes C₁-C₃-Alkyl; mit Cyano, Hydroxy oder Carbonamid (-C(=O)N(R)₂, wobei R unabhängig voneinander für H oder C₁-C₃-Alkyl steht, substituiertes C₁-C₃-Alkyl steht; C₃-Cycloalkyl; mit Cyano, Halogen, Nitro oder halogenierten C₁-C₂-Alkyl substituierten C₃-Cycloalkyl; einem gegebenenfalls mit ein, zwei, oder drei V substituierten, ungesättigten 4-, 5- bzw. 6-gliedrigen, heterozyklischen Ring enthaltend ein oder zwei Heteroatome ausgewählt aus einer Gruppe bestehend aus N, O und S, wobei V unabhängig voneinander für Halogen, Cyano, Nitro, Oxo (=O), gegebenenfalls mit Halogen substituiertes C₁-C₆-Alkyl steht, steht;
besonders bevorzugt für fluoriertes C₁-C₃-Alkyl wie CF₃, CH₂CF₃ oder CH₂CH₂CF₃; für mit Carbonamid (-C(=O)N(R)₂, wobei R unabhängig voneinander für H, C₁-C₃-Alkyl oder mit Halogen substituiertes C₁-C₃-Alkyl steht) substituiertes C₁-C₃-Alkyl wie 2-oxo-2-(2,2,2-trifluoroethylamino)ethyl; für Cyclopropyl; für mit Cyano oder fluorierten C₁-C₂-Alkyl substituierte Cyclopropyl wie 1 -(cyano)cyclopropyl oder 1-(trifluoromethyl)cyclopropyl); für einem 4-gliedrigen heterocyclischem Ring enthaltend ein Heteroatom ausgewählt aus einer Gruppe bestehend aus N, O oder S wie Thietan-3-yl steht.
- R⁷ und R⁹: jeweils für H stehen;
- R¹¹: jeweils für H steht;
- R¹: für Methyl steht;
- R²: für H, Halogen oder C₁-C₄-alkyl steht, bevorzugt für H, Fluor, Chlor oder Methyl steht;
- R⁴: für H oder Halogen steht, bevorzugt für H, Fluor oder Chlor steht;
- R⁵: für H oder Halogen steht, bevorzugt für H, Fluor oder Chlor steht;
- B₅: für N oder C-R¹⁰ steht, bevorzugt für C-R¹⁰, worin
- R¹⁰: für Wasserstoff, C₁-C₃-Alkyl, C₁-C₃-Alkoxy, mit Halogen substituiertes C₁-C₃-Alkyl (bevorzugt perfluoriertes C₁-C₃-Alkyl (CF₃, C₂F₅ oder C₃F₇)), mit Halogen substituiertes C₁-C₃-Alkoxy (bevorzugt perfluoriertes C₁-C₃-Alkoxy (OCF₃, OC₂F₅ oder OC₃F₇)), C₁-C₃-Alkylsulfanyl, C₁-C₃-Alkylsulfinyl, C₁-C₃-Alkylsulfonyl Fluor, Brom oder Chlor steht;
- A₂: für N or C-R³ steht, bevorzugt für C-R³, worin
- R³: für H, Halogen, oder gegebenenfalls substituiertes C₁-C₄-alkyl steht, bevorzugt für H, Fluor, Chlor oder gegebenenfalls mit Halogen substituiertes C₁-C₂-alkyl steht, besonders bevorzugt für H oder mit Fluoro substituiertes Methyl wie z. B. Perfluormethyl steht;
- R⁶: für Wasserstoff, C₁-C₃-Alkyl, C₁-C₃-Alkoxy, mit Halogen substituiertes C₁-C₃-Alkyl (bevorzugt perfluoriertes C₁-C₃-Alkyl (CF₃, C₂F₅ oder C₃F₇)), mit Halogen substituiertes C₁-C₃-Alkoxy (bevorzugt perfluoriertes C₁-C₃-Alkoxy (OCF₃, OC₂F₅ oder OC₃F₇)), C₁-C₃-Alkylsulfanyl, C₁-C₃-Alkylsulfinyl, C₁-C₃-Alkylsulfonyl Fluor, Brom oder Chlor steht, bevorzugt für Fluor, Brom, Chlor, C₁-C₂-alkyl, mit Halogen substituiertes C₁-C₂-alkyl (z. B. Perfluormethyl) oder gegebenenfalls mit Halogen substituiertes C₁-C₂-alkoxy, besonders bevorzugt für Fluor, Brom, Chlor, Methyl, Ethyl, fluoriertes Methyl oder fluoriertes Ethyl (besonders bevorzugt Perfluormethyl oder Perfluorethyl), fluoriertes Methoxy oder fluoriertes Ethoxy (besonders bevorzugt Perfluormethoxy) steht;
- R⁸: für Halogen oder gegebenenfalls mit Halogen substituiertes C₁-C₄-alkyl oder gegebenenfalls mit Halogen substituiertes C₁-C₄-alkoxy steht, bevorzugt für mit Halogen substituiertes C₁-C₃-alkyl oder mit Halogen substituiertes C₁-C₃-alkoxy, mehr bevorzugt für mit Halogen substituiertes C₁-C₃-alkyl wie fluoriertes C₁-C₃-alkyl (z. B. fluoriertes C₃-alkyl wie Perfluorpropyl) steht.

Eine weiter bevorzugte Ausführungsform betrifft Verbindungen der Formel (Io) (T = T22) 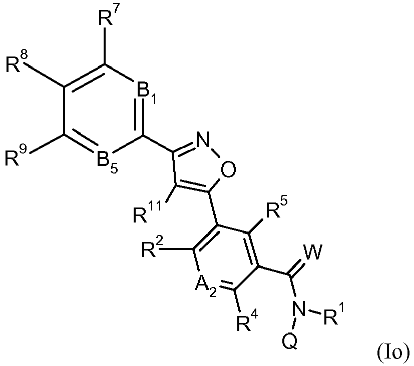 worin R¹, Q, W, A₂, B₁, B₅, R², R⁴, R⁵, R⁶, R⁷, R⁸, R⁹ und R¹¹ wie hierin beschrieben definiert sind bevorzugt, worin R¹ H darstellt oder worin R¹ Methyl darstellt.

Eine bevorzugte Ausführungsform betrifft Verbindungen der Formel (Io) worin
- W: für O steht;
- Q: für gegebenenfalls substituiertes C₁-C₄-Alkyl oder gegebenenfalls substituiertes C₃-C₆-Cycloalkyl oder für einen gegebenenfalls mit ein, zwei oder drei V substituierten, ungesättigten 4-, 5- bzw. 6-gliedrigen, heterozyklischen Ring steht, wobei V unabhängig voneinander für Halogen, Cyano, Nitro, Oxo (=O), gegebenenfalls mit Halogen substituiertes C₁-C₆-Alkyl, C₁-C₄-Alkenyl, C₁-C₆-Alkoxy, C₁-C₆-Alkylsulfanyl, C₁-C₆-Alkylsulfinyl, C₁-C₆-Alkylsulfonyl steht;
bevorzugt für mit Halogen substituiertes C₁-C₃-Alkyl; mit Cyano, Hydroxy oder Carbonamid (-C(=O)N(R)₂, wobei R unabhängig voneinander für H oder C₁-C₃-Alkyl steht, substituiertes C₁-C₃-Alkyl steht; C₃-Cycloalkyl; mit Cyano, Halogen, Nitro oder halogenierten C₁-C₂-Alkyl substituierten C₃-Cycloalkyl; einem gegebenenfalls mit ein, zwei, oder drei V substituierten, ungesättigten 4-, 5- bzw. 6-gliedrigen, heterozyklischen Ring enthaltend ein oder zwei Heteroatome ausgewählt aus einer Gruppe bestehend aus N, O und S, wobei V unabhängig voneinander für Halogen, Cyano, Nitro, Oxo (=O), gegebenenfalls mit Halogen substituiertes C₁-C₆-Alkyl steht, steht;
besonders bevorzugt für fluoriertes C₁-C₃-Alkyl wie CF₃, CH₂CF₃ oder CH₂CH₂CF₃; für mit Carbonamid (-C(=O)N(R)₂, wobei R unabhängig voneinander für H, C₁-C₃-Alkyl oder mit Halogen substituiertes C₁-C₃-Alkyl steht) substituiertes C₁-C₃-Alkyl wie 2-oxo-2-(2,2,2-trifluoroethylamino)ethyl; für Cyclopropyl; für mit Cyano oder fluorierten C₁-C₂-Alkyl substituierte Cyclopropyl wie 1 -(cyano)cyclopropyl oder 1-(trifluoromethyl)cyclopropyl); für einem 4-gliedrigen heterocyclischem Ring enthaltend ein Heteroatom ausgewählt aus einer Gruppe bestehend aus N, O oder S wie Thietan-3-yl steht.
- R⁷ und R⁹: jeweils für H stehen;
- R¹¹: jeweils für H steht;
- R¹: für H steht;
- R²: für H, Halogen oder C₁-C₄-alkyl steht, bevorzugt für H, Fluor, Chlor oder Methyl steht;
- R⁴: für H oder Halogen steht, bevorzugt für H, Fluor oder Chlor steht;
- R⁵: für H oder Halogen steht, bevorzugt für H, Fluor oder Chlor steht;
- B₅: für N oder C-R¹⁰ steht, bevorzugt für C-R¹⁰, worin
- R¹⁰: für Wasserstoff, C₁-C₃-Alkyl, C₁-C₃-Alkoxy, mit Halogen substituiertes C₁-C₃-Alkyl (bevorzugt perfluoriertes C₁-C₃-Alkyl (CF₃, C₂F₅ oder C₃F₇)), mit Halogen substituiertes C₁-C₃-Alkoxy (bevorzugt perfluoriertes C₁-C₃-Alkoxy (OCF₃, OC₂F₅ oder OC₃F₇)), C₁-C₃-Alkylsulfanyl, C₁-C₃-Alkylsulfinyl, C₁-C₃-Alkylsulfonyl Fluor, Brom oder Chlor steht;
- A₂: für N or C-R³ steht, bevorzugt für C-R³, worin
- R³: für H, Halogen, oder gegebenenfalls substituiertes C₁-C₄-alkyl steht, bevorzugt für H, Fluor, Chlor oder gegebenenfalls mit Halogen substituiertes C₁-C₂-alkyl steht, besonders bevorzugt für H oder mit Fluoro substituiertes Methyl wie z. B. Perfluormethyl steht;
- R⁶: für Wasserstoff, C₁-C₃-Alkyl, C₁-C₃-Alkoxy, mit Halogen substituiertes C₁-C₃-Alkyl (bevorzugt perfluoriertes C₁-C₃-Alkyl (CF₃, C₂F₅ oder C₃F₇)), mit Halogen substituiertes C₁-C₃-Alkoxy (bevorzugt perfluoriertes C₁-C₃-Alkoxy (OCF₃, OC₂F₅ oder OC₃F₇)), C₁-C₃-Alkylsulfanyl, C₁-C₃-Alkylsulfinyl, C₁-C₃-Alkylsulfonyl Fluor, Brom oder Chlor steht, bevorzugt für Fluor, Brom, Chlor, C₁-C₂-alkyl, mit Halogen substituiertes C₁-C₂-alkyl (z. B. Perfluormethyl) oder gegebenenfalls mit Halogen substituiertes C₁-C₂-alkoxy, besonders bevorzugt für Fluor, Brom, Chlor, Methyl, Ethyl, fluoriertes Methyl oder fluoriertes Ethyl (besonders bevorzugt Perfluormethyl oder Perfluorethyl), fluoriertes Methoxy oder fluoriertes Ethoxy (besonders bevorzugt Perfluormethoxy) steht;
- R⁸: für Halogen oder gegebenenfalls mit Halogen substituiertes C₁-C₄-alkyl oder gegebenenfalls mit Halogen substituiertes C₁-C₄-alkoxy steht, bevorzugt für mit Halogen substituiertes C₁-C₃-alkyl oder mit Halogen substituiertes C₁-C₃-alkoxy, mehr bevorzugt für mit Halogen substituiertes C₁-C₃-alkyl wie fluoriertes C₁-C₃-alkyl (z. B. fluoriertes C₃-alkyl wie Perfluorpropyl) steht.

Eine bevorzugte Ausführungsform betrifft Verbindungen der Formel (Io) worin
- W: für O steht;
- Q: für gegebenenfalls substituiertes C₁-C₄-Alkyl oder gegebenenfalls substituiertes C₃-C₆-Cycloalkyl oder für einen gegebenenfalls mit ein, zwei oder drei V substituierten, ungesättigten 4-, 5- bzw. 6-gliedrigen, heterozyklischen Ring steht, wobei V unabhängig voneinander für Halogen, Cyano, Nitro, Oxo (=O), gegebenenfalls mit Halogen substituiertes C₁-C₆-Alkyl, C₁-C₄-Alkenyl, C₁-C₆-Alkoxy, C₁-C₆-Alkylsulfanyl, C₁-C₆-Alkylsulfinyl, C₁-C₆-Alkylsulfonyl steht;
bevorzugt für mit Halogen substituiertes C₁-C₃-Alkyl; mit Cyano, Hydroxy oder Carbonamid (-C(=O)N(R)₂, wobei R unabhängig voneinander für H oder C₁-C₃-Alkyl steht, substituiertes C₁-C₃-Alkyl steht; C₃-Cycloalkyl; mit Cyano, Halogen, Nitro oder halogenierten C₁-C₂-Alkyl substituierten C₃-Cycloalkyl; einem gegebenenfalls mit ein, zwei, oder drei V substituierten, ungesättigten 4-, 5- bzw. 6-gliedrigen, heterozyklischen Ring enthaltend ein oder zwei Heteroatome ausgewählt aus einer Gruppe bestehend aus N, O und S, wobei V unabhängig voneinander für Halogen, Cyano, Nitro, Oxo (=O), gegebenenfalls mit Halogen substituiertes C₁-C₆-Alkyl steht, steht;
besonders bevorzugt für fluoriertes C₁-C₃-Alkyl wie CF₃, CH₂CF₃ oder CH₂CH₂CF₃; für mit Carbonamid (-C(=O)N(R)₂, wobei R unabhängig voneinander für H, C₁-C₃-Alkyl oder mit Halogen substituiertes C₁-C₃-Alkyl steht) substituiertes C₁-C₃-Alkyl wie 2-oxo-2-(2,2,2-trifluoroethylamino)ethyl; für Cyclopropyl; für mit Cyano oder fluorierten C₁-C₂-Alkyl substituierte Cyclopropyl wie 1-(cyano)cyclopropyl oder 1-(trifluoromethyl)cyclopropyl); für einem 4-gliedrigen heterocyclischem Ring enthaltend ein Heteroatom ausgewählt aus einer Gruppe bestehend aus N, O oder S wie Thietan-3-yl steht.
- R⁷ und R⁹: jeweils für H stehen;
- R¹¹: jeweils für H steht;
- R¹: für Methyl steht;
- R²: für H, Halogen oder C₁-C₄-alkyl steht, bevorzugt für H, Fluor, Chlor oder Methyl steht;
- R⁴: für H oder Halogen steht, bevorzugt für H, Fluor oder Chlor steht;
- R⁵: für H oder Halogen steht, bevorzugt für H, Fluor oder Chlor steht;
- B₅: für N oder C-R¹⁰ steht, bevorzugt für C-R¹⁰, worin
- R¹⁰: für Wasserstoff, C₁-C₃-Alkyl, C₁-C₃-Alkoxy, mit Halogen substituiertes C₁-C₃-Alkyl (bevorzugt perfluoriertes C₁-C₃-Alkyl (CF₃, C₂F₅ oder C₃F₇)), mit Halogen substituiertes C₁-C₃-Alkoxy (bevorzugt perfluoriertes C₁-C₃-Alkoxy (OCF₃, OC₂F₅ oder OC₃F₇)), C₁-C₃-Alkylsulfanyl, C₁-C₃-Alkylsulfinyl, C₁-C₃-Alkylsulfonyl Fluor, Brom oder Chlor steht;
- A₂: für N or C-R³ steht, bevorzugt für C-R³, worin
- R³: für H, Halogen, oder gegebenenfalls substituiertes C₁-C₄-alkyl steht, bevorzugt für H, Fluor, Chlor oder gegebenenfalls mit Halogen substituiertes C₁-C₂-alkyl steht, besonders bevorzugt für H oder mit Fluoro substituiertes Methyl wie z. B. Perfluormethyl steht;
- R⁶: für Wasserstoff, C₁-C₃-Alkyl, C₁-C₃-Alkoxy, mit Halogen substituiertes C₁-C₃-Alkyl (bevorzugt perfluoriertes C₁-C₃-Alkyl (CF₃, C₂F₅ oder C₃F₇)), mit Halogen substituiertes C₁-C₃-Alkoxy (bevorzugt perfluoriertes C₁-C₃-Alkoxy (OCF₃, OC₂F₅ oder OC₃F₇)), C₁-C₃-Alkylsulfanyl, C₁-C₃-Alkylsulfinyl, C₁-C₃-Alkylsulfonyl Fluor, Brom oder Chlor steht, bevorzugt für Fluor, Brom, Chlor, C₁-C₂-alkyl, mit Halogen substituiertes C₁-C₂-alkyl (z. B. Perfluormethyl) oder gegebenenfalls mit Halogen substituiertes C₁-C₂-alkoxy, besonders bevorzugt für Fluor, Brom, Chlor, Methyl, Ethyl, fluoriertes Methyl oder fluoriertes Ethyl (besonders bevorzugt Perfluormethyl oder Perfluorethyl), fluoriertes Methoxy oder fluoriertes Ethoxy (besonders bevorzugt Perfluormethoxy) steht;
- R⁸: für Halogen oder gegebenenfalls mit Halogen substituiertes C₁-C₄-alkyl oder gegebenenfalls mit Halogen substituiertes C₁-C₄-alkoxy steht, bevorzugt für mit Halogen substituiertes C₁-C₃-alkyl oder mit Halogen substituiertes C₁-C₃-alkoxy, mehr bevorzugt für mit Halogen substituiertes C₁-C₃-alkyl wie fluoriertes C₁-C₃-alkyl (z. B. fluoriertes C₃-alkyl wie Perfluorpropyl) steht.

Beispielhaft für Verbindungen der Formel (I) seien folgende Strukturen genannt: 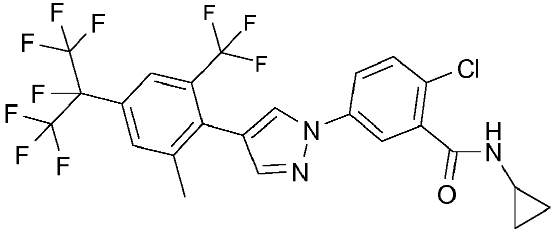 2-Chlor-N-cyclopropyl-5-[1-[4-(1,1,1,2,3,3,3-heptafluorpropan-2-yl)-2-methyl-6-(trifluormethyl)phenyl]-1H-pyrazol-4-yl]benzamid, 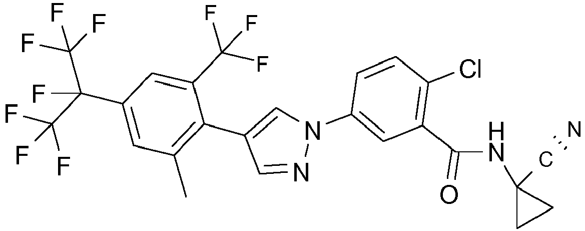 2-Chlor-N-(1-cyancyclopropyl-5-[1-[4-(1,1,1,2,3,3,3-heptafluorpropan-2-yl)-2-methyl-6-(trifluormethyl)phenyl]-1H-pyrazol-4-yl]benzamid, 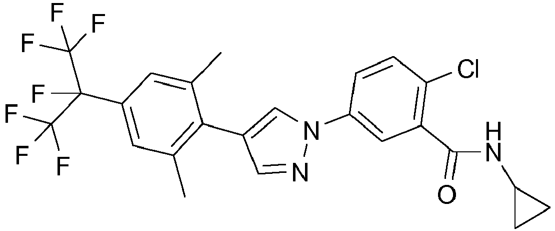 2-Chlor-N-cyclopropyl-5-[4-[2,6-dimethyl-4-[1,2,2,2-tetrafluor-1-(trifluormethyl)ethyl]phenyl]pyrazol-1-yl]benzamid, 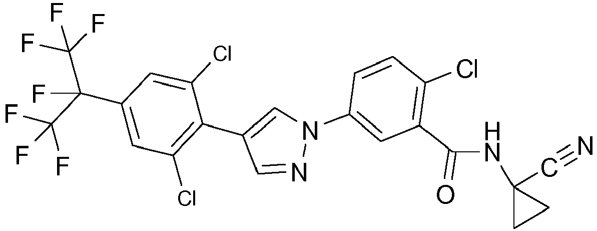 2-Chlor-N-(1-cyanocyclopropyl)-5-[4-[2,6-dichlor-4-[1,2,2,2-tetrafluor-1-(trifluormethyl)ethyl]phenyl]pyrazol-1-yl]benzamid, 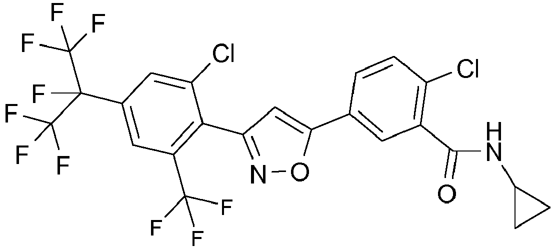 2-Chlor-5-[3-[2-chlor-4-[1,2,2,2-tetrafluor-1-(trifluormethyl)ethyl]-6-(trifluormethyl)phenyl]isoxazol-5-yl]-N-cyclopropyl-benzamid, 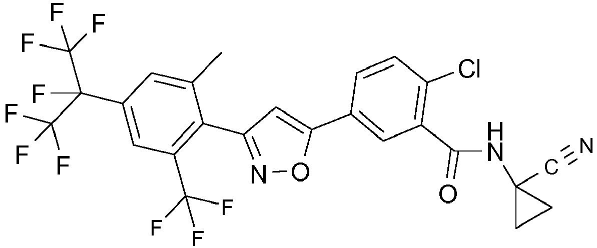 2-Chlor-N-(1-cyanocyclopropyl)-5-[3-[2-methyl-4-[1,2,2,2-tetrafluor-1-(trifluormethyl)ethyl]-6-(trifluormethyl)phenyl]isoxazol-5-yl]benzamid,
Weitere erfindungsgemäße Verbindungen sind 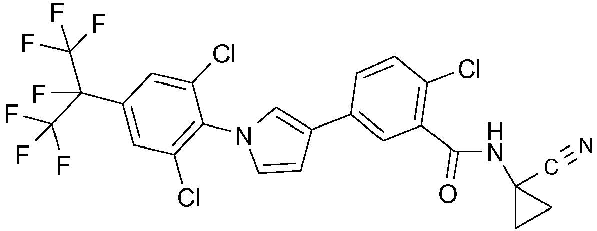 2-Chlor-N-(1-cyanocyclopropyl)-5-[1-[2,6-dichlor-4-[1,2,2,2-tetrafluor-1-(trifluormethyl)ethyl]phenyl]pyrrol-3-yl]benzamid, 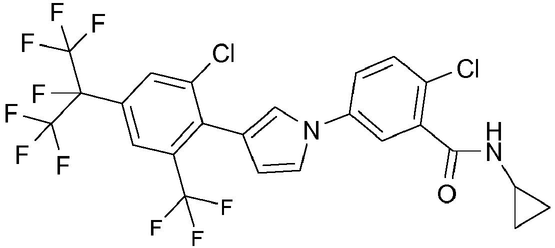 2-Chlor-5-[3-[2-chlor-4-[1,2,2,2-tetrafluor-1-(trifluormethyl)ethyl]-6-(trifluormethyl)phenyl]pyrrol-1-yl]-N-cyclopropyl-benzamid.

### R1 Methyl

Eine bevorzugte Ausführungsform bezieht sich auf Verbindungen der Formel (I), in der R¹ für Methyl steht und alle anderen Parameter wie in Paragraph [0078] definiert sind.

### T3 - Methyl

Eine weitere bevorzugte Ausführungsform bezieht sich auf Verbindungen der Formel (I) in der R¹ für Methyl steht, T für T3 steht, R¹¹ in T3 für H steht, W für O steht und alle anderen Parameter wie in Paragraph [0078] und Paragraph [0105] definiert sind.

Eine weitere bevorzugte Ausführungsform bezieht sich auf Verbindungen der Formel (I) in der R¹ für Methyl steht, T für T3 steht, R¹¹ in T3 für H steht, W für O steht, A₁ für CR², A₂ für CR³ oder N, A₃ für CR⁴, A₄ für CR⁵, B₁ für CR⁶, B₂ für CR⁷, B₃ für CR⁸, B₄ für CR⁹, B₅ für CR¹⁰ steht und alle anderen Parameter wie in Paragraph [0078] und Paragraph [0105] definiert sind.

Eine weitere bevorzugte Ausführungsform bezieht sich auf Verbindungen der Formel (I) in der R¹ für Methyl steht, T für T3 steht, R¹¹ in T3 für H steht, W für O steht, A₁ für CH, A₂ für CH oder N, A₃ für CR⁴, A₄ für CH, B₁ für CR⁶, B₂ für CH, B₃ für CR⁸, B₄ für CH, B₅ für CR¹⁰ steht wobei R⁶ und R¹⁰ jeweils für einen Substituenten ausgewählt aus Halogen (bevorzugt Chlor, Brom oder Fluor), C₁-C₃-alkyl, mit Halogen substituiertes C₁-C₃-alkyl, C₁-C₃-alkoxy oder mit Halogen substituiertes C₁-C₃-alkoxy und alle anderen Parameter wie in Paragraph [0078] und Paragraph [0105] definiert sind.

Eine weitere bevorzugte Ausführungsform bezieht sich auf Verbindungen der Formel (I) in der R¹ für Methyl steht, T für T3 steht, R¹¹ in T3 für H steht, W für O steht, A₁ für CH, A₂ für CH oder N, A₃ für CR⁴, A₄ für CH, B₁ für CR⁶, B₂ für CH, B₃ für CR⁸, B₄ für CH, B₅ für CR¹⁰ steht wobei R⁶ und R¹⁰ jeweils für einen Substituenten ausgewählt aus Halogen (bevorzugt Chlor, Brom oder Fluor), C₁-C₃-alkyl, mit Halogen substituiertes C₁-C₃-alkyl, C₁-C₃-alkoxy oder mit Halogen substituiertes C₁-C₃-alkoxy und alle anderen Parameter wie in Paragraph [0078] und Paragraph [0105] definiert sind.

### T2 - Methyl

Eine weitere bevorzugte Ausführungsform bezieht sich auf Verbindungen der Formel (I) in der R¹ für Methyl steht, T für T2 steht, R¹¹ in T2 für H steht, W für O steht und alle anderen Parameter wie in Paragraph [0078] und Paragraph [0105] definiert sind.

Eine weitere bevorzugte Ausführungsform bezieht sich auf Verbindungen der Formel (I) in der R¹ für Methyl steht, T für T2 steht, R¹¹ in T2 für H steht, W für O steht, A₁ für CR², A₂ für CR³ oder N, A₃ für CR⁴, A₄ für CR⁵, B₁ für CR⁶, B₂ für CR⁷, B₃ für CR⁸, B₄ für CR⁹, B₅ für CR¹⁰ steht und alle anderen Parameter wie in Paragraph [0078] und Paragraph [0105] definiert sind.

Eine weitere bevorzugte Ausführungsform bezieht sich auf Verbindungen der Formel (I) in der R¹ für Methyl steht, T für T2 steht, R¹¹ in T2 für H steht, W für O steht, A₁ für CH, A₂ für CH oder N, A₃ für CR⁴, A₄ für CH, B₁ für CR⁶, B₂ für CH, B₃ für CR⁸, B₄ für CH, B₅ für CR¹⁰ steht wobei R⁶ und R¹⁰ jeweils für einen Substituenten ausgewählt aus Halogen (bevorzugt Chlor, Brom oder Fluor), C₁-C₃-alkyl, mit Halogen substituiertes C₁-C₃-alkyl, C₁-C₃-alkoxy oder mit Halogen substituiertes C₁-C₃-alkoxy und alle anderen Parameter wie in Paragraph [0078] und Paragraph [0105] definiert sind.

Eine weitere bevorzugte Ausführungsform bezieht sich auf Verbindungen der Formel (I) in der R¹ für Methyl steht, T für T2 steht, R¹¹ in T2 für H steht, W für O steht, A₁ für CH, A₂ für CH oder N, A₃ für CR⁴, A₄ für CH, B₁ für CR⁶, B₂ für CH, B₃ für CR⁸, B₄ für CH, B₅ für CR¹⁰ steht wobei R⁶ und R¹⁰ jeweils für einen Substituenten ausgewählt aus Halogen (bevorzugt Chlor, Brom oder Fluor), C₁-C₃-alkyl, mit Halogen substituiertes C₁-C₃-alkyl, C₁-C₃-alkoxy oder mit Halogen substituiertes C₁-C₃-alkoxy und alle anderen Parameter wie in Paragraph [0078] und Paragraph [0105] definiert sind.

### T4 - Methyl

Eine weitere bevorzugte Ausführungsform bezieht sich auf Verbindungen der Formel (I) in der R¹ für Methyl steht, T für T4 steht, R¹¹ in T4 für H steht, W für O steht und alle anderen Parameter wie in Paragraph [0078] und Paragraph [0105] definiert sind.

Eine weitere bevorzugte Ausführungsform bezieht sich auf Verbindungen der Formel (I) in der R¹ für Methyl steht, T für T4 steht, R¹¹ in T4 für H steht, W für O steht, A₁ für CR², A₂ für CR³ oder N, A₃ für CR⁴, A₄ für CR⁵, B₁ für CR⁶, B₂ für CR⁷, B₃ für CR⁸, B₄ für CR⁹, B₅ für CR¹⁰ steht und alle anderen Parameter wie in Paragraph [0078] und Paragraph [0105] definiert sind.

Eine weitere bevorzugte Ausführungsform bezieht sich auf Verbindungen der Formel (I) in der R¹ für Methyl steht, T für T4 steht, R¹¹ in T4 für H steht, W für O steht, A₁ für CH, A₂ für CH oder N, A₃ für CR⁴, A₄ für CH, B₁ für CR⁶, B₂ für CH, B₃ für CR⁸, B₄ für CH, B₅ für CR¹⁰ steht wobei R⁶ und R¹⁰ jeweils für einen Substituenten ausgewählt aus Halogen (bevorzugt Chlor, Brom oder Fluor), C₁-C₃-alkyl, mit Halogen substituiertes C₁-C₃-alkyl, C₁-C₃-alkoxy oder mit Halogen substituiertes C₁-C₃-alkoxy und alle anderen Parameter wie in Paragraph [0078] und Paragraph [0105] definiert sind.

Eine weitere bevorzugte Ausführungsform bezieht sich auf Verbindungen der Formel (I) in der R¹ für Methyl steht, T für T4 steht, R¹¹ in T4 für H steht, W für O steht, A₁ für CH, A₂ für CH oder N, A₃ für CR⁴, A₄ für CH, B₁ für CR⁶, B₂ für CH, B₃ für CR⁸, B₄ für CH, B₅ für CR¹⁰ steht wobei R⁶ und R¹⁰ jeweils für einen Substituenten ausgewählt aus Halogen (bevorzugt Chlor, Brom oder Fluor), C₁-C₃-alkyl, mit Halogen substituiertes C₁-C₃-alkyl, C₁-C₃-alkoxy oder mit Halogen substituiertes C₁-C₃-alkoxy und alle anderen Parameter wie in Paragraph [0078] und Paragraph [0105] definiert sind.

### T22 - Methyl

Eine weitere bevorzugte Ausführungsform bezieht sich auf Verbindungen der Formel (I) in der R¹ für Methyl steht, T für T22 steht, R¹¹ in T22 für H steht, W für O steht und alle anderen Parameter wie in Paragraph [0078] und Paragraph [0105] definiert sind.

Eine weitere bevorzugte Ausführungsform bezieht sich auf Verbindungen der Formel (I) in der R¹ für Methyl steht, T für T22 steht, R¹¹ in T22 für H steht, W für O steht, A₁ für CR², A₂ für CR³ oder N, A₃ für CR⁴, A₄ für CR⁵, B₁ für CR⁶, B₂ für CR⁷, B₃ für CR⁸, B₄ für CR⁹, B₅ für CR¹⁰ steht und alle anderen Parameter wie in Paragraph [0078] und Paragraph [0105] definiert sind.

Eine weitere bevorzugte Ausführungsform bezieht sich auf Verbindungen der Formel (I) in der R¹ für Methyl steht, T für T22 steht, R¹¹ in T22 für H steht, W für O steht, A₁ für CH, A₂ für CH oder N, A₃ für CR⁴, A₄ für CH, B₁ für CR⁶, B₂ für CH, B₃ für CR⁸, B₄ für CH, B₅ für CR¹⁰ steht wobei R⁶ und R¹⁰ jeweils für einen Substituenten ausgewählt aus Halogen (bevorzugt Chlor, Brom oder Fluor), C₁-C₃-alkyl, mit Halogen substituiertes C₁-C₃-alkyl, C₁-C₃-alkoxy oder mit Halogen substituiertes C₁-C₃-alkoxy und alle anderen Parameter wie in Paragraph [0078] und Paragraph [0105] definiert sind.

Eine weitere bevorzugte Ausführungsform bezieht sich auf Verbindungen der Formel (I) in der R¹ für Methyl steht, T für T22 steht, R¹¹ in T22 für H steht, W für O steht, A₁ für CH, A₂ für CH oder N, A₃ für CR⁴, A₄ für CH, B₁ für CR⁶, B₂ für CH, B₃ für CR⁸, B₄ für CH, B₅ für CR¹⁰ steht wobei R⁶ und R¹⁰ jeweils für einen Substituenten ausgewählt aus Halogen (bevorzugt Chlor, Brom oder Fluor), C₁-C₃-alkyl, mit Halogen substituiertes C₁-C₃-alkyl, C₁-C₃-alkoxy oder mit Halogen substituiertes C₁-C₃-alkoxy und alle anderen Parameter wie in Paragraph [0078] und Paragraph [0105] definiert sind.

### T23 - Methyl

Eine weitere bevorzugte Ausführungsform bezieht sich auf Verbindungen der Formel (I) in der R¹ für Methyl steht, T für T23 steht, R¹¹ in T23 für H steht, W für O steht und alle anderen Parameter wie in Paragraph [0078] und Paragraph [0106] ff. definiert sind.

Eine weitere bevorzugte Ausführungsform bezieht sich auf Verbindungen der Formel (I) in der R¹ für Methyl steht, T für T23 steht, R¹¹ in T23 für H steht, W für O steht, A₁ für CR², A₂ für CR³ oder N, A₃ für CR⁴, A₄ für CR⁵, B₁ für CR⁶, B₂ für CR⁷, B₃ für CR⁸, B₄ für CR⁹, B₅ für CR¹⁰ steht und alle anderen Parameter wie in Paragraph [0078] und Paragraph [0106] ff. definiert sind.

Eine weitere bevorzugte Ausführungsform bezieht sich auf Verbindungen der Formel (I) in der R¹ für Methyl steht, T für T23 steht, R¹¹ in T23 für H steht, W für O steht, A₁ für CH, A₂ für CH oder N, A₃ für CR⁴, A₄ für CH, B₁ für CR⁶, B₂ für CH, B₃ für CR⁸, B₄ für CH, B₅ für CR¹⁰ steht wobei R⁶ und R¹⁰ jeweils für einen Substituenten ausgewählt aus Halogen (bevorzugt Chlor, Brom oder Fluor), C₁-C₃-alkyl, mit Halogen substituiertes C₁-C₃-alkyl, C₁-C₃-alkoxy oder mit Halogen substituiertes C₁-C₃-alkoxy und alle anderen Parameter wie in Paragraph [0078] und Paragraph [0106] ff. definiert sind.

Eine weitere bevorzugte Ausführungsform bezieht sich auf Verbindungen der Formel (I) in der R¹ für Methyl steht, T für T23 steht, R¹¹ in T23 für H steht, W für O steht, A₁ für CH, A₂ für CH oder N, A₃ für CR⁴, A₄ für CH, B₁ für CR⁶, B₂ für CH, B₃ für CR⁸, B₄ für CH, B₅ für CR¹⁰ steht wobei R⁶ und R¹⁰ jeweils für einen Substituenten ausgewählt aus Halogen (bevorzugt Chlor, Brom oder Fluor), C₁-C₃-alkyl, mit Halogen substituiertes C₁-C₃-alkyl, C₁-C₃-alkoxy oder mit Halogen substituiertes C₁-C₃-alkoxy und alle anderen Parameter wie in Paragraph [0078] und Paragraph [0106] ff. definiert sind.

### T3 - H

Eine bevorzugte Ausführungsform bezieht sich auf Verbindungen der Formel (I), in der R¹ für Wasserstoff (H) steht und alle anderen Parameter wie in Paragraph [0106] ff. definiert sind.

Eine weitere bevorzugte Ausführungsform bezieht sich auf Verbindungen der Formel (I) in der R¹ für H steht, T für T3 steht, R¹¹ in T3 für H steht, W für O steht und alle anderen Parameter wie in Paragraph [0078] und Paragraph [0106] ff. definiert sind.

Eine weitere bevorzugte Ausführungsform bezieht sich auf Verbindungen der Formel (I) in der R¹ für H steht, T für T3 steht, R¹¹ in T3 für H steht, W für O steht, A₁ für CR², A₂ für CR³ oder N, A₃ für CR⁴, A₄ für CR⁵, B₁ für CR⁶, B₂ für CR⁷, B₃ für CR⁸, B₄ für CR⁹, B₅ für CR¹⁰ steht und alle anderen Parameter wie in Paragraph [0078] und Paragraph [0106] ff. definiert sind.

Eine weitere bevorzugte Ausführungsform bezieht sich auf Verbindungen der Formel (I) in der R¹ für H steht, T für T3 steht, R¹¹ in T3 für H steht, W für O steht, A₁ für CH, A₂ für CH oder N, A₃ für CR⁴, A₄ für CH, B₁ für CR⁶, B₂ für CH, B₃ für CR⁸, B₄ für CH, B₅ für CR¹⁰ steht wobei R⁶ und R¹⁰ jeweils für einen Substituenten ausgewählt aus Halogen (bevorzugt Chlor, Brom oder Fluor), C₁-C₃-alkyl, mit Halogen substituiertes C₁-C₃-alkyl, C₁-C₃-alkoxy oder mit Halogen substituiertes C₁-C₃-alkoxy und alle anderen Parameter wie in Paragraph [0078] und Paragraph [0106] ff. definiert sind.

Eine weitere bevorzugte Ausführungsform bezieht sich auf Verbindungen der Formel (I) in der R¹ für H steht, T für T3 steht, R¹¹ in T3 für H steht, W für O steht, A₁ für CH, A₂ für CH oder N, A₃ für CR⁴, A₄ für CH, B₁ für CR⁶, B₂ für CH, B₃ für CR⁸, B₄ für CH, B₅ für CR¹⁰ steht wobei R⁶ und R¹⁰ jeweils für einen Substituenten ausgewählt aus Halogen (bevorzugt Chlor, Brom oder Fluor), C₁-C₃-alkyl, mit Halogen substituiertes C₁-C₃-alkyl, C₁-C₃-alkoxy oder mit Halogen substituiertes C₁-C₃-alkoxy und alle anderen Parameter wie in Paragraph [0078] und Paragraph [0106] ff. definiert sind.

### T2 - H

Eine weitere bevorzugte Ausführungsform bezieht sich auf Verbindungen der Formel (I) in der R¹ für H steht, T für T2 steht, R¹¹ in T2 für H steht, W für O steht und alle anderen Parameter wie in Paragraph [0078] und Paragraph [0106] ff. definiert sind.

Eine weitere bevorzugte Ausführungsform bezieht sich auf Verbindungen der Formel (I) in der R¹ für H steht, T für T2 steht, R¹¹ in T2 für H steht, W für O steht, A₁ für CR², A₂ für CR³ oder N, A₃ für CR⁴, A₄ für CR⁵, B₁ für CR⁶, B₂ für CR⁷, B₃ für CR⁸, B₄ für CR⁹, B₅ für CR¹⁰ steht und alle anderen Parameter wie in Paragraph [0078] und Paragraph [0106] ff. definiert sind.

Eine weitere bevorzugte Ausführungsform bezieht sich auf Verbindungen der Formel (I) in der R¹ für H steht, T für T2 steht, R¹¹ in T2 für H steht, W für O steht, A₁ für CH, A₂ für CH oder N, A₃ für CR⁴, A₄ für CH, B₁ für CR⁶, B₂ für CH, B₃ für CR⁸, B₄ für CH, B₅ für CR¹⁰ steht wobei R⁶ und R¹⁰ jeweils für einen Substituenten ausgewählt aus Halogen (bevorzugt Chlor, Brom oder Fluor), C₁-C₃-alkyl, mit Halogen substituiertes C₁-C₃-alkyl, C₁-C₃-alkoxy oder mit Halogen substituiertes C₁-C₃-alkoxy und alle anderen Parameter wie in Paragraph [0078] und Paragraph [0106] ff. definiert sind.

Eine weitere bevorzugte Ausführungsform bezieht sich auf Verbindungen der Formel (I) in der R¹ für H steht, T für T2 steht, R¹¹ in T2 für H steht, W für O steht, A₁ für CH, A₂ für CH oder N, A₃ für CR⁴, A₄ für CH, B₁ für CR⁶, B₂ für CH, B₃ für CR⁸, B₄ für CH, B₅ für CR¹⁰ steht wobei R⁶ und R¹⁰ jeweils für einen Substituenten ausgewählt aus Halogen (bevorzugt Chlor, Brom oder Fluor), C₁-C₃-alkyl, mit Halogen substituiertes C₁-C₃-alkyl, C₁-C₃-alkoxy oder mit Halogen substituiertes C₁-C₃-alkoxy und alle anderen Parameter wie in Paragraph [0078] und Paragraph [0106] ff. definiert sind.

### T4 - H

Eine weitere bevorzugte Ausführungsform bezieht sich auf Verbindungen der Formel (I) in der R¹ für H steht, T für T4 steht, R¹¹ in T4 für H steht, W für O steht und alle anderen Parameter wie in Paragraph [0078] und Paragraph [0106] ff. definiert sind.

Eine weitere bevorzugte Ausführungsform bezieht sich auf Verbindungen der Formel (I) in der R¹ für H steht, T für T4 steht, R¹¹ in T4 für H steht, W für O steht, A₁ für CR², A₂ für CR³ oder N, A₃ für CR⁴, A₄ für CR⁵, B₁ für CR⁶, B₂ für CR⁷, B₃ für CR⁸, B₄ für CR⁹, B₅ für CR¹⁰ steht und alle anderen Parameter wie in Paragraph [0078] und Paragraph [0106] ff. definiert sind.

Eine weitere bevorzugte Ausführungsform bezieht sich auf Verbindungen der Formel (I) in der R¹ für H steht, T für T4 steht, R¹¹ in T4 für H steht, W für O steht, A₁ für CH, A₂ für CH oder N, A₃ für CR⁴, A₄ für CH, B₁ für CR⁶, B₂ für CH, B₃ für CR⁸, B₄ für CH, B₅ für CR¹⁰ steht wobei R⁶ und R¹⁰ jeweils für einen Substituenten ausgewählt aus Halogen (bevorzugt Chlor, Brom oder Fluor), C₁-C₃-alkyl, mit Halogen substituiertes C₁-C₃-alkyl, C₁-C₃-alkoxy oder mit Halogen substituiertes C₁-C₃-alkoxy und alle anderen Parameter wie in Paragraph [0078] und Paragraph [0106] ff. definiert sind.

Eine weitere bevorzugte Ausführungsform bezieht sich auf Verbindungen der Formel (I) in der R¹ für H steht, T für T4 steht, R¹¹ in T4 für H steht, W für O steht, A₁ für CH, A₂ für CH oder N, A₃ für CR⁴, A₄ für CH, B₁ für CR⁶, B₂ für CH, B₃ für CR⁸, B₄ für CH, B₅ für CR¹⁰ steht wobei R⁶ und R¹⁰ jeweils für einen Substituenten ausgewählt aus Halogen (bevorzugt Chlor, Brom oder Fluor), C₁-C₃-alkyl, mit Halogen substituiertes C₁-C₃-alkyl, C₁-C₃-alkoxy oder mit Halogen substituiertes C₁-C₃-alkoxy und alle anderen Parameter wie in Paragraph [0078] und Paragraph [0106] ff. definiert sind.

### T22 - H

Eine weitere bevorzugte Ausführungsform bezieht sich auf Verbindungen der Formel (I) in der R¹ für H steht, T für T22 steht, R¹¹ in T22 für H steht, W für O steht und alle anderen Parameter wie in Paragraph [0078] und Paragraph [0106] ff. definiert sind.

Eine weitere bevorzugte Ausführungsform bezieht sich auf Verbindungen der Formel (I) in der R¹ für H steht, T für T22 steht, R¹¹ in T22 für H steht, W für O steht, A₁ für CR², A₂ für CR³ oder N, A₃ für CR⁴, A₄ für CR⁵, B₁ für CR⁶, B₂ für CR⁷, B₃ für CR⁸, B₄ für CR⁹, B₅ für CR¹⁰ steht und alle anderen Parameter wie in Paragraph [0078] und Paragraph [0106] ff. definiert sind.

Eine weitere bevorzugte Ausführungsform bezieht sich auf Verbindungen der Formel (I) in der R¹ für H steht, T für T22 steht, R¹¹ in T22 für H steht, W für O steht, A₁ für CH, A₂ für CH oder N, A₃ für CR⁴, A₄ für CH, B₁ für CR⁶, B₂ für CH, B₃ für CR⁸, B₄ für CH, B₅ für CR¹⁰ steht wobei R⁶ und R¹⁰ jeweils für einen Substituenten ausgewählt aus Halogen (bevorzugt Chlor, Brom oder Fluor), C₁-C₃-alkyl, mit Halogen substituiertes C₁-C₃-alkyl, C₁-C₃-alkoxy oder mit Halogen substituiertes C₁-C₃-alkoxy und alle anderen Parameter wie in Paragraph [0078] und Paragraph [0106] ff. definiert sind.

Eine weitere bevorzugte Ausführungsform bezieht sich auf Verbindungen der Formel (I) in der R¹ für H steht, T für T22 steht, R¹¹ in T22 für H steht, W für O steht, A₁ für CH, A₂ für CH oder N, A₃ für CR⁴, A₄ für CH, B₁ für CR⁶, B₂ für CH, B₃ für CR⁸, B₄ für CH, B₅ für CR¹⁰ steht wobei R⁶ und R¹⁰ jeweils für einen Substituenten ausgewählt aus Halogen (bevorzugt Chlor, Brom oder Fluor), C₁-C₃-alkyl, mit Halogen substituiertes C₁-C₃-alkyl, C₁-C₃-alkoxy oder mit Halogen substituiertes C₁-C₃-alkoxy und alle anderen Parameter wie in Paragraph [0078] und Paragraph [0106] ff. definiert sind.

### T23 - H

Eine weitere bevorzugte Ausführungsform bezieht sich auf Verbindungen der Formel (I) in der R¹ für H steht, T für T23 steht, R¹¹ in T23 für H steht, W für O steht und alle anderen Parameter wie in Paragraph [0078] und Paragraph [0106] ff definiert sind.

Eine weitere bevorzugte Ausführungsform bezieht sich auf Verbindungen der Formel (I) in der R¹ für H steht, T für T23 steht, R¹¹ in T23 für H steht, W für O steht, A₁ für CR², A₂ für CR³ oder N, A₃ für CR⁴, A₄ für CR⁵, B₁ für CR⁶, B₂ für CR⁷, B₃ für CR⁸, B₄ für CR⁹, B₅ für CR¹⁰ steht und alle anderen Parameter wie in Paragraph [0078] und Paragraph [0106] ff. definiert sind.

Eine weitere bevorzugte Ausführungsform bezieht sich auf Verbindungen der Formel (I) in der R¹ für H steht, T für T23 steht, R¹¹ in T23 für H steht, W für O steht, A₁ für CH, A₂ für CH oder N, A₃ für CR⁴, A₄ für CH, B₁ für CR⁶, B₂ für CH, B₃ für CR⁸, B₄ für CH, B₅ für CR¹⁰ steht wobei R⁶ und R¹⁰ jeweils für einen Substituenten ausgewählt aus Halogen (bevorzugt Chlor, Brom oder Fluor), C₁-C₃-alkyl, mit Halogen substituiertes C₁-C₃-alkyl, C₁-C₃-alkoxy oder mit Halogen substituiertes C₁-C₃-alkoxy und alle anderen Parameter wie in Paragraph [0078] und Paragraph [0106] ff. definiert sind.

Eine weitere bevorzugte Ausführungsform bezieht sich auf Verbindungen der Formel (I) in der R¹ für H steht, T für T23 steht, R¹¹ in T23 für H steht, W für O steht, A₁ für CH, A₂ für CH oder N, A₃ für CR⁴, A₄ für CH, B₁ für CR⁶, B₂ für CH, B₃ für CR⁸, B₄ für CH, B₅ für CR¹⁰ steht wobei R⁶ und R¹⁰ jeweils für einen Substituenten ausgewählt aus Halogen (bevorzugt Chlor, Brom oder Fluor), C₁-C₃-alkyl, mit Halogen substituiertes C₁-C₃-alkyl, C₁-C₃-alkoxy oder mit Halogen substituiertes C₁-C₃-alkoxy und alle anderen Parameter wie in Paragraph [0078] und Paragraph [0106] ff. definiert sind.

Eine weitere bevorzugte Ausführungsform bezieht sich auf Verbindungen der Formel (I), in der B₁ für C-R⁶ steht und R⁶ für Halogen (bevorzugt für Chlor oder Fluor), C₁-C₄-Alkyl, C₁-C₄-Alkoxy, C₁-C₄-Halogenalkyl (bevorzugt perfluoriertes C₁-C₄-Alkyl), C₁-C₄-Halogenalkoxy (bevorzugt perfluoriertes C₁-C₄-Alkoxy), C₁-C₄-Alkylsulfanyl oder C₁-C₄-Alkylsulfonyl steht.

Erfindungsgemäß geeignete Salze der erfindungsgemäßen Verbindungen, beispielsweise Salze mit Basen oder Säureadditionssalze, sind alle üblichen nicht toxischen Salze, vorzugsweise landwirtschaftlich und/oder physiologisch annehmbare Salze. Bevorzugt werden Salze mit anorganischen Basen, wie beispielsweise Alkalimetallsalze (z.B. Natrium-, Kalium- oder Cäsiumsalze), Erdalkalimetallsalze (z.B. Calcium- oder Magnesiumsalze), Ammoniumsalze oder Salze mit organischen Basen, insbesondere mit organischen Aminen, wie beispielsweise Triethylammonium-, Dicyclohexylammonium-, *N,N'*-Dibenzylethylendiammonium-, Pyridinium-, Picolinium- oder Ethanolammoniumsalze, Salze mit anorganischen Säuren (z.B. Hydrochloride, Hydrobromide, Dihydrosulfate, Trihydrosulfate, oder Phosphate), Salze mit organischen Carbonsäuren oder organischen Sulfosäuren (z.b. Formiate, Acetate, Trifluoracetate, Maleate, Tartrate, Methansulfonate, Benzolsulfonate oder 4-Toluolsulfonate). Bekannterweise können t-Amine, wie beispielsweise manche der erfindungsgemäßen Verbindungen, *N*-Oxide bilden, welche ebenfalls erfindungsgemäße Salze darstellen.

Die Verbindungen der Formel (Ia") können in Abhängigkeit von der Art der Substituenten als geometrische und/oder als optisch aktive Isomere oder entsprechende Isomerengemische in unterschiedlicher Zusammensetzung vorliegen. Diese Stereoisomere sind beispielsweise Enantiomere, Diastereomere, Atropisomere oder geometrische Isomere. Die Erfindung umfasst somit reine Stereoisomere als auch beliebige Gemische dieser Isomere.

Die Erfindung betrifft auch Verfahren zur Bekämpfung von tierischen Schädlingen, bei dem man Verbindungen der Formel (Ia") auf tierische Schädlinge und/oder ihren Lebensraum einwirken lässt. Bevorzugt wird die Bekämpfung der tierischen Schädlinge in der Land- und Forstwirtschaft und im Materialschutz durchgeführt. Hierunter vorzugsweise ausgeschlossen sind Verfahren zur chirurgischen oder therapeutischen Behandlung des menschlichen oder tierischen Körpers und Diagnostizierverfahren, die am menschlichen oder tierischen Körper vorgenommen werden.

Die Erfindung betrifft ferner die Verwendung der Verbindungen der Formel (Ia") als Schädlingsbekämpfungsmittel, insbesondere Pflanzenschutzmittel.

Im Rahmen der vorliegenden Anmeldung umfasst der Begriff Schädlingsbekämpfungsmittel immer auch den Begriff Pflanzenschutzmittel.

Die Verbindungen der Formel (Ia") eignen sich bei guter Pflanzenverträglichkeit, günstiger Warmblütertoxizität und guter Umweltverträglichkeit zum Schutz von Pflanzen und Pflanzenorganen vor biotischen und abiotischen Stressfaktoren, zur Steigerung der Ernteerträge, Verbesserung der Qualität des Erntegutes und zur Bekämpfung von tierischen Schädlingen, insbesondere Insekten, Spinnentieren, Helminthen, Nematoden und Mollusken, die in der Landwirtschaft, im Gartenbau, bei der Tierzucht, in Aquakulturen, in Forsten, in Gärten und Freizeiteinrichtungen, im Vorrats- und Materialschutz sowie auf dem Hygienesektor vorkommen. Sie können vorzugsweise als Schädlingsbekämpfungsmittel eingesetzt werden. Sie sind gegen normal sensible und resistente Arten sowie gegen alle oder einzelne Entwicklungsstadien wirksam. Zu den oben erwähnten Schädlingen gehören:
Schädlinge aus dem Stamm der Arthropoda, insbesondere aus der Klasse der Arachnida z.B. Acarus spp., z.B. Acarus siro, Aceria kuko, Aceria sheldoni, Aculops spp., Aculus spp., z.B. Aculus fockeui, Aculus schlechtendali, Amblyomma spp., Amphitetranychus viennensis, Argas spp., Boophilus spp., Brevipalpus spp., z.B. Brevipalpus phoenicis, Bryobia graminum, Bryobia praetiosa, Centruroides spp., Chorioptes spp., Dermanyssus gallinae, Dermatophagoides pteronyssinus, Dermatophagoides farinae, Dermacentor spp., Eotetranychus spp., z.B. Eotetranychus hicoriae, Epitrimerus pyri, Eutetranychus spp., z.B. Eutetranychus banksi, Eriophyes spp., z.B. Eriophyes pyri, Glycyphagus domesticus, Halotydeus destructor, Hemitarsonemus spp., z.B. Hemitarsonemus latus (=Polyphagotarsonemus latus), Hyalomma spp., Ixodes spp., Latrodectus spp., Loxosceles spp., Neutrombicula autumnalis, Nuphersa spp., Oligonychus spp., z.B. Oligonychus coniferarum, Oligonychus ilicis, Oligonychus indicus, Oligonychus mangiferus, Oligonychus pratensis, Oligonychus punicae, Oligonychus yothersi, Ornithodorus spp., Ornithonyssus spp., Panonychus spp., z.B. Panonychus citri (=Metatetranychus citri), Panonychus ulmi (=Metatetranychus ulmi), Phyllocoptruta oleivora, Platytetranychus multidigituli, Polyphagotarsonemus latus, Psoroptes spp., Rhipicephalus spp., Rhizoglyphus spp., Sarcoptes spp., Scorpio maurus, Steneotarsonemus spp., Steneotarsonemus spinki, Tarsonemus spp., z.B. Tarsonemus confusus, Tarsonemus pallidus, Tetranychus spp., z.B. Tetranychus canadensis, Tetranychus cinnabarinus, Tetranychus turkestani, Tetranychus urticae, Trombicula alfreddugesi, Vaejovis spp., Vasates lycopersici;
aus der Klasse der Chilopoda z.B. Geophilus spp., Scutigera spp.;
aus der Ordnung oder der Klasse der Collembola z.B. Onychiurus armatus; Sminthurus viridis;
aus der Klasse der Diplopoda z.B. Blaniulus guttulatus;
aus der Klasse der Insecta, z.B. aus der Ordnung der Blattodea z.B. Blatta orientalis, Blattella asahinai, Blattella germanica, Leucophaea maderae, Panchlora spp., Parcoblatta spp., Periplaneta spp., z.B. Periplaneta americana, Periplaneta australasiae, Supella longipalpa;
aus der Ordnung der Coleoptera z.B. Acalymma vittatum, Acanthoscelides obtectus, Adoretus spp., Agelastica alni, Agriotes spp., z.B. Agriotes linneatus, Agriotes mancus, Alphitobius diaperinus, Amphimallon solstitialis, Anobium punctatum, Anoplophora spp., Anthonomus spp., z.B. Anthonomus grandis, Anthrenus spp., Apion spp., Apogonia spp., Atomaria spp., z.B. Atomaria linearis, Attagenus spp., Baris caerulescens, Bruchidius obtectus, Bruchus spp., z.B. Bruchus pisorum, Bruchus rufimanus, Cassida spp., Cerotoma trifurcata, Ceutorrhynchus spp., z.B. Ceutorrhynchus assimilis, Ceutorrhynchus quadridens, Ceutorrhynchus rapae, Chaetocnema spp., z.B. Chaetocnema confinis, Chaetocnema denticulata, Chaetocnema ectypa, Cleonus mendicus, Conoderus spp., Cosmopolites spp., z.B. Cosmopolites sordidus, Costelytra zealandica, Ctenicera spp., Curculio spp., z.B. Curculio caryae, Curculio caryatrypes,Curculio obtusus, Curculio sayi, Cryptolestes ferrugineus, Cryptolestes pusillus, Cryptorhynchus lapathi, Cryptorhynchus mangiferae, Cylindrocopturus spp., Cylindrocopturus adspersus, Cylindrocopturus furnissi, Dermestes spp., Diabrotica spp., z.B. Diabrotica balteata, Diabrotica barberi, Diabrotica undecimpunctata howardi, Diabrotica undecimpunctata undecimpunctata, Diabrotica virgifera virgifera, Diabrotica virgifera zeae, Dichocrocis spp., Dicladispa armigera, Diloboderus spp., Epilachna spp., z.B. Epilachna borealis, Epilachna varivestis, Epitrix spp., z.B. Epitrix cucumeris, Epitrix fuscula, Epitrix hirtipennis, Epitrix subcrinita, Epitrix tuberis, Faustinus spp., Gibbium psylloides, Gnathocerus cornutus, Hellula undalis, Heteronychus arator, Heteronyx spp., Hylamorpha elegans, Hylotrupes bajulus, Hypera postica, Hypomeces squamosus, Hypothenemus spp., z.B. Hypothenemus hampei, Hypothenemus obscurus, Hypothenemus pubescens, Lachnosterna consanguinea, Lasioderma serricorne, Latheticus oryzae, Lathridius spp., Lema spp., Leptinotarsa decemlineata, Leucoptera spp., z.B. Leucoptera coffeella, Lissorhoptrus oryzophilus, Lixus spp., Luperomorpha xanthodera, Luperodes spp., Lyctus spp., Megascelis spp., Melanotus spp., z.B. Melanotus longulus oregonensis, Meligethes aeneus, Melolontha spp., z.B. Melolontha melolontha, Migdolus spp., Monochamus spp., Naupactus xanthographus, Necrobia spp., Niptus hololeucus, Oryctes rhinoceros, Oryzaephilus surinamensis, Oryzaphagus oryzae, Otiorhynchus spp., z.B. Otiorhynchus cribricollis, Otiorhynchus ligustici, Otiorhynchus ovatus, Otiorhynchus rugosostriarus, Otiorhynchus sulcatus, Oxycetonia jucunda, Phaedon cochleariae, Phyllophaga spp., Phyllophaga helleri, Phyllotreta spp., z.B. Phyllotreta armoraciae, Phyllotreta pusilla, Phyllotreta ramosa, Phyllotreta striolata, Popillia japonica, Premnotrypes spp., Prostephanus truncatus, Psylliodes spp., z.B. Psylliodes affinis, Psylliodes chrysocephala, Psylliodes punctulata, Ptinus spp., Rhizobius ventralis, Rhizopertha dominica, Sitophilus spp., z.B. Sitophilus granarius, Sitophilus linearis, Sitophilus oryzae, Sitophilus zeamais, Sphenophorus spp., Stegobium paniceum, Sternechus spp., z.B. Sternechus paludatus, Symphyletes spp., Tanymecus spp., z.B. Tanymecus dilaticollis, Tanymecus indicus, Tanymecus palliatus, Tenebrio molitor, Tenebrioides mauretanicus, Tribolium spp., z.B. Tribolium audax, Tribolium castaneum, Tribolium confusum, Trogoderma spp., Tychius spp., Xylotrechus spp., Zabrus spp., z.B. Zabrus tenebrioides;
aus der Ordnung der Diptera z.B. Aedes spp., z.B. Aedes aegypti, Aedes albopictus, Aedes sticticus, Aedes vexans, Agromyza spp., z.B. Agromyza frontella, Agromyza parvicornis, Anastrepha spp., Anopheles spp., z.B. Anopheles quadrimaculatus, Anopheles gambiae, Asphondylia spp., Bactrocera spp., z.B. Bactrocera cucurbitae, Bactrocera dorsalis, Bactrocera oleae, Bibio hortulanus, Calliphora erythrocephala, Calliphora vicina, Ceratitis capitata, Chironomus spp., Chrysomya spp., Chrysops spp., Chrysozona pluvialis, Cochliomya spp., Contarinia spp., z.B. Contarinia johnsoni, Contarinia nasturtii, Contarinia pyrivora, Contarinia schulzi, Contarinia sorghicola, Contarinia tritici,Cordylobia anthropophaga, Cricotopus sylvestris, Culex spp., z.B. Culex pipiens, Culex quinquefasciatus, Culicoides spp., Culiseta spp., Cuterebra spp., Dacus oleae, Dasineura spp., z.B. Dasineura brassicae, Delia spp., z.B. Delia antiqua, Delia coarctata, Delia florilega, Delia platura, Delia radicum, Dermatobia hominis, Drosophila spp., z.B. Drosphila melanogaster, Drosophila suzukii, Echinocnemus spp., Fannia spp., Gasterophilus spp., Glossina spp., Haematopota spp., Hydrellia spp., Hydrellia griseola, Hylemya spp., Hippobosca spp., Hypoderma spp., Liriomyza spp., z.B. Liriomyza brassicae, Liriomyza huidobrensis, Liriomyza sativae, Lucilia spp., z.B. Lucilia cuprina, Lutzomyia spp., Mansonia spp., Musca spp., z.B. Musca domestica, Musca domestica vicina, Oestrus spp., Oscinella frit, Paratanytarsus spp., Paralauterborniella subcincta, Pegomya spp., z.B. Pegomya betae, Pegomya hyoscyami, Pegomya rubivora, Phlebotomus spp., Phorbia spp., Phormia spp., Piophila casei, Prodiplosis spp., Psila rosae, Rhagoletis spp., z.B. Rhagoletis cingulata, Rhagoletis completa, Rhagoletis fausta, Rhagoletis indifferens, Rhagoletis mendax, Rhagoletis pomonella, Sarcophaga spp., Simulium spp., z.B. Simulium meridionale, Stomoxys spp., Tabanus spp., Tetanops spp., Tipula spp., z.B. Tipula paludosa, Tipula simplex;
aus der Ordnung der Heteroptera z.B. Anasa tristis, Antestiopsis spp., Boisea spp., Blissus spp., Calocoris spp., Campylomma livida, Cavelerius spp., Cimex spp., z.B. Cimex adjunctus, Cimex hemipterus, Cimex lectularius, Cimex pilosellus, Collaria spp., Creontiades dilutus, Dasynus piperis, Dichelops furcatus, Diconocoris hewetti, Dysdercus spp., Euschistus spp., z.B. Euschistus heros, Euschistus servus, Euschistus tristigmus, Euschistus variolarius, Eurygaster spp., Halyomorpha halys, Heliopeltis spp., Horcias nobilellus, Leptocorisa spp., Leptocorisa varicornis, Leptoglossus occidentalis, Leptoglossus phyllopus, Lygocoris spp., z.B. Lygocoris pabulinus, Lygus spp., z.B. Lygus elisus, Lygus hesperus, Lygus lineolaris, Macropes excavatus, Monalonion atratum, Nezara spp., z.B. Nezara viridula, Oebalus spp., Piesma quadrata, Piezodorus spp., z.B. Piezodorus guildinii, Psallus spp., Pseudacysta persea, Rhodnius spp., Sahlbergella singularis, Scaptocoris castanea, Scotinophora spp., Stephanitis nashi, Tibraca spp., Triatoma spp.;
aus der Ordnung der Homoptera z.B. Acizzia acaciaebaileyanae, Acizzia dodonaeae, Acizzia uncatoides, Acrida turrita, Acyrthosipon spp., z.B. Acyrthosiphon pisum, Acrogonia spp., Aeneolamia spp., Agonoscena spp., Aleyrodes proletella, Aleurolobus barodensis, Aleurothrixus floccosus, Allocaridara malayensis, Amrasca spp., z.B. Amrasca bigutulla, Amrasca devastans, Anuraphis cardui, Aonidiella spp., z.B. Aonidiella aurantii, Aonidiella citrina, Aonidiella inornata, Aphanostigma piri, Aphis spp., z.B. Aphis craccivora, Aphis fabae, Aphis forbesi, Aphis glycines, Aphis gossypii, Aphis hederae, Aphis illinoisensis, Aphis middletoni, Aphis nasturtii, Aphis nerii, Aphis pomi, Aphis spiraecola, Aphis viburniphila, Arboridia apicalis, Arytainilla spp., Aspidiella spp., Aspidiotus spp., z.B. Aspidiotus nerii, Atanus spp., Aulacorthum solani, Bemisia tabaci, Blastopsylla occidentalis, Boreioglycaspis melaleucae, Brachycaudus helichrysi, Brachycolus spp., Brevicoryne brassicae, Cacopsylla spp., z.B. Cacopsylla pyricola, Calligypona marginata, Carneocephala fulgida, Ceratovacuna lanigera, Cercopidae, Ceroplastes spp., Chaetosiphon fragaefolii, Chionaspis tegalensis, Chlorita onukii, Chondracris rosea, Chromaphis juglandicola, Chrysomphalus ficus, Cicadulina mbila, Coccomytilus halli, Coccus spp., z.B. Coccus hesperidum, Coccus longulus, Coccus pseudomagnoliarum, Coccus viridis, Cryptomyzus ribis, Cryptoneossa spp., Ctenarytaina spp., Dalbulus spp., Dialeurodes citri, Diaphorina citri, Diaspis spp., Drosicha spp., Dysaphis spp., z.B. Dysaphis apiifolia, Dysaphis plantaginea, Dysaphis tulipae, Dysmicoccus spp., Empoasca spp., z.B. Empoasca abrupta, Empoasca fabae, Empoasca maligna, Empoasca solana, Empoasca stevensi, Eriosoma spp., z.B. Eriosoma americanum, Eriosoma lanigerum, Eriosoma pyricola, Erythroneura spp., Eucalyptolyma spp., Euphyllura spp., Euscelis bilobatus, Ferrisia spp., Geococcus coffeae, Glycaspis spp., Heteropsylla cubana, Heteropsylla spinulosa, Homalodisca coagulata, Hyalopterus arundinis, Hyalopterus pruni, Icerya spp., z.B. Icerya purchasi, Idiocerus spp., Idioscopus spp., Laodelphax striatellus, Lecanium spp., z.B. Lecanium corni (=Parthenolecanium corni), Lepidosaphes spp., z.B. Lepidosaphes ulmi, Lipaphis erysimi, Macrosiphum spp., z.B. Macrosiphum euphorbiae, Macrosiphum lilii, Macrosiphum rosae, Macrosteles facifrons, Mahanarva spp., Melanaphis sacchari, Metcalfiella spp., Metopolophium dirhodum, Monellia costalis, Monelliopsis pecanis, Myzus spp., z.B. Myzus ascalonicus, Myzus cerasi, Myzus ligustri, Myzus ornatus, Myzus persicae,. Myzus nicotianae, Nasonovia ribisnigri, Nephotettix spp., z.B. Nephotettix cincticeps,, Nephotettix nigropictus, Nilaparvata lugens, Oncometopia spp., Orthezia praelonga, Oxya chinensis, Pachypsylla spp., Parabemisia myricae, Paratrioza spp., z.B. Paratrioza cockerelli, Parlatoria spp., Pemphigus spp., z.B. Pemphigus bursarius, Pemphigus populivenae, Peregrinus maidis, Phenacoccus spp., z.B. Phenacoccus madeirensis, Phloeomyzus passerinii, Phorodon humuli, Phylloxera spp., z.B. Phylloxera devastatrix, Phylloxera notabilis, Pinnaspis aspidistrae, Planococcus spp., z.B. Planococcus citri, Prosopidopsylla flava, Protopulvinaria pyriformis, Pseudaulacaspis pentagona, Pseudococcus spp., z.B. Pseudococcus calceolariae, Pseudococcus comstocki, Pseudococcus longispinus, Pseudococcus maritimus, Pseudococcus viburni, Psyllopsis spp., Psylla spp., z.B. Psylla buxi, Psylla mali, Psylla pyri, Pteromalus spp., Pyrilla spp., Quadraspidiotus spp., z.B. Quadraspidiotus juglansregiae, Quadraspidiotus ostreaeformis, Quadraspidiotus perniciosus, Quesada gigas, Rastrococcus spp., Rhopalosiphum spp., z.B. Rhopalosiphum maidis, Rhopalosiphum oxyacanthae, Rhopalosiphum padi, Rhopalosiphum rufiabdominale, Saissetia spp., z.B. Saissetia coffeae, Saissetia miranda, Saissetia neglecta, Saissetia oleae, Scaphoideus titanus, Schizaphis graminum, Selenaspidus articulatus, Sitobion avenae, Sogata spp., Sogatella furcifera, Sogatodes spp., Stictocephala festina, Siphoninus phillyreae, Tenalaphara malayensis,Tetragonocephela spp., Tinocallis caryaefoliae, Tomaspis spp., Toxoptera spp., z.B. Toxoptera aurantii, Toxoptera citricidus, Trialeurodes vaporariorum, Trioza spp., z.B. Trioza diospyri, Typhlocyba spp., Unaspis spp., Viteus vitifolii, Zygina spp.;
aus der Ordnung der Hymenoptera z.B. Acromyrmex spp., Athalia spp., z.B. Athalia rosae, Atta spp., Diprion spp., z.B. Diprion similis, Hoplocampa spp., z.B. Hoplocampa cookei, Hoplocampa testudinea, Lasius spp., Monomorium pharaonis, Sirex spp., Solenopsis invicta, Tapinoma spp., Urocerus spp., Vespa spp., z.B. Vespa crabro, Xeris spp.;
aus der Ordnung der Isopoda z.B. Armadillidium vulgare, Oniscus asellus, Porcellio scaber;
aus der Ordnung der Isoptera z.B. Coptotermes spp., z.B. Coptotermes formosanus, Comitermes cumulans, Cryptotermes spp., Incisitermes spp., Microtermes obesi, Odontotermes spp., Reticulitermes spp., z.B. Reticulitermes flavipes, Reticulitermes hesperus;
aus der Ordnung der Lepidoptera z.B. Achroia grisella, Acronicta major, Adoxophyes spp., z.B. Adoxophyes orana, Aedia leucomelas, Agrotis spp., z.B. Agrotis segetum, Agrotis ipsilon, Alabama spp., z.B. Alabama argillacea, Amyelois transitella, Anarsia spp., Anticarsia spp., z.B. Anticarsia gemmatalis, Argyroploce spp., Barathra brassicae, Borbo cinnara, Bucculatrix thurberiella, Bupalus piniarius, Busseola spp., Cacoecia spp., Caloptilia theivora, Capua reticulana, Carpocapsa pomonella, Carposina niponensis, Cheimatobia brumata, Chilo spp., z.B. Chilo plejadellus, Chilo suppressalis, Choristoneura spp., Clysia ambiguella, Cnaphalocerus spp., Cnaphalocrocis medinalis, Cnephasia spp., Conopomorpha spp., Conotrachelus spp., Copitarsia spp., Cydia spp., z.B. Cydia nigricana, Cydia pomonella, Dalaca noctuides, Diaphania spp., Diatraea saccharalis, Earias spp., Ecdytolopha aurantium, Elasmopalpus lignosellus, Eldana saccharina, Ephestia spp., z.B. Ephestia elutella, Ephestia kuehniella, Epinotia spp., Epiphyas postvittana, Etiella spp., Eulia spp., Eupoecilia ambiguella, Euproctis spp., z.B. Euproctis chrysorrhoea, Euxoa spp., Feltia spp., Galleria mellonella, Gracillaria spp., Grapholitha spp., z.B. Grapholita molesta, Grapholita prunivora, Hedylepta spp., Helicoverpa spp., z.B. Helicoverpa armigera, Helicoverpa zea, Heliothis spp., z.B. Heliothis virescens Hofmannophila pseudospretella, Homoeosoma spp., Homona spp., Hyponomeuta padella, Kakivoria flavofasciata, Laphygma spp., Leucinodes orbonalis, Leucoptera spp., z.B. Leucoptera coffeella, Lithocolletis spp., z.B. Lithocolletis blancardella, Lithophane antennata, Lobesia spp., z.B. Lobesia botrana, Loxagrotis albicosta, Lymantria spp., z.b. Lymantria dispar, Lyonetia spp., z.B. Lyonetia clerkella, Malacosoma neustria, Maruca testulalis, Mamestra brassicae, Melanitis leda, Mocis spp., Monopis obviella, Mythimna separata, Nemapogon cloacellus, Nymphula spp., Oiketicus spp., Oria spp., Orthaga spp., Ostrinia spp., z.B. Ostrinia nubilalis, Oulema melanopus, Oulema oryzae, Panolis flammea, Parnara spp., Pectinophora spp., z.B. Pectinophora gossypiella, Perileucoptera spp., Phthorimaea spp., z.B. Phthorimaea operculella, Phyllocnistis citrella, Phyllonorycter spp., z.B. Phyllonorycter blancardella, Phyllonorycter crataegella, Pieris spp., z.B. Pieris rapae, Platynota stultana, Plodia interpunctella, Plusia spp., Plutella xylostella (=Plutella maculipennis), Prays spp., Prodenia spp., Protoparce spp., Pseudaletia spp., z.B. Pseudaletia unipuncta, Pseudoplusia includens, Pyrausta nubilalis, Rachiplusia nu, Schoenobius spp., z.B. Schoenobius bipunctifer, Scirpophaga spp., z.B. Scirpophaga innotata, Scotia segetum, Sesamia spp., z.B. Sesamia inferens, Sparganothis spp., Spodoptera spp., z.b. Spodoptera eradiana, Spodoptera exigua, Spodoptera frugiperda, Spodoptera praefica, Stathmopoda spp., Stomopteryx subsecivella, Synanthedon spp., Tecia solanivora, Thermesia gemmatalis, Tinea cloacella, Tinea pellionella, Tineola bisselliella, Tortrix spp., Trichophaga tapetzella, Trichoplusia spp., z.B. Trichoplusia ni, Tryporyza incertulas, Tuta absoluta, Virachola spp.;
aus der Ordnung der Orthoptera oder Saltatoria z.B. Acheta domesticus, Dichroplus spp., Gryllotalpa spp., z.B. Gryllotalpa gryllotalpa, Hieroglyphus spp., Locusta spp., z.B. Locusta migratoria, Melanoplus spp., z.B. Melanoplus devastator, Schistocerca gregaria;
aus der Ordnung der Phthiraptera z.B. Damalinia spp., Haematopinus spp., Linognathus spp., Pediculus spp., Phylloxera vastatrix, Phthirus pubis, Trichodectes spp.;
aus der Ordnung der Psocoptera z.B. Lepinotus spp., Liposcelis spp.;
aus der Ordnung der Siphonaptera z.B. Ceratophyllus spp., Ctenocephalides spp., z.B. Ctenocephalides canis, Ctenocephalides felis, Pulex irritans, Tunga penetrans, Xenopsylla cheopis;
aus der Ordnung der Thysanoptera z.B. Anaphothrips obscurus, Baliothrips biformis, Drepanothrips reuteri, Enneothrips flavens, Frankliniella spp., z.B. Frankliniella fusca, Frankliniella occidentalis, Frankliniella schultzei, Frankliniella tritici, Frankliniella vaccinii, Frankliniella williamsi, Heliothrips spp., Hercinothrips femoralis, Rhipiphorothrips cruentatus, Scirtothrips spp., Taeniothrips cardamomi, Thrips spp., z.B. Thrips palmi, Thrips tabaci;
aus der Ordnung der Zygentoma (= Thysanura), z. B. Ctenolepisma spp., Lepisma saccharina, Lepismodes inquilinus, Thermobia domestica;
aus der Klasse der Symphyla z.B. Scutigerella spp., z.B. Scutigerella immaculata;
Schädlinge aus dem Stamm der Mollusca, insbesondere aus der Klasse der Bivalvia, z.B. Dreissena spp.;
sowie aus der Klasse der Gastropoda z.B. Arion spp., z.B. Arion ater rufus, Biomphalaria spp., Bulinus spp., Deroceras spp., z.B. Deroceras laeve, Galba spp., Lymnaea spp., Oncomelania spp., Pomacea spp., Succinea spp.;
Tierparasiten aus den Stämmen der Plathelminthes und Nematoda, z.B. Ancylostoma spp., z.B. Ancylostoma duodenale, Ancylostoma ceylanicum, Ancylostoma braziliensis, Ascaris spp., Brugia malayi, Brugia timori, Bunostomum spp., Chabertia spp., Clonorchis spp., Cooperia spp., Dicrocoelium spp., Dictyocaulus filaria, Diphyllobothrium latum, Dracunculus medinensis, Echinococcus granulosus, Echinococcus multilocularis, Enterobius vermicularis, Faciola spp., Haemonchus spp., Heterakis spp., Hymenolepis nana, Hyostrongulus spp., Loa Loa, Nematodirus spp., Oesophagostomum spp., Opisthorchis spp., Onchocerca volvulus, Ostertagia spp., Paragonimus spp., Schistosomen spp., Strongyloides fuelleborni, Strongyloides stercoralis, Stronyloides spp., Taenia saginata, Taenia solium, Trichinella spiralis, Trichinella nativa, Trichinella britovi, Trichinella nelsoni, Trichinella pseudopsiralis, Trichostrongulus spp., Trichuris trichuria, Wuchereria bancrofti;
Pflanzenschädlinge aus dem Stamm der Nematoda, d.h. pflanzenparasitäre Nematoden, insbesondere Aglenchus spp., z.B. Aglenchus agricola, Anguina spp., z.B. Anguina tritici, Aphelenchoides spp., z.B. Aphelenchoides arachidis, Aphelenchoides fragariae, Belonolaimus spp., z.B. Belonolaimus gracilis, Belonolaimus longicaudatus, Belonolaimus nortoni, Bursaphelenchus spp., z.B. Bursaphelenchus cocophilus, Bursaphelenchus eremus, Bursaphelenchus xylophilus, Cacopaurus spp., z.B. Cacopaurus pestis, Criconemella spp., z.B. Criconemella curvata, Criconemella onoensis, Criconemella ornata, Criconemella rusium, Criconemella xenoplax (= Mesocriconema xenoplax), Criconemoides spp., z.B. Criconemoides ferniae, Criconemoides onoense, Criconemoides ornatum, Ditylenchus spp., z.B. Ditylenchus dipsaci, Dolichodorus spp., Globodera spp., z.B. Globodera pallida, Globodera rostochiensis, Helicotylenchus spp., z.B. Helicotylenchus dihystera, Hemicriconemoides spp., Hemicycliophora spp., Heterodera spp., z.B. Heterodera avenae, Heterodera glycines, Heterodera schachtii, Hoplolaimus spp., Longidorus spp., z.B. Longidorus africanus, Meloidogyne spp., z.B. Meloidogyne chitwoodi, Meloidogyne fallax, Meloidogyne hapla, Meloidogyne incognita, Meloinema spp., Nacobbus spp., Neotylenchus spp., Paraphelenchus spp., Paratrichodorus spp., z.B. Paratrichodorus minor, Pratylenchus spp., z.B. Pratylenchus penetrans, Pseudohalenchus spp., Psilenchus spp., Punctodera spp., Quinisulcius spp., Radopholus spp., z.B. Radopholus citrophilus, Radopholus similis, Rotylenchulus spp., Rotylenchus spp., Scutellonema spp., Subanguina spp., Trichodorus spp., z.B. Trichodorus obtusus, Trichodorus primitivus, Tylenchorhynchus spp., z.B. Tylenchorhynchus annulatus, Tylenchulus spp., z.B. Tylenchulus semipenetrans, Xiphinema spp., z.B. Xiphinema index;
Weiterhin lässt sich aus dem Unterreich der Protozoa die Ordnung der Coccidia z.B. Eimeria spp. bekämpfen.

Die Verbindungen der Formel (Ia") können gegebenenfalls in bestimmten Konzentrationen bzw. Aufwandmengen auch als Herbizide, Safener, Wachstumsregulatoren oder Mittel zur Verbesserung der Pflanzeneigenschaften, als Mikrobizide oder Gametozide, beispielsweise als Fungizide, Antimykotika, Bakterizide, Virizide (einschließlich Mittel gegen Viroide) oder als Mittel gegen MLO (Mycoplasmalike-organism) und RLO (Rickettsia-like-organism) verwendet werden. Sie lassen sich gegebenenfalls auch als Zwischen- oder Vorprodukte für die Synthese weiterer Wirkstoffe einsetzen.

Die vorliegende Erfindung betrifft weiterhin Formulierungen und daraus bereitete Anwendungsformen als Schädlingsbekämpfungsmittel wie z. B. Drench-, Drip- und Spritzbrühen, umfassend mindestens eine Verbindung der Formel (Ia"). Gegebenenfalls enthalten die Anwendungsformen weitere Schädlingsbekämpfungsmittel und/oder die Wirkung verbessernde Adjuvantien wie Penetrationsförderer, z. B. vegetative Öle wie beispielsweise Rapsöl, Sonnenblumenöl, Mineralöle wie beispielsweise Paraffinöle, Alkylester vegetativer Fettsäuren wie beispielsweise Rapsöl- oder Sojaölmethylester oder Alkanol-alkoxylate und/oder Spreitmittel wie beispielsweise Alkylsiloxane und/oder Salze z.B. organische oder anorganische Ammonium- oder Phosphoniumsalze wie beispielsweise Ammoniumsulfat oder Diammonium-hydrogenphosphat und/oder die Retention fördernde Mittel wie z. B. Dioctylsulfosuccinat oder Hydroxypropyl-guar Polymere und/oder Humectants wie z.B. Glycerin und/oder Dünger wie beispielsweise Ammonium-, Kalium- oder Phosphor-enthaltende Dünger.

Übliche Formulierungen sind beispielsweise wasserlösliche Flüssigkeiten (SL), Emulsionskonzentrate (EC), Emulsionen in Wasser (EW), Suspensionskonzentrate (SC, SE, FS, OD), in Wasser dispergierbare Granulate (WG), Granulate (GR) und Kapselkonzentrate (CS); diese und weitere mögliche Formuliertypen sind beispielsweise durch Crop Life International und in Pesticide Specifications, Manual on development and use of FAO and WHO specifications for pesticides, FAO Plant Production and Protection Papers - 173, prepared by the FAO/WHO Joint Meeting on Pesticide Specifications, 2004, ISBN: 9251048576 beschrieben. Gegebenenfalls enthalten die Formulierungen neben einem oder mehreren Verbindungen der Formel (Ia") weitere agrochemische Wirkstoffe.

Vorzugsweise handelt es sich um Formulierungen oder Anwendungsformen, welche Hilfsstoffe wie beispielsweise Streckmittel, Lösemittel, Spontanitätsförderer, Trägerstoffe, Emulgiermittel, Dispergiermittel, Frostschutzmittel, Biozide, Verdicker und/oder weitere Hilfsstoffe wie beispielsweise Adjuvantien enthalten. Ein Adjuvant in diesem Kontext ist eine Komponente, die die biologische Wirkung der Formulierung verbessert, ohne dass die Komponente selbst eine biologische Wirkung hat. Beispiele für Adjuvantien sind Mittel, die die Retention, das Spreitverhalten, das Anhaften an der Blattoberfläche oder die Penetration fördern.

Diese Formulierungen werden in bekannter Weise hergestellt, z.B. durch Vermischen der Verbindungen der Formel (Ia") mit Hilfsstoffen wie beispielsweise Streckmitteln, Lösemitteln und/oder festen Trägerstoffen und/oder weiteren Hilfsstoffen wie beispielsweise oberflächenaktive Stoffe. Die Herstellung der Formulierungen erfolgt entweder in geeigneten Anlagen oder auch vor oder während der Anwendung.

Als Hilfsstoffe können solche Stoffe Verwendung finden, die geeignet sind, der Formulierung der Verbindungen der Formel (Ia") oder den aus diesen Formulierungen bereiteten Anwendungsformen (wie z.B. gebrauchsfähigen Schädlingsbekämpfungsmitteln wie Spritzbrühen oder Saatgutbeizen) besondere Eigenschaften, wie bestimmte physikalische, technische und/oder biologische Eigenschaften zu verleihen.

Als Streckmittel eignen sich z.B. Wasser, polare und unpolare organische chemische Flüssigkeiten z.B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Aceton, Cyclohexanon), Ester (auch Fette und Öle) und (Poly-)Ether, der einfachen und substituierten Amine, Amide, Lactame (wie N-Alkylpyrrolidone) und Lactone, der Sulfone und Sulfoxide (wie Dimethylsulfoxid).

Im Falle der Benutzung von Wasser als Streckmittel können z.B. auch organische Lösemittel als Hilfslösemittel verwendet werden. Als flüssige Lösemittel kommen im Wesentlichen infrage: Aromaten wie Xylol, Toluol oder Alkylnaphthaline, chlorierte Aromaten oder chlorierte aliphatische Kohlenwasser-stoffe wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole wie Butanol oder Glykol sowie deren Ether und Ester, Ketone wie Aceton, Methylethylketon, Methylisobutylketon oder Cyclohexanon, stark polare Lösemittel wie Dimethylformamid und Dimethylsulfoxid sowie Wasser.

Grundsätzlich können alle geeigneten Lösemittel verwendet werden. Geeignete Lösemittel sind beispielsweise aromatische Kohlenwasserstoffe wie z.B. Xylol, Toluol oder Alkylnaphthaline, chlorierte aromatische oder aliphatische Kohlenwasserstoffe wie z.B. Chlorbenzol, Chlorethylen, oder Methylenchlorid, aliphatische Kohlenwasserstoffe wie z.B. Cyclohexan, Paraffine, Erdölfraktionen, mineralische und pflanzliche Öle, Alkohole wie z.B. Methanol, Ethanol, iso-Propanol, Butanol oder Glykol sowie deren Ether und Ester, Ketone wie z.B. Aceton, Methylethylketon, Methylisobutylketon oder Cyclohexanon, stark polare Lösemittel wie Dimethylsulfoxid sowie Wasser.

Grundsätzlich können alle geeigneten Trägerstoffe eingesetzt werden. Als Trägerstoffe kommen insbesondere infrage: z.B. Ammoniumsalze und natürliche Gesteinsmehle wie Kaoline, Tonerden, Talkum, Kreide, Quarz, Attapulgit, Montmorillonit oder Diatomeenerde und synthetische Gesteinsmehl, wie hochdisperse Kieselsäure, Aluminiumoxid und natürliche oder synthetische Silikate, Harze, Wachse und /oder feste Düngemittel. Mischungen solcher Trägerstoffe können ebenfalls verwendet werden. Als Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Papier, Kokosnussschalen, Maiskolben und Tabakstängel.

Auch verflüssigte gasförmige Streckmittel oder Lösemittel können eingesetzt werden. Insbesondere eignen sich solche Streckmittel oder Trägerstoffe, welche bei normaler Temperatur und unter Normaldruck gasförmig sind, z.B. Aerosol-Treibgase wie Halogenkohlenwasserstoffe sowie Butan, Propan, Stickstoff und Kohlendioxid.

Beispiele für Emulgier- und/oder Schaum erzeugende Mittel, Dispergiermittel oder Benetzungsmittel mit ionischen oder nicht-ionischen Eigenschaften oder Mischungen dieser oberflächenaktiven Stoffe sind Salze von Polyacrylsäure, Salze von Lignosulphonsäure, Salze von Phenolsulphonsäure oder Naphthalinsulphonsäure, Polykondensate von Ethylenoxid mit Fettalkoholen oder mit Fettsäuren oder mit Fettaminen, mit substituierten Phenolen (vorzugsweise Alkylphenole oder Arylphenole), Salze von Sulphobernsteinsäureestern, Taurinderivate (vorzugsweise Alkyltaurate), Phosphorsäureester von polyethoxylierten Alkoholen oder Phenole, Fettsäureester von Polyolen und Derivate der Verbindungen enthaltend Sulphate, Sulphonate und Phosphate, z.B. Alkylarylpolyglycolether, Alkylsulfonate, Alkylsulfate, Arylsulfonate, Eiweißhydrolysate, Lignin-Sulfitablaugen und Methylcellulose. Die Anwesenheit einer oberflächenaktiven Substanz ist vorteilhaft, wenn eine der Verbindungen der Formel (Ia") und/oder einer der inerten Trägerstoffe nicht in Wasser löslich ist und wenn die Anwendung in Wasser erfolgt.

Als weitere Hilfsstoffe können in den Formulierungen und den daraus abgeleiteten Anwendungsformen Farbstoffe wie anorganische Pigmente, z.B. Eisenoxid, Titanoxid, Ferrocyanblau und organische Farbstoffe wie Alizarin-, Azo- und Metallphthalocyaninfarbstoffe und Nähr- und Spurennährstoffe wie Salze von Eisen, Mangan, Bor, Kupfer, Kobalt, Molybdän und Zink vorhanden sein.

Weiterhin enthalten sein können Stabilisatoren wie Kältestabilisatoren, Konservierungsmittel, Oxidationsschutzmittel, Lichtschutzmittel oder andere die chemische und / oder physikalische Stabilität verbessernde Mittel. Weiterhin enthalten sein können schaumerzeugende Mittel oder Entschäumer.

Ferner können die Formulierungen und daraus abgeleiteten Anwendungsformen als zusätzliche Hilfsstoffe auch Haftmittel wie Carboxymethylcellulose, natürliche und synthetische pulverige, körnige oder latexförmige Polymere enthalten wie Gummiarabikum, Polyvinylalkohol, Polyvinylacetat sowie natürliche Phospholipide wie Kephaline und Lecithine und synthetische Phospholipide. Weitere Hilfsstoffe können mineralische und vegetabile Öle sein.

Gegebenenfalls können noch weitere Hilfsstoffe in den Formulierungen und den daraus abgeleiteten Anwendungsformen enthalten sein. Solche Zusatzstoffe sind beispielsweise Duftstoffe, schützende Kolloide, Bindemittel, Klebstoffe, Verdicker, thixotrope Stoffe, Penetrationsförderer, Retentionsförderer, Stabilisatoren, Sequestiermittel, Komplexbildner, Humectans, Spreitmittel. Im Allgemeinen können die Verbindungen der Formel (Ia") mit jedem festen oder flüssigen Zusatzstoff, welches für Formulierungszwecke gewöhnlich verwendet wird, kombiniert werden.

Als Retentionsförderer kommen alle diejenigen Substanzen in Betracht, die die dynamische Oberflächenspannung verringern wie beispielsweise Dioctylsulfosuccinat oder die die Visko-Elastizität erhöhen wie beispielsweise Hydroxypropyl-guar Polymere.

Als Penetrationsförderer kommen im vorliegenden Zusammenhang alle diejenigen Substanzen in Betracht, die üblicherweise eingesetzt werden, um das Eindringen von agrochemischen Wirkstoffen in Pflanzen zu verbessern. Penetrationsförderer werden in diesem Zusammenhang dadurch definiert, dass sie aus der (in der Regel wässerigen) Applikationsbrühe und/oder aus dem Spritzbelag in die Kutikula der Pflanze eindringen und dadurch die Stoffbeweglichkeit (Mobilität) der Wirkstoffe in der Kutikula erhöhen können. Die in der Literatur (Baur et al., 1997, Pesticide Science 51, 131-152) beschriebene Methode kann zur Bestimmung dieser Eigenschaft eingesetzt werden. Beispielhaft werden genannt Alkoholalkoxylate wie beispielsweise Kokosfettethoxylat (10) oder Isotridecylethoxylat (12), Fettsäureester wie beispielsweise Rapsöl- oder Sojaölmethylester, Fettamine Alkoxylate wie beispielsweise Tallowamine-ethoxylat (15) oder Ammonium- und/oder Phosphonium-Salze wie beispielsweise Ammoniumsulfat oder Diammonium-hydrogenphosphat.

Die Formulierungen enthalten bevorzugt zwischen 0,00000001 und 98 Gew.-% der Verbindung der Formel (Ia"), besonders bevorzugt zwischen 0,01 und 95 Gew.-% der Verbindung der Formel (Ia"), ganz besonders bevorzugt zwischen 0,5 und 90 Gew.-% der Verbindung der Formel (Ia"), bezogen auf das Gewicht der Formulierung.

Der Gehalt an der Verbindung der Formel (Ia") in den aus den Formulierungen bereiteten Anwendungsformen (insbesondere Schädlingsbekämpfungsmittel) kann in weiten Bereichen variieren. Die Konzentration der Verbindung der Formel (Ia") in den Anwendungsformen kann üblicherweise zwischen 0,00000001 und 95 Gew.-% der Verbindung der Formel (Ia"), vorzugsweise zwischen 0,00001 und 1 Gew.-%, bezogen auf das Gewicht der Anwendungsform, liegen. Die Anwendung geschieht in einer den Anwendungsformen angepaßten üblichen Weise.

Die Verbindungen der Formel (Ia") können auch in Mischung mit einem oder mehreren geeigneten Fungiziden, Bakteriziden, Akariziden, Molluskiziden, Nematiziden, Insektiziden, Mikrobiologika, Nützlingen, Herbizide, Düngemitteln, Vogelrepellentien, Phytotonics, Sterilantien, Safenern, Semiochemicals und/oder Pflanzenwachstumsregulatoren verwendet werden, um so z.B. das Wirkungsspektrum zu verbreitern, die Wirkdauer zu verlängern, die Wirkgeschwindigkeit zu steigern, Repellenz zu verhindern oder Resistenzentwicklungen vorzubeugen. Desweiteren können solche Wirkstoffkombinationen das Pflanzenwachstum und/oder die Toleranz gegenüber abiotischen Faktoren wie z. B. hohen oder niedrigen Temperaturen, gegen Trockenheit oder gegen erhöhten Wasser- bzw. Bodensalzgehalt verbessern. Auch lässt sich das Blüh- und Fruchtverhalten verbessern, die Keimfähigkeit und Bewurzelung optimieren, die Ernte erleichtern und Ernteerträge steigern, die Reife beeinflussen, die Qualität und/oder den Ernährungswert der Ernteprodukte steigern, die Lagerfähigkeit verlängern und/oder die Bearbeitbarkeit der Ernteprodukte verbessern.

Weiterhin können die Verbindungen der Formel (Ia") in Mischung mit weiteren Wirkstoffen oder Semiochemicals, wie Lockstoffen und/oder Vogelrepellentien und/oder Pflanzenaktivatoren und/oder Wachstumsregulatoren und/oder Düngemitteln vorliegen. Gleichfalls können die Verbindungen der Formel (Ia") in Mischungen mit Mitteln zur Verbesserung der Pflanzeneigenschaften wie zum Beispiel Wuchs, Ertrag und Qualität des Erntegutes eingesetzt werden.

In einer besonderen erfindungsgemäßen Ausführungsform liegen die Verbindungen der Formel (Ia") in Formulierungen bzw. in den aus diesen Formulierungen bereiteten Anwendungsformen in Mischung mit weiteren Verbindungen vor, vorzugsweise solchen wie nachstehend beschrieben.

Wenn eine der im Folgenden genannten Verbindungen in verschiedenen tautomeren Formen vorkommen kann sind auch diese Formen mit umfasst, auch wenn sie sie nicht in jedem Fall explizit genannt wurden.

### Insektizide / Akarizide / Nematizide

Die hier mit ihrem "common name" genannten Wirkstoffe sind bekannt und beispielsweise im Pestizidhandbuch ("The Pesticide Manual" 16th Ed., British Crop Protection Council 2012) beschrieben oder im Internet recherchierbar (z.B. http://www.alanwood.net/pesticides).
(1) Acetylcholinesterase (AChE) Inhibitoren, wie beispielsweise Carbamate, z.B. Alanycarb, Aldicarb, Bendiocarb, Benfuracarb, Butocarboxim, Butoxycarboxim, Carbaryl, Carbofuran, Carbosulfan, Ethiofencarb, Fenobucarb, Formetanate, Furathiocarb, Isoprocarb, Methiocarb, Methomyl, Metolcarb, Oxamyl, Pirimicarb, Propoxur, Thiodicarb, Thiofanox, Triazamate, Trimethacarb, XMC und Xylylcarb oder organophosphate, z.B. Acephate, Azamethiphos, Azinphos-ethyl, Azinphos-methyl, Cadusafos, Chlorethoxyfos, Chlorfenvinphos, Chlormephos, Chlorpyrifos, Chlorpyrifos-methyl, Coumaphos, Cyanophos, Demeton-S-methyl, Diazinon, Dichlorvos/DDVP, Dicrotophos, Dimethoate, Dimethylvinphos, Disulfoton, EPN, Ethion, Ethoprophos, Famphur, Fenamiphos, Fenitrothion, Fenthion, Fosthiazate, Heptenophos, Imicyafos, Isofenphos, Isopropyl O-(methoxyaminothio-phosphoryl) salicylat, Isoxathion, Malathion, Mecarbam, Methamidophos, Methidathion, Mevinphos, Monocrotophos, Naled, Omethoate, Oxydemeton-methyl, Parathion, Parathion-methyl, Phenthoate, Phorate, Phosalone, Phosmet, Phosphamidon, Phoxim, Pirimiphos-methyl, Profenofos, Propetamphos, Prothiofos, Pyraclofos, Pyridaphenthion, Quinalphos, Sulfotep, Tebupirimfos, Temephos, Terbufos, Tetrachlorvinphos, Thiometon, Triazophos, Triclorfon und Vamidothion.
(2) GABA-gesteuerte Chlorid-Kanal-Antagonisten, wie beispielsweise Cyclodien-organochlorine, z.B. Chlordane und Endosulfan oder Phenylpyrazole (Fiprole), z.B. Ethiprole und Fipronil.
(3) Natrium-Kanal-Modulatoren / Spannungsabhängige Natrium-Kanal-Blocker, wie beispielsweise Pyrethroide, z.B. Acrinathrin, Allethrin, d-cis-trans Allethrin, d-trans Allethrin, Bifenthrin, Bioallethrin, Bioallethrin S-cyclopentenyl Isomer, Bioresmethrin, Cycloprothrin, Cyfluthrin, beta-Cyfluthrin, Cyhalothrin, lambda-Cyhalothrin, gamma-Cyhalothrin, Cypermethrin, alpha-Cypermethrin, beta-Cypermethrin, theta-Cypermethrin, zeta-Cypermethrin, Cyphenothrin [(1R)-trans-Isomere], Deltamethrin, Empenthrin [(EZ)-(1R)-Isomere), Esfenvalerate, Etofenprox, Fenpropathrin, Fenvalerate, Flucythrinate, Flumethrin, tau-Fluvalinate, Halfenprox, Imiprothrin, Kadethrin, Permethrin, Phenothrin [(1R)-trans-Isomer), Prallethrin, Pyrethrine (pyrethrum), Resmethrin, Silafluofen, Tefluthrin, Tetramethrin, Tetramethrin [(1R)- Isomere)], Tralomethrin und Transfluthrin oder DDT oder Methoxychlor.
(4) Nikotinerge Acetylcholin-Rezeptor (nAChR) Agonisten, wie beispielsweise Neonikotinoide, z.B. Acetamiprid, Clothianidin, Dinotefuran, Imidacloprid, Nitenpyram, Thiacloprid und Thiamethoxam oder Nikotin oder Sulfoxaflor.
(5) Nikotinerge Acetylcholin-Rezeptor (nAChR) allosterische Aktivatoren, wie beispielsweise Spinosine, z.B. Spinetoram und Spinosad.
(6) Chlorid-Kanal-Aktivatoren, wie beispielsweise Avermectine/Milbemycine, z.B. Abamectin, Emamectin-benzoat, Lepimectin und Milbemectin.
(7) Juvenilhormon-Imitatoren, wie beispielsweise Juvenilhormon-Analoge, z.B. Hydroprene, Kinoprene und Methoprene oder Fenoxycarb oder Pyriproxyfen.
(8) Wirkstoffe mit unbekannten oder nicht spezifischen Wirkmechanismen, wie beispielsweise Alkylhalide, z.B. Methylbromid und andere Alkylhalide; oderChloropicrin oder Sulfurylfluorid oder Borax oder Brechweinstein.
(9) Selektive Fraßhemmer, z.B. Pymetrozine oder Flonicamid.
(10) Milbenwachstumsinhibitoren, z.B. Clofentezine, Hexythiazox und Diflovidazin oder Etoxazole.
(11) Mikrobielle Disruptoren der Insektendarmmembran, z.B. Bacillus thuringiensis Subspezies israelensis, Bacillus sphaericus, Bacillus thuringiensis Subspezies aizawai, Bacillus thuringiensis Subspezies kurstaki, Bacillus thuringiensis Subspezies tenebrionis und BT Pflanzenproteine: Cry1Ab, Cry1Ac, Cry1Fa, Cry2Ab, mCry3A, Cry3Ab, Cry3Bb, Cry34/35Ab1.
(12) Inhibitoren der oxidativen Phosphorylierung, ATP-Disruptoren, wie beispielsweise Diafenthiuron oder Organozinnverbindungen, z.B. Azocyclotin, Cyhexatin und Fenbutatin-oxid oder Propargite oder Tetradifon.
(13) Entkoppler der oxidativen Phoshorylierung durch Unterbrechung des H-Protongradienten, wie beispielsweise Chlorfenapyr, DNOC und Sulfluramid.
(14) Nikotinerge Acetylcholin-Rezeptor-Antagonisten, wie beispielsweise Bensultap, Cartaphydrochlorid, Thiocyclam und Thiosultap-Natrium.
(15) Inhibitoren der Chitinbiosynthese, Typ 0, wie beispielsweise Bistrifluron, Chlorfluazuron, Diflubenzuron, Flucycloxuron, Flufenoxuron, Hexaflumuron, Lufenuron, Novaluron, Noviflumuron, Teflubenzuron und Triflumuron.
(16) Inhibitoren der Chitinbiosynthese, Typ 1, wie beispielsweise Buprofezin.
(17) Häutungsstörende Wirkstoffe, (insbesondere bei Dipteren, d.h.Zweiflüglern) wie beispielsweise Cyromazine.
(18) Ecdyson-Rezeptor Agonisten, wie beispielsweise Chromafenozide, Halofenozide, Methoxyfenozide und Tebufenozide.
(19) Oktopaminerge Agonisten, wie beispielsweise Amitraz.
(20) Komplex-III-Elektronentransportinhibitoren, wie beispielsweise Hydramethylnon oder Acequinocyl oder Fluacrypyrim.
(21) Komplex-I-Elektronentransportinhibitoren, beispielsweise METI-Akarizide, z.B. Fenazaquin, Fenpyroximate, Pyrimidifen, Pyridaben, Tebufenpyrad und Tolfenpyrad oder Rotenone (Derris).
(22) Spannungsabhängige Natriumkanal-Blocker, z.B. Indoxacarb oder Metaflumizone.
(23) Inhibitoren der Acetyl-CoA-Carboxylase, wie beispielsweise Tetron- und Tetramsäurederivate, z.B. Spirodiclofen, Spiromesifen und Spirotetramat.
(24) Komplex-IV-Elektronentransportinhibitoren, wie beispielsweise Phosphine, z.B. Aluminiumphosphid, Calciumphosphid, Phosphin und Zinkphosphid oder Cyanid.
(25) Komplex-II-Elektronentransportinhibitoren, wie beispielsweise Cyenopyrafen und Cyflumetofen.
(28) Ryanodinrezeptor-Effektoren, wie beispielsweise Diamide, z.B. Chlorantraniliprole, Cyantraniliprole und Flubendiamide,
weitere Wirkstoffe wie beispielsweise Afidopyropen, Azadirachtin, Benclothiaz, Benzoximate, Bifenazate, Bromopropylate, Chinomethionat, Cryolite,

Dicofol, Diflovidazin, Fluensulfone, Flometoquin, Flufenerim, Flufenoxystrobin, Flufiprole, Fluopyram, Flupyradifurone, Fufenozide, Heptafluthrin, Imidaclothiz, Iprodione, Meperfluthrin, Paichongding, Pyflubumide, Pyrifluquinazon, Pyriminostrobin, Tetramethylfluthrin und Iodmethan; desweiteren Präparate auf Basis von Bacillus firmus (I-1582, BioNeem, Votivo), sowie folgende Verbindungen:3-Brom-N-{2-brom-4-chlor-6-[(1-cyclopropylethyl)carbamoyl]phenyl}-1-(3-chlorpyridin-2-yl)-1H-pyrazol-5-carboxamid (bekannt aus WO2005/077934) und 1-{2-Fluor-4-methyl-5-[(2,2,2-trifluorethyl)sulfinyl]phenyl}-3-(trifluormethyl)-1H-1,2,4-triazol-5-amin (bekannt aus WO2006/043635), {1'-[(2E)-3-(4-Chlorphenyl)prop-2-en-1-yl]-5-fluorspiro[indol-3,4'-piperidin]-1(2H)-yl}(2-chlorpyridin-4-yl)methanon (bekannt aus WO2003/106457), 2-Chlor-N-[2-{1-[(2E)-3-(4-chlorphenyl)prop-2-en-1-yl]piperidin-4-yl}-4-(trifluormethyl)phenyl]isonicotinamid (bekannt aus WO2006/003494), 3-(2,5-Dimethylphenyl)-4-hydroxy-8-methoxy-1,8-diazaspiro[4.5]dec-3-en-2-on (bekannt aus WO2009/049851), 3-(2,5-Dimethylphenyl)-8-methoxy-2-oxo-1,8-diazaspiro[4.5]dec-3-en-4-yl-ethylcarbonat (bekannt aus WO2009/049851), 4-(But-2-in-1-yloxy)-6-(3,5-dimethylpiperidin-1-yl)-5-fluorpyrimidin (bekannt aus WO2004/099160), 4-(But-2-in-1-yloxy)-6-(3-chlorphenyl)pyrimidin (bekannt aus WO2003/076415), PF1364 (CAS-Reg.No. 1204776-60-2), 4-[5-(3,5-Dichlorphenyl)-5-(trifluormethyl)-4,5-dihydro-1,2-oxazol-3-yl]-2-methyl-N-{2-oxo-2-[(2,2,2-trifluorethyl)amino]ethyl}benzamid (bekannt aus WO2005/085216), 4-{5-[3-Chlor-5-(trifluormethyl)phenyl]-5-(trifluormethyl)-4,5-dihydro-1,2-oxazol-3-yl}-N-{2-oxo-2-[(2,2,2-trifluorethyl)amino]ethyl}-1-naphthamid (bekannt aus WO2009/002809), Methyl-2-[2-({[3-brom-1-(3-chlorpyridin-2-yl)-1H-pyrazol-5-yl]carbonyl}amino)-5-chlor-3-methylbenzoyl]-2-methylhydrazincarboxylat (bekannt aus WO2005/085216), Methyl-2-[2-({[3-brom-1-(3-chlorpyridin-2-yl)-1H-pyrazol-5-yl]carbonyl}amino)-5-cyan-3-methylbenzoyl]-2-ethylhydrazincarboxylat (bekannt aus WO2005/085216), Methyl-2-[2-({[3-brom-1-(3-chlorpyridin-2-yl)-1H-pyrazol-5-yl]carbonyl}amino)-5-cyan-3-methylbenzoyl]-2-methylhydrazincarboxylat (bekannt aus WO2005/085216), Methyl-2-[3,5-dibrom-2-({[3-brom-1-(3-chlorpyridin-2-yl)-1H-pyrazol-5-yl]carbonyl}amino)benzoyl]-2-ethylhydrazincarboxylat (bekannt aus WO2005/085216), 1-(3-Chlorpyridin-2-yl)-N-[4-cyan-2-methyl-6-(methylcarbamoyl)phenyl]-3-{[5-(trifluormethyl)-2H-tetrazol-2-yl]methyl}-1H-pyrazol-5-carboxamid (bekannt aus WO2010/069502), N-[2-(5-Amino-1,3,4-thiadiazol-2-yl)-4-chlor-6-methylphenyl]-3-brom-1-(3-chlorpyridin-2-yl)-1H-pyrazol-5-carboxamid (bekannt aus CN102057925), 3-Chlor-N-(2-cyanpropan-2-yl)-N-[4-(1,1,1,2,3,3,3-heptafluorpropan-2-yl)-2-methylphenyl]phthalamid (bekannt aus WO2012/034472), 8-Chlor-N-[(2-chlor-5-methoxyphenyl)sulfonyl]-6-(trifluormethyl)imidazo[1,2-a]pyridin-2-carboxamid (bekannt aus WO2010/129500), 4-[5-(3,5-Dichlorphenyl)-5-(trifluormethyl)-4,5-dihydro-1,2-oxazol-3-yl]-2-methyl-N-(1-oxidothietan-3-yl)benzamid (bekannt aus WO2009/080250), 4-[5-(3,5-Dichlorphenyl)-5-(trifluormethyl)-4,5-dihydro-1,2-oxazol-3-yl]-2-methyl-N-(1-oxidothietan-3-yl)benzamid (bekannt aus WO2012/029672), 1-[(2-Chlor-1,3-thiazol-5-yl)methyl]-4-oxo-3-phenyl-4H-pyrido[1,2-a]pyrimidin-1-ium-2-olat (bekannt aus WO2009/099929), 1-[(6-Chlorpyridin-3-yl)methyl]-4-oxo-3-phenyl-4H-pyrido[1,2-a]pyrimidin-1-ium-2-olat (bekannt aus WO2009/099929), (5S,8R)-1-[(6-Chlorpyridin-3-yl)methyl]-9-nitro-2,3,5,6,7,8-hexahydro-1H-5,8-epoxyimidazo[1,2-a]azepin (bekannt aus WO2010/069266), (2E)-1-[(6-Chlorpyridin-3-yl)methyl]-N'-nitro-2-pentylidenhydrazincarboximidamid (bekannt aus WO2010/060231), 4-(3-{2,6-Dichlor-4-[(3,3-dichlorprop-2-en-1-yl)oxy]phenoxy}propoxy)-2-methoxy-6-(trifluormethyl)pyrimidin (bekannt aus CN101337940), N-[2-(tert-Butylcarbamoyl)-4-chlor-6-methylphenyl]-1-(3-chlorpyridin-2-yl)-3-(fluormethoxy)-1H-pyrazol-5-carboxamid (bekannt aus WO2008/134969).

### Fungizide

Die hier mit ihrem "common name" spezifizierten Wirkstoffe sind bekannt, beispielsweise beschrieben im "Pesticide Manual" oder im Internet (beispielsweise: http://www.alanwood.net/pesticides).
(1) Inhibitoren der Ergosterol-Biosynthese, wie beispielsweise (1.1) Aldimorph, (1.2) Azaconazol, (1.3) Bitertanol, (1.4) Bromuconazol, (1.5) Cyproconazol, (1.6) Diclobutrazol, (1.7) Difenoconazol, (1.8) Diniconazol, (1.9) Diniconazol-M, (1.10) Dodemorph, (1.11) Dodemorph Acetat, (1.12) Epoxiconazol, (1.13) Etaconazol, (1.14) Fenarimol, (1.15) Fenbuconazol, (1.16) Fenhexamid, (1.17) Fenpropidin, (1.18) Fenpropimorph, (1.19) Fluquinconazol, (1.20) Flurprimidol, (1.21) Flusilazol, (1.22) Flutriafol, (1.23) Furconazol, (1.24) Furconazol-Cis, (1.25) Hexaconazol, (1.26) Imazalil, (1.27) Imazalil Sulfat, (1.28) Imibenconazol, (1.29) Ipconazol, (1.30) Metconazol, (1.31) Myclobutanil, (1.32) Naftifin, (1.33) Nuarimol, (1.34) Oxpoconazol, (1.35) Paclobutrazol, (1.36) Pefurazoat, (1.37) Penconazol, (1.38) Piperalin, (1.39) Prochloraz, (1.40) Propiconazol, (1.41) Prothioconazol, (1.42) Pyributicarb, (1.43) Pyrifenox, (1.44) Quinconazol, (1.45) Simeconazol, (1.46) Spiroxamin, (1.47) Tebuconazol, (1.48) Terbinafin, (1.49) Tetraconazol, (1.50) Triadimefon, (1.51) Triadimenol, (1.52) Tridemorph, (1.53) Triflumizol, (1.54) Triforin, (1.55) Triticonazol, (1.56) Uniconazol, (1.57) Uniconazol-p, (1.58) Viniconazol, (1.59) Voriconazol, (1.60) 1-(4-Chlorphenyl)-2-(1H-1,2,4-triazol-1-yl)cycloheptanol, (1.61) Methyl-1-(2,2-dimethyl-2,3-dihydro-1H-inden-1-yl)-1H-imidazol-5-carboxylat, (1.62) N'-{5-(Difluormethyl)-2-methyl-4-[3-(trimethylsilyl)propoxy]phenyl}-N-ethyl-N-methylimidoformamid, (1.63) N-Ethyl-N-methyl-N'- {2-methyl-5-(trifluormethyl)-4-[3-(trimethylsilyl)propoxy]phenyl}imidoformamid und (1.64) O-[1-(4-Methoxyphenoxy)-3,3-dimethylbutan-2-yl]-1H-imidazol-1-carbothioat, (1.65) Pyrisoxazole.
(2) Inhibitoren der Respiration (Atmungsketten-Inhibitoren), wie beispielsweise (2.1) Bixafen, (2.2) Boscalid, (2.3) Carboxin, (2.4) Diflumetorim, (2.5) Fenfuram, (2.6) Fluopyram, (2.7) Flutolanil, (2.8) Fluxapyroxad, (2.9) Furametpyr, (2.10) Furmecyclox, (2.11) Isopyrazam Mischung des syn-epimeren Razemates 1RS,4SR,9RS und des anti-empimeren Razemates 1RS,4SR,9SR, (2.12) Isopyrazam (anti-epimeres Razemat), (2.13) Isopyrazam (anti-epimeres Enantiomer 1R,4S,9S), (2.14) Isopyrazam (anti-epimeres Enantiomer 1S,4R,9R), (2.15) Isopyrazam (syn-epimeres Razemat 1RS,4SR,9RS), (2.16) Isopyrazam (syn-epimeres Enantiomer 1R,4S,9R), (2.17) Isopyrazam (syn-epimeres Enantiomer 1S,4R,9S), (2.18) Mepronil, (2.19) Oxycarboxin, (2.20) Penflufen, (2.21) Penthiopyrad, (2.22) Sedaxane, (2.23) Thifluzamid, (2.24) 1-Methyl-N-[2-(1,1,2,2-tetrafluorethoxy)phenyl]-3-(trifluormethyl)-1H-pyrazol-4-carboxamid, (2.25) 3-(Difluormethyl)-1-methyl-N-[2-(1,1,2,2-tetrafluorethoxy)phenyl]-1H-pyrazol-4-carboxamid, (2.26) 3-(Difluormethyl)-N-[4-fluor-2-(1,1,2,3,3,3-hexafluorpropoxy)phenyl]-1-methyl-1H-pyrazol-4-carboxamid, (2.27) N-[1-(2,4-Dichlorphenyl)-1-methoxypropan-2-yl]-3-(difluormethyl)-1-methyl-1H-pyrazol-4-carboxamid, (2.28) 5,8-Difluor-N-[2-(2-fluor-4-{[4-(trifluormethyl)pyridin-2-yl]oxy}phenyl)ethyl]quinazolin-4-amin, (2.29) Benzovindiflupyr, (2.30) N-[(1S,4R)-9-(Dichlormethylen)-1,2,3,4-tetrahydro-1,4-methanonaphthalen-5-yl]-3-(difluormethyl)-1-methyl-1H-pyrazol-4-carboxamid und (2.31) N-[(1R,4S)-9-(Dichlormethylen)-1,2,3,4-tetrahydro-1,4-methanonaphthalen-5-yl]-3-(difluormethyl)-1-methyl-1H-pyrazol-4-carboxamid, (2.32) 3-(Difluormethyl)-1-methyl-N-(1,1,3-trimethyl-2,3-dihydro-1H-inden-4-yl)-1H-pyrazol-4-carboxamid, (2.33) 1,3,5-Trimethyl-N-(1,1,3-trimethyl-2,3-dihydro-1H-inden-4-yl)-1H-pyrazol-4-carboxamid, (2.34) 1-Methyl-3-(trifluormethyl)-N-(1,1,3-trimethyl-2,3-dihydro-1H-inden-4-yl)-1H-pyrazol-4-carboxamid, (2.35) 1-Methyl-3-(trifluormethyl)-N-[(3R)-1,1,3-trimethyl-2,3-dihydro-1H-inden-4-yl]-1H-pyrazol-4-carboxamid, (2.36) 1-Methyl-3-(trifluormethyl)-N-[(3S)-1,1,3-trimethyl-2,3-dihydro-1H-inden-4-yl]-1H-pyrazol-4-carboxamid, (2.37) 3-(Difluormethyl)-1-methyl-N-[(3S)-1,1,3-trimethyl-2,3-dihydro-1H-inden-4-yl]-1H-pyrazol-4-carboxamid, (2.38) 3-(Difluormethyl)-1-methyl-N-[(3R)-1,1,3-trimethyl-2,3-dihydro-1H-inden-4-yl]-1H-pyrazol-4-carboxamid, (2.39) 1,3,5-Trimethyl-N-[(3R)-1,1,3-trimethyl-2,3-dihydro-1H-inden-4-yl]-1H-pyrazol-4-carboxamid, (2.40) 1,3,5-Trimethyl-N-[(3S)-1,1,3-trimethyl-2,3-dihydro-1H-inden-4-yl]-1H-pyrazol-4-carboxamid, (2.41) Benodanil, (2.42) 2-Chlor-N-(1,1,3-trimethyl-2,3-dihydro-1H-inden-4-yl)pyridine-3-carboxamid, (2.43) Isofetamid
(3) Inhibitoren der Respiration (Atmungsketten-Inhibitoren) am Komplex III der Atumungskette, wie beispielsweise (3.1) Ametoctradin, (3.2) Amisulbrom, (3.3) Azoxystrobin, (3.4) Cyazofamid, (3.5) Coumethoxystrobin, (3.6) Coumoxystrobin, (3.5) Dimoxystrobin, (3.8) Enestroburin, (3.9) Famoxadon, (3.10) Fenamidon, (3.11) Flufenoxystrobin, (3.12) Fluoxastrobin, (3.13) Kresoxim-Methyl, (3.14) Metominostrobin, (3.15) Orysastrobin, (3.16) Picoxystrobin, (3.17) Pyraclostrobin, (3.18) Pyrametostrobin, (3.19) Pyraoxystrobin, (3.20) Pyribencarb, (3.21) Triclopyricarb, (3.22) Trifloxystrobin, (3.23) (2E)-2-(2-{[6-(3-Chlor-2-methylphenoxy)-5-fluorpyrimidin-4-yl]oxy}phenyl)-2-(methoxyimino)-N-methylethanamid, (3.24) (2E)-2-(Methoxyimino)-N-methyl-2-(2-{[({(1E)-1-[3-(trifluormethyl)phenyl]ethyliden}amino)oxy]methyl}phenyl)ethanamid, (3.25) (2E)-2-(Methoxyimino)-N-methyl-2-{2-[(E)-({1-[3-(trifluormethyl)phenyl]ethoxy}imino)methyl]phenyl}ethanamid, (3.26) (2E)-2-{2-[({[(1E)-1-(3-{[(E)-1-Fluor-2-phenylethenyl]oxy}phenyl)ethyliden]amino}oxy)methyl]phenyl}-2-(methoxyimino)-N-methylethanamid, (3.27) (2E)-2- {2-[({[(2E,3E)-4-(2,6-Dichlorphenyl)but-3-en-2-yliden]amino}oxy)methyl]phenyl}-2-(methoxyimino)-N-methylethanamid, (3.28) 2-Chlor-N-(1,1,3-trimethyl-2,3-dihydro-1H-inden-4-yl)pyridin-3-carboxamid, (3.29) 5-Methoxy-2-methyl-4-(2-{[({(1E)-1-[3-(trifluormethyl)phenyl]ethyliden}amino)oxy]methyl}phenyl)-2,4-dihydro-3H-1,2,4-triazol-3-on, (3.30) Methyl-(2E)-2-{2-[({cyclopropyl[(4-methoxyphenyl)imino]methyl}sulfanyl)methyl]phenyl}-3-methoxyprop-2-enoat, (3.31) N-(3-Ethyl-3,5,5-trimethylcyclohexyl)-3-(formylamino)-2-hydroxybenzamid, (3.32) 2-{2-[(2,5-Dimethylphenoxy)methyl]phenyl}-2-methoxy-N-methylacetamid,
(4) Inhibitoren der Mitose und Zellteilung, wie beispielsweise (4.1) Benomyl, (4.2) Carbendazim, (4.3) Chlorfenazol, (4.4) Diethofencarb, (4.5) Ethaboxam, (4.6) Fluopicolid, (4.7) Fuberidazol, (4.8) Pencycuron, (4.9) Thiabendazol, (4.10) Thiophanat-Methyl, (4.11) Thiophanat, (4.12) Zoxamid, (4.13) 5-Chlor-7-(4-methylpiperidin-1-yl)-6-(2,4,6-trifluorphenyl)[1,2,4]triazolo[1,5-a]pyrimidin und (4.14) 3-Chlor-5-(6-chlorpyridin-3-yl)-6-methyl-4-(2,4,6-trifluorphenyl)pyridazin.
(5) Verbindungen mit Multisite-Aktivität, wie beispielsweise (5.1) Bordeauxmischung, (5.2) Captafol, (5.3) Captan, (5.4) Chlorthalonil, (5.5) Kupferzubereitungen wie Kupferhydroxid, (5.6) Kupfernaphthenat, (5.7) Kupferoxid, (5.8) Kupferoxychlorid, (5.9) Kupfersulfat, (5.10) Dichlofluanid, (5.11) Dithianon, (5.12) Dodine, (5.13) Dodine freie Base, (5.14) Ferbam, (5.15) Fluorfolpet, (5.16) Folpet, (5.17) Guazatin, (5.18) Guazatinacetat, (5.19) Iminoctadin, (5.20) Iminoctadinalbesilat, (5.21) Iminoctadintriacetat, (5.22) Mankupfer, (5.23) Mancozeb, (5.24) Maneb, (5.25) Metiram, (5.26) Zinkmetiram, (5.27) Kupfer-Oxin, (5.28) Propamidin, (5.29) Propineb, (5.30) Schwefel und Schwefelzubereitungen wie beispielsweise Calciumpolysulfid, (5.31) Thiram, (5.32) Tolylfluanid, (5.33) Zineb, (5.34) Ziram und (5,35) Anilazin.
(6) Resistenzinduktoren, wie beispielsweise (6.1) Acibenzolar-S-Methyl, (6.2) Isotianil, (6.3) Probenazol, (6.4) Tiadinil und (6.5) Laminarin.
(7) Inhibitoren der Aminosäure- und Protein-Biosynthese, wie beispielsweise (7.1), (7.2) Blasticidin-S, (7.3) Cyprodinil, (7.4) Kasugamycin, (7.5) Kasugamycin Hydrochlorid Hydrat, (7.6) Mepanipyrim, (7.7) Pyrimethanil, (7.8) 3-(5-Fluor-3,3,4,4-tetramethyl-3,4-dihydroisochinolin-1-yl)chinolin und (7.9) Oxytetracyclin und (7.10) Streptomycin.
(8) Inhibitoren der ATP Produktion, wie beispielsweise (8.1) Fentin Acetat, (8.2) Fentin Chlorid, (8.3) Fentin Hydroxid und (8.4) Silthiofam.
(9) Inhibitoren der Zellwandsynthese, wie beispielsweise (9.1) Benthiavalicarb, (9.2) Dimethomorph, (9.3) Flumorph, (9.4) Iprovalicarb, (9.5) Mandipropamid, (9.6) Polyoxins, (9.7) Polyoxorim, (9.8) Validamycin A, (9.9) Valifenalat und (9.10) Polyoxin B.
(10) Inhibitoren der Lipid- und Membran-Synthese, wie beispielsweise (10.1) Biphenyl, (10.2) Chlorneb, (10.3) Dicloran, (10.4) Edifenphos, (10.5) Etridiazol, (10.6) Iodocarb, (10.7) Iprobenfos, (10.8) Isoprothiolan, (10.9) Propamocarb, (10.10) Propamocarb Hydrochlorid, (10.11) Prothiocarb, (10.12) Pyrazophos, (10.13) Quintozen, (10.14) Tecnazene und (10.15) Tolclofos-Methyl.
(11) Inhibitoren der Melanin-Biosynthese, wie beispielsweise (11.1) Carpropamid, (11.2) Diclocymet, (11.3) Fenoxanil, (11.4) Fthalid, (11.5) Pyroquilon, (11.6) Tricyclazol, und (11.7) 2,2,2-Trifluorethyl {3-methyl-1-[(4-methylbenzoyl)amino]butan-2-yl}carbamat.
(12) Inhibitoren der Nukleinsäuresynthese, wie beispielsweise (12.1) Benalaxyl, (12.2) Benalaxyl-M (Kiralaxyl), (12.3) Bupirimat, (12.4) Clozylacon, (12.5) Dimethirimol, (12.6) Ethirimol, (12.7) Furalaxyl, (12.8) Hymexazol, (12.9) Metalaxyl, (12.10) Metalaxyl-M (Mefenoxam), (12.11) Ofurace, (12.12) Oxadixyl, (12.13) Oxolinsäure und (12.14) Octhilinon.
(13) Inhibitoren der Signaltransduktion, wie beispielsweise (13.1) Chlozolinat, (13.2) Fenpiclonil, (13.3) Fludioxonil, (13.4) Iprodion, (13.5) Procymidon, (13.6) Quinoxyfen, (13.7) Vinclozolin und (13.8) Proquinazid.
(14) Entkoppler, wie beispielsweise (14.1) Binapacryl, (14.2) Dinocap, (14.3) Ferimzon, (14.4) Fluazinam und (14.5) Meptyldinocap.
(15) Weitere Verbindungen, wie beispielsweise (15.1) Benthiazol, (15.2) Bethoxazin, (15.3) Capsimycin, (15.4) Carvon, (15.5) Chinomethionat, (15.6) Pyriofenon (Chlazafenon), (15.7) Cufraneb, (15.8) Cyflufenamid, (15.9) Cymoxanil, (15.10) Cyprosulfamid, (15.11) Dazomet, (15.12) Debacarb, (15.13) Dichlorphen, (15.14) Diclomezin, (15.15) Difenzoquat, (15.16) Difenzoquat Methylsulphat, (15.17) Diphenylamin, (15.18) Ecomat, (15.19) Fenpyrazamin, (15.20) Flumetover, (15.21) Fluorimid, (15.22) Flusulfamid, (15.23) Flutianil, (15.24) Fosetyl-Aluminium, (15.25) Fosetyl-Calcium, (15.26) Fosetyl-Natrium, (15.27) Hexachlorbenzol, (15.28) Irumamycin, (15.29) Methasulfocarb, (15.30) Methylisothiocyanat, (15.31) Metrafenon, (15.32) Mildiomycin, (15.33) Natamycin, (15.34) Nickel Dimethyldithiocarbamat, (15.35) Nitrothal-Isopropyl, (15.36) Octhilinone, (15.37) Oxamocarb, (15.38) Oxyfenthiin, (15.39) Pentachlorphenol und dessen Salze, (15.40) Phenothrin, (15.41) Phosphorsäure und deren Salze, (15.42) Propamocarb-Fosetylat, (15.43) Propanosin-Natrium, (15.44) Pyrimorph, (15.45) (2E)-3-(4-Tert-butylphenyl)-3-(2-chlorpyridin-4-yl)-1-(morpholin-4-yl)prop-2-en-1-on, (15.46) (2Z)-3-(4-Tert-butylphenyl)-3-(2-chlorpyridin-4-yl)-1-(morpholin-4-yl)prop-2-en-1-on, (15.47) Pyrrolnitrin, (15.48) Tebufloquin, (15.49) Tecloftalam, (15.50) Tolnifanide, (15.51) Triazoxid, (15.52) Trichlamid, (15.53) Zarilamid, (15.54) (3S,6S,7R,8R)-8-Benzyl-3-[({3-[(isobutyryloxy)methoxy]-4-methoxypyridin-2-yl}carbonyl)amino]-6-methyl-4,9-dioxo-1,5-dioxonan-7-yl 2-methylpropanoat, (15.55) 1-(4-{4-[(5R)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, (15.56) 1-(4-{4-[(5S)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, (15.57) 1-(4-{4-[5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, (15.58) 1-(4-Methoxyphenoxy)-3,3-dimethylbutan-2-yl 1H-imidazole-1-carboxylat, (15.59) 2,3,5,6-Tetrachlor-4-(methylsulfonyl)pyridin, (15.60) 2,3-Dibutyl-6-chlorthieno[2,3-d]pyrimidin-4(3H)-on, (15.61) 2,6-Dimethyl-1H,5H-[1,4]dithiino[2,3-c:5,6-c']dipyrrole-1,3,5,7(2H,6H)-tetron, (15.62) 2-[5-Methyl-3-(trifluormethyl)-1H-pyrazol-1-yl]-1-(4-{4-[(SR)-5-phenyl-4,5-dihydro-1,2-oxazol-3-yl]-1,3-thiazol-2-yl}piperidin-1-yl)ethanon, (15.63) 2-[5-Methyl-3-(trifluormethyl)-1H-pyrazol-1-yl]-1-(4-{4-[(5S)-5-phenyl-4,5-dihydro-1,2-oxazol-3-yl]-1,3-thiazol-2-yl}piperidin-1-yl)ethanon, (15.64) 2-[5-Methyl-3-(trifluormethyl)-1H-pyrazol-1-yl]-1-{4-[4-(5-phenyl-4,5-dihydro-1,2-oxazol-3-yl)-1,3-thiazol-2-yl]piperidin-1-yl}ethanon, (15.65) 2-Butoxy-6-iodo-3-propyl-4H-chromen-4-on, (15.66) 2-Chlor-5-[2-chlor-1-(2,6-difluor-4-methoxyphenyl)-4-methyl-1H-imidazol-5-yl]pyridin, (15.67) 2-Phenylphenol und Salze, (15.68) 3-(4,4,5-Trifluor-3,3-dimethyl-3,4-dihydroisochinolin-1-yl)chinolin, (15.69) 3,4,5-Trichlorpyridine-2,6-dicarbonitril, (15.70) 3-Chlor-5-(4-chlorphenyl)-4-(2,6-difluorphenyl)-6-methylpyridazin, (15.71) 4-(4-Chlorphenyl)-5-(2,6-difluorphenyl)-3,6-dimethylpyridazin, (15.72) 5-Amino-1,3,4-thiadiazole-2-thiol, (15.73) 5-Chlor-N'-phenyl-N'-(prop-2-yn-1-yl)thiophene-2-sulfonohydrazid, (15.74) 5-Fluor-2-[(4-fluorbenzyl)oxy]pyrimidin-4-amin, (15.75) 5-Fluor-2-[(4-methylbenzyl)oxy]pyrimidin-4-amin, (15.76) 5-Methyl-6-octyl[1,2,4]triazolo[1,5-a]pyrimidin-7-amin, (15.77) Ethyl (2Z)-3-amino-2-cyano-3-phenylacrylat, (15.78) N'-(4-{[3-(4-Chlorbenzyl)-1,2,4-thiadiazol-5-yl]oxy}-2,5-dimethylphenyl)-N-ethyl-N-methylimidoformamid, (15.79) N-(4-Chlorbenzyl)-3-[3-methoxy-4-(prop-2-yn-1-yloxy)phenyl]propanamid, (15.80) N-[(4-Chlorphenyl)(cyano)methyl] -3 - [3 -methoxy-4-(prop-2-yn-1 -yloxy)phenyl]propanamid, (15.81) N-[(5-Brom-3-chlorpyridin-2-yl)methyl]-2,4-dichlornicotinamid, (15.82) N-[1-(5-Brom-3-chlorpyridin-2-yl)ethyl]-2,4-dichlornicotinamid, (15.83) N-[1-(5-Brom-3-chlorpyridin-2-yl)ethyl]-2-fluor-4-iodonicotinamid, (15.84) N- {(E)-[(Cyclopropylmethoxy)imino] [6-(difluormethoxy)-2,3-difluorphenyl]methyl}-2-phenylacetamid, (15.85)N-{(Z)-[(Cyclopropylmethoxy)imino][6-(difluormethoxy)-2,3-difluorphenyl]methyl} -2-phenylacetamid, (15.86) N'- {4-[(3-Tert-butyl-4-cyano-1,2-thiazol-5-yl)oxy]-2-chlor-5-methylphenyl}-N-ethyl-N-methylimidoformamid, (15.87) N-Methyl-2-(1-{[5-methyl-3-(trifluormethyl)-1H-pyrazol-1-yl]acetyl}piperidin-4-yl)-N-(1,2,3,4-tetrahydronaphthalen-1 -yl)-1,3 -thiazole-4-carboxamid, (15.88) N-Methyl-2-(1 - {[5-methyl-3 - (trifluormethyl)-1H-pyrazol-1 -yl]acetyl}piperidin-4-yl)-N-[(1R)-1,2,3,4-tetrahydronaphthalen-1-yl]-1,3-thiazole-4-carboxamid, (15.89) N-Methyl-2-(1-{[5-methyl-3-(trifluormethyl)-1H-pyrazol-1-yl]acetyl}piperidin-4-yl)-N-[(1S)-1,2,3,4-tetrahydronaphthalen-1-yl]-1,3-thiazole-4-carboxamid, (15.90) Pentyl {6-[({[(1-methyl-1H-tetrazol-5-yl)(phenyl)methylene]amino}oxy)methyl]pyridin-2-yl}carbamat, (15.91) Phenazine-1-carbonsäure, (15.92) Chinolin-8-ol, (15.93) Chinolin-8-ol sulfate (2:1), (15.94) Tert-butyl {6-[({[(1-methyl-1H-tetrazol-5-yl)(phenyl)methylene]amino}oxy)methyl]pyridin-2-yl}carbamat, (15.95) 1-Methyl-3-(trifluormethyl)-N-[2'-(trifluormethyl)biphenyl-2-yl]-1H-pyrazol-4-carboxamid, (15.96) N-(4'-Chlorbiphenyl-2-yl)-3-(difluormethyl)-1-methyl-1H-pyrazol-4-carboxamid, (15.97) N-(2',4'-Dichlorbiphenyl-2-yl)-3-(difluormethyl)-1-methyl-1H-pyrazol-4-carboxamid, (15.98) 3-(Difluormethyl)-1-methyl-N-[4'-(trifluormethyl)biphenyl-2-yl]-1H-pyrazol-4-carboxamid, (15.99) N-(2',5'-Difluorbiphenyl-2-yl)-1-methyl-3-(trifluormethyl)-1H-pyrazol-4-carboxamid, (15.100) 3-(Difluormethyl)-1-methyl-N-[4'-(prop-1-yn-1-yl)biphenyl-2-yl]-1H-pyrazol-4-carboxamid, (15.101) 5-Fluor-1,3-dimethyl-N-[4'-(prop-1-yn-1-yl)biphenyl-2-yl]-1H-pyrazol-4-carboxamid, (15.102) 2-Chlor-N-[4'-(prop-1-yn-1-yl)biphenyl-2-yl]nicotinamid, (15.103) 3-(Difluormethyl)-N-[4'-(3,3-dimethylbut-1-yn-1-yl)biphenyl-2-yl]-1-methyl-1H-pyrazol-4-carboxamid, (15.104) N-[4'-(3,3 -dimethylbut-1-yn-1-yl)biphenyl-2-yl]-5-fluor-1,3-dimethyl-1H-pyrazol-4-carboxamid, (15.105) 3-(Difluormethyl)-N-(4'-ethinylbiphenyl-2-yl)-1-methyl-1H-pyrazol-4-carboxamid, (15.106) N-(4'-Ethinylbiphenyl-2-yl)-5-fluor-1,3-dimethyl-1H-pyrazol-4-carboxamid, (15.107) 2-Chlor-N-(4'-ethinylbiphenyl-2-yl)nicotinamid, (15.108) 2-Chlor-N-[4'-(3,3-dimethylbut-1-yn-1-yl)biphenyl-2-yl]nicotinamid, (15.109) 4-(Difluormethyl)-2-methyl-N-[4'-(trifluormethyl)biphenyl-2-yl]-1,3-thiazole-5-carboxamid, (15.110) 5-Fluor-N-[4'-(3-hydroxy-3-methylbut-1-yn-1-yl)biphenyl-2-yl]-1,3-dimethyl-1H-pyrazol-4-carboxamid, (15.111) 2-Chlor-N-[4'-(3-hydroxy-3-methylbut-1-yn-1-yl)biphenyl-2-yl]nicotinamid, (15.112) 3-(Difluormethyl)-N-[4'-(3-methoxy-3-methylbut-1-yn-1-yl)biphenyl-2-yl]-1-methyl-1H-pyrazol-4-carboxamid, (15.113) 5-Fluor-N-[4'-(3-methoxy-3-methylbut-1-yn-1-yl)biphenyl-2-yl]-1,3-dimethyl-1H-pyrazol-4-carboxamid, (15.114) 2-Chlor-N-[4'-(3-methoxy-3-methylbut-1-yn-1-yl)biphenyl-2-yl]nicotinamid, (15.115) (5-Brom-2-methoxy-4-methylpyridin-3-yl)(2,3,4-trimethoxy-6-methylphenyl)methanon, (15.116) N-[2-(4-{[3-(4-Chlorphenyl)prop-2-yn-1-yl]oxy}-3-methoxyphenyl)ethyl]-N2-(methylsulfonyl)valinamid, (15.117) 4-Oxo-4-[(2-phenylethyl)amino]butansäure, (15.118) But-3-yn-1-yl {6-[({[(Z)-(1-methyl-1H-tetrazol-5-yl)(phenyl)methylene]amino}oxy)methyl]pyridin-2-yl}carbamat, (15.119) 4-Amino-5-fluorpyrimidin-2-ol (Tautomere Form: 4-Amino-5-fluorpyrimidin-2(1H)-on), (15.120) Propyl 3,4,5-trihydroxybenzoat, (15.121) 1,3-Dimethyl-N-(1,1,3-trimethyl-2,3-dihydro-1H-inden-4-yl)-1H-pyrazol-4-carboxamid, (15.122) 1,3-Dimethyl-N-[(3R)-1,1,3-trimethyl-2,3-dihydro-1H-inden-4-yl]-1H-pyrazol-4-carboxamid, (15.123) 1,3-Dimethyl-N-[(3S)-1,1,3-trimethyl-2,3-dihydro-1H-inden-4-yl]-1H-pyrazol-4-carboxamid, (15.124) [3-(4-Chlor-2-fluorphenyl)-5-(2,4-difluorphenyl)-1,2-oxazol-4-yl](pyridin-3-yl)methanol, (15.125) (S)-[3-(4-Chlor-2-fluorphenyl)-5-(2,4-difluorphenyl)-1,2-oxazol-4-yl](pyridin-3-yl)methanol, (15.126) (R)-[3-(4-Chlor-2-fluorphenyl)-5-(2,4-difluorphenyl)-1,2-oxazol-4-yl](pyridin-3-yl)methanol, (15.127) 2-{[3-(2-Chlorphenyl)-2-(2,4-difluorphenyl)oxiran-2-yl]methyl}-2,4-dihydro-3H-1,2,4-triazol-3-thion, (15.128) 1-{[3-(2-Chlorphenyl)-2-(2,4-difluorphenyl)oxiran-2-yl]methyl}-1H-1,2,4-triazol-5-yl thiocyanat, (15.129) 5-(Allylsulfanyl)-1-{[3-(2-chlorphenyl)-2-(2,4-difluorphenyl)oxiran-2-yl]methyl}-1H-1,2,4-triazol, (15.130) 2-[1-(2,4-Dichlorphenyl)-5-hydroxy-2,6,6-trimethylheptan-4-yl]-2,4-dihydro-3H-1,2,4-triazol-3-thion, (15.131) 2-{[rel(2R,3S)-3-(2-Chlorphenyl)-2-(2,4-difluorphenyl)oxiran-2-yl]methyl}-2,4-dihydro-3H-l,2,4-triazol-3-thion, (15.132) 2-{[rel(2R,3R)-3-(2-Chlorphenyl)-2-(2,4-difluorphenyl)oxiran-2-yl]methyl}-2,4-dihydro-3H-1,2,4-triazol-3-thion, (15.133) 1-{[rel(2R,3S)-3-(2-Chlorphenyl)-2-(2,4-difluorphenyl)oxiran-2-yl]methyl}-1H-1,2,4-triazol-5-yl thiocyanat, (15.134) 1-{[rel(2R,3R)-3-(2-Chlorphenyl)-2-(2,4-difluorphenyl)oxiran-2-yl]methyl}-1H-1,2,4-triazol-5-yl thiocyanat, (15.135) 5-(Allylsulfanyl)-1-{[rel(2R,3S)-3-(2-chlorphenyl)-2-(2,4-difluorphenyl)oxiran-2-yl]methyl}-1H-1,2,4-triazol, (15.136) 5-(Allylsulfanyl)-1-{[rel(2R,3R)-3-(2-chlorphenyl)-2-(2,4-difluorphenyl)oxiran-2-yl]methyl}-1H-1,2,4-triazol,(15.137)2-[(2S,4S,5S)-1-(2,4-Dichlorphenyl)-5-hydroxy-2,6,6-trimethylheptan-4-yl]-2,4-dihydro-3H-1,2,4-triazol-3-thion, (15.138) 2-[(2R,4S,5S)-1-(2,4-Dichlorphenyl)-5-hydroxy-2,6,6-trimethylheptan-4-yl]-2,4-dihydro-3H-1,2,4-triazol-3-thion, (15.139) 2-[(2R,4R,5R)-1-(2,4-Dichlorphenyl)-5-hydroxy-2,6,6-trimethylheptan-4-yl]-2,4-dihydro-3H-1,2,4-triazol-3-thion, (15.140) 2-[(2S,4R,5R)-1-(2,4-Dichlorphenyl)-5-hydroxy-2,6,6-trimethylheptan-4-yl]-2,4-dihydro-3H-1,2,4-triazol-3-thion, (15.141) 2-[(2S,4S,5R)-1-(2,4-Dichlorphenyl)-5-hydroxy-2,6,6-trimethylheptan-4-yl]-2,4-dihydro-3H-1,2,4-triazol-3-thion, (15.142) 2-[(2R,4S,5R)-1-(2,4-Dichlorphenyl)-5-hydroxy-2,6,6-trimethylheptan-4-yl]-2,4-dihydro-3H-1,2,4-triazol-3-thion, (15.143) 2-[(2R,4R,5S)-1-(2,4-Dichlorphenyl)-5-hydroxy-2,6,6-trimethylheptan-4-yl]-2,4-dihydro-3H-1,2,4-triazol-3-thion, (15.144) 2-[(2S,4R,5S)-1-(2,4-Dichlorphenyl)-5-hydroxy-2,6,6-trimethylheptan-4-yl]-2,4-dihydro-3H-1,2,4-triazol-3-thion, (15.145) 2-Fluor-6-(trifluormethyl)-N-(1,1,3-trimethyl-2,3-dihydro-1H-inden-4-yl)benzamid, (15.146) 2-(6-Benzylpyridin-2-yl)quinazolin, (15.147) 2-[6-(3-Fluor-4-methoxyphenyl)-5-methylpyridin-2-yl]quinazolin, (15.148) 3-(4,4-Difluor-3,3-dimethyl-3,4-dihydroisochinolin-1-yl)chinolin, (15.149) Abscisinsäure, (15.150) 3-(Difluormethyl)-N-methoxy-1-methyl-N-[1-(2,4,6-trichlorphenyl)propan-2-yl]-1H-pyrazol-4-carboxamid, (15.151) N'-[5-Brom-6-(2,3-dihydro-1H-inden-2-yloxy)-2-methylpyridin-3-yl]-N-ethyl-N-methylimidoformamid, (15.152) N'-{5-Brom-6-[1-(3,5-difluorphenyl)ethoxy]-2-methylpyridin-3-yl}-N-ethyl-N-methylimidoformamid, (15.153) N'-{5-Brom-6-[(1R)-1-(3,5-difluorphenyl)ethoxy]-2-methylpyridin-3-yl}-N-ethyl-N-methylimidoformamid, (15.154) N'-{5-Brom-6-[(1S)-1-(3,5-difluorphenyl)ethoxy]-2-methylpyridin-3-yl} -N-ethyl-N-methylimidoformamid, (15.155) N'- {5-Brom-6-[(cis-4-isopropylcyclohexyl)oxy]-2-methylpyridin-3-yl} -N-ethyl-N-methylimidoformamid, (15.156) N'- {5-Brom-6-[(trans-4-isopropylcyclohexyl)oxy]-2-methylpyridin-3-yl}-N-ethyl-N-methylimidoformamid, (15.157) N-Cyclopropyl-3-(difluormethyl)-5-fluor-N-(2-isopropylbenzyl)-1-methyl-1H-pyrazol-4-carboxamid, (15.158) N-Cyclopropyl-N-(2-cyclopropylbenzyl)-3-(difluormethyl)-5-fluor-1-methyl-1H-pyrazol-4-carboxamid, (15.159) N-(2-Tert-butylbenzyl)-N-cyclopropyl-3-(difluormethyl)-5-fluor-1-methyl-1H-pyrazol-4-carboxamid, (15.160) N-(5-Chlor-2-ethylbenzyl)-N-cyclopropyl-3-(difluormethyl)-5-fluor-1-methyl-1H-pyrazol-4-carboxamid, (15.161) N-(5-Chlor-2-isopropylbenzyl)-N-cyclopropyl-3-(difluormethyl)-5-fluor-1-methyl-1H-pyrazol-4-carboxamid, (15.162) N-Cyclopropyl-3-(difluormethyl)-N-(2-ethyl-5-fluorbenzyl)-5-fluor-1 -methyl-1H-pyrazol-4-carboxamid, (15.163) N-Cyclopropyl-3-(difluormethyl)-5-fluor-N-(5-fluor-2-isopropylbenzyl)-1 -methyl-1H-pyrazol-4-carboxamid, (15.164) N-Cyclopropyl-N-(2-cyclopropyl-5-fluorbenzyl)-3-(difluormethyl)-5-fluor-1-methyl-1H-pyrazol-4-carboxamid, (15.165) N-(2-Cyclopentyl-5-fluorbenzyl)-N-cyclopropyl-3-(difluormethyl)-5-fluor-1-methyl-1H-pyrazol-4-carboxamid, (15.166) N-Cyclopropyl-3-(difluormethyl)-5-fluor-N-(2-fluor-6-isopropylbenzyl)-1 -methyl-1H-pyrazol-4-carboxamid, (15.167) N-Cyclopropyl-3 -(difluormethyl)-N-(2-ethyl-5-methylbenzyl)-5-fluor-1-methyl-1H-pyrazol-4-carboxamid, (15.168) N-Cyclopropyl-3-(difluormethyl)-5-fluor-N-(2-isopropyl-5-methylbenzyl)-1-methyl-1H-pyrazol-4-carboxamid, (15.169) N-Cyclopropyl-N-(2-cyclopropyl-5-methylbenzyl)-3-(difluormethyl)-5-fluor-1-methyl-1H-pyrazol-4-carboxamid, (15.170) N-(2-Tert-butyl-5-methylbenzyl)-N-cyclopropyl-3-(difluormethyl)-5-fluor-1-methyl-1H-pyrazol-4-carboxamid, (15.171) N-[5-Chlor-2-(trifluormethyl)benzyl]-N-cyclopropyl-3-(difluormethyl)-5-fluor-1-methyl-1H-pyrazol-4-carboxamid, (15.172) N-Cyclopropyl-3-(difluormethyl)-5-fluor-1-methyl-N-[5-methyl-2-(trifluormethyl)benzyl]-1H-pyrazol-4-carboxamid, (15.173) N-[2-Chlor-6-(trifluormethyl)benzyl]-N-cyclopropyl-3-(difluormethyl)-5-fluor-1-methyl-1H-pyrazol-4-carboxamid, (15.174) N-[3-Chlor-2-fluor-6-(trifluormethyl)benzyl]-N-cyclopropyl-3-(difluormethyl)-5-fluor-1-methyl-1H-pyrazol-4-carboxamid, (15.175) N-Cyclopropyl-3 -(difluormethyl)-N-(2-ethyl-4,5-dimethylbenzyl)-5-fluor-1-methyl-1H-pyrazol-4-carboxamid, (15.176) N-Cyclopropyl-3-(difluormethyl)-5-fluor-N-(2-isopropylbenzyl)-1-methyl-1H-pyrazol-4-carbothioamid, (15.177) 3-(Difluormethyl)-N-(7-fluor-1,1,3-trimethyl-2,3-dihydro-1H-inden-4-yl)-1-methyl-1H-pyrazol-4-carboxamid, (15.178) 3-(Difluormethyl)-N-[(3R)-7-fluor-1,1,3-trimethyl-2,3-dihydro-1H-inden-4-yl]-1-methyl-1H-pyrazol-4-carboxamid, (15.179) 3-(Difluormethyl)-N-[(3S)-7-fluor-1,1,3-trimethyl-2,3-dihydro-1H-inden-4-yl]-1-methyl-1H-pyrazol-4-carboxamid, (15.180) N'-(2,5-Dimethyl-4-phenoxyphenyl)-N-ethyl-N-methylimidoformamid, (15.181) N'-{4-[(4,5-Dichlor-1,3-thiazol-2-yl)oxy]-2,5-dimethylphenyl}-N-ethyl-N-methylimidoformamid, (15.182) N-(4-Chlor-2,6-difluorphenyl)-4-(2-chlor-4-fluorphenyl)-1,3-dimethyl-1H-pyrazol-5-amin. Alle genannten Mischpartner der Klassen (1) bis (15) können, wenn sie auf Grund ihrer funktionellen Gruppen dazu imstande sind, gegebenenfalls mit geeigneten Basen oder Säuren Salze bilden.

### Biologische Schädlingsbekämpfungsmittel als Mischungspartner

Die Verbindungen der Formel (Ia") können mit biologischen Schädlingsbekämpfungsmitteln kombiniert werden.

Biologische Schädlingsbekämpfungsmittel umfassen insbesondere Bakterien, Pilze, Hefen, Pflanzenextrakte, und solche Produkte, die von Mikroorganismen gebildet wurden inklusive Proteine und sekundäre Stoffwechselprodukte.

Biologische Schädlingsbekämpfungsmittel umfassen Bakterien wie sporenbildende Bakterien, wurzelbesiedelnde Bakterien und Bakterien, die als biologische Insektizide, Fungizide oder Nematizide wirken.

Beispiele für solche Bakterien, die als biologische Schädlingsbekämpfungsmittel eingesetzt werden bzw. verwendet werden können, sind:
*Bacillus amyloliquefaciens,* Stamm FZB42 (DSM 231179), oder *Bacillus cereus,* insbesondere *B. cereus* Stamm CNCM I-1562 oder *Bacillus firmus,* Stamm I-1582 (Accession number CNCM I-1582) oder *Bacillus pumilus,* insbesondere Stamm GB34 (Accession No. ATCC 700814), und Stamm QST2808 (Accession No. NRRL B-30087), oder *Bacillus subtilis,* insbesondere Stamm GB03 (Accession No. ATCC SD-1397), oder *Bacillus subtilis* Stamm QST713 (Accession No. NRRL B-21661) oder *Bacillus subtilis* Stamm OST 30002 (Accession No. NRRL B-50421) *Bacillus thuringiensis,* insbesondere *B. thuringiensis* subspecies *israelensis* (serotype H-14), Stamm AM65-52 (Accession No. ATCC 1276), oder *B. thuringiensis subsp. aizawai,* insbesondere Stamm ABTS-1857 (SD-1372), oder *B. thuringiensis subsp. kurstaki* Stamm HD-1, oder *B. thuringiensis subsp. tenebrionis* Stamm NB 176 (SD-5428), *Pasteuria penetrans, Pasteuria spp.* (Rotylenchulus reniformis nematode)-PR3 (Accession Number ATCC SD-5834), *Streptomyces microflavus* Stamm AQ6121 (= QRD 31.013, NRRL B-50550), *Streptomyces galbus* Stamm AQ 6047 (Acession Number NRRL 30232).

Beispiele für Pilze und Hefen, die als biologische Schädlingsbekämpfungsmittel eingesetzt werden bzw. verwendet werden können, sind:
*Beauveria bassiana,* insbesondere Stamm ATCC 74040, *Coniothyrium minitans,* insbesondere Stamm CON/M/91-8 (Accession No. DSM-9660), *Lecanicillium spp.,* insbesondere Stamm HRO LEC 12, *Lecanicillium lecanii,* (ehemals bekannt als *Verticillium lecanii*), insbesondereStamm KV01, *Metarhizium anisopliae,* insbesondere Stamm F52 (DSM3884/ ATCC 90448), *Metschnikowia fructicola,* insbesondere Stamm NRRL Y-30752, *Paecilomyces fumosoroseus* (neu: *Isaria fumosorosea*), insbesondere Stamm IFPC 200613, oder Stamm Apopka 97 (Accesion No. ATCC 20874), *Paecilomyces lilacinus,* insbesondere *P. lilacinus* Stamm 251 (AGAL 89/030550), *Talaromyces flavus,* insbesondere Stamm V117b, *Trichoderma atroviride,* insbesondere Stamm SC1 (Accession Number CBS 122089), *Trichoderma harzianum,* insbesondere *T. harzianum rifai T39.* (Accession Number CNCM I-952).

Beispiele für Viren, die als biologische Schädlingsbekämpfungsmittel eingesetzt werden bzw. verwendet werden können, sind:
*Adoxophyes orana* (Apfelschalenwickler) Granulosevirus (GV), *Cydia pomonella* (Apfelwickler) Granulosevirus (GV), *Helicoverpa armigera* (Baumwollkapselwurm) Nuklear Polyhedrosis Virus (NPV), *Spodoptera exigua* (Zuckerrübeneule) mNPV, *Spodoptera frugiperda* (Heerwurm) mNPV, *Spodoptera littoralis* (Afrikanischer Baumwollwurm) NPV.

Es sind auch Bakterien und Pilze umfasst, die als 'Inokulant' Pflanzen oder Pflanzenteilen oder Pflanzenorganen beigegeben werden und durch ihre besonderen Eigenschaften das Pflanzenwachstum und die Pflanzengesundheit fördern. Als Beispiele sind genannt:
*Agrobacterium spp., Azorhizobium caulinodans, Azospirillum spp., Azotobacter spp., Bradyrhizobium spp., Burkholderia spp.,* insbesondere *Burkholderia cepacia* (ehemals bekannt als *Pseudomonas cepacia*), *Gigaspora spp.,* oder *Gigaspora monosporum, Glomus spp., Laccaria spp., Lactobacillus buchneri, Paraglomus spp., Pisolithus tinctorus, Pseudomonas spp., Rhizobium spp.,* insbesondere *Rhizobium trifolii, Rhizopogon spp., Scleroderma spp., Suillus spp., Streptomyces spp..*

### Safener als Mischpartner

Die Verbindungen der Formel (Ia") können mit Safenern kombiniert werden, wie zum Beispiel Benoxacor, Cloquintocet (-mexyl), Cyometrinil, Cyprosulfamide, Dichlormid, Fenchlorazole (-ethyl), Fenclorim, Flurazole, Fluxofenim, Furilazole, Isoxadifen (-ethyl), Mefenpyr (-diethyl), Naphthalic anhydride, Oxabetrinil, 2-Methoxy-N-({4-[(methylcarbamoyl)amino]phenyl}sulfonyl)benzamid (CAS 129531-12-0), 4-(Dichloroacetyl)-1-oxa-4-azaspiro[4.5]decane der (CAS 71526-07-3), 2,2,5-Trimethyl-3-(dechloroacetyl)-1,3-oxazolidine (CAS 52836-31-4).

### Planzen und Pflanzenteile

Erfindungsgemäß können alle Pflanzen und Pflanzenteile behandelt werden. Unter Pflanzen werden hierbei alle Pflanzen und Pflanzenpopulationen verstanden wie erwünschte und unerwünschte Wildpflanzen oder Kulturpflanzen (einschließlich natürlich vorkommender Kulturpflanzen), beispielsweise Getreide (Weizen, Reis, Triticale, Gerste, Roggen, Hafer), Mais, Soja, Kartoffel, Zuckerrüben, Zuckerrohr, Tomaten, Erbsen und andere Gemüsesorten, Baumwolle, Tabak, Raps, sowie Obstpflanzen (mit den Früchten Äpfel, Birnen, Zitrusfrüchten und Weintrauben). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Verbindungen der Formel (Ia") erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen, Injizieren und bei Vermehrungsmaterial, insbesondere bei Saatgut, weiterhin durch ein- oder mehrschichtiges Umhüllen.

Wie bereits oben erwähnt können erfindungsgemäß alle Pflanzen und deren Teile behandelt werden. In einer bevorzugten Ausführungsform werden wild vorkommende oder durch konventionelle biologische Zuchtmethoden wie Kreuzung oder Protoplastenfusion erhaltene Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Der Begriff "Teile" bzw. "Teile von Pflanzen" oder "Pflanzenteile" wurde oben erläutert. Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Rassen, Bio- und Genotypen sein.

### Transgene Pflanze, Saatgutbehandlung und Integrationsereignisse

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehrfähigkeit der Pflanzen gegen tierische und mikrobielle Schädlinge, wie Insekten, Spinnentiere, Nematoden, Milben, Schnecken, bewirkt z.B. durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden, ferner eine erhöhte Abwehrfähigkeit der Pflanzen gegen pflanzenpathogene Pilze, Bakterien und/oder Viren, bewirkt z.B. durch Systemisch Akquirierte Resistenz (SAR), Systemin, Phytoalexine, Elicitoren sowie Resistenzgene und entsprechend exprimierte Proteine und Toxine, sowie eine erhöhte Toleranz der Pflanzen gegen bestimmte herbizide Wirkstoffe, beispielsweise Imidazolinonen, Sulfonyl-harnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele transgener Pflanzen werden die wichtigen Kulturpflanzen, wie Getreide (Weizen, Reis, Triticale, Gerste, Roggen, Hafer), Mais, Soja, Kartoffel, Zuckerrüben, Zuckerrohr, Tomaten, Erbsen und andere Gemüsesorten, Baumwolle, Tabak, Raps, sowie Obstpflanzen (mit den Früchten Äpfel, Birnen, Zitrusfrüchten und Weintrauben) erwähnt, wobei Mais, Soja, Weizen, Reis, Kartoffel, Baumwolle, Zuckerrohr, Tabak und Raps besonders hervorgehoben werden. Als Eigenschaften ("Traits") werden besonders hervorgehoben die erhöhte Abwehrfähigkeit der Pflanzen gegen Insekten, Spinnentiere, Nematoden und Schnecken.

### Pflanzenschutz - Behandlungsarten

Die Behandlung der Pflanzen und Pflanzenteile mit den Verbindungen der Formel (Ia") erfolgt direkt oder durch Einwirkung auf deren Umgebung, Lebensraum oder Lagerraum nach den üblichen Behandlungsmethoden, z.B. durch Tauchen, (Ver-) Spritzen, (Ver-)Sprühen, Berieseln, Verdampfen, Zerstäuben, Vernebeln, (Ver-)Streuen, Verschäumen, Bestreichen, Verstreichen, Injizieren, Gießen (drenchen), Tröpfchenbewässerung und bei Vermehrungsmaterial, insbesondere bei Saatgut, weiterhin durch Trockenbeizen, Nassbeizen, Schlämmbeizen, Inkrustieren, ein- oder mehrschichtiges Umhüllen, usw. Es ist ferner möglich, die Verbindungen der Formel (Ia") nach dem Ultra-Low-Volume-Verfahren auszubringen oder die Anwendungsform oder die Verbindung der Formel (Ia") selbst in den Boden zu injizieren.

Eine bevorzugte direkte Behandlung der Pflanzen ist die Blattapplikation, d.h. Verbindungen der Formel (Ia") werden auf das Blattwerk aufgebracht, wobei die Behandlungsfrequenz und die Aufwandmenge auf den Befallsdruck des jeweiligen Schädlings abgestimmt sein sollte.

Bei systemisch wirksamen Verbindungen gelangen die Verbindungen der Formel (Ia") auch über das Wurzelwerk in die Pflanzen. Die Behandlung der Pflanzen erfolgt dann durch Einwirkung der Verbindungen der Formel (Ia") auf den Lebensraum der Pflanze. Das kann beispielsweise durch Drenchen, Einmischen in den Boden oder die Nährlösung sein, d.h. der Standort der Pflanze (z.B. Boden oder hydroponische Systeme) wird mit einer flüssigen Form der Verbindungen der Formel (Ia") getränkt, oder durch die Bodenapplikation, d.h. die Verbindungen der Formel (Ia") werden in fester Form, (z.B. in Form eines Granulats) in den Standort der Pflanzen eingebracht. Bei Wasserreiskulturen kann das auch durch Zudosieren der Verbindung der Formel (Ia") in einer festen Anwendungsform (z.B. als Granulat) in ein überflutetes Reisfeld sein.

### Saatgutbehandlung

Die Bekämpfung von tierischen Schädlingen durch die Behandlung des Saatguts von Pflanzen ist seit langem bekannt und ist Gegenstand ständiger Verbesserungen. Dennoch ergeben sich bei der Behandlung von Saatgut eine Reihe von Problemen, die nicht immer zufriedenstellend gelöst werden können. So ist es erstrebenswert, Verfahren zum Schutz des Saatguts und der keimenden Pflanze zu entwickeln, die das zusätzliche Ausbringen von Schädlingsbekämpfungsmitteln bei der Lagerung, nach der Saat oder nach dem Auflaufen der Pflanzen überflüssig machen oder zumindest deutlich verringern. Es ist weiterhin erstrebenswert, die Menge des eingesetzten Wirkstoffs dahingehend zu optimieren, dass das Saatgut und die keimende Pflanze vor dem Befall durch tierische Schädlinge bestmöglich geschützt werden, ohne jedoch die Pflanze selbst durch den eingesetzten Wirkstoff zu schädigen. Insbesondere sollten Verfahren zur Behandlung von Saatgut auch die intrinsischen insektiziden bzw. nematiziden Eigenschaften schädlingsresistenter bzw. - toleranter transgener Pflanzen einbeziehen, um einen optimalen Schutz des Saatguts und der keimenden Pflanze bei einem minimalen Aufwand an Schädlingsbekämpfungsmitteln zu erreichen.

Die vorliegende Erfindung bezieht sich daher insbesondere auch auf ein Verfahren zum Schutz von Saatgut und keimenden Pflanzen vor dem Befall von Schädlingen, indem das Saatgut mit einer der Verbindungen der Formel (Ia") behandelt wird. Das erfindungsgemäße Verfahren zum Schutz von Saatgut und keimenden Pflanzen vor dem Befall von Schädlingen umfasst ferner ein Verfahren, in dem das Saatgut gleichzeitig in einem Vorgang oder sequentiell mit einer Verbindung der Formel (Ia") und Mischungspartner behandelt wird. Es umfasst ferner auch ein Verfahren, in dem das Saatgut zu unterschiedlichen Zeiten mit einer Verbindung der Formel (Ia") und Mischungspartner behandelt wird.

Die Erfindung bezieht sich ebenfalls auf die Verwendung der Verbindungen der Formel (Ia") zur Behandlung von Saatgut zum Schutz des Saatguts und der daraus entstehenden Pflanze vor tierischen Schädlingen.

Weiterhin bezieht sich die Erfindung auf Saatgut, welches zum Schutz vor tierischen Schädlingen mit einer Verbindung der Formel (Ia") behandelt wurde. Die Erfindung bezieht sich auch auf Saatgut, welches zur gleichen Zeit mit einer Verbindung der Formel (Ia") und Mischungspartner behandelt wurde. Die Erfindung bezieht sich weiterhin auf Saatgut, welches zu unterschiedlichen Zeiten mit einer Verbindung der Formel (Ia") und Mischungspartner behandelt wurde. Bei Saatgut, welches zu unterschiedlichen Zeiten mit einer Verbindung der Formel (Ia") und Mischungspartner behandelt wurde, können die einzelnen Substanzen in unterschiedlichen Schichten auf dem Saatgut enthalten sein. Dabei können die Schichten, die eine Verbindung der Formel (Ia") und Mischungspartner enthalten, gegebenenfalls durch eine Zwischenschicht getrennt sein. Die Erfindung bezieht sich auch auf Saatgut, bei dem eine Verbindung der Formel (Ia") und Mischungspartner als Bestandteil einer Umhüllung oder als weitere Schicht oder weitere Schichten zusätzlich zu einer Umhüllung aufgebracht sind.

Des Weiteren bezieht sich die Erfindung auf Saatgut, welches nach der Behandlung mit einer Verbindung der Formel (Ia") einem Filmcoating - Verfahren unterzogen wird, um Staubabrieb am Saatgut zu vermeiden.

Einer der auftretenden Vorteile, wenn eine der Verbindungen der Formel (Ia") systemisch wirkt, ist es, dass die Behandlung des Saatguts nicht nur das Saatgut selbst, sondern auch die daraus hervorgehenden Pflanzen nach dem Auflaufen vor tierischen Schädlingen schützt. Auf diese Weise kann die unmittelbare Behandlung der Kultur zum Zeitpunkt der Aussaat oder kurz danach entfallen.

Ein weiterer Vorteil ist darin zu sehen, dass durch die Behandlung des Saatguts mit einer Verbindung der Formel (Ia") Keimung und Auflauf des behandelten Saatguts gefördert werden können.

Ebenso ist es als vorteilhaft anzusehen, dass Verbindungen der Formel (Ia") insbesondere auch bei transgenem Saatgut eingesetzt werden können.

Verbindungen der Formel (Ia") können ferner in Kombination mit Mitteln der Signaltechnologie eingesetzt werden, wodurch eine bessere Besiedlung mit Symbionten, wie zum Beispiel Rhizobien, Mycorrhiza und/oder endophytischen Bakterien oder Pilzen, stattfindet und/oder es zu einer optimierten Stickstofffixierung kommt.

Die Verbindungen der Formel (Ia") eignen sich zum Schutz von Saatgut jeglicher Pflanzensorte, die in der Landwirtschaft, im Gewächshaus, in Forsten oder im Gartenbau eingesetzt wird. Insbesondere handelt es sich dabei um Saatgut von Getreide (z. B. Weizen, Gerste, Roggen, Hirse und Hafer), Mais, Baum-wolle, Soja, Reis, Kartoffeln, Sonnenblume, Kaffee, Tabak, Canola, Raps, Rübe (z.B. Zuckerrübe und Futterrübe), Erdnuss, Gemüse (z. B. Tomate, Gurke, Bohne, Kohlgewächse, Zwiebeln und Salat), Obstpflanzen, Rasen und Zierpflanzen. Besondere Bedeutung kommt der Behandlung des Saatguts von Getreide (wie Weizen, Gerste, Roggen und Hafer), Mais, Soja, Baumwolle, Canola, Raps und Reis zu.

Wie vorstehend bereits erwähnt, kommt auch der Behandlung von transgenem Saatgut mit einer Verbindung der Formel (Ia") eine besondere Bedeutung zu. Dabei handelt es sich um das Saatgut von Pflanzen, die in der Regel zumindest ein heterologes Gen enthalten, das die Expression eines Polypeptids mit insbesondere insektiziden bzw. nematiziden Eigenschaften steuert. Die heterologen Gene in transgenem Saatgut können dabei aus Mikro-organismen wie Bacillus, Rhizobium, Pseudomonas, Serratia, Trichoderma, Clavibacter, Glomus oder Gliocladium stammen. Die vorliegende Erfindung eignet sich besonders für die Behandlung von trans-genem Saatgut, das zumindest ein heterologes Gen enthält, das aus Bacillus sp. stammt. Besonders bevorzugt handelt es sich dabei um ein heterologes Gen, das aus Bacillus thuringiensis stammt.

Im Rahmen der vorliegenden Erfindung wird die Verbindung der Formel (Ia") auf das Saatgut aufgebracht. Vorzugsweise wird das Saatgut in einem Zustand behandelt, in dem es so stabil ist, dass keine Schäden bei der Behandlung auftreten. Im Allgemeinen kann die Behandlung des Saatguts zu jedem Zeitpunkt zwischen der Ernte und der Aussaat erfolgen. Üblicherweise wird Saatgut verwendet, das von der Pflanze getrennt und von Kolben, Schalen, Stängeln, Hülle, Wolle oder Fruchtfleisch befreit wurde. So kann zum Beispiel Saatgut verwendet werden, das geerntet, gereinigt und bis zu einem lagerfähigen Feuchtigkeitsgehalt getrocknet wurde. Alternativ kann auch Saatgut verwendet werden, das nach dem Trocknen z.B. mit Wasser behandelt und dann erneut getrocknet wurde, zum Beispiel Priming.

Im Allgemeinen muss bei der Behandlung des Saatguts darauf geachtet werden, dass die Menge der auf das Saatgut aufgebrachten Verbindung der Formel (Ia") und/oder weiterer Zusatzstoffe so gewählt wird, dass die Keimung des Saatguts nicht beeinträchtigt bzw. die daraus hervorgehende Pflanze nicht geschädigt wird. Dies ist vor allem bei Wirkstoffen zu beachten, die in bestimmten Aufwandmengen phytotoxische Effekte zeigen können.

Die Verbindungen der Formel (Ia") werden in der Regel in Form einer geeigneten Formulierung auf das Saatgut aufgebracht. Geeignete Formulierungen und Verfahren für die Saatgutbehandlung sind dem Fachmann bekannt.

Die Verbindungen der Formel (Ia") können in die üblichen Beizmittel-Formulierungen überführt werden, wie Lösungen, Emulsionen, Suspensionen, Pulver, Schäume, Slurries oder andere Hüllmassen für Saatgut, sowie ULV-Formulierungen.

Diese Formulierungen werden in bekannter Weise hergestellt, indem man Verbindungen der Formel (Ia") mit üblichen Zusatzstoffen vermischt, wie zum Beispiel übliche Streckmittel sowie Lösungs- oder Verdünnungsmittel, Farbstoffe, Netzmittel, Dispergiermittel, Emulgatoren, Entschäumer, Konservierungsmittel, sekundäre Verdickungsmittel, Kleber, Gibberelline und auch Wasser.

Als Farbstoffe, die in den erfindungsgemäß verwendbaren Beizmittel-Formulierungen enthalten sein können, kommen alle für derartige Zwecke üblichen Farbstoffe in Betracht. Dabei sind sowohl in Wasser wenig lösliche Pigmente als auch in Wasser lösliche Farbstoffe verwendbar. Als Beispiele genannt seien die unter den Bezeichnungen Rhodamin B, C.I. Pigment Red 112 und C.I. Solvent Red 1 bekannten Farbstoffe.

Als Netzmittel, die in den erfindungsgemäß verwendbaren Beizmittel-Formulierungen enthalten sein können, kommen alle zur Formulierung von agrochemischen Wirkstoffen üblichen, die Benetzung fördernden Stoffe in Frage. Vorzugsweise verwendbar sind Alkylnaphthalin-Sulfonate, wie Diisopropyl- oder Diisobutyl-naphthalin-Sulfonate.

Als Dispergiermittel und/oder Emulgatoren, die in den erfindungsgemäß verwendbaren Beizmittel-Formulierungen enthalten sein können, kommen alle zur Formulierung von agrochemischen Wirkstoffen üblichen nichtionischen, anionischen und kationischen Dispergiermittel in Betracht. Vorzugsweise verwendbar sind nichtionische oder anionische Dispergiermittel oder Gemische von nichtionischen oder anionischen Dispergiermitteln. Als geeignete nichtionische Dispergiermittel sind insbesondere Ethylenoxid-Propylenoxid Blockpolymere, Alkylphenolpolyglykolether sowie Tristryrylphenolpolyglykolether und deren phosphatierte oder sulfatierte Derivate zu nennen. Geeignete anionische Dispergiermittel sind insbesondere Ligninsulfonate, Polyacrylsäuresalze und Arylsulfonat-Formaldehydkondensate.

Als Entschäumer können in den erfindungsgemäß verwendbaren Beizmittel-Formulierungen alle zur Formulierung von agrochemischen Wirkstoffen üblichen schaumhemmenden Stoffe enthalten sein. Vorzugsweise verwendbar sind Silikonentschäumer und Magnesiumstearat.

Als Konservierungsmittel können in den erfindungsgemäß verwendbaren Beizmittel-Formulierungen alle für derartige Zwecke in agrochemischen Mitteln einsetzbaren Stoffe vorhanden sein. Beispielhaft genannt seien Dichlorophen und Benzylalkoholhemiformal.

Als sekundäre Verdickungsmittel, die in den erfindungsgemäß verwendbaren Beizmittel-Formulierungen enthalten sein können, kommen alle für derartige Zwecke in agrochemischen Mitteln einsetzbaren Stoffe in Frage. Vorzugsweise in Betracht kommen Cellulosederivate, Acrylsäurederivate, Xanthan, modifizierte Tone und hochdisperse Kieselsäure.

Als Kleber, die in den erfindungsgemäß verwendbaren Beizmittel-Formulierungen enthalten sein können, kommen alle üblichen in Beizmitteln einsetzbaren Bindemittel in Frage. Vorzugsweise genannt seien Polyvinylpyrrolidon, Polyvinylacetat, Polyvinylalkohol und Tylose.

Als Gibberelline, die in den erfindungsgemäß verwendbaren Beizmittel-Formulierungen enthalten sein können, kommen vorzugsweise die Gibberelline A1, A3 (= Gibberellinsäure), A4 und A7 infrage, be-sonders bevorzugt verwendet man die Gibberellinsäure. Die Gibberelline sind bekannt (vgl. R. Wegler "Chemie der Pflanzenschutz- und Schädlingsbekämpfungsmittel", Bd. 2, Springer Verlag, 1970, S. 401-412).

Die erfindungsgemäß verwendbaren Beizmittel-Formulierungen können entweder direkt oder nach vorherigem Verdünnen mit Wasser zur Behandlung von Saatgut der verschiedensten Art eingesetzt werden. So lassen sich die Konzentrate oder die daraus durch Verdünnen mit Wasser erhältlichen Zubereitungen einsetzen zur Beizung des Saatgutes von Getreide, wie Weizen, Gerste, Roggen, Hafer und Triticale, sowie des Saatgutes von Mais, Reis, Raps, Erbsen, Bohnen, Baumwolle, Sonnenblumen, Soja und Rüben oder auch von Gemüsesaatgut der verschiedensten Natur. Die erfindungsgemäß verwendbaren Beizmittel-Formulierungen oder deren verdünnte Anwendungsformen können auch zum Beizen von Saatgut transgener Pflanzen eingesetzt werden.

Zur Behandlung von Saatgut mit den erfindungsgemäß verwendbaren Beizmittel-Formulierungen oder daraus hergestellten Anwendungsformen kommen alle üblicherweise für die Beizung einsetzbaren Mischgeräte in Betracht. Im einzelnen geht man bei der Beizung so vor, dass man das Saatgut in einen Mischer im diskontinuierlichem oder kontinuierlichem Betrieb gibt, die jeweils gewünschte Menge an Beizmittel-Formulierungen entweder als solche oder nach vorherigem Verdünnen mit Wasser hinzufügt und bis zur gleichmäßigen Verteilung der Formulierung auf dem Saatgut mischt. Gegebenenfalls schließt sich ein Trocknungsvorgang an.

Die Aufwandmenge an den erfindungsgemäß verwendbaren Beizmittel-Formulierungen kann inner-halb eines größeren Bereiches variiert werden. Sie richtet sich nach dem jeweiligen Gehalt der Verbindungen der Formel (Ia") in den Formulierungen und nach dem Saatgut. Die Aufwandmengen bei der Verbindung der Formel (Ia") liegen im Allgemeinen zwischen 0,001 und 50 g pro Kilogramm Saatgut, vorzugsweise zwischen 0,01 und 15 g pro Kilogramm Saatgut.

### Verwendung Animal Health (nicht-erfindungsgemäss)

Im Bereich Tiergesundheit, d.h. auf dem veterinärmedizinischen Gebiet, wirken die Wirkstoffe gemäß der vorliegenden Erfindung gegen tierische Parasiten, insbesondere Ektoparasiten oder, in einer weiteren Ausführungsform auch Endoparasiten. Der Begriff Endoparasiten schließt insbesondere Helminthen wie Cestoden, Nematoden oder Trematoden, und Protozoen wie Kokzidien ein. Ektoparasiten sind typischerweise und vorzugsweise Arthropoden, insbesondere Insekten wie Fliegen (stechend und leckend), parasitische Fliegenlarven, Läuse, Haarlinge, Federlinge, Flöhe und dergleichen; oder Akariden wie Zecken, zum Beispiel Schildzecken oder Lederzecken, oder Milben wie Räudemilben, Laufmilben, Federmilben und dergleichen sowie aquatische Ektoparasiten wie Copepoden.

Auf dem Gebiet der Tiermedizin eignen sich die Verbindungen der Formel (Ia"), die eine günstige Toxizität gegenüber Warmblütern aufweisen, für die Bekämpfung von Parasiten, die in der Tierzucht und Tierhaltung bei Nutztieren, Zuchttieren, Zootieren, Laboratoriumstieren, Versuchstieren und Haustieren auftreten. Sie sind gegen alle oder einzelne Entwicklungsstadien der Parasiten wirksam.

Zu den landwirtschaftlichen Nutztieren zählen zum Beispiel Säugetiere wie Schafe, Ziegen, Pferde, Esel, Kamele, Büffel, Kaninchen, Rentiere, Damhirsche und insbesondere Rinder und Schweine; Geflügel wie Truthähne, Enten, Gänse und insbesondere Hühner; Fische und Krustentiere, z.B. in der Aquakultur und auch Insekten wie Bienen.

Zu den Haustieren zählen zum Beispiel Säugetiere wie Hamster, Meerschweinchen, Ratten, Mäuse, Chinchillas, Frettchen und insbesondere Hunde, Katzen, Stubenvögel, Reptilien, Amphibien und Aquariumfische.

Gemäß einer bevorzugten Ausführungsform werden die Verbindungen der Formel (Ia") an Säugetiere verabreicht.

Gemäß einer weiteren bevorzugten Ausführungsform werden die Verbindungen der Formel (Ia") an Vögel, nämlich Stubenvögel und insbesondere Geflügel, verabreicht.

Durch Verwendung der Verbindungen der Formel (Ia") für die Bekämpfung von Tierparasiten sollen Krankheit, Todesfälle und Leistungsminderungen (bei Fleisch, Milch, Wolle, Häuten, Eiern, Honig und dergleichen) verringert bzw. vorgebeugt werden, so dass eine wirtschaftlichere und einfachere Tierhaltung ermöglicht wird und ein besseres Wohlbefinden der Tiere erzielbar ist.

In Bezug auf das Gebiet der Tiergesundheit bedeutet der Begriff "Bekämpfung" oder "bekämpfen", dass durch die Verbindungen der Formel (Ia") wirksam das Auftreten des jeweiligen Parasiten in einem Tier, das mit solchen Parasiten in einem harmlosen Ausmaß infiziert ist, reduziert werden kann. Genauer gesagt bedeutet "bekämpfen" im vorliegenden Zusammenhang, dass die Verbindung der Formel (Ia") den jeweiligen Parasiten abtöten, sein Wachstum verhindern oder seine Vermehrung verhindern kann.

### Zu diesen Parasiten gehören:

Aus der Ordnung der Anoplurida z.B. Haematopinus spp., Linognathus spp., Pediculus spp., Phthirus spp., Solenopotes spp.; spezielle Beispiele sind: Linognathus setosus, Linognathus vituli, Linognathus ovillus, Linognathus oviformis, Linognathus pedalis, Linognathus stenopsis, Haematopinus asini macrocephalus, Haematopinus eurysternus, Haematopinus suis, Pediculus humanus capitis, Pediculus humanus corporis, Phylloera vastatrix, Phthirus pubis, Solenopotes capillatus;

Aus der Ordnung der Mallophagida und den Unterordnungen Amblycerina und Ischnocerina z.B. Trimenopon spp., Menopon spp., Trinoton spp., Bovicola spp., Werneckiella spp., Lepikentron spp., Damalina spp., Trichodectes spp., Felicola spp.; spezielle Beispiele sind: Bovicola bovis, Bovicola ovis, Bovicola limbata, Damalina bovis, Trichodectes canis, Felicola subrostratus, Bovicola caprae, Lepikentron ovis, Werneckiella equi;

Aus der Ordnung der Diptera und den Unterordnungen Nematocerina und Brachycerina z.B. Aedes spp., Anopheles spp., Culex spp., Simulium spp., Eusimulium spp., Phlebotomus spp., Lutzomyia spp., Culicoides spp., Chrysops spp., Odagmia spp., Wilhelmia spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp., Rhinoestrus spp., Tipula spp.; spezielle Beispiele sind: Aedes aegypti, Aedes albopictus, Aedes taeniorhynchus, Anopheles gambiae, Anopheles maculipennis, Calliphora erythrocephala, Chrysozona pluvialis, Culex quinquefasciatus, Culex pipiens, Culex tarsalis, Fannia canicularis, Sarcophaga carnaria, Stomoxys calcitrans, Tipula paludosa, Lucilia cuprina, Lucilia sericata, Simulium reptans, Phlebotomus papatasi, Phlebotomus longipalpis, Odagmia ornata, Wilhelmia equina, Boophthora erythrocephala, Tabanus bromius, Tabanus spodopterus, Tabanus atratus, Tabanus sudeticus, Hybomitra ciurea, Chrysops caecutiens, Chrysops relictus, Haematopota pluvialis, Haematopota italica, Musca autumnalis, Musca domestica, Haematobia irritans irritans, Haematobia irritans exigua, Haematobia stimulans, Hydrotaea irritans, Hydrotaea albipuncta, Chrysomya Chloropyga, Chrysomya bezziana, Oestrus ovis, Hypoderma bovis, Hypoderma lineatum, Przhevalskiana silenus, Dermatobia hominis, Melophagus ovinus, Lipoptena capreoli, Lipoptena cervi, Hippobosca variegata, Hippobosca equina, Gasterophilus intestinalis, Gasterophilus haemorroidalis, Gasterophilus inermis, Gasterophilus nasalis, Gasterophilus nigricornis, Gasterophilus pecorum, Braula coeca;

Aus der Ordnung der Siphonapterida z.B. Pulex spp., Ctenocephalides spp., Tunga spp., Xenopsylla spp., Ceratophyllus spp.; spezielle Beispiele sind: Ctenocephalides canis, Ctenocephalides felis, Pulex irritans, Tunga penetrans, Xenopsylla cheopis;

Aus der Ordnung der Heteropterida z.B. Cimex spp., Triatoma spp., Rhodnius spp., Panstrongylus spp.

Aus der Ordnung der Blattarida z.B. Blatta orientalis, Periplaneta americana, Blattela germanica, Supella spp. (z.B. Suppella longipalpa);

Aus der Unterklasse der Acari (Acarina) und den Ordnungen der Meta- und Mesostigmata z.B. Argas spp., Ornithodorus spp., Otobius spp., Ixodes spp., Amblyomma spp., Rhipicephalus (Boophilus) spp., Dermacentor spp., Haemophysalis spp., Hyalomma spp., Dermanyssus spp., Rhipicephalus spp. (der ursprünglichen Gattung der Mehrwirtszecken), Ornithonyssus spp., Pneumonyssus spp., Raillietia spp., Pneumonyssus spp., Stemostoma spp., Varroa spp., Acarapis spp.; spezielle Beispiele sind: Argas persicus, Argas reflexus, Ornithodorus moubata, Otobius megnini, Rhipicephalus (Boophilus) microplus, Rhipicephalus (Boophilus) decoloratus, Rhipicephalus (Boophilus) annulatus, Rhipicephalus (Boophilus) calceratus, Hyalomma anatolicum, Hyalomma aegypticum, Hyalomma marginatum, Hyalomma transiens, Rhipicephalus evertsi, Ixodes ricinus, Ixodes hexagonus, Ixodes canisuga, Ixodes pilosus, Ixodes rubicundus, Ixodes scapularis, Ixodes holocyclus, Haemaphysalis concinna, Haemaphysalis punctata, Haemaphysalis cinnabarina, Haemaphysalis otophila, Haemaphysalis leachi, Haemaphysalis longicorni, Dermacentor marginatus, Dermacentor reticulatus, Dermacentor pictus, Dermacentor albipictus, Dermacentor andersoni, Dermacentor variabilis, Hyalomma mauritanicum, Rhipicephalus sanguineus, Rhipicephalus bursa, Rhipicephalus appendiculatus, Rhipicephalus capensis, Rhipicephalus turanicus, Rhipicephalus zambeziensis, Amblyomma americanum, Amblyomma variegatum, Amblyomma maculatum, Amblyomma hebraeum, Amblyomma cajennense, Dermanyssus gallinae, Ornithonyssus bursa, Ornithonyssus sylviarum, Varroa jacobsoni;

Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp.; spezielle Beispiele sind: Cheyletiella yasguri, Cheyletiella blakei, Demodex canis, Demodex bovis, Demodex ovis, Demodex caprae, Demodex equi, Demodex caballi, Demodex suis, Neotrombicula autumnalis, Neotrombicula desaleri, Neoschöngastia xerothermobia, Trombicula akamushi, Otodectes cynotis, Notoedres cati, Sarcoptis canis, Sarcoptes bovis, Sarcoptes ovis, Sarcoptes rupicaprae (=S. caprae), Sarcoptes equi, Sarcoptes suis, Psoroptes ovis, Psoroptes cuniculi, Psoroptes equi, Chorioptes bovis, Psoergates ovis, Pneumonyssoidic mange, Pneumonyssoides caninum, Acarapis woodi.

Aus Unterklasse der Copepoden mit der Ordnung der Siphonostomatoida insbesondere die Gattungen Lepeophtheirus und Caligus, beispielhaft und besonders bevorzugt seien die Arten Lepeophtheirus salmonis, Caligus elongatus und Caligus clemensi genannt

Im Allgemeinen können die erfindungsgemäßen Wirkstoffe, wenn sie für die Behandlung von Tieren eingesetzt werden, direkt angewendet werden. Vorzugsweise werden sie als pharmazeutische Zusammensetzungen angewendet, die im Stand der Technik bekannte pharmazeutisch unbedenkliche Exzipienten und/oder Hilfsstoffe enthalten können.

Die Anwendung (= Verabreichung) der Wirkstoffe im Bereich Tiergesundheit und in der Tierhaltung erfolgt in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subkutan, intravenös, intraperitoneal u.a.), Implantate, durch nasale Applikation, durch dermale Applikation in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw. Die Wirkstoffe können als Shampoo oder als geeignete, in Aerosolen oder drucklosen Sprays, z.B. Pumpsprays und Zerstäubersprays, anwendbare, Formulierungen formuliert werden.

Bei der Anwendung für Nutztiere, Geflügel, Haustiere etc. kann man die erfindungsgemäßen Wirkstoffe als Formulierungen (beispielsweise Pulver, Spritzpulver [wettable powders, "WP"], Emulsionen, Emulsionskonzentrate [emulsifiable concentrates ,"EC"], fließfähige Mittel, homogene Lösungen und Suspensionskonzentrate [suspension concentrates, "SC"]), die die Wirkstoffe in einer Menge von 1 bis 80 Gew.-% enthalten, direkt oder nach Verdünnung (z.B. 100- bis 10 000facher Verdünnung) anwenden oder sie als chemisches Bad verwenden.

Beim Einsatz im Bereich Tiergesundheit können die erfindungsgemäßen Wirkstoffe zur Erweiterung des Wirkspektrums in Kombination mit geeigneten Synergisten, Repellentien oder anderen Wirkstoffen wie beispielsweise Akariziden, Insektiziden, Anthelmintika, Mitteln gegen Protozoen, verwendet werden. Potentielle Mischpartner für erfindungsgemäße Verbindungen der Formel (Ia") können bei Anwendungen in der Tiergesundheit eine oder mehrere Verbindungen der Gruppen (In-1) bis (In-25) sein.
(In-1) Acetylcholinesterase (AChE) Inhibitoren, wie beispielsweise Carbamate, z. B. Alanycarb, Aldicarb, Bendiocarb, Benfuracarb, Butocarboxim, Butoxycarboxim, Carbaryl, Carbofuran, Carbosulfan, Ethiofencarb, Fenobucarb, Formetanate, Furathiocarb, Isoprocarb, Methiocarb, Methomyl, Metolcarb, Oxamyl, Pirimicarb, Propoxur, Thiodicarb, Thiofanox, Triazamate, Trimethacarb, XMC und Xylylcarb; besonders bevorzugt seien hier für Anwendungen gegen Ektoparasiten Bendiocarb, Carbaryl, Methomyl, Promacyl und Propoxur genannt; oder
   Organophosphate, z. B. Acephate, Azamethiphos, Azinphos (-methyl, -ethyl), Cadusafos, Chlorethoxyfos, Chlorfenvinphos, Chlormephos, Chlorpyrifos (-methyl), Coumaphos, Cyanophos, Demeton-S-methyl, Diazinon, Dichlorvos/DDVP, Dicrotophos, Dimethoate, Dimethylvinphos, Disulfoton, EPN, Ethion, Ethoprophos, Famphur, Fenamiphos, Fenitrothion, Fenthion, Fosthiazate, Heptenophos, Isofenphos, Isopropyl O-(methoxyaminothio-phosphoryl) salicylat, Isoxathion, Malathion, Mecarbam, Methamidophos, Methidathion, Mevinphos, Monocrotophos, Naled, Omethoate, Oxydemeton-methyl, Parathion (-methyl), Phenthoate, Phorate, Phosalone, Phosmet, Phosphamidon, Phoxim, Pirimiphos (-methyl), Profenofos, Propetamphos, Prothiofos, Pyraclofos, Pyridaphenthion, Quinalphos, Sulfotep, Tebupirimfos, Temephos, Terbufos, Tetrachlorvinphos, Thiometon, Triazophos, Triclorfon und Vamidothion; besonders bevorzugt seien hier für Anwendungen gegen Ektoparasiten Azamethiphos, Chlorfenvinphos, Chlorpyrifos, Coumaphos, Cythioate, Diazinon (Dimpylate), Dichlorvos (DDVP), Dicrotophos, Dimethoate, Ethion (Diethion), Famphur (Famophos), Fenitrothion, Fenthion (MPP), Heptenophos, Malathion, Naled, Phosmet (PMP, Phtalofos) Phoxim, Propetamphos, Temephos, Tetrachlorvinphos (CVMP) und Triclorfon/Metrifonate genannt.
(In-2) GABA-gesteuerte Chlorid-Kanal-Antagonisten, wie beispielsweise Organochlorine, z. B. Bromocyclene, Chlordane und Endosulfan (alpha-), Heptachlor, Lindan, und Toxaphene; besonders bevorzugt seien hier für Anwendungen gegen Ektoparasiten Endosulfan (alpha-) und Lindan genannt; oder
   Fiprole (Phenylpyrazole), z. B. Acetoprole, Ethiprole, Fipronil, Pyrafluprole und Pyriprole, Rizazole; besonders. bevorzugt seien hier für Anwendungen gegen Ektoparasiten Fipronil und Pyriprole genannt; oder
   Arylisoxazoline, Arylpyrroline, Arylpyrrolidine z. B. Fluralaner (bekannt aus WO2009/2024541, Bsp . 11-1; aber auch Verbindungen aus WO2012007426, WO2012042006, WO2012042007, WO2012107533, WO2012120135, WO2012165186, WO2012155676, WO2012017359, WO2012127347, WO2012038851, WO2012120399, WO2012156400, WO2012163959, WO2011161130, WO2011073444, WO2011092287, WO2011075591, WO2011157748, WO 2007/075459, WO 2007/125984, WO 2005/085216, WO 2009/002809), Afoxolaner (z.B. in WO2011149749) und strukturell verwandte Arylpyrroline (bekannt z.B. aus WO2009/072621, WO 2010020522, WO 2009112275, WO 2009097992, WO 2009072621, JP 2008133273, JP 2007091708), oder Arylpyrrolidine (z.B. in WO2012004326, WO2012035011, WO2012045700,WO 2010090344, WO 2010043315, WO 2008128711, JP 2008110971), und Verbindungen aus der Gruppe der sogenannten Metadiamide (bekannt z.B. aus WO2012020483, WO2012020484, WO2012077221, WO2012069366, WO2012175474, WO2011095462, WO2011113756, WO2011093415, WO2005073165) besonders bevorzugt sei hier für Anwendungen gegen Ektoparasiten Afoxolaner und Fluaralaner genannt.
(In-3) Natrium-Kanal-Modulatoren / Spannungsabhängige Natrium-Kanal-Blocker, wie beispielsweise Pyrethroide, z. B. Acrinathrin, Allethrin (d-cis-trans, d-trans), Bifenthrin, Bioallethrin, Bioallethrin-S-cyclopentenyl, Bioresmethrin, Cycloprothrin, Cyfluthrin (beta-), Cyhalothrin (gamma-, lambda-), Cypermethrin (alpha-, beta-, theta-, zeta-), Cyphenothrin [(1*R*)-*trans*-Isomere], Deltamethrin, Dimefluthrin, Empenthrin [(*EZ*)-(1*R*)-Isomere], Esfenvalerate, Etofenprox, Fenpropathrin, Fenvalerate, Flucythrinate, Flumethrin, Fluvalinate (tau-), Halfenprox, Imiprothrin, Metofluthrin, Permethrin, Phenothrin [(1*R*)-trans-Isomer], Prallethrin, Profluthrin, Pyrethrine (pyrethrum), Resmethrin, RU 15525, Silafluofen, Tefluthrin, Tetramethrin [(1*R*)- Isomere], Tralomethrin, Transfluthrin und ZXI 8901; besonders bevorzugt seien hier für Anwendungen gegen Ektoparasiten die Typ I Pyrethroide Allethrin, Bioallethrin, Permethrin, Phenothrin, Resmethrin, Tetramethrin und die Typ II Pyrethroide (Alphacyanopyrethroide) Alpha-cypermethrin, Cyfluthrin (beta-), Cyhalothrin (lambda-), Cypermethrin (alpha-, zeta-), Deltamethrin, Fenvalerate, Flucythrinate, Flumethrin, Fluvalinate (tau-), und die esterfreien Pyrethroide Etofenprox und Silafluofen genannt; oder Organochlorverbindungen z. B. DDT; oder Methoxychlor. Wirkstoffe aus dieser Klasse eignen sich ganz besonders als Mischpartner da sie eine längeranhaltende Kontakt-repellierende Wirkung haben und somit das Wirkspektrum um diese Komponente erweitern.
(In-4) Nikotinerge Acetylcholin-Rezeptor-Agonisten, wie beispielsweise Neonikotinoide, z. B. Acetamiprid, Clothianidin, Dinotefuran, Imidacloprid, Imidaclothiz, Nitenpyram, Thiacloprid, Thiamethoxam; besonders bevorzugt seien hier für Anwendungen gegen Ektoparasiten Clothianidin, Dinotefuran, Imidacloprid, Nitenpyram, und Thiacloprid genannt; oder Nikotin.
(In-5) Allosterische Acetylcholin-Rezeptor-Modulatoren (Agonisten), wie beispielsweise Spinosyne, z. B. Spinetoram und Spinosad; besonders bevorzugt seien hier für Anwendungen gegen Ektoparasiten Spinosad und Spinetoram genannt.
(In-6) Chlorid-Kanal-Aktivatoren, wie beispielsweise Avermectine/Milbemycine, z. B. Abamectin, Doramectin, Emamectin-benzoate, Eprinomectin, Ivermectin, Latidectin, Lepimectin, Milbemycin oxime, Milbemectin, Moxidectin, und Selamectin; Indolterpenoide wie z.B. Nodulisporinsäurederivate insbesondere Nodulisporinsäure A; besonders bevorzugt seien hier für Anwendungen gegen Ektoparasiten Doramectin, Eprinomectin, Ivermectin, Milbemycin oxime, Moxidectin, Selamectin und Nodulisporinsäure A genannt.
(In-7) Juvenilhormon-Analoge, z. B. Hydroprene (S-), Kinoprene, Methoprene (S-); oder Fenoxycarb; Pyriproxyfen; besonders bevorzugt seien hier für Anwendungen gegen Ektoparasiten Methoprene (S-) und Pyriproxyfen genannt.
(In-8) Milbenwachstumsinhibitoren, z. B. Clofentezine, Diflovidazin, Hexythiazox, Etoxazole; besonders bevorzugt sei hier für Anwendungen gegen Ektoparasiten Etoxazole genannt.
(In-9) Slo-1- und Latrophilin-Rezeptor Agonisten, wie beispielsweise zyklische Depsipeptide, z. B. Emodepsid sowie seine Ausgangsverbindung PF1022A (bekannt aus EP 382173, compound I); besonders bevorzugt sei hier für Anwendungen gegen Ektoparasiten Emodepsid genannt.
(In-10) Inhibitoren der oxidativen Phosphorylierung, ATP-Disruptoren, wie beispielsweise Diafenthiuron.
(In-12) Nikotinerge Acetylcholin-Rezeptor-Antagonisten, wie beispielsweise Bensultap, Cartap (-Hydrochlorid), Thiocylam, und Thiosultap (-sodium).
(In-13) Inhibitoren der Chitinbiosynthese, Typ 0, wie beispielsweise Benzoylharnstoffe, z. B. Bistrifluron, Chlorfluazuron, Diflubenzuron, Flucycloxuron, Flufenoxuron, Hexaflumuron, Lufenuron, Novaluron, Noviflumuron, Teflubenzuron und Triflumuron; besonders bevorzugt seien hier für Anwendungen gegen Ektoparasiten Diflubenzuron, Fluazuron, Lufenuron und Triflumuron genannt.
(In-14) Inhibitoren der Chitinbiosynthese, Typ 1, wie beispielsweise Buprofezin.
(In-15) Häutungsstörende Wirkstoffe, wie beispielsweise Cyromazine und Dicyclanil; besonders bevorzugt seien hier für Anwendungen gegen Ektoparasiten Cyromazin und Dicyclanil genannt.
(In-16) Ecdysonagonisten/-disruptoren, wie beispielsweise Diacylhydrazine, z. B. Chromafenozide, Halofenozide, Methoxyfenozide und Tebufenozide.
(In-17) Oktopaminerge Agonisten, wie beispielsweise Amitraz, Cymiazole, Chlordimeform und Demiditraz; besonders bevorzugt seien hier für Anwendungen gegen Ektoparasiten Amitraz, Cymiazole und Demiditraz genannt.
(In-18) Komplex-III-Elektronentransportinhibitoren, wie beispielsweise Hydramethylnon; Acequinocyl; Fluacrypyrim.
(In-19) Komplex-I-Elektronentransportinhibitoren, wie beispielsweise aus der Gruppe der METI-Akarizide, z. B. Fenazaquin, Fenpyroximate, Pyrimidifen, Pyridaben, Tebufenpyrad, Tolfenpyrad; besonders bevorzugt seien hier für Anwendungen gegen Ektoparasiten Fenpyroximate, Pyrimidifen und Tolfenpyrad genannt;
(In-20) Blocker des spannungsabhängigen Natriumkanals, wie beispielsweise Indoxacarb und Metaflumizone; besonders bevorzugt seien hier für Anwendungen gegen Ektoparasiten Indoxacarb und Metaflumizone genannt.
(In-21) Inhibitoren der Acetyl-CoA-Carboxylase, wie beispielsweise Tetronsäure-Derivate, z. B. Spirodiclofen und Spiromesifen; oder Tetramsäure-Derivate, z. B. Spirotetramat.
(In-22) Komplex-II-Elektronentransportinhibitoren, wie beispielsweise Cyenopyrafen.
(In-23) Ryanodinrezeptor-Effektoren, wie beispielsweise Diamide, z. B. Flubendiamide, Chlorantraniliprole (Rynaxypyr), Cyantraniliprole (Cyazypyr) sowie 3-Brom-N-{2-brom-4-chlor-6-[(1-cyclopropylethyl)carbamoyl]phenyl}-1-(3-chlorpyridin-2-yl)-1H-pyrazol-5-carboxamid (bekannt aus WO2005/077934) oder Methyl-2-[3,5-dibrom-2-({[3-brom-1-(3-chlorpyridin-2-yl)-1H-pyrazol-5-yl]carbonyl}amino)benzoyl]-1,2-dimethylhydrazincarboxylat (bekannt aus WO2007/043677).
(In-24) Weitere Wirkstoffe mit unbekanntem Wirkmechanismus, wie beispielsweise Azadirachtin, Amidoflumet, Benzoximate, Bifenazate, Chinomethionat, Cryolite, Cyflumetofen, Dicofol, Fluensulfone (5-Chloro-2-[(3,4,4-trifluorobut-3-en-1-yl)sulfonyl]-1,3-thiazole), Flufenerim, Pyridalyl und Pyrifluquinazon; desweiteren Präparate auf Basis von Bacillus firmus (I-1582, BioNeem, Votivo) sowie folgende bekannte wirksame Verbindungen 4-{[(6-Brompyrid-3-yl)methyl](2-fluorethyl)amino}furan-2(5H)-on (bekannt aus WO 2007/115644), 4-{[(6-Fluorpyrid-3-yl)methyl](2,2-difluorethyl)-amino}furan-2(5H)-on (bekannt aus WO 2007/115644), 4-{[(2-Chlor-1,3-thiazol-5-yl)methyl](2-fluorethyl)amino}furan-2(5H)-on (bekannt aus WO 2007/115644), 4-{[(6-Chlorpyrid-3-yl)methyl](2-fluorethyl)amino}furan-2(5H)-on (bekannt aus WO 2007/ 115644), 4-{[(6-Chlorpyrid-3-yl)methyl](2,2-difluorethyl)amino}furan-2(5H)-on (bekannt aus WO 2007/115644), 4-{[(6-Chlor-5-fluorpyrid-3-yl)methyl](methyl)amino}furan-2(5H)-on (bekannt aus WO 2007/115643), 4-{[(5,6-Dichlorpyrid-3-yl)methyl](2-fluorethyl)amino}furan-2(5H)-on (bekannt aus WO 2007/115646), 4-{[(6-Chlor-5-fluorpyrid-3-yl)methyl](cyclopropyl)amino}furan-2(5H)-on (bekannt aus WO 2007/115643), 4-{[(6-Chlorpyrid-3-yl)methyl](cyclopropyl)amino}furan-2(5H)-on (bekannt aus EP-A-0 539 588), 4-{[(6-Chlorpyrid-3-yl)methyl](methyl)amino}furan-2(5H)-on (bekannt aus EP-A-0 539 588), [(6-Chlorpyridin-3-yl)methyl](methyl)oxido-λ⁴-sulfanylidencyanamid (bekannt aus WO 2007/149134), [1-(6-Chlorpyridin-3-yl)ethyl](methyl)oxido-λ⁴-sulfanylidencyanamid (bekannt aus WO 2007/149134), [(6-Trifluormethylpyridin-3-yl)methyl](methyl)oxido-λ⁴-sulfanylidencyanamid (bekannt aus WO 2007/095229), Sulfoxaflor (ebenfalls bekannt aus WO 2007/149134), 11-(4-Chlor-2,6-dimethylphenyl)-12-hydroxy-1,4-dioxa-9-azadispiro[4.2.4.2]tetradec-11-en-10-on (bekannt aus WO 2006/089633), 3-(4'-Fluor-2,4-dimethylbiphenyl-3-yl)-4-hydroxy-8-oxa-1-azaspiro[4.5]dec-3-en-2-on (bekannt aus WO 2008/067911), 1-[2-Fluoro-4-methyl-5-[(2,2,2-trifluoroethyl)sulfinyl]phenyl]-3-(trifluoromethyl)-1H-1,2,4-Triazol-5-amine (bekannt aus WO 2006/ 043635), [(3S,4aR,12R,12aS,12bS)-3-[(Cyclopropylcarbonyl)oxy]-6,12-dihydroxy-4,12b-dimethyl-11-oxo-9-(pyridin-3-yl)-1,3,4,4a,5,6,6a,12,12a,12b-decahydro-2H,11H-benzo[f]pyrano[4,3-b]chromen-4-yl]methylcyclo-propancarboxylat (bekannt aus WO 2006/129714), 2-Cyano-3-(diFluoromethoxy)-N-ethyl-benzenesulfonamide (bekannt aus WO 2005/035486), N-[1-(2,3-Dimethylphenyl)-2-(3,5-dimethylphenyl)ethyl]-4,5-dihydro-2-thiazolamine (bekannt aus WO 2008/104503); Penigequinolone A (bekannt aus EP 2248422 (compound I) und WO 2009/060015 (compound No. 11).
(In-25) Als geeignete Synergisten bei Verwendung mit Ektoparasitiziden seien hier MGK264 (N-Octylbicycloheptencarboxamid), Piperonylbutoxid (PBO) und Verbutin genannt; besonders bevorzugt seien hier Piperonylbutoxid und MGK264 genannt.

Zusätzlich zu diesen Gruppen können in Mischungen oder in Kombinationsanwendung auch Kurzzeitrepellentien verwendet werden. Beispiele sind DEET (N,N-diethyl-3-methylbenzamide), icaridin (1-Piperidine carboxylic acid), (1S, 20S)-2-methylpiperidinyl-3-cyclohexene-1-carboxamide (SS220), indalone (butyl 3,4-dihydro-2, 2-dimethyl-4-oxo-2H-pyran-6-carboxylate), dihydronepetalactones, nootkatone, IR3535 (3-[N-butyl-N-acetyl]-aminopropionic acid ethyl ester), 2-Ethylhexane-1,3-diol, (1R,2R,5R)-2-(2-Hydroxypropan-2-yl)-5-methyl-cyclohexan-1-ol, Dimethyl benzene-1,2-dicarboxylate, Dodecanoic acid, Undecan-2-one, N,N-Diethyl-2-phenyl-acetamide und aetherische Öle oder andere Pflanzeninhaltsstofef mit bekannter repellierender Wirkung wie z.B. Borneol, Callicarpenal, 1,8-Cineol (eucalyptol), Carvacrol, b-Citronellol, a-Copaene, Coumarin (oder seine synthetischen Derviate bekannt aus US20120329832), Beim Einsatz gegen Ectoparasiten besonders bevorzugt sind icaridin, indalone und IR3535 (3-[N-butyl-N-acetyl]-aminopropionic acid ethyl ester)

Von den vorstehend genannten Gruppen (In-1) bis (In-25) sind die folgenden Gruppen als Mischpartner bevorzugt: (In-2), (In-3), (In-4), (In-5), (In-6), (In-17), (In-25).

Besonders bevorzugte Beispiele für insektizid oder akarizid wirksame Verbindungen, Synergisten oder Repellentien als Mischpartner der erfindungsgemäßen Verbindungen der Formel (Ia") sind Afoxolaner, Allethrin, Amitraz, Bioallethrin, Chlothianidin, Cyfluthrin (beta-), Cyhalothrin (lambda-), Cymiazole, Cypermethrin (alpha-, zeta-), Cyphenothrin, Deltamethrin, Demiditraz, Dinotefuran, Doramectin, Eprinomectin, Etofenprox, Fenvalerate, Fipronil, Fluazuron, Flucythrinate, Flumethrin, Fluralaner, Fluvalinate (tau-), Icaridin, Imidacloprid, Ivermectin, MGK264, Milbemycin oxime, Moxidectin, Nitenpyram, Permethrin, Phenothrin, Piperonylbutoxid, Pyriprole, Resmethrin, Selamectin, Silafluofen, Spinetoram, Spinosad, Tetramethrin, Thiacloprid.

### Vektorkontrolle

Die Verbindungen der Formel (Ia") können auch in der Vektorkontrolle eingesetzt werden. Ein Vektor im Sinne der vorliegenden Erfindung ist ein Arthropode, insbesondere ein Insekt oder Arachnide, der in der Lage ist, Krankheitserreger wie z. B. Viren, Würmer, Einzeller und Bakterien aus einem Reservoir (Pflanze, Tier, Mensch, etc.) auf einen Wirt zu übertragen. Die Krankheitserreger können entweder mechanisch (z.B. Trachoma durch nicht-stechende Fliegen) auf einem Wirt, oder nach Injektion (z.B. Malaria-Parasiten durch Mücken) in einen Wirt übertragen werden.

Beispiele für Vektoren und die von ihnen übertragenen Krankheiten bzw. Krankheitserreger sind:
1) Mücken
   - Anopheles: Malaria, Filariose;
   - Culex: Japanische Encephalitis, Filariasis, weitere virale Erkrankungen, Übertragung von Würmern;
   - Aedes: Gelbfieber, Dengue-Fieber, Filariasis, weitere virale Erkrankungen;
   - Simulien: Übertragung von Würmern insbesondere Onchocerca volvulus;
2) Läuse: Hautinfektionen, Fleckfieber (epidemic typhus);
3) Flöhe: Pest, endemisches Fleckfieber;
4) Fliegen: Schlafkrankheit (Trypanosomiasis); Cholera, weitere bakterielle Erkrankungen;
5) Milben: Acariose, Fleckfieber, Rickettsipocken, Tularämie, Saint-Louis-Enzephalitis, virale Hirnhaut-entzündung (FSME), Krim-Kongo-Fieber, Borreliose;
6) Zecken: Borelliosen wie Borrelia duttoni, Frühsommer-Meningoenzephalitis, Q-Fieber (Coxiella burnetii), Babesien (Babesia canis canis).

Beispiele für Vektoren im Sinne der vorliegenden Erfindung sind Insekten wie Aphiden, Fliegen, Zikaden oder Thripse, die Pflanzenviren auf Pflanzen übertragen können. Weitere Vektoren, die Pflanzenviren übertragen können, sind Spinnmilben, Läuse, Käfer und Nematoden.

Weitere Beispiele für Vektoren im Sinne der vorliegenden Erfindung sind Insekten und Arachniden wie Mücken, insbesondere der Gattungen Aedes, Anopheles, z.B. A. gambiae, A. arabiensis, A. funestus, A. dirus (Malaria) und Culex, Läuse, Flöhe, Fliegen, Milben und Zecken, die Krankheitserreger auf Tiere und/oder Menschen übertragen können.

Eine Vektorkontrolle ist auch möglich, wenn die Verbindungen der Formel (Ia") Resistenzbrechend sind.

Verbindungen der Formel (Ia") sind zur Verwendung in der Prävention von Krankheiten bzw. vor Krankheitserregern, die durch Vektoren übertragen werden, geeignet. Somit ist ein weiterer Aspekt der vorliegenden Erfindung die Verwendung von Verbindungen der Formel (Ia") zur Vektorkontrolle, z.B. in der Landwirtschaft, im Gartenbau, in Forsten, in Gärten und Freizeiteinrichtungen sowie im Vorrats- und Materialschutz.

### Schutz von technischen Materialen

Die Verbindungen der Formel (Ia") eignen sich zum Schutz von technischen Materialien gegen Befall oder Zerstörung durch Insekten, z.B. aus der Ordnung Coleoptera, Hymenoptera, Isoptera, Lepidoptera, Psocoptera und Zygentoma.

Unter technischen Materialien sind im vorliegenden Zusammenhang nicht lebende Materialien zu verstehen, wie vorzugsweise Kunststoffe, Klebstoffe, Leime, Papiere und Kartone, Leder, Holz, Holzverarbeitungsprodukte und Anstrichmittel. Die Anwendung der Erfindung zum Schutz von Holz ist besonders bevorzugt.

In einer weiteren Ausführungsform werden die Verbindungen der Formel (Ia") zusammen mit mindestens einem weiteren Insektizid und/oder mindestens einem Fungizid eingesetzt.

In einer weiteren Ausführungsform liegen die Verbindungen der Formel (Ia") als ein anwendungsfertiges (ready-to-use) Schädlingsbekämpfungsmittel vor, d.h., es kann ohne weitere Änderungen auf das entsprechende Material aufgebracht werden. Als weitere Insektizide oder als Fungizide kommen insbesondere die oben genannten in Frage.

Überraschenderweise wurde auch gefunden, dass die Verbindungen der Formel (Ia") zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, verwendet werden können. Gleichfalls können die Verbindungen der Formel (Ia") allein oder in Kombinationen mit anderen Wirkstoffen als Antifouling-Mittel eingesetzt werden.

### Bekämpfung von tierischen Schädlingen auf dem Hygienesektor

Die Verbindungen der Formel (Ia") eignen sich zur Bekämpfung von tierischen Schädlingen auf dem Hygienesektor. Insbesondere kann die Erfindung im Haushalts-, Hygiene- und Vorratsschutz verwendet werden, vor allem zur Bekämpfung von Insekten, Spinnentieren und Milben, die in geschlossenen Räumen, wie beispielsweise Wohnungen, Fabrikhallen, Büros, Fahrzeugkabinen vorkommen. Zur Bekämpfung der tierischen Schädlinge werden die Verbindungen der Formel (Ia") allein oder in Kombination mit anderen Wirk- und/oder Hilfsstoffen verwendet. Bevorzugt werden sie in Haushaltsinsektizid-Produkten verwendet. Die Verbindungen der Formel (Ia") sind gegen sensible und resistente Arten sowie gegen alle Entwicklungsstadien wirksam.

Zu diesen Schädlingen gehören beispielsweise Schädlinge aus der Klasse Arachnida, aus den Ordnungen Scorpiones, Araneae und Opiliones, aus den Klassen Chilopoda und Diplopoda, aus der Klasse Insecta die Ordnung Blattodea, aus den Ordnungen Coleoptera, Dermaptera, Diptera, Heteroptera, Hymenoptera, Isoptera, Lepidoptera, Phthiraptera, Psocoptera, Saltatoria oder Orthoptera, Siphonaptera und Zygentoma und aus der Klasse Malacostraca die Ordnung Isopoda.

Die Anwendung erfolgt beispielsweise in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

### Herstellungs Verfahren

Die erfindungsgemäßen Verbindungen können nach üblichen, dem Fachmann bekannten Methoden hergestellt werden.

Die Verbindungen der Struktur (I-T1) und (I-T2) können nach den in der Literatur für analoge Verbindungen bereits beschrieben Methoden hergestellt werden:

### Verfahren I-T1

Die Verbindungen der Struktur (I-T1) können nach dem im Reaktionsschema 1 angegebenen Verfahren hergestellt werden.

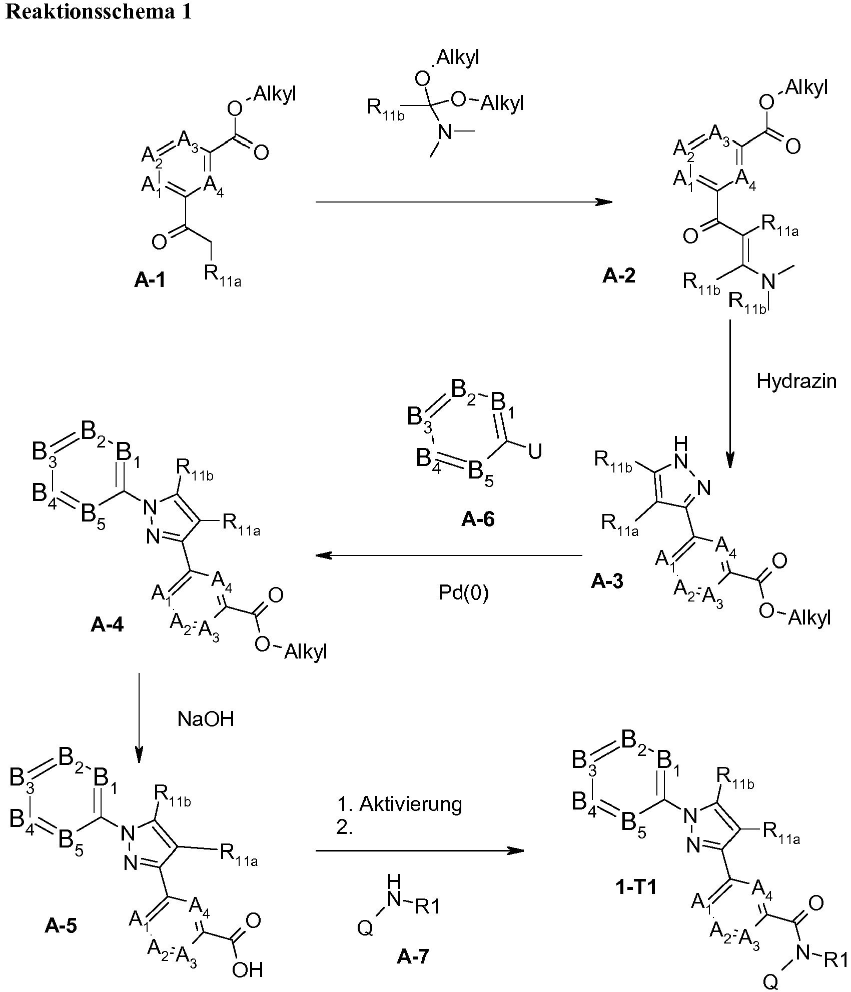

Die Reste A₁-A₄, B₁-B₅, Alkyl, Q, R¹ und R¹¹ haben die oben beschriebenen Bedeutungen. U steht z. B. für Brom, Iod oder Triflat. Ausgangsverbindungen der Struktur (**A-1**) (z.B. WO 2004/099146, S. 75-76) und (**A-7**) (z.B. US 5,739,083 Seite 10, US 2003/187233A1, S. 6) sind bekannt oder können nach bekannten Methoden hergestellt werden.

Verbindungen der allgemeinen Struktur (**A-2**) lassen sich in Analogie zu literaturbekannten Methoden aus den Verbindungen der allgemeinen Struktur (**A-1**) und Carbonsäureamidacetalen (**B-8**) herstellen (z.B. WO 2013/009791, S. 50, Example 43; WO 2004/099146, S. 75-76). Verbindungen der allgemeinen Struktur (**A-3**) lassen sich in Analogie zu literaturbekannten Methoden aus den Verbindungen der allgemeinen Struktur (**A-2**) und Hydrazin herstellen (z.B. WO 2013/009791, S. 50, Example 43; WO 2004/099146, S. 75-76). Verbindungen der allgemeinen Struktur (**A-4**) lassen sich in Analogie zu literaturbekannten Methoden aus den Verbindungen der allgemeinen Struktur (**A-3**) und (**A-6**) herstellen (z.B. WO 2013/009791, S. 50, Example 44). Verbindungen der allgemeinen Struktur (**A-5**) lassen sich in Analogie zu literaturbekannten Verfahren durch Esterspaltung aus Verbindungen der allgemeinen Struktur (**A-4**) herstellen (siehe z. B. WO 2010/051926 oder WO 2010/133312). Erfindungsgemäße Verbindungen der allgemeinen Struktur (**I-T1**) können in Analogie zu literaturbekannten Peptidkupplungsmethoden aus den Ausgangsmaterialien (**A-5**) und (**A-7**) hergestellt werden (z. B. WO 2010/051926 oder WO 2010/133312).

### Verfahren I-T2

Die Verbindungen der Struktur (**I-T2**) können nach dem im Reaktionsschema 2 angegebenen Verfahren hergestellt werden.

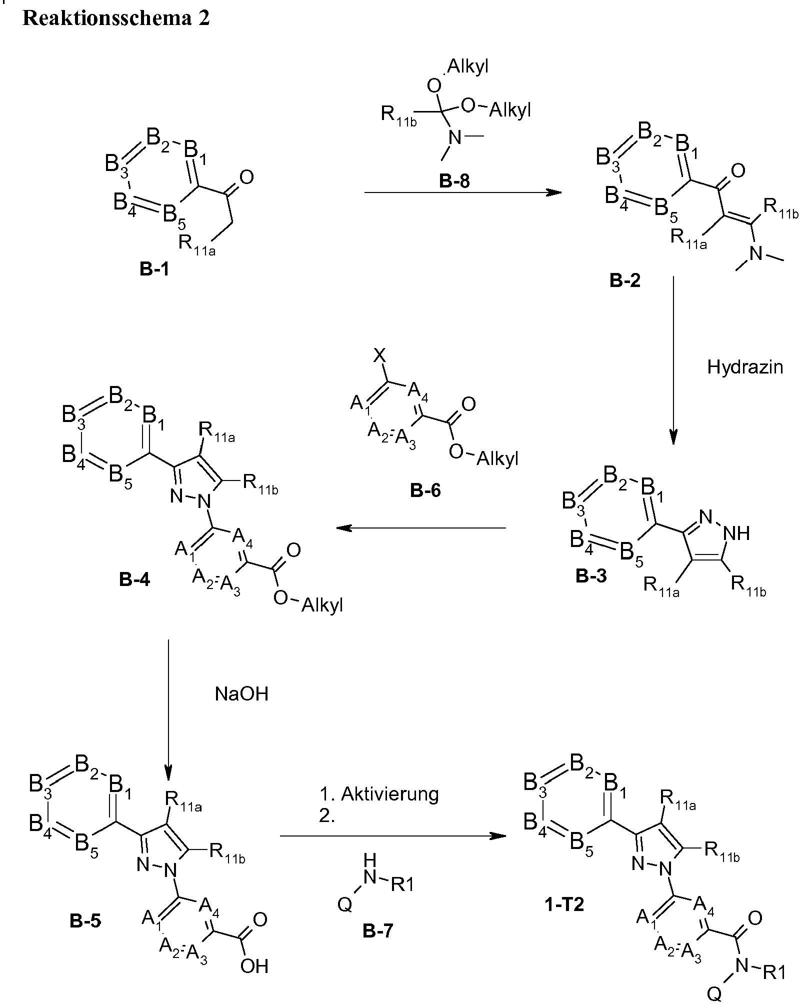

Die Reste A₁ bis A₄, B₁ bis B₅, Alkyl, Q, R¹ und R¹¹ haben die oben beschriebenen Bedeutungen. X steht z. B. für Cl, Br, I oder einen Boronsäure- bzw. Boronsäureester-Rest. Ausgangsverbindungen der Struktur (**B-1**) (z.B. Filler, Robert; Kong, Zhengrong; Zhang, Zhaoxu; Sinha, Arun Kr.; Li, Xiaofang Journal of Fluorine Chemistry, 80 (1996) S. 71 - 76; US2003/187233, S. 14, Example 21) und (**B-6**) sind bekannt oder können nach bekannten Methoden hergestellt werden.

Verbindungen der allgemeinen Struktur (**B-2**) lassen sich in Analogie zu literaturbekannten Methoden aus den Verbindungen der allgemeinen Struktur (**B-1**) und Carbonsäureamidacetalen (**B-8**) herstellen (z.B. WO 2006/044505, Compound 60, Part A; WO 2012/4604, Intermediate 2). Verbindungen der allgemeinen Struktur (**B-3**) lassen sich in Analogie zu literaturbekannten Methoden aus den Verbindungen der allgemeinen Struktur (**B-2**) und Hydrazin herstellen (z.B. WO 2013/009791, S. 50, Example 43; WO 2004/099146, S. 75-76). Verbindungen der allgemeinen Struktur (**B-4**) lassen sich in Analogie zu literaturbekannten Methoden aus den Verbindungen der allgemeinen Struktur (**B-3**) und (**B-6**) herstellen (z.B. WO 2013/009791, S. 50, Example 44, X = Br). Verbindungen der allgemeinen Struktur (**B-5**) lassen sich in Analogie zu literaturbekannten Verfahren durch Esterspaltung aus Verbindungen der allgemeinen Struktur (**B-4**) herstellen (z. B. WO 2010/051926 oder WO 2010/133312). Erfindungsgemäße Verbindungen der allgemeinen Struktur (**I-T1**) können in Analogie zu literaturbekannten Peptidkupplungsmethoden aus den Ausgangsmaterialien (**B-5**) und (**B-7**) hergestellt werden (z. B. WO 2010/051926 oder WO 2010/133312).

### Stufe 1 Dialkylaminoalkenylierung

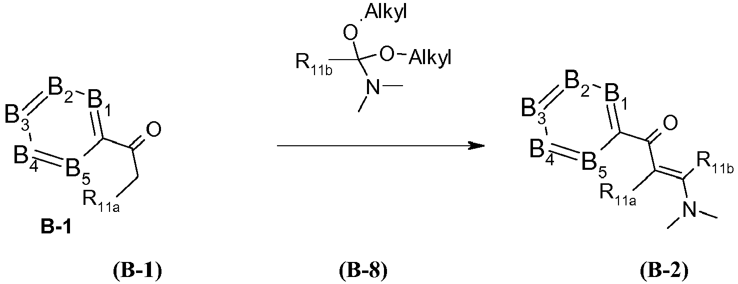

Verbindungen der allgemeinen Struktur (B-2) können in Analogie zu literaturbekannten Methoden aus den Ausgangsmaterialien der Struktur (**B-1**) und (**B-8**) hergestellt werden. Die Reste B¹-B⁵, Alkyl und R¹¹ haben die weiter oben beschriebenen Bedeutungen. Ausgangsverbindungen der Struktur (**B-1**) (z.B. Filler, Robert; Kong, Zhengrong; Zhang, Zhaoxu; Sinha, Arun Kr.; Li, Xiaofang Journal of Fluorine Chemistry, 80 (1996) S. 71 - 76; US2003/187233, S. 14, Example 21 [0294], US5739083, Example 6) sind bekannt oder können nach bekannten Methoden hergestellt werden. Die Reaktion wird durchgeführt indem man die Verbindungen (**B-1**) mit den Verbindungen (**B-8**) unter den für analoge Reaktionen in der Literatur bekannten Bedingungen umsetzt (z.B. EP1204323, S. 25, Example 13).

### Stufe 2 Pyrazol Ringschluss

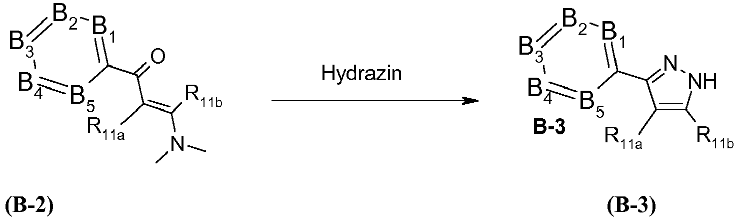

Verbindungen der allgemeinen Struktur (**B-3**) können in Analogie zu literaturbekannten Methoden aus den Ausgangsmaterialien der Struktur (**B-2**) und Hydrazin hergestellt werden. Die Reste B¹-B⁵, und R¹¹ haben die weiter oben beschriebenen Bedeutungen. Die Herstellung der Ausgangsverbindungen der Struktur (**B-2**) ist weiter oben beschrieben. Die Reaktion wird durchgeführt indem man die Verbindungen (**B-2**) mit Hydrazin unter den für analoge Reaktionen in der Literatur bekannten Bedingungen umsetzt (EP1382603, Example 3, S. 43)

### Stufe 3 Aryl-Kupplung

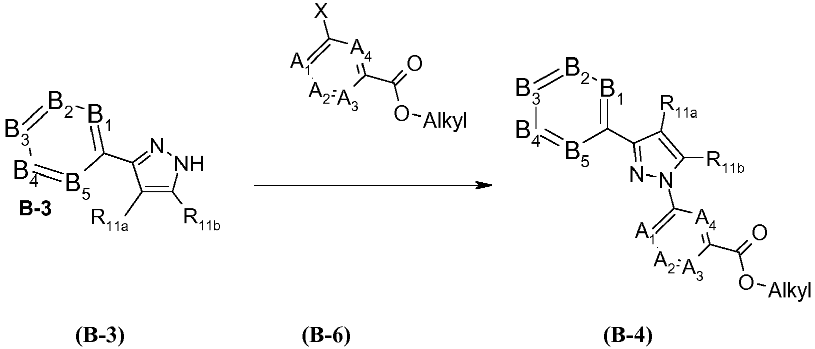

Verbindungen der allgemeinen Struktur (**B-4**) können in Analogie zu literaturbekannten Methoden aus den Ausgangsmaterialien der Struktur (**B-3**) und (**B-6**) hergestellt werden. Die Reste A¹-A⁴, B¹-B⁵, Alkyl, R¹ und R¹¹ haben die oben beschriebenen Bedeutungen. X steht für einen Boronsäure- bzw. einen Boronsäureesterrest. Die Herstellung der Ausgangsverbindungen der Struktur (**B-3**) ist weiter oben beschrieben. Die Verbindungen der allgemeinen Struktur (**B-6**) sind entweder kommerziell erhältlich oder können nach dem Fachmann bekannten Verfahren hergestellt werden. Die Reaktion wird unter den für analoge Reaktionen in der Literatur bekannten Bedingungen durchgeführt (WO2009140342, S. 96).

### Stufen 4, 5 Verseifung, Amidierung

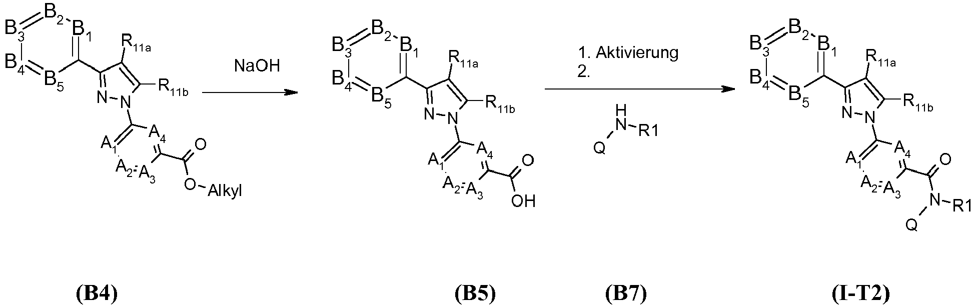

Erfindungsgemäße Verbindungen der allgemeinen Struktur (**I-T2**) können in Analogie zu literaturbekannten Peptidkupplungsmethoden aus den Ausgangsmaterialien (**B5**) und (**B7**) hergestellt werden [WO2010-051926; WO2010-133312]. Verbindungen der allgemeinen Struktur (**B5**) lassen sich in Analogie zu literaturbekannten Verfahren durch Esterspaltung aus Verbindungen der allgemeinen Struktur (**B4**) herstellen [WO2010-051926; WO2010-133312]. Die Reste A¹-A⁴, B¹-B⁵, Alkyl, Q, R¹ und R¹¹ haben die oben beschriebenen Bedeutungen. Die Herstellung der Verbindungen der Struktur (**B-7**) ist weiter oben beschrieben.

### Verfahren I-T3

Die Verbindungen der Struktur (I-T3) können nach dem im Reaktionsschema 3a angegebenen Verfahren hergestellt werden. 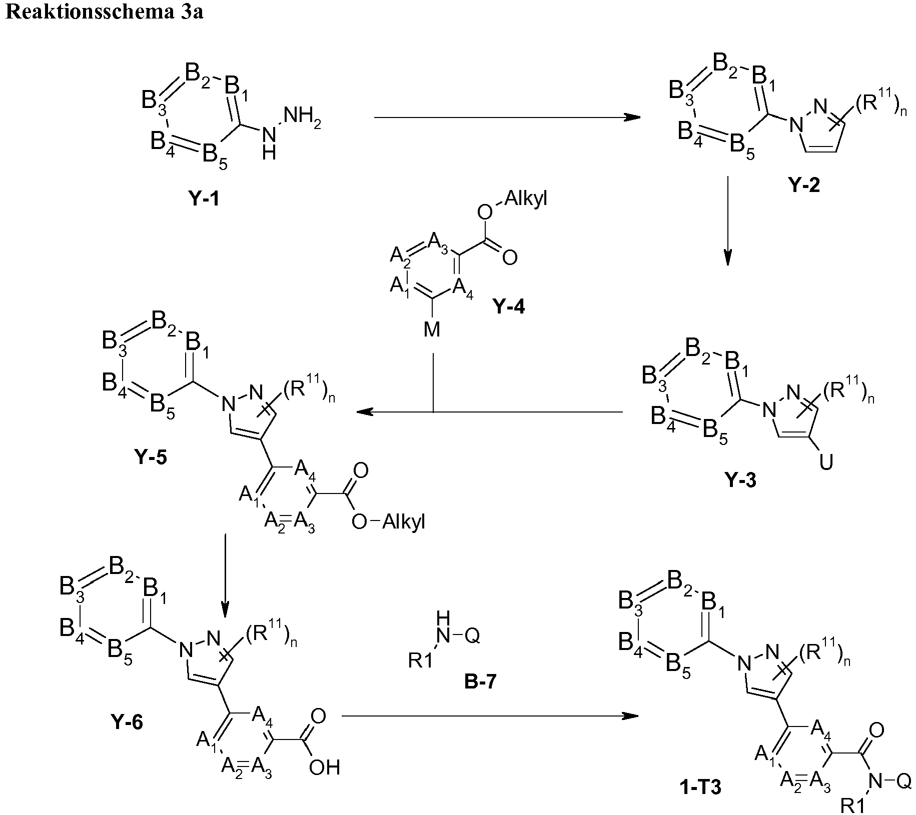 Die Reste A₁ bis A₄, B₁ bis B₅, Alkyl, Q, R¹, n und R¹¹ haben die oben beschriebenen Bedeutungen. M steht z.B. für eine Boronsäure, Boronsäureester oder Trifluorboronat . U steht z. B. für Brom, Iod oder Triflat. X steht z. B. für Cl, Br, I.

### Stufe 1 Pyrazol

Stufe 1 des Darstellungsverfahrens der erfindungsgemäßen Verbindungen (1-T3):

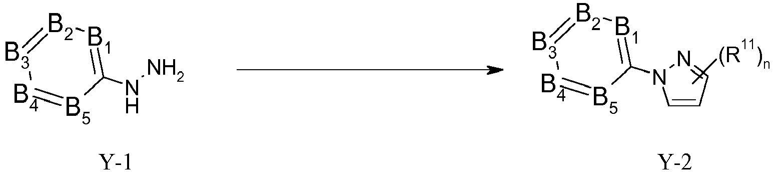

Erfindungsgemäße Verbindungen der allgemeinen Struktur (Y-2) können in Analogie zu literaturbekannten Methoden aus den Ausgangsmaterialien der Struktur (Y-1) hergestellt werden. Die Reste B₁-B₅ und R¹¹ haben die oben beschriebenen Bedeutungen. Ausgangsverbindungen der Struktur (Y1) sind bekannt oder können nach bekannten Methoden hergestellt werden. Als Beispiele seien [2,6-Dichlor-4-(trifluormethyl)phenyl]hydrazin, [3 -Chlor-5-(trifluormethyl)-2-pyridyl]hydrazin, [2,6-Dichlor-4-[1,2,2,2-tetrafluor-1-(trifluormethyl)ethyl]phenyl]hydrazin, [2,6-Dimethyl-4-[1,2,2,2-tetrafluor-1-(trifluormethyl)ethyl]phenyl]hydrazin, [2-Methyl-4-[1,2,2,2-tetrafluor-1-(trifluormethyl)ethyl]-6-(trifluormethyl)phenyl]hydrazin, oder [2-Chlor-4-[1,2,2,2-tetrafluor-1-(trifluormethyl)ethyl]-6-(trifluormethyl)phenyl]hydrazin genannt. Sie können z.B. nach US 2003/187233, S. 13; Haga, Takahiro et al., Heterocycles, 22 (1984), S. 117-124 beschriebenen Methoden hergestellt werden.

### Stufe 2 Iod-Pyrazol

Stufe 2 des Darstellungsverfahrens der erfindungsgemäßen Verbindungen (1-T3):

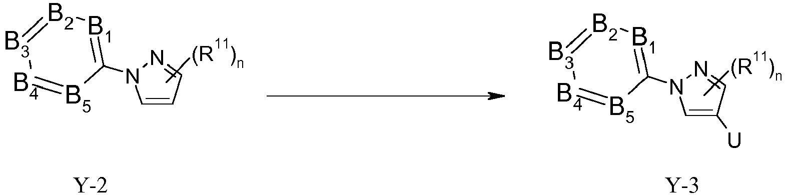

Die Reste B₁-B₅, n und R¹¹ haben die oben beschriebenen Bedeutungen. U steht z. B. für Brom oder Iod.

Die Verbindungen der Strukturformel (Y-3) sind z.B. 1-(2,6-dichloro-4-trifluoromethyl-phenyl)-4-iod-pyrazol, 3-Chlor-2-(4-iodpyrazol-1-yl)-5-(trifluormethyl)pyridin (CAS-RN: 8611-89-2), 1-(2,6-Dichlor-4-heptafluorisopropyl-phenyl)-4-iod-pyrazol, 1-(2,6-Dimethyl-4-heptafluorisopropyl-phenyl)-4-iod-pyrazol, 1-[2-Methyl-4-[1,2,2,2-tetrafluor-1-(trifluormethyl)ethyl]-6-(trifluormethyl)phenyl]-4-iod-pyrazol, 1-[2-Chlor-4-[1,2,2,2-tetrafluor-1-(trifluormethyl)ethyl]-6-(trifluormethyl)phenyl]-4-iod-pyrazol, oder 1-[2-Ethyl-6-methyl-4-[1,2,2,2-tetrafluor-1-(trifluormethyl)ethyl]phenyl]-4-iod-pyrazol.

Erfindungsgemäße Verbindungen der allgemeinen Struktur (Y-3) werden hergestellt indem man die Pyrazole der Struktur (Y-2) mit halogenierenden Mitteln umsetzt. Die Reste B¹ bis B⁵ und R¹¹ haben die oben beschriebenen Bedeutungen. Geeignete halogenierende Verbindungen sind dem Fachmann bekannt wie z.B. Chlor, Brom, Iod, N-Chlorsuccinimid, N-Bromsuccinimid, N-Iodsuccinimid, 1,3-Dichlor-5,5-dimethylhydantoin, 1,3-Dibrom-5,5-dimethylhydantoin, Natriumhypochlorit und Jodmonochlorid. Bevorzugt werden Brom, Iod und Iodsuccinimid eingesetzt. Gegebenenfalls ist es vorteilhaft, die Reaktion in Gegenwart eines Oxidationsmittels z.B. Wasserstoffperoxid durchzuführen. Die Umsetzung folgt den aus der Literatur bekannten Bedingungen z. B. Guo Li et al., Tetrahedron Letters 48 (2007), 4595-4599; Mary M. Kim et al., Tetrahedron Letters 49 (2008), 4026-4028.

### Alternativ Kupplung mit Pyrazol

Alternativ lassen sich die Verbindungen der Struktur Y-3 auch nach literaturbekannten Verfahren durch direkte Kupplung von Iodpyrazolen mit entsprechenden Arylhalogeniden herstellen (z. B. Sammelson, Robert E. et al., J. of Organic Chemistry, 68 (2003), 8075-8079).

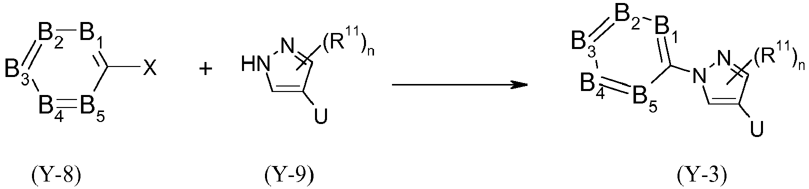

Die Reste B₁ bis B₅, n und R¹¹ haben die oben beschriebenen Bedeutungen. X steht z. B. für ein Halogen. U steht z. B. für Brom, Iod oder Triflat

Ausgangsverbindungen der Struktur (Y-8) sind bekannt oder können nach bekannten Methoden hergestellt werden. Als Beispiele seien 2-Brom-1,3-dichlor-5-[1,2,2,2-tetrafluor-1-(trifluormethyl)ethyl]benzol, 2-Brom-1,3-dimethyl-5-[1,2,2,2-tetrafluor-1-(trifluormethyl)ethyl]benzol, 2-Brom-1-ethyl-3-methyl-5-[1,2,2,2-tetrafluor-1-(trifluormethyl)ethyl]benzol, 2-Brom-1-chlor-5-[1,2,2,2-tetrafluor-1-(trifluormethyl)ethyl]-3-(trifluormethyl)benzol, 2-Brom-1-methyl-5-[1,2,2,2-tetrafluor-1-(trifluormethyl)ethyl]-3-(trifluormethyl)benzol, 2-Brom-1-chlor-5-[1,2,2,2-tetrafluor-1-(trifluormethyl)ethyl]-3-(trifluormethoxy)benzol, 2-Brom-1-methyl-5-[1,2,2,2-tetrafluor-1-(trifluormethyl)ethyl]-3-(trifluormethoxy)benzol genannt. Sie können z.B. nach den in EP1253128, Seiten 8-10 beschriebenen Methoden hergestellt werden.

### Stufe 3 Boronsäure Kupplung

Stufe 3 des Darstellungsverfahrens der erfindungsgemäßen Verbindungen (1-T3):

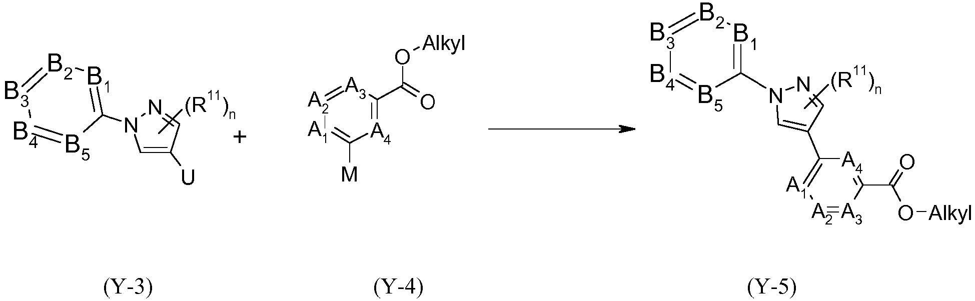

Die Reste A₁ bis A₄, B₁ bis B₅, Alkyl, n und R¹¹ haben die oben beschriebenen Bedeutungen. U steht z. B. für Brom, Iod oder Triflat, wenn M für eine Boronsäure, Boronsäureester oder Trifluorboronat steht; oder U steht z. B. für eine Boronsäure, Boronsäureester oder Trifluorboronat, wenn M für Brom, Iod oder Triflat steht.

Erfindungsgemäße Verbindungen der allgemeinen Struktur **(Y-5)** können nach literaturbekannten Verfahren mittels Palladium katalysierten Reaktionen aus den Reaktionspartnern (**Y-3)** und **(Y-4)** hergestellt werden (z. B. WO 2005/040110 oder WO 2009/089508). Die Verbindungen der allgemeinen Struktur **(Y-4)** sind entweder kommerziell erhältlich oder können nach dem Fachmann bekannten Verfahren hergestellt werden.

### Stufen 4, 5 Verseifung, Amidierung

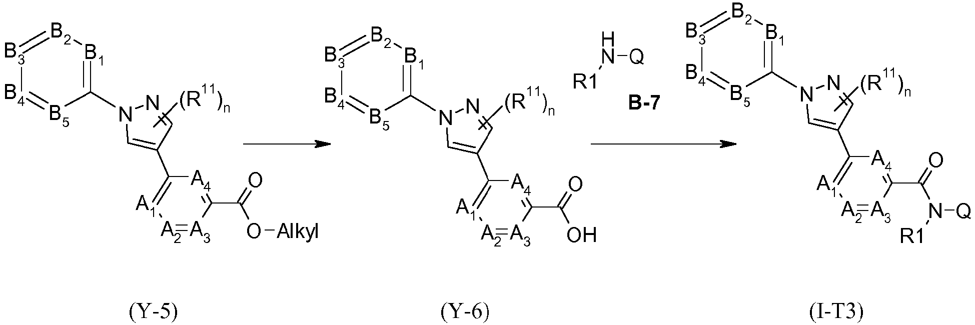

Erfindungsgemäße Verbindungen der allgemeinen Struktur **(I-T3)** können in Analogie zu literaturbekannten Peptidkupplungsmethoden aus den Ausgangsmaterialien **(Y-6)** und **(Y-7)** hergestellt werden (z. B. WO 2010/051926 oder WO 2010/133312). Verbindungen der allgemeinen Struktur **(Y-6)** lassen sich in Analogie zu literaturbekannten Verfahren durch Esterspaltung aus Verbindungen der allgemeinen Struktur (Y-5) herstellen (z. B. WO 2010/051926 oder WO 2010/133312). Die Reste A₁ bis A₄, B₁ bis B₅, Alkyl, Q, R¹ und R¹¹ haben die oben beschriebenen Bedeutungen.

### Stufe 3 Alternativ: Kupplung mit Amiden

Alternativ können die erfindungsgemäßen Verbindungen **(I-T3)** durch das allgemeine Darstellungsverfahren 3b dargestellt werden.

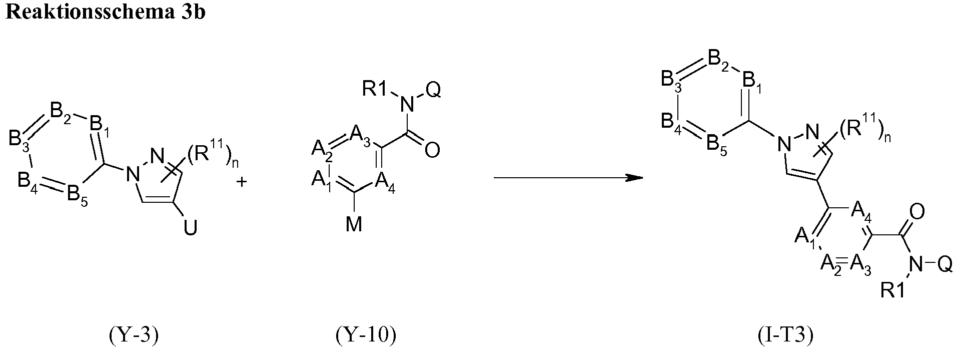

Die Reste A₁ bis A₄, B₁ bis B₅, Alkyl, Q, R¹, n und R¹¹ haben die oben beschriebenen Bedeutungen. U steht für Brom, Iod oder Triflat, wenn M für eine Boronsäure, Boronsäureester oder Trifluorboronat steht. U steht für eine Boronsäure, Boronsäureester oder Trifluorboronat, wenn M für Brom, Iod oder Triflat steht.

Erfindungsgemäße Verbindungen der allgemeinen Struktur **(I-T3)** können nach literaturbekannten Verfahren mittels Palladium katalysierten Reaktionen aus den Reaktionspartnern **(Y-3)** und **(Y-10)** hergestellt werden (z. B. WO 2005/040110 oder WO 2009/089508). Die Verbindungen der allgemeinen Struktur **(Y-10)** sind entweder kommerziell erhältlich oder können nach dem Fachmann bekannten Verfahren hergestellt werden. Die Herstellung Verbindungen der Struktur **(Y-3)** ist bereits oben beschrieben.

### Verfahren I-T4

Die Verbindungen der Struktur (**I-T4**) können nach dem im Reaktionsschema 4 angegebenen Verfahren hergestellt werden.

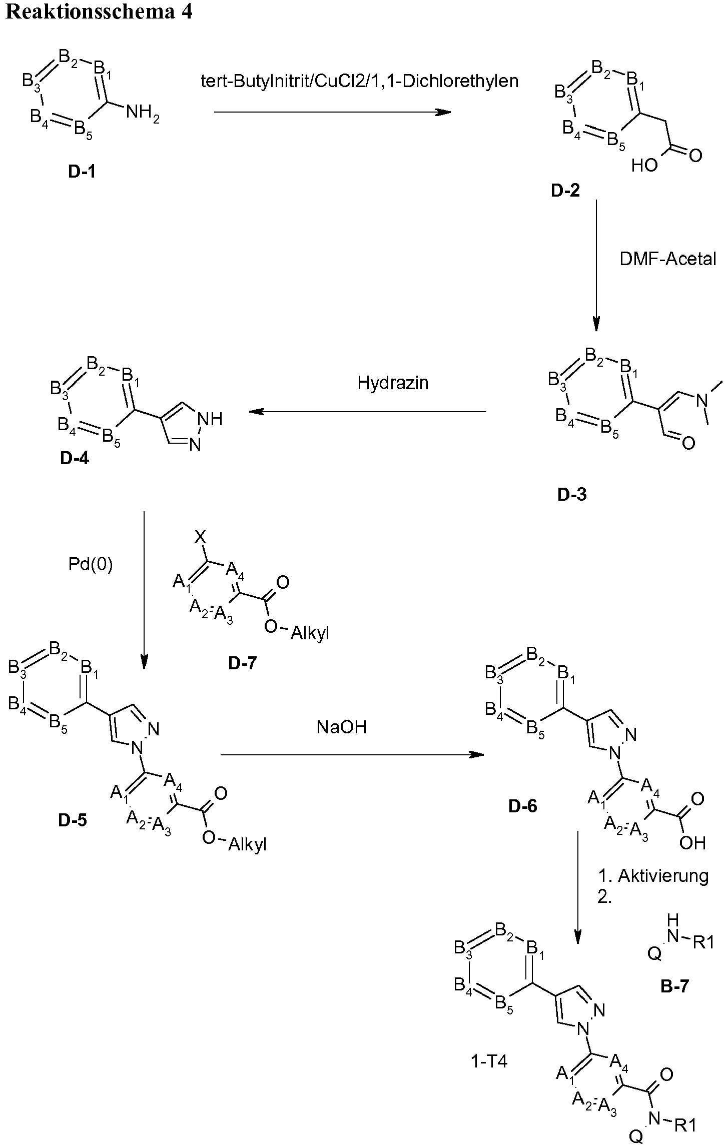

Die Reste A₁ bis A₄, B₁ bis B₅, Alkyl, Q, und R¹ haben die oben beschriebenen Bedeutungen. X steht für Cl, Br, I. Ausgangsverbindungen der Struktur (D-1) (z.B. EP2319830, S. 330) und (D-7) sind bekannt oder können nach bekannten Methoden hergestellt werden.

Die Durchführung der Reaktionen kann nach den in der Literatur beschriebenen Verfahren erfolgen z.B. WO 2012/149236, Majumder, Supriyo et al., Advanced Synthesis and Catalysis, 351 (2009), 2013-2023, oder US 5,061,705.

Verbindungen der allgemeinen Struktur (**D2**) lassen sich in Analogie zu literaturbekannten Methoden aus den Verbindungen der allgemeinen Struktur (**D1**) hergestellen (z.B. WO2008148868A1, S. 87). Verbindungen der allgemeinen Struktur (**D3**) können in Analogie zu literaturbekannten Reaktionen aus den Verbindungen der allgemeinen Struktur (**D2**) und einem Iminium Salz hergestellt werden (z.B. Knorr, Rudolf; Loew, Peter; Hassel, Petra; Bronberger, Hildegard Journal of Organic Chemistry, 49 (1984) S. 1288-1290). Verbindungen der allgemeinen Struktur (**D4**) können in Analogie zu literaturbekannten Methoden aus den Verbindungen der allgemeinen Struktur (**D3**) und Hydrazin hergestellt werden (z.B. WO2008080969 A1, S. 102-103, Example 104). Verbindungen der allgemeinen Struktur (**D5**) lassen sich in Analogie zu literaturbekannten Methoden aus den Verbindungen der allgemeinen Struktur (**D4**) und (**D7**) herstellen (z.B. WO2013009791, S. 50, Example44). Verbindungen der allgemeinen Struktur (**D6**) lassen sich in Analogie zu literaturbekannten Verfahren durch Esterspaltung aus Verbindungen der allgemeinen Struktur (**D5**) herstellen [WO2010-051926; WO2010-133312]. Erfindungsgemäße Verbindungen der allgemeinen Struktur (**I-T4**) können in Analogie zu literaturbekannten Peptidkupplungsmethoden aus den Ausgangsmaterialien (**D6**) und (**D8**) hergestellt werden [WO2010-051926; WO2010-133312].

Die Verbindungen der Struktur (I-T4) können alternativ nach dem im Raktionsschema 5 angegebenen Verfahren hergestellt werden.

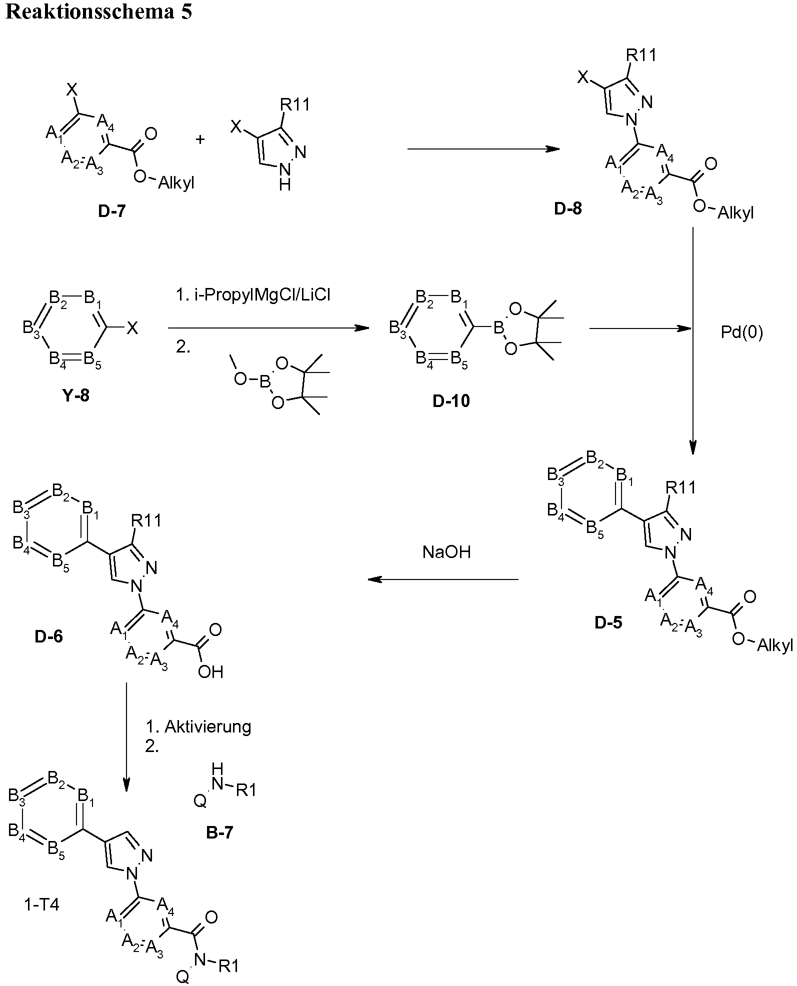

Die Reste A₁ bis A₄, B₁ bis B₅, Alkyl, Q, R¹ und R¹¹ haben die hierin beschriebenen Bedeutungen. X steht für Cl, Br, I. Ausgangsverbindungen der Struktur (**D-7**), (**D-9**) und (**D-11**) (z.B. EP1253128, S. 8-10) sind bekannt und teilweise käuflich oder können nach bekannten Methoden hergestellt werden.

Die Durchführung der Reaktionen kann nach den in der Literatur beschriebenen Verfahren erfolgen:

### Stufe 1 Pyrazol-Kupplung

Stufe 1 des Darstellungsverfahrens der erfindungsgemäßen Verbindungen (**I-T4**):

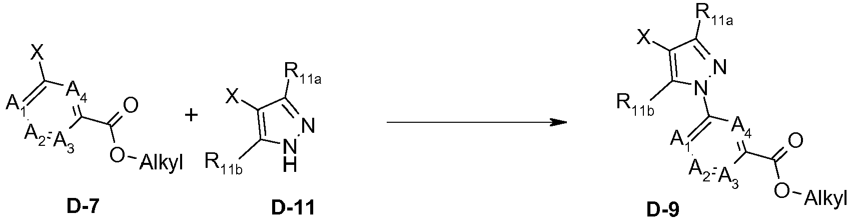

Verbindungen der allgemeinen Struktur (**D-9**) können in Analogie zu literaturbekannten Methoden aus den Ausgangsmaterialien der Struktur (**D-7**) und (**D-11**) hergestellt werden. Die Reste A¹-A⁴, Alkyl und X haben die weiter oben beschriebenen Bedeutungen. Ausgangsverbindungen der Struktur (**D-7**) sind bekannt (z.B. WO2004099146A1, S. 68-69) oder können nach bekannten Methoden hergestellt werden. Als Beispiele seien genannt: 2-Chlor-5-iod-benzoesäuremethylester, 2-Brom-5-iod-benzoesäureethylester, 5-Brom-2-chlor-3-fluor-benzoesäuremethylester, 5-Brom-2-chlor-nicotinsäureethylester. Die Ausgangsverbindungen der Struktur (**D-11**) sind bekannt und teilweise käuflich oder können nach bekannten Methoden hergestellt werden. Als Beispiele seien 4-Brompyrazol, 4-Brom-3-methyl-pyrazol 4-Brom-3,5-dimethylpyrazol und 4-Brom-3-(trifluormethyl)-pyrazol genannt.

Die Herstellung der noch unbekannten Verbindungen (**D-9**) kann in Analogie zu den bekannten Verfahren zur Verknüpfung von Pyrazolen mit Aromaten erfolgen. (z.B. WO2013009791, S. 50, Example 44).

### Pyrazol Alternative Herstellung

Alternativ können die erfindungsgemäßen Verbindungen der allgemeinen Struktur (**D9**) über den im Reaktionsschema 6 angegebenen Weg erhalten werden.

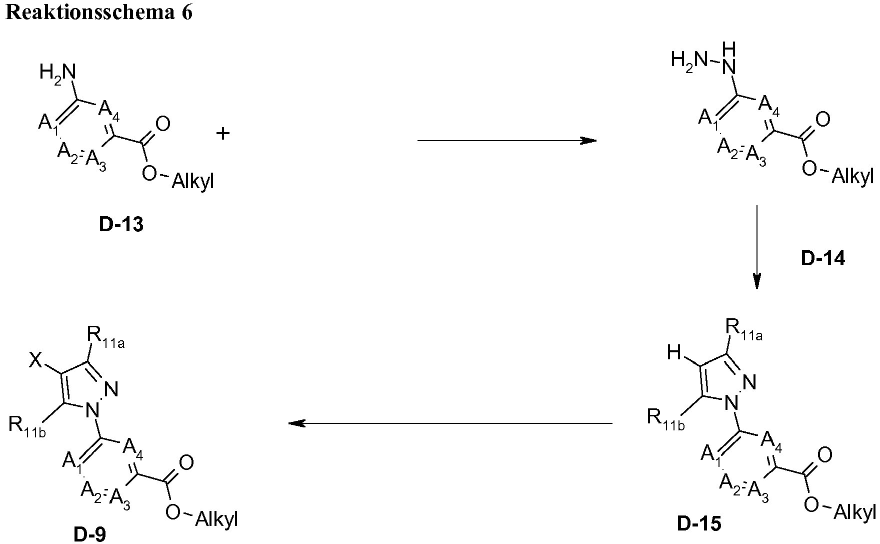

Die Reste A₁ bis A₄, Alkyl, und R₁₁ haben die weiter oben beschriebenen Bedeutungen. X steht für Cl, Br, I. Ausgangsverbindungen der Struktur (**D-13**) sind bekannt (z.B. WO2004099146A1, S. 68-69) oder können nach bekannten Methoden hergestellt werden. Als Beispiele seien genannt: 5-Amino-2-chlor-benzoesäuremethylester, 5-Amino-2-chlor-benzoesäureethylester, 5-Amino-2-chlor-3-fluor-benzoesäuremethylester, 5-Amino-2-chlor-nicotinsäureethylester.

Die Herstellung der noch unbekannten Verbindungen (**D-14**) kann in Analogie zu den bekannten Verfahren zur Herstellung von Arylhydrazinen erfolgen (z.B. WO 2004058731, S.65).

Erfindungsgemäße Verbindungen der allgemeinen Struktur (**D-15**) können in Analogie zu literaturbekannten Methoden aus den Ausgangsmaterialien der Struktur (**D-14**) hergestellt werden. Die Reste A₁ bis A₄, Alkyl, und R₁₁ haben die oben beschriebenen Bedeutungen. Ausgangsverbindungen der Struktur (**D-14**) sind bekannt oder können nach bekannten Methoden hergestellt werden. Als Beispiele seien 2-Chlor-5-hydrazino-benzoesäuremethylester, 2-Chlor-5-hydrazino-benzoesäureethylester, 2-Chlor-3-fluor-5-hydrazino-benzoesäuremethylester, 2-Chlor-5-hydrazino-nicotinsäureethylester genannt. Die Reaktion kann analog zu den in der Literatur bekannten Bedingungen zum Pyrazol Ringschluss durchgeführt werden (z.B. Sachweh, Volker; Langhals, Heinz Chemische Berichte, 119 (1986) 1627-1639).

Erfindungsgemäße Verbindungen der allgemeinen Struktur (**D9**) werden hergestellt indem man die Pyrazole der Struktur (**D-15**) mit halogenierenden Mitteln umsetzt. Die Reste A₁ bis A₄, Alkyl, und R₁₁ haben die oben beschriebenen Bedeutungen. Als bevorzugte Verbindungen der Struktur (**D15**) seien 2-Chlor-5-(pyrazol-1-yl)-benzoesäuremethylester, 2-Chlor-5-(pyrazol-1-yl)-benzoesäureethylester, 2-Chlor-3-fluor-5-(pyrazol-1-yl)-benzoesäuremethylester, 2-Chlor-5-(pyrazol-1-yl)-nicotinsäureethylester genannt

Geeignete halogenierende Verbindungen sind dem Fachmann bekannt wie z.B. z.B. Chlor, Brom, Iod, N-Chlorsuccinimid, N-Bromsuccinimid, N-Iodsuccinimid, 1,3-Dichlor-5,5-dimethylhydantoin, 1,3-Dibrom-5,5-dimethylhydantoin, Natriumhypochlorit und Jodmonochlorid. Bevorzugt werden Brom, Iod, Bromsuccinimid und Iodsuccinimid eingesetzt. Gegebenenfalls ist es vorteilhaft, die Reaktion in Gegenwart eines Oxidationsmittels z.B. Wasserstoffperoxid durchzuführen. Die Umsetzung folgt den aus der Literatur bekannten Bedingungen z. B. Guo Li et al., Tetrahedron Letters 48 (2007), 4595-4599; Mary M. Kim et al., Tetrahedron Letters 49 (2008), 4026-4028.

### Stufe 2 Boronester

Stufe 2: Herstellung der Ausgangsverbindungen der Struktur (**D12**)

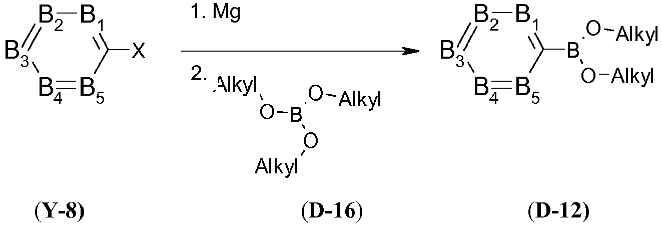

Erfindungsgemäße Verbindungen der allgemeinen Struktur (**D-12**) können in Analogie zu literaturbekannten Methoden (Chien, Yuh-Yih; Chou, Meng-Yen; Leung, Man-Kit; Liao, Yuan-Li; Lin, Chang-Chih; Wong, Ken-Tsung; Journal of Organic Chemistry, 67 (2002) S. 1041-1044) aus den Ausgangsmaterialien der Struktur (**D-10**) durch Umsetzung mit Magnesium und anschliessende Umsetzung mit Borsäureestern der Struktur (**D-16**) hergestellt werden.

Die Reste B¹-B⁵ und Alkyl haben die oben beschriebenen Bedeutungen.

Die in der Reaktion verwendeten Borsäureester der Struktur (**D-13**) sind bekannt oder können nach bekannten Methoden hergestellt werden. Als Beispiele seien Borsäuretrimethylester, Borsäuretriethylester und 2-Methoxy-4,4,5,5-tetramethyl-1,3,2-dioxaborolan genannt.

Alternativ können die erfindungsgemäßen Verbindungen der allgemeinen Struktur (**D-12**) in Analogie zu literaturbekannten Methoden (Tang, Wenjun; Keshipeddy, Santosh; Zhang, Yongda; Wei, Xudong; Savoie, Jolaine; Patel, Nitinchandra D.; Yee, Nathan K.; Senanayake, Chris H.; Organic Letters, 13 (2011) S. 1366-1369) aus den Ausgangsmaterialien der Struktur (**D-10**) durch Umsetzung mit Diboranen der Struktur (**D-14**) in Gegenwart von Katalysatoren hergestellt werden.

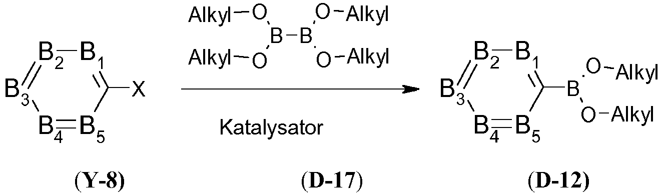

Die in der Reaktion verwendeten Borsäureester der Struktur (**D-17**) sind bekannt oder können nach bekannten Methoden hergestellt werden. Als Beispiele sei 4,4,5,5-Tetramethyl-2-(4,4,5,5-tetramethyl-1,3,2-dioxaborolan-2-yl)-1,3,2-dioxaborolan (Bis(pinacolato)dibor) genannt.

Als Katalysatoren können vor allem Verbindungen und Komplexe von Palladium und Cu(I) verwendet werden.

### Stufe 3 Aryl-Kupplung

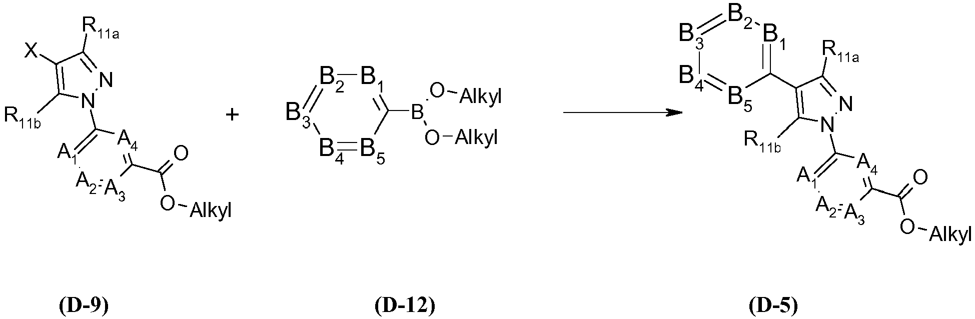

Erfindungsgemäße Verbindungen der allgemeinen Struktur (**D-5**) werden hergestellt indem man die Verbindungen der Struktur (**D-9**) mit Boronsäureestern der Struktur (**D-12**) umsetzt.

Die Reste A¹-A⁴, B¹-B⁵, R¹¹, Alkyl und X haben die weiter oben beschriebenen Bedeutungen.

Die Herstellung der Verbindungen der Strukturen (**D-9**) und (**D-12**) ist weiter oben beschrieben.

Als Beispiele für Verbindungen der Struktur (**D-9**) seien genannt: 5-(4-Brompyrazol-1-yl)-2-chlor-benzoesäuremethylester, 5-(4-Iodpyrazol-1-yl)-2-chlor-benzoesäuremethylester, 5-(4-Brom-3 - methylpyrazol-1-yl)-2-chlor-benzoesäureethylester, 5-(4-Brom-3-(trifluormethyl)pyrazol-1-yl)-2-chlor-benzoesäuremethylester, 5-(4-Brom-3-(trifluormethyl)pyrazol-1-yl)-2-chlor-benzoesäuremethylester, 5-(4-Brom-3,5-dimethylpyrazol-1-yl)-2-chlor-benzoesäuremethylester und 5-(4-Brom-3-methylpyrazol-1-yl)-2-chlor-nikotinsäureethylester.

Die Reaktion wird unter den in der Literatur beschriebenen Bedingungen durchgeführt z.B. WO 2005040110 oder WO 2009089508.

### Stufen 4, 5 Verseifung, Amidierung

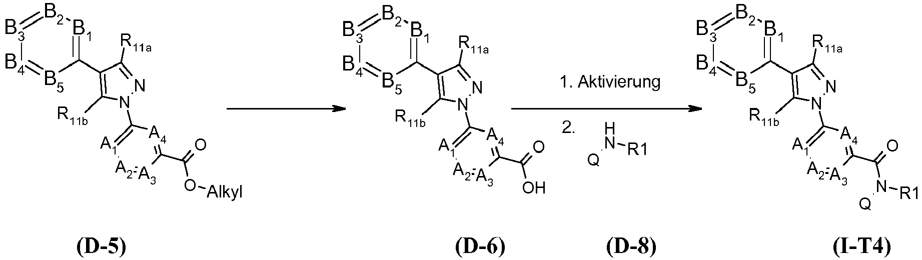

Erfindungsgemäße Verbindungen der allgemeinen Struktur (**I-T4**) können in Analogie zu literaturbekannten Peptidkupplungsmethoden aus den Ausgangsmaterialien (**D-6**) und (**D-8**) hergestellt werden [WO2010051926; WO2010133312]. Verbindungen der allgemeinen Struktur (**D-6**) lassen sich in Analogie zu literaturbekannten Verfahren durch Esterspaltung aus Verbindungen der allgemeinen Struktur (**D-5**) herstellen [WO2010-051926; WO2010133312]. Die Reste A₁-A₄, B₁-B₅, Alkyl, Q, R¹ und R¹¹ haben die oben beschriebenen Bedeutungen. Die Herstellung der Verbindungen der Struktur (**D5**) ist weiter oben beschrieben.
(I-T5): Verbindungen der Formel (I-T5) können z.B. analog zu Friedrich, L.E. et al. Journal of Organic Chemistry, 43 (1978), 34-38; oder Huettel, R. et al. Chemische Berichte, 93 (1960), S. 1425-1432; oder Sato, T et al., Bulletin of the Chemical Society of Japan, 41 (1968), S. 3017-3018 hergestellt werden.
(I-T8): Verbindungen der Formel (I-T8) können z.B. analog zu EP 1 405 636, Example 5; oder EP 2 301 538, S. 162; oder Schmidt, Bernd et al., European Journal of Organic Chemistry, (2011), S. 4814-4822 hergestellt werden.
(I-T9): Verbindungen der Formel (I-T9) können z.B. analog zu Ma, Shengming et al., Chemistry-A European Journal, 9 (2003), S. 2447-2456 hergestellt werden.
(I-T10): Verbindungen der Formel (I-T10) können z.B. analog zu EP 2 301 538, S. 162 hergestellt werden.
(I-T11): Verbindungen der Formel (I-T11) können z.B. analog zu EP 2 301 538, S. 165 hergestellt werden.
(I-T12): Verbindungen der Formel (I-T12) können z.B. analog zu EP 2 301 538, S. 164 hergestellt werden.
(I-T13): Verbindungen der Formel (I-T13) können z.B. analog zu EP 2 301 538, S. 164 hergestellt werden.
(I-T14): Verbindungen der Formel (I-T14) können z.B. analog zu Hibi, Shigeki et al., Bioorganic & Medicinal Chemistry Letters, 10 (2000), S. 623-626 oder Wang, Xiang et al. Journal of Organic Chemistry, 72 (2007), 1476-1479; EP1405636, Seite 31 hergestellt werden.
(I-T15): Verbindungen der Formel (I-T15) können z.B. analog zu Chattopadhyay, Buddhadeb et al., Organic Letters, 13 (2011), S. 3746-3749 hergestellt werden.
(I-T16): Verbindungen der Formel (I-T16) können z.B. analog zu Campi, Eva M. et al. Tetrahedron Letters, 32 (1991), S. 1093-1094; oder Thompson, Benjamin B. et al., Organic Letters, 13 (2011), S. 3289-3291; oder Kloetzel et al. Journal of the American Chemical Society, 79 (1957), S. 4222; oder Chi, Yonggui Robin et al., Journal of the American Chemical Society, 135 (2013), S. 8113-8116 hergestellt werden.
(I-T18): Verbindungen der Formel (I-T18) können z.B. analog zu EP 2 311 455, S. 150; oder Balaban, A.T. et al. Tetrahedron, 19 (1963), S. 2199-2207 hergestellt werden.
(I-T19): Verbindungen der Formel (I-T19) können z.B. analog zu WO 2004/14366, S. 108 hergestellt werden.
(I-T20): Verbindungen der Formel (I-T20) können z.B. analog zu Araki, Hiroshi; Katoh, Tadashi; Inoue, Munenori; Synlett, (2006), S. 555-558; US 6,545,009, S. 27, Example 1 hergestellt werden.
(I-T21): Verbindungen der Formel (I-T21) können z.B. analog zu WO 2004/72050, S. 13; oder US 6,545,009, S. 27 hergestellt werden.

### Verfahren I-T22

Die Verbindungen der Struktur **(I-T22**) können nach dem im Schema 7 angegebenen Verfahren hergestellt werden.

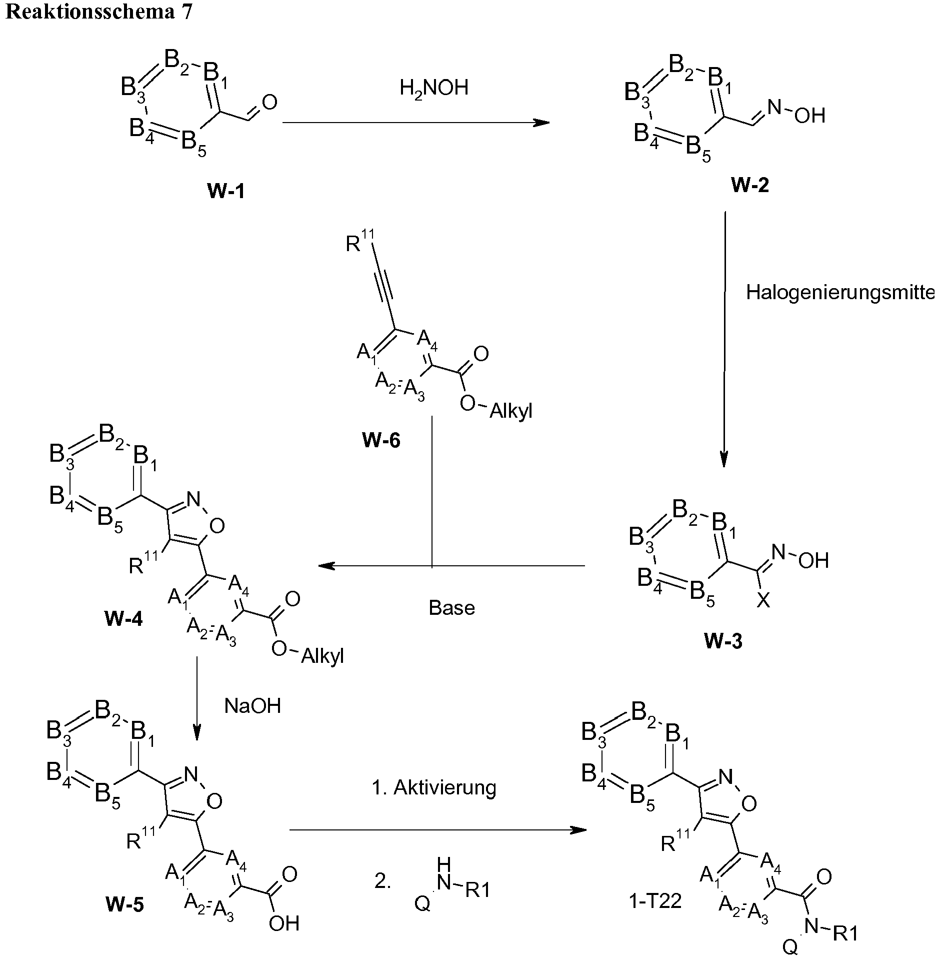

Die Reste A₁-A₄, B₁-B₅, Alkyl, Q, R¹ und R¹¹ haben die oben beschriebenen Bedeutungen. X steht für Cl, Br, I. Ausgangsverbindungen der Struktur (W-1) und (W-6) sind bekannt (W1 z.B. US 2011/53904 S. 19, W6 z.B. WO 2012/175474, S. 117-118) oder können nach bekannten Methoden hergestellt werden. Die Reaktionen werden analog den zur Herstellung der Verbindungen (**I-T23**) angegebenen Bedingungen durchgeführt.

### Stufe 1 Aldehyd

Stufe 1 des Darstellungsverfahrens der erfindungsgemäßen Verbindungen (**I-T22**):

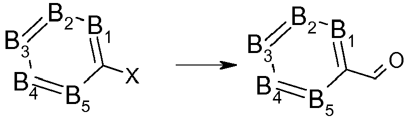

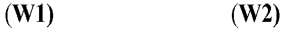

Erfindungsgemäße Verbindungen der allgemeinen Struktur (**W2**) können in Analogie zu literaturbekannten Methoden (US5739083, Example 2; WO2011/23667, S. 34) aus den Ausgangsmaterialien der Struktur (**W1**) hergestellt werden.

Die Reste B¹-B⁵ und X haben die oben beschriebenen Bedeutungen. X steht z.B. für Chlor, Brom oder Iod.

Ausgangsverbindungen der Struktur (**B1**) sind bekannt oder können nach bekannten Methoden hergestellt werden. Als Beispiele seien 2-Brom-1,3-dichlor-5-[1,2,2,2-tetrafluor-1-(trifluormethyl)ethyl]benzol, 2-Brom-1,3-dimethyl-5-[1,2,2,2-tetrafluor-1-(trifluormethyl)ethyl]benzol, 2-Brom-1-ethyl-3-methyl-5-[1,2,2,2-tetrafluor-1-(trifluormethyl)ethyl]benzol, 2-Brom-1-chlor-5-[1,2,2,2-tetrafluor-1-(trifluormethyl)ethyl]-3-(trifluormethyl)benzol, 2-Brom-1-methyl-5-[1,2,2,2-tetrafluor-1-(trifluormethyl)ethyl]-3-(trifluormethyl)benzol, 2-Brom-1-chlor-5-[1,2,2,2-tetrafluor-1-(trifluormethyl)ethyl]-3-(trifluormethoxy)benzol, 2-Brom-1-methyl-5-[1,2,2,2-tetrafluor-1-(trifluormethyl)ethyl]-3-(trifluormethoxy)benzol, 1,3-Dimethyl-2-iod-5-[1,2,2,2-tetrafluor-l-(trifluormethyl)ethyl]benzol, 2-Iod-1-methyl-5-[1,2,2,2-tetrafluor-1-(trifluormethyl)ethyl]-3-(trifluormethyl)benzol genannt. Sie können z.B. nach den in EP1253128, Seiten 8-10 beschriebenen Methoden hergestellt werden.

### Stufe 2 Oxim

Stufe 2 des Darstellungsverfahrens der erfindungsgemäßen Verbindungen (**1-T22**):

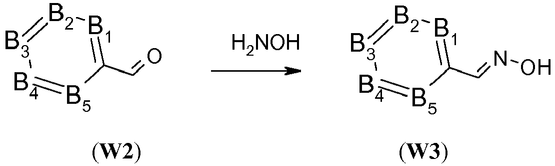

Erfindungsgemäße Verbindungen der allgemeinen Struktur (**W3**) können in Analogie zu literaturbekannten Methoden aus den Ausgangsmaterialien der Struktur (**W2**) hergestellt werden. Die Reste B₁-B₅ haben die oben beschriebenen Bedeutungen. Die Herstellung der Ausgangsverbindungen der Struktur (**W2**) ist weiter oben beschrieben. Als Beispiele seien 2,6-Dichlor-4-[1,2,2,2-tetrafluor-1-(trifluormethyl)ethyl]benzaldehyd, 2,6-Dimethyl-4-[1,2,2,2-tetrafluor-1-(trifluormethyl)ethyl]benzaldehyd, 2-Ethyl-6-methyl-5-[1,2,2,2-tetrafluor-1-(trifluormethyl)ethyl]benzaldehyd, 2-Chlor-4-[1,2,2,2-tetrafluor-1-(trifluormethyl)ethyl]-6-(trifluormethyl)benzaldehyd, 2-Methyl-4- [1,2,2,2-tetrafluor-1-(trifluormethyl)ethyl] -6-(trifluormethyl)benzaldehyd, 2-Chlor-4-[1,2,2,2-tetrafluor-1-(trifluormethyl)ethyl]-6-(trifluormethoxy)benzaldehyd, 2-Methyl-4- [1,2,2,2-tetrafluor-1 -(trifluormethyl)ethyl] -6-(trifluormethoxy)benzaldehyd, genannt. Ihre Herstellung ist weiter oben beschrieben.

Die Verbindungen der Strukturformel (**W3**) sind neu. Die Herstellung der noch unbekannten Verbindungen (**W3**) kann in Analogie zu den bekannten Verfahren zur Herstellung von Oximen aus Aldehyden erfolgen (H. Metzger in Houben-Weyl, Band X/4, Seite 55 ff, Georg Thieme Verlag Stuttgart 1968). Die Verbindungen der Strukturformel (**W3**) können als reine Stereoisomere, aber auch als Mischungen der Stereoisomere vorliegen.

### Stufe 3 Hydroxamsäurechlorid

Stufe 3 des Darstellungsverfahrens der erfindungsgemäßen Verbindungen (**1-T22**):

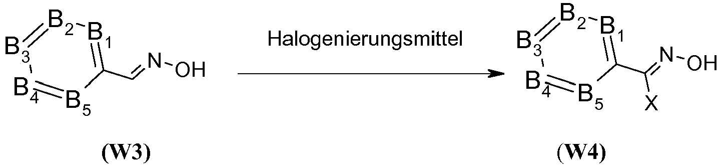

Erfindungsgemäße Verbindungen der allgemeinen Struktur (**W4**) werden hergestellt indem man die Oxime der Struktur (**W3**) mit halogenierenden Mitteln umsetzt.

Die Reste B1-B5 haben die oben beschriebenen Bedeutungen. X ist Chlor, Brom oder Iod.

Typische Verbindungen der Struktur (**W4**) sind z.B. 2,6-Dichlor-4-[1,2,2,2-tetrafluor-1-(trifluormethyl)ethyl]-N-hydroxybenzimidoylchlorid, 2,6-Dimethyl-4-[1,2,2,2-tetrafluor-1-(trifluormethyl)ethyl]-N-hydroxybenzimidoylchlorid, 2-Ethyl-6-methyl-5-[1,2,2,2-tetrafluor-1-(trifluormethyl)ethyl]-N-hydroxybenzimidoylchlorid, 2-Chlor-4-[1,2,2,2-tetrafluor-1-(trifluormethyl)ethyl]-6-(trifluormethyl)-N-hydroxybenzimidoylchlorid, 2-Methyl-4-[1,2,2,2-tetrafluor-1-(trifluormethyl)ethyl]-6-(trifluormethyl)-N-hydroxybenzimidoylchlorid, 2-Chlor-4-[1,2,2,2-tetrafluor-1-(trifluormethyl)ethyl]-6-(trifluormethoxy)-N-hydroxybenzimidoylchlorid, 2-Methyl-4-[1,2,2,2-tetrafluor-1-(trifluormethyl)ethyl]-6-(trifluormethoxy)-N-hydroxybenzimidoylchlorid, 2-Methyl-4-[1,2,2,2-tetrafluor-1-(trifluormethyl)ethyl]-6-(trifluormethyl)-N-hydroxybenzimidoylbromid.

Geeignete halogenierende Verbindungen sind dem Fachmann bekannt wie z.B. Chlor, Brom, Iod, N-Chlorsuccinimid, N-Bromsuccinimid, N-Iodsuccinimid, 1,3-Dichlor-5,5-dimethylhydantoin, 1,3-Dibrom-5,5-dimethylhydantoin, Benzyltrimethylammonium-tetrachloroiodat und Natriumhypochlorit. Bevorzugt werden chlorierende Reagenzien eingesetzt.

Zur Durchführung der Reaktion können geeignete Lösungsmittel verwendet werden.

Als Verdünnungs- bzw. Lösungsmittel zur Durchführung der erfindungsgemäßen Verfahren kommen grundsätzlich alle unter den spezifischen Reaktionsbedingungen inerten organischen Lösungsmittel in Frage. Als Beispiele sind zu nennen: Halogenkohlenwasserstoffe (z.B. Chlorkohlenwasserstoffe, wie Tetraethylen, Tetrachlorethan, Dichlorpropan, Methylenchlorid, Dichlorbutan, Chloroform, Tetrachlorkohlenstoff, Trichlorethan, Trichlorethylen, Pentachlorethan, Difluorbenzol, 1,2-Dichlorethan, Chlorbenzol, Brombenzol, Dichlorbenzol, Chlortoluol, Trichlorbenzol), Alkohole (z.B. Methanol, Ethanol, Isopropanol, Butanol), Ether (z.B. Ethylpropylether, Methyl-tert-butylether, n-Butylether, Anisol, Phenetol, Cyclohexylmethylether, Dimethylether, Diethylether, Dipropylether, Diisopropylether, Di-n-butylether, Diisobutylether, Diisoamylether, Ethylenglycoldimethylether, Tetrahydrofuran, Dioxan, Dichlordiethylether und Polyether des Ethylenoxids und/oder Propylenoxids), Amine (z.B. Trimethyl-, Triethyl-, Tripropyl-, Tributylamin, N-Methylmorpholin, Pyridin und Tetramethylendiamin), Nitrokohlenwasserstoffe (z.B. Nitromethan, Nitroethan, Nitropropan, Nitrobenzol, Chlornitrobenzol, o-Nitrotoluol; Nitrile wie Acetonitril, Propionitril, Butyronitril, Isobutyronitril, Benzonitril, m-Chlorbenzonitril), Tetrahydrothiophendioxid, Dimethylsulfoxid, Tetramethylensulfoxid, Dipropylsulfoxid, Benzylmethylsulfoxid, Diisobutylsulfoxid, Dibutylsulfoxid, Diisoamylsulfoxid, Sulfone (z.B. Dimethyl-, Diethyl-, Dipropyl-, Dibutyl-, Diphenyl-, Dihexyl-, Methylethyl-, Ethylpropyl-, Ethylisobutyl- und Pentamethylensulfon), aliphatische, cycloaliphatische oder aromatische Kohlenwasserstoffe (z.B. Pentan, Hexan, Heptan, Oktan, Nonan und technische Kohlenwasserstoffe), ferner sogenannte "White Spirits" mit Komponenten mit Siedepunkten im Bereich von beispielsweise 40 °C bis 250 °C, Cymol, Benzinfraktionen innerhalb eines Siedeinterwalles von 70°C bis 190 °C, Cyclohexan, Methylcyclohexan, Petrolether, Ligroin, Octan, Benzol, Toluol, Chlorbenzol, Brombenzol, Nitrobenzol, Xylol, Ester (z.B. Methyl-, Ethyl-, Butyl-, Isobutylacetat, Dimethyl-, Dibutyl-, Ethylencarbonat); Amide (z.B. Hexamethylenphosphorsäuretriamid, Formamid, N-Methyl-formamid, N,N-Dimethyl-formamid, N,N-Dipropyl-formamid, N,N-Dibutyl-formamid, N-Methyl-pyrrolidin, N-Methyl-caprolactam, 1,3-Dimethyl-3,4,5,6-tetrahydro-2(1H)-pyrimidin, Octylpyrrolidon, Octylcaprolactam, 1,3-Dimethyl-2-imidazolindion, N-Formyl-piperidin, N,N'-1,4-Diformyl-piperazin) und Ketone (z.B. Aceton, Acetophenon, Methylethylketon, Methylbutylketon).

Als bevorzugtes Verdünnungsmittel kann jedes Lösungsmittel verwendet werden, das die Reaktion nicht beeinträchtigt, wie zum Beispiel Wasser. In Frage kommen aromatische Kohlenwasserstoffe wie Benzol, Toluol, Xylol oder Chlorbenzol; halogenierte Kohlenwasserstoffe wie Dichlormethan, Chloroform, 1,2-Dichlorethan oder Tetrachlorkohlenwasserstoff, offenkettige oder zyklische Ether wie Diethylether, Dioxan, Tetrahydrofuran oder 1,2-Dimethoxyethan; Ester wie Ethylacetat und Butylacetat; Ketone wie zum Beispiel Aceton, Methyl-isobutylketon und Cyclohexanon; Amide wie Dimethylformamid und Dimethylacetamid, N-Methyl-pyrrolidinon; Nitrile wie Acetonitril oder Propionitril; und andere inerte Lösungsmittel wie 1,3-Dimethyl-2-imidazolidinon; die Lösungsmittel können allein oder in Kombination von 2 oder mehr eingesetzt werden.

Die Reaktion kann in einem weiten Temperaturbereich ausgeführt werden. Gewöhnlich wird sie in einem Temperaturbereich von -78 bis 200 °C, bevorzugt bei Temperaturen zwischen -10 und 150 °C durchgeführt. Die Reaktion kann unter erhöhtem als auch vermindertem Druck ausgeführt werden. Bevorzugt wird sie aber unter Normaldruck durchgeführt. Die Reaktionszeiten liegen zwischen 0,1 und 72 Stunden, bevorzugt zwischen 1 und 24 Stunden.

Zur Durchführung der Reaktion werden 1 bis 3 Mol, bevorzugt 1 bis 1,5 Mol an Halogenierungsmittel pro Mol der Verbindung der Struktur (**W3**) in einem Lösungsmittel wie z.B. Dimethylformamid (DMF) umgesetzt.

### Stufe 4 Ringschluss

Stufe 4 des Darstellungsverfahrens der erfindungsgemäßen Verbindungen (**1-T22**): 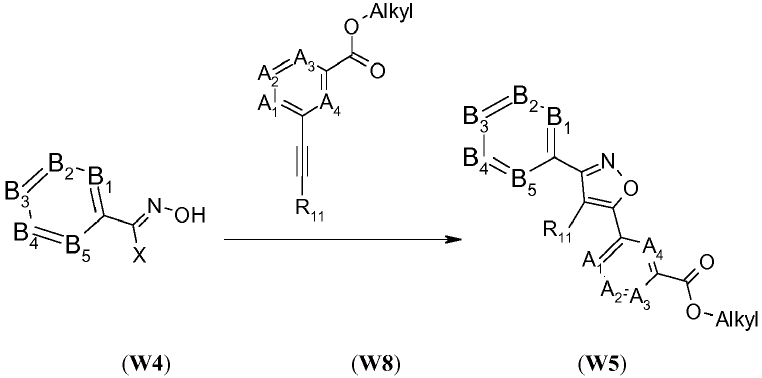 Erfindungsgemäße Verbindungen der allgemeinen Struktur (**W5**) werden hergestellt indem man die Hydroxamsäurechloride der Struktur (**W4**) mit Acetylenen der Struktur (**W8**) umsetzt.

Die Reste A₁-A₄, B₁-B₅, R¹¹ und Alkyl haben die oben beschriebenen Bedeutungen. X steht für Halogen, wie Chlor, Brom, Iod.

Die Herstellung der Verbindungen der Struktur (**W4**) ist weiter oben beschrieben. Typische Verbindungen der Struktur (**W4**) sind z.B. 2,6-Dichlor-4-[1,2,2,2-tetrafluor-1-(trifluormethyl)ethyl]-N-hydroxybenzimidoylchlorid, 2,6-Dimethyl-4-[1,2,2,2-tetrafluor-1-(trifluormethyl)ethyl]-N-hydroxybenzimidoylchlorid, 2-Ethyl-6-methyl-5-[1,2,2,2-tetrafluor-1-(trifluormethyl)ethyl]-N-hydroxybenzimidoylchlorid, 2-Chlor-4-[1,2,2,2-tetrafluor-1-(trifluormethyl)ethyl]-6-(trifluormethyl)-N-hydroxybenzimidoylchlorid, 2-Methyl-4-[1,2,2,2-tetrafluor-1-(trifluormethyl)ethyl]-6-(trifluormethyl)-N-hydroxybenzimidoylchlorid, 2-Chlor-4-[1,2,2,2-tetrafluor-1-(trifluormethyl)ethyl]-6-(trifluormethoxy)-N-hydroxybenzimidoylchlorid, 2-Methyl-4-[1,2,2,2-tetrafluor-1-(trifluormethyl)ethyl]-6-(trifluormethoxy)-N-hydroxybenzimidoylchlorid, 2-Methyl-4-[1,2,2,2-tetrafluor-1-(trifluormethyl)ethyl]-6-(trifluormethyl)-N-hydroxybenzimidoylbromid.

Die Verbindungen der Struktur (**W8**) sind bekannt (WO2012107434, S. 103) oder können nach literaturbekannten Methoden (Chinchilla, Rafael; Najera, Carmen, Chemical Society Reviews (2011), 40(10), 5084-5121, Chinchilla, Rafael; Najera, Carmen, Chemical Reviews (Washington, DC, United States) (2007), 107(3), 874-922) hergestellt werden. Typische Verbindungen der Struktur (**W8**) sind z.B. 2-Chlor-5-ethinyl-benzoesäuremethylester, 2-Brom-5-ethinyl-benzoesäureethylester, 2-Chlor-5-ethinyl-3-fluor-benzoesäuremethylester, 2-Chlor-5-ethinyl-nicotinsäureethylester, 5-Ethinyl-2-methyl-nicotinsäureethylester

Zur Durchführung der Reaktion können geeignete Lösungsmittel verwendet werden.

Als Verdünnungs- bzw. Lösungsmittel zur Durchführung der erfindungsgemäßen Verfahren kommen grundsätzlich alle unter den spezifischen Reaktionsbedingungen inerten organischen Lösungsmittel in Frage. Als Beispiele sind zu nennen: Halogenkohlenwasserstoffe (z.B. Chlorkohlenwasserstoffe, wie Tetraethylen, Tetrachlorethan, Dichlorpropan, Methylenchlorid, Dichlorbutan, Chloroform, Tetrachlorkohlenstoff, Trichlorethan, Trichlorethylen, Pentachlorethan, Difluorbenzol, 1,2-Dichlorethan, Chlorbenzol, Brombenzol, Dichlorbenzol, Chlortoluol, Trichlorbenzol), Alkohole (z.B. Methanol, Ethanol, Isopropanol, Butanol), Ether (z.B. Ethylpropylether, Methyl-tert-butylether, n-Butylether, Anisol, Phenetol, Cyclohexylmethylether, Dimethylether, Diethylether, Dipropylether, Diisopropylether, Di-n-butylether, Diisobutylether, Diisoamylether, Ethylenglycoldimethylether, Tetrahydrofuran, Dioxan, Dichlordiethylether und Polyether des Ethylenoxids und/oder Propylenoxids), Amine (z.B. Trimethyl-, Triethyl-, Tripropyl-, Tributylamin, N-Methylmorpholin, Pyridin und Tetramethylendiamin), Nitrokohlenwasserstoffe (z.B. Nitromethan, Nitroethan, Nitropropan, Nitrobenzol, Chlornitrobenzol, o-Nitrotoluol; Nitrile wie Acetonitril, Propionitril, Butyronitril, Isobutyronitril, Benzonitril, m-Chlorbenzonitril), Tetrahydrothiophendioxid, Dimethylsulfoxid, Tetramethylensulfoxid, Dipropylsulfoxid, Benzylmethylsulfoxid, Diisobutylsulfoxid, Dibutylsulfoxid, Diisoamylsulfoxid, Sulfone (z.B. Dimethyl-, Diethyl-, Dipropyl-, Dibutyl-, Diphenyl-, Dihexyl-, Methylethyl-, Ethylpropyl-, Ethylisobutyl- und Pentamethylensulfon), aliphatische, cycloaliphatische oder aromatische Kohlenwasserstoffe (z.B. Pentan, Hexan, Heptan, Oktan, Nonan und technische Kohlenwasserstoffe), ferner sogenannte "White Spirits" mit Komponenten mit Siedepunkten im Bereich von beispielsweise 40 °C bis 250 °C, Cymol, Benzinfraktionen innerhalb eines Siedeinterwalles von 70 °C bis 190 °C, Cyclohexan, Methylcyclohexan, Petrolether, Ligroin, Octan, Benzol, Toluol, Chlorbenzol, Brombenzol, Nitrobenzol, Xylol, Ester (z.B. Methyl-, Ethyl-, Butyl-, Isobutylacetat, Dimethyl-, Dibutyl-, Ethylencarbonat); Amide (z.B. Hexamethylenphosphorsäuretriamid, Formamid, N-Methyl-formamid, N,N-Dimethyl-formamid, N,N-Dipropyl-formamid, N,N-Dibutyl-formamid, N-Methyl-pyrrolidin, N-Methyl-caprolactam, 1,3-Dimethyl-3,4,5,6-tetrahydro-2(1H)-pyrimidin, Octylpyrrolidon, Octylcaprolactam, 1,3-Dimethyl-2-imidazolindion, N-Formyl-piperidin, N,N'-1,4-Diformyl-piperazin) und Ketone (z.B. Aceton, Acetophenon, Methylethylketon, Methylbutylketon).

Als bevorzugte-Verdünnungsmittel kann jedes Lösungsmittel verwendet werden, das die Reaktion nicht beeinträchtigt, wie zum Beispiel Wasser. In Frage kommen aromatische Kohlenwasserstoffe wie Benzol, Toluol, Xylol oder Chlorbenzol; halogenierte Kohlenwasserstoffe wie Dichlormethan, Chloroform, 1,2-Dichlorethan oder Tetrachlorkohlenwasserstoff, offenkettige oder zyklische Ether wie Diethylether, Dioxan, Tetrahydrofuran oder 1 ,2-Dimethoxyethan; Ester wie Ethylacetat und Butylacetat; Ketone wie zum Beispiel Aceton, Methyl-isobutylketon und Cyclohexanon; Amide wie Dimethylformamid und Dimethylacetamid, N-Methyl-pyrrolidinon; Nitrile wie Acetonitril oder Propionitril; und andere inerte Lösungsmittel wie 1,3-Dimethyl-2- imidazolidinon; die Lösungsmittel können allein oder in Kombination von 2 oder mehr eingesetzt werden.

Bei den Umsetzungen der Verbindungen der Struktur (**W4**) mit den Acetylenen der Struktur (**W8**) können Basen zugesetzt werden. Als Beispiele sind zu nennen Erdalkali- oder Alkalimetallverbindungen (z.B. Hydroxide, Hydride, Oxide und Carbonate des Lithiums, Natriums, Kaliums, Magnesiums, Calciums und Bariums), Amidinbasen oder Guanidinbasen (z.B. 7-Methyl-1,5,7-triaza-bicyclo(4.4.0)dec-5-en (MTBD); Diazabicyclo(4.3.0)nonen (DBN), Diazabicyclo(2.2.2)octan (DABCO), 1,8-Diazabicyclo(5.4.0)undecen (DBU), Cyclohexyltetra-butyl-guanidin (CyTBG), Cyclohexyltetramethylguanidin (CyTMG), N,N,N,N-Tetramethyl-1,8-naphthalindiamin, Pentamethylpiperidin) und Amine, insbesondere tertiäre Amine, (z.B. Triethylamin, Trimethylamin, Tribenzylamin, Triisopropylamin, Tributylamin, Tricyclohexylamin, Triamylamin, Trihexylamin, N,N-Dimethylanilin, N,N-Dimethyl-toluidin, N,N-Dimethyl-p-aminopyridin, N-Methyl-pyrrolidin, N-Methyl-piperidin, N-Methyl-imidazol, N-Methyl-pyrazol, N-Methyl-morpholin, N-Methylhexamethylendiamin, Pyridin, 4-Pyrrolidinopyridin, 4-Dimethyl-amino-pyridin, Chinolin, α-Picolin, β-Picolin, Isochinolin, Pyrimidin, Acridin, N,N,N',N'-Tetra-methylendiamin, N,N,N',N'-Tetraethylendiamin, Chinoxalin, N-Propyl-diisopropylamin, N-Ethyl-diisopropylamin, N,N'-Dimethylcyclohexylamin, 2,6-Lutidin, 2,4-Lutidin oder Triethyldiamin).

Als bevorzugtes basisches Reaktionshilfsmittel kann eine organische Base wie Triethylamin, Ethyl-diisopropylamin, Tri-n-butylamin, Pyridin und 4-Dimethylamino-pyridin verwendet werden; Des Weiteren können beispielsweise folgende Basen eingesetzt werden: Alkalimetallhydroxide wie z.B Natriumhydroxid und Kaliumhydroxid; Carbonate wie Natriumhydrogencarbonat und Kaliumcarbonat; Phosphate wie Dikalium-hydrogenphosphat und Tri-natriumphosphat.

Die Reaktion kann in einem weiten Temperaturbereich ausgeführt werden. Gewöhnlich wird sie in einem Temperaturbereich von -78 bis 200 °C, bevorzugt bei Temperaturen zwischen -10 und 150 °C durchgeführt. Die Reaktion kann unter erhöhtem als auch vermindertem Druck ausgeführt werden. Bevorzugt wird sie aber unter Normaldruck durchgeführt. Die Reaktionszeiten liegen zwischen 0,1 und 72 Stunden, bevorzugt zwischen 1 und 24 Stunden.

Zur Durchführung der Reaktion werden z.B. 1-2 Moläquivalente der Verbindungen der Struktur (**W8**) und 1 Moläquivalent bis zu einem leichten Überschuss an Base pro Mol der Verbindung der Struktur (**W4**) in einem Lösungsmittel wie z.B. Dimethylformamid (DMF) umgesetzt

Die Stufen 3 und 4 zur Herstellung der Verbindungen der Struktur (**W5**) können in einzelnen Schritten oder auch als Eintopf-Reaktion durchgeführt werden.

### Stufen 5, 6 Verseifung, Amidierung

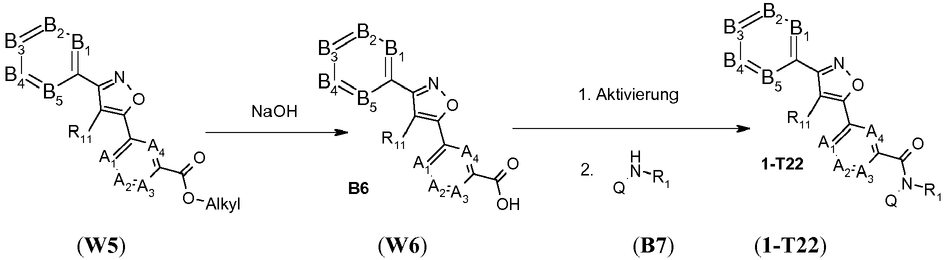

Erfindungsgemäße Verbindungen der allgemeinen Struktur (**I-T22**) können in Analogie zu literaturbekannten Peptidkupplungsmethoden aus den Ausgangsmaterialien (**W6**) und (**W9**) hergestellt werden (WO2010051926; WO2010133312). Verbindungen der allgemeinen Struktur (**W6**) lassen sich in Analogie zu literaturbekannten Verfahren durch Esterspaltung aus Verbindungen der allgemeinen Struktur (**W5**) herstellen (WO2010051926; WO2010133312). Die Reste A₁-A₄, B₁-B₅, Alkyl, Q, R¹ und R¹¹ haben die oben beschriebenen Bedeutungen.

### Verfahren I-T23

Die Verbindungen der Struktur (**I-T23**) können nach dem im Reaktionsschema 8 angegebenen Verfahren hergestellt werden.

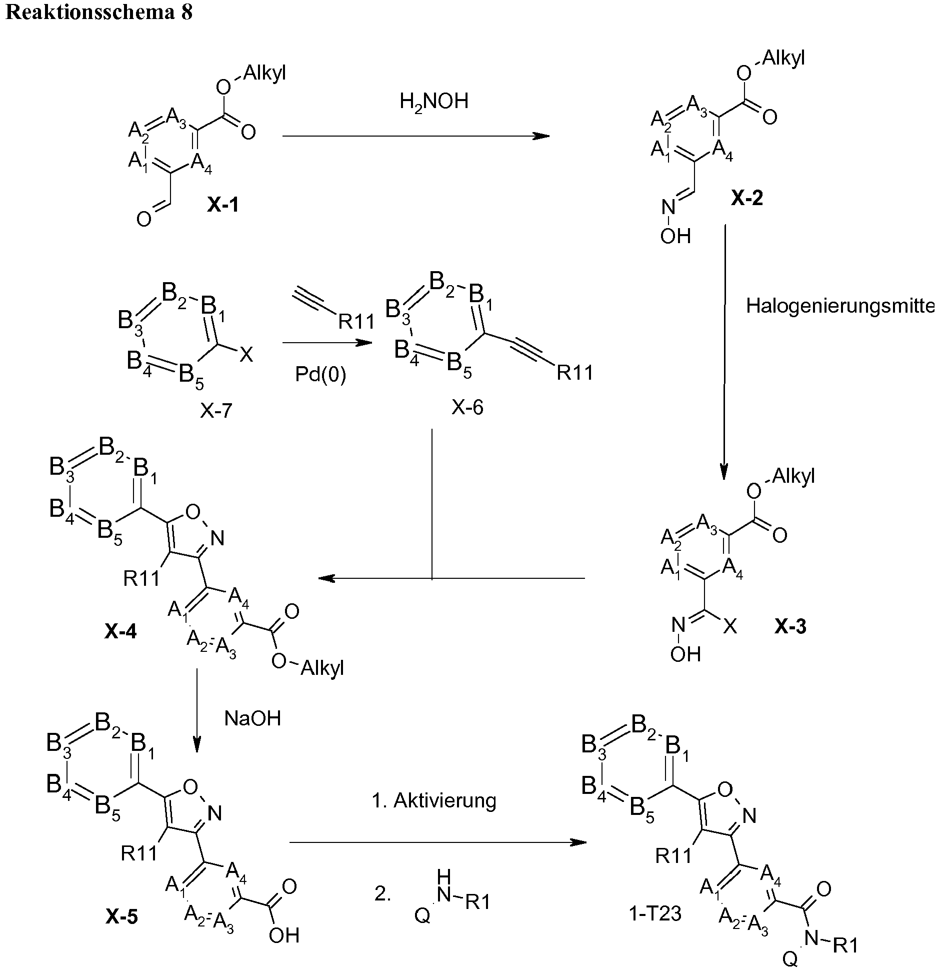

Die Reste A₁-A₄, B₁-B₅, Alkyl, Q, R¹ und R¹¹ haben die oben beschriebenen Bedeutungen. X steht z. B. für Cl, Br, I.

### Stufe 1 Oxim

Stufe 1 des Darstellungsverfahrens der erfindungsgemäßen Verbindungen (I-T23):

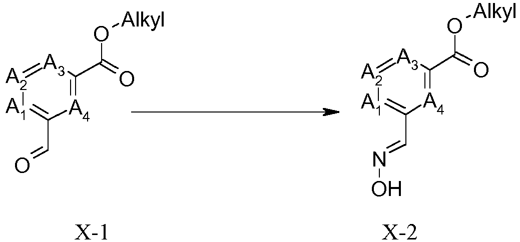

Erfindungsgemäße Verbindungen der allgemeinen Struktur (**X-2**) können in Analogie zu literaturbekannten Methoden aus den Ausgangsmaterialien der Struktur (**X-1**) hergestellt werden. Die Reste A¹-A⁴ und Alkyl haben die oben beschriebenen Bedeutungen. Ausgangsverbindungen der Struktur (**X-1**) sind bekannt oder können nach bekannten Methoden hergestellt werden. Als Beispiele seien 3-Carbomethoxy-benzaldehyd, 3-Carbomethoxy-4-chlor-benzaldehyd, 3-Carbomethoxy-4-brom-benzaldehyd,3-Carbomethoxy-4-fluor-benzaldehyd, 3-Carbomethoxy-4-chlor-5-fluor-benzaldehyd bzw. die entsprechenden Ethylester genannt. Sie können z.B. nach den in WO 2010/011584, S. 19-20; Journal of Organic Chemistry, 76 (2011), S. 1062 - 1071; WO 2012/114268, S. 137; Journal of the American Chemical Society, 108 (1986), S. 452-461 beschriebenen Methoden hergestellt werden.

Die Herstellung der noch unbekannten Verbindungen **(X-2)** kann in Analogie zu den bekannten Verfahren zur Herstellung von Oximen aus Aldehyden erfolgen (H. Metzger in Houben-Weyl, Band X/4, S. 55 ff, Georg Thieme Verlag Stuttgart 1968). Die Verbindungen der Strukturformel **(X-2)** können als reine Stereoisomere, aber auch als Mischungen der Stereoisomere vorliegen.

### Stufe 2 Hydroxamsäurechlorid

Stufe 2 des Darstellungsverfahrens der erfindungsgemäßen Verbindungen **(I-T23):**

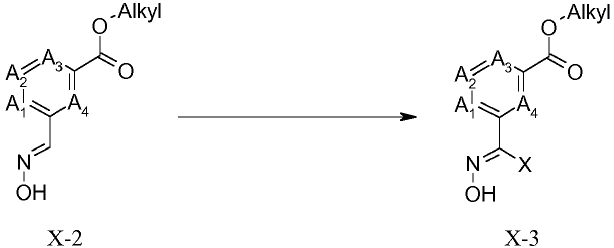

Erfindungsgemäße Verbindungen der allgemeinen Struktur **(X-3)** werden hergestellt indem man die Oxime der Struktur **(X-2)** mit halogenierenden Mitteln umsetzt.

Die Reste A₁-A₄ und Alkyl haben die oben beschriebenen Bedeutungen.

Typische Verbindungen der Struktur **(X-3)** sind z.B. Carbomethoxy-4-chlor-N-hydroxybenzimidoylchlorid, 3-Carbomethoxy-4-fluor- N-hydroxybenzimidoylchlorid, 3-Carbomethoxy-4-chlor-5-fluor- N-hydroxybenzimidoylchlorid, 3-Carbomethoxy-4-brom- N-hydroxybenzimidoylchlorid.

Geeignete halogenierende Verbindungen sind dem Fachmann bekannt wie z.B. Chlor, Brom, Iod, N-Chlorsuccinimid, N-Bromsuccinimid, N-Iodsuccinimid, 1,3-Dichlor-5,5-dimethylhydantoin, 1,3-Dibrom-5,5-dimethylhydantoin, Benzyltrimethylammonium-tetrachloroiodat und Natriumhypochlorit. Bevorzugt werden chlorierende Reagenzien eingesetzt.

Als Verdünnungs- bzw. Lösungsmittel zur Durchführung der erfindungsgemäßen Verfahren kommen grundsätzlich alle unter den spezifischen Reaktionsbedingungen inerten organischen Lösungsmittel in Frage. Als Beispiele sind zu nennen: Halogenkohlenwasserstoffe (z.B. Chlorkohlenwasserstoffe, wie Tetraethylen, Tetrachlorethan, Dichlorpropan, Methylenchlorid, Dichlorbutan, Chloroform, Tetrachlorkohlenstoff, Trichlorethan, Trichlorethylen, Pentachlorethan, Difluorbenzol, 1,2-Dichlorethan, Chlorbenzol, Brombenzol, Dichlorbenzol, Chlortoluol, Trichlorbenzol), Alkohole (z.B. Methanol, Ethanol, Isopropanol, Butanol), Ether (z.B. Ethylpropylether, Methyl-tert-butylether, n-Butylether, Anisol, Phenetol, Cyclohexylmethylether, Dimethylether, Diethylether, Dipropylether, Diisopropylether, Di-n-butylether, Diisobutylether, Diisoamylether, Ethylenglycoldimethylether, Tetrahydrofuran, Dioxan, Dichlordiethylether und Polyether des Ethylenoxids und/oder Propylenoxids), Amine (z.B. Trimethyl-, Triethyl-, Tripropyl-, Tributylamin, N-Methylmorpholin, Pyridin und Tetramethylendiamin), Nitrokohlenwasserstoffe (z.B. Nitromethan, Nitroethan, Nitropropan, Nitrobenzol, Chlornitrobenzol, o-Nitrotoluol; Nitrile wie Acetonitril, Propionitril, Butyronitril, Isobutyronitril, Benzonitril, m-Chlorbenzonitril, Tetrahydrothiophendioxid, Dimethylsulfoxid, Tetramethylensulfoxid, Dipropylsulfoxid, Benzylmethylsulfoxid, Diisobutylsulfoxid, Dibutylsulfoxid, Diisoamylsulfoxid, Sulfone (z.B. Dimethyl-, Diethyl-, Dipropyl-, Dibutyl-, Diphenyl-, Dihexyl-, Methylethyl-, Ethylpropyl-, Ethylisobutyl- und Pentamethylensulfon), aliphatische, cycloaliphatische oder aromatische Kohlenwasserstoffe (z.B. Pentan, Hexan, Heptan, Oktan, Nonan und technische Kohlenwasserstoffe), ferner sogenannte "White Spirits" mit Komponenten mit Siedepunkten im Bereich von beispielsweise 40 °C bis 250 °C, Cymol, Benzinfraktionen innerhalb eines Siedeinterwalles von 70°C bis 190 °C, Cyclohexan, Methylcyclohexan, Petrolether, Ligroin, Octan, Benzol, Toluol, Chlorbenzol, Brombenzol, Nitrobenzol, Xylol, Ester (z.B. Methyl-, Ethyl-, Butyl-, Isobutylacetat, Dimethyl-, Dibutyl-, Ethylencarbonat); Amide (z.B. Hexamethylenphosphorsäuretriamid, Formamid, N-Methyl-formamid, N,N-Dimethyl-formamid, N,N-Dipropyl-formamid, N,N-Dibutyl-formamid, N-Methyl-pyrrolidin, N-Methyl-caprolactam, 1,3-Dimethyl-3,4,5,6-tetrahydro-2(1H)-pyrimidin, Octylpyrrolidon, Octylcaprolactam, 1,3-Dimethyl-2-imidazolindion, N-Formyl-piperidin, N,N'-1,4-Diformyl-piperazin) und Ketone (z.B. Aceton, Acetophenon, Methylethylketon, Methylbutylketon).

Als bevorzugtes Verdünnungsmittel kann jedes Lösungsmittel verwendet werden, das die Reaktion nicht beeinträchtigt, wie zum Beispiel Wasser. In Frage kommen aromatische Kohlenwasserstoffe wie Benzol, Toluol, Xylol oder Chlorbenzol; halogenierte Kohlenwasserstoffe wie Dichlormethan, Chloroform, 1,2-Dichlorethan oder Tetrachlorkohlenwasserstoff, offenkettige oder zyklische Ether wie Diethylether, Dioxan, Tetrahydrofuran oder 1 ,2-Dimethoxyethan; Ester wie Ethylacetat und Butylacetat; Ketone wie zum Beispiel Aceton, Methyl-isobutylketon und Cyclohexanon; Amide wie Dimethylformamid und Dimethylacetamid, N-Methyl-pyrrolidinon; Nitrile wie Acetonitril oder Propionitril; und andere inerte Lösungsmittel wie 1,3-Dimethyl-2- imidazolidinon; die Lösungsmittel können allein oder in Kombination von 2 oder mehr eingesetzt werden.

Die Reaktion kann in einem weiten Temperaturbereich ausgeführt werden. Gewöhnlich wird sie in einem Temperaturbereich von -78 bis 200 °C, bevorzugt bei Temperaturen zwischen -10 und 150 °C durchgeführt. Die Reaktion kann unter erhöhtem als auch vermindertem Druck ausgeführt werden. Bevorzugt wird sie aber unter Normaldruck durchgeführt. Die Reaktionszeiten liegen zwischen 0,1 und 72 Stunden, bevorzugt zwischen 1 und 24 Stunden.

Zur Durchführung der Reaktion werden 1 bis 3 Mol, bevorzugt 1 bis 1,5 Mol an Halogenierungsmittel pro Mol der Verbindung der Struktur **(X-2)** in einem Lösungsmittel wie z.B. Dimethylformamid (DMF) umgesetzt

### Stufe 3 Ringschluss

Stufe 3 des Darstellungsverfahrens der erfindungsgemäßen Verbindungen (1-T23):

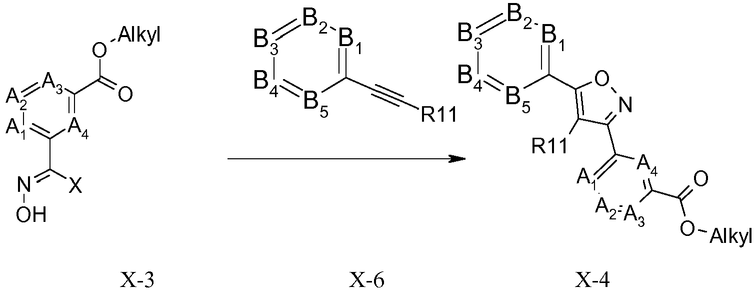

Erfindungsgemäße Verbindungen der allgemeinen Struktur **(X-4)** werden hergestellt indem man die Hydroxamsäurechloride der Struktur **(X-3)** mit Acetylenen der Struktur **(X-6)** umsetzt.

Die Reste A¹-A⁴, B¹-B⁵, R¹¹ und Alkyl haben die oben beschriebenen Bedeutungen.

Typische Verbindungen der Struktur **(X-3)** sind z.B. Carbomethoxy-4-chlor-N-hydroxybenzimidoylchlorid, 3-Carbomethoxy-4-fluor-N-hydroxy-benzimidoylchlorid, 3-Carbomethoxy-4-chlor-5-fluor-N-hydroxy-benzimidoylchlorid, 3-Carbomethoxy-4-brom-N-hydroxy-benzimidoylchlorid.

Als Verdünnungs- bzw. Lösungsmittel zur Durchführung der erfindungsgemäßen Verfahren kommen grundsätzlich alle unter den spezifischen Reaktionsbedingungen inerten organischen Lösungsmittel in Frage. Als Beispiele sind zu nennen: Halogenkohlenwasserstoffe (z.B. Chlorkohlenwasserstoffe, wie Tetraethylen, Tetrachlorethan, Dichlorpropan, Methylenchlorid, Dichlorbutan, Chloroform, Tetrachlorkohlenstoff, Trichlorethan, Trichlorethylen, Pentachlorethan, Difluorbenzol, 1,2-Dichlorethan, Chlorbenzol, Brombenzol, Dichlorbenzol, Chlortoluol, Trichlorbenzol), Alkohole (z.B. Methanol, Ethanol, Isopropanol, Butanol), Ether (z.B. Ethylpropylether, Methyl-tert-butylether, n-Butylether, Anisol, Phenetol, Cyclohexylmethylether, Dimethylether, Diethylether, Dipropylether, Diisopropylether, Di-n-butylether, Diisobutylether, Diisoamylether, Ethylenglycoldimethylether, Tetrahydrofuran, Dioxan, Dichlordiethylether und Polyether des Ethylenoxids und/oder Propylenoxids), Amine (z.B. Trimethyl-, Triethyl-, Tripropyl-, Tributylamin, N-Methylmorpholin, Pyridin und Tetramethylendiamin), Nitrokohlenwasserstoffe (z.B. Nitromethan, Nitroethan, Nitropropan, Nitrobenzol, Chlornitrobenzol, o-Nitrotoluol; Nitrile wie Acetonitril, Propionitril, Butyronitril, Isobutyronitril, Benzonitril, m-Chlorbenzonitril, Tetrahydrothiophendioxid, Dimethylsulfoxid, Tetramethylensulfoxid, Dipropylsulfoxid, Benzylmethylsulfoxid, Diisobutylsulfoxid, Dibutylsulfoxid, Diisoamylsulfoxid, Sulfone (z.B. Dimethyl-, Diethyl-, Dipropyl-, Dibutyl-, Diphenyl-, Dihexyl-, Methylethyl-, Ethylpropyl-, Ethylisobutyl- und Pentamethylensulfon), aliphatische, cycloaliphatische oder aromatische Kohlenwasserstoffe (z.B. Pentan, Hexan, Heptan, Oktan, Nonan und technische Kohlenwasserstoffe), ferner sogenannte "White Spirits" mit Komponenten mit Siedepunkten im Bereich von beispielsweise 40 °C bis 250 °C, Cymol, Benzinfraktionen innerhalb eines Siedeinterwalles von 70 °C bis 190 °C, Cyclohexan, Methylcyclohexan, Petrolether, Ligroin, Octan, Benzol, Toluol, Chlorbenzol, Brombenzol, Nitrobenzol, Xylol, Ester (z.B. Methyl-, Ethyl-, Butyl-, Isobutylacetat, Dimethyl-, Dibutyl-, Ethylencarbonat); Amide (z.B. Hexamethylenphosphorsäuretriamid, Formamid, N-Methyl-formamid, N,N-Dimethyl-formamid, N,N-Dipropyl-formamid, N,N-Dibutyl-formamid, N-Methyl-pyrrolidin, N-Methyl-caprolactam, 1,3-Dimethyl-3,4,5,6-tetrahydro-2(1H)-pyrimidin, Octylpyrrolidon, Octylcaprolactam, 1,3-Dimethyl-2-imidazolindion, N-Formyl-piperidin, N,N'-1,4-Diformyl-piperazin) und Ketone (z.B. Aceton, Acetophenon, Methylethylketon, Methylbutylketon).

Als bevorzugtes Verdünnungsmittel kann jedes Lösungsmittel verwendet werden, das die Reaktion nicht beeinträchtigt, wie zum Beispiel Wasser. In Frage kommen aromatische Kohlenwasserstoffe wie Benzol, Toluol, Xylol oder Chlorbenzol; halogenierte Kohlenwasserstoffe wie Dichlormethan, Chloroform, 1,2-Dichlorethan oder Tetrachlorkohlenwasserstoff, offenkettige oder zyklische Ether wie Diethylether, Dioxan, Tetrahydrofuran oder 1,2-Dimethoxyethan; Ester wie Ethylacetat und Butylacetat; Ketone wie zum Beispiel Aceton, Methyl-isobutylketon und Cyclohexanon; Amide wie Dimethylformamid und Dimethylacetamid, N-Methyl-pyrrolidinon; Nitrile wie Acetonitril oder Propionitril; und andere inerte Lösungsmittel wie 1,3-Dimethyl-2- imidazolidinon; die Lösungsmittel können allein oder in Kombination von 2 oder mehr eingesetzt werden.

Bei den Umsetzungen der Verbindungen der Struktur **(X-3)** mit den Acetylenen der Struktur (X-6) können Basen zugesetzt werden. Als Beispiele sind zu nennen Erdalkali- oder Alkalimetallverbindungen (z.B. Hydroxide, Hydride, Oxide und Carbonate des Lithiums, Natriums, Kaliums, Magnesiums, Calciums und Bariums), Amidinbasen oder Guanidinbasen (z.B. 7-Methyl-1,5,7-triaza-bicyclo(4.4.0)dec-5-en (MTBD); Diazabicyclo(4.3.0)nonen (DBN), Diazabicyclo(2.2.2)octan (DABCO), 1,8-Diazabicyclo(5.4.0)undecen (DBU), Cyclohexyltetra-butyl-guanidin (CyTBG), Cyclohexyltetramethylguanidin (CyTMG), N,N,N,N-Tetramethyl-1,8-naphthalindiamin, Pentamethylpiperidin) und Amine, insbesondere tertiäre Amine, (z.B. Triethylamin, Trimethylamin, Tribenzylamin, Triisopropylamin, Tributylamin, Tricyclohexylamin, Triamylamin, Trihexylamin, N,N-Dimethylanilin, N,N-Dimethyl-toluidin, N,N-Dimethyl-p-aminopyridin, N-Methyl-pyrrolidin, N-Methyl-piperidin, N-Methyl-imidazol, N-Methyl-pyrazol, N-Methyl-morpholin, N-Methylhexamethylendiamin, Pyridin, 4-Pyrrolidinopyridin, 4-Dimethyl-amino-pyridin, Chinolin, α-Picolin, β-Picolin, Isochinolin, Pyrimidin, Acridin, N,N,N',N'-Tetra-methylendiamin, N,N,N',N'-Tetraethylendiamin, Chinoxalin, N-Propyl-diisopropylamin, N-Ethyl-diisopropylamin, N,N'-Dimethylcyclohexylamin, 2,6-Lutidin, 2,4-Lutidin oder Triethyldiamin).

Als bevorzugtes basisches Reaktionshilfsmittel kann eine organische Base wie Triethylamin, Ethyl-diisopropylamin, Tri-n-butylamin, Pyridin und 4-Dimethylamino-pyridin verwendet werden; Des Weiteren können beispielsweise folgende Basen eingesetzt werden: Alkalimetallhydroxide wie z.B Natriumhydroxid und Kaliumhydroxid; Carbonate wie Natriumhydrogencarbonat und Kaliumcarbonat; Phosphate wie Dikalium-hydrogenphosphat und Tri-natriumphosphat.

Die Reaktion kann in einem weiten Temperaturbereich ausgeführt werden. Gewöhnlich wird sie in einem Temperaturbereich von -78 bis 200 °C, bevorzugt bei Temperaturen zwischen -10 und 150 °C durchgeführt. Die Reaktion kann unter erhöhtem als auch vermindertem Druck ausgeführt werden. Bevorzugt wird sie aber unter Normaldruck durchgeführt. Die Reaktionszeiten liegen zwischen 0,1 und 72 Stunden, bevorzugt zwischen 1 und 24 Stunden.

Zur Durchführung der Reaktion werden z.B. 1-2 Moläquivalente der Verbindungen der Struktur **(X-6)** und 1 Moläquivalent bis zu einem leichten Überschuss an Base pro Mol der Verbindung der Struktur **(X-3)** in einem Lösungsmittel wie z.B. Dimethylformamid (DMF) umgesetzt

Die Stufen 2 und 3 zur Herstellung der Verbindungen der Struktur **(X-4)** können in einzelnen Schritten oder auch als Eintopf-Reaktion durchgeführt werden.

### Stufen 4, 5 Verseifung, Amidierung

Die letzten Stufen (Stufen 4 und 5) zur Herstellung der erfindungsgemäßen Verbindungen (1-T23), Hydrolyse des Carbonsäureesters **(X-4)** und Amidierung der Carbonsäure X-5, können nach den weiter oben beschriebenen allgemeinen Verfahren (Reaktionsschema) der Ester Hydrolyse und Amidierung der Carbonsäure durchgeführt werden.

### Stufe 6 Herstellung der Acetylene

Stufe 6 Herstellung der Ausgangsverbindungen der Struktur **(X-6)**

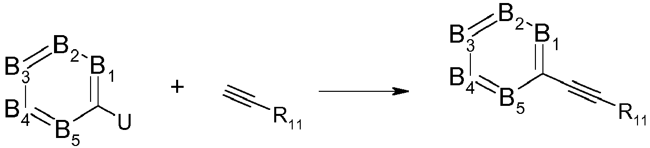

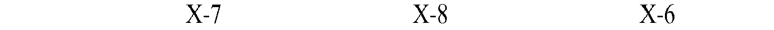

Die Reste B₁-B₅, R¹¹ und U haben die oben beschriebenen Bedeutungen. U steht z.B. für Brom, Iod oder Triflat.

Erfindungsgemäße Verbindungen der allgemeinen Struktur **(X-6)** können in Analogie zu literaturbekannten Methoden (Chinchilla, Rafael et al., Chemical Society Reviews (2011), 40(10), S. 5084-5121, Chinchilla, Rafael et al., Chemical Reviews (Washington, DC, United States) (2007), 107(3), S. 874-922) aus den Ausgangsmaterialien der Struktur **(X-7)** unter Katalyse mittels Übergangsmetall Katalysatoren mit Palladium und Kupfer hergestellt werden.

Ausgangsverbindungen der Struktur **(X-7)** sind bekannt oder können nach bekannten Methoden hergestellt werden. Als Beispiele seien 2-Brom-1,3-dichlor-5-[1,2,2,2-tetrafluor-1-(trifluormethyl)ethyl]benzol, 2-Brom-1,3-dimethyl-5-[1,2,2,2-tetrafluor-1-(trifluormethyl)ethyl]benzol, 2-Brom-1-ethyl-3-methyl-5-[1,2,2,2-tetrafluor-1-(trifluormethyl)ethyl]benzol, 2-Brom-1-chlor-5-[1,2,2,2-tetrafluor-1-(trifluormethyl)ethyl]-3-(trifluormethyl)benzol, 2-Brom-1-methyl-5-[1,2,2,2-tetrafluor-1-(trifluormethyl)ethyl]-3-(trifluormethyl)benzol, 2-Brom-1-chlor-5-[1,2,2,2-tetrafluor-1-(trifluormethyl)ethyl]-3-(trifluormethoxy)benzol, 2-Brom-1-methyl-5-[1,2,2,2-tetrafluor-1-(trifluormethyl)ethyl]-3-(trifluormethoxy)benzol genannt. Sie können z.B. nach den in EP 1 253 128, Seiten 8-10 beschriebenen Methoden hergestellt werden.

Ausgangsverbindungen der Struktur **(X-8)** sind bekannt oder können nach bekannten Methoden hergestellt werden. Für den Fall R⁶=H kann in diesem Verfahren eine Schutzgruppe statt R⁶ verwendet werden. Geeignete Schutzgruppen sind zum Beispiel Trimethylsilyl, Triethylsilyl und Dimethylhydroxymethyl. Weitere geeignete Schutzgruppen und Methoden zum Einbringen und Abspalten sind in der Literatur beschrieben [siehe Auflistungen in Greene's protective groups in organic synthesis, 4. Edition, P. G. M. Wuts, T. W. Greene, John Wiley & Sons, Inc., Hoboken, New Jersey, 2007, Seite 927-933.]

### Stufe 3 Alternativ: Kupplung mit Amiden

Alternativ können die erfindungsgemäßen Verbindungen **(I-T23)** durch das allgemeine Darstellungsverfahren B (Reaktionsschema 9) dargestellt werden.

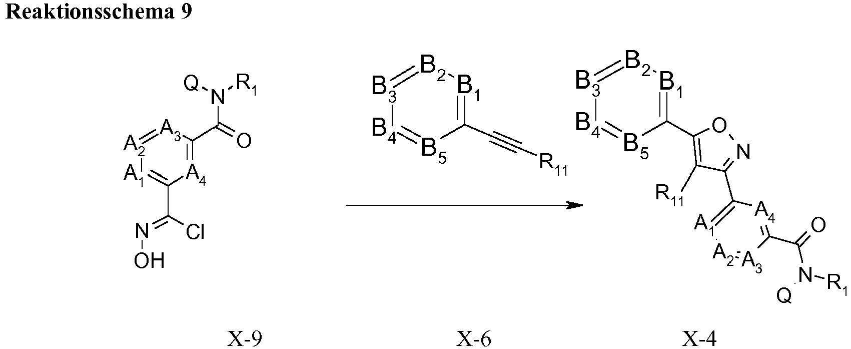

Die Reste A₁-A₄, B₁-B₅, Q, R¹ und R¹¹ haben die oben beschriebenen Bedeutungen.

Erfindungsgemäße Verbindungen der allgemeinen Struktur **(X-4)** werden hergestellt indem man die Hydroxamsäurechloride der Struktur **(X-9)** mit Acetylenen der Struktur **(X-6)** umsetzt.

Die Herstellung der Verbindungen der Struktur **(X-6)** ist weiter oben beschrieben. Die Herstellung der Verbindungen der Struktur **(X-9)** erfolgt analog der oben beschriebenen Herstellung der Verbindungen der Struktur **(X-3).**

Typische Verbindungen der Struktur **(X-3)** sind z.B. 4-Chlor-3-(cyclopropylcarbamoyl)-N-hydroxy-benzimidoyl chlorid, 3-(Cyclopropylcarbamoyl)-4-fluor-N-hydroxy-benzimidoyl chlorid, 4-Chlor-3-(cyclopropylcarbamoyl)- 5-fluor-N-hydroxy-benzimidoyl chlorid, 4-Brom-3-(cyclopropylcarbamoyl)-N-hydroxy-benzimidoyl chlorid.
(I-T24): Verbindungen der Formel (I-24) können z. B. analog zu Furukawa, Hirotoshi et al. Heterocycles, 79 (2009), S. 303-309; US 6,545,009, S. 34, Example 111 hergestellt werden.
(I-T25): Verbindungen der Formel (I-25) können z. B. analog zu WO 2004/14366, S. 113 hergestellt werden.
(I-T26): Verbindungen der Formel (I-26) können z. B. analog zu Chihiro, Masatoshi et al., Journal of Medicinal Chemistry, 38 (1995), S. 353-358 hergestellt werden.
(I-T27): Verbindungen der Formel (I-27) können z. B. analog zu US 6,545,009, S. 31, Beispiel 74 hergestellt werden.

Die Verbindungen der Struktur **(I-T28)** können nach dem im Reaktionsschema 10 angegebenen Verfahren hergestellt werden.

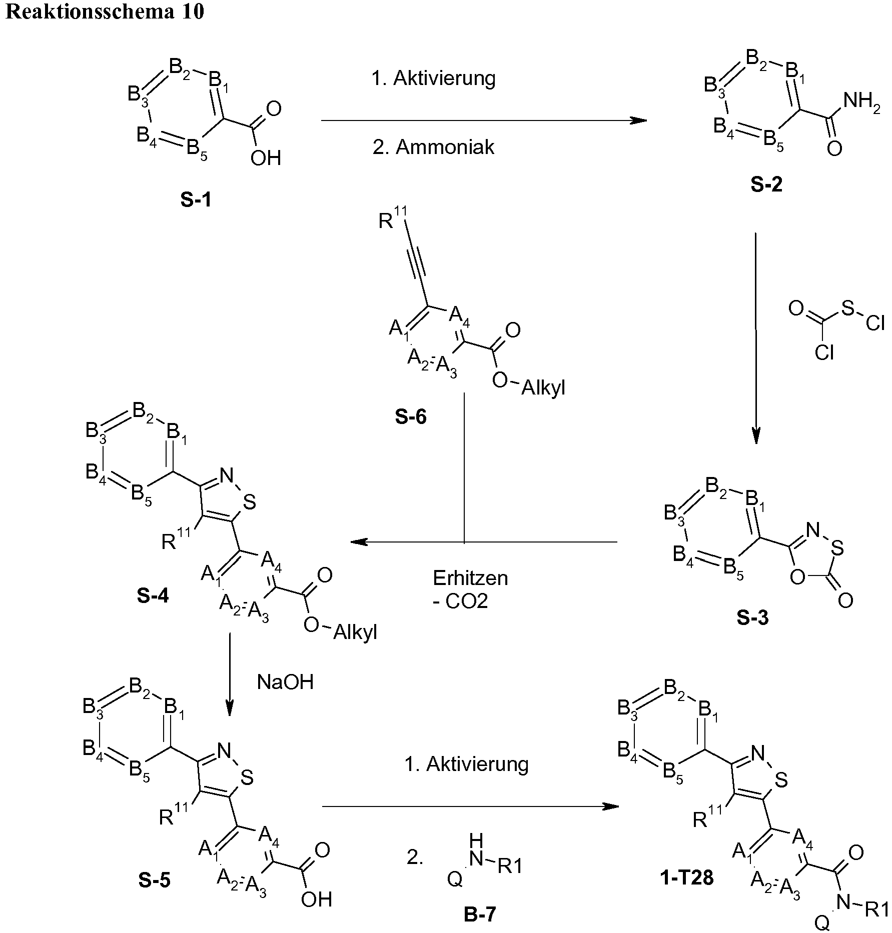

Die Reste A¹-A⁴, B¹-B⁵, Alkyl, Q, R¹ und R¹¹ haben die oben beschriebenen Bedeutungen. Ausgangsverbindungen der Struktur **(S-1)** sind bekannt (z.B. US 5,739,083 S. 10, oder WO 2012/175474, S. 117-118) oder können nach bekannten Methoden hergestellt werden. Die Reaktionen werden unter den in der Literatur angegebenen Bedingungen durchgeführt (z.B. Abdelrahman S. Mayhoub et al., Bioorg. Med. Chem. 20 (2012) S. 2427-2434 oder WO 2009/023372).

### Verfahren I-T29

Die Verbindungen der Struktur **(I-T29)** können nach dem im Reaktionsschema 11 angegebenen Verfahren hergestellt werden.

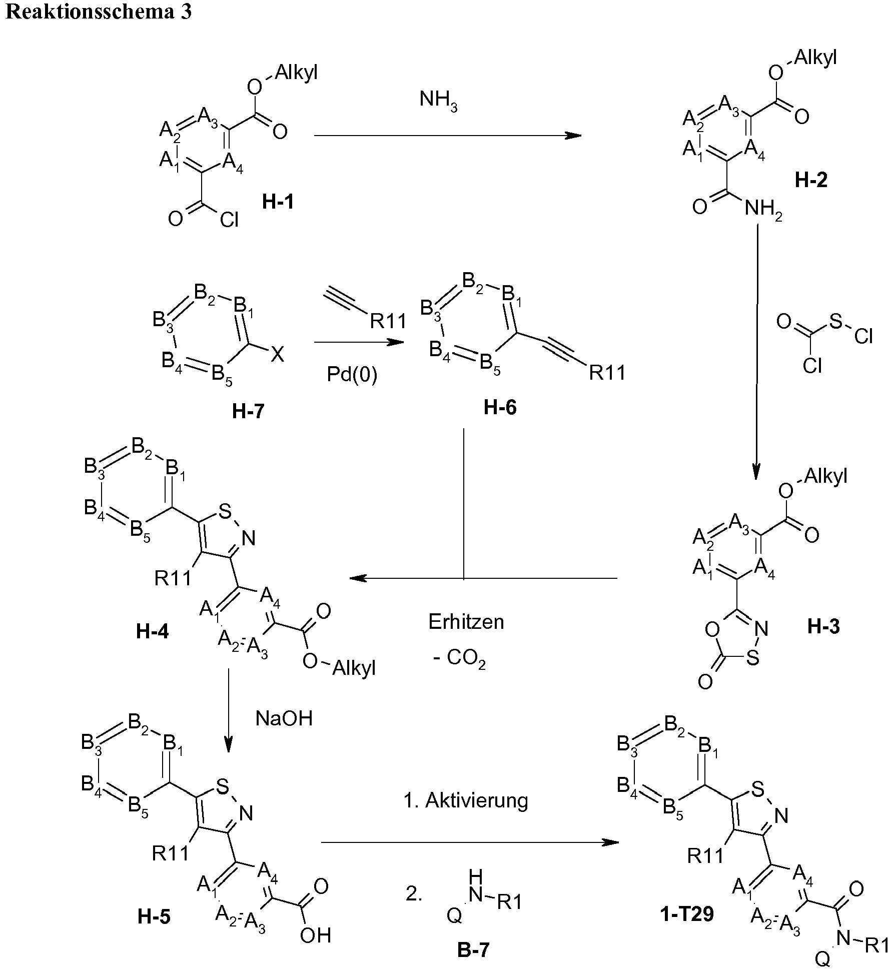

Die Reste A¹-A⁴, B¹-B⁵, Alkyl, Q, R¹ und R¹¹ haben die oben beschriebenen Bedeutungen. Ausgangsverbindungen der Struktur **(H-1)** und **(H-7)** sind bekannt (z.B. US 3,725,417 S. 7 oder WO 2012/175474, S. 117-118) oder können nach bekannten Methoden hergestellt werden. Die Reaktionen werden unter den in der Literatur angegebenen Bedingungen durchgeführt (z.B. Abdelrahman S. Mayhoub, et al., Bioorg. Med. Chem. 20 (2012) S. 2427-2434 oder WO 2009/023372).

### Verfahren I-T30

(I-T30): Verbindungen der Formel (I-T30) können z. B. analog zu WO 2011/9484, S. 104; oder Gamber, Gabriel G. et al., Bioorganic and Medicinal Chemistry Letters, 21 (2011), S. 1447-1451 hergestellt werden.
(I-T31): Verbindungen der Formel (I-T31) können z. B. analog zu Bishop, Brian C. et al., Synthesis, (2004), S. 43-52; oder Heller, Stephen T. et al., Organic Letters, 8 (2006), S. 2675-2678; oder Baddar, F.G. et al. Journal of Heterocyclic Chemistry, 15 (1978), S. 385-393 hergestellt werden.
(I-T32): Verbindungen der Formel (I-T32) können z. B. analog zu Joo, Jung Min et al., Journal of Organic Chemistry, 75 (2010), S. 4911-4920 hergestellt werden.
(I-T33): Verbindungen der Formel (I-T33) können z. B. analog zu Joo, Jung Min et al., Journal of Organic Chemistry, 75 (2010), S. 4911-4920; oder WO 2004/91610, S. 70 hergestellt werden.
(I-T34): Verbindungen der Formel (I-T34) können z. B. analog zu Al-Tel, Taleb et al., Journal of Medicinal Chemistry, 54 (2011), S. 8373-8385 hergestellt werden.
(I-T35): Verbindungen der Formel (I-T35) können z. B. analog zu Yang, Shu-wie et al., Bioorganic and Medicinal Chemistry Letters, 21 (2011), S. 182-185; oder Kennedy, Andrew J. et al, Journal of Medicinal Chemistry, 54 (2011), S. 3524-3548 hergestellt werden.

Die Verbindungen der Struktur (I-T45) können nach dem im Reaktionsschema 12 angegebenen Verfahren hergestellt werden.

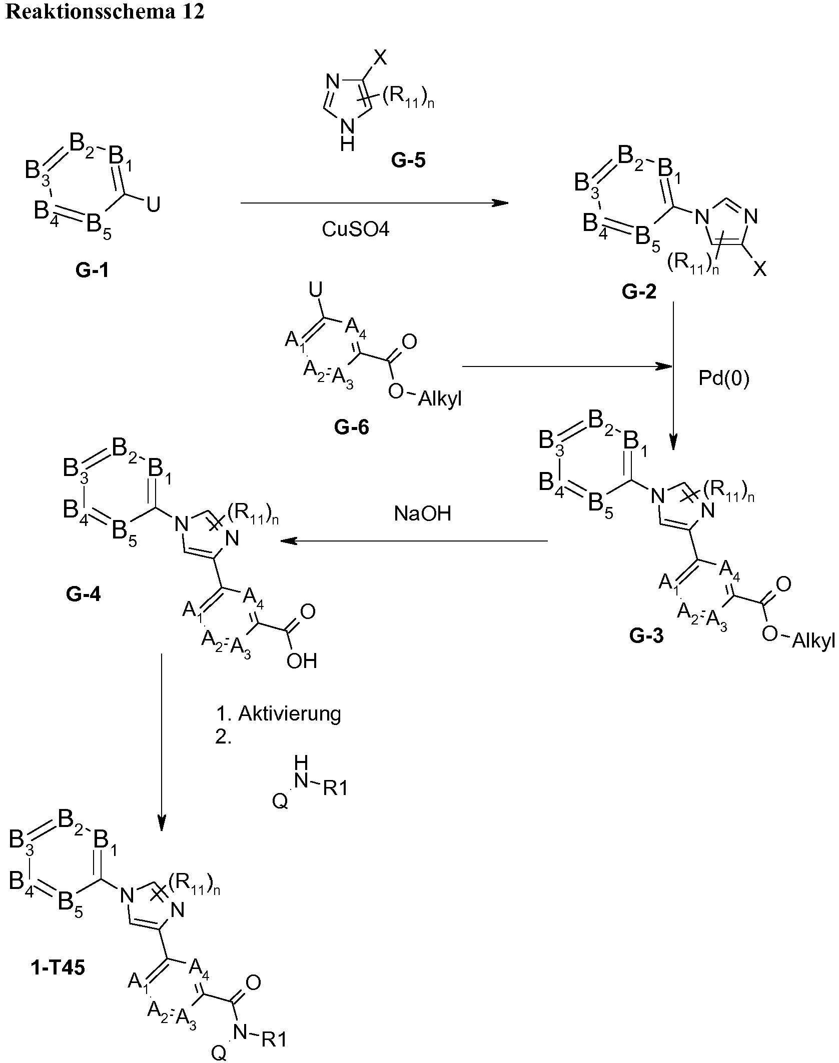

Die Reste A₁-A₄, B₁-B₅, Alkyl, Q, R¹ und R¹¹ haben die oben beschriebenen Bedeutungen. U steht für eine Boronsäure, Boronsäureester oder Trifluorboronat. X steht für Brom, Iod oder Triflat. Ausgangsverbindungen der Struktur (**G-1**), (**G-5**) und (**G6**) sind bekannt oder können nach bekannten Methoden hergestellt werden.

Die Durchführung der Reaktionen kann nach den in der Literatur beschriebenen Verfahren erfolgen (siehe z.B. Stufe G1->G2 US 2013/0012532, S. 29).

### Verfahren I-T46

Die Verbindungen der Struktur (I-T46) können nach dem im Reaktionsschema 13 angegebenen Verfahren hergestellt werden.

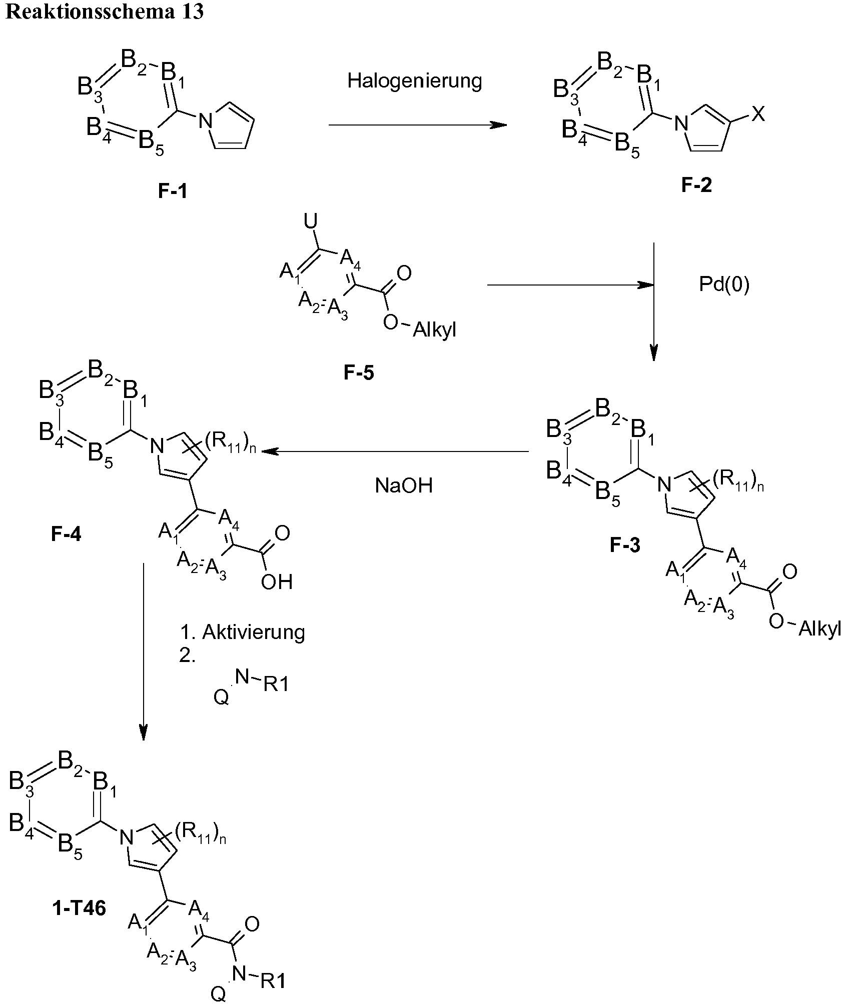

Die Reste A₁-A₄, B₁-B₅, Alkyl, Q, R¹ und R¹¹ haben die oben beschriebenen Bedeutungen. U steht für eine Boronsäure, Boronsäureester oder Trifluorboronat. X steht für Brom, Iod oder Triflat. Ausgangsverbindungen der Struktur (**F-1**) und (**F-5**) sind bekannt (z.B. F-1: Hulcoop, David G. et al., Organic Letters, 9 (2007), S. 1761-1764), oder können nach bekannten Methoden hergestellt werden.

Die Durchführung der Reaktionen kann nach den in der Literatur beschriebenen Verfahren erfolgen z.B. US2009/209476, S. 18-19.

### Verfahren I-T47

Die Verbindungen der Struktur (**I-T47**) können nach dem im Reaktionsschema 14 angegebenen Verfahren hergestellt werden.

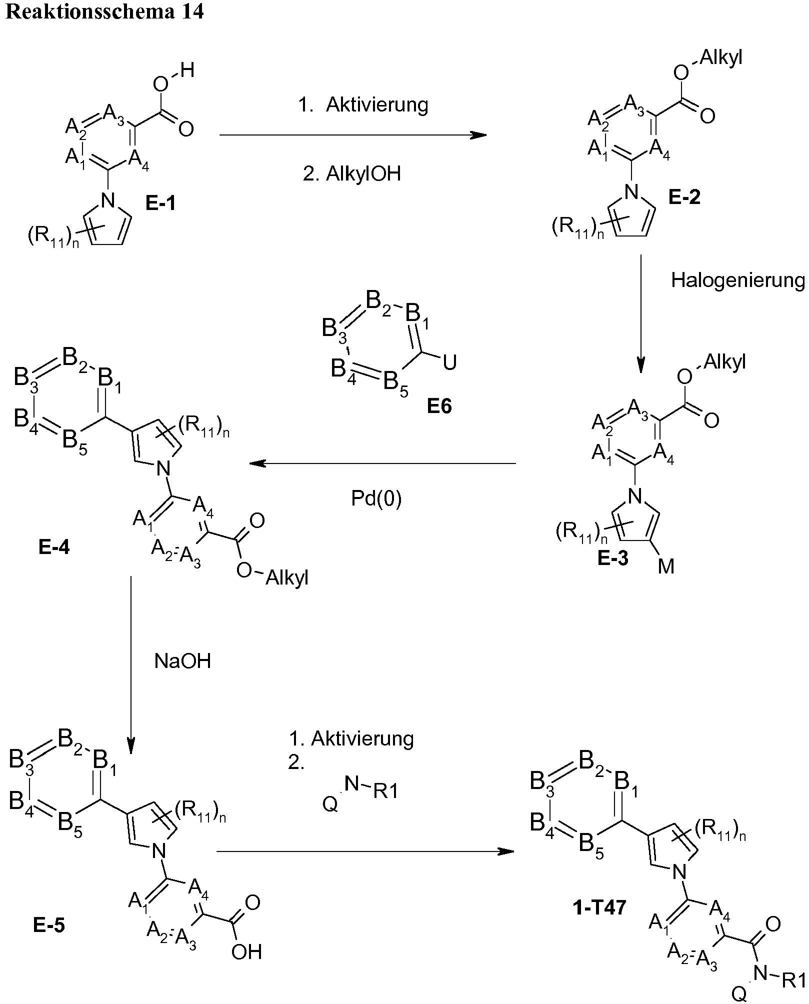

Die Reste A₁-A₄, B₁-B₅, Alkyl, Q, R¹ und R¹¹ haben die oben beschriebenen Bedeutungen. U steht für Brom, Iod oder Triflat, wenn M für eine Boronsäure, Boronsäureester oder Trifluorboronat steht. U steht für eine Boronsäure, Boronsäureester oder Trifluorboronat, wenn M für Brom, Iod oder Triflat steht. Ausgangsverbindungen der Struktur (**E-1**) und (**E-6**) sind bekannt (z.B. Liu, Kun et al., Journal of Medicinal Chemistry, 51 (2008), S. 7843-7854; oder Cornet, Stephanie M. et al., Transactions, (2003), S. 4395-4405), oder können nach bekannten Methoden hergestellt werden.

Die Durchführung der Reaktionen kann nach den in der Literatur beschriebenen Verfahren erfolgen z.B. US 2009/209476, S. 18-19.

### Verfahren zur Herstellung von Thioamiden

Die Verbindungen der Struktur (**Ii**) können nach dem im Reaktionsschema 15 angegebenen Verfahren aus Verbindungen der Struktur (**Ih**) durch Umsetzung mit Schwefel übertragenden Reagenzien hergestellt werden.

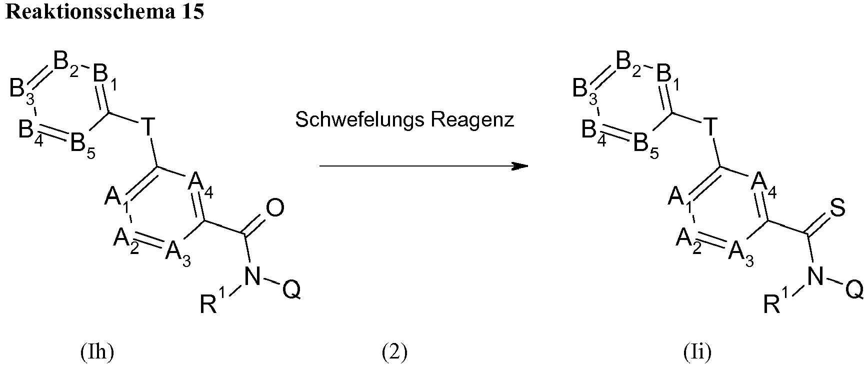

Die Reste A₁-A₄, B₁-B₅, Alkyl, Q, T, und R¹ haben die oben beschriebenen Bedeutungen. Als Schwefelungs Reagenz (**2**) kann z. B. P₄S₁₀ oder Lawessons Reagenz (2,4-Bis(4-methoxyphenyl)-1,3,2,4-dithiadiphosphetan-2,4-disulfid) verwendet werden.

Die Herstellung der Verbindungen (**Ih**) ist weiter oben beschrieben.

Die Schwefelungs Reagenzien sind kommerziell erhältlich oder können nach dem Fachmann bekannten Verfahren bzw. in Analogie zu diesen Verfahren hergestellt werden.

Die Durchführung der Reaktion erfolgt in Analogie zu literaturbekannten Methoden zur Schwefelung von Carbonamiden (z.B. WO2012056372, S. 77; WO2003066050, S. 31).

### Verfahren zur Darstellung von (Ik)

Die erfindungsgemäßen Verbindungen (**Ik**) können nach dem im Reaktionsschema 16 angegebenen Verfahren aus den Verbindungen (**Ij**) durch Umsetzung mit Schwefelverbindungen der Struktur (**Y-3**) hergestellt werden.

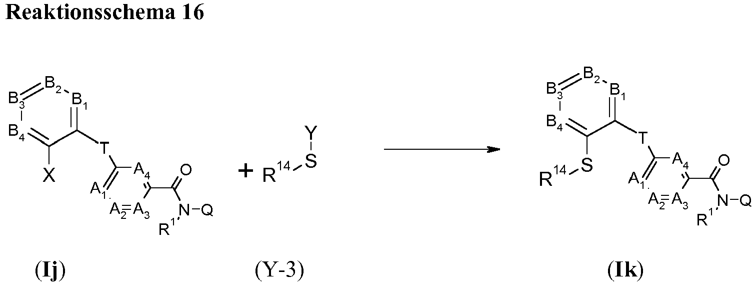

Die Reste A₁ bis A₄, B₁ bis B4, Alkyl, Q, R¹, n und R¹¹ haben die oben beschriebenen Bedeutungen. X steht für eine geeignete Abgangsgruppe wie z.B. Fluor, Chlor, Brom oder Iod. R¹⁴ steht für gegebenenfalls substituiertes C₁-C₆-Alkyl. Y steht für Wasserstoff oder ein Alkalimetall wie z.B. Natrium oder Lithium.

Die Durchführung der Reaktion erfolgt in Analogie zu literaturbekannten Methoden zur Einführung von Alkylthioresten in Aromaten [z.B. Organometallics 1989, 8(5), 1303-1308; WO1998056761, Example 63, S. 97].

### Verfahren zur Darstellung von (Ika) und (Ikb)

Die erfindungsgemäßen Verbindungen (**Ika**) bzw. (**Ikb**) können nach dem im Reaktionsschema 17 angegebenen Verfahren aus den Verbindungen der Struktur (Ik) durch Umsetzung mit oxidierenden Reagenzien hergestellt werden.

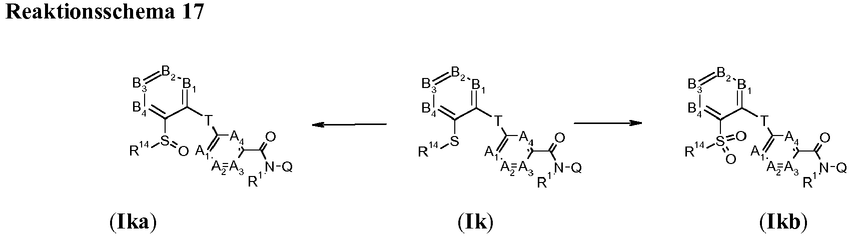

Die Reste A₁ bis A₄, B₁ bis B4, Alkyl, Q, R¹, n und R¹¹ haben die oben beschriebenen Bedeutungen. R¹⁴ steht für gegebenenfalls substituiertes C₁-C₆-Alkyl.

Die Herstellung der Verbindungen der Struktur (**Ik**) ist weiter oben beschrieben.

Als Oxidationsmittel können die dem Fachmann aus der Literatur bekannten Reagenzien zur Herstellung von Sulfoxiden und Sulfonen verwendet werden. Sie sind kommerziell erhältlich oder können nach dem Fachmann bekannten Verfahren bzw. in Analogie zu diesen Verfahren hergestellt werden. Als Beispiele seien genannt: Wasserstoffperoxid, Peroxiessigsäure, 3-Chlorperbenzoesäure und Trifluorperoxiessigsäure.

Die Durchführung der Reaktion erfolgt in Analogie zu literaturbekannten Methoden zur Herstellung von Sulfoxiden und Sulfonen [Sulfoxidderivate: WO2006/097766; WO2005/019151; Sulfonderivate: WO2008/125214; WO2005/121087].

### Verfahren zur Herstellung von N-Alkyl Verbindungen

Die Verbindungen der Struktur (**I**) können nach dem im Reaktionsschema 18 angegebenen Verfahren aus Verbindungen der Struktur (**Im**) durch Umsetzung mit Alkylierungmitteln hergestellt werden.

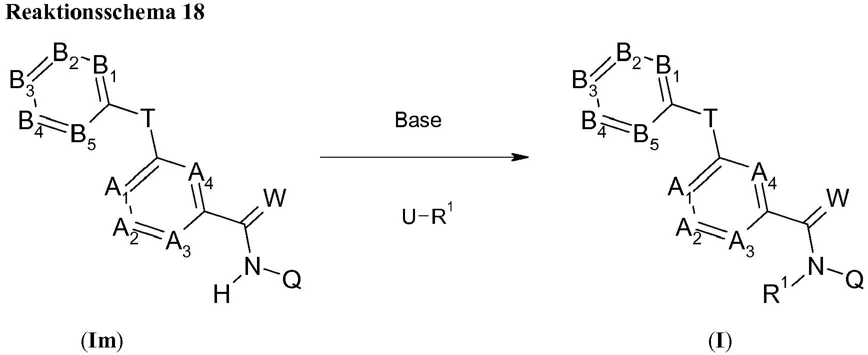

Die Reste A₁-A₄, B₁-B₅, Alkyl, , und Q haben die oben beschriebenen Bedeutungen. U steht z. B. für Brom, Iod oder Triflat. R¹ steht für jeweils primäres oder sekundäres, gegebenenfalls substituiertes C₁-C₆-Alkyl, C₃-C₆-Alkenyl, C₃-C₆-Alkinyl, C₄-C₇-Cycloalkyl, C₁-C₆-Alkylcarbonyl, C₁-C₆-Alkoxycarbonyl, Aryl(C₁-C₃)-alkyl, Heteroaryl(C₁-C₃)-alkyl. W steht für Sauerstoff.

Verbindungen der Struktur U-R¹ sind kommerziell erhältlich oder literaturbekannt oder können in Analogie zu literaturbekannten Methoden hergestellt werden. Als Beispiele sind zu nennen: Methylchlorid, Methylbromid, Methyliodid, Dimethylsulfat, Methyltriflat, Ethylbromid, Ethyliodid, Diethylsulfat, und Ethyltriflat.

Die für die Reaktion verwendeten Basen sind kommerziell erhältlich. Als Beispiele sind zu nennen Erdalkali- oder Alkalimetallverbindungen (z.B. Hydroxide, Hydride, Oxide und Carbonate des Lithiums, Natriums, Kaliums, Magnesiums, Calciums und Bariums) wie z.B. Natriumhydrid, Natriumhydroxid und Kaliumhydroxid; Carbonate wie Natriumhydrogencarbonat und Kaliumcarbonat

Die Durchführung der Reaktion erfolgt in Analogie zu literaturbekannten Methoden zur *N-*Alkylierung sekundärer Amide (z. B. G.L. Gisele, A. Lüttringhaus, Synthesis (1971) S.266, Übersicht siehe: B.C. Challis, J.A. Challis in: The Chemistry of Functional Groups, The Chemistry of Amides, S. Patai, J. Zabicky, editors, Interscience Publishers, London, 1970, S. 734 ff).

### Herstellung des Ausgangsprodukts 4-Heptafluorisopropyl-2-methyl-6-trifluormethyl-anilin

Das Ausgangsprodukt 4-Heptafluorisopropyl-2-methyl-6-trifluormethyl-anilin der Struktur (**D-1a**) ist bisher in der Literatur noch nicht beschrieben. Die Herstellung kann nach 2 verschiedenen Verfahren durchgeführt werden.

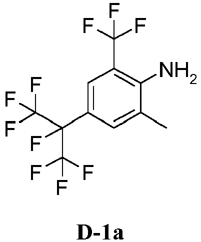

### Verfahren zur Herstellung von Thioamiden

Die Verbindungen der Struktur (**Ii**) können nach dem im Reaktionsschema 15 angegebenen Verfahren aus Verbindungen der Struktur (**Ih**) durch Umsetzung mit Schwefel übertragenden Reagenzien hergestellt werden.

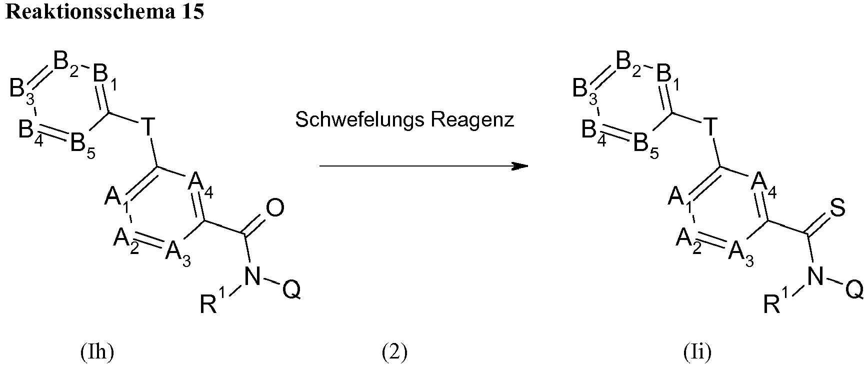

Die Reste A₁-A₄, B₁-B₅, Alkyl, Q, T, und R¹ haben die oben beschriebenen Bedeutungen. Als Schwefelungs Reagenz (**2**) kann z. B. P₄S₁₀ oder Lawessons Reagenz (2,4-Bis(4-methoxyphenyl)-1,3,2,4-dithiadiphosphetan-2,4-disulfid) verwendet werden.

Die Herstellung der Verbindungen (**Ih**) ist weiter oben beschrieben.

Die Schwefelungs Reagenzien sind kommerziell erhältlich oder können nach dem Fachmann bekannten Verfahren bzw. in Analogie zu diesen Verfahren hergestellt werden.

Die Durchführung der Reaktion erfolgt in Analogie zu literaturbekannten Methoden zur Schwefelung von Carbonamiden *(z.B.* WO2012056372, S. 77; WO2003066050, S. 31).

### Verfahren zur Darstellung von (Ik)

Die erfindungsgemäßen Verbindungen (**Ik**) können nach dem im Reaktionsschema 16 angegebenen Verfahren aus den Verbindungen (**Ij**) durch Umsetzung mit Schwefelverbindungen der Struktur (**Y-3**) hergestellt werden.

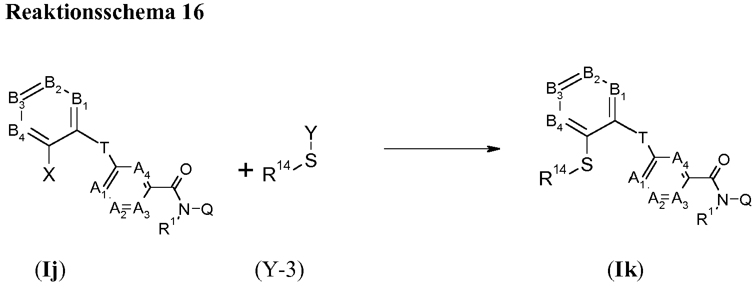

Die Reste A₁ bis A₄, B₁ bis B4, Alkyl, Q, R¹, n und R¹¹ haben die oben beschriebenen Bedeutungen. X steht für eine geeignete Abgangsgruppe wie z.B. Fluor, Chlor, Brom oder Iod. R¹⁴ steht für gegebenenfalls substituiertes C₁-C₆-Alkyl. Y steht für Wasserstoff oder ein Alkalimetall wie z.B. Natrium oder Lithium.

Die Durchführung der Reaktion erfolgt in Analogie zu literaturbekannten Methoden zur Einführung von Alkylthioresten in Aromaten. [z.B. Organometallics 1989, 8(5), 1303-1308; WO1998056761, Example 63, S. 97].

### Verfahren 1:

4-Heptafluorisopropyl-2-methyl-6-trifluormethyl-anilin der Struktur (**K-1**) kann ausgehend von 2-Methyl-6-trifluormethyl-anilin nach dem in Reaktionsschema 1 angegebenen Verfahren durch Umsetzung mit Heptafluorisopropyliodid in Gegenwart von Wasserstoffperoxid hergestellt werden.

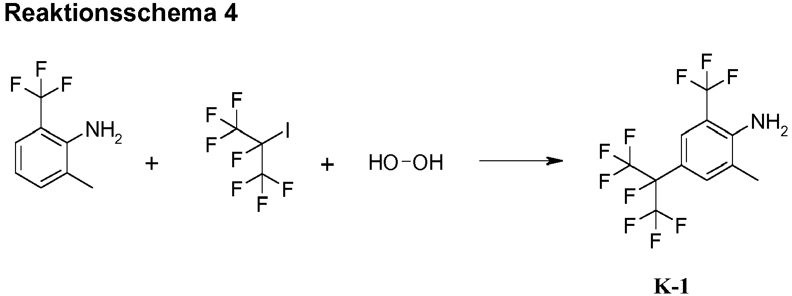

2-Methyl-6-trifluormethyl-anilin ist literaturbekannt (John P. Chupp, Terry M. Balthazor, Michael J. Miller, and Mark J. Pozzo, J. Org. Chem. 49 (1984),4711-4716 oder Thomas E. Nickson J. Org. Chem. 51 (1986) 3903-3904) und Heptafluorisopropyliodid ist kommerziell erhältlich.

Die Reaktion wird in Analogie zu bekannten Verfahren zur Trifluormethylierung von Aromaten durchgeführt (Tatsuhito Kino, Yu Nagase, Yuhki Ohtsuka, Kyoko Yamamoto, Daisuke Uraguchi,Kenji Tokuhisa and Tetsu Yamakawa, Journal of Fluorine Chemistry 131 (2010) 98-105).

### Verfahren 2

Weiterhin kann 4-Heptafluorisopropyl-2-methyl-6-trifluormethyl-anilin der Struktur (**K-1**) ausgehend von 4-Heptafluorisopropyl-2-methyl-anilin nach dem in Schema 2 angegebenen Verfahren durch Umsetzung mit Natrium-trifluormethylsulfinat in Gegenwart von Oxidationsmitteln und Übergangsmetall Katalysatoren hergestellt werden. 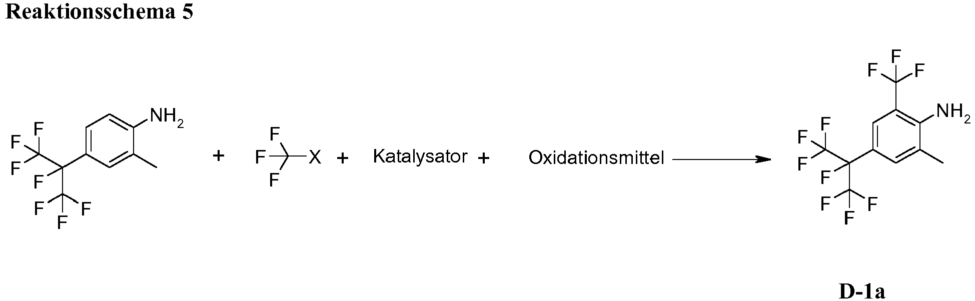 X bedeutet Br, I, NaSO₂⁻ (Natriumtrifluormethylsulfinat), KSO₂⁻ (Kaliumtrifluormethylsulfinat). Besonders bevorzugt ist Natriumtrifluormethylsulfinat.

4-Heptafluorisopropyl-2-methyl-anilin ist bekannt (US2004/92762).

Geeignete Katalysatoren sind Übergangsmetalle wie Eisen(II)sulfat, Eisen(III)nitrat, Kupfer(II)triflat oder Ferrocen. Besonders bevorzugt ist Eisen(II)sulfat.

Geeignete Oxidationsmittel sind vor allem Peroxide wie Wasserstoffperoxid, tert.-Butylhydroperoxid oder Natriumperoxodisulfat, Kaliumperoxodisulfat, Natriumperoxomonosulfat oder Kaliumperoxomonosulfat. Besonders bevorzugt ist tert.-Butylhydroperoxid.

Bei der Durchführung der Reaktion können geeignete Lösungsmittel verwendet werden.

Als Verdünnungs- bzw. Lösungsmittel zur Durchführung der erfindungsgemäßen Verfahren kommen grundsätzlich alle unter den spezifischen Reaktionsbedingungen inerten organischen Lösungsmittel in Frage. Als Beispiele sind zu nennen: Nitrile wie Acetonitril, Propionitril, Butyronitril, Isobutyronitril; Wasser, Tetrahydrothiophendioxid, Dimethylsulfoxid, Tetramethylensulfoxid, Dipropylsulfoxid, Diisobutylsulfoxid, Dibutylsulfoxid, Diisoamylsulfoxid, Sulfone (z.B. Dimethyl-, Diethyl-, Dipropyl-, Dibutyl-, Dihexyl-, Methylethyl-, Ethylpropyl-, Ethylisobutyl- und Pentamethylensulfon); aliphatische, cycloaliphatische (z.B. Pentan, Hexan, Heptan, Oktan, Nonan und technische Kohlenwasserstoffe), ferner sogenannte "White Spirits" mit Komponenten mit Siedepunkten im Bereich von beispielsweise 40 °C bis 250 °C, Benzinfraktionen innerhalb eines Siedeinterwalles von 70°C bis 190 °C, Cyclohexan, Methylcyclohexan, Petrolether, Ligroin, Octan.

Als bevorzugtes Verdünnungsmittel kann jedes Lösungsmittel verwendet werden, das die Reaktion nicht beeinträchtigt, wie zum Beispiel Wasser; Nitrile wie Acetonitril, Propionitril, Butyronitril, Isobutyronitril. Die Lösungsmittel können allein oder in Kombination von 2 oder mehr eingesetzt werden.

Bei den Umsetzungen können Basen zugesetzt werden. Als Beispiele sind zu nennen Erdalkali- oder Alkalimetallverbindungen (z.B. Hydroxide, Hydride, Oxide und Carbonate des Lithiums, Natriums, Kaliums, Magnesiums, Calciums und Bariums).

Als bevorzugtes basisches Reaktionshilfsmittel kann Natriumhydrogencarbonat verwendet werden; des Weiteren können beispielsweise folgende Basen eingesetzt werden: Alkalimetallhydroxide wie z.B Natriumhydroxid und Kaliumhydroxid; Carbonate wie Natriumhydrogencarbonat und Kaliumcarbonat; Phosphate wie Natrium-dihydrogenphosphat, Dikalium-hydrogenphosphat und Tri-natriumphosphat.

Die Reaktion kann in einem weiten Temperaturbereich ausgeführt werden. Gewöhnlich wird sie in einem Temperaturbereich von -78 bis 200 °C, bevorzugt bei Temperaturen zwischen -10 und 150 °C durchgeführt. Die Reaktion kann unter erhöhtem als auch vermindertem Druck ausgeführt werden. Bevorzugt wird sie aber unter Normaldruck durchgeführt. Die Reaktionszeiten liegen zwischen 0,1und 72 Stunden, bevorzugt zwischen 1 und 24 Stunden.

Zur Durchführung der Reaktion werden 1 bis 10 Mol, bevorzugt 1 bis 4 Mol an Trifluormethylierungsmittel; 1 bis 20 Mol, bevorzugt 1 bis 8 Mol Oxidationsmittel und 0,01 bis 1 Mol, bevorzugt 0,05 bis 0,4 Mol Katalysator pro Mol 4-Heptafluorisopropyl-2-methyl-6-trifluormethyl-anilin in einem Lösungsmittel oder Lösungsmittelgemisch wie z.B. in einem Gemisch aus Acetonitril und Wasser eingesetzt.

### Verfahren I-T46 erweitert

Die Verbindungen der Struktur (I-T46) können nach dem im Reaktionsschema angegebenen Verfahren hergestellt werden.

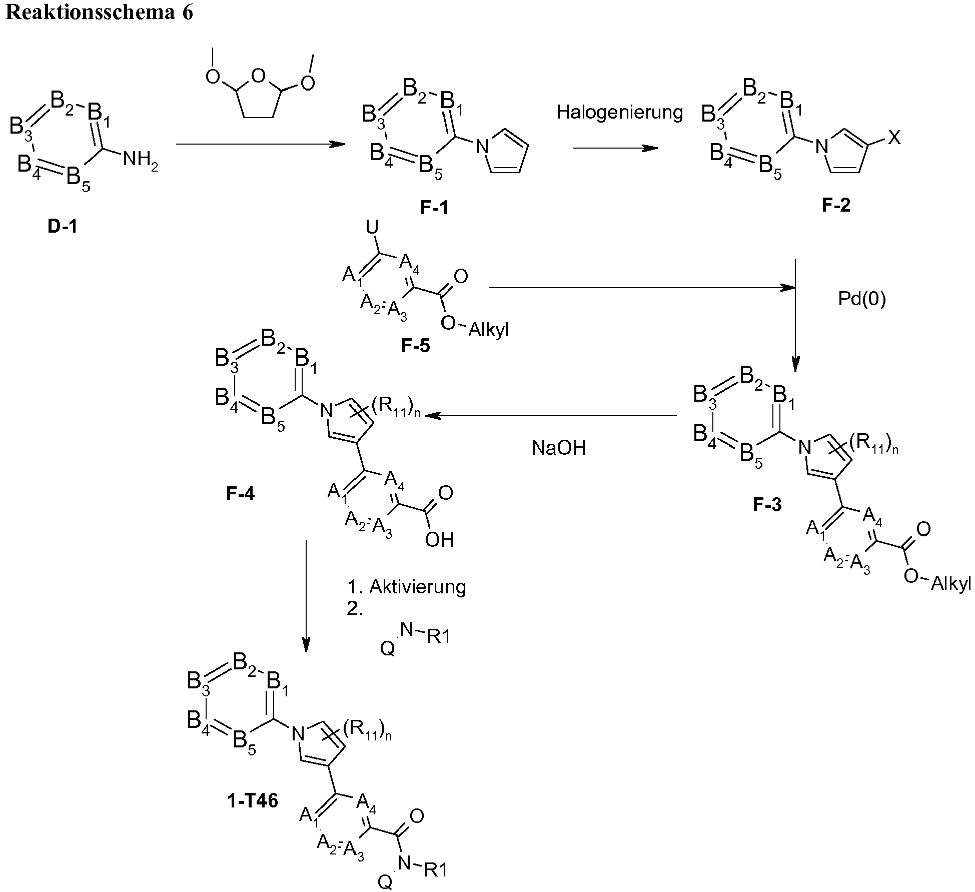

Die Reste A₁-A₄, B₁-B₅, Alkyl, Q, R¹ und R¹¹ haben die oben beschriebenen Bedeutungen. U steht für eine Boronsäure, Boronsäureester oder Trifluorboronat. X steht für Brom, Iod oder Triflat. Ausgangsverbindungen der Struktur (**F-1**) und (**F-5**) sind bekannt (z.B. **F-1**: Hulcoop, David G. et al., Organic Letters, 9 (2007), S. 1761-1764, Supporting information Seite 1 ff), oder können nach bekannten Methoden (z.B. aus **D-1**) hergestellt werden.

Die Durchführung der Reaktionen kann nach den in der Literatur beschriebenen Verfahren erfolgen z.B. US2009/209476, S. 18-19.

### Stufe 1 Pyrrol Ringschluss

Stufe 1 des Darstellungsverfahrens der erfindungsgemäßen Verbindungen (**I-T46**):

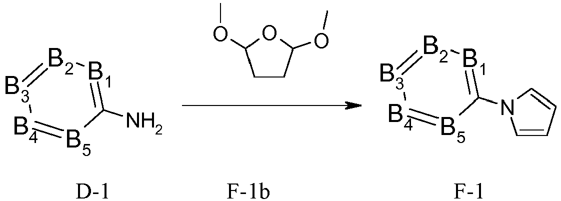

Erfindungsgemäße Verbindungen der allgemeinen Struktur **(F-1)** können in Analogie zu literaturbekannten Methoden aus den Ausgangsmaterialien der Struktur (**D-1**) und (**F-1b**) hergestellt werden. Die Reste B¹-B⁵ haben die oben beschriebenen Bedeutungen. Die Verbindungen der Strukture (**D-1**) sind literaturbekannt (z.B. US2002/198399, WO2009/30457, Seite 28) oder können nach literaturbekannten Methoden hergestellt werden. Die Verbindung (**F1-b**) ist komerziell erhältlich. Als typische Vetreter der Verbindungen der Struktur (**D-1**) seien 2-Amino-1,3-dichlor-5-[1,2,2,2-tetrafluor-1-(trifluormethyl)ethyl]benzol, 2-Amino-1,3-dimethyl-5-[1,2,2,2-tetrafluor-1-(trifluormethyl)ethyl]benzol, 2- Amino -1-ethyl-3-methyl-5-[1,2,2,2-tetrafluor-1-(trifluormethyl)ethyl]benzol, 2- Amino -1-chlor-5-[1,2,2,2-tetrafluor-1-(trifluormethyl)ethyl]-3-(trifluormethyl)benzol, 2- Amino -1-methyl-5-[1,2,2,2-tetrafluor-1-(trifluormethyl)ethyl]-3-(trifluormethyl)benzol, 2- Amino -1-chlor-5-[1,2,2,2-tetrafluor-1-(trifluormethyl)ethyl]-3-(trifluormethoxy)benzol, 2- Amino -1-methyl-5-[1,2,2,2-tetrafluor-1-(trifluormethyl)ethyl]-3-(trifluormethoxy)benzol genannt. Die Reaktion wird unter den in der Literatur für analoge Verbindungen bekannten Bedingungen durchgeführt (z.B. Hulcoop, David G. et al., Organic Letters, 9 (2007), S. 1761-1764, Supporting information Seite 1 ff)

### Stufe 2 Halogenierung

Stufe 1 des Darstellungsverfahrens der erfindungsgemäßen Verbindungen **(I-T46):**

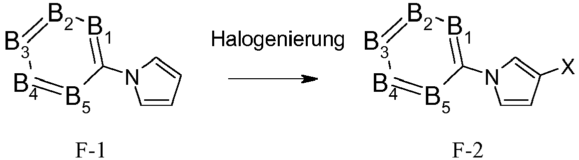

Erfindungsgemäße Verbindungen der allgemeinen Struktur (**F-2**) können in Analogie zu literaturbekannten Methoden aus den Ausgangsmaterialien der Struktur (**F-1**) durch Halogenierung hergestellt werden. Die Reste B¹-B⁵ haben die oben beschriebenen Bedeutungen. Die Verbindungen der Strukture (**F-1**) sind literaturbekannt (z.B. F-1: Hulcoop, David G. et al., Organic Letters, 9 (2007), S. 1761-1764, Supporting information Seite 1 ff) oder können nach der weiter oben beschriebenen Methode hergestellt werden. Als typische Vetreter der Verbindungen der Struktur (**F-1**) seien 1-[2,6-Dichlor-4-[1,2,2,2-tetrafluor-1-(trifluormethyl)ethyl]phenyl]pyrrol, 1-[2,6-Dimethyl-4-[1,2,2,2-tetrafluor-1 -(trifluormethyl)ethyl]phenyl]pyrrol, 1-[2-Ethyl-6-methyl-4-[1,2,2,2-tetrafluor-1-(trifluormethyl)ethyl]phenyl]pyrrol, 1-[2-Chlor-4-[1,2,2,2-tetrafluor-1-(trifluormethyl)ethyl]-6-(trifluormethyl)phenyl]pyrrol, 1-[2-Methyl-4-[1,2,2,2-tetrafluor-1-(trifluormethyl)ethyl]-6-(trifluormethyl)phenyl]pyrrol, 1-[1-Chlor-5-[1,2,2,2-tetrafluor-1-(trifluormethyl)ethyl]-6-(trifluormethoxy)phenyl]pyrrol, 1-[1-Methyl-5-[1,2,2,2-tetrafluor-1-(trifluormethyl)ethyl]-6-(trifluormethoxy)phenyl]pyrrol genannt.

Geeignete halogenierende Verbindungen sind dem Fachmann bekannt wie z.B. Brom, Iod, N-Bromsuccinimid, N-Iodsuccinimid, 1,3-Dibrom-5,5-dimethylhydantoin und Benzyltrimethylammonium-tetrachloroiodat. Bevorzugt werden Brom, Iod und Iodsuccinimid eingesetzt. Die Umsetzung folgt den aus der Literatur bekannten Bedingungen (z. B. Tatsuta; Itoh Bulletin of the Chemical Society of Japan, 67 (1994) 1449-1455).

### Stufe 3 Boronsäure Kupplung

Stufe 3 des Darstellungsverfahrens der erfindungsgemäßen Verbindungen (**I-T46**):

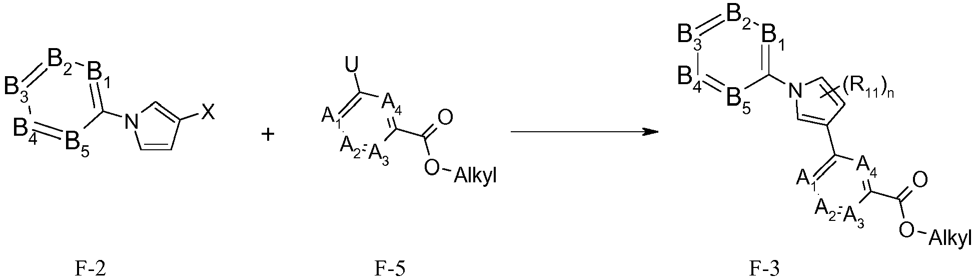

Die Reste A₁ bis A₄, B₁ bis B₅, Alkyl, n und R¹¹ haben die oben beschriebenen Bedeutungen. U steht z. B. für eine Boronsäure, Boronsäureester oder Trifluorboronat, X steht für Brom, Iod oder Triflat.

Erfindungsgemäße Verbindungen der allgemeinen Struktur (**F-3**) können nach literaturbekannten Verfahren mittels Palladium katalysierten Reaktionen aus den Reaktionspartnern der allgemeinen Struktur (**F-2**) und (**F-5**) hergestellt werden (z. B. WO 2005/040110 oder WO 2009/089508). Die Verbindungen der allgemeinen Struktur (**F-5**) sind entweder kommerziell erhältlich oder können nach dem Fachmann bekannten Verfahren hergestellt werden.

### Stufen 4, 5 Verseifung, Amidierung

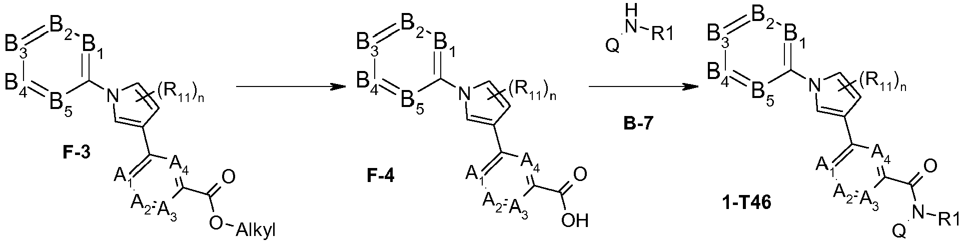

Erfindungsgemäße Verbindungen der allgemeinen Struktur (**I-T46**) können in Analogie zu literaturbekannten Peptidkupplungsmethoden aus den Ausgangsmaterialien (**F-4**) und (**B-7**) hergestellt werden (z. B. WO 2010/051926 oder WO 2010/133312). Verbindungen der allgemeinen Struktur (**F-4**) lassen sich in Analogie zu literaturbekannten Verfahren durch Esterspaltung aus Verbindungen der allgemeinen Struktur (**F-3**) herstellen (z. B. WO 2010/051926 oder WO 2010/133312). Die Reste A₁ bis A₄, B₁ bis B₅, Alkyl, Q, R¹ und R¹¹ haben die oben beschriebenen Bedeutungen.

### Q

In einer mehr bevorzugten Ausführungsform steht Q in einer Verbindung der Formel (I) oder (Ia") oder (IT-2) oder (I-T3) oder (I-T4) oder (I-T22) oder (I-T23) oder (I-T46) für C₁-C₄-Alkyl, 2-oxo-2-(2,2,2-trifluoroethylamino)-ethyl, mit Fluor oder 1-cyano-propyl oder Pyridin substituiertes C₁-C₄-Alkyl wie 2,2,2-Trifluorethyl, 2,2-Difluorethyl, 3,3,3-Trifluropropyl, Pyridin-2-ylmethyl oder (1-Cyano-cyclopropyl)methyl; C₃-C₄-Cycloalkyl wie Cyclopropyl oder Cyclobutyl; gegebenenfalls substituiertes C₃-C₄-Cycloalkyl wie mit optional mit Fluor substituiertes C₁-C₄-Alkyl substituiertes Cyclopropyl (z. B. 1 -Trifluoromethyl-cyclopropyl, 1-tert.Butyl-cyclopropyl), 1-Thiocarbamoyl-cyclopropyl, 1-Carbamoylcyclopropyl, 1-Cyano-cyclopropyl, trans-2-Fluorcyclopropyl, cis-2-Fluorcyclopropyl; C₄-C₆-Heterocycloalkyl wie Oxetan-3-yl, Thietan-3-yl, 1-Oxido-thietan-3-yl, oder 1,1-Dioxido-thietan-3-yl; oder jeweils gegebenenfalls mit C₁-C₄-Alkyl substituiertes Benzyl; Pyrazol (wie N-methyl-pyrazol-3-yl), Pyridin; Methylsulfonyl; oder 2-oxo-2-(2,2,2-trifluoroethylamino)ethyl steht.

In einer besonders bevorzugten Ausführungsform steht Q in einer Verbindung der Formel (I) oder (Ia") oder (IT-2) oder (I-T3) oder (I-T4) oder (I-T22) oder (I-T23) oder (I-T46) für mit Fluor substituiertes C₁-C₃-Alkyl wie 2,2,2-trifluoroethyl oder 3,3,3-trifluoropropyl; Cyclopropyl; gegebenenfalls mit Cyano, C₁-C₄-Alkyl substituiertes Cyclopropyl wie 1-Cyano-cyclopropyl oder 1-TriFluoromethyl-cyclopropyl; Thietan-3-yl; oder 2-oxo-2-(2,2,2-trifluoroethyl)aminoethyl.

### Formel (I)

Eine weitere bevorzugte Ausführungsform betrifft Verbindungen der Formel (I) in der T für T2, T3, T4, T22, T23 oder T46 steht und alle weiteren Parameter wie in Paragraph [0009] definiert sind.

Eine weitere bevorzugte Ausführungsform betrifft Verbindungen der Formel (I) in der T für T2, T3, T4, T22, T23 oder T46 steht und alle weiteren Parameter wie in Paragraph [0009] definiert sind.

Eine weitere bevorzugte Ausführungsform betrifft Verbindungen der Formel (I) in der T für T2 oder T4 steht und alle weiteren Parameter wie in Paragraph [0009] definiert sind.

Eine weitere bevorzugte Ausführungsform betrifft Verbindungen der Formel (I) in der T für T3 oder T46 steht und alle weiteren Parameter wie in Paragraph [0009] definiert sind.

Eine weitere bevorzugte Ausführungsform betrifft Verbindungen der Formel (I) in der T für T22 oder T23 steht und alle weiteren Parameter wie in Paragraph [0009] definiert sind.

Eine weitere bevorzugte Ausführungsform betrifft Verbindungen der Formel (I) in der T für T2, T3, T4, T22, T23 oder T46 steht, B3 für C-R8 steht und R8 für ein jeweils substituiertes (C1-C6)-Alkyl, (C1-C6)-Alkoxy oder Alkylsulfanyl steht, wobei die Substituenten ausgewählt sind aus Halogen und Hydroxy wobei mindestens ein Substituent Halogen ist und alle weiteren Parameter wie in Paragraph [0009] definiert sind.

Eine weitere bevorzugte Ausführungsform betrifft Verbindungen der Formel (I) in der T für T2, T3, T4, T22, T23 oder T46 steht, B3 für C-R8 steht und R8 für ein jeweils substituiertes (C1-C6)-Alkyl, (C1-C6)-Alkoxy oder Alkylsulfanyl steht, wobei die Substituenten ausgewählt sind aus Halogen und Hydroxy wobei mindestens ein Substituent Halogen ist und alle weiteren Parameter wie in Paragraph [0009] definiert sind.

Eine weitere bevorzugte Ausführungsform betrifft Verbindungen der Formel (I) in der T für T2 oder T4 steht, B3 für C-R8 steht und R8 für ein jeweils substituiertes (C1-C6)-Alkyl, (C1-C6)-Alkoxy oder Alkylsulfanyl steht, wobei die Substituenten ausgewählt sind aus Halogen und Hydroxy wobei mindestens ein Substituent Halogen ist und alle weiteren Parameter wie in Paragraph [0009] definiert sind.

Eine weitere bevorzugte Ausführungsform betrifft Verbindungen der Formel (I) in der T für T3 oder T46 steht, B3 für C-R8 steht und R8 für ein jeweils substituiertes (C1-C6)-Alkyl, (C1-C6)-Alkoxy oder Alkylsulfanyl steht, wobei die Substituenten ausgewählt sind aus Halogen und Hydroxy wobei mindestens ein Substituent Halogen ist und alle weiteren Parameter wie in Paragraph [0009] definiert sind.

Eine weitere bevorzugte Ausführungsform betrifft Verbindungen der Formel (I) in der T für T22 oder T23 steht, B3 für C-R8 steht und R8 für ein jeweils substituiertes (C1-C6)-Alkyl, (C1-C6)-Alkoxy oder Alkylsulfanyl steht, wobei die Substituenten ausgewählt sind aus Halogen und Hydroxy wobei mindestens ein Substituent Halogen ist und alle weiteren Parameter wie in Paragraph [0009] definiert sind. Hierbei bezieht sich eine besonders bevorzugte Ausführungsform auf Verbindungen in denen R⁸ für perfluoriertes (C1-C6)-Alkyl, (C1-C6)-Alkoxy oder Alkylsulfanyl steht, ganz besonders bevorzugt für perfluoriertes (C1-C4)-Alkyl, (Cl-C4)-Alkoxy steht.

Eine weitere bevorzugte Ausführungsform bezieht sich auf Verbindungen der Formel (I) in der T für T2, T3, T4, T22, T23 oder T46 steht, B3 für C-R8 steht und R8 für ein jeweils substituiertes (C1-C6)-Alkyl, (C1-C6)-Alkoxy oder Alkylsulfanyl steht, wobei die Substituenten ausgewählt sind aus Halogen und Hydroxy wobei mindestens ein Substituent Halogen ist, besonders bevorzugt worin R8 für perfluoriertes (Cl-C6)-Alkyl, (C1-C6)-Alkoxy oder Alkylsulfanyl steht, ganz besonders bevorzugt für perfluoriertes (C1-C4)-Alkyl, (C1-C4)-Alkoxy steht, in der B₁, B₂, B₄ und B₅ für CR⁶, CR⁷, CR⁹ bzw. CR¹⁰ stehen worin R⁶, R⁷, R⁹ und R¹⁰ unabhängig voneinander für H, Halogen, Cyano, Nitro, jeweils gegebenenfalls mit mindestens einem Substituenten ausgewählt aus Halogen und Hydroxy, wobei mindetens ein Subtituent ein Halogen ist, substituiertes C₁-C₄-Alkyl, C₃-C₄-Cycloalkyl, C₁-C₄-Alkoxy, *N*-Alkoxyiminoalkyl, C₁-C₄-Alkylsulfanyl, C₁-C₄-Alkylsulfinyl, C₁-C₄-Alkylsulfonyl, *N-*C₁-C₄-Alkylamino, *N*,*N*-Di-C₁-C₄-alkylamino stehen und alle weiteren Parameter wie in Paragraph [0009] definiert sind. In einer weiteren bevorzugten Ausführungsform stehen R⁶ und R¹⁰ jeweils für Halogen (wie Cl, Br oder F), jeweils für C₁-C₃-alkyl, oder jeweils für mit Halogen substituiertes C₁-C₃-alkyl wie z. B. perfluoriertes C₁-C₃-alkyl (Perfluormethyl, perFluoroethyl oder Perfluorpropyl).

Eine weitere bevorzugte Ausführungsform bezieht sich auf Verbindungen der Formel (I) in der T für T2, T3, T4, T22, T23 oder T46 steht, B3 für C-R8 steht und R8 für ein jeweils substituiertes (C1-C6)-Alkyl, (C1-C6)-Alkoxy oder Alkylsulfanyl steht, wobei die Substituenten ausgewählt sind aus Halogen und Hydroxy wobei mindestens ein Substituent Halogen ist, besonders bevorzugt worin R8 für perfluoriertes (C1-C6)-Alkyl,(C1-C6)-Alkoxy oder Alkylsulfanyl steht, ganz besonders bevorzugt für perfluoriertes (C1-C4)-Alkyl, (C1-C4)-Alkoxy steht, in der B₁, B₂ und B₄ für CR⁶, CR⁷, bzw. CR⁹ stehen und B₅ für N steht worin R⁶, R⁷ und R⁹ unabhängig voneinander für H, Halogen, Cyano, Nitro, jeweils gegebenenfalls mit mindestens einem Substituenten ausgewählt aus Halogen und Hydroxy, wobei mindetens ein Subtituent ein Halogen ist, substituiertes C₁-C₄-Alkyl, C₃-C₄-Cycloalkyl, C₁-C₄-Alkoxy, *N*-Alkoxyiminoalkyl, C₁-C₄-Alkylsulfanyl, C₁-C₄-Alkylsulfinyl, C₁-C₄-Alkylsulfonyl, *N-*C₁-C₄-Alkylamino, *N*,*N*-Di-C₁-C₄-alkylamino stehen und alle weiteren Parameter wie in Paragraph [0009] definiert sind.

Eine weitere bevorzugte Ausführungsform bezieht sich auf Verbindungen der Formel (I) in der T für T2, T3, T4, T22, T23 oder T46 steht, B3 für C-R8 steht und R8 für ein jeweils substituiertes (C1-C6)-Alkyl, (C1-C6)-Alkoxy oder Alkylsulfanyl steht, wobei die Substituenten ausgewählt sind aus Halogen und Hydroxy wobei mindestens ein Substituent Halogen ist, besonders bevorzugt worin R8 für perfluoriertes (C1-C6)-Alkyl, (C1-C6)-Alkoxy oder Alkylsulfanyl steht, ganz besonders bevorzugt für perfluoriertes (C1-C4)-Alkyl, (C1-C4)-Alkoxy steht, in der B₁, B₂, B₄ und B₅ für CR⁶, CR⁷, CR⁹ bzw. CR¹⁰ stehen worin R⁶, R⁷, R⁹ und R¹⁰ unabhängig voneinander für H, Halogen, Cyano, Nitro, jeweils gegebenenfalls mit mindestens einem Substituenten ausgewählt aus Halogen und Hydroxy, wobei mindetens ein Subtituent ein Halogen ist, substituiertes C₁-C₄-Alkyl, C₃-C₄-Cycloalkyl, C₁-C₄-Alkoxy, *N*-Alkoxyiminoalkyl, C₁-C₄-Alkylsulfanyl, C₁-C₄-Alkylsulfinyl, C₁-C₄-Alkylsulfonyl, *N-*C₁-C₄-Alkylamino, *N*,*N*-Di-C₁-C₄-alkylamino stehen, R¹¹ jeweils unabhängig voneinander für H, Amino (NH₂) oder Cyano, bevorzugt für H, steht, W für O steht, R¹ für H, Methyl, Ethyl, n-Propyl, Isopropyl, n-Butyl, Isobutyl, s-Butyl, t-Butyl steht, bevorzugt für H oder Methyl steht und alle weiteren Parameter wie in Paragraph [0009] definiert sind. In einer weiteren bevorzugten Ausführungsform stehen R⁶ und R¹⁰ jeweils für Halogen (wie Cl, Br oder F), jeweils für C₁-C₃-alkyl, oder jeweils für mit Halogen substituiertes C₁-C₃-alkyl wie z. B. perfluoriertes C₁-C₃-alkyl (Perfluormethyl, perFluoroethyl oder Perfluorpropyl).

Eine weitere bevorzugte Ausführungsform bezieht sich auf Verbindungen der Formel (I) in der T für T2, T3, T4, T22, T23 oder T46 steht, B3 für C-R8 steht und R8 für ein jeweils substituiertes (C1-C6)-Alkyl, (C1-C6)-Alkoxy oder Alkylsulfanyl steht, wobei die Substituenten ausgewählt sind aus Halogen und Hydroxy wobei mindestens ein Substituent Halogen ist, besonders bevorzugt worin R8 für perfluoriertes (C1-C6)-Alkyl, (C1-C6)-Alkoxy oder Alkylsulfanyl steht, ganz besonders bevorzugt für perfluoriertes (C1-C4)-Alkyl, (C1-C4)-Alkoxy steht in der B₁, B₂ und B₄ für CR⁶, CR⁷, bzw. CR⁹ stehen und B₅ für N steht worin R⁶, R⁷ und R⁹ unabhängig voneinander für H, Halogen, Cyano, Nitro, jeweils gegebenenfalls mit mindestens einem Substituenten ausgewählt aus Halogen und Hydroxy, wobei mindetens ein Subtituent ein Halogen ist, substituiertes C₁-C₄-Alkyl, C₃-C₄-Cycloalkyl, C₁-C₄-Alkoxy, *N*-Alkoxyiminoalkyl, C₁-C₄-Alkylsulfanyl, C₁-C₄-Alkylsulfinyl, C₁-C₄-Alkylsulfonyl, *N-*C₁-C₄-Alkylamino, *N*,*N*-Di-C₁-C₄-alkylamino stehen, R¹¹ jeweils unabhängig voneinander für H, Amino (NH₂) oder Cyano, bevorzugt für H, steht, W für O steht, R¹ für H, Methyl, Ethyl, n-Propyl, Isopropyl, n-Butyl, Isobutyl, s-Butyl, t-Butyl steht, bevorzugt für H oder Methyl steht und alle weiteren Parameter wie in Paragraph [0009] definiert sind.

Eine weitere bevorzugte Ausführungsform bezieht sich auf Verbindungen der Formel (I) in der T für T2, T3, T4, T22, T23 oder T46 steht, B3 für C-R8 steht und R8 für ein jeweils substituiertes (C1-C6)-Alkyl, (C1-C6)-Alkoxy oder Alkylsulfanyl steht, wobei die Substituenten ausgewählt sind aus Halogen und Hydroxy wobei mindestens ein Substituent Halogen ist, besonders bevorzugt worin R8 für perfluoriertes (Cl-C6)-Alkyl, (C1-C6)-Alkoxy oder Alkylsulfanyl steht, ganz besonders bevorzugt für perfluoriertes (C1-C4)-Alkyl, (C1-C4)-Alkoxy steht, in der B₁, B₂, B₄ und B₅ für CR⁶, CR⁷, CR⁹ bzw. CR¹⁰ stehen worin R⁶, R⁷, R⁹ und R¹⁰ unabhängig voneinander für H, Halogen, Cyano, Nitro, jeweils gegebenenfalls mit mindestens einem Substituenten ausgewählt aus Halogen und Hydroxy, wobei mindetens ein Subtituent ein Halogen ist, substituiertes C₁-C₄-Alkyl, C₃-C₄-Cycloalkyl, C₁-C₄-Alkoxy, *N*-Alkoxyiminoalkyl, C₁-C₄-Alkylsulfanyl, C₁-C₄-Alkylsulfinyl, C₁-C₄-Alkylsulfonyl, *N-*C₁-C₄-Alkylamino, *N*,*N*-Di-C₁-C₄-alkylamino stehen, R¹¹ jeweils unabhängig voneinander für H, Amino (NH₂) oder Cyano, bevorzugt für H, steht, W für O steht, R¹ für H, Methyl, Ethyl, n-Propyl, Isopropyl, n-Butyl, Isobutyl, s-Butyl, t-Butyl steht, bevorzugt für H oder Methyl steht, Q für C₁-C₄-Alkyl, 2-oxo-2-(2,2,2-trifluoroethylamino)-ethyl, mit Fluor oder 1-cyano-propyl oder Pyridin substituiertes C₁-C₄-Alkyl wie 2,2,2-Trifluorethyl, 2,2-Difluorethyl, 3,3,3-Trifluropropyl, Pyridin-2-ylmethyl oder (1-Cyano-cyclopropyl)methyl; C₃-C₄-Cycloalkyl wie Cyclopropyl oder Cyclobutyl; gegebenenfalls substituiertes C₃-C₄-Cycloalkyl wie mit optional mit Fluor substituiertes C₁-C₄-Alkyl substituiertes Cyclopropyl (z. B. 1-Trifluoromethyl-cyclopropyl, 1-tert.Butyl-cyclopropyl), 1-Thiocarbamoyl-cyclopropyl, 1-Carbamoylcyclopropyl, 1-Cyano-cyclopropyl, trans-2-Fluorcyclopropyl, cis-2-Fluorcyclopropyl; C₄-C₆-Heterocycloalkyl wie Oxetan-3-yl, Thietan-3-yl, 1-Oxido-thietan-3-yl, oder 1,1-Dioxido-thietan-3-yl; oder jeweils gegebenenfalls mit C₁-C₄-Alkyl substituiertes Benzyl; Pyrazol (wie N-methyl-pyrazol-3-yl), Pyridin; Methylsulfonyl; oder 2-oxo-2-(2,2,2-trifluoroethylamino)ethyl, bevorzugt für mit Fluor substituiertes C₁-C₃-Alkyl wie 2,2,2-trifluoroethyl oder 3,3,3-trifluoropropyl; Cyclopropyl; gegebenenfalls substituiertes Cyclopropyl wie 1-Cyano-cyclopropyl oder 1-TriFluoromethyl-cyclopropyl, Thietan-3-yl; oder 2-oxo-2-(2,2,2-trifluoroethyl)aminoethyl, steht und alle weiteren Parameter wie in Paragraph [0009] definiert sind. In einer weiteren bevorzugten Ausführungsform stehen R⁶ und R¹⁰ jeweils für Halogen (wie Cl, Br oder F), jeweils für C₁-C₃-alkyl, oder jeweils für mit Halogen substituiertes C₁-C₃-alkyl wie z. B. perfluoriertes C₁-C₃-alkyl (Perfluormethyl, perFluoroethyl oder Perfluorpropyl).

Eine weitere bevorzugte Ausführungsform bezieht sich auf Verbindungen der Formel (I) in der T für T2, T3, T4, T22, T23 oder T46 steht, B3 für C-R8 steht und R8 für ein jeweils substituiertes (C1-C6)-Alkyl, (C1-C6)-Alkoxy oder Alkylsulfanyl steht, wobei die Substituenten ausgewählt sind aus Halogen und Hydroxy wobei mindestens ein Substituent Halogen ist, besonders bevorzugt worin R8 für perfluoriertes (C1-C6)-Alkyl, (C1-C6)-Alkoxy oder Alkylsulfanyl steht, ganz besonders bevorzugt für perfluoriertes (C1-C4)-Alkyl, (C1-C4)-Alkoxy steht in der B₁, B₂ und B₄ für CR⁶, CR⁷, bzw. CR⁹ stehen und B₅ für N steht worin R⁶, R⁷ und R⁹ unabhängig voneinander für H, Halogen, Cyano, Nitro, jeweils gegebenenfalls mit mindestens einem Substituenten ausgewählt aus Halogen und Hydroxy, wobei mindetens ein Subtituent ein Halogen ist, substituiertes C₁-C₄-Alkyl, C₃-C₄-Cycloalkyl, C₁-C₄-Alkoxy, *N*-Alkoxyiminoalkyl, C₁-C₄-Alkylsulfanyl, C₁-C₄-Alkylsulfinyl, C₁-C₄-Alkylsulfonyl, *N-*C₁-C₄-Alkylamino, *N*,*N*-Di-C₁-C₄-alkylamino stehen, R¹¹ jeweils unabhängig voneinander für H, Amino (NH₂) oder Cyano, bevorzugt für H, steht, W für O steht, R¹ für H, Methyl, Ethyl, n-Propyl, Isopropyl, n-Butyl, Isobutyl, s-Butyl, t-Butyl steht, bevorzugt für H oder Methyl steht, Q für C₁-C₄-Alkyl, 2-oxo-2-(2,2,2-trifluoroethylamino)-ethyl, mit Fluor oder 1-cyano-propyl oder Pyridin substituiertes C₁-C₄-Alkyl wie 2,2,2-Trifluorethyl, 2,2-Difluorethyl, 3,3,3-Trifluropropyl, Pyridin-2-ylmethyl oder (1-Cyano-cyclopropyl)methyl; C₃-C₄-Cycloalkyl wie Cyclopropyl oder Cyclobutyl; gegebenenfalls substituiertes C₃-C₄-Cycloalkyl wie mit optional mit Fluor substituiertes C₁-C₄-Alkyl substituiertes Cyclopropyl (z. B. 1 -Trifluoromethyl-cyclopropyl, 1-tert.Butyl-cyclopropyl), 1-Thiocarbamoyl-cyclopropyl, 1-Carbamoylcyclopropyl, 1-Cyano-cyclopropyl, trans-2-Fluorcyclopropyl, cis-2-Fluorcyclopropyl; C₄-C₆-Heterocycloalkyl wie Oxetan-3-yl, Thietan-3-yl, 1-Oxido-thietan-3-yl, oder 1,1-Dioxido-thietan-3-yl; oder jeweils gegebenenfalls mit C₁-C₄-Alkyl substituiertes Benzyl; Pyrazol (wie N-methyl-pyrazol-3-yl), Pyridin; Methylsulfonyl; oder 2-oxo-2-(2,2,2-trifluoroethylamino)ethyl, bevorzugt für mit Fluor substituiertes C₁-C₃-Alkyl wie 2,2,2-trifluoroethyl oder 3,3,3-trifluoropropyl; Cyclopropyl; gegebenenfalls substituiertes Cyclopropyl wie 1-Cyano-cyclopropyl oder 1-TriFluoromethyl-cyclopropyl, Thietan-3-yl; oder 2-oxo-2-(2,2,2-trifluoroethyl)aminoethyl,, steht und alle weiteren Parameter wie in Paragraph [0009] definiert sind.

Eine weitere bevorzugte Ausführungsform bezieht sich auf Verbindungen der Formel (I) in der T für T2, T3, T4, T22, T23 oder T46 steht, B3 für C-R8 steht und R8 für ein jeweils substituiertes (C1-C6)-Alkyl, (C1-C6)-Alkoxy oder Alkylsulfanyl steht, wobei die Substituenten ausgewählt sind aus Halogen und Hydroxy wobei mindestens ein Substituent Halogen ist, besonders bevorzugt worin R8 für perfluoriertes (Cl-C6)-Alkyl, (C1-C6)-Alkoxy oder Alkylsulfanyl steht, ganz besonders bevorzugt für perfluoriertes (C1-C4)-Alkyl, (C1-C4)-Alkoxy steht, in der B₁, B₂, B₄ und B₅ für CR⁶, CR⁷, CR⁹ bzw. CR¹⁰ stehen worin R⁶, R⁷, R⁹ und R¹⁰ unabhängig voneinander für H, Halogen, Cyano, Nitro, jeweils gegebenenfalls mit mindestens einem Substituenten ausgewählt aus Halogen und Hydroxy, wobei mindetens ein Subtituent ein Halogen ist, substituiertes C₁-C₄-Alkyl, C₃-C₄-Cycloalkyl, C₁-C₄-Alkoxy, *N*-Alkoxyiminoalkyl, C₁-C₄-Alkylsulfanyl, C₁-C₄-Alkylsulfinyl, C₁-C₄-Alkylsulfonyl, *N-*C₁-C₄-Alkylamino, *N*,*N*-Di-C₁-C₄-alkylamino stehen, R¹¹ jeweils unabhängig voneinander für H, Amino (NH₂) oder Cyano, bevorzugt für H, steht, W für O steht, R¹ für H, Methyl, Ethyl, n-Propyl, Isopropyl, n-Butyl, Isobutyl, s-Butyl, t-Butyl steht, bevorzugt für H oder Methyl steht, Q für C₁-C₄-Alkyl, 2-oxo-2-(2,2,2-trifluoroethylamino)-ethyl, mit Fluor oder 1-cyano-propyl oder Pyridin substituiertes C₁-C₄-Alkyl wie 2,2,2-Trifluorethyl, 2,2-Difluorethyl, 3,3,3-Trifluropropyl, Pyridin-2-ylmethyl oder (1-Cyano-cyclopropyl)methyl; C₃-C₄-Cycloalkyl wie Cyclopropyl oder Cyclobutyl; gegebenenfalls substituiertes C₃-C₄-Cycloalkyl wie mit optional mit Fluor substituiertes C₁-C₄-Alkyl substituiertes Cyclopropyl (z. B. 1-Trifluoromethyl-cyclopropyl, 1-tert.Butyl-cyclopropyl), 1-Thiocarbamoyl-cyclopropyl, 1-Carbamoylcyclopropyl, 1-Cyano-cyclopropyl, trans-2-Fluorcyclopropyl, cis-2-Fluorcyclopropyl; C₄-C₆-Heterocycloalkyl wie Oxetan-3-yl, Thietan-3-yl, 1-Oxido-thietan-3-yl, oder 1,1-Dioxido-thietan-3-yl; oder jeweils gegebenenfalls mit C₁-C₄-Alkyl substituiertes Benzyl; Pyrazol (wie N-methyl-pyrazol-3-yl), Pyridin; Methylsulfonyl; oder 2-oxo-2-(2,2,2-trifluoroethylamino)ethyl, bevorzugt für mit Fluor substituiertes C₁-C₃-Alkyl wie 2,2,2-trifluoroethyl oder 3,3,3-trifluoropropyl; Cyclopropyl; gegebenenfalls substituiertes Cyclopropyl wie 1-Cyano-cyclopropyl oder 1-TriFluoromethyl-cyclopropyl, Thietan-3-yl; oder 2-oxo-2-(2,2,2-trifluoroethyl)aminoethyl, steht, A₁ für CR² oder N, A₂ für CR³ oder N, A₃ für CR⁴ und A₄ für CR⁵ oder N steht, wobei R² für H, C1-C4-Alkyl oder Halogen (wie Methyl, F, Cl oder H) steht, R³ für H oder halogeniertes C1-C4-Alkyl (wie H oder -CF₃) steht, R⁴ für H, C1-C4-Alkyl, C1-C4-Alkylamin (wie -NH-CH₃), Cyclopropylamin, C1-C4-Alkoxy (wie -O-CH₃), C1-C4-Alkoxy-C1-C4-Alkylamin (wie NH-CH₂-CH₂-O-CH₃) oder Halogen (wie F oder Cl), steht. In einer weiteren bevorzugten Ausführungsform stehen R⁶ und R¹⁰ jeweils für Halogen (wie Cl, Br oder F), jeweils für C₁-C₃-aₗkyl, oder jeweils für mit Halogen substituiertes C₁-C₃-alkyl wie z. B. perfluoriertes C₁-C₃-alkyl (Perfluormethyl, perFluoroethyl oder Perfluorpropyl).

Eine weitere bevorzugte Ausführungsform bezieht sich auf Verbindungen der Formel (I) in der T für T2, T3, T4, T22, T23 oder T46 steht, B3 für C-R8 steht und R8 für ein jeweils substituiertes (C1-C6)-Alkyl, (C1-C6)-Alkoxy oder Alkylsulfanyl steht, wobei die Substituenten ausgewählt sind aus Halogen und Hydroxy wobei mindestens ein Substituent Halogen ist, besonders bevorzugt worin R8 für perfluoriertes (C1-C6)-Alkyl, (C1-C6)-Alkoxy oder Alkylsulfanyl steht, ganz besonders bevorzugt für perfluoriertes (C1-C4)-Alkyl, (C1-C4)-Alkoxy steht in der B₁, B₂ und B₄ für CR⁶, CR⁷, bzw. CR⁹ stehen und B₅ für N steht worin R⁶, R⁷ und R⁹ unabhängig voneinander für H, Halogen, Cyano, Nitro, jeweils gegebenenfalls mit mindestens einem Substituenten ausgewählt aus Halogen und Hydroxy, wobei mindetens ein Subtituent ein Halogen ist, substituiertes C₁-C₄-Alkyl, C₃-C₄-Cycloalkyl, C₁-C₄-Alkoxy, *N*-Alkoxyiminoalkyl, C₁-C₄-Alkylsulfanyl, C₁-C₄-Alkylsulfinyl, C₁-C₄-Alkylsulfonyl, *N-*C₁-C₄-Alkylamino, *N*,*N*-Di-C₁-C₄-alkylamino stehen, R¹¹ jeweils unabhängig voneinander für H, Amino (NH₂) oder Cyano, bevorzugt für H, steht, W für O steht, R¹ für H, Methyl, Ethyl, n-Propyl, Isopropyl, n-Butyl, Isobutyl, s-Butyl, t-Butyl steht, bevorzugt für H oder Methyl steht, Q für C₁-C₄-Alkyl, 2-oxo-2-(2,2,2-trifluoroethylamino)-ethyl, mit Fluor oder 1-cyano-propyl oder Pyridin substituiertes C₁-C₄-Alkyl wie 2,2,2-Trifluorethyl, 2,2-Difluorethyl, 3,3,3-Trifluropropyl, Pyridin-2-ylmethyl oder (1-Cyano-cyclopropyl)methyl; C₃-C₄-Cycloalkyl wie Cyclopropyl oder Cyclobutyl; gegebenenfalls substituiertes C₃-C₄-Cycloalkyl wie mit optional mit Fluor substituiertes C₁-C₄-Alkyl substituiertes Cyclopropyl (z. B. 1-Trifluoromethyl-cyclopropyl, 1-tert.Butyl-cyclopropyl), 1-Thiocarbamoyl-cyclopropyl, 1-Carbamoylcyclopropyl, 1-Cyano-cyclopropyl, trans-2-Fluorcyclopropyl, cis-2-Fluorcyclopropyl; C₄-C₆-Heterocycloalkyl wie Oxetan-3-yl, Thietan-3-yl, 1-Oxido-thietan-3-yl, oder 1,1-Dioxido-thietan-3-yl; oder jeweils gegebenenfalls mit C₁-C₄-Alkyl substituiertes Benzyl; Pyrazol (wie N-methyl-pyrazol-3-yl), Pyridin; Methylsulfonyl; oder 2-oxo-2-(2,2,2-trifluoroethylamino)ethyl, bevorzugt für mit Fluor substituiertes C₁-C₃-Alkyl wie 2,2,2-trifluoroethyl oder 3,3,3-trifluoropropyl; Cyclopropyl; gegebenenfalls substituiertes Cyclopropyl wie 1-Cyano-cyclopropyl oder 1-TriFluoromethyl-cyclopropyl, Thietan-3-yl; oder 2-oxo-2-(2,2,2-trifluoroethyl)aminoethyl,, steht, A₁ für CR² oder N, A₂ für CR³ oder N, A₃ für CR⁴ und A₄ für CR⁵ oder N steht, wobei R² für H, C1-C4-Alkyl oder Halogen (wie Methyl, F, Cl oder H) steht, R³ für H oder halogeniertes C1-C4-Alkyl (wie H oder -CF₃) steht, R⁴ für H, C1-C4-Alkyl, C1-C4-Alkylamin (wie -NH-CH₃), Cyclopropylamin, C1-C4-Alkoxy (wie -O-CH₃), C1-C4-Alkoxy-C1-C4-Alkylamin (wie NH-CH₂-CH₂-O-CH₃) oder Halogen (wie F oder Cl), steht.

In einer weiteren bevorzugten Ausführungsform steht R⁶ für perfluoriertes C₁-C₃-alkyl (z. B. Perfluormethyl) und R¹⁰ für Cl, Br oder F, besonders bevorzugt für Cl oder Br.

### Formel (Ia") (erfindungsgemäß)

Eine weitere bevorzugte erfindungsgemäße Ausführungsform betrifft Verbindungen der Formel (Ia") 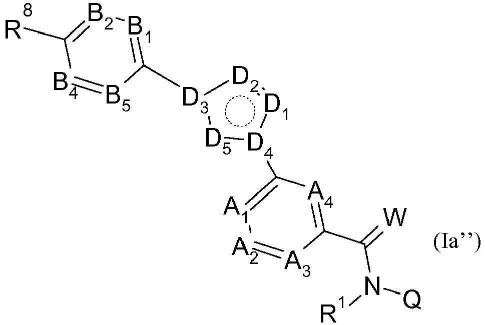 worin ein D ausgewählt aus D1 und D2 für N steht und das jeweils andere D ausgewählt aus D1 und D2 für O steht; oder D4 für N steht und ein D ausgewählt aus D1 und D5 für N steht; oder D3 für N steht und D1, D2 und D5 für C-R11 stehen und D4 für C steht und ansonsten alle Parameter wie in Paragraph [0009] beschrieben definiert sind.

In einer weiteren bevorzugten Ausführungsform stehen R⁶ und R¹⁰ (wenn die entsprechende Gruppierung B für CR steht) unabhängig voneinander für H, Halogen, Cyano, Nitro, jeweils gegebenenfalls substituiertes C₁-C₄-Alkyl, C₃-C₄-Cycloalkyl, C₁-C₄-Alkoxy, *N*-Alkoxyiminoalkyl, C₁-C₄-Alkylsulfanyl, C₁-C₄-Alkylsulfinyl, C₁-C₄-Alkylsulfonyl, *N*-C₁-C₄-Alkylamino, *N,N*-Di-C₁-C₄-alkylamino.

In einer weiteren bevorzugten Ausführungsform stehen R⁶ und R¹⁰ unabhängig voneinander für H, Halogen, Cyano, Nitro, Methyl, Ethyl, Fluormethyl, Difluormethyl, Chlordifluormethyl, Trifluormethyl, 2,2,2-Trifluorethyl, Methoxy, Ethoxy, n-Propoxy, 1-Methylethoxy, Fluormethoxy, Difluormethoxy, Chlor-difluormethoxy, Dichlor-fluormethoxy, Trifluormethoxy, 2,2,2-Trifluorethoxy, 2-Chlor-2,2-difluorethoxy, Pentafluorethoxy, N-Methoxyiminomethyl, 1-(N-Methoxyimino)-ethyl, Methylsulfanyl, Trifluormethylsulfanyl, Methylsulfonyl, Methylsulfinyl, Trifluormethylsulfonyl, Trifluormethylsulfinyl.

In einer weiteren bevorzugten Ausführungsform stehen R⁶ und R¹⁰ unabhängig voneinander für H, Halogen (insbesondere Chlor, Brom, Fluor), Cyano, Nitro, Methyl, Ethyl, Difluormethyl, Chlordifluormethyl, Trifluormethyl, Methoxy, Ethoxy, 1-Methylethoxy, Difluormethoxy, Chlor-difluormethoxy, Dichlor-fluormethoxy, Trifluormethoxy, 2,2,2-Trifluorethoxy, 2-Chlor-2,2-difluorethoxy, Methylsulfanyl, Trifluormethylsulfanyl, Methylsulfonyl, Methylsulfinyl, Trifluormethylsulfonyl, Trifluormethylsulfinyl.

In einer weiteren bevorzugten Ausführungsform stehen R⁶ und R¹⁰ für die hierin beschriebenen Substituenten, jedoch stehen R⁶ und R¹⁰ nicht gleichzeitig in einer Verbindung für H. Mit anderen Worten, wenn R⁶ in einer Verbindung für H steht, steht R¹⁰ für einen der anderen hierin beschriebenen Substituenten und umgekehrt.

In einer weiteren bevorzugten Ausführungsform stehen R⁶ und R¹⁰ jeweils für einen Substituenten ausgewählt aus Halogen (bevorzugt Cl, Br oder F), C₁-C₃-alkyl, mit Halogen substituiertes C₁-C₃-alkyl, C₁-C₃-alkoxy oder mit Halogen substituiertes C₁-C₃-alkoxy.

In einer weiteren bevorzugten Ausführungsform stehen R⁶ und R¹⁰ jeweils für Halogen (wie Cl, Br oder F), jeweils für C₁-C₃-alkyl, oder jeweils für mit Halogen substituiertes C₁-C₃-alkyl wie z. B. perfluoriertes C₁-C₃-alkyl (Perfluormethyl, perFluoroethyl oder Perfluorpropyl).

In einer weiteren bevorzugten Ausführungsform steht R⁶ für perfluoriertes C₁-C₃-alkyl (z. B. Perfluormethyl) und R¹⁰ für Cl, Br oder F, besonders bevorzugt für Cl oder Br.

### T46-H

Eine weitere bevorzugte Ausführungsform bezieht sich auf Verbindungen der Formel (Ia") in der R¹ für H steht, T für T46 steht, R¹¹ in T46 für H steht, W für O steht und alle anderen Parameter wie in Paragraph [0078] und Paragraph [0105] definiert sind.

Eine weitere bevorzugte Ausführungsform bezieht sich auf Verbindungen der Formel (Ia") in der R¹ für H steht, T für T46 steht, R¹¹ in T46 für H steht, W für O steht, A₁ für CH, A₂ für CH oder N, A₃ für CR⁴, A₄ für CH, B₁ für CR⁶, B₂ für CH, B₄ für CH, B₅ für CR¹⁰ steht wobei R⁶ und R¹⁰ jeweils für einen Substituenten ausgewählt aus Halogen (bevorzugt Chlor, Brom oder Fluor), C₁-C₃-alkyl, mit Halogen substituiertes C₁-C₃-alkyl, C₁-C₃-alkoxy oder mit Halogen substituiertes C₁-C₃-alkoxy und alle anderen Parameter wie in Paragraph [0078] und Paragraph [0106] ff. definiert sind.

Eine weitere bevorzugte Ausführungsform bezieht sich auf Verbindungen der Formel (Ia") in der R¹ für H steht, T für T46 steht, R¹¹ in T46 für H steht, W für O steht, A₁ für CH, A₂ für CH oder N, A₃ für CR⁴, A₄ für CH, B₁ für CR⁶, B₂ für CH, B₄ für CH, B₅ für CR¹⁰ steht wobei R⁶ und R¹⁰ jeweils für einen Substituenten ausgewählt aus Halogen (bevorzugt Chlor, Brom oder Fluor), C₁-C₃-alkyl, mit Halogen substituiertes C₁-C₃-alkyl, C₁-C₃-alkoxy oder mit Halogen substituiertes C₁-C₃-alkoxy und alle anderen Parameter wie in Paragraph [0078] und Paragraph [0106] ff. definiert sind.

Eine weitere erfindungsgemäße Ausführungsform richtet sich auf Verbindungen der Formel (I-T46): 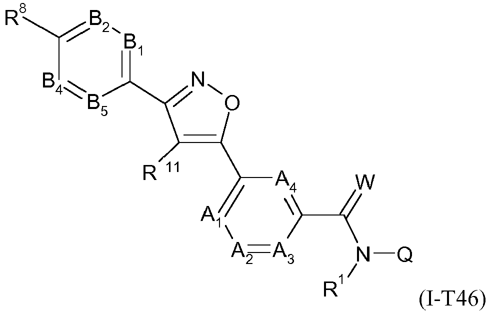 worin
R¹, A₁, A₂, A₃, A₄, R¹¹, B₁, B₂, B₄, B₅, R⁸, R¹¹ Q und W wie hierin beschrieben beschrieben definiert sind.

Eine weitere Ausführungsfrom richtet sich auf Verbindungen der Formel (I-T2), (I-T3), (I-T4), (I-T22), (I-T23) oder (I-T46) worin R¹, A₁, A₂, A₃, A₄, R¹¹, B₁, B₂, B₄, B₅, R⁸, Q und W wie in Paragraph [0113] beschrieben sind.

Eine weitere Ausführungsfrom richtet sich auf Verbindungen der Formel (I-T2), (I-T3), (I-T4), (I-T22), (I-T23) oder (I-T46) worin R¹, A₁, A₂, A₃, A₄, R¹¹, B₁, B₂, B₄, B₅, R⁸, Q und W wie in Paragraph [0114] beschrieben sind.

Eine weitere bevorzugte Ausführungsform richtet sich auf Verbindung D-1a

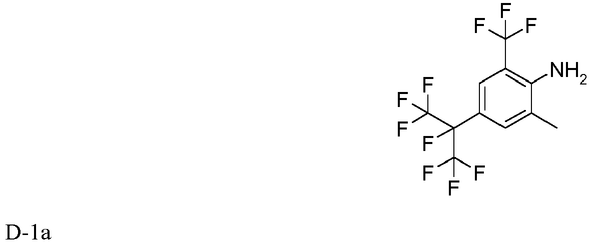

Eine weitere Ausführungsform richtet sich auf die Verwendung der Verbindung D-1a zur Herstellung von Verbindungen der Formel (I).

Eine weitere Ausführungsform richtet sich auf ein Verfahren zur Herstellung einer Verbindung der Formel (I), bevorzugt worin T = T4 ist, umfassend die Verwendung der Verbindung D-1a, bevorzugt in einer Reaktionsabfolge gemäß Reaktionsschema 4.

Eine weitere Ausführungsform richtet sich auf Verbindung D-lb

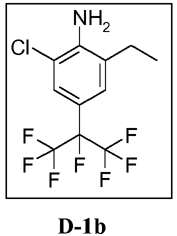

Eine weitere Ausführungsform richtet sich auf die Verwendung der Verbindung D-1b zur Herstellung von Verbindungen der Formel (I).

Eine weitere Ausführungsform richtet sich auf ein Verfahren zur Herstellung einer Verbindung der Formel (I), bevorzugt worin T = T4 ist, umfassend die Verwendung der Verbindung D-1b, bevorzugt in einer Reaktionsabfolge gemäß Reaktionsschema 4.

Eine weitere Ausführungsform richtet sich auf Verbindung D-1c

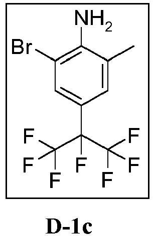

Eine weitere Ausführungsform richtet sich auf die Verwendung der Verbindung D-1c zur Herstellung von Verbindungen der Formel (I).

Eine weitere Ausführungsform richtet sich auf ein Verfahren zur Herstellung einer Verbindung der Formel (I), bevorzugt worin T = T4 ist, umfassend die Verwendung der Verbindung D-1c, bevorzugt in einer Reaktionsabfolge gemäß Reaktionsschema 4.

Eine weitere Ausführungsform richtet sich auf die Verbindung 2-(3,5-Dichlor-4-hydrazino-phenyl)-1,1,1,3,3,3 -hexafluor-propan-2-ol.

Eine weitere Ausführungsform richtet sich auf die Verwendung der Verbindung 2-(3,5-Dichlor-4-hydrazino-phenyl)-1,1,1,3,3,3-hexafluor-propan-2-ol zur Herstellung von Verbindungen der Formel (I).

Eine weitere Ausführungsform richtet sich auf ein Verfahren zur Herstellung einer Verbindung der Formel (I), bevorzugt worin T = T4 ist, umfassend die Verwendung der Verbindung 2-(3,5-Dichlor-4-hydrazino-phenyl)-1,1,1,3,3,3-hexafluor-propan-2-ol, bevorzugt in einer Reaktionsabfolge gemäß Reaktionsschema 4.

### Experimenteller Teil

### Darstellungsverfahren I-T2

### Beispiel I-T2-1

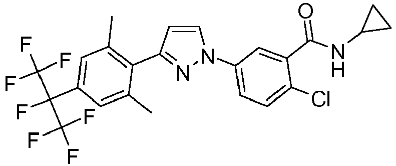

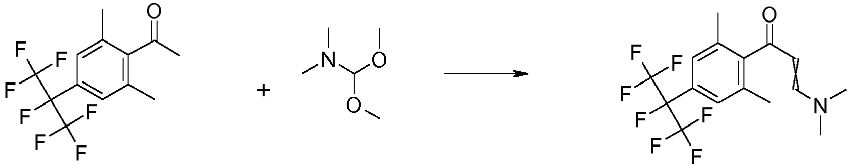

710 mg (2,24 mmol) 1-[2,6-Dimethyl-4-[1,2,2,2-tetrafluor-1-(trifluormethyl)ethyl]phenyl]ethanon wurden zu 401 mg (3,36 mmol) N,N-Dimethylformamiddimethylacetal gegeben und 5 Stunden zum Rückfluss erhitzt. Zur Aufarbeitung wurde etwas abgekühlt und alle flüchtigen Bestandteile am Rotationsverdampfer im Vakuum abgedampft. Der Rückstand wurde über eine Kartusche mit 40 g Kieselgel und einem Gradienten in Cyclohexan/Essigester von 90:10 bis 50:50 (v/v) chromatographiert. Es wurden 675 mg 3-(Dimethylamino)-1-[2,6-dimethyl-4-[1,2,2,2-tetrafluor-1-(trifluormethyl)ethyl]phenyl]prop-2-en-1-on erhalten.

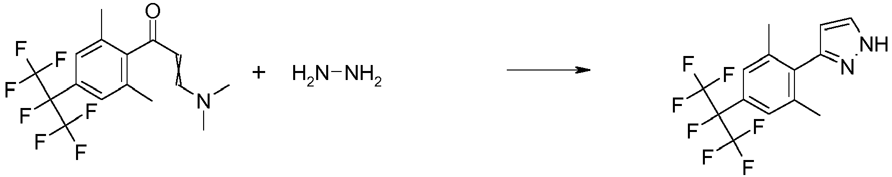

1,2 g (3,23 mmol) 3-(Dimethylamino)-1-[2,6-dimethyl-4-[1,2,2,2-tetrafluor-1-(trifluormethyl)ethyl]phenyl]prop-2-en-1-on wurden zu 15,5 ml Ethanol gegeben, 170 mg (3,39 mmol) Hydrazinhydrat und 192 mg (3,2 mmol) Eisessig zugegeben. Die Mischung wurde 7 Stunden bei Raumtemperatur gerührt. Dann wurden weitere 170 mg (3,39 mmol) Hydrazinhydrat zugefügt und weitere 4 Stunden bei Raumtemperatur gerührt. Da der Umsatz immer noch nicht vollständig war, wurden noch 190 mg (3,2 mmol) Eisessig zugefügt und 17 Stunden bei 60 °C gerührt. Zur Aufarbeitung wurde am Rotationsverdampfer im Vakuum eingedampft und der Rückstand zwischen Essigester und Wasser verteilt. Die organische Phase wurde abgetrennt, mit Wasser gewaschen, mit Natriumsulfat getrocknet und am Rotationsverdampfer im Vakuum eingedampft. Als Rückstand verblieben 1,04 g (3-[2,6-Dimethyl-4-[1,2,2,2-tetrafluor-1-(trifluormethyl)ethyl]phenyl]-1H-pyrazol.

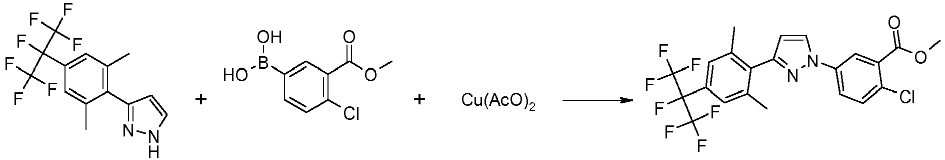

23 ml Dichlormethan, 353 mg (4.46 mmol) Pyridin, 609 mg (3,35 mmol) Kupfer(II)acetat 958 mg (4.46 mmol) 3-Carboxymethyl-4-chlor-phenylboronsäure und 760 mg (2.23 mmol) (3-[2,6-Dimethyl-4-[1,2,2,2-tetrafluor-1-(trifluormethyl)ethyl]phenyl]-1H-pyrazol wurden vorgelegt und dann 1,1 g frisch gemahlenes 3 Å Molsieb zugegeben. Der Ansatz wurde dann 20 Stunden bei Raumtemperatur gerührt. Zur Aufarbeitung wurde über eine Schicht Kieselgur filtriert und mit Dichlormethan nachgewaschen. Das Filtrat wurde am Rotationsverdampfer im Vakuum eingedampft. Zur Reinigung wurde erst über eine Kartusche mit 40 g Kieselgel und einem Gradienten in Cyclohexan/Essigester von 95:5 bis 75:25 (v/v) chromatographiert. Die Produkt enthaltenden Fraktionen wurden eingedampft und über eine zweite Kartusche mit 40 g Kieselgel mit Toluol als Laufmittel chromatographiert. Nach Eindampfen wurden 628 mg 2-Chlor-5-[3-[2,6-dimethyl-4-[1,2,2,2-tetrafluor-1-(trifluoromethyl)ethyl]phenyl]pyrazol-1-yl]benzoesäuremethylester erhalten.

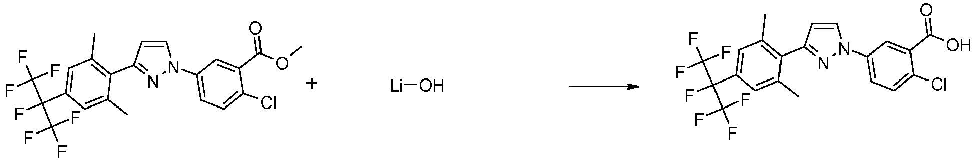

609 mg (1.19 mmol) 2-Chlor-5-[3-[2,6-dimethyl-4-[1,2,2,2-tetrafluor-1-(trifluoromethyl)ethyl]phenyl]pyrazol-1-yl]benzoesäuremethylester wurden in einer Mischung aus 14 ml Dioxan und 5 ml Wasser vorgelegt und 53 mg (1.25 mmol) Lithiumhydroxid Hydrat zugegeben und bei Raumtemperatur gerührt. Nach 2 Stunden wurden weitere 25 mg (0,6 mmol) Lithiumhydroxyd Hydrat nachgegeben und eine weitere Stunde bei Raumtemperatur gerührt. Danach wurden die flüchtigen Bestandteile am Rotationsverdampfer im Vakuum entfernt. Der Rückstand wurde zwischen verdünnter Salzsäure und Dichlormethan verteilt. Die organische Phase wurde abgetrennt und die wässrige Phase noch zweimal mit Dichlormethan extrahiert. Die vereinigten organischen Phasen wurden danach mit gesättigter wässriger Kochsalzlösung gewaschen, mit Natriumsulfat getrocknet und am Rotationsverdampfer im Vakuum eingedampft. Als Rückstand wurden 554 mg 2-Chlor-5-[3-[2,6-dimethyl-4-[1,2,2,2-tetrafluor-1-(trifluoromethyl)ethyl]phenyl]pyrazol-1-yl]benzoesäure erhalten.

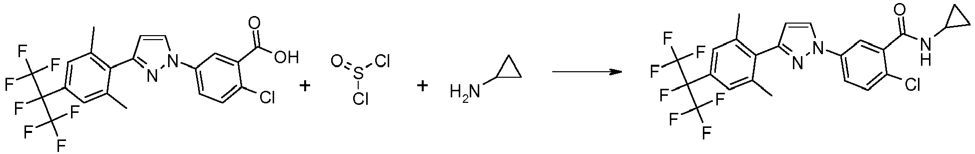

100 mg (0,2 mmol) 2-Chlor-5-[3-[2,6-dimethyl-4-[1,2,2,2-tetrafluor-1-(trifluoromethyl)ethyl]phenyl]pyrazol-1-yl]benzoesäure wurden in 2 ml trockenem Toluol vorgelegt, dann 120 mg (1 mmol) Thionylchlorid (SOC12) und 1 Tropfen Dimethylformamid (DMF) zugegeben und zum Rückfluss erhitzt. Nach dem Ende der Gasentwicklung wurde noch 30 Minuten unter Rückfluss nachgerührt und dann im Vakuum im Rotationsverdampfer eingedampft. Der Rückstand wurde in 1 ml trockenem Dichlormethan gelöst und zu einer Lösung von 29 mg (0,5 mmol) Cyclopropylamin in 1 ml Dichlormethan bei 0 °C zugetropft. Anschließend wurde 2 Stunden bei Raumtemperatur nachgerührt. Zur Aufarbeitung wurde auf 5 %ige wässrige Natriumhydrogencarbonat-Lösung gegossen, die organische Phase abgetrennt, mit gesättigter wässriger Kochsalzlösung gewaschen, mit Natriumsulfat getrocknet und am Rotationsverdampfer im Vakuum eingedampft. Zur Reinigung wurde über eine Kartusche mit 40 g Kieselgel und einem Gradienten in Cyclohexan/Essigester von 90:10 bis 50:50 (v/v) chromatographiert. Es wurden 159,5 mg 2-Chlor-N-cyclopropyl-5-[3-[2,6-dimethyl-4-[1,2,2,2-tetrafluor-1-(trifluoromethyl)ethyl]phenyl]pyrazol-1-yl]benzamid (Verbindung **I-T2-1**) erhalten.
HPLC-MS^{a)}: logP = 4,9, Masse (m/z) = 534 [M+H]+.
H-NMR (400 MHz, d₃-Acetonitril): δ (ppm) = 8.29 (d, J=2,5 Hz, 1 H), 7,82-7,85 (m, 2H), 7,52 (d, J=8.8 Hz, 1 H), 7,44 (s, 2 H), 6,97 (s (breit), 1 H (N-H)), 6,54 (d, J=2.5 Hz, 1 H), 2,82-2,86 (m, 1H), 0,74-0,79 (m, 2 H), 0,59-0,61 (m, 2 H).

### Herstellung der Ausgangsverbindungen:

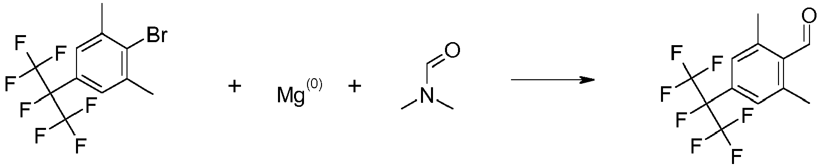

271mg (11,1 mg-Atom) Magnesiumspäne wurden vorgelegt, mit etwas trockenem Tetrahydrofuran bedeckt und, nach Zugabe einiger Tropfen einer Lösung von 3 g (8,49 mmol) 2-Brom-1,3-dimethyl-5-[1,2,2,2-tetrafluor-1-(trifluormethyl)ethyl]benzol (hergestellt nach US2003/187233, S. 6, Example 2/4 [0080]) in 10 ml trockenem Tetrahydrofuran, ein Krümel Iod zugefügt. Zum Anstarten wurde die Mischung auf 60 °C erhitzt. Nach dem Anspringen der Reaktion wurde dann der Rest der das 2-Brom-1,3-dimethyl-5-[1,2,2,2-tetrafluor-1-(trifluormethyl)ethyl]benzol enthaltenden Lösung bei 60 °C zugetropft. Die Mischung wurde nach beendeter Zugabe noch eine Stunde bei 60 °C gerührt. Danach wurde mit einem Eisbad auf 0 °C gekühlt und 1,86 g (25,4 mmol) N,N-Dimethylformamid, gelöst in 5 ml trockenem Tetrahydrofuran, zugetropft. Es wurde dann ohne Kühlung gerührt bis die Mischung Raumtemperatur erreicht hatte. Zur Aufarbeitung wurde auf gesättigte wässrige Ammoniumchlorid-Lösung gegossen. Die Phasen wurden getrennt, die wässrige Phase mit Essigester extrahiert. Die vereinigten organischen Phasen wurden mit gesättigter wässriger Kochsalzlösung gewaschen, mit Natriumsulfat getrocknet und am Rotationsverdampfer im Vakuum eingedampft. Als Rückstand verblieben 2,34 g 2,6-Dimethyl-4-[1,2,2,2-tetrafluor-1-(trifluormethyl)ethyl]benzaldehyd, der ohne Reinigung in der nächsten Stufe verwendet wurde.

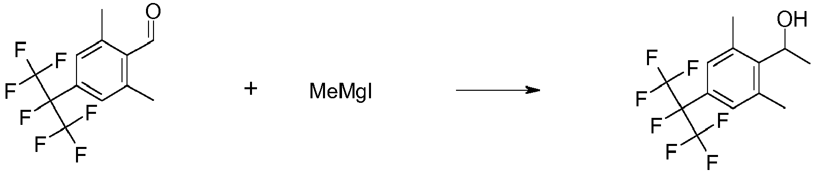

2,34 g (7,74 mmol) 2,6-Dimethyl-4-[1,2,2,2-tetrafluor-1-(trifluormethyl)ethyl]benzaldehyd wurden in 15,5 ml trockenem Tetrahydrofuran vorgelegt und 2,58 ml (7,74 mmol) einer 3 M Lösung von Methylmagnesiumiodid in Diethylether unter Kühlung mit einem Eisbad zugetropft. Anschließend wurde eine Stunde ohne Kühlung weitergerührt. Zur Aufarbeitung wurde die Mischung auf 100 ml gesättigte wässrige Ammoniumchlorid-Lösung gegossen. Es wurde zweimal mit Essigester extrahiert. Die vereinigten organischen Phasen wurden mit gesättigter wässriger Kochsalzlösung gewaschen, mit Natriumsulfat getrocknet und am Rotationsverdampfer im Vakuum eingedampft. Der Rückstand wurde über eine 40 g Kartusche mit Kieselgel mit einem Gradienten in Cylohexan/Essigester von 90:10 bis 70:30 (v/v) chromatographiert und ergab 1,0 g 1-[2,6-Dimethyl-4-[1,2,2,2-tetrafluor-l-(trifluormethyl)ethyl]phenyl]ethanol.

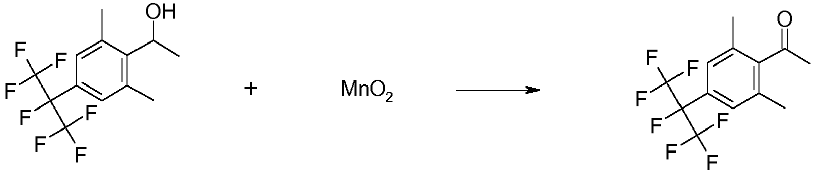

1,49 g (4,68 mmol) 1-[2,6-Dimethyl-4-[1,2,2,2-tetrafluor-1-(trifluormethyl)ethyl]phenyl]ethanol wurden in 84 ml Toluol vorgelegt und 10,8 g (124 mmol) Mangan(IV)oxid zugegeben. Die Mischung wurde eine Stunde unter Rühren zum Rückfluss erhitzt. Danach wurde abgekühlt, über eine Schicht Kieselgur filtriert und mit Essigester nachgewaschen. Das Filtrat wurde am Rotationsverdampfer im Vakuum eingedampft. Der Rückstand wurde über eine Kartusche mit 50 g Kieselgel mit einem Gradienten in Cyclohexan/Essigester von 95:5 bis 70:30 (v/v) chromatographiert. Es wurden 1,03 g 1-[2,6-Dimethyl-4-[1,2,2,2-tetrafluor-1-(trifluormethyl)ethyl]phenyl]ethanon erhalten.

### Darstellungsverfahren I-T3

### (Nur Beispiele, welche unter die Ansprüche fallen, sind erfindungsgemäss).

### Beispiel I-T3-1:

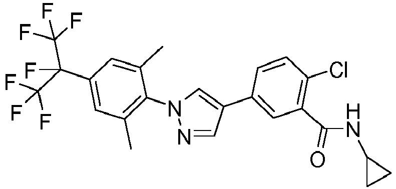

Die Herstellung der Vorstufe [2,6-Dimethyl-4-[1,2,2,2-tetrafluor-1-(trifluormethyl)ethyl]phenyl]hydrazin ist in der Literatur beschrieben (US 2003/187233).

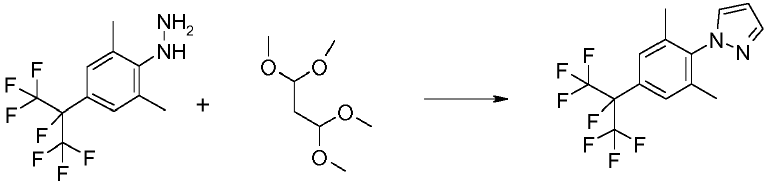

In einem 25 ml Kolben wurden 3,41 g (11,2 mmol) [2,6-Dimethyl-4-[1,2,2,2-tetrafluor-1-(trifluormethyl)ethyl]phenyl]hydrazin (freie Base) in 13 ml Ethanol vorgelegt. Dann wurden 1,84 g (11,2 mmol) Tetramethoxypropan und anschliessend 0,55 g (5,6 mmol) 96 %ige Schwefelsäure zugegeben. Der Ansatz wurde 2 Stunden zum Rückfluss erhitzt. Ethanol wurde an einem Rotationsverdampfer im Vakuum abgedampft. Der Rückstand wurde zwischen Essigester und gesättigter wässriger Natriumhydrogencarbonat-Lösung verteilt. Die organische Phase wurde abgetrennt, mit Natriumsulfat getrocknet und am Rotationsverdampfer im Vakuum eingedampft. Der Rückstand wurde im Vakuum bei 1 mbar und 150 °C im Kugelrohr destilliert und ergab 2,5 g an 1-[2,6-Dimethyl-4-[1,2,2,2-tetrafluor-1-(trifluormethyl)ethyl]phenyl]pyrazol.

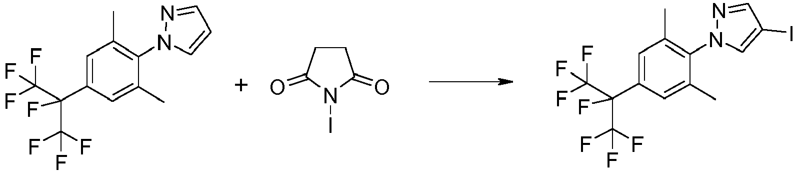

In einem 250 ml Kolben wurden 2,5 g (7,34 mmol) 1-[2,6-Dimethyl-4-[1,2,2,2-tetrafluor-1-(trifluormethyl)ethyl]phenyl]pyrazol in 30 ml Acetonitril vorgelegt und 8,3 g (36,9 mmol) N-Jodsuccinimid in 50 ml Acetonitril zugetropft. Anschliessend wurde zum Rückfluss erhitzt. Zur Aufarbeitung wurde eingedampft, der Rückstand zwischen Wasser und Essigester verteilt. Die organische Phase wurde abgetrennt, erst mit gesättigter wässriger Natriumhydrogensulfit-Lösung, dann mit gesättigter Kochsalzlösung gewaschen, mit Natriumsulfat getrocknet und eingedampft. Der Rückstand wurde durch Chromatographie mit Kieselgel mittels eines Gradienten von 90:10 bis 70:30 (v/v) in Cyclohexan/Essigester gereinigt. Nach dem Eindampfen der das Produkt enthaltenden Fraktionen wurden 2,5 g eines Rückstands erhalten der aus 1-[2,6-Dimethyl-4-[1,2,2,2-tetrafluor-1-(trifluormethyl)ethyl]phenyl]-4-iod-pyrazol und etwas Toluol bestand.

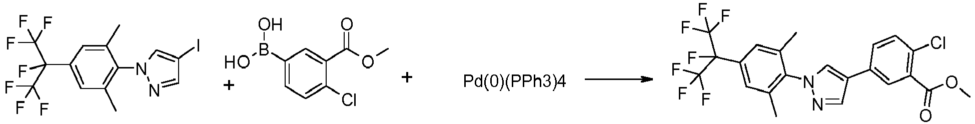

In einem 100 ml Kolben wurden 280 mg (0,6 mmol) 1-[2,6-Dimethyl-4-[1,2,2,2-tetrafluor-1-(trifluormethyl)ethyl]phenyl]-4-iod-pyrazol und 0,129 g (0,60 mmol) [4-Chlor-3-(methoxycarbonyl)phenyl]boronsäure in 21 ml Isopropanol vorgelegt und zuletzt 1,84 ml (1,84 mmol) entgaste 1 molare Natriumhydrogencarbonat-Lösung zugegeben. Es wurden 0,035 g (0,03 mmol) Tetrakis(triphenylphosphin)palladium(0) zugefügt. Danach wurde zum Rückfluss erwärmt. Zur Aufarbeitung wurde am Rotationsverdampfer abgedampft und der Rückstand zwischen Wasser und Essigester verteilt. Die organische Phase wurde abgetrennt, einmal mit gesättigter Kochsalzlösung gewaschen und am Rotationsverdampfer im Vakuum eingedampft. Der Rückstand wurde durch Chromatographie mit Kieselgel mittels eines Gradienten von 90:10 bis 70:30 (v/v) in Cyclohexan/Essigester gereinigt und ergab 151 mg an 2-Chlor-5-[1-[2,6-dimethyl-4-[1,2,2,2-tetrafluor-1-(trifluormethyl)ethyl]phenyl]pyrazol-4-yl]benzoesäuremethylester.

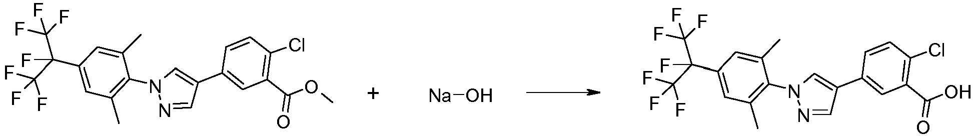

0,151 g (0,29 mmol) 2-Chlor-5-[1-[2,6-dimethyl-4-[1,2,2,2-tetrafluor-1-(trifluormethyl)ethyl]phenyl]pyrazol-4-yl]benzoesäuremethylester wurden in 11 ml Methanol vorgelegt und 0,3 ml (0,3 mmol) 1M Natronlauge zugegeben. Anschliessend wurde 6 Stunden zum Rückfluss erhitzt, überschüssiges Lösungsmittel im Vakuum eingedampft, der Rückstand mit verdünnter Salzsäure aufgenommen und dreimal mit Essigester extrahiert. Die vereinigten Extrakte wurden mit gesättigter Kochsalzlösung gewaschen, mit Natriumsulfat getrocknet, eingedampft und ergaben 130 mg an 2-Chlor-5-[1-[2,6-dimethyl-4-[1,2,2,2-tetrafluor-1-(trifluormethyl)ethyl]phenyl]pyrazol-4-yl]benzoesäure.

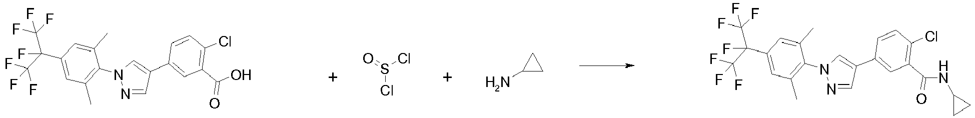

0,134 g (0,27 mmol) 2-Chlor-5-[1-[2,6-dimethyl-4-[1,2,2,2-tetrafluor-1-(trifluormethyl)ethyl]phenyl]pyrazol-4-yl]benzoesäure wurden in 1,26 ml Toluol gelöst und 0,161 g (1,35 mmol) Thionylchlorid zugegeben. Die Mischung wurde 2 Stunden auf 80 °C erhitzt. Danach wurde im Vakuum eingedampft. Der Rückstand wurde in 1,26 ml Dichlormethan gelöst und zu einer Lösung von 39 mg (0,67 mmol) Cyclopropylamin in 0,63 ml Dichlormethan unter Kühlung bei 0°C zugetropft und die Lösung gekühlt. Zur Aufarbeitung wurde 5 %ige wässrige Natriumdihydrogenphosphat-Lösung zugefügt, die organische Phase abgetrennt, mit Natriumsulfat getrocknet und im Vakuum am Rotationsverdampfer eingedampft. Der Rückstand wurde mit Kieselgel mit einem Gradienten von Cyclohexan/Essigester 9:1 bis 7:3 (v/v) getrennt und ergab 46 mg 2-Chlor-N-cyclopropyl-5-[1-[2,6-dimethyl-4-[1,2,2,2-tetrafluor-1-(trifluormethyl)ethyl]phenyl]pyrazol-4-yl]benzamid (Verbindung **I-T3-1**).
HPLC-MS^{a)}: logP = 4,36, Masse (m/z) = 534 [M+H]+.
¹H-NMR (400 MHz, d₃-Acetonitril): δ = 8,11 (s, 1 H), 8,06 (s, 1 H), 7,68 (d, J1=2,2 Hz, 1 H), 7,62-7,65 (dd, J1=8,4 Hz, J2=2,2 Hz, 1H), 7,45 (d, J=8,4 Hz, 1 H), 6,9 (s (breit), 1 H (N-H)), 3,97 (s, 3 H), 2,82-2,88 (m, 1H), 0,76-0,8 (m, 2 H), 0,57-0,61 (m, 2 H).

### Beispiele I-T3-48 und IT-T3-50

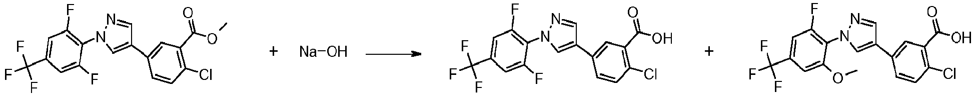

2,46 g (5,9 mmol) Methyl-2-chlor-5-[1-[2,6-difluor-4-(trifluormethyl)phenyl]pyrazol-4-yl]benzoat wurden in 127 ml Methanol vorgelegt und 5,9 mg (5,9 mmol) 1 molare Natronlauge zugegeben. Die Mischung wurde 2 Stunden zum Rückfluss erhitzt. Danach wurde abgekühlt und der grösste Teil des Methanols am Rotationsverdampfer im Vakuum entfernt. Der wässrige Rückstand wurde mit Dichlormethan extrahiert. Der Extrakt wurde verworfen. Die wässrige Phase wurde mit 33 %iger Salzsäure auf pH 1 gestellt und zweimal mit Dichlormethan extrahiert. Die vereinigten Extrakte wurden mit gesättigter wässriger Kochsalzlösung gewaschen, mit Natriumsulfat getrocknet und eingedampft. Es wurden 1,41 g Rückstand als ein 45:55 (LC/MS-Flächen) Gemisch von 2-Chlor-5-[1-[2,6-difluor-4-(trifluormethyl)phenyl]pyrazol-4-yl]benzoesäure und 2-Chlor-5-[1-[2-fluor-6-methoxy-4-(trifluormethyl)phenyl]pyrazol-4-yl]benzoesäure erhalten.

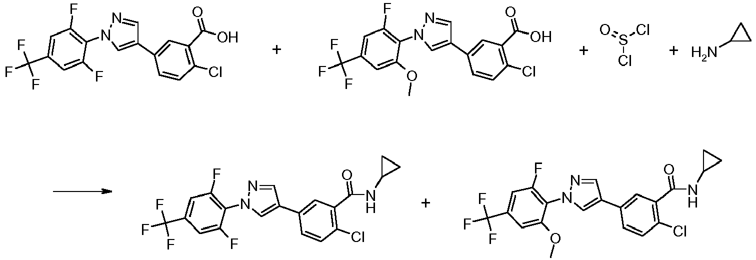

700 mg (ca. 1,7 mmol) einer 45:55 (LC/MS-Flächen) Mischung von 2-Chlor-5-[1-[2,6-difluor-4-(trifluormethyl)phenyl]pyrazol-4-yl]benzoesäure und 2-Chlor-5-[1-[2-fluor-6-methoxy-4-(trifluormethyl)phenyl]pyrazol-4-yl]benzoesäure wurden in 6,6 ml Toluol gelöst und 1,34 g (8,7 mmol) Thionylchlorid zugegeben. Die Mischung wurde 2 Stunden auf 80 °C erhitzt. Danach wurden am Rotationsverdampfer im Vakuum alle flüchtigen Bestandteile abgezogen. Der Rückstand wurde in 3,3 ml Dichlormethan gelöst und zu einer Lösung von 248 mg (4,34 mmol) Cyclopropylamin in 3,3 ml Dichlormethan bei 0 °C zugetropft. Die Mischung wurde dann 2 Stunden ohne Kühlung gerührt. Danach wurde die Lösung mit 5%iger wässriger Natriumdihydrogenphosphat-Lösung gewaschen, mit Natriumsulfat getrocknet und eingedampft. Der Rückstand wurde über eine Kartusche mit 40 g Kieselgel und einem Gradienten in Cyclohexan/Essigester von 90:10 bis 50:50 (v/v) chromatographiert. Es wurden 240 mg 2-Chlor-N-cyclopropyl-5-[1-[2,6-difluor-4-(trifluormethyl)phenyl]pyrazol-4-yl]benzamid (Beispiel **I-T3-48)**
HPLC-MS^{a)}: logP = 3,2, Masse (m/z) = 442 [M+H]+.
¹H-NMR (400 MHz, d₃-Acetonitril): δ (ppm) = 8,26 (s, 1 H), 8,19 (s, 1H), 7,61-7,69 (m, 4H), 7,46 (d, J = 8,3 Hz, 1H), 6,94 (s, 1 H (breit)), 2,82-2,88 (m, 1H), 0,75-0,80 (m, 2 H), 0,58-0,62 (m, 2 H).
und 2-Chlor-N-cyclopropyl-5-[1-[2-fluor-6-methoxy-4-(trifluormethyl)phenyl]pyrazol-4-yl]benzamid (Beispiel **I-T3-50**) erhalten.
HPLC-MS^{a)}: logP = 3,1, Masse (m/z) = 454 [M+H]+.
¹H-NMR (400 MHz, d₃-Acetonitril): δ (ppm) = 8,13 (s, 1 H), 8,11 (s, 1H), 7,67 (d, J = 2,2 Hz, 1 H), 7,62 (dd, J₁ = 8,3 Hz, J₂ = 2,2 Hz, 1 H), 7,45 (d, J = 8,3 Hz, 1H), 7,32 (s, 1 H), 7,30 (s, 1 H), 6,91 (s, 1 H (breit)), 3,90 (s, 3 H), 2,83-2,87 (m, 1H), 0,75-0,79 (m, 2 H), 0,57-0,61 (m, 2 H).

### Beispiel I-T3-121:

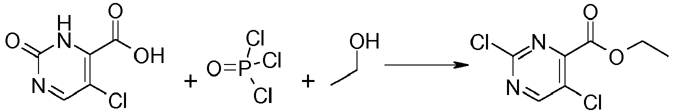

4,6 ml (49,6 mmol) Phosphoroxychlorid wurden vorgelegt und 1,3 g (7,44 mmol) 5-Chlor-2-oxo-1H-pyrimidin-6-carbonsäure (kommerziell erhältlich bzw. kann nach literaturbekannten Vorschriften hergestellt werden (z.B. Gacek, Michel; Ongstad, Leif; Undheim, Kjell; Acta Chemica Scandinavica, Series B: Organic Chemistry and Biochemistry B33(2), (1979), S.150-1)) eingetragen. Es wurde langsam hochgeheizt und 2 Stunden unter Rückfluss gehalten. Danach wurde etwas abgekühlt und das überschüssige Phosphoroxychlorid im Vakuum am Rotationsverdampfer abgezogen. Zum Rückstand wurden 20 ml trockenes Ethanol zugegeben und anschliessend über Nacht bei Raumtemperatur gerührt. Danach wurde überschüssiges Ethanol im Vakuum am Rotationsverdampfer abgezogen. Der Rückstand wurde in Dichlormethan aufgenommen und dreimal mit gesättigter wässriger Natriumhydrogencarbonatlösung gewaschen. Die wässrigen Phasen wurden mit Dichlormethan rückextrahiert, die vereinigten organischen Phasen dann mit gesättigter wässriger Kochsalzlösung gewaschen, mit Natriumsulfat getrocknet und im Vakuum am Rotationsverdampfer eingedampft. Der Rückstand wurde über eine Kartusche mit 15 g Kieselgel mit einem Gradienten von reinem Cyclohexan bis Cyclohexan/Ethylacetat 50:50 (v/v) chromatographiert und ergab 115 mg 2,5-Dichloropyrimidin-4-carbonsäureethylester.

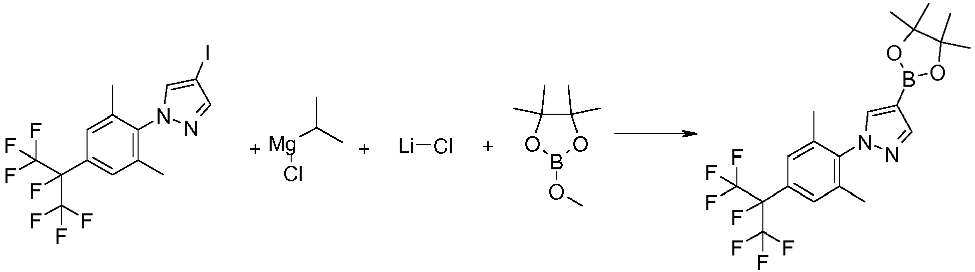

In einem ausgeheizten 25 ml Dreihalskolben wurden 5,94 ml (7,72 mmol) einer 1,3 molaren Lösung von i-Propyl-magnesium-chlorid/Lithiumchlorid Komplex vorgelegt und eine Lösung von 4-Iod-1-[2-methyl-4-[1,2,2,2-tetrafluor-1-(trifluormethyl)ethyl]-6-(trifluormethyl)phenyl]pyrazole (Herstellung siehe Beispiel **I-T3-1**) in 3,4 ml trockenem Tetrahydrofuran zugetropft. Es wurde über Nacht bei Raumtemperatur nachgerührt, dann auf -20 °C gekühlt und 1,63 g (10,2 mmol) 2-Methoxy-4,4,5,5-tetramethyl-1,3,2-dioxaborolan zugetropft. Die Mischung wurde noch 1 Stunde bei 0 - 10 °C nachgerührt. Zur Aufarbeitung wurde auf 30 ml gesättigte wässrige Ammoniumchloridlösung gegossen und mit Cyclohexan verdünnt. Die Phasen wurden getrennt, die wässrige Phase mit Cyclohexan nachextrahiert. Die vereinigten organischen Phasen wurden erst mit gesättigter wässriger Natriumhydrogencarbonatlösung und dann mit gesättigter wässriger Kochsalzlösung gewaschen, mit Natriumsulfat getrocknet und im Vakuum am Rotationsverdampfer eingedampft. Nach Chromatographie über eine 40 g Kartusche mit Kieselgel mit einem Gradienten ausgehend von reinem Cyclohexan bis nach Cyclohexan/Essigester 80:20 (v/v) wurden 0,6 g 1-[2,6-Dimethyl-4-[1,2,2,2-tetrafluor-1-(trifluormethyl)ethyl]phenyl]-4-(4,4,5,5-tetramethyl-1,3,2-dioxaborolan-2-yl)pyrazol erhalten.

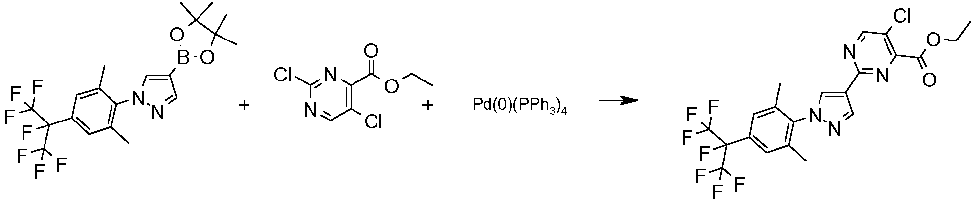

155 mg (0,7 mmol) 2,5-Dichloropyrimidin-4-carbonsäureethylester und 327 mg (0,7 mmol) 1-[2,6-Dimethyl-4-[1,2,2,2-tetrafluor-1-(trifluormethyl)ethyl]phenyl]-4-(4,4,5,5-tetramethyl-1,3,2-dioxaborolan-2-yl)pyrazol wurden in 25 ml Dioxan vorgelegt und 234 mg (2,2 mmol) Natriumcarbonat und 1,25 ml Wasser wurden zugegeben. Den Ansatz wurde mit Argon entgast und dann 81 mg (0,07 mmol) Tetrakis(triphenylphosphin)palladium(0) zugegeben. Es wurde nochmals mit Argon entgast und über Nacht bei 100°C gerührt. Am nächsten Morgen wurde abgekühlt und das Lösungsmittel im Vakuum am Rotationsverdampfer abgezogen. Der Rückstand wurde zwischen Wasser und Ethylacetat verteilt. Die organische Phase wurde abgetrennt, einmal mit gesättigter wässriger Kochsalzlösung gewaschen und dann im Vakuum am Rotationsverdampfer eingedampft. Zur Reinigung wurde über eine Kartusche mit 15 g Kieselgel und einem Gradienten ausgehend von reinem Cyclohexan bis zu einer Mischung von Cyclohexan/Ethylacetat 70:30 (v/v) chromatographiert. Es wurden 120 mg 5-Chlor-2-[1-[2,6-dimethyl-4-[1,2,2,2-tetrafluor-1-(trifluormethyl)ethyl]phenyl]pyrazol-4-yl]pyrimidin-4-carbonsäureethylester erhalten.

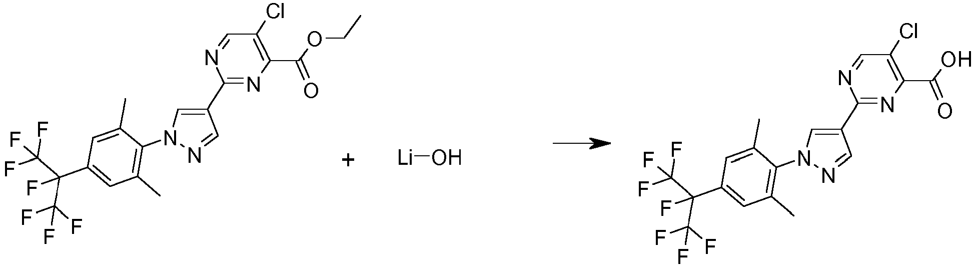

0,120 g (0,23 mmol) 5-Chlor-2-[1-[2,6-dimethyl-4-[1,2,2,2-tetrafluor-1-(trifluormethyl)ethyl]phenyl]pyrazol-4-yl]pyrimidin-4-carbonsäureethylester wurden in einer Mischung aus 4,1 ml Dioxan und 1,44 ml Wasseer vorgelegt und 31 mg (0,74 mmol) Lithiumhydroxid Monohydrat zugegeben. Anschliessend wurde 4 Stunden bei Raumtemperatur gerührt, danach überschüssiges Lösungsmittel im Vakuum abgedampft, der Rückstand mit verdünnter Salzsäure aufgenommen und dreimal mit Dichlormethan extrahiert. Die vereinigten Extrakte wurden mit gesättigter wässriger Kochsalzlösung gewaschen, mit Natriumsulfat getrocknet, eingedampft und ergaben 115 mg an roher 5-Chlor-2-[1-[2,6-dimethyl-4-[1,2,2,2-tetrafluor-1-(trifluormethyl)ethyl]phenyl]pyrazol-4-yl]pyrimidin-4-carbonsäure.

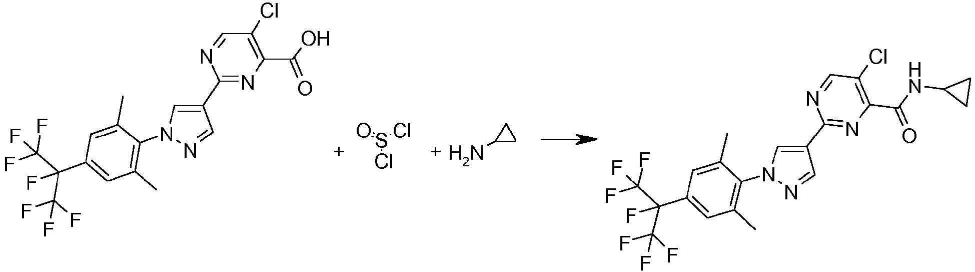

0,110 g (0,22 mmol) rohe 5-Chlor-2-[1-[2,6-dimethyl-4-[1,2,2,2-tetrafluor-1-(trifluormethyl)ethyl]phenyl]pyrazol-4-yl]pyrimidin-4-carbonsäure wurden in 2 ml Toluol gelöst, 0,132 g (1,1 mmol) Thionylchlorid zugegeben und ein kleiner Tropfen Dimethylformamid zugefügt. Die Mischung wurde 2 Stunden auf 80 °C erhitzt. Danach wurde im Vakuum eingedampft. Der Rückstand wurde in 1 ml Dichlormethan gelöst und zu einer Lösung von 32 mg (0,55 mmol) Cyclopropylamin in 1 ml Dichlormethan unter Kühlung bei 0°C zugetropft und anschliessend 2 Stunden ohne Kühlung gerührt. Zur Aufarbeitung wurde 5 %ige wässrige Natriumdihydrogenphosphat-Lösung zugefügt, die organische Phase abgetrennt, mit Natriumsulfat getrocknet und im Vakuum am Rotationsverdampfer eingedampft. Der Rückstand wurde über eine Kartusche mit 15 g Kieselgel mit einem Gradienten von Cyclohexan/Essigester 9:1 bis 7:3 (v/v) getrennt und ergab 49 mg 5-Chlor-N-cyclopropyl-2-[1-[2,6-dimethyl-4-[1,2,2,2-tetrafluor-1-(trifluormethyl)ethyl]phenyl]pyrazol-4-yl]pyrimidin-4-carboxamid (Verbindung **I-T3-121).**
HPLC-MS^{a)}: logP = 4,5, Masse (m/z) = 536 [M+H]+.
¹H-NMR (400 MHz, d₃-Acetonitril): δ (ppm) = 8,84 (s, 1 H), 8,46 (s, 1 H), 8,44 (s, 1H), 7,87 (s, 1 H (breit)), 7,55 (s, 2 H), 2,84-2,91 (m, 1 H), 2,2 (s, 6 H), 0,79-0,83 (m, 2 H), 0,64-0,68 (m, 2 H).

### Beispiel I-T3-134:

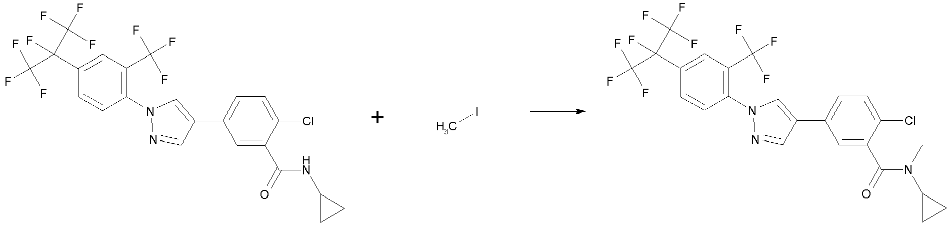

Zu einem auf 0°C gekühlten Gemisch von 6.5 mg (0.163 mmol) Natriumhydrid (60% in Mineralöl) in 2 ml trockenem Tetrahydrofuran wurden 49.3 mg (0,08 mmol) 2-Chlor-N-cyclopropyl-5-{1-[4-(1,1,1,2,3,3,3-heptafluorpropan-2-yl)-2-(trifluormethyl)phenyl]-1H-pyrazol-4-yl}benzamid gegeben. Nach 30 Minuten wurden 35 mg (0.24 mmol) Methyliodid zugegeben und das Gemisch für 1 Stunde bei 0°C und gerührt, anschließend über 1 Stunde auf Raumtemperatur erwärmt und für weitere 14 Stunden bei Raumtemperatur gerührt. Danach wurde das Gemisch auf Wasser gegeben, mit Essigester extrahiert, die organische Phase über Natriumsulfat getrocknet und das Lösungsmittel unter vermindertem Druck entfernt. Der Rückstand wurde durch Chromatographie an Reversed-Phase-Kieselgel (C18) mit Wasser/Acetonitril (Gradient) als Laufmittel gereinigt. Es wurden 40.0 mg (0.068 mmol, 78%) 2-Chlor-N-cyclopropyl-5-[5-[2,6-dichlor-4-[1,2,2,2-tetrafluor-1-(trifluormethyl)ethyl]phenyl]isoxazol-3-yl]benzamid (Verbindung **I-T3-134)** erhalten.
HPLC-MS^{a)}: logP = 4.88, Masse (m/z) = 588 [M+H]+.
¹H-NMR (400 MHz, d₆-DMSO): δ (ppm) = 8,82 (s, 1 H), 8,43 (s, 1 H), 8,25 (d, 1H), 8,11 (d, 1 H), 8,06 (d, 1 H), 7,81 (d, 1 H), 7,75 (m, 1 H), 7,54 (d, 1 H), 3,02 (s, 3 H), 2,72 (m, 1 H), 0,55 (m, 2 H), 0,46 (m, 2 H).

### Beispiel I-T3-156:

### 2-Chlor-N-cyclopropyl-5-{1-[3-(ethylsulfanyl)-5-(1,1,1,2,3,3,3-heptafluorpropan-2-yl)pyridin-2-yl]-1H-pyrazol-4-yl}benzamid

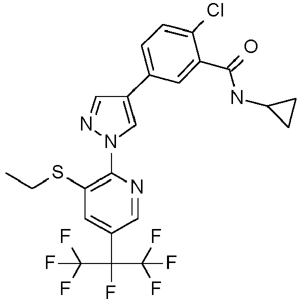

### 2-(4-Brom-1H-pyrazol-1-yl)-3-chlor-5-(1,1,1,2,3,3,3-heptafluorpropan-2-yl)pyridin

1,0g (3,16 mmol) 2,3-Dichlor-5-(1,1,1,2,3,3,3-heptafluorpropan-2-yl)pyridin wurden zu einer Suspension von 0,51 g (3,48 mmol) 4-Brom-1H-pyrazol und 2,58 g (7,91 mmol) Cäsiumcarbonat in 10,0 mL Dimethylformamid p.a. getropft. Die Reaktion wurde 3h bei Raumtemperatur gerührt. Das Reaktionsgemisch wurde dann mit Ethylacetat verdünnt und dann mit halbgesättigter wässriger Ammoniumchlorid Lösung gewaschen. Die wässrige Phase wurde dann mehrfach mit Ethylacetat extrahiert und die vereinigten organischen Phasen anschließend mit dest. Wasser und gesättigter Natriumchlorid Lösung gewaschen. Die organische Phase wurde über Magnesiumsulfat getrocknet, filtriert und am Rotationsverdampfer unter vermindertem Druck eingeengt. Das Rohprodukt wurde an Kieselgel säulenchromatographisch aufgereinigt.

Man erhält 1,34 g (3,14 mmol) 2-(4-Brom-1*H*-pyrazol-1-yl)-3-chlor-5-(1,1,1,2,3,3,3-heptafluorpropan-2-yl)pyridin als farbloses Öl.
HPLC-MS^{a)}: logP = 4,74, Masse (m/z) = 428 [M+H]⁺.
¹H-NMR (400 MHz, D6-DMSO): 8,90 (s,1H), 8,67 (s,1H), 8,63 (d,1H), 8,06 (s,1H).

### 2-Chlor-5-{1-[3-chlor-5-(1,1,1,2,3,3,3-heptafluorpropan-2-yl)pyridin-2-yl]-1H-pyrazol-4-yl}-N-cyclopropylbenzamid

150 mg (0,35 mmol) 2-(4-Brom-1*H*-pyrazol-1-yl)-3-chlor-5-(1,1,1,2,3,3,3-heptafluorpropan-2-yl)pyridine, 136 mg (0,42 mmol) 2-Chlor-*N*-cyclopropyl-5-(4,4,5,5-tetramethyl-1,3,2-dioxaborolan-2-yl)benzamid, 59 mg (0,70 mmol) Natriumhydrogencarbonat und 20 mg Tetrakis(triphenylphosphin)palladium (0,01 mmol) wurden in einer Mischung aus 1,5 mL Dioxan und 0,5 mL dest. Wasser gelöst. Die Lösungsmittel wurden vor der Verwendung ca. 30 Minuten mit Argon gesättigt in dem durch die Lösungsmittel Argongas durchgeleitet wurde. Das Reaktionsgemisch wurde 16 Stunden im Ölbad auf 100°C erhitzt. Nach dem das Reaktionsgemisch auf Raumtemperatur abgekühlt wurde, wurde das Gemisch mit Wasser versetzt und das Rohprodukt mehrfach mit Ethylacetat extrahiert. Die vereinigten org. Phasen wurden über Magnesiumsulfat getrocknet und über Kieselgel filtriert. Die Lösungsmittel wurden am Rotationsverdampfer unter vermindertem Druck entfernt. Das Rohprodukt wurde an Kiesegel säulenchromatographisch aufgereinigt.

Man erhielt 25 mg (0,05 mmol) 2-Chlor-5-{1-[3-chlor-5-(1,1,1,2,3,3,3-heptafluorpropan-2-yl)pyridin-2-yl]-1*H*-pyrazol-4-yl}-*N*-cyclopropylbenzamid als farblosen Feststoff.
HPLC-MS^{a)}: logP = 4,08, Masse (m/z) = 541 [M+H]⁺.
1H-NMR (400 MHz, D6-DMSO): 9,02(s,1H), 8,89(s,1H), 8,61(d,1H), 8,54-8,52(m,1H), 8,50(s,1H), 7,83-7,81(m,2H), 7,52(d,1H), 2,87-2,81(m,1H), 0,74-0,65(m,2H), 0,60-0,50(m,2H)

### 2-Chlor-N-cyclopropyl-5-{1-[3-(ethylsulfanyl)-5-(1,1,1,2,3,3,3-heptafluorpropan-2-yl)pyridin-2-yl]-1H-pyrazol-4-yl}benzamid

300 mg (0,55 mmol) 2-Chlor-5-{1-[3-chlor-5-(1,1,1,2,3,3,3-heptafluorpropan-2-yl)pyridin-2-yl]-1*H*-pyrazol-4-yl}-*N-*cyclopropylbenzamid wurden in 5,0 mL DMF abs. gelöst und mit einem Trockeneis/Acetonbad gekühlt. Zu der gekühlten Reaktionsmischung wurde eine Lösung von 81,6 mg (0,97 mmol) Natriumethanthiolat in 5 mL DMF abs. getropft. Nach 3 Stunden wurde die Reaktion auf Raumtemperatur erwärmt und vorsichtig auf Wasser gegossen. Das Rohprodukt wurde mehrfach mit Ethylacetat extrahiert. Die vereinigten organischen Phasen wurden mit gesättigter wässriger Natriumchlorid Lösung gewaschen, über Magnesiumsulfat getrocknet, filtriert und am Rotationsverdampfer unter vermindertem Druck eingeengt. Das Rohprodukt wurde an Kiesegel säulenchromatographisch aufgereinigt.

Man erhält 226 mg (0,40 mmol) 2-Chlor-*N*-cyclopropyl-5-{1-[3-(ethylsulfanyl)-5-(1,1,1,2,3,3,3-heptafluorpropan-2-yl)pyridin-2-yl]-1*H*-pyrazol-4-yl}benzamid als farblosen Feststoff.
HPLC-MS^{a)}: logP = 4,69, Masse (m/z) = 567 [M+H]⁺.
1H-NMR (400 MHz, D6-DMSO): 9,08 (d, 1H), 8,59 (d, 1H), 8,53 (d, 1H), 8,47 (s, 1H), 8,02 (d, 1H), 7,85-7,82 (m, 2H), 7,53-7,50 (m, 1H), 3,08 (q, 2H), 2,87-2,81 (m, 1H), 1,22 (t, 3H), 0,74-0,69 (m, 2H), 0,58-0,54 (m, 2H).

### Beispiel I-T3-157:

### 2-Chlor-N-cyclopropyl-5-{1-[3-(ethylsulfinyl)-5-(1,1,1,2,3,3,3-heptafluorpropan-2-yl)pyridin-2-yl]-1H-pyrazol-4-yl}benzamid

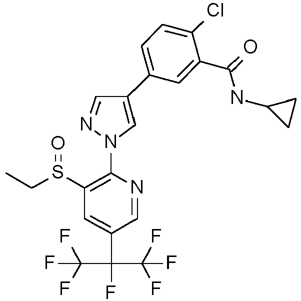

100 mg (0,17 mmol) 2-Chlor-*N*-cyclopropyl-5-{1-[3-(ethylsulfanyl)-5-(1,1,1,2,3,3,3-heptafluorpropan-2-yl)pyridin-2-yl]-1*H*-pyrazol-4-yl}benzamid wurden in 10,0 mL Dichlormethan gelöst und mit einem Eisbad gekühlt. Es wurden portionsweise 43,5 mg 3-Chlorperbenzoesäure hinzugefügt. Das Reaktionsgemisch wurde 2 Stunden unter Eiskühlung gerührt. Das Reaktionsgemisch wurde mit 5mL IN Natronlauge versetzt. Nach 5 Minuten wurde die wässrige Phase abgetrennt. Die organische Phase wurde nach Prüfung auf Peroxide am Rotationsverdampfer unter vermindertem Druck eingeengt. Das Rohprodukt wurde an Kiesegel säulenchromatographisch aufgereinigt.

Man erhielt 61 mg 2-Chlor-*N*-cyclopropyl-5-{1-[3-(ethylsulfinyl)-5-(1,1,1,2,3,3,3-heptafluorpropan-2-yl)pyridin-2-yl]-1*H*-pyrazol-4-yl}benzamid als farblosen Feststoff.
HPLC-MS^{a)}: logP = 3,79, Masse (m/z) = 583 [M+H]⁺.
1H-NMR (400 MHz, D6-DMSO): 9,36 (s, 1H), 8,96 (d, 1H), 8,63 (s, 1H), 8,58 (s, 1H), 8,53 (d, 1H), 7,91 (s, 1H), 7,89 (d, 1H), 7,53 (d, 1H), 3,45-3,30 (m, 1H unter Wasser), 2,95-2,88 (m, 1H), 2,86-2,81 (m, 1H), 1,08 (t, 3H), 0,74-0,69 (m, 2H), 0,60-0,50 (m, 2H).

### Darstellung der Ausgangsverbindungen

### 2,3-Dichlor-5-(1,1,1,2,3,3,3-heptafluorpropan-2-yl)pyridin

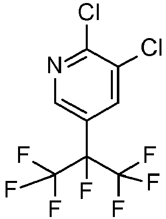

### 1. Stufe: 3-Chlor-5-(1,1,1,2,3,3,3-heptafluorpropan-2-yl)pyridin-2-amin

130,6 g (750 mmol) Natriumdithionit wurden zu einer auf 0-5°C temperierten Mischung aus 64,3 g (500 mmol) 3-Chlorpyridin-2-amin, 222 g (750 mmol) 1,1,1,2,3,3,3-Heptafluor-2-iodpropan und 126 g (1500 mmol) Natriumhydrogencarbonat in 2000 mL einer 3:1 Mischung aus Acetonitril/Wasser (v/v) unter Schutzgas hinzugegeben. Das Reaktionsgemisch wurde für 48 Stunden bei Raumtemperatur gerührt. Anschließend wurde am Rotationsverdampfer unter vermindertem Druck das Acetonitril entfernt. Der Rückstand wurde mit 500 mL Wasser verdünnt. Das Rohprodukt wurde mehrfach mit Ethylacetat aus der wässrigen Phase extrahiert. Die vereinigten organischen Phasen wurden über Natriumsulfat getrocknet, filtriert und anschließend am Rotationsverdampfer unter vermindertem Druck eingeengt. Das Rohprodukt wurde an Kiesegel säulenchromatographisch aufgereinigt.

### 2. Stufe: 3-Chlor-5-(1,1,1,2,3,3,3-heptafluorpropan-2-yl)pyridin-2(1H)-on

5,8 g (19,5 mmol) 3-Chlor-5-(1,1,1,2,3,3,3-heptafluorpropan-2-yl)pyridin-2-amin wurden in 150 mL Schwefelsäure (20%, w/w) gelöst und auf 0-5°C gekühlt. Die Lösung wurde portionsweise mit 2,7 g (40 mmol) Natriumnitrit versetzt. Das Reaktionsgemisch wurde 16 Stunden bei Raumtemperatur gerührt. Das Rohprodukt wurde mehrfach mit Dichlormethan (DCM) aus dem Reaktionsgemisch extrahiert. Die vereinigten organischen Phasen wurden über Natriumsulfat getrocknet, filtriert und anschließend am Rotationsverdampfer unter vermindertem Druck eingeengt. Das Rohprodukt wurde ohne Aufreinigung in der nächsten Stufe eingesetzt.

### 3. Stufe: 2,3-Dichlor-5-(1,1,1,2,3,3,3-heptafluorpropan-2-yl)pyridin

15,4 g (51,7 mmol) 3-Chlor-5-(1,1,1,2,3,3,3-heptafluorpropan-2-yl)pyridin-2(1H)-on und 150 mL Phosphorylchlorid wurden 5 Stunden auf 105°C erhitzt. Das Reaktionsgemisch wurde vorsichtig mit Natriumhydrogencarbonat Lösung neutralisiert. Das Rohprodukt wurde mehrfach mit DCM aus dem Reaktionsgemisch extrahiert. Die vereinigten organischen Phasen wurden mit gesättigter wässriger Natriumchlorid Lösung gewaschen, über Natriumsulfat getrocknet, filtriert und anschließend am Rotationsverdampfer unter vermindertem Druck eingeengt. Das Produkt wurde durch Vakuumdestillation (Sdp. 40°C bei 1 mbar) aufgereinigt.

Man erhielt 14,8 g 2,3-Dichlor-5-(1,1,1,2,3,3,3-heptafluorpropan-2-yl)pyridin als farblose Flüssigkeit.
MS: Masse (m/z) = 315 [M]⁺.
1H-NMR (400 MHz, dl-Chloroform): 8,48 (s, 1H), 7,95 (s, 1H).

### Beispiel I-T3-161:

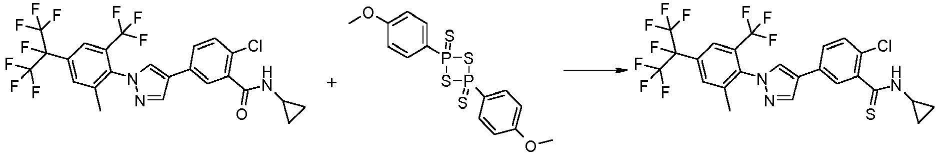

294 mg (0,5 mmol) 2-Chlor-N-cyclopropyl-5-[1-[2-methyl-4-[1,2,2,2-tetrafluor-1-(trifluormethyl)ethyl]-6-(trifluormethyl)phenyl]pyrazol-4-yl]benzamid wurden in einer Mischung aus 0,5 ml ethanolfreiem Trichlormethan und 1,5 ml 1,2-Dimethoxyethan vorgelegt und 101 mg (0,25 mmol) Lawessons-Reagenz (2,4-Bis(4-methoxyphenyl)-1,3,2,4-dithiadiphosphetan-2,4-disulfid) zugefügt. Die Mischung wurde 4 Stunden auf 50°C erwärmt. Danach wurde abgekühlt und das Lösungsmittel im Vakuum am Rotationsverdampfer abgezogen. Der Rückstand wurde zwischen Ethylacetat und gesättigter wässriger Natriumhydrogencarbonat Lösung verteilt, die wässrige Phase einmal mit Ethylacetat nachextrahiert. Die vereinigten organischen Phasen wurden mit Natriumsulfat getrocknet und im Vakuum am Rotationsverdampfer eingedampft. Zur Reinigung wurde über eine Kartusche mit 40 g Kieselgel und einem Gradienten in Cyclohexan/Essigester von 90:10 bis 50:50 (v/v) chromatographiert. Es wurden 248 mg 2-Chlor-N-cyclopropyl-5-[1-[2-methyl-4-[1,2,2,2-tetrafluor-1-(trifluormethyl)ethyl] -6-(trifluormethyl)phenyl]pyrazol-4-yl]benzolcarbothioamid (Verbindung **I-T3-161)** erhalten.
HPLC-MS^{a)}: logP = 5,0, Masse (m/z) = 604 [M+H]+.
¹H-NMR (400 MHz, d₃-Acetonitril): δ (ppm) = 8,62 (s, 1 H (breit)), 8,14 (s, 1 H), 8,10 (s, 1H), 8,0 (s, 1 H), 7,95 (s, 1 H), 7,63 (d, J=2,2 Hz, 1 H), 7,57-7,60 (m, 1 H), 7,42 (d, J=8,4 Hz, 1 H), 3,02 (s, 3 H), 3,37-3,44 (m, 1 H), 0,92-0,95 (m, 2 H), 0,74-0,78 (m, 2 H).

### Darstellungsverfahren I-T4

### Beispiel I-T4-1:

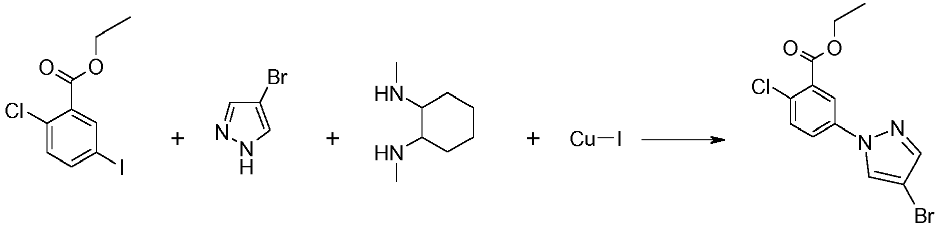

3,81 g (12,2 mmol) 2-Chlor-5-iod-benzoesäureethylester wurden in 37 ml Dimethylformamid vorgelegt, 2,885 g (19,6 mmol) 4-Brom-pyrazol, 5,09 g (36,8 mmol) frisch gemahlenes Kaliumcarbonat, 0,349 g (2,4 mmol) 1,2 Bis-(methylamino)-cyclohexan (racemisch,trans) und 0,234 g (1,22 mmol) Kupfer(I)iodid zugegeben. Der Ansatz wurde mit Argon entgast und dann eine Stunde zum Rückfluss erhitzt. Zur Aufarbeitung wurde abgekühlt, auf 100 ml Wasser gegossen und zweimal mit je 100 ml Essigester extrahiert. Die vereinigten organischen Phasen wurden zweimal mit 100 ml Wasser und anschliessend mit gesättigter Kochsalzlösung gewaschen, mit Natriumsulfat getrocknet und am Rotationsverdampfer im Vakuum eingedampft. Der Rückstand wurde zur Reinigung über eine 120 g-Kartusche mit Kieselgel mit einem Gradienten, von Cyclohexan/Essigester von 90:10 bis 70:30 (v/v) chromatographiert. Es wurden 1,41 g 5-(4-Brompyrazol-1-yl)-2-chlor-benzoesäureethylester erhalten.

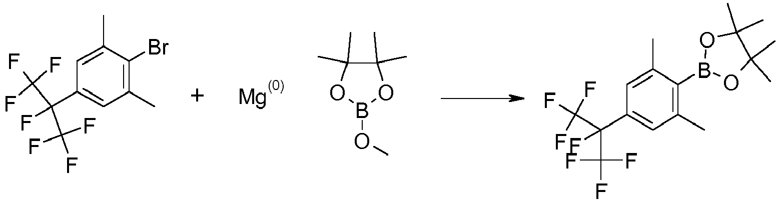

0,158 g (6,49 mg) Magnesiumspäne wurden mit 1,5 ml trockenem Tetrahydrofuran bedeckt. Es wurden einige Tropfen einer Lösung von 1,75 g (4,95 mmol) 2-Brom-1,3-dimethyl-5-[1,2,2,2-tetrafluor-1-(trifluormethyl)ethyl]benzol (hergestellt nach US2003/187233, S. 6) in 2,5 ml trockenem Tetrahydrofuran zugegeben. Zum Starten wurde ein Krümel Jod zugegeben und auf ca. 55 °C erwärmt. Nach dem Start der Reaktion wurde die restliche Lösung des 2-Brom-1,3-dimethyl-5-[1,2,2,2-tetrafluor-1-(trifluormethyl)ethyl]benzols bei einer Temperatur von 55 °C zugetropft. Nach beendeter Zugabe wurde noch 1 Stunde bei 55 °C nachgerührt, dann auf 0 °C gekühlt und eine Lösung von 2-Methoxy-4,4,5,5-tetramethyl-1,3,2-dioxaborolan in 2,5 ml trockenen Tetrahydrofuran zugetropft. Danach liess man auf Raumtemperatur kommen. Zur Aufarbeitung wurde der Ansatz auf gesättigte wässrige Ammoniumchloridlösung gegossen. Die Phasen wurden getrennt, die wässrige Phase mit Essigester nachextrahiert, die vereinigten organischen Phasen dann mit gesättigter wässriger Kochsalzlösung gewaschen, mit Natriumsulfat getrocknet und am Rotationsverdampfer im Vakuum eingedampft. Der Rückstand wurde im Kugelrohr bei einem Vakuum von 1 mbar und 220 °C destilliert. Es wurden 1,85 g 2-[2,6-Dimethyl-4-[1,2,2,2-tetrafluor-1-(trifluormethyl)ethyl]phenyl]-4,4,5,5-tetramethyl-1,3,2-dioxaborolan erhalten.

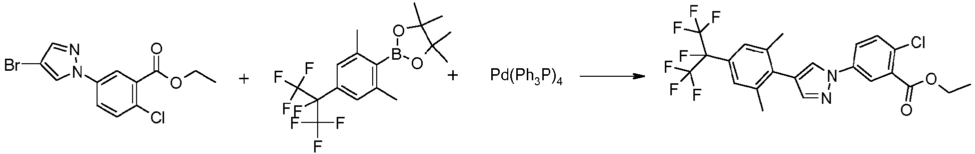

0,947 g (2,87 mmol) 5-(4-Brompyrazol-1-yl)-2-chlor-benzoesäureethylester und 1,15 g (2,87 mmol) 2-[2,6-Dimethyl-4-[1,2,2,2-tetrafluor-1-(trifluormethyl)ethyl]phenyl]-4,4,5,5-tetramethyl-1,3,2-dioxaborolan wurden in 62 ml Isopropanol vorgelegt, 8,7 ml (8,7 mmol) entgaste 1 molare wässrige Natriumhydrogencarbonat-Lösung zugegeben. Der Ansatz wurde mit Argon entgast und 0,166 g (0,14 mmol) Tetrakis(triphenylphosphin)palladium(0) zugegeben und über Nacht zum Rückfluß erhitzt.

Zur Aufarbeitung wurde am Rotationsverdampfer im Vakuum eingedampft und der Rückstand zwischen Wasser und Essigester verteilt. Die organische Phase wurde abgetrennt, die wäßrige Phase mit Essigester nachextrahiert. Die vereinigten organischen Phasen wurden dann einmal mit gesättigter wässriger Kochsalzlösung gewaschen und am Rotationsverdampfer im Vakuum eingedampft. Als Rückstand wurden 1,17 g an rohem 2-Chlor-5-[4-[2,6-dimethyl-4-[1,2,2,2-tetrafluor-1-(trifluormethyl)ethyl]phenyl]pyrazol-1-yl]benzoesäureethylester erhalten.

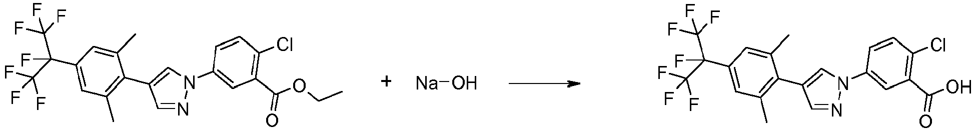

1,76 g (3,36 mmol) 2-Chlor-5-[4-[2,6-dimethyl-4-[1,2,2,2-tetrafluor-1-(trifluormethyl)ethyl]phenyl]pyrazol-1-yl]benzoesäureethylester wurden in 72 ml Methanol vorgelegt und 4,03 ml (4,03 mmol) 1 molare Natronlauge zugegeben. Anschliessend wurde 3 Stunden zum Rückfluss erhitzt. Zur Aufarbeitung wurde am Rotationsverdampfer im Vakuum eingedampft, der Rückstand mit verdünnter Salzsäure aufgenommen und dreimal mit Essigester extrahiert. Die vereinigten Extrakte wurden mit gesättigter Kochsalzlösung gewaschen, mit Natriumsulfat getrocknet, eingedampft und ergaben 1,36 g rohe 2-Chlor-5-[4-[2,6-dimethyl-4-[1,2,2,2-tetrafluor-1-(trifluormethyl)ethyl]phenyl]pyrazol-1-yl]benzoesäure.

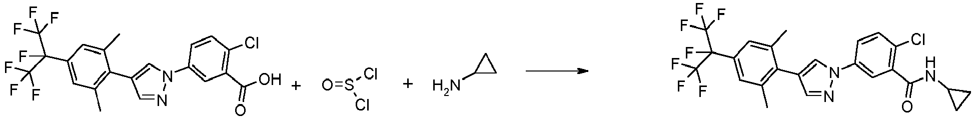

1,36 g (2,76 mmol) rohe 2-Chlor-5-[4-[2,6-dimethyl-4-[1,2,2,2-tetrafluor-1-(trifluormethyl)ethyl]phenyl]pyrazol-1-yl]benzoesäure wurden in 14 ml trockenem Toluol gelöst, 1 ml (13,8 mmol) Thionylchlorid zugegeben und dann 2 Stunden auf 80 °C erhitzt. Danach wurde im Vakuum am Rotationsverdampfer eingedampft, 1 ml trockenes Toluol zugefügt und erneut eingedampft. Es wurden 1,4 g rohes Säurechlorid als Rückstand erhalten. 0,7 g des Rückstands wurden in 5 ml Dichlormethan gelöst und zu einer Lösung von 0,195 g (3,41 mmol) Cyclopropylamin in 2 ml Dichlormethan bei Raumtemperatur zugetropft. Der Ansatz wurde 2 Stunden bei Raumtemperatur nachgerührt, dann auf 20 ml 5 %ige wässrige Natriumdihydrogenphosphatlösung gegossen. Die organische Phase wurde abgetrennt und mit gesättigter Kochsalzlösung gewaschen, mit Natriumsulfat getrocknet und am Rotationsverdampfer im Vakuum eingedampft. Der Rückstand wurde durch zweimalige Chromatographie über eine Kartusche mit 15 g Kieselgel mit einem Gradienten von Cyclohexan/Essigester von 90:10 bis 50:50 (v/v) gereinigt. Es wurden 91 mg (1,36 mmol) N-Cyclopropyl-2-chlor-5-[4-[2,6-dimethyl-4-[1,2,2,2-tetrafluor-1-(trifluormethyl)ethyl]phenyl]pyrazol-1-yl]benzamid (Verbindung **I**-**T4-1**) erhalten.
HPLC-MSₐ₎: logP = 4,74, Masse (m/z) = 534 [M+H]+.
¹H-NMR (400 MHz, d₃-Acetonitril): δ = (ppm) 8,17 (s, 1 H), 7,86 (s, 1 H), 7,84 (d, J1=2,7 Hz, 1 H), 7,69 (s, 1 H), 7,54(d, J1=8,8 Hz, 1H), 7,44 (s, 2 H), 6,97 (s (breit), 1 H (N-H)), 2,83-2,87 (m, 1H), 2,25 (s, 6 H), 0,76-0,8 (m, 2 H), 0,58-0,62 (m, 2 H).

### Beispiel I-T4-3:

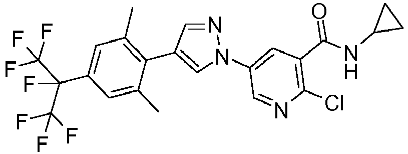

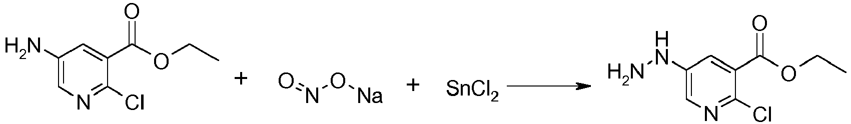

2 g (9,96 mmol) 5-Amino-2-chlor-nicotinsäureethylester (kommerziell erhältlich) wurden in 8,6 ml 33 %iger wässriger Salzsäure vorgelegt und 30 Minuten bei Raumtemperatur gerührt. Danach wurden noch 7 ml Wasser zugegeben und mit einem Eisbad auf 0 °C gekühlt. Zu dieser Mischung wurde eine Lösung aus 750 mg (10,8 mmol) Natriumnitrit in 6,92 ml Wasser innerhalb von 30 Minuten zugetropft. Die Temperatur wurde mit einem Eisbad unterhalb von +5 °C gehalten. Es wurde 15 Minuten bei 0 °C nachgerührt.

In einem zweiten Kolben wurden 5,77 g (25,5 mmol) Zinn(II)chlorid Dihydrat in 24 ml 16 %iger wässriger Salzsäure vorgelegt und die oben hergestellte Diazoniumsalz-Suspension langsam, bei 0°C, zugetropft. Es wurde 1 Stunde bei 0 °C nachgerührt. Danach wurden 50 ml Acetonitril und 40 ml gesättigte wässrige Kochsalzlösung zugesetzt. Die gebildeten Phasen wurden getrennt. Die wässrige Phase wurde zweimal mit je 50 ml Acetonitril nachextrahiert. Die vereinigten organischen Phasen wurden mit Natriumsulfat getrocknet und am Rotationsverdampfer im Vakuum eingedampft. Als Rückstand wurden 10,7 g an rohem 2-Chlor-5-hydrazino-nicotinsäureethylester erhalten.

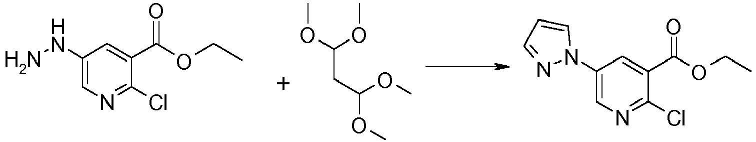

10,7 g roher 2-Chlor-5-hydrazino-nicotinsäureethylester wurden in 50 ml Ethanol vorgelegt, danach 1,63 g (9,92 mmol) 1,1,3,3-Tetramethoxypropan und 487 mg 96 %ige Schwefelsäure zugegeben. Die Mischung wurde anschliessend 2 Stunden zum Rückfluss erhitzt. Der grösste Teil des Ethanols wurde im Vakuum am Rotationsverdampfer entfert und der Rückstand zwischen gesättigter wässriger Natriumhydrogencarbonat-Lösung und Ethylacetat verteilt. Die organische Phase wurde abgetrennt, mit gesättigter wässriger Kochsalzlösung gewaschen, über Natriumsulfat getrocknet und im Vakuum am Rotationsverdampfer eingedampft. Der Rückstand wurde über eine Kartusche mit 15 g Kieselgel und einem Gradienten ausgehend von reinem Cyclohexan bis Cyclohexan/Ethylacetat 50:50 (v/v) chromatographiert. Es wurden 396 mg 2-Chlor-5-(pyrazol-1-yl)-nicotinsäureethylester erhalten.

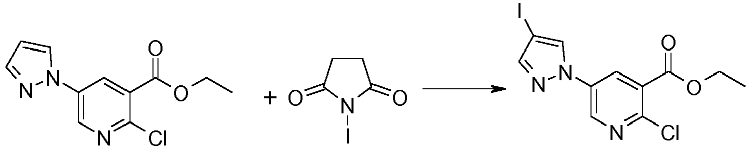

396 mg (1,57 mmol) 2-Chlor-5-(pyrazol-1-yl)-nicotinsäureethylester wurden in 10 ml Acetonitril vorgelegt und 1,062 g (4,72 mmol) N-Iodsuccinimid zugegeben. Anschliessend wurde unter Argon 3 Stunden unter Rückfluss erwärmt. Der Ansatz wurde etwas abgekühlt und das Lösungsmittel im Vakuum am Rotationsverdampfer entfernt. Der Rückstand zwischen Wasser und Essigester verteilt. Die organische Phase wurde abgetrennt, erst mit gesättigter wässriger Natriumhydrogensulfit-Lösung, danach mit gesättigter wässriger Natriumhydrogencarbonat-Lösung und zuletzt mit gesättigter wässriger Kochsalz-Lösung gewaschen, mit Natriumsulfat getrocknet und im Vakuum am Rotationsverdampfer eingedampft. Der Rückstand wurde über eine Kartusche mit 15 g Kiesel und einem Gradienten ausgehend von reinem Cyclohexan bis Cyclohexan/Ethylacetat 50:50 (v/v) chromatographiert.

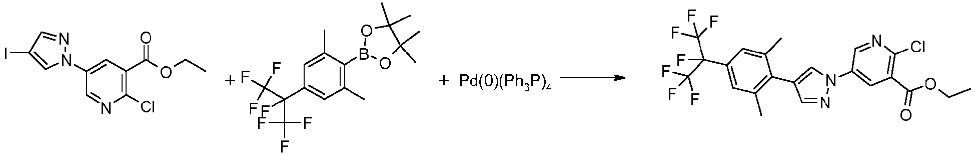

401 mg (1,06 mmol) 2-Chloro-5-(4-iodpyrazol-1-yl)pyridin-3-carbonsäureethylester und 425 mg (1,06 mmol) 2-[2,6-Dimethyl-4-[1,2,2,2-tetrafluor-1-(trifluormethyl)ethyl]phenyl]-4,4,5,5-tetramethyl-1,3,2-dioxaborolan wurden in 23 ml Isopropanol vorgelegt, dann 3,24 ml (3,24 mmol) entgaste 1 molare wässrige Natriumhydrogencarbonat-Lösung und 61 mg (0,05 mmol) Tetrakis(triphenylphosphin)palladium(0) zugegeben. Es wurde nochmals mit Argon entgast und über Nacht zum Rückfluss erhitzt. Danach wurde abgekühlt und die flüchtigen Bestandteile im Vakuum am Rotationsverdampfer abgezogen. Der Rückstand wurde zwischen Wasser und Ethylacetat verteilt. Die organische Phase wurde abgetrennt, einmal mit gesättigter wässriger Kochsalzlösung gewaschen und im Vakuum am Rotationsverdampfer eingedampft. Es wurden 415 mg an rohem 2-Chlor-5-[4-[2,6-dimethyl-4-[1,2,2,2-tetrafluor-1-(trifluormethyl)ethyl]phenyl]pyrazol-1 -yl]pyridin-3 - carbonsäureethylester erhalten.

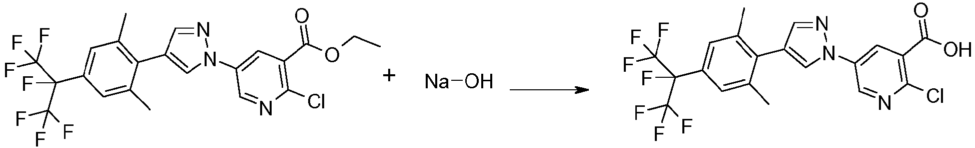

416 mg (0,79 mmol) roher 2-Chloro-5-[4-[2,6-dimethyl-4-[1,2,2,2-tetrafluor-1-(trifluormethyl)ethyl]phenyl]pyrazol-1-yl]pyridin-3-carbonsäureethylester wurden in 16,9 ml Methanol gelöst und 0,952 ml (0,95 mmol) 1 M Natronlauge zugegeben. Es wurde 6 Stunden unter Rückfluss erhitzt, danach abgekühlt und am Rotationsverdampfer im Vakuum eingedampft. Der Rückstand wurde zwischen Essigester und verdünnter Salzsäure verteilt. Die wässrige Phase wurde zweimal mit Essigester rückextrahiert. Die vereinigten organischen Phasen wurden mit gesättigter wässriger Kochsalzlösung gewaschen, mit Natriumsulfat getrocknet und am Rotationsverdampfer im Vakuum eingedampft. Es wurden 380 mg rohe 2-Chloro-5-[4-[2,6-dimethyl-4-[1,2,2,2-tetrafluor-1-(trifluormethyl)ethyl]phenyl]pyrazol-1-yl]pyridin-3-carbonsäure erhalten.

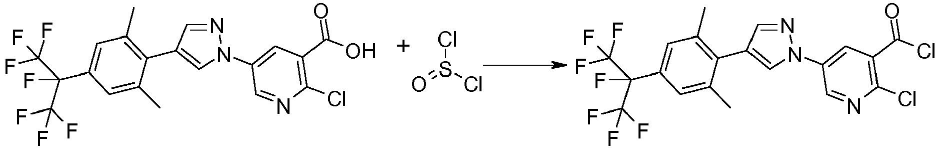

380 mg (0,76 mmol) rohe 2-Chlor-5-[4-[2,6-dimethyl-4-[1,2,2,2-tetrafluor-1-(trifluormethyl)ethyl]phenyl]pyrazol-1-yl]pyridin-3-carbonsäure wurden in Toluol gelöst und 456 mg (3,83 mmol) Thionylchlorid zugegeben. Den Ansatz wurde 2 Stunden auf 80 °C erhitzt und dann im Vakuum am Rotationsverdampfer eingedampft. Es wurden 400 mg an rohem 2-Chlor-5-[4-[2,6-dimethyl-4-[1,2,2,2-tetrafluor-1-(trifluormethyl)ethyl]phenyl]pyrazol-1-yl]pyridine-3-carbonsäurechlorid erhalten.

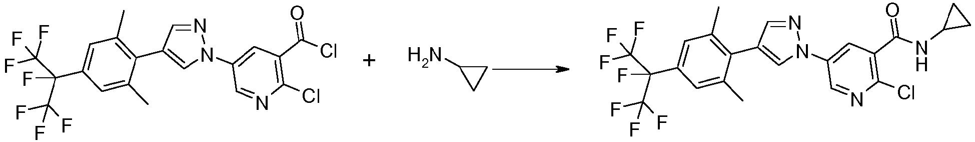

138 mg (0,26 mmol) 2-Chlor-5-[4-[2,6-dimethyl-4-[1,2,2,2-tetrafluor-1-(trifluormethyl)ethyl]phenyl]pyrazol-1-yl]pyridin-3-carbonsäurechlorid wurden in 1 ml Dichlormethan gelöst und zu einer Lösung von 38 mg Cyclopropylamin in 1 ml Dichlormethan bei Raumtemperatur zugetropft. Die Mischung wurde 2 Stunden bei Raumtemperatur nachgerührt. Danach wurde die Mischung mit 5 %iger Natriumdihydrogenphosphatlösung und anschliessend mit gesättigter wässriger Kochsalzlösung gewaschen, mit Natriumsulfat getrocknet und am Rotationsverdampfer im Vakuum eingedampft. Zur Reinigung wurde über eine Kartusche mit 15 g Kieselgel mit einem Gradienten in Cyclohexan/Ethylacetat von 90:10 bis 50:50 (v/v) chromatographiert. Es wurden 30 mg 2-Chlor-N-cyclopropyl-5-[4-[2,6-dimethyl-4-[1,2,2,2-tetrafluor-1-(trifluormethyl)ethyl]phenyl]pyrazol-1-yl]pyridine-3 -carboxamid erhalten.
HPLC-MSₐ₎: logP = 4,42, Masse (m/z) = 534 [M+H]+.
¹H-NMR (400 MHz, d₃-Acetonitril): δ (ppm) = 8,92 (d, J = 2,8 Hz, 1 H), 8,22 (d, J1=2,8 Hz, 1 H), 8,20 (s, 1 H), 7,75(s, 1H), 7,44 (s, 2 H), 5,1 (s (breit), 1 H (N-H)), 2,84-2,88 (m, 1H), 2,25 (s, 6 H), 0,78-0,81 (m, 2 H), 0,59-0,63 (m, 2 H).

### Darstellungsverfahren I-T22

### Beispiel I-T22-1:

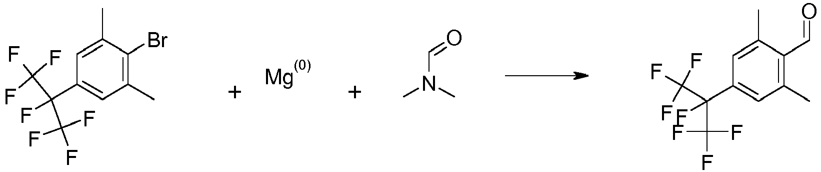

Die Herstellung von 2,6-Dimethyl-4-heptafluorisopropyl-brombenzol ist in US2003/187233, S. 6 [0080] beschrieben.

In einem 25 ml Dreihalskolben wurden 158 mg (6,5 mg-Atom) Magnesiumspäne mit trockenem Tetrahydrofuran (THF) bedeckt. Dann wurden einige Tropfen einer Lösung von 1,75 g (4,95 mmol) 2,6-Dimethyl-4-heptafluorisopropyl-brombenzol in 2,5 ml trockenem THF zugegeben. Zum Start der Reaktion wurde ein Krümel Jod zugegeben und auf ca. 60 °C erwärmt. Nach gestarteter Reaktion wurde die restliche Lösung des 2,6-Dimethyl-4-heptafluorisopropyl-brombenzols bei ca. 60 °C zugetropft. Nach beendeter Zugabe wurde noch eine Stunde bei 60 °C nachgerührt, danach auf 0 °C gekühlt und eine Lösung von 1,09 g (14,8 mmol) Dimethylformamid in 2,5 ml trockenem THF zugetropft. Danach liess man den Ansatz auf Raumtemperatur kommen. Zur Aufarbeitung wurde überschüssige gesättigte wässrige Ammoniumchlorid-Lösung zugefügt, die Phasen getrennt, die wässrige Phase mit Essigester nachextrahiert. Die vereinigten organischen Phasen wurden mit gesättigter Kochsalzlösung gewaschen, mit Natriumsulfat getrocknet und am Rotationsverdampfer im Vakuum eingedampft. Als Rückstand verblieben 1,3 g an rohem 2,6-Dimethyl-4-heptafluorisopropyl-benzaldehyd (Reinheit ca. 80 %), der ohne Reinigung weiterverwendet wurde.

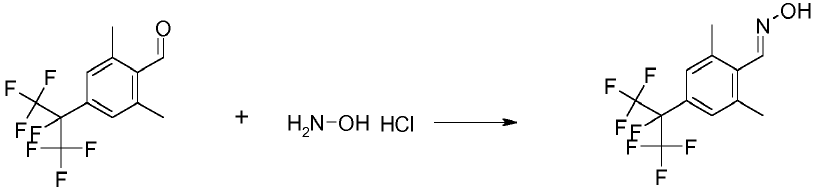

1,3 g (ca. 3,44 mmol) roher 2,6-Dimethyl-4-heptafluorisopropyl-benzaldehyd wurden in 26 ml Methanol gelöst, 361 mg (4,3 mmol) Natriumhydrogencarbonat zugegeben und die Mischung auf 0 °C gekühlt. Danach wurden 1,2 g (17,2 mmol) Hydroxylammoniumchlorid zugegeben und über Nacht bei Raumtemperatur gerührt. Zur Aufarbeitung wurde die Mischung am Rotationsverdampfer im Vakuum eingedampft und der Rückstand in 100 ml Essigester aufgenommen. Ungelöste Bestandteile wurden abfiltriert und das Filtrat im Vakuum am Rotationsverdampfer eingeengt. Der Rückstand wurde dann durch Chromatographie über eine 40 g Kartusche mit Kiesel und einem Gradienten ausgehend von reinem Cyclohexan bis Cyclohexan/Essigester 70:30 (v/v) gereinigt. Es wurden 0,5 g 2,6-Dimethyl-4-heptafluorisopropyl-benzaldehydoxim erhalten.

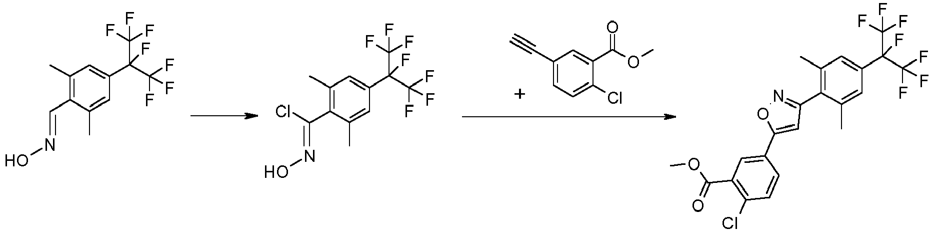

505 mg (1,59 mmol) 2,6-Dimethyl-4-heptafluorisopropyl-benzaldehydoxim wurden in 3,5 ml Dimethylformamid (DMF) vorgelegt und 234 mg (1,75 mmol) N-Chlorsuccinimid zugegeben. Es wurde 3,5 Stunden bei Raumtemperatur gerührt. Danach wurde der Ansatz auf 0 °C gekühlt und eine Lösung von 310 mg (1,59 mmol) 2-Chlor-5-ethinyl-benzoesäuremethylester (hergestellt nach WO2012/107434, S. 103) in 1,5 ml DMF zugetropft, gefolgt von 355 mg (3,5 mmol) Triethylamin. Die Reaktionsmischung wurde dann über Nacht bei Raumtemperatur gerührt. Zur Aufarbeitung wurde auf Wasser gegossen, zweimal mit Dichlormethan extrahiert, die vereinigten Extrakte mit Wasser gewaschen, mit Natriumsulfat getrocknet und im Vakuum am Rotationsverdampfer eingedampft. Zur Reinigung wurde über eine 40 g Kartusche mit Kieselgel und einem Gradienten ausgehend von reinem Cyclohexan nach Cyclohexan/Essigester 80:20 (v/v) gereingt. Es wurden 488 mg 2-Chlor-5-[3-[2,6-dimethyl-4-[1,2,2,2-tetrafluor-1-(trifluormethyl)ethyl]phenyl]isoxazol-5-yl]benzoesäuremethylester erhalten.

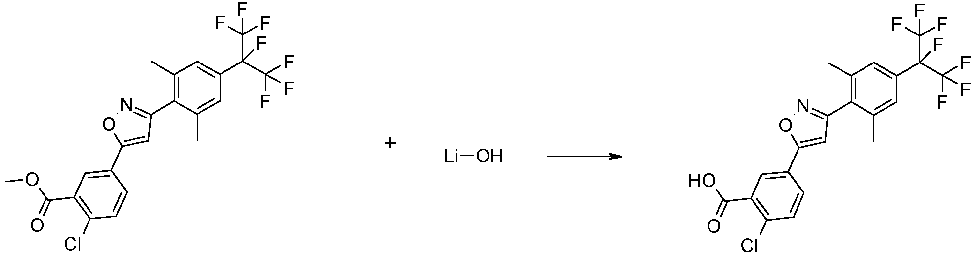

0,8 g (1,56 mmol) 2-Chlor-5-[3-[2,6-dimethyl-4-[1,2,2,2-tetrafluor-1-(trifluormethyl)ethyl]phenyl]isoxazol-5-yl]benzoesäuremethylester wurden in einem Gemisch aus 18 ml Dioxan und 6,5 ml Wasser vorgelegt, 86 mg (2,04 mmol) Lithiumhydroxid-Monohydrat zugegeben und über Nacht bei Raumtemperatur gerührt. Zur Aufarbeitung wurde im Vakuum eingedampft und der Rückstand zwischen einer Mischung aus verdünnter Salzsäure und Dichlormethan verteilt. Die organische Phase wurde abgetrennt, die wässrige Phase erst mit Dichlormethan, dann mit Essigester extrahiert. Die vereinigten organischen Phasen wurden mit gesättigter wässriger Kochsalzlösung gewaschen, mit Natriumsulfat getrocknet und im Vakuum am Rotationsverdampfer eingedampft. Es wurden 680 mg 2-Chlor-5-[3-[2,6-dimethyl-4-[1,2,2,2-tetrafluor-1-(trifluormethyl)ethyl]phenyl]isoxazol-5-yl]benzoesäure erhalten.

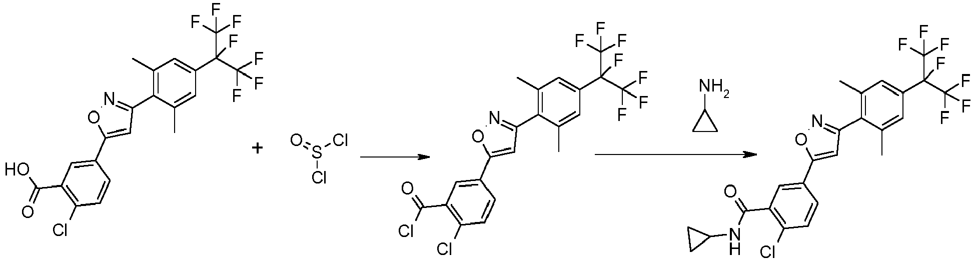

680 mg (1,37 mmol) 2-Chlor-5-[3-[2,6-dimethyl-4-[1,2,2,2-tetrafluor-1-(trifluormethyl)ethyl]phenyl]isoxazol-5-yl]benzoesäure wurden in 7 ml Toluol gelöst und 0,5 ml (6,89 mmol) Thionylchlorid zugegeben. Die Mischung wurde zwei Stunden auf 80 °C erhitzt und danach im Vakuum am Rotationsverdampfer eingedampft. 200 mg (0,38 mmol) des so erhaltenen rohen Säurechlorids wurden in 1 ml Dichlormethan gelöst und zu einer Lösung von 56 mg (0,97 mmol) Cyclopropylamin in 0,95 ml Dichlormethan bei Raumtemperatur zugetropft. Die Mischung wurde danach über Nacht bei Raumtemperatur gerührt. Zur Aufarbeitung wurde die Mischung auf 5 %ige wässrige Natriumdihydrogenphosphatlösung gegossen, die organische Phase abgetrennt, mit Natriumsulfat getrocknet und im Vakuum am Rotationsverdampfer eingedampft. Zur Reinigung wurde über eine Kartusche mit 15 g Kieselgel und einem Gradienten von reinem Cyclohexan bis Cyclohexan/Essigester 80:20 (v/v) chromatographiert. Es wurden 165 mg 2-Chlor-N-cyclopropyl-5-[3-[2,6-dimethyl-4-[1,2,2,2-tetrafluor-1-(trifluormethyl)ethyl]phenyl]isoxazol-5-yl]benzamid (Verbindung **I-T22-1**) erhalten.
HPLC-MS^{a)}: logP = 4,75, Masse (m/z) = 535 [M+H]+.
¹H-NMR (400 MHz, d₃-Acetonitril): δ (ppm) = 7,93 (d, J=2,2 Hz, 1 H), 7,89 (dd, J1=8,4 Hz, J2=2,2 Hz, 1H), 7,6 (d, J=8,4 Hz, 1H), 7,49 (s, 2 H), 7,03 (s (breit), 1 H (N-H)), 6,86 (s, 1 H), 2,83-2,88 (m, 1H), 0,75-0,79 (m, 2 H), 0,59-0,62 (m, 2 H).

### Darstellungsverfahren I-T23

### Beispiel I-T23-1:

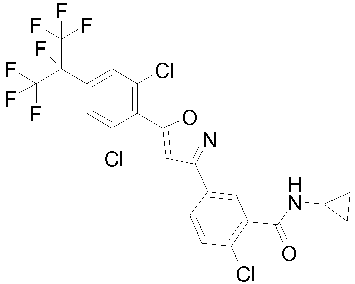

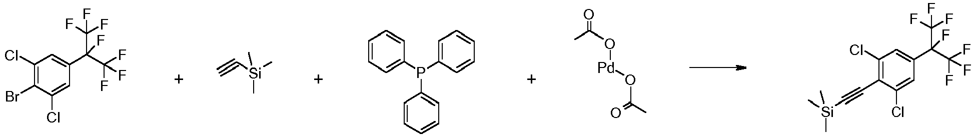

3 g (7,61 mmol) 2-Brom-1,3-dichlor-5-[1,2,2,2-tetrafluor-1-(trifluormethyl)ethyl]benzol (Herstellung siehe EP 1 253 128, Seite 10), 1,21 g (12,3 mmol) Ethinyltrimethylsilan, 86 mg (0,38 mmol) Palladium(II)acetat und 260 mg (1,0 mmol) Triphenylphosphin wurden in 20 ml trockenem Triethylamin vorgelegt und zum Rückfluss erhitzt. Nach Einengen des Volumens am Rotationsverdampfer bei 30 °C wurde der Rückstand mit 20 ml gesättigter Natriumhydrogencarbonatlösung versetzt und dreimal mit Dichlormethan extrahiert. Die vereinigten Extrakte wurden mit 5 %iger wässriger NaH2PO4-Lösung und anschliessend mit gesättigter Kochsalzlösung gewaschen. Nach Trocknung der Lösung mit Natriumsulfat und Einengung des Volumens am Rotationsverdampfer bei 30 °C erfolgte die Reinigung mittels Chromatographie an Kieselgel mit Cyclohexan als Laufmittel. Ausbeute: 1,4 g 2-[2,6-Dichlor-4-[1,2,2,2-tetrafluor-1-(trifluormethyl)ethyl]phenyl]ethinyl-trimethyl-silan in einer Reinheit von ca. 50 % (LC/MS-Fläche).

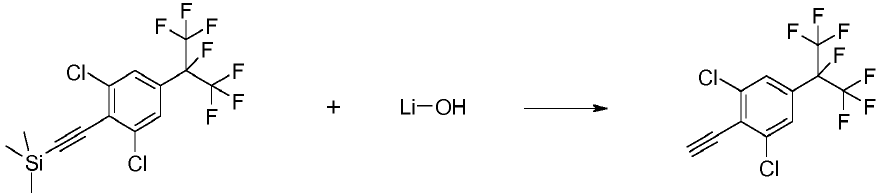

1,4 g (3,4 mmol) 2-[2,6-Dichlor-4-[1,2,2,2-tetrafluor-1-(trifluormethyl)ethyl]phenyl]ethinyl-trimethyl-silan wurden in 7 ml Tetrahydrofuran gelöst und eine Mischung aus 7 ml Methanol und 214 mg (5,1 mmol) Lithiumhydroxidmonohydrat bei Raumtemperatur zugegeben. Die Reaktionslösung am Rotationsverdampfer eingedampft und der Rückstand mit einem Gemisch aus Dichlormethan und Wasser aufgenommen. Die organische Phase wurde abgetrennt, mit Natriumsulfat getrocknet, im Vakuum am Rotationsverdampfer eingedampft und ergab 880 mg ca. 50 %iges 1,3-Dichlor-2-ethinyl-5-[1,2,2,2-tetrafluor-1-(trifluortnethyl)ethyl]benzol.

Die Herstellung von 4-Chlor-3-carbomethoxy-benzaldehyd ist bereits in der Literatur beschrieben (siehe z. B. WO 2010/011584, S. 19-20).

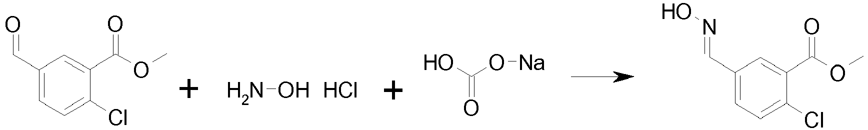

4,1 g (20,6 mmol) 4-Chlor-3-carbomethoxy-benzaldehyd wurden in 82 ml Methanol gelöst, 1,734 g (20,6 mmol) Natriumhydrogencarbonat zugegeben und auf 0 °C gekühlt. Dann wurden 5,738 g (82,5 mmol) Hydroxylamin Hydrochlorid zugegeben und gerührt. Zur Aufarbeitung wurde am Rotationsverdampfer eingedampft und Rest in 100 ml Essigester aufgenommen. Der Feststoff wurde abfiltriert und das Filtrat im Vakuum am Rotationsverdampfer eingedampft. Der Rückstand wurde zur Reinigung mit Kieselgel mittels eines Gradienten in Cyclohexan/Essigester 9:1 bis 7:3 (v/v) chromatographiert und ergab 2,68 g 2-Chlor-5-[(E)-hydroxyiminomethyl]benzoesäureethylester.

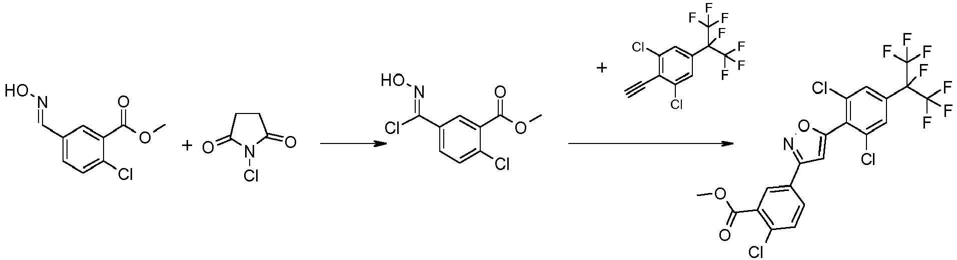

277 mg (1,29 mmol) 2-Chloro-5-[(E)-hydroxyiminomethyl]benzoesäureethylester wurden in 4,6 ml Dimethylformamid vorgelegt und 381 mg (2,84 mmol) N-Chlor-succinimid zugegeben und bei Raumtemperatur gerührt. Der Ansatz wurde dann mit einem Eisbad auf 0 °C gekühlt und eine Lösung von 880 mg (ca. 50 %ig, 1,29 mmol) 1,3-Dichlor-2-ethinyl-5-[1,2,2,2-tetrafluor-1-(trifluormethyl)ethyl]benzol in 1,5 ml Dimethylformamid zugetropft, gefolgt von 289 mg (2,85 mmol) Triethylamin. Der Ansatz wurde bei Raumtemperatur gerührt. Zur Aufarbeitung wurde mit Wasser verdünnt und zweimal mit Dichlormethan extrahiert. Die vereinigten Extrakte wurden mit Wasser gewaschen, mit Natriumsulfat getrocknet und am Rotationsverdampfer eingedampft. Der Rückstand wurde durch zweimalige Chromatographie an Kiesel und einem Gradienten ausgehend von reinem Cyclohexan bis Cyclohexan/Essigester 80:20 (v/v) als Laufmittel gereinigt und ergab 410 mg 2-Chlor-5-[5-[2,6-dichlor-4-[1,2,2,2-tetrafluor-1-(trifluormethyl)ethyl]phenyl]isoxazol-3-yl]benzoesäuremethylester.

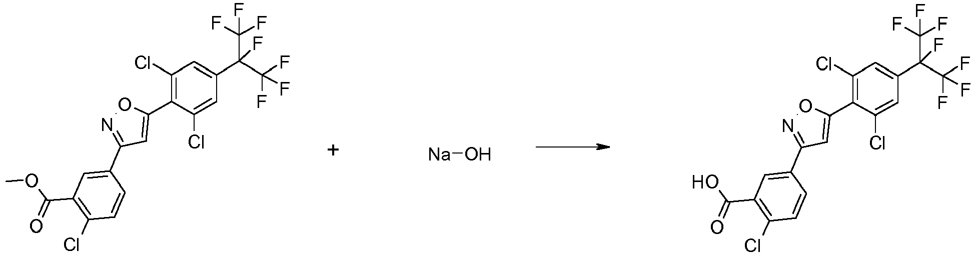

410 mg (0,74 mmol) 2-Chlor-5-[5-[2,6-dichlor-4-[1,2,2,2-tetrafluor-1-(trifluormethyl)ethyl]phenyl]isoxazol-3-yl]benzoesäuremethylester wurden in 21 ml Methanol vorgelegt 0,74 ml (0,74 mmol) 1M Natronlauge zugegeben und unter Rückfluss gerührt. Anschließend wurde das Methanol am Rotationsverdampfer entfernt. Der Rückstand wurde mit verdünnter Salzsäure versetzt und dreimal mit Essigester extrahiert. Die vereinigten Extrakte trocknete man mit Natriumsulfat und dampfte im Vakuum am Rotationsverdampfer ein. Es wurden 405 mg 2-Chlor-5-[5-[2,6-dichlor-4-[1,2,2,2-tetrafluor-1-(trifluormethyl)ethyl]phenyl]isoxazol-3-yl]benzoesäure als Rückstand erhalten.

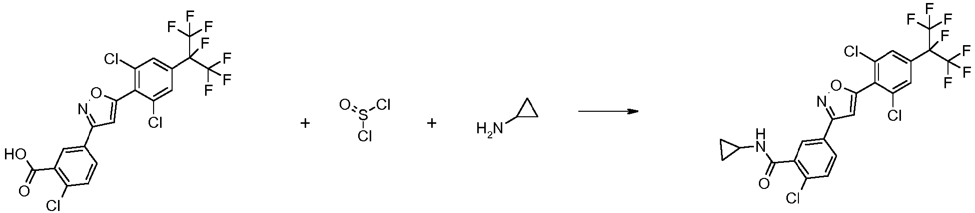

125 mg (0,23 mmol) 2-Chlor-5-[5-[2,6-dichlor-4-[1,2,2,2-tetrafluor-1-(trifluormethyl)ethyl]phenyl]isoxazol-3-yl]benzoesäure wurden in 1,1 ml trockenem Toluol gelöst und 0,14 g (1,16 mmol) Thionylchlorid zugegeben. Der Ansatz wurde auf 80 °C erhitzt und danach am Rotationsverdampfer eingedampft. Der Rückstand wurde in 0,25 ml Dichlormethan gelöst und zu einer Lösung von 33 mg (0,58 mmol) Cyclopropylamin in 0,75 ml Dichlormethan bei 0 °C zugetropft und 2 Stunden bei Raumtemperatur gerührt. Zur Aufarbeitung wurde 5 %ige wässrige Natriumdihydrogenphosphatlösung zugegeben und anschließend die organische Phase abgetrennt. Die organische Phase wurde mit Natriumsulfat getrocknet und am Rotationsverdampfer eingedampft. Der Rückstand wurde durch Chromatographie mit Kieselgel und Cyclohexan/Essigester 70:30 (v/v) als Laufmittel gereinigt. Es wurden 49 mg 2-Chlor-N-cyclopropyl-5-[5-[2,6-dichlor-4-[1,2,2,2-tetrafluor-1-(trifluormethyl)ethyl]phenyl]isoxazol-3-yl]benzamid (Verbindung **I-T23-1**) erhalten.
HPLC-MS^{a)}: logP = 4,96, Masse (m/z) = 575 [M+H]+.
¹H-NMR (400 MHz, d₃-Acetonitril): δ = 7,96 (s, 1 H), 7,94-7,96 (dd, J1=8,4 Hz, J2=2,2 Hz, 1H), 7,86 (s, 2 H), 7,6 (d, J1=7,6 Hz, J2=1,2, 1 H), 7,15 (s, 1 H), 6,9 (s (breit), 1 H (N-H)), 3,97 (s, 3 H), 2,83-2,88 (m, 1H), 0,75-0,79 (m, 2 H), 0,58-0,62 (m, 2 H).

### Verfahren 1 Beispiel 4-Heptafluorisopropyl-2-methyl-6-trifluormethyl-anilin

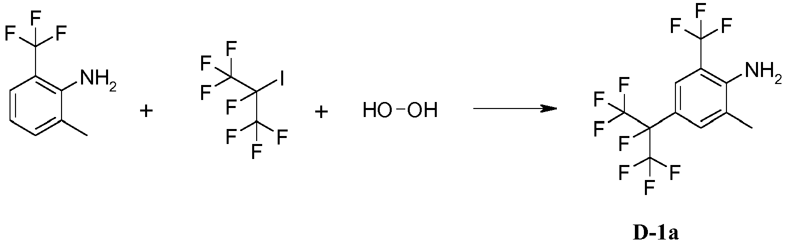

In einem Dreihalskolben wurden 17,48 g (100 mmol) 2-Methyl-6-trifluormethyl-anilin in 498 ml Dimethylsulfoxid vorgelegt und dann 44,3g (21,095 ml, 150 mmol) 2-Iodheptafluorpropan, 29,9 ml (29,9 mmol) 1 molare Eisen(II)-sulfatlösung in Wasser und 5,43 ml (104 mmol) 96 %ige Schwefelsäure zugegeben. Die Mischung wurde dann mit Argon entgast und dann mittels einer Spritzenpumpe 20,4 ml 30 %ige wässrige Wasserstoffperoxid Lösung innerhalb von 15 Minuten zugetropft. Die Temperatur stieg auf 54°C. Gegen Ende des Zutropfens wurde noch kurzzeitig auf 60°C erwärmt. Der Ansatz wurde 20 Minuten ohne Heizung nachgerührt, dabei sank die Temperatur auf 36 °C. Zur Aufarbeitung wurde der Ansatz auf gesättigte wässrige Natriumhydrogencarbonatlösung gegossen und das Produkt mit Essigester extrahiert. Die vereinigten Extrakte wurden erst mit Wasser und danach mit gesättigter wässriger Kochsalzlösung gewaschen, mit Natriumsulfat getrocknet und am Rotationsverdampfer im Vakuum eingedampft. Zur Reinigung wurde in zwei Portionen über eine Säule mit 120 g Kieselgel und einem Gradienten von reinem Cyclohexan bis Cyclohexan/Essigester 95:5 (v/v) chromatographiert. Es wurden 18,9 g 4-Heptafluorisopropyl-2-methyl-6-trifluormethyl-anilin erhalten.

Analog wurde auch 2-Chlor-4-heptafluorisopropyl-6-trifluormethyl-anilin ausgehend von 2-Chlor-6-trifluormethyl-anilin und 2-Iodheptafluorpropan erhalten:

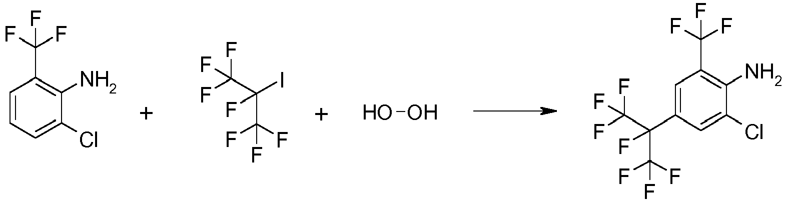

In einem Dreihalskolben wurden 30 g (0,153 mol) 2-Chlor-6-trifluormethylanilin (kommerziell erhältlich) in 765 ml Dimethylsulfoxid (DMSO) vorgelegt und dann 68,1 g (0,23 mol) 2-Iodheptafluorpropan, 46 ml einer 1 molaren wässrigen Eisen(II)-sulfatlösung und 15,4 g 98%ige Schwefelsäure zugegeben. Die Mischung wurde mit Argon entgast und dann mit einer Spritzenpumpe 34,8 g 30 %ige wässrige Wasserstoffperoxid Lösung innerhalb von 30 Minuten zugetropft. Dabei stieg die Temperatur auf 70°C. Der Ansatz wurde 20 Minuten nachgerührt, dabei sank die Temperatur auf 30 °C. Die Reaktionsmischung wurde dann auf gesättigte wässrige Natriumhydrogencarbonatlösung gegossen und mit Ethylacetat extrahiert. Die vereinigten Extrakte wurden zuerst mit Wasser, dann mit gesättigter wässriger Bisulfitlösung und gesättigter wässriger Kochsalz Lösung gewaschen, mit Natriumsulfat getrocknet und im Vakuum am Rotationsverdampfer eingedampft. Zur Reinigung wurde über eine Kartusche mit 330 g Kieselgel und einem Gradienten ausgehend von reinem Cyclohexan bis Cyclohexan/Ethylacetat 90:10 (v/v) chromatographiert. Es wurden 46,1 g 2-Chlor-4-heptafluorisopropyl-6-trifluormethyl-anilin erhalten.

### Verfahren 2 Beispiel 4-Heptafluorisopropyl-2-methyl-6-trifluormethyl-anilin

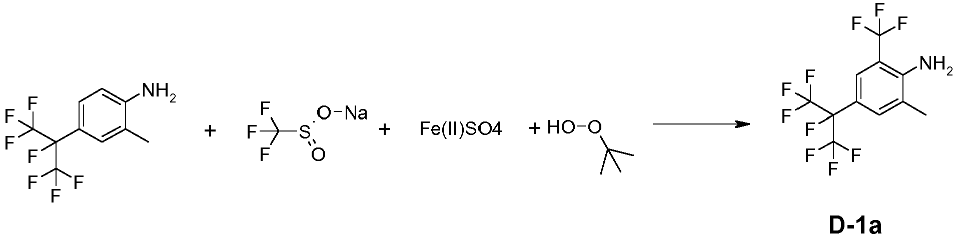

In einem 1000 ml Dreihalskolben wurden 25 g (91 mmol) 4-Heptafluorisopropyl-2-methylanilin zu einer Mischung aus 363,4 ml Wasser und 181,7 ml Acetonitril gegeben. Dann wurden 27,3 ml (27,3 mmol) wässrige 1 molare Eisen(II) sulfat-Lösung und 31,19g (200 mmol) Natriumtrifluormethylsulfinat zugefügt. Die Mischung wurde mit Argon entlüftet und anschließend 35,1 g (273 mmol) einer 70%igen wässrigen tert.-Butylhydroperoxid Lösung mit einer Spritzenpumpe innerhalb von 4,5 Stunden ohne Kühlung zudosiert. Die Temperatur stieg bis auf 34°C an. Nach beendeter Zugabe wurde 1 Stunde nachgerührt. Zur Aufarbeitung wurde der Ansatz auf 425 ml gesättigte wässrige Natriumhydrogensulfit-Lösung gegossen und 15 Minuten gerührt. Dann wurden 425 ml gesättigte Natriumhydrogencarbonat-Lösung zugegeben und dreimal mit Essigester extrahiert. Die vereinigten organischen Phasen wurden zuerst mit Wasser und dann mit gesättigter wässriger Kochsalzlösung gewaschen, mit Natriumsulfat getrocknet und im Vakuum am Rotationsverdampfer eingedampft. Das Rohprodukt wurde in zwei Portionen über eine Kartusche mit 120 g Kieselgel und einem Gradienten mit Cyclohexan/Essigester von 95:5 bis 85:15 (v/v) chromatographiert. Es wurden 19,5 g 4-Heptafluorisopropyl-2-methyl-6-trifluormethyl-anilin erhalten.
HPLC-MS^{a)}: logP = 4,67
GC/MS: Masse (m/z) = 343, Retentionszeit: 2,98 min, Kovats Index: 1089
(Agilent 6890 GC, HP5979 MSD, 10m DB-1, iD=0.18mm, FILM=0.4µm, Inj.:250°C, const. flow: 1.6mm/min He, Det.:MSD:280°C, FID: 320°C, Oven:50°C(1 min) - 40°C/min - 320°C (3.25 min)) ¹H-NMR (AV400, 400 MHz, d₃-Acetonitril): δ (ppm) = 7,50 (s, 1 H), 7,48 (s, 1H), 5,03 (s, 2H, breit), 2,23 (s, 3 H).

### Herstellung des Ausgangsprodukts 2-Chlor-6-ethyl-4-heptafluorisopropyl-anilin

Das Ausgangsprodukt 2-Chlor-6-ethyl-4-heptafluorisopropyl-anilin der Struktur (**D-1b**) ist bisher in der Literatur noch nicht beschrieben. Die Herstellung kann aus literaturbekanntem 2-Ethyl-4-heptafluorisopropyl-anilin (z.B. US2002/198399) mittels bekannter chlorierender Verfahren erfolgen.

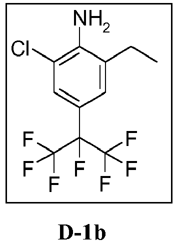

4,9 g (16,9 mmol) 2-Ethyl-4-heptafluorisopropyl-anilin (hergestellt nach US2002/198399) wurden in 100ml Chloroform vorgelegt, auf 45 - 50 °C erwärmt und dann 2,18 ml (26,7 mmol) Sulfurylchlorid, gelöst in in 400 ml Chloroform, langsam zugetropft. Den Ansatz wurde über Nacht bei 50°C gerührt danach wurden weitere 0,34 ml (4,2 mmol) Sulfurylchlorid gelöst in 2 ml Chloroform zugetropft und weitere 3 Stunden bei 50 °C gerührt. Danach wurde abgekühlt und das Lösungsmittel im Vakuum am Rotationsverdampfer abgezogen. Der Rückstand in Dichlormethan aufgenommen, erst mit Natriumhydrogensulfit und dann mit verdünnter Natronlauge gewaschen, mit Natriumsulfat getrocknet und das Lösungmittel im Vakuum am Rotationsverdampfer abdestilliert. Zur Reinigung wurde über eine Kartusche mit 120g Kieselgel mit einem Gradienten ausgehend von reinem Cyclohexan bis Cyclohexan/Ethylacetat 90:10 (v/v) chromatographiert. Es wurden 4,25 g 2-Chlor-6-ethyl-4-heptafluorisopropyl-anilin erhalten.
HPLC-MS^{a)}: logP = 4,67, Masse (m/z) = 324 [M+H]+.
¹H-NMR (AV400, 400 MHz, d₃-Acetonitril): δ (ppm) = 7,84 (s, 1 H), 7,82 (s, 1H), 7,53-7,56 (s, 2H, breit), 2,37 (q, J = 7,6 Hz, 2 H), 1,06 (t, J = 7,6 Hz, 3 H).

### Herstellung des Ausgangsprodukts 2-Brom-6-methyl-4-heptafluorisopropyl-anilin

Das Ausgangsprodukt 2-Brom-6-methyl-4-heptafluorisopropyl-anilin der Struktur (**D-1c**) ist bisher in der Literatur noch nicht beschrieben. Die Herstellung kann aus literaturbekanntem 2-Methyl-4-heptafluorisopropyl-anilin (z.B. US2004/92762) mittels bekannter bromierender Verfahren (z.B. EP2319830, S. 327) erfolgen.

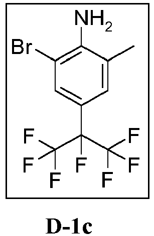

3,4g (12,356 mmol) 2-Methyl-4-heptafluorisopropyl-anilin wurden in 27 ml Dimethylformamid gelöst,dann 2,44g (13,6 mmol) N-Bromsuccinimid zugegeben und 1 Stunde bei 60°C gerührt. Der Ansatz wurde abgekühlt, mit Wasser versetzt und dreimal mit je 15 ml n-Hexan extrahiert. Die vereingten organischen Phasen wurden mit Wasser gewaschen, mit Natriumsulfat getrocknet und im Vakuum am Rotationsverdampfer eingedampft. Chromatographie über eine 120g Kartusche mit Kieselgel mit einen Gradienten beginnend mit reinem Cyclohexan nach Cyclohexan/Ethylacetat 90:10 (v/v) liefert 2,44 g 2-Brom-6-ethyl-4-heptafluorisopropyl-anilin.
HPLC-MS^{a)}: logP = 4,38, Masse (m/z) = 354 [M+H]+.
¹H-NMR (AV400, 400 MHz, d₃-Acetonitril): δ (ppm) = 7,51 (s, 1 H), 7,23 (s, 1H), 4,86 (s, 2H, breit), 2,23 (s, 3 H).

### Herstellung der Ausgangsverbindung 2-(3,5-Dichlor-4-hydrazino-phenyl)-1,1,1,3,3,3-hexafluorpropan-2-ol

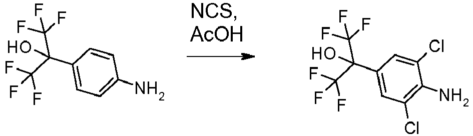

Zu einer Lösung von 2-(4-Aminophenyl)-1,1,1,3,3,3-hexafluorpropan-2-ol (2.50 g, 9.64 mmol) (Herstellung z.B. W. A. Sheppard, J. Am. Chem. Soc. 1965, 87, 2410-2420) in Eisessig (40 mL) wurde bei RT *N*-Chlorsuccinimid (2.71 g, 20.2 mmol) gegeben. Das Gemisch wurde 3 h bei 75°C und anschließend 14 h bei RT gerührt. Anschließend wurde das Gemisch auf Wasser gegeben und mit EtOAc extrahiert. Die organischen Phase wurde mit Wasser und gesättigter wässriger NaHCO₃-Lösung gewaschen und über Magnesiumsulfat getrocknet. Nach entfernen des Lösungsmittels wurde der Rückstand in MTBE aufgenommen und vom Feststoff abfiltriert. Das Filtrat wurde unter vermindertem Druck eingeengt und das Rohprodukt mittels Säulenchromatographie an SiO2 (*n*-Hexan/EtOAc-Gradient) aufgereinigt. Es wurden 2.89 g (91%) von 2-(4-Amino-3,5-dichlorphenyl)-1,1,1,3,3,3-hexafluorpropan-2-ol erhalten.
HPLC-MS^{a)}: logP = 3.04, Masse (m/z) = 328 [M+H]⁺.
¹H-NMR (400 MHz, d₃-Acetonitril): δ = 5.13 (br s, 2 H), 6.02 (br s, 1 H), 7.51 (s, 2 H).

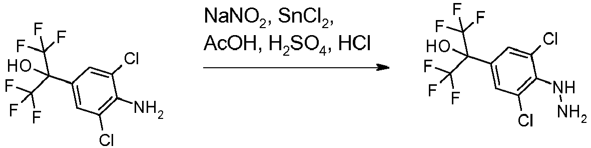

Zu einer auf 55°C erhitzen Lösung von 2-(4-Amino-3,5-dichlorphenyl)-1,1,1,3,3,3-hexafluorpropan-2-ol (1.88 g, 5.73 mmol) in 5 mL Eisessig wurde eine Lösung von Natriumnitrit (455 mg, 6.59 mmol) in 2.5 mL Schwefelsäure getropft und das Gemisch für eine weitere Stunde bei dieser Temperatur gerührt. Anschließend wurde das Gemisch auf 0°C gekühlt und tropfenweise mit einer Lösung von Zinn(II)chlorid (3.37 g, 17.7 mmol) in konz. HCl (10 mL) versetzt. Das Gemisch rührte für eine weitere Stunde bei 0°C, wurde danach auf Eis gegeben, mit Natronlauge alkalisch gestellt und mit EtOAc extrahiert. Die organische Phase wurde mit gesättigter Natriumchloridlösung gewaschen, über Magnesiumsulfat getrocknet und das Lösungsmittel unter vermindertem Druck entfernt. Es wurden 1.41 g (90%ig, 64% d. Th.) 2-(3,5-Dichlor-4-hydrazinophenyl)-1,1,1,3,3,3-hexafluorpropan-2-ol erhalten.
HPLC-MS^{a)}: logP = 1.92, Masse (m/z) = 343 [M+H]⁺.
¹H-NMR (600 MHz, d₃-Acetonitril): δ = 4.14 (br s, 2 H), 5.90 (br s, 1 H), 6.50 (br s, 1 H), 7.58 (s, 2 H).

### Beispiele I-T46-1

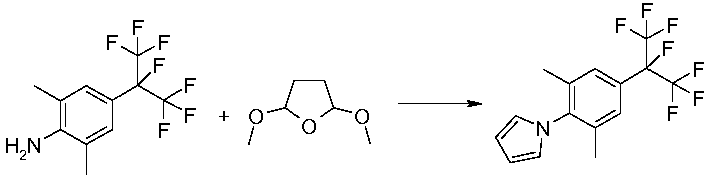

10 g (34,6mmol) 2,6-Dimethyl-4-[1,2,2,2-tetrafluor-1-(trifluormethyl)ethyl]anilin wurden in 60 ml Eisessig vorgelegt und 5,02 g (38,04 mmol) 2,6-Dimethoxy-tetrahydrofuran zugegeben. Die entstandene Lösung wurde für zwei Stunden bei 120 °C erhitzt. Anschliessend wurde etwas abgekühlt und die flüchtigen Bestandteile am Rotationsverdampfer im Vakuum abgedampft. Der Rückstand wurde mit Wasser verrührt und der Feststoff abgesaugt. Der Filterkuchen wurde dann in Dichlormethan gelöst, die Lösung mit Natriumsulfat getrocknet und am Rotationsverdampfer im Vakuum eingedampft. Es wurden 10,38 g 1-[2,6-Dimethyl-4-[1,2,2,2-tetrafluor-1-(trifluormethyl)ethyl]phenyl]pyrrol erhalten.

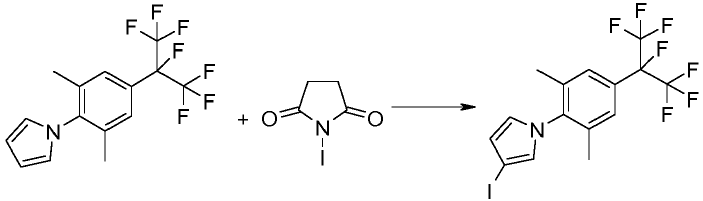

1,5 g (4,293mmol) 1-[2,6-Dimethyl-4-[1,2,2,2-tetrafluor-1-(trifluormethyl)ethyl]phenyl]pyrrol wurden in 60 ml n-Hexan gelöst und 966 mg (4,3 mmol) N-Iodsuccinimid zugegeben. Anschliessend wurde die Mischung auf Raumtemperatur kommen gelassen und 6 Tage bei Raumtemperatur gerührt. Danach wurden weitere 242 mg (1,1 mmol) N-Iodsuccinimid zugegeben und über Nacht bei Raumtemeperatur gerührt. Anschliessend wurde überschüssige wässrige Natriumhydrogensulfit-Lösung und etwas Essigester zugegeben. Die organische Phase wurde abgetrennt und erst zweimal mit wässriger Natriumhydrogensulfit-Lösung, dann mit gesättigter Kochsalzlösung gewaschen, mit Natriumsulfat getrocknet und eingedampft. Zur Reinigung wurde über eine Kartusche mit 120 g Kieselgel und einem Gradienten ausgehend von reinem Cyclohexan bis Cyclohexan/Essigester 95:5 (v/v) chromatographiert. Es wurden 453 mg eines Gemischs aus 80 % 1-[2,6-Dimethyl-4-[1,2,2,2-tetrafluor-1-(trifluormethyl)ethyl]phenyl]-3-iod-pyrrol und 16 % 1-[2,6-Dimethyl-4-[1,2,2,2-tetrafluor-1-(trifluormethyl)ethyl]phenyl]-2-iod-pyrrol erhalten.

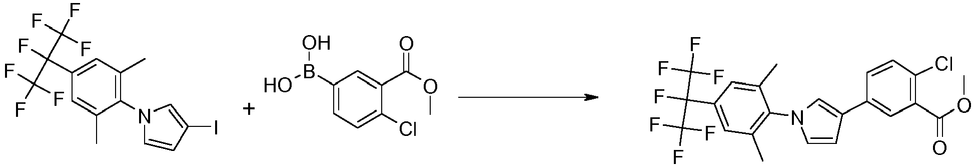

998 mg (1,696 mmol) eines Gemischs aus 80 % 1-[2,6-Dimethyl-4-[1,2,2,2-tetrafluor-1-(trifluormethyl)ethyl]phenyl]-3-iod-pyrrol und 16 % 1-[2,6-Dimethyl-4-[1,2,2,2-tetrafluor-1-(trifluormethyl)ethyl]phenyl]-2-iod-pyrrol und 364 mg (1,7 mmol) 4-Chloro-3-(methoxycarbonyl)phenylboronsäure wurden in 10 ml 2-Propanol vorgelegt. Danach wurde die Luft durch Argon verdrängt und 5,2 ml 1 molare wässrige Natriumhydrogencarbonat-Lösung und 98 mg (0,085 mmol) Tetrakis-triphenylphospin-palladium(O) zugegeben unter Argon zugegeben. Anschliessend wurde 3 Stunden zum Rückfluss erhitzt. Zur Aufarbeitung wurde etwas abgekühlt, dann die Mischung am Rotationsverdampfer im Vakuum eingedampft. Der Rückstand wurde zwischen Essigester und Wasser verteilt. Die organische Phase wurde abgetrennt, mit gesättigter Kochsalzlösung gewaschen und im Vakuum am Rotationsverdampfer eingedampft. Es wurden 1,57 g an rohem 2-Chlor-5-[1-[2,6-dimethyl-4-[1,2,2,2-tetrafluor-1-(trifluormethyl)ethyl]phenyl]pyrrol-3-yl]benzoesäuremethylester.

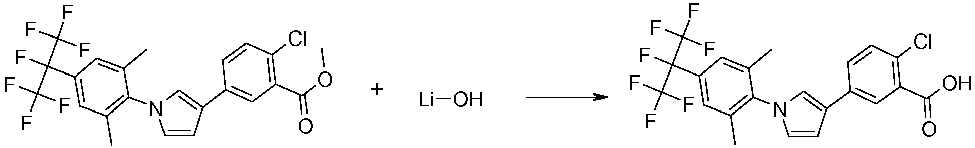

416 mg (0,33 mmol, ca. 40 %ig) roher 2-Chlor-5-[1-[2,6-dimethyl-4-[1,2,2,2-tetrafluor-1-(trifluormethyl)ethyl]phenyl]pyrrol-3-yl]benzoesäuremethylester werden in einem Gemisch aus 18 ml Dioxan und 6 ml Wasser vorgelegt und 61 mg (1,46 mmol) Lithiumhydroxid Hydrat zugefügt. Es wurde bis zur vollständigen Lösung bei Raumtemperatur gerührt, dann 2 Stunden unter Rückfluss erhitzt. anschliessend wurde im Vakuum am Rotationsverdampfer eingedampft, der Rückstand mit etwas Wasser versetzt und mit konzentrierter Salzsäure auf pH 1 gestellt. Es wurde dann zweimal mit Essigester extrahiert, die vereinigten Extrakte mit gesättigter Kochsalzlösung gewaschen, mit Natriumsulfat getrocknet und eingedampft. Als Rückstand verblieben 207 mg rohe 2-Chlor-5-[1-[2,6-dimethyl-4-[1,2,2,2-tetrafluor-1-(trifluormethyl)ethyl]phenyl]pyrrol-3-yl]benzoesäure.

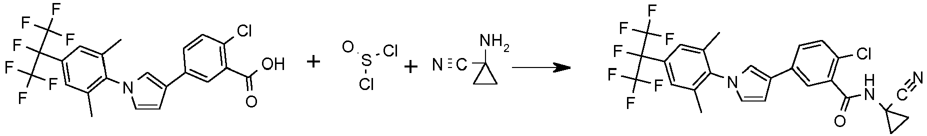

137 mg (0,11 mmol, Reinheit ca. 38 %) rohe 2-Chlor-5-[1-[2,6-dimethyl-4-[1,2,2,2-tetrafluor-1-(trifluormethyl)ethyl]phenyl]pyrrol-3-yl]benzoesäure wurden in 15 ml Toluol gelöst und 230 mg (1,93 mmol) Thionylchlorid zugegeben.Die Mischung wurde 3 Stunden zum Rückfluss erhitzt. Danach wurden alle flüchtigen Anteile am Rotationsverdampfer im Vakuum abgezogen. Der Rückstand wurde in 4 ml Dichlormethan aufgenommen und zu einer Mischung aus 82 mg (0,69 mmol) 1-Cyancyclopropylamin Hydrochlorid und 98 mg (0,96 mmol) Triethylamin in 2 ml Dichlormethan bei 0 °C zugetropft. Anschliessend wurde über Nacht bei Raumtemperatur gerührt. Zur Aufarbeitung wurde mit 5 %iger wässriger Natriumdihydrogenphosphat-Lösung, danach mit gesättigter Kochsalzlösung gewaschen, die organische Phase mit Natriumsulfat getrocknet und eingedampft. Der Rückstand wurde über eine Kartusche mit 15 g Kieselgel und Cyclohexan/Essigester 85:15 (v/v) chromatographiert. Die das Produkt enthaltenden Fraktionen wurden eingedampft und über präparative HPLC (Zorbax Eclipse Plus C18 1,8 µm, 50x4,6mm in einem Gradienten in Acetonitril/0,1%ige wässrige H₃PO₄ gereinigt. Es wurden 13 mg 2-Chlor-N-cyclopropyl-5-[1-[2,6-dimethyl-4-[1,2,2,2-tetrafluor-1-(trifluormethyl)ethyl]phenyl]pyrrol-3-yl]benzamid (Verbindung **I-T46-1**) erhalten.
HPLC-MS^{a)}: logP = 4,90, Masse (m/z) = 558 [M+H]+.
¹H-NMR (400 MHz, d₃-Acetonitril): δ = 7,63-7,67 (m, 2 H), 7,56 (s (breit), 1 H (N-H)), 7,51 (s, 2H), 7,41 (d, J=8,3 Hz, 1 H), 7,16-7,17 (m, 1 H), 6,75-6,77 (m, 1 H), 6,72-6,73 (m, 1 H), 2,14 (s, 6H), 1,55-1,59 (m, 2 H), 1,32-1,39 (m, 2 H).

**Tabelle I-T2**

| | | | | | | | | | | | | | | |
|---|---|---|---|---|---|---|---|---|---|---|---|---|---|---|
| 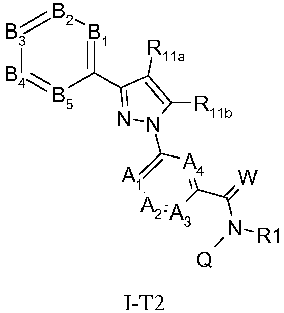 | | | | | | | | | | | | | | |

| B₂ und B₄ = C-H, W = O | | | | | | | | | | | | | | |
|---|---|---|---|---|---|---|---|---|---|---|---|---|---|---|
| **Bsp.-Nr.** | **B₁** | **B₃** | **B₅** | **R₁** | **R₁₁ₐ** | **R_{11b}** | **A₁** | **A₂** | **A₃** | **A₄** | **Q** | | **logP^{a)}** | **Masse [m/z]^{a)1)}** |
| I-T2-1 | C-CH₃ | C-i-C₃F₇ | C-CH₃ | H | H | H | C-H | C-H | C-Cl | C-H | cyclopropyl | | 4,9 | 534 |
| I-T2-2 | C-CH₃ | C-i-C₃F₇ | C-CH₃ | H | H | H | C-H | C-H | C-Cl | C-H | 1-(cyano)cyclopropyl | | 4,8 | 559 |

| **Bsp.-Nr.** | **B₁** | **B₃** | **B₅** | **R^{11a}** | **R^{11b}** | **A₁** | **A₂** | **A₃** | **A₄** | **W** | **R1** | **Q** | **logP^{a)}** | **Masse [m/z] a)1)** |
|---|---|---|---|---|---|---|---|---|---|---|---|---|---|---|
| I-T3-1 | C-CH₃ | C-i-C₃F₇ | C-CH₃ | H | H | C-H | C-H | C-Cl | C-H | O | H | cyclopropyl | 4,4 | 534 |
| I-T3-2 | C-CH₃ | C-i-C₃F₇ | C-CH₃ | H | H | C-H | C-H | C-Cl | C-H | O | H | CH₂CF₃ | 4,7 | 577 |
| I-T3-3 | C-CH₃ | C-i-C₃F₇ | C-CH₃ | H | H | C-H | C-H | C-Cl | C-H | O | H | 1-(cyano)cyclopropyl | 4,2 | 559 |
| I-T3-4 | C-CH₃ | C-i-C₃F₇ | C-CH₃ | H | H | C-H | C-H | C-Cl | C-H | O | H | Thietan-3-yl | 4,7 | 566 |
| I-T3-5 | C-CH₃ | C-i-C₃F₇ | C-CH₃ | H | H | C-H | C-H | C-Cl | C-H | O | H | 1-(trifluoromethyl)-cyclopropyl | 4,6 | 602 |
| I-T3-6 | C-CH₃ | C-i-C₃F₇ | C-CH₃ | H | H | C-H | C-H | C-Cl | C-H | O | H | 2-oxo-2-(2,2,2-trifluoroethylamino)-ethyl | 4,1 | 633 |
| I-T3-7 | C-CH₃ | C-i-C₃F₇ | C-CH₃ | H | H | C-H | N | C-H | C-H | O | H | cyclopropyl | 3,4 | 501 |
| I-T3-8 | C-CH₃ | C-i-C₃F₇ | C-CH₃ | H | H | C-H | N | C-H | C-H | O | H | 1-(cyano)cyclopropyl | 3,4 | 526 |
| I-T3-9 | C-CH₃ | C-i-C₃F₇ | C-CH₃ | H | H | C-H | C-H | C-H | CF | O | H | cyclopropyl | 4,3 | 518 |
| I-T3-10 | C-CH₃ | C-i-C₃F₇ | C-CH₃ | H | H | C-H | C-H | C-H | CF | O | H | 1-(cyano)cyclopropyl | 4,2 | 543 |
| I-T3-11 | C-CH₃ | C-i-C₃F₇ | C-CH₃ | H | H | C-Cl | C-H | C-F | C-H | O | H | cyclopropyl | 4,9 | 552 |
| I-T3-12 | C-CH₃ | C-i-C₃F₇ | C-CH₃ | H | H | C-Cl | C-H | C-F | C-H | O | H | 1-(cyano)cyclopropyl | 4,7 | 577 |
| I-T3-13 | C-CH₃ | C-i-C₃F₇ | C-CH₃ | H | H | CF | C-H | C-H | C-H | O | H | cyclopropyl | 4,5 | 518 |
| I-T3-14 | C-CH₃ | C-i-C₃F₇ | C-CH₃ | H | H | CF | C-H | C-Cl | C-H | O | H | cyclopropyl | 4,5 | 552 |
| I-T3-15 | C-CH₃ | C-i-C₃F₇ | C-CH₃ | H | H | C-H | C-CF₃ | C-H | C-H | O | H | cyclopropyl | 4,9 | 568 |
| I-T3-16 | C-CH₃ | C-i-C₃F₇ | C-CH₃ | H | H | CH₃ | C-H | C-H | C-H | O | H | cyclopropyl | 4,4 | 514 |
| I-T3-17 | C-CH₃ | C-i-C₃F₇ | C-CH₃ | H | H | CF | C-H | C-Cl | C-H | O | H | 1-(cyano)cyclopropyl | 4,4 | 577 |
| I-T3-18 | C-CH₃ | C-i-C₃F₇ | C-CH₃ | H | H | C-H | C-CF₃ | C-H | C-H | O | H | 1-(cyano)cyclopropyl | 4,7 | 593 |
| I-T3-19 | C-CH₃ | C-i-C₃F₇ | C-CH₃ | H | H | CF | C-H | C-H | C-H | O | H | 1-(cyano)cyclopropyl | 4,3 | 543 |
| I-T3-20 | C-CH₃ | C-i-C₃F₇ | C-CH₃ | H | H | C-H | N | C-Cl | C-H | O | H | cyclopropyl | 3,8 | 535 |
| I-T3-21 | C-CH₃ | C-i-C₃F₇ | C-CH₃ | H | H | C-H | N | C-Cl | C-H | O | H | 1-(cyano)cyclopropyl | 3,7 | 560 |
| I-T3-22 | C-Cl | C-i-C₃F₇ | C-Cl | H | H | C-H | C-H | C-H | C-F | O | H | 1-(cyano)cyclopropyl | 4,1 | 583 |
| I-T3-23 | C-CH₃ | C-i-C₃F₇ | C-CH₃ | H | H | C-H | C-H | C-Cl | C-H | O | H | 1-(cyano)cyclopropyl | 4,2 | 599 |
| I-T3-24 | C-CH₃ | C-i-C₃F₇ | C-CH₃ | H | H | C-H | C-H | C-Cl | C-H | O | H | cyclopropyl | 4,3 | 574 |
| I-T3-25 | C-CH₃ | C-i-C₃F₇ | C-CH₃ | H | H | C-H | C-H | C-Cl | C-H | O | H | CH₂CF₃ | 4,7 | 616 |
| I-T3-26 | C-CH₃ | C-i-C₃F₇ | C-CH₃ | H | H | C-H | C-H | C-Cl | C-H | O | H | CH₂CH₂CF₃ | 4,7 | 630 |
| I-T3-27 | C-CH₃ | C-CF₃ | C-CH₃ | H | H | C-H | C-H | C-Cl | H | O | H | 1-(cyano)cyclopropyl | 3,4 | 499 |
| I-T3-28 | C-CH₃ | C-CF₃ | C-CH₃ | H | H | C-H | C-H | C-Cl | H | O | H | cyclopropyl | 3,5 | 474 |
| I-T3-29 | C-CH₃ | C-i-C₃F₇ | C-CH₃ | H | H | C-H | N | C-H | C-H | O | H | cyclopropyl | 3,3 | 541 |
| I-T3-30 | C-CH₃ | C-i-C₃F₇ | C-CH₃ | H | H | C-H | N | C-H | C-H | O | H | 1-(cyano)cyclopropyl | 3,2 | 566 |
| I-T3-31 | C-Cl | C-i-C₃F₇ | C-H | H | H | C-H | C-H | C-Cl | C-H | O | H | cyclopropyl | 4,3 | 540 |
| I-T3-32 | C-Cl | C-i-C₃F₇ | C-H | H | H | C-H | C-H | C-Cl | C-H | O | H | CH₂CF₃ | 4,7 | 582 |
| I-T3-33 | C-Cl | C-i-C₃F₇ | C-H | H | H | C-H | C-H | C-Cl | C-H | O | H | CH₂CH₂CF₃ | 4,7 | 596 |
| I-T3-34 | C-OCF ₃ | C-i-C₃F₇ | C-H | H | H | C-H | C-H | C-Cl | C-H | O | H | 1-(cyano)cyclopropyl | 4,6 | 615 |
| I-T3-35 | C-OCF ₃ | C-i-C₃F₇ | C-H | H | H | C-H | C-H | C-Cl | C-H | O | H | cyclopropyl | 4,7 | 590 |
| I-T3-36 | C-OCF ₃ | C-i-C₃F₇ | C-H | H | H | C-H | C-H | C-Cl | C-H | O | H | CH₂CF₃ | 5,0 | 632 |
| I-T3-37 | C-OCF ₃ | C-i-C₃F₇ | C-H | H | H | C-H | C-H | C-Cl | C-H | O | H | Thietan-3-yl | 4,9 | 622 |
| I-T3-38 | C-C₂H₅ | C-i-C₃F₇ | C-H | H | H | C-H | C-H | C-Cl | C-H | O | H | 1-(cyano)cyclopropyl | 4,5 | 559 |
| I-T3-39 | C-C₂H₅ | C-i-C₃F₇ | C-H | H | H | C-H | C-H | C-Cl | C-H | O | H | cyclopropyl | 4,6 | 534 |
| I-T3-40 | C-C₂H₅ | C-i-C₃F₇ | C-H | H | H | C-H | C-H | C-Cl | C-H | O | H | CH₂CF₃ | 4,9 | 576 |
| I-T3-41 | C-C₂H₅ | C-i-C₃F₇ | C-H | H | H | C-H | C-H | C-Cl | C-H | O | H | Thietan-3-yl | 4,9 | 566 |
| I-T3-42 | C-Cl | C-i-C₃F₇ | C-Cl | H | H | C-H | N | C-Cl | C-H | O | H | cyclopropyl | 3,8 | 575 |
| I-T3-43 | C-Cl | C-i-C₃F₇ | C-Cl | H | H | C-H | N | C-Cl | C-H | O | H | 1-(cyano)cyclopropyl | 3,8 | 600 |
| I-T3-44 | C-C₂H₅ | C-i-C₃F₇ | C-CH₃ | H | H | C-H | C-H | C-Cl | C-H | O | H | cyclopropyl | 4,7 | 548 |
| I-T3-45 | C-C₂H₅ | C-i-C₃F₇ | C-CH₃ | H | H | C-H | C-H | C-Cl | C-H | O | H | CH₂CF₃ | 5,0 | 590 |
| I-T3-46 | C-C₂H₅ | C-i-C₃F₇ | C-CH₃ | H | H | C-H | C-H | C-Cl | C-H | O | H | 1-(cyano)cyclopropyl | 4,6 | 573 |
| I-T3-47 | C-C₂H₅ | C-i-C₃F₇ | C-CH₃ | H | H | C-H | C-H | C-Cl | C-H | O | H | Thietan-3-yl | 5,0 | 580 |
| I-T3-48 | C-F | C-CF₃ | C-F | H | H | C-H | C-H | C-Cl | C-H | O | H | cyclopropyl | 3,2 | 442 |
| I-T3-49 | C-F | C-CF₃ | C-F | H | H | C-H | C-H | C-Cl | C-H | O | H | 1-(cyano)cyclopropyl | 3,2 | 467 |
| I-T3-50 | C-F | C-CF₃ | C-OCH₃ | H | H | C-H | C-H | C-Cl | C-H | O | H | cyclopropyl | 3,1 | 454 |
| I-T3-51 | C-F | C-CF₃ | C-OCH₃ | H | H | C-H | C-H | C-Cl | C-H | O | H | 1-(cyano)cyclopropyl | 3,1 | 479 |
| I-T3-52 | C-CF₃ | C-i-C₃F₇ | C-H | H | H | C-H | C-H | C-Cl | C-H | O | H | cyclopropyl | 4,3 | 574 |
| I-T3-53 | C-CF₃ | C-i-C₃F₇ | C-H | H | H | C-H | C-H | C-Cl | C-H | O | H | 1-(cyano)cyclopropyl | 4,3 | 599 |
| I-T3-54 | C-CF₃ | C-i-C₃F₇ | C-CH₃ | H | H | C-H | C-H | C-Cl | C-H | O | H | cyclopropyl | 4,4 | 588 |
| I-T3-55 | C-CF₃ | C-i-C₃F₇ | C-CH₃ | H | H | C-H | C-H | C-Cl | C-H | O | H | 1-(cyano)cyclopropyl | 4,3 | 613 |
| I-T3-56 | C-CF₃ | C-i-C₃F₇ | C-Cl | H | H | C-H | C-H | C-Cl | C-H | O | H | cyclopropyl | 4,4 | 608 |
| I-T3-57 | C-CF₃ | C-i-C₃F₇ | C-Cl | H | H | C-H | C-H | C-Cl | C-H | O | H | 1-(cyano)cyclopropyl | 4,3 | 633 |
| I-T3-58 | C-CF₃ | C-i-C₃F₇ | C-Cl | H | H | C-H | C-H | C-Cl | C-H | O | H | CH₂CF₃ | 4,7 | 650 |
| I-T3-59 | C-Cl | C-CF₃ | N | H | H | C-H | C-H | C-Cl | C-H | O | H | 1-(cyano)cyclopropyl | 3,1 | 466 |
| I-T3-60 | C-Cl | C-CF₃ | N | H | H | C-H | C-H | C-Cl | C-H | O | H | cyclopropyl | 3,2 | 441 |
| I-T3-61 | C-F | C-i-C₃F₇ | C-F | H | H | C-H | C-H | C-Cl | C-H | O | H | cyclopropyl | 4,0 | 542 |
| I-T3-62 | C-F | C-i-C₃F₇ | C-F | H | H | C-H | C-H | C-Cl | C-H | O | H | 1-(cyano)cyclopropyl | 3,9 | 567 |
| I-T3-63 | C-CH₃ | C-i-C₃F₇ | C-Cl | H | H | C-H | C-H | C-Cl | C-H | O | H | cyclopropyl | 4,4 | 554 |
| I-T3-64 | C-CH₃ | C-i-C₃F₇ | C-Cl | H | H | C-H | C-H | C-Cl | C-H | O | H | 1-(cyano)cyclopropyl | 4,2 | 579 |
| I-T3-65 | C-CH₃ | C-i-C₃F₇ | C-CH₃ | H | H | C-H | C-H | C-Cl | C-F | O | H | 1-(cyano)cyclopropyl | 4,4 | 577 |
| I-T3-66 | C-C₂H₅ | C-i-C₃F₇ | C-CH₃ | H | H | C-H | C-H | C-Cl | C-F | O | H | 1-(cyano)cyclopropyl | 4,6 | 591 |
| I-T3-67 | C-CH₃ | C-i-C₃F₇ | C-CH₃ | H | H | C-H | C-H | C-Cl | C-F | O | H | cyclopropyl | 4,4 | 552 |
| I-T3-68 | C-C₂H₅ | C-i-C₃F₇ | C-CH₃ | H | H | C-H | C-H | C-Cl | C-F | O | H | cyclopropyl | 4,6 | 566 |
| I-T3-69 | C-C₂H₅ | C-i-C₃F₇ | C-CH₃ | H | H | C-H | C-H | C-H | C-F | O | H | 1-(cyano)cyclopropyl | 4,3 | 557 |
| I-T3-70 | C-C₂H₅ | C-i-C₃F₇ | C-CH₃ | H | H | C-H | C-H | C-H | C-F | O | H | cyclopropyl | 4,3 | 532 |
| I-T3-71 | C-C₂H₅ | C-i-C₃F₇ | C-Cl | H | H | C-H | C-H | C-Cl | C-H | O | H | 1-(cyano)cyclopropyl | 4,5 | 593 |
| I-T3-72 | C-C₂H₅ | C-i-C₃F₇ | C-Cl | H | H | C-H | C-H | C-Cl | C-H | O | H | cyclopropyl | 4,6 | 568 |
| I-T3-73 | C-Cl | C-i-C₃F₇ | C-Cl | H | H | C-H | C-H | C-Cl | C-F | O | H | cyclopropyl | 4,5 | 592 |
| I-T3-74 | C-C₂H₅ | C-i-C₃F₇ | C-H | H | H | C-H | C-H | C-H | C-F | O | H | 1-(cyano)cyclopropyl | 4,1 | 543 |
| I-T3-75 | C-C₂H₅ | C-i-C₃F₇ | C-H | H | H | C-H | C-H | C-H | C-F | O | H | cyclopropyl | 4,2 | 518 |
| I-T3-76 | C-C₂H₅ | C-i-C₃F₇ | C-CH₃ | H | H | C-H | N | C-H | C-H | O | H | 1-(cyano)cyclopropyl | 3,4 | 540 |
| I-T3-77 | C-C₂H₅ | C-i-C₃F₇ | C-CH₃ | H | H | C-H | N | C-H | C-H | O | H | cyclopropyl | 3,4 | 515 |
| I-T3-78 | C-C₂H₅ | C-i-C₃F₇ | C-H | H | H | C-H | N | C-H | C-H | O | H | 1-(cyano)cyclopropyl | 3,2 | 526 |
| I-T3-79 | C-C₂H₅ | C-i-C₃F₇ | C-H | H | H | C-H | N | C-H | C-H | O | H | cyclopropyl | 3,3 | 501 |
| I-T3-80 | C-C₂H₅ | C-C₂F₅ | C-CH₃ | H | H | C-H | C-H | C-Cl | C-H | O | H | 1-(cyano)cyclopropyl | 4,2 | 523 |
| I-T3-81 | C-C₂H₅ | C-C₂F₅ | C-CH₃ | H | H | C-H | C-H | C-Cl | C-H | O | H | cyclopropyl | 4,3 | 498 |
| I-T3-82 | C-C₂H₅ | C-C₂F₅ | C-CH₃ | H | H | C-H | C-H | C-Cl | C-H | O | H | CH₂CF₃ | 4,0 | 540 |
| I-T3-83 | C-C₂H₅ | C-C₂F₅ | C-CH₃ | H | H | C-H | C-H | C-Cl | C-H | O | H | Thietan-3-yl | 4,6 | 530 |
| I-T3-84 | C-CF₃ | C-i-C₃F₇ | C-CH₃ | H | H | C-H | C-H | C-Cl | C-H | O | H | CH₂CF₃ | 4,7 | 630 |
| I-T3-85 | C-CF₃ | C-i-C₃F₇ | C-CH₃ | H | H | C-H | C-H | C-Cl | C-H | O | H | Thietan-3-yl | 4,7 | 620 |
| I-T3-86 | C-CH₃ | C-i-C₃F₇ | C-Br | H | H | C-H | C-H | C-Cl | C-H | O | H | cyclopropyl | 4,5 | 598 |
| I-T3-87 | C-Cl | C-OCF₃ | C-Cl | H | H | C-H | C-H | C-Cl | C-H | O | H | 1-(cyano)cyclopropyl | 3,6 | 515 |
| I-T3-88 | C-Cl | C-OCF ₃ | C-Cl | H | H | C-H | C-H | C-Cl | C-H | O | H | cyclopropyl | 3,6 | 490 |
| I-T3-89 | C-Cl | C-OCF₃ | C-H | H | H | C-H | C-H | C-Cl | C-H | O | H | 1-(cyano)cyclopropyl | 3,5 | 481 |
| I-T3-90 | C-Cl | C-OCF₃ | C-H | H | H | C-H | C-H | C-Cl | C-H | O | H | cyclopropyl | 3,6 | 456 |
| I-T3-91 | C-Cl | C-i-C₃F₇ | C-Cl | H | H | C-H | N | C-CH₃ | C-H | O | H | cyclopropyl | 3,3 | 555 |
| I-T3-92 | C-Cl | C-i-C₃F₇ | C-Cl | H | H | C-H | N | C-CH₃ | C-H | O | H | 1-(cyano)cyclopropyl | 3,4 | 580 |
| I-T3-93 | C-Cl | C-i-C₃F₇ | C-Cl | H | H | C-H | C-H | C-Cl | C-H | O | H | 1-(tert.butyl)cyclopropyl | 4,7 | 588 |
| I-T3-94 | C-CF₃ | C-i-C₃F₇ | C-CH₃ | H | H | C-H | C-H | C-Cl | C-H | O | H | 1-(tert.butyl)cyclopropyl | 4,7 | 602 |
| I-T3-95 | C-Cl | C-CF₃ | C-Cl | H | H | C-H | N | C-Cl | C-H | O | H | 1-(cyano)cyclopropyl | 3,1 | 500 |
| I-T3-96 | C-CH₃ | C-i-C₃F₇ | C-CH₃ | H | H | C-H | N | C-CH₃ | C-H | O | H | cyclopropyl | 3,3 | 515 |
| I-T3-97 | C-CH₃ | C-i-C₃F₇ | C-CH₃ | H | H | C-H | N | C-CH₃ | C-H | O | H | 1-(cyano)cyclopropyl | 3,4 | 540 |
| I-T3-98 | C-Cl | C-CF₃ | C-Cl | H | H | C-H | N | C-Cl | C-H | O | H | cyclopropyl | 3,1 | 475 |
| I-T3-99 | C-Cl | C-CF₃ | C-Cl | H | H | C-H | N | C-Cl | C-H | O | CH₃ | cyclopropyl | 3,5 | 489 |
| I-T3-100 | C-Cl | C-i-C₃F₇ | C-Cl | H | H | C-H | N | C-Cl | C-H | O | CH₃ | cyclopropyl | 4,4 | 589 |
| I-T3-101 | C-CH₃ | C-i-C₃F₇ | C-CH₃ | H | H | C-H | N | C-Cl | C-H | O | CH₃ | cyclopropyl | 4,4 | 549 |
| I-T3-102 | C-Cl | C-i-C₃F₇ | C-Cl | H | H | C-H | N | C-Cl | C-H | O | CH₃ | 1-(cyano)cyclopropyl | 4,3 | 614 |
| I-T3-103 | C-CH₃ | C-i-C₃F₇ | C-CH₃ | H | H | C-H | N | C-Cl | C-H | O | CH₃ | 1-(cyano)cyclopropyl | 4,3 | 574 |
| I-T3-104 | C-C₂H₅ | C-i-C₃F₈ | C-H | H | H | C-H | N | C-Cl | C-H | O | H | cyclopropyl | 4,2 | 535 |
| I-T3-105 | C-C₂H₅ | C-i-C₃F₈ | C-H | H | H | C-H | N | C-Cl | C-H | O | H | 1-(cyano)cyclopropyl | 4,1 | 560 |
| I-T3-106 | C-CF₃ | C-i-C₃F₇ | C-CH₃ | H | H | C-H | N | C-Cl | C-H | O | H | cyclopropyl | 4,0 | 589 |
| I-T3-107 | C-CF₃ | C-i-C₃F₇ | C-CH₃ | H | H | C-H | N | C-Cl | C-H | O | CH₃ | cyclopropyl | 4,5 | 603 |
| I-T3-108 | C-CF₃ | C-i-C₃F₇ | C-CH₃ | H | H | C-H | N | C-Cl | C-H | O | H | 1-(cyano)cyclopropyl | 4,0 | 614 |
| I-T3-109 | C-C₂H₅ | C-i-C₃F₇ | C-CH₃ | H | H | C-H | N | C-Cl | C-H | O | H | cyclopropyl | 4,2 | 549 |
| I-T3-110 | C-C₂H₅ | C-i-C₃F₇ | C-CH₃ | H | H | C-H | N | C-Cl | C-H | O | H | 1-(cyano)cyclopropyl | 4,1 | 574 |
| I-T3-111 | C-CF₃ | C-i-C₃F₇ | C-CH₃ | H | H | C-H | N | C-Cl | C-H | O | CH₃ | 1-(cyano)cyclopropyl | 4,4 | 628 |
| I-T3-112 | C-CF₃ | C-i-C₃F₇ | C-Cl | H | H | C-H | N | C-Cl | C-H | O | H | cyclopropyl | 4,0 | 609 |
| I-T3-113 | C-CF₃ | C-i-C₃F₇ | C-Cl | H | H | C-H | N | C-Cl | C-H | O | CH₃ | cyclopropyl | 4,0 | 623 |
| I-T3-114 | C-CF₃ | C-i-C₃F₇ | C-Cl | H | H | C-H | N | C-Cl | C-H | O | H | 1-(cyano)cyclopropyl | 4,0 | 634 |
| I-T3-115 | C-CH₃ | C-i-C₃F₇ | C-CH₃ | H | H | C-H | N | C-CH₃ | C-H | O | CH₃ | 1-(cyano)cyclopropyl | 3,8 | 554 |
| I-T3-116 | C-CF₃ | C-i-C₃F₇ | C-Cl | H | H | C-H | N | C-Cl | C-H | O | CH₃ | 1-(cyano)cyclopropyl | 4,3 | 648 |
| I-T3-117 | C-CF₃ | C-i-C₃F₇ | C-CH₃ | H | H | C-H | N | C-CH₃ | C-H | O | H | cyclopropyl | 3,5 | 569 |
| I-T3-118 | C-CF₃ | C-i-C₃F₇ | C-CH₃ | H | H | C-H | N | C-CH₃ | C-H | O | CH₃ | cyclopropyl | 3,8 | 583 |
| I-T3-119 | C-CF₃ | C-i-C₃F₇ | C-CH₃ | H | H | C-H | N | C-CH₃ | C-H | O | H | 1-(cyano)cyclopropyl | 3,6 | 594 |
| I-T3-120 | C-CH₃ | C-i-C₃F₇ | C-CH₃ | H | H | C-H | N | C-CH₃ | C-H | O | CH₃ | cyclopropyl | 3,6 | 529 |
| I-T3-121 | C-CH₃ | C-i-C₃F₇ | C-CH₃ | H | H | N | C-H | C-Cl | N | O | H | cyclopropyl | 4,5 | 536 |
| I-T3-122 | C-Cl | C-CF₃ | N | H | H | C-H | C-H | C-H | C-H | O | H | cyclopropyl | 3,0 | 407 |
| I-T3-123 | C-CF₃ | C-i-C₃F₇ | C-CH₃ | H | H | C-H | N | C-CH₃ | C-H | O | CH₃ | 1 -(cyano)cyclopropyl | 3,9 | 608 |
| I-T3-124 | C-CF₃ | C-i-C₃F₇ | C-Cl | H | H | C-H | N | C-CH₃ | C-H | O | H | cyclopropyl | 3,5 | 589 |
| I-T3-125 | C-CF₃ | C-i-C₃F₇ | C-Cl | H | H | C-H | N | C-CH₃ | C-H | O | H | 1-(cyano)cyclopropyl | 3,8 | 614 |
| I-T3-126 | C-Cl | C-i-C₃F₇ | C-Cl | CN | NH₂ | C-H | C-H | C-Cl | C-H | O | H | 1-(cyano)cyclopropyl | 4,1 | 639 |
| I-T3-127 | C-Cl | C-CF₃ | C-Cl | H | H | C-H | N | C-CH₃ | C-H | O | H | cyclopropyl | 2,5 | 455 |
| I-T3-128 | C-Cl | C-CF₃ | C-Cl | H | H | C-H | N | C-CH₃ | C-H | O | CH₃ | cyclopropyl | 2,8 | 469 |
| I-T3-129 | C-Cl | C-CF₃ | C-Cl | H | H | C-H | N | C-CH₃ | C-H | O | H | 1-(cyano)cyclopropyl | 2,6 | 480 |
| I-T3-130 | C-CF₃ | C-i-C₃F₇ | C-Cl | H | H | C-H | N | C-CH₃ | C-H | O | CH₃ | cyclopropyl | 3,8 | 603 |
| I-T3-131 | C-CF₃ | C-i-C₃F₇ | C-Cl | H | H | C-H | N | C-CH₃ | C-H | O | CH₃ | 1 -(cyano)cyclopropyl | 3,9 | 628 |
| I-T3-132 | C-Cl | C-CF₃ | C-Cl | H | H | C-H | N | C-CH₃ | C-H | O | CH₃ | 1 -(cyano)cyclopropyl | 2,9 | 494 |
| I-T3-133 | C-Cl | C-i-C₃F₇ | C-Cl | H | H | C-H | N | C-CH₃ | C-H | O | CH₃ | cyclopropyl | 3,7 | 569 |
| I-T3-134 | C-CF₃ | C-i-C₃F₇ | C-H | H | H | C-H | C-H | C-Cl | C-H | O | CH₃ | cyclopropyl | 4,9 | 588 |
| I-T3-135 | C-Cl | C-i-C₃F₇ | C-Cl | H | H | C-H | N | C-(1-pyrrol idinyl ) | C-H | O | H | cyclopropyl | 4,3 | 610 |
| I-T3-136 | C-Cl | C-i-C₃F₇ | C-Cl | H | H | C-H | N | C-NHC H₃ | C-H | O | H | cyclopropyl | 3,5 | 570 |
| I-T3-137 | C-Cl | C-i-C₃F₇ | C-Cl | H | H | C-H | N | C-NHcyclo propy l | C-H | O | H | cyclopropyl | 3,4 | 596 |
| I-T3-138 | C-Cl | C-i-C₃F₇ | C-Cl | H | H | C-H | N | C-NH-CH₂C H₂OC H₃ | C-H | O | H | cyclopropyl | 4,1 | 614 |
| I-T3-139 | C-CH₃ | C-i-C₃F₇ | C-CH₃ | H | H | C-H | N | C-cyclo propyl | C-H | O | H | 1-(cyano)cyclopropyl | 4,1 | 566 |
| I-T3-140 | C-CH₃ | C-i-C₃F₇ | C-CH₃ | H | H | C-H | N | C-cyclo propyl | C-H | O | H | cyclopropyl | 4,2 | 541 |
| I-T3-141 | C-CH₃ | C-i-C₃F₇ | C-CH₃ | H | H | C-H | N | C-CH3 | C-H | O | H | 1-(cyano)cyclopropyl | 4,4 | 556 |
| I-T3-142 | C-CH₃ | C-i-C₃F₇ | C-CH₃ | H | H | C-H | N | C-cyclo propyl | C-H | O | CH₃ | cyclopropyl | 4,6 | 555 |
| I-T3-143 | C-CH₃ | C-i-C₃F₇ | C-CH₃ | H | H | C-H | N | C-OCH₃ | C-H | O | H | cyclopropyl | 4,6 | 531 |
| I-T3-144 | C-CH₃ | C-i-C₃F₇ | C-CH₃ | H | H | C-H | N | C-OCH₃ | C-H | O | CH₃ | cyclopropyl | 4,4 | 545 |
| I-T3-145 | C-Cl | C-CF₃ | C-Cl | H | H | C-H | N | C-Cl | C-H | O | C₂H₅ | cyclopropyl | 3,8 | 503 |
| I-T3-146 | C-Cl | C-CF₃ | C-Cl | H | H | C-H | N | C-Cl | C-H | O | H | N-methyl-pyrazole-3-yl | 2,8 | 515 |
| I-T3-147 | C-Cl | C-CF₃ | C-Cl | H | H | C-H | N | C-CH₃ | C-H | O | H | C₂H₅ | 4,0 | 443 |
| I-T3-148 | C-CF₃ | C-i-C₃F₇ | C-Br | H | H | C-H | C-H | C-Cl | C-H | O | H | cyclopropyl | 4,5 | 652 |
| I-T3-149 | C-CF₃ | C-CF₃ | N | H | H | C-H | C-H | C-Cl | C-H | O | H | 1-(cyano)cyclopropyl | 3,5 | 500 |
| I-T3-150 | C-CF₃ | C-CF₃ | N | H | H | C-H | C-H | C-Cl | C-H | O | H | cyclopropyl | 3,6 | 475 |
| I-T3-151 | C-Cl | C-i-C₃F₇ | N | H | H | C-H | C-H | C-Cl | C-H | O | H | 1-(cyano)cyclopropyl | 4,0 | 566 |
| I-T3-152 | C-Cl | C-i-C₃F₇ | N | H | H | C-H | C-H | C-Cl | C-H | O | H | cyclopropyl | 4,1 | 541 |
| I-T3-153 | C-S(O)C₂ H₅ | C-i-C₃F₇ | N | H | H | C-H | C-H | C-Cl | C-H | O | H | 1-(cyano)cyclopropyl | 3,7 | 608 |
| I-T3-154 | C-S(O₂)C₂ H₅ | C-i-C₃F₇ | N | H | H | C-H | C-H | C-Cl | C-H | O | H | 1-(cyano)cyclopropyl | 3,8 | 624 |
| I-T3-155 | C-CF₃ | C-i-C₃F₇ | C-Br | H | H | C-H | C-H | C-Cl | C-H | O | H | 1-(cyano)cyclopropyl | 4,4 | 677 |
| I-T3-156 | C-SC₂H₅ | C-i-C₃F₇ | N | H | H | C-H | C-H | C-Cl | C-H | O | H | cyclopropyl | 4,7 | 567 |
| I-T3-157 | C-S(O)C₂ H₅ | C-i-C₃F₇ | N | H | H | C-H | C-H | C-Cl | C-H | O | H | cyclopropyl | 3,8 | 583 |
| I-T3-158 | C-S(O₂)C₂ H₅ | C-i-C₃F₇ | N | H | H | C-H | C-H | C-Cl | C-H | O | H | cyclopropyl | 3,9 | 599 |
| I-T3-159 | C-CF₃ | C-i-C₃F₇ | C-Cl | H | H | C-H | N | C-Cl | C-H | O | H | 1-(thiocarbamoyl)-cyclopropyl | 3,8 | 668 |
| I-T3-160 | C-CF₃ | C-i-C₃F₇ | C-Br | H | H | C-H | N | C-Cl | C-H | O | H | cyclopropyl | 4,0 | 653 |
| I-T3-161 | C-CF₃ | C-i-C₃F₇ | C-CH₃ | H | H | C-H | C-H | C-Cl | C-H | S | H | cyclopropyl | 5,0 | 604 |
| I-T3-162 | C-OCF ₃ | C-i-C₃F₇ | C-Cl | H | H | C-H | C-H | C-Cl | C-H | O | H | cyclopropyl | 4,6 | 624 |
| I-T3-163 | C-OCF₃ | C-i-C₃F₇ | C-Cl | H | H | C-H | C-H | C-Cl | C-H | O | H | 1-(cyano)cyclopropyl | 4,5 | 649 |

**Tabelle I-T3**

| | | | | | | | | | | | | | | | | |
|---|---|---|---|---|---|---|---|---|---|---|---|---|---|---|---|---|
| 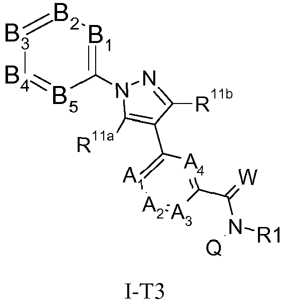 | | | | | | | | | | | | | | | | |
| B₂ und B₄ = C-H | | | | | | | | | | | | | | | | |

| **Bsp.-Nr.** | **B₁** | **B₂** | **B₃** | **B₄** | **B₅** | **R^{11a}** | **R^{11b}** | **A₁** | **A₂** | **A₃** | **A₄** | **W** | **R1** | **Q** | **logP^{a)}** | **Masse [m/z] ^{a)1)}** |
|---|---|---|---|---|---|---|---|---|---|---|---|---|---|---|---|---|
| I-T3-1 | C-CH₃ | C-H | C-i-C₃F₇ | C-H | C-CH₃ | H | H | C-H | C-H | C-Cl | C-H | O | H | cyclopropyl | 4,4 | 534 |
| I-T3-2 | C-CH₃ | C-H | C-i-C₃F₇ | C-H | C-CH₃ | H | H | C-H | C-H | C-Cl | C-H | O | H | CH₂CF₃ | 4,7 | 577 |
| I-T3-3 | C-CH₃ | C-H | C-i-C₃F₇ | C-H | C-CH₃ | H | H | C-H | C-H | C-Cl | C-H | O | H | 1-(cyano)cyclopro pyl | 4,2 | 559 |
| I-T3-4 | C-CH₃ | C-H | C-i-C₃F₇ | C-H | C-CH₃ | H | H | C-H | C-H | C-Cl | C-H | O | H | Thietan-3-yl | 4,7 | 566 |
| I-T3-5 | C-CH₃ | C-H | C-i-C₃F₇ | C-H | C-CH₃ | H | H | C-H | C-H | C-Cl | C-H | O | H | 1-(trifluoromethyl )cyclopropyl | 4,6 | 602 |
| I-T3-6 | C-CH₃ | C-H | C-i-C₃F₇ | C-H | C-CH₃ | H | H | C-H | C-H | C-Cl | C-H | O | H | 2-oxo-2-(2,2,2-trifluoroethylam ino)-ethyl | 4,1 | 633 |
| I-T3-7 | C-CH₃ | C-H | C-i-C₃F₇ | C-H | C-CH₃ | H | H | C-H | N | C-H | C-H | O | H | cyclopropyl | 3,4 | 501 |
| I-T3-8 | C-CH₃ | C-H | C-i-C₃F₇ | C-H | C-CH₃ | H | H | C-H | N | C-H | C-H | O | H | 1-(cyano)cyclopro pyl | 3,4 | 526 |
| I-T3-9 | C-CH₃ | C-H | C-i-C₃F₇ | C-H | C-CH₃ | H | H | C-H | C-H | C-H | C-F | O | H | cyclopropyl | 4,3 | 518 |
| I-T3-10 | C-CH₃ | C-H | C-i-C₃F₇ | C-H | C-CH₃ | H | H | C-H | C-H | C-H | C-F | O | H | 1-(cyano)cyclopro pyl | 4,2 | 543 |
| I-T3-11 | C-CH₃ | C-H | C-i-C₃F₇ | C-H | C-CH₃ | H | H | C-Cl | C-H | C-F | C-H | O | H | cyclopropyl | 4,9 | 552 |
| I-T3-12 | C-CH₃ | C-H | C-i-C₃F₇ | C-H | C-CH₃ | H | H | C-Cl | C-H | C-F | C-H | O | H | 1-(cyano)cyclopro pyl | 4,7 | 577 |
| I-T3-13 | C-CH₃ | C-H | C-i-C₃F₇ | C-H | C-CH₃ | H | H | C-F | C-H | C-H | C-H | O | H | cyclopropyl | 4,5 | 518 |
| I-T3-14 | C-CH₃ | C-H | C-i-C₃F₇ | C-H | C-CH₃ | H | H | C-F | C-H | C-Cl | C-H | O | H | cyclopropyl | 4,5 | 552 |
| I-T3-15 | C-CH₃ | C-H | C-i-C₃F₇ | C-H | C-CH₃ | H | H | C-H | C-CF3 | C-H | C-H | O | H | cyclopropyl | 4,9 | 568 |
| I-T3-16 | C-CH₃ | C-H | C-i-C₃F₇ | C-H | C-CH₃ | H | H | C-CH₃ | C-H | C-H | C-H | O | H | cyclopropyl | 4,4 | 514 |
| I-T3-17 | C-CH₃ | C-H | C-i-C₃F₇ | C-H | C-CH₃ | H | H | C-F | C-H | C-Cl | C-H | O | H | 1-(cyano)cyclopro pyl | 4,4 | 577 |
| I-T3-18 | C-CH₃ | C-H | C-i-C₃F₇ | C-H | C-CH₃ | H | H | C-H | C-CF₃ | C-H | C-H | O | H | 1-(cyano)cyclopro pyl | 4,7 | 593 |
| I-T3-19 | C-CH₃ | C-H | C-i-C₃F₇ | C-H | C-CH₃ | H | H | C-F | C-H | C-H | C-H | O | H | 1-(cyano)cyclopro pyl | 4,3 | 543 |
| I-T3-20 | C-CH₃ | C-H | C-i-C₃F₇ | C-H | C-CH₃ | H | H | C-H | N | C-Cl | C-H | O | H | cyclopropyl | 3,8 | 535 |
| I-T3-21 | C-CH₃ | C-H | C-i-C₃F₇ | C-H | C-CH₃ | H | H | C-H | N | C-Cl | C-H | O | H | 1-(cyano)cyclopro pyl | 3,7 | 560 |
| I-T3-22 | C-Cl | C-H | C-i-C₃F₇ | C-H | C-Cl | H | H | C-H | C-H | C-H | C-F | O | H | 1-(cyano)cyclopro pyl | 4,1 | 583 |
| I-T3-23 | C-CH₃ | C-H | C-i-C₃F₇ | C-H | C-CH₃ | H | H | C-H | C-H | C-Cl | C-H | O | H | 1-(cyano)cyclopro pyl | 4,2 | 599 |
| I-T3-24 | C-CH₃ | C-H | C-i-C₃F₇ | C-H | C-CH₃ | H | H | C-H | C-H | C-Cl | C-H | O | H | cyclopropyl | 4,3 | 574 |
| I-T3-25 | C-CH₃ | C-H | C-i-C₃F₇ | C-H | C-CH₃ | H | H | C-H | C-H | C-Cl | C-H | O | H | CH₂CF₃ | 4,7 | 616 |
| I-T3-26 | C-CH₃ | C-H | C-i-C₃F₇ | C-H | C-CH₃ | H | H | C-H | C-H | C-Cl | C-H | O | H | CH₂CH₂CF₃ | 4,7 | 630 |
| I-T3-27 | C-CH₃ | C-H | C-CF₃ | C-H | C-CH₃ | H | H | C-H | C-H | C-Cl | C-H | O | H | 1-(cyano)cyclopro pyl | 3,4 | 499 |
| I-T3-28 | C-CH₃ | C-H | C-CF₃ | C-H | C-CH₃ | H | H | C-H | C-H | C-Cl | C-H | O | H | cyclopropyl | 3,5 | 474 |
| I-T3-29 | C-CH₃ | C-H | C-i-C₃F₇ | C-H | C-CH₃ | H | H | C-H | N | C-H | C-H | O | H | cyclopropyl | 3,3 | 541 |
| I-T3-30 | C-CH₃ | C-H | C-i-C₃F₇ | C-H | C-CH₃ | H | H | C-H | N | C-H | C-H | O | H | 1-(cyano)cyclopro pyl | 3,2 | 566 |
| I-T3-31 | C-Cl | C-H | C-i-C₃F₇ | C-H | C-H | H | H | C-H | C-H | C-Cl | C-H | O | H | cyclopropyl | 4,3 | 540 |
| I-T3-32 | C-Cl | C-H | C-i-C₃F₇ | C-H | C-H | H | H | C-H | C-H | C-Cl | C-H | O | H | CH₂CF₃ | 4,7 | 582 |
| I-T3-33 | C-Cl | C-H | C-i-C₃F₇ | C-H | C-H | H | H | C-H | C-H | C-Cl | C-H | O | H | CH₂CH₂CF₃ | 4,7 | 596 |
| I-T3-34 | C-OCF₃ | C-H | C-i-C₃F₇ | C-H | C-H | H | H | C-H | C-H | C-Cl | C-H | O | H | 1-(cyano)cyclopro pyl | 4,6 | 615 |
| I-T3-35 | C-OCF₃ | C-H | C-i-C₃F₇ | C-H | C-H | H | H | C-H | C-H | C-Cl | C-H | O | H | cyclopropyl | 4,7 | 590 |
| I-T3-36 | C-OCF₃ | C-H | C-i-C₃F₇ | C-H | C-H | H | H | C-H | C-H | C-Cl | C-H | O | H | CH₂CF₃ | 5,0 | 632 |
| I-T3-37 | C-OCF₃ | C-H | C-i-C₃F₇ | C-H | C-H | H | H | C-H | C-H | C-Cl | C-H | O | H | Thietan-3-yl | 4,9 | 622 |
| I-T3-38 | C-C₂H₅ | C-H | C-i-C₃F₇ | C-H | C-H | H | H | C-H | C-H | C-Cl | C-H | O | H | 1-(cyano)cyclopro pyl | 4,5 | 559 |
| I-T3-39 | C-C₂H₅ | C-H | C-i-C₃F₇ | C-H | C-H | H | H | C-H | C-H | C-Cl | C-H | O | H | cyclopropyl | 4,6 | 534 |
| I-T3-40 | C-C₂H₅ | C-H | C-i-C₃F₇ | C-H | C-H | H | H | C-H | C-H | C-Cl | C-H | O | H | CH₂CF₃ | 4,9 | 576 |
| I-T3-41 | C-C₂H₅ | C-H | C-i-C₃F₇ | C-H | C-H | H | H | C-H | C-H | C-Cl | C-H | O | H | Thietan-3-yl | 4,9 | 566 |
| I-T3-42 | C-Cl | C-H | C-i-C₃F₇ | C-H | C-Cl | H | H | C-H | N | C-Cl | C-H | O | H | cyclopropyl | 3,8 | 575 |
| I-T3-43 | C-Cl | C-H | C-i-C₃F₇ | C-H | C-Cl | H | H | C-H | N | C-Cl | C-H | O | H | 1-(cyano)cyclopro pyl | 3,8 | 600 |
| I-T3-44 | C-C₂H₅ | C-H | C-i-C₃F₇ | C-H | C-CH₃ | H | H | C-H | C-H | C-Cl | C-H | O | H | cyclopropyl | 4,7 | 548 |
| I-T3-45 | C-C₂H₅ | C-H | C-i-C₃F₇ | C-H | C-CH₃ | H | H | C-H | C-H | C-Cl | C-H | O | H | CH₂CF₃ | 5,0 | 590 |
| I-T3-46 | C-C₂H₅ | C-H | C-i-C₃F₇ | C-H | C-CH₃ | H | H | C-H | C-H | C-Cl | C-H | O | H | 1-(cyano)cyclopro pyl | 4,6 | 573 |
| I-T3-47 | C-C₂H₅ | C-H | C-i-C₃F₇ | C-H | C-CH₃ | H | H | C-H | C-H | C-Cl | C-H | O | H | Thietan-3-yl | 5,0 | 580 |
| I-T3-48 | C-F | C-H | C-CF₃ | C-H | C-F | H | H | C-H | C-H | C-Cl | C-H | O | H | cyclopropyl | 3,2 | 442 |
| I-T3-49 | C-F | C-H | C-CF₃ | C-H | C-F | H | H | C-H | C-H | C-Cl | C-H | O | H | 1-(cyano)cyclopro pyl | 3,2 | 467 |
| I-T3-50 | C-F | C-H | C-CF₃ | C-H | C-OCH₃ | H | H | C-H | C-H | C-Cl | C-H | O | H | cyclopropyl | 3,1 | 454 |
| I-T3-51 | C-F | C-H | C-CF₃ | C-H | OCH₃ | H | H | C-H | C-H | C-Cl | C-H | O | H | 1-(cyano)cyclopro pyl | 3,1 | 479 |
| I-T3-52 | C-CF₃ | C-H | C-i-C₃F₇ | C-H | C-H | H | H | C-H | C-H | C-Cl | C-H | O | H | cyclopropyl | 4,3 | 574 |
| I-T3-53 | C-CF₃ | C-H | C-i-C₃F₇ | C-H | C-H | H | H | C-H | C-H | C-Cl | C-H | O | H | 1-(cyano)cyclopro pyl | 4,3 | 599 |
| I-T3-54 | C-CF₃ | C-H | C-i-C₃F₇ | C-H | C-CH₃ | H | H | C-H | C-H | C-Cl | C-H | O | H | cyclopropyl | 4,4 | 588 |
| I-T3-55 | C-CF₃ | C-H | C-i-C₃F₇ | C-H | C-CH₃ | H | H | C-H | C-H | C-Cl | C-H | O | H | 1-(cyano)cyclopro pyl | 4,3 | 613 |
| I-T3-56 | C-CF₃ | C-H | C-i-C₃F₇ | C-H | C-Cl | H | H | C-H | C-H | C-Cl | C-H | O | H | cyclopropyl | 4,4 | 608 |
| I-T3-57 | C-CF₃ | C-H | C-i-C₃F₇ | C-H | C-Cl | H | H | C-H | C-H | C-Cl | C-H | O | H | 1-(cyano)cyclopro pyl | 4,3 | 633 |
| I-T3-58 | C-CF₃ | C-H | C-i-C₃F₇ | C-H | C-Cl | H | H | C-H | C-H | C-Cl | C-H | O | H | CH₂CF₃ | 4,7 | 650 |
| I-T3-59 | C-Cl | C-H | C-CF₃ | C-H | N | H | H | C-H | C-H | C-Cl | C-H | O | H | 1-(cyano)cyclopro pyl | 3,1 | 466 |
| I-T3-60 | C-Cl | C-H | C-CF₃ | C-H | N | H | H | C-H | C-H | C-Cl | C-H | O | H | cyclopropyl | 3,2 | 441 |
| I-T3-61 | C-F | C-H | C-i-C₃F₇ | C-H | C-F | H | H | C-H | C-H | C-Cl | C-H | O | H | cyclopropyl | 4,0 | 542 |
| I-T3-62 | C-F | C-H | C-i-C₃F₇ | C-H | C-F | H | H | C-H | C-H | C-Cl | C-H | O | H | 1-(cyano)cyclopro pyl | 3,9 | 567 |
| I-T3-63 | C-CH₃ | C-H | C-i-C₃F₇ | C-H | C-Cl | H | H | C-H | C-H | C-Cl | C-H | O | H | cyclopropyl | 4,4 | 554 |
| I-T3-64 | C-CH₃ | C-H | C-i-C₃F₇ | C-H | C-Cl | H | H | C-H | C-H | C-Cl | C-H | O | H | 1-(cyano)cyclopro pyl | 4,2 | 579 |
| I-T3-65 | C-CH₃ | C-H | C-i-C₃F₇ | C-H | C-CH₃ | H | H | C-H | C-H | C-Cl | C-F | O | H | 1-(cyano)cyclopro pyl | 4,3 | 577 |
| I-T3-66 | C-C₂H₅ | C-H | C-i-C₃F₇ | C-H | C-CH₃ | H | H | C-H | C-H | C-Cl | C-F | O | H | 1-(cyano)cyclopro pyl | 4,6 | 591 |
| I-T3-67 | C-CH₃ | C-H | C-i-C₃F₇ | C-H | C-CH₃ | H | H | C-H | C-H | C-Cl | C-F | O | H | cyclopropyl | 4,4 | 552 |

| I-T3-68 | C-C₂H₅ | C-H | C-i-C₃F₇ | C-H | C-CH₃ | H | H | C-H | C-H | C-Cl | C-F | O | H | cyclopropyl | 4,8 | 566 |
|---|---|---|---|---|---|---|---|---|---|---|---|---|---|---|---|---|
| I-T3-69 | C-C₂H₅ | C-H | C-i-C₃F₇ | C-H | C-CH₃ | H | H | C-H | C-H | C-H | C-F | O | H | 1-(cyano)cyclopro pyl | 4,5 | 557 |
| I-T3-70 | C-C₂H₅ | C-H | C-i-C₃F₇ | C-H | C-CH₃ | H | H | C-H | C-H | C-H | C-F | O | H | cyclopropyl | 4,7 | 532 |
| I-T3-71 | C-C₂H₅ | C-H | C-i-C₃F₇ | C-H | C-Cl | H | H | C-H | C-H | C-Cl | C-H | O | H | 1-(cyano)cyclopro pyl | 4,5 | 593 |
| I-T3-72 | C-C₂H₅ | C-H | C-i-C₃F₇ | C-H | C-Cl | H | H | C-H | C-H | C-Cl | C-H | O | H | cyclopropyl | 4,6 | 568 |
| I-T3-73 | C-Cl | C-H | C-i-C₃F₇ | C-H | C-Cl | H | H | C-H | C-H | C-Cl | C-F | O | H | cyclopropyl | 4,5 | 592 |
| I-T3-74 | C-C₂H₅ | C-H | C-i-C₃F₇ | C-H | C-H | H | H | C-H | C-H | C-H | C-F | O | H | 1-(cyano)cyclopro pyl | 4,5 | 543 |
| I-T3-75 | C-C₂H₅ | C-H | C-i-C₃F₇ | C-H | C-H | H | H | C-H | C-H | C-H | C-F | O | H | cyclopropyl | 4,7 | 518 |
| I-T3-76 | C-C₂H₅ | C-H | C-i-C₃F₇ | C-H | C-CH₃ | H | H | C-H | N | C-H | C-H | O | H | 1-(cyano)cyclopro pyl | 3,4 | 540 |
| I-T3-77 | C-C₂H₅ | C-H | C-i-C₃F₇ | C-H | C-CH₃ | H | H | C-H | N | C-H | C-H | O | H | cyclopropyl | 3,4 | 515 |
| I-T3-78 | C-C₂H₅ | C-H | C-i-C₃F₇ | C-H | C-H | H | H | C-H | N | C-H | C-H | O | H | 1-(cyano)cyclopro pyl | 3,8 | 526 |
| I-T3-79 | C-C₂H₅ | C-H | C-i-C₃F₇ | C-H | C-H | H | H | C-H | N | C-H | C-H | O | H | cyclopropyl | 3,3 | 501 |
| I-T3-80 | C-C₂H₅ | C-H | C-C₂F₅ | C-H | C-CH₃ | H | H | C-H | C-H | C-Cl | C-H | O | H | 1-(cyano)cyclopro pyl | 4,2 | 523 |
| I-T3-81 | C-C₂H₅ | C-H | C-C₂F₅ | C-H | C-CH₃ | H | H | C-H | C-H | C-Cl | C-H | O | H | cyclopropyl | 4,3 | 498 |
| I-T3-82 | C-C₂H₅ | C-H | C-C₂F₅ | C-H | C-CH₃ | H | H | C-H | C-H | C-Cl | C-H | O | H | CH₂CF₃ | 4,0 | 540 |
| I-T3-83 | C-C₂H₅ | C-H | C-C₂F₅ | C-H | C-CH₃ | H | H | C-H | C-H | C-Cl | C-H | O | H | Thietan-3-yl | 4,6 | 530 |
| I-T3-84 | C-CF₃ | C-H | C-i-C₃F₇ | C-H | C-CH₃ | H | H | C-H | C-H | C-Cl | C-H | O | H | CH₂CF₃ | 4,7 | 630 |
| I-T3-85 | C-CF₃ | C-H | C-i-C₃F₇ | C-H | C-CH₃ | H | H | C-H | C-H | C-Cl | C-H | O | H | Thietan-3-yl | 4,7 | 620 |
| I-T3-86 | C-CH₃ | C-H | C-i-C₃F₇ | C-H | C-Br | H | H | C-H | C-H | C-Cl | C-H | O | H | cyclopropyl | 4,5 | 598 |
| I-T3-87 | C-Cl | C-H | C-OCF₃ | C-H | C-Cl | H | H | C-H | C-H | C-Cl | C-H | O | H | 1-(cyano)cyclopro pyl | 3,6 | 515 |
| I-T3-88 | C-Cl | C-H | C-OCF₃ | C-H | C-Cl | H | H | C-H | C-H | C-Cl | C-H | O | H | cyclopropyl | 3,6 | 490 |
| I-T3-89 | C-Cl | C-H | C-OCF₃ | C-H | C-H | H | H | C-H | C-H | C-Cl | C-H | O | H | 1-(cyano)cyclopro pyl | 3,5 | 481 |
| I-T3-90 | C-Cl | C-H | C-OCF₃ | C-H | C-H | H | H | C-H | C-H | C-Cl | C-H | O | H | cyclopropyl | 3,6 | 456 |
| I-T3-91 | C-Cl | C-H | C-i-C₃F₇ | C-H | C-Cl | H | H | C-H | N | C-CH₃ | C-H | O | H | cyclopropyl | 3,3 | 555 |
| I-T3-92 | C-Cl | C-H | C-i-C₃F₇ | C-H | C-Cl | H | H | C-H | N | C-CH₃ | C-H | O | H | 1-(cyano)cyclopro pyl | 3,4 | 580 |
| I-T3-93 | C-Cl | C-H | C-i-C₃F₇ | C-H | C-Cl | H | H | C-H | C-H | C-Cl | C-H | O | H | 1-(tert.butyl)cyclo propyl | 4,7 | 588 |
| I-T3-94 | C-CF₃ | C-H | C-i-C₃F₇ | C-H | C-CH₃ | H | H | C-H | C-H | C-Cl | C-H | O | H | 1-(tert.butyl)cyclo propyl | 4,7 | 602 |
| I-T3-95 | C-Cl | C-H | C-CF₃ | C-H | C-Cl | H | H | C-H | N | C-Cl | C-H | O | H | 1-(cyano)cyclopro pyl | 3,1 | 500 |
| I-T3-96 | C-CH₃ | C-H | C-i-C₃F₇ | C-H | C-CH₃ | H | H | C-H | N | C-CH₃ | C-H | O | H | cyclopropyl | 3,3 | 515 |
| I-T3-97 | C-CH₃ | C-H | C-i-C₃F₇ | C-H | C-CH₃ | H | H | C-H | N | C-CH₃ | C-H | O | H | 1-(cyano)cyclopro pyl | 3,4 | 540 |
| I-T3-98 | C-Cl | C-H | C-CF₃ | C-H | C-Cl | H | H | C-H | N | C-Cl | C-H | O | H | cyclopropyl | 3,1 | 475 |
| I-T3-99 | C-Cl | C-H | C-CF₃ | C-H | C-Cl | H | H | C-H | N | C-Cl | C-H | O | CH₃ | cyclopropyl | 3,5 | 489 |
| I-T3-100 | C-Cl | C-H | C-i-C₃F₇ | C-H | C-Cl | H | H | C-H | N | C-Cl | C-H | O | CH₃ | cyclopropyl | 4,4 | 589 |
| I-T3-101 | C-CH₃ | C-H | C-i-C₃F₇ | C-H | C-CH₃ | H | H | C-H | N | C-Cl | C-H | O | CH₃ | cyclopropyl | 4,4 | 549 |
| I-T3-102 | C-Cl | C-H | C-i-C₃F₇ | C-H | C-Cl | H | H | C-H | N | C-Cl | C-H | O | CH₃ | 1-(cyano)cyclopro pyl | 4,3 | 614 |
| I-T3-103 | C-CH₃ | C-H | C-i-C₃F₇ | C-H | C-CH₃ | H | H | C-H | N | C-Cl | C-H | O | CH₃ | 1-(cyano)cyclopro pyl | 4,3 | 574 |
| I-T3-104 | C-C₂H₅ | C-H | C-i-C₃F₈ | C-H | C-H | H | H | C-H | N | C-Cl | C-H | O | H | cyclopropyl | 4,2 | 535 |
| I-T3-105 | C-C₂H₅ | C-H | C-i-C₃F₈ | C-H | C-H | H | H | C-H | N | C-Cl | C-H | O | H | 1-(cyano)cyclopro pyl | 4,1 | 560 |
| I-T3-106 | C-CF₃ | C-H | C-i-C₃F₇ | C-H | C-CH₃ | H | H | C-H | N | C-Cl | C-H | O | H | cyclopropyl | 4,0 | 589 |
| I-T3-107 | C-CF₃ | C-H | C-i-C₃F₇ | C-H | C-CH₃ | H | H | C-H | N | C-Cl | C-H | O | CH₃ | cyclopropyl | 4,5 | 603 |
| I-T3-108 | C-CF₃ | C-H | C-i-C₃F₇ | C-H | C-CH₃ | H | H | C-H | N | C-Cl | C-H | O | H | 1-(cyano)cyclopro pyl | 4,0 | 614 |
| I-T3-109 | C-C₂H₅ | C-H | C-i-C₃F₇ | C-H | C-CH₃ | H | H | C-H | N | C-Cl | C-H | O | H | cyclopropyl | 4,2 | 549 |
| I-T3-110 | C-C₂H₅ | C-H | C-i-C₃F₇ | C-H | C-CH₃ | H | H | C-H | N | C-Cl | C-H | O | H | 1-(cyano)cyclopro pyl | 4,1 | 574 |
| I-T3-111 | C-CF₃ | C-H | C-i-C₃F₇ | C-H | C-CH₃ | H | H | C-H | N | C-Cl | C-H | O | CH₃ | 1-(cyano)cyclopro pyl | 4,4 | 628 |
| I-T3-112 | C-CF₃ | C-H | C-i-C₃F₇ | C-H | C-Cl | H | H | C-H | N | C-Cl | C-H | O | H | cyclopropyl | 4,0 | 609 |
| I-T3-113 | C-CF₃ | C-H | C-i-C₃F₇ | C-H | C-Cl | H | H | C-H | N | C-Cl | C-H | O | CH₃ | cyclopropyl | 4,0 | 623 |
| I-T3-114 | C-CF₃ | C-H | C-i-C₃F₇ | C-H | C-Cl | H | H | C-H | N | C-Cl | C-H | O | H | 1-(cyano)cyclopro pyl | 4,0 | 634 |
| I-T3-115 | C-CH₃ | C-H | C-i-C₃F₇ | C-H | C-CH₃ | H | H | C-H | N | C-CH₃ | C-H | O | CH₃ | 1-(cyano)cyclopro pyl | 3,8 | 554 |
| I-T3-116 | C-CF₃ | C-H | C-i-C₃F₇ | C-H | C-Cl | H | H | C-H | N | C-Cl | C-H | O | CH₃ | 1-(cyano)cyclopro pyl | 4,3 | 648 |
| I-T3-117 | C-CF₃ | C-H | C-i-C₃F₇ | C-H | C-CH₃ | H | H | C-H | N | C-CH₃ | C-H | O | H | cyclopropyl | 3,5 | 569 |
| I-T3-118 | C-CF₃ | C-H | C-i-C₃F₇ | C-H | C-CH₃ | H | H | C-H | N | C-CH₃ | C-H | O | CH₃ | cyclopropyl | 3,8 | 583 |
| I-T3-119 | C-CF₃ | C-H | C-i-C₃F₇ | C-H | C-CH₃ | H | H | C-H | N | C-CH₃ | C-H | O | H | 1-(cyano)cyclopro pyl | 3,6 | 594 |
| I-T3-120 | C-CH₃ | C-H | C-i-C₃F₇ | C-H | C-CH₃ | H | H | C-H | N | C-CH₃ | C-H | O | CH₃ | cyclopropyl | 3,6 | 529 |
| I-T3-121 | C-CH₃ | C-H | C-i-C₃F₇ | C-H | C-CH₃ | H | H | N | C-H | C-Cl | N | O | H | cyclopropyl | 4,5 | 536 |
| I-T3-122 | C-Cl | C-H | C-CF₃ | C-H | N | H | H | C-H | C-H | C-H | C-H | O | H | cyclopropyl | 3,0 | 407 |
| I-T3-123 | C-CF₃ | C-H | C-i-C₃F₇ | C-H | C-CH₃ | H | H | C-H | N | C-CH₃ | C-H | O | CH₃ | 1-(cyano)cyclopro pyl | 3,9 | 608 |
| I-T3-124 | C-CF₃ | C-H | C-i-C₃F₇ | C-H | C-Cl | H | H | C-H | N | C-CH₃ | C-H | O | H | cyclopropyl | 3,5 | 589 |
| I-T3-125 | C-CF₃ | C-H | C-i-C₃F₇ | C-H | C-Cl | H | H | C-H | N | C-CH₃ | C-H | O | H | 1-(cyano)cyclopro pyl | 3,8 | 614 |
| I-T3-126 | C-Cl | C-H | C-i-C₃F₇ | C-H | C-Cl | CN | NH₂ | C-H | C-H | C-Cl | C-H | O | H | 1-(cyano)cyclopro pyl | 4,1 | 639 |
| I-T3-127 | C-Cl | C-H | C-CF₃ | C-H | C-Cl | H | H | C-H | N | C-CH₃ | C-H | O | H | cyclopropyl | 2,5 | 455 |
| I-T3-128 | C-Cl | C-H | C-CF₃ | C-H | C-Cl | H | H | C-H | N | C-CH₃ | C-H | O | CH₃ | cyclopropyl | 2,8 | 469 |
| I-T3-129 | C-Cl | C-H | C-CF₃ | C-H | C-Cl | H | H | C-H | N | C-CH₃ | C-H | O | H | 1-(cyano)cyclopro pyl | 2,6 | 480 |
| I-T3-130 | C-CF₃ | C-H | C-i-C₃F₇ | C-H | C-Cl | H | H | C-H | N | C-CH₃ | C-H | O | CH₃ | cyclopropyl | 3,8 | 603 |
| I-T3-131 | C-CF₃ | C-H | C-i-C₃F₇ | C-H | C-Cl | H | H | C-H | N | C-CH₃ | C-H | O | CH₃ | 1-(cyano)cyclopro pyl | 3,9 | 628 |
| I-T3-132 | C-Cl | C-H | C-CF₃ | C-H | C-Cl | H | H | C-H | N | C-CH₃ | C-H | O | CH₃ | 1-(cyano)cyclopro pyl | 2,9 | 494 |
| I-T3-133 | C-Cl | C-H | C-i-C₃F₇ | C-H | C-Cl | H | H | C-H | N | C-CH₃ | C-H | O | CH₃ | cyclopropyl | 3,7 | 569 |
| I-T3-134 | C-CF₃ | C-H | C-i-C₃F₇ | C-H | C-H | H | H | C-H | C-H | C-Cl | C-H | O | CH₃ | cyclopropyl | 4,9 | 588 |
| I-T3-135 | C-Cl | C-H | C-i-C₃F₇ | C-H | C-Cl | H | H | C-H | N | C-(1-pyrrolidi nyl) | C-H | O | H | cyclopropyl | 4,3 | 610 |
| I-T3-136 | C-Cl | C-H | C-i-C₃F₇ | C-H | C-Cl | H | H | C-H | N | C-NHCH₃ | C-H | O | H | cyclopropyl | 3,5 | 570 |
| I-T3-137 | C-Cl | C-H | C-i-C₃F₇ | C-H | C-Cl | H | H | C-H | N | C-NH-cyclopro pyl | C-H | O | H | cyclopropyl | 3,4 | 596 |
| I-T3-138 | C-Cl | C-H | C-i-C₃F₇ | C-H | C-Cl | H | H | C-H | N | C-NH-CH₂CH₂ OCH₃ | C-H | O | H | cyclopropyl | 4,1 | 614 |
| I-T3-139 | C-CH₃ | C-H | C-i-C₃F₇ | C-H | C-CH₃ | H | H | C-H | N | C-cyclopro pyl | C-H | O | H | 1-(cyano)cyclopro pyl | 4,1 | 566 |
| I-T3-140 | C-CH₃ | C-H | C-i-C₃F₇ | C-H | C-CH₃ | H | H | C-H | N | C-cyclopro pyl | C-H | O | H | cyclopropyl | 4,2 | 541 |
| I-T3-141 | C-CH₃ | C-H | C-i-C₃F₇ | C-H | C-CH₃ | H | H | C-H | N | C-OCH₃ | C-H | O | H | 1-(cyano)cyclopro pyl | 4,4 | 556 |
| I-T3-142 | C-CH₃ | C-H | C-i-C₃F₇ | C-H | C-CH₃ | H | H | C-H | N | C-cyclopro pyl | C-H | O | CH₃ | cyclopropyl | 4,6 | 555 |
| I-T3-143 | C-CH₃ | C-H | C-i-C₃F₇ | C-H | C-CH₃ | H | H | C-H | N | C-OCH₃ | C-H | O | H | cyclopropyl | 4,6 | 531 |
| I-T3-144 | C-CH₃ | C-H | C-i-C₃F₇ | C-H | C-CH₃ | H | H | C-H | N | C-OCH₃ | C-H | O | CH₃ | cyclopropyl | 4,4 | 545 |
| I-T3-145 | C-Cl | C-H | C-CF₃ | C-H | C-Cl | H | H | C-H | N | C-Cl | C-H | O | C₂H₅ | cyclopropyl | 3,8 | 503 |
| I-T3-146 | C-Cl | C-H | C-CF₃ | C-H | C-Cl | H | H | C-H | N | C-Cl | C-H | O | H | N-methyl-pyrazol-3-yl | 2,8 | 515 |
| I-T3-147 | C-Cl | C-H | C-CF₃ | C-H | C-Cl | H | H | C-H | N | C-CH₃ | C-H | O | H | C₂H₅ | 4,0 | 443 |
| I-T3-148 | C-CF₃ | C-H | C-i-C₃F₇ | C-H | C-Br | H | H | C-H | C-H | C-Cl | C-H | O | H | cyclopropyl | 4,5 | 652 |
| I-T3-149 | C-CF₃ | C-H | C-CF₃ | C-H | N | H | H | C-H | C-H | C-Cl | C-H | O | H | 1-(cyano)cyclopro pyl | 3,5 | 500 |
| I-T3-150 | C-CF₃ | C-H | C-CF₃ | C-H | N | H | H | C-H | C-H | C-Cl | C-H | O | H | cyclopropyl | 3,6 | 475 |
| I-T3-151 | C-Cl | C-H | C-i-C₃F₇ | C-H | N | H | H | C-H | C-H | C-Cl | C-H | O | H | 1-(cyano)cyclopro pyl | 4,0 | 566 |
| I-T3-152 | C-Cl | C-H | C-i-C₃F₇ | C-H | N | H | H | C-H | C-H | C-Cl | C-H | O | H | cyclopropyl | 4,1 | 541 |
| I-T3-153 | C-S(O)C₂H₅ | C-H | C-i-C₃F₇ | C-H | N | H | H | C-H | C-H | C-Cl | C-H | O | H | 1-(cyano)cyclopro pyl | 3,7 | 608 |
| I-T3-154 | C-S(O₂)C₂H ₅ | C-H | C-i-C₃F₇ | C-H | N | H | H | C-H | C-H | C-Cl | C-H | O | H | 1-(cyano)cyclopro pyl | 3,8 | 624 |
| I-T3-155 | C-CF₃ | C-H | C-i-C₃F₇ | C-H | C-Br | H | H | C-H | C-H | C-Cl | C-H | O | H | 1-(cyano)cyclopro pyl | 4,4 | 677 |
| I-T3-156 | C-SC₂H₅ | C-H | C-i-C₃F₇ | C-H | N | H | H | C-H | C-H | C-Cl | C-H | O | H | cyclopropyl | 4,7 | 567 |
| I-T3-157 | C-S(O)C₂H₅ | C-H | C-i-C₃F₇ | C-H | N | H | H | C-H | C-H | C-Cl | C-H | O | H | cyclopropyl | 3,8 | 583 |
| I-T3-158 | C-S(O₂)C₂H ₅ | C-H | C-i-C₃F₇ | C-H | N | H | H | C-H | C-H | C-Cl | C-H | O | H | cyclopropyl | 3,9 | 599 |
| I-T3-159 | C-CF₃ | C-H | C-i-C₃F₇ | C-H | C-Cl | H | H | C-H | N | C-Cl | C-H | O | H | 1-(thiocarbamoyl) -cyclopropyl | 3,8 | 668 |
| I-T3-160 | C-CF₃ | C-H | C-i-C₃F₇ | C-H | C-Br | H | H | C-H | N | C-Cl | C-H | O | H | cyclopropyl | 4,0 | 653 |
| I-T3-161 | C-CF₃ | C-H | C-i-C₃F₇ | C-H | C-CH₃ | H | H | C-H | C-H | C-Cl | C-H | S | H | cyclopropyl | 5,0 | 604 |
| I-T3-162 | C-OCF₃ | C-H | C-i-C₃F₇ | C-H | C-Cl | H | H | C-H | C-H | C-Cl | C-H | O | H | cyclopropyl | 4,6 | 624 |
| I-T3-163 | C-OCF₃ | C-H | C-i-C₃F₇ | C-H | C-Cl | H | H | C-H | C-H | C-Cl | C-H | O | H | 1-(cyano)cyclopro pyl | 4,5 | 649 |
| I-T3-164 | C-Cl | C-H | C-CF₃ | C-H | C-CN | H | H | C-H | C-H | C-Cl | C-H | O | H | 1-(cyano)cyclopro pyl | 3,1 | 490 |
| I-T3-165 | C-CF₃ | C-H | C-i-C₃F₇ | C-H | C-Br | H | H | C-H | N | C-Cl | C-H | O | H | 1-(cyano)cyclopro pyl | 3,9 | 678 |
| I-T3-166 | C-SCH₃ | C-H | C-i-C₃F₇ | C-H | N | H | H | C-H | C-H | C-Cl | C-H | O | H | 1-(cyano)cyclopro pyl | 4,3 | 578 |
| I-T3-167 | C-SC₂H₅ | C-H | C-i-C₃F₇ | C-H | N | H | H | C-H | C-H | C-Cl | C-H | O | H | 1-(cyano)cyclopro pyl | 4,6 | 592 |
| I-T3-168 | C-Cl | C-H | C-CF₃ | C-H | C-NO₂ | H | H | C-H | C-H | C-Cl | C-H | O | H | cyclopropyl | 3,3 | 485 |
| I-T3-169 | C-Cl | C-H | C-CF₃ | C-H | C-NO₂ | H | H | C-H | C-H | C-Cl | C-H | O | H | 1-(cyano)cyclopro pyl | 3,2 | 510 |
| I-T3-170 | C-SC₂H₅ | C-H | C-CF₃ | C-H | C-Cl | H | H | C-H | C-H | C-Cl | C-H | O | H | 1-(cyano)cyclopro pyl | 3,7 | 525 |
| I-T3-171 | C-SCH₂CF₃ | C-H | C-i-C₃F₇ | C-H | N | H | H | C-H | C-H | C-Cl | C-H | O | H | 1-(cyano)cyclopro pyl | 4,5 | 646 |
| I-T3-172 | C-SC₂H₅ | C-H | C-CF₃ | C-H | C-Cl | H | H | C-H | C-H | C-Cl | C-H | O | H | cyclopropyl | 3,9 | 500 |
| I-T3-173 | C-Cl | C-H | C-CF₃ | C-H | C-Cl | H | H | C-H | N | C-OCH₃ | C-H | O | CH₃ | cyclopropyl | 4,4 | 585 |
| I-T3-174 | C-Cl | C-H | C-CF₃ | C-H | C-CN | H | H | C-H | C-H | C-Cl | C-H | O | H | cyclopropyl | 3,1 | 465 |
| I-T3-175 | C-CH₃ | C-H | C-i-C₃F₇ | C-H | C-I | H | H | C-H | C-H | C-Cl | C-H | O | H | cyclopropyl | 4,5 | 646 |
| I-T3-176 | C-CH₃ | C-H | C-i-C₃F₇ | C-H | C-I | H | H | C-H | C-H | C-Cl | C-H | O | H | 1-(cyano)cyclopro pyl | 4,4 | 671 |
| I-T3-177 | C-SC₂H₅ | C-H | C-CF₃ | C-H | C-CN | H | H | C-H | C-H | C-Cl | C-H | O | H | 1-(cyano)cyclopro pyl | 3,4 | 516 |
| I-T3-178 | C-S(O₂)C₂H ₅ | C-H | C-CF₃ | C-H | C-Cl | H | H | C-H | C-H | C-Cl | C-H | O | H | cyclopropyl | 3,2 | 532 |
| I-T3-179 | C-S(O)C₂H₅ | C-H | C-CF₃ | C-H | C-Cl | H | H | C-H | C-H | C-Cl | C-H | O | H | cyclopropyl | 3,0 | 516 |
| I-T3-180 | C-S(O₂)C₂H ₅ | C-H | C-CF₃ | C-H | C-CN | H | H | C-H | C-H | C-Cl | C-H | O | H | cyclopropyl | 2,8 | 523 |
| I-T3-181 | C-S(O)C₂H₅ | C-H | C-CF₃ | C-H | C-CN | H | H | C-H | C-H | C-Cl | C-H | O | H | cyclopropyl | 2,7 | 507 |
| I-T3-182 | C-S(O)C₂H₅ | C-H | C-CF₃ | C-H | C-CN | H | H | C-H | C-H | C-Cl | C-H | O | H | 1-(cyano)cyclopro pyl | 3,0 | 541 |
| I-T3-183 | C-S(O₂)C₂H ₅ | C-H | C-CF₃ | C-H | C-Cl | H | H | C-H | C-H | C-Cl | C-H | O | H | 1-(cyano)cyclopro pyl | 3,1 | 557 |
| I-T3-184 | C-Cl | C-F | C-CF₃ | C-H | C-Cl | H | H | C-H | C-H | C-Cl | C-H | O | H | cyclopropyl | 3,6 | 492 |
| I-T3-185 | C-Cl | C-F | C-CF₃ | C-H | C-Cl | H | H | C-H | C-H | C-Cl | C-H | O | H | 1-(cyano)cyclopro pyl | 3,6 | 517 |
| I-T3-186 | C-Cl | C-H | C-CF₃ | C-H | C-F | H | H | C-H | C-H | C-Cl | C-H | O | H | cyclopropyl | 3,6 | 458 |
| I-T3-187 | C-Cl | C-H | C-CF₃ | C-H | C-F | H | H | C-H | C-H | C-Cl | C-H | O | H | 1-(cyano)cyclopro pyl | 3,3 | 483 |
| I-T3-188 | C-F | C-F | C-CF₃ | C-F | C-F | H | H | C-H | C-H | C-Cl | C-H | O | H | 1-(cyano)cyclopro pyl | 3,4 | 503 |
| I-T3-189 | C-OCF₃ | C-H | C-i-C₃F₇ | C-H | Cl | H | H | C-H | N | C-Cl | C-H | O | H | cyclopropyl | 4,3 | 625 |
| I-T3-190 | C-S(O₂)C₂H ₅ | C-H | C-CF₃ | C-H | C-CN | H | H | C-H | C-H | C-Cl | C-H | O | H | 1-(cyano)cyclopro pyl | 2,9 | 548 |
| I-T3-191 | C-SC₂H₅ | C-H | C-i-C₃F₇ | C-H | C-SC₂H₅ | H | H | C-H | C-H | C-Cl | C-H | O | H | 1-(cyano)cyclopro pyl | 4,7 | 651 |
| I-T3-192 | C-SC₂H₅ | C-H | C-i-C₃F₇ | C-H | C-Cl | H | H | C-H | C-H | C-Cl | C-H | O | H | 1-(cyano)cyclopro pyl | 4,5 | 625 |
| I-T3-193 | C-CHF₂ | C-H | C-i-C₃F₇ | C-H | C-Cl | H | H | C-H | C-H | C-Cl | C-H | O | H | cyclopropyl | 4,4 | 590 |
| I-T3-194 | C-F | C-F | C-CF₃ | C-F | C-F | H | H | C-H | C-H | C-Cl | C-H | O | H | 1 -(carbamoyl)-cyclopropyl | 2,8 | 521 |
| I-T3-195 | C-S(O)C₂H₅ | C-H | C-i-C₃F₇ | C-H | C-Cl | H | H | C-H | C-H | C-Cl | C-H | O | H | 1-(cyano)cyclopro pyl | 3,8 | 641 |
| I-T3-196 | C-OCHF₂ | C-H | C-i-C₃F₇ | C-H | Cl | H | H | C-H | N | C-Cl | C-H | O | H | cyclopropyl | 3,9 | 607 |
| I-T3-197 | C-CF₃ | C-H | C-i-C₃F₇ | C-H | C-CH₃ | H | H | C-H | N | C-Cl | C-H | O | H | (1-cyano-cyclopropyl)met hyl | 4,0 | 628 |
| I-T3-198 | C-S(O₂)C₂H ₅ | C-H | C-i-C₃F₈ | C-H | C-S(O₂) C₂H₅ | H | H | C-H | C-H | C-Cl | C-H | O | H | 1-(cyano)cyclopro pyl | 3,7 | 715 |
| I-T3-199 | C-CF₃ | C-H | C-i-C₃F₇ | C-H | C-Cl | H | H | C-H | N | C-Cl | C-H | O | H | (1-cyano-cyclopropyl)met hyl | 1,17 min^{b)} | 648 |
| I-T3-200 | C-OCF₃ | C-H | C-i-C₃F₇ | C-H | C-Cl | H | H | C-H | N | C-Cl | C-H | O | H | 1-(cyano)cyclopro pyl | 4,1 | 650 |
| I-T3-201 | C-S(O₂)C₂H ₅ | C-H | C-i-C₃F₈ | C-H | C-Cl | H | H | C-H | C-H | C-Cl | C-H | O | H | 1-(cyano)cyclopro pyl | 4,0 | 657 |
| I-T3-202 | C-SC₂H₅ | C-H | C-i-C₃F₈ | C-H | C-CH₃ | H | H | C-H | C-H | C-Cl | C-H | O | H | 1-(cyano)cyclopro pyl | 4,5 | 605 |
| I-T3-203 | C-SC₂H₅ | C-H | C-i-C₃F₈ | C-H | C-CH₃ | H | H | C-H | C-H | C-Cl | C-H | O | H | cyclopropyl | 4,6 | 580 |
| I-T3-204 | C-CN | C-H | C-i-C₃F₇ | C-H | C-Cl | H | H | C-H | N | C-Cl | C-H | O | CH₃ | cyclopropyl | 3,9 | 580 |
| I-T3-205 | C-CN | C-H | C-i-C₃F₇ | C-H | C-Cl | H | H | C-H | N | C-Cl | C-H | O | H | cyclopropyl | 3,5 | 566 |
| I-T3-206 | C-S(O)C₂H₅ | C-H | C-i-C₃F₈ | C-H | C-CH₃ | H | H | C-H | C-H | C-Cl | C-H | O | H | 1-(cyano)cyclopro pyl | 3,6 | 621 |
| I-T3-207 | C-CN | C-H | C-i-C₃F₇ | C-H | C-Cl | H | H | C-H | N | C-Cl | C-H | O | H | 1-(cyano)cyclopro pyl | 3,5 | 591 |
| I-T3-208 | C-OCF₃ | C-H | C-i-C₃F₇ | C-H | C-Cl | H | H | C-H | N | C-Cl | C-H | O | CH₃ | cyclopropyl | 4,6 | 639 |
| I-T3-209 | C-S(O₂)C₂H | C-H | C-i-C₃F₈ | C-H | C-CH₃ | H | H | C-H | C-H | C-Cl | C-H | O | H | 1-(cyano)cyclopropyl | 3,8 | 637 |
| I-T3-210 | C-S(O₂)C₂H | C-H | C-i-C₃F₈ | C-H | C-CH₃ | H | H | C-H | C-H | C-Cl | C-H | O | H | cyclopropyl | 3,9 | 612 |
| I-T3-211 | C-OC₂H₅ | C-H | C-i-C₃F₉ | C-H | C-OC₂H₅ | H | H | C-H | N | C-Cl | C-H | O | CH₃ | cyclopropyl | 4,7 | 609 |
| I-T3-212 | C-I | C-H | C-i-C₃F₇ | C-H | C-Cl | H | H | C-H | C-H | C-Cl | C-H | O | H | 1-(cyano)cyclopro pyl | 4,3 | 691 |
| I-T3-213 | C-I | C-H | C-i-C₃F₇ | C-H | C-Cl | H | H | C-H | C-H | C-Cl | C-H | O | H | cyclopropyl | 4,4 | 666 |
| I-T3-214 | C-OCHF₂ | C-H | C-i-C₃F₇ | C-H | C-Cl | H | H | C-H | C-H | C-Cl | C-H | O | H | cyclopropyl | 4,3 | 606 |
| I-T3-215 | C-OCHF₂ | C-H | C-i-C₃F₇ | C-H | C-Cl | H | H | C-H | C-H | C-Cl | C-H | O | H | 1-(cyano)cyclopro pyl | 4,1 | 631 |
| I-T3-216 | C-OCHF₂ | C-H | C-i-C₃F₇ | C-H | C-Cl | H | H | C-H | C-H | C-Cl | C-H | O | CH₃ | cyclopropyl | 4,7 | 620 |
| I-T3-217 | C-OCHF₂ | C-H | C-i-C₃F₇ | C-H | C-Cl | H | H | C-H | N | C-Cl | C-H | O | CH₃ | cyclopropyl | 4,2 | 621 |
| I-T3-218 | C-OCHF₂ | C-H | C-i-C₃F₇ | C-H | C-Cl | H | H | C-H | N | C-Cl | C-H | O | H | 1-(cyano)cyclopro pyl | 3,7 | 632 |
| I-T3-219 | C-OCF₃ | C-H | C-i-C₃F₇ | C-H | C-Cl | H | H | C-H | C-H | C-Cl | C-H | O | CH₃ | cyclopropyl | 5,1 | 638 |
| I-T3-220 | C-Cl | C-H | C-C(CF₃)₂ OH | C-H | C-Cl | H | H | C-H | N | C-Cl | C-H | O | CH₃ | cyclopropyl | 3,4 | 587 |
| I-T3-221 | C-Cl | C-H | C-C(CF₃)₂ OH | C-H | C-Cl | H | H | C-H | N | C-Cl | C-H | O | H | cyclopropyl | 3,0 | 573 |
| I-T3-222 | C-Cl | C-H | C-C(CF₃)₂ OH | C-H | C-Cl | H | H | C-H | N | C-Cl | C-H | O | H | 1-(cyano)cyclopro pyl | 3,0 | 598 |
| I-T3-223 | C-Cl | C-H | C-C(CF₃)₂ OH | C-H | C-Cl | H | H | C-H | N | C-Cl | C-H | O | CH₃ | 1-(cyano)cyclopro pyl | 3,3 | 612 |
| I-T3-224 | C-OCF₃ | C-H | C-i-C₃F₇ | C-H | C-I | H | H | C-H | C-H | C-Cl | C-H | O | H | cyclopropyl | 4,7 | 716 |
| I-T3-225 | C-OCF₃ | C-H | C-i-C₃F₇ | C-H | C-I | H | H | C-H | C-H | C-Cl | C-H | O | CH₃ | cyclopropyl | 5,2 | 729 |
| I-T3-226 | C-OCF₃ | C-H | C-i-C₃F₇ | C-H | C-I | H | H | C-H | C-H | C-Cl | C-H | O | H | 1-(cyano)cyclopro pyl | 4,6 | 740 |
| I-T3-227 | C-CF₃ | C-H | C-i-C₃F₇ | C-H | C-I | H | H | C-H | C-H | C-Cl | C-H | O | H | cyclopropyl | 4,5 | 700 |
| I-T3-228 | C-CF₃ | C-H | C-i-C₃F₇ | C-H | C-I | H | H | C-H | C-H | C-Cl | C-H | O | H | 1-(cyano)cyclopro pyl | 4,4 | 725 |
| I-T3-229 | C-CF₃ | C-H | C-i-C₃F₇ | C-H | C-I | H | H | C-H | C-H | C-Cl | C-H | O | CH₃ | cyclopropyl | 5,1 | 714 |

| | | | | | | | | | | | | | | | | |
|---|---|---|---|---|---|---|---|---|---|---|---|---|---|---|---|---|
| ^{b)} Retentionszeit gemessen mit: Instrument: Waters ACQUITY SQD UPLC System; Säule: Waters Acquity UPLC HSS T3 1.8 µ 50 x 1 mm; Eluent A: 11 Wasser + 0.25 ml 99%ige Ameisensäure , Eluent B: 11 Acetonitril + 0.25 ml 99%ige Ameisensäure; Gradient: 0.0 min 90% A → 1.2 min 5% A → 2.0 min 5% A Ofen: 50°C; Fluss: 0.40 ml/min; UV-Detektion: 208 - 400 nm. | | | | | | | | | | | | | | | | |

**Tabelle I-T4**

| | | | | | | | | | | | | | | | |
|---|---|---|---|---|---|---|---|---|---|---|---|---|---|---|---|
| 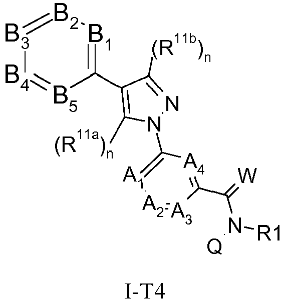 | | | | | | | | | | | | | | | |
| B₂ und B₄ = C-H | | | | | | | | | | | | | | | |

| **Bsp.-Nr.** | **B₁** | **B₃** | **B₅** | **R₁** | **R^{11a}** | **R^{11b}** | **A₁** | **A₂** | **A₃** | **A₄** | **W** | **R₁** | **Q** | **logP^{a)}** | **Masse [m/z] ^{a)1)}** |
|---|---|---|---|---|---|---|---|---|---|---|---|---|---|---|---|
| I-T4-1 | C-CH₃ | C-i-C₃F₇ | C-CH₃ | H | H | H | C-H | C-H | C-Cl | C-H | O | H | cyclopropyl | 4,7 | 534 |
| I-T4-2 | C-CH₃ | C-i-C₃F₇ | C-CH₃ | H | H | H | C-H | C-H | C-Cl | C-H | O | H | 1-(cyano)cyclopropyl | 4,6 | 559 |
| I-T4-3 | C-CH₃ | C-i-C₃F₇ | C-CH₃ | H | H | H | C-H | N | C-Cl | C-H | O | H | cyclopropyl | 4,4 | 535 |
| I-T4-4 | C-CH₃ | C-i-C₃F₇ | C-CH₃ | H | H | H | C-H | N | C-Cl | C-H | O | H | 1-(cyano)cyclopropyl | 4,3 | 560 |

**Tabelle I-T22**

| | | | | | | | | | | | | |
|---|---|---|---|---|---|---|---|---|---|---|---|---|
| 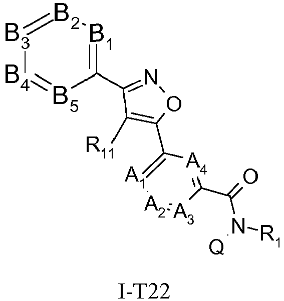 | | | | | | | | | | | | |
| B₂ und B₄ = C-H, W = O | | | | | | | | | | | | |

| **Bsp.-Nr.** | **B₁** | **B₃** | **B₅** | **R₁** | **R₁₁** | **A₁** | **A₂** | **A₃** | **A₄** | **Q** | **logP^{a)}** | **Masse [m/z]^{a)1)}** |
|---|---|---|---|---|---|---|---|---|---|---|---|---|
| I-T22-1 | C-CH3 | C-i-C₃F₇ | C-CH3 | H | H | C-H | C-H | C-Cl | C-H | cyclopropyl | 4,8 | 535 |
| I-T22-2 | C-CH3 | C-i-C₃F₇ | C-CH3 | H | H | C-H | C-H | C-Cl | C-H | 1-(cyano)cyclopropyl | 4,6 | 560 |
| I-T22-3 | C-CH3 | C-i-C₃F₇ | C-CH3 | H | H | C-H | C-H | C-Cl | C-H | CH2CF3 | 5 | 577 |
| I-T22-4 | C-CF3 | C-i-C₃F₇ | C-CH3 | H | H | C-H | N | C-Cl | C-H | 1-(cyano)cyclopropyl | 4,4 | 615 |
| I-T22-5 | C-CF3 | C-i-C₃F₇ | C-CH3 | H | H | C-H | C-H | C-Cl | C-H | cyclopropyl | 4,9 | 589 |
| I-T22-6 | C-CF3 | C-i-C₃F₇ | C-CH3 | H | H | C-H | N | C-Cl | C-H | cyclopropyl | 4,5 | 590 |
| I-T22-7 | C-CF3 | C-i-C₃F₇ | C-CH3 | H | H | C-H | C-H | C-Cl | C-H | 1-(cyano)cyclopropyl | 4,7 | 614 |

**Tabelle I-T23**

| | | | | | | | | | | | | | |
|---|---|---|---|---|---|---|---|---|---|---|---|---|---|
| 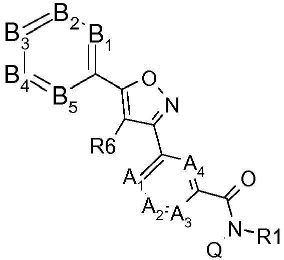 | | | | | | | | | | | | | |
| B₂ und B₄ = C-H, R⁶=R¹¹ | | | | | | | | | | | | | |

| **Bsp.-Nr.** | **B₁** | **B₃** | **B₅** | **R¹** | **R¹¹** | **A₁** | **A₂** | **A₃** | **A₄** | **W** | **Q** | **logP^{a)}** | **Masse [m/z] ^{a)1)}** |
|---|---|---|---|---|---|---|---|---|---|---|---|---|---|
| I-T23-1 | C-Cl | C-i-C₃F₇ | C-Cl | H | H | C-H | C-H | C-Cl | C-H | O | cyclopropyl | 5,0 | 575 |
| I-T23-2 | C-Cl | C-i-C₃F₇ | C-Cl | H | H | C-H | C-H | C-Cl | C-H | O | 1-(cyano)cyclopropyl | 4,8 | 600 |

**Tabelle I-T46**

| | | | | | | | | | | | | | | | |
|---|---|---|---|---|---|---|---|---|---|---|---|---|---|---|---|
| 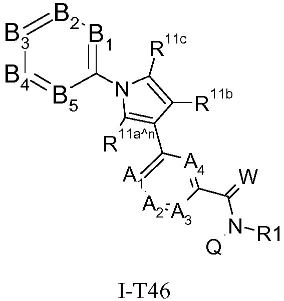 | | | | | | | | | | | | | | | |
| B₂ und B₄ = C-H | | | | | | | | | | | | | | | |

| **Bsp.-Nr.** | **B₁** | **B₃** | **B₅** | **R^{11a}** | **R^{11b}** | **R^{11c}** | **A₁** | **A₂** | **A₃** | **A₄** | **W** | **R₁** | **Q** | **logP^{a)}** | **Masse [m/z] ^{a)1)}** |
|---|---|---|---|---|---|---|---|---|---|---|---|---|---|---|---|
| I-T46-1 | C-CH₃ | C-i-C₃F₇ | C-CH₃ | H | H | H | C-H | C-H | C-Cl | C-H | O | H | 1-(cyano)cyclopropyl | 4,9 | 558 |
| I-T46-2 | C-CH₃ | C-i-C₃F₇ | C-CH₃ | H | H | H | C-H | C-H | C-Cl | C-H | O | H | cyclopropyl | 5,0 | 533 |
| I-T46-3 | C-Cl | C-i-C₃F₇ | C-Cl | H | H | H | C-H | N | C-Cl | C-H | O | CH₃ | cyclopropyl | 5,0 | 588 |
| I-T46-4 | C-Cl | C-i-C₃F₇ | C-Cl | H | H | H | C-H | N | C-Cl | C-H | O | H | cyclopropyl | 4,5 | 574 |
| I-T46-5 | C-Cl | C-i-C₃F₇ | C-Cl | H | H | H | C-H | N | C-Cl | C-H | O | H | 1-(cyano)cyclopropyl | 4,4 | 599 |
| I-T46-6 | C-Cl | C-i-C₃F₇ | C-Cl | H | H | H | C-H | N | C-Cl | C-H | O | CH₃ | 1-(cyano)cyclopropyl | 4,8 | 613 |

### NMR-Daten ausgewählter Beispiele

Die ¹H-NMR-Daten ausgewählter Beispiele werden in Form von ¹H-NMR-Peaklisten notiert. Zu jedem Signalpeak wird erst der δ-Wert in ppm und dann die Signalintensität in runden Klammern aufgeführt. Die δ-Wert - Signalintensitäts- Zahlenpaare von verschiedenen Signalpeaks werden durch Semikolons voneinander getrennt aufgelistet.

Die Peakliste eines Beispieles hat daher die Form:
δ₁ (Intensität₁); δ₂ (Intensität₂);........; δᵢ (Intensitätᵢ); ......; δₙ (Intensitätₙ)

Die Intensität scharfer Signale korreliert mit der Höhe der Signale in einem gedruckten Beispiel eines NMR-Spektrums in cm und zeigt die wirklichen Verhältnisse der Signalintensitäten. Bei breiten Signalen können mehrere Peaks oder die Mitte des Signals und ihre relative Intensität im Vergleich zum intensivsten Signal im Spektrum gezeigt werden.

Zur Kalibrierung der chemischen Verschiebung von ¹H-NMR-Spektren benutzen wir Tetramethylsilan und/oder die chemische Verschiebung des Lösungsmittels, besondern im Falle von Spektren, die in DMSO gemessen werden. Daher kann in NMR-Peaklisten der Tetramethylsilan-Peak vorkommen, muss es aber nicht.

Die Listen der ¹H-NMR-Peaks sind ähnlich den klassischen ¹H-NMR-Ausdrucken und enthalten somit gewöhnlich alle Peaks, die bei einer klassischen NMR-Interpretation aufgeführt werden.

Darüber hinaus können sie wie klassische ¹H-NMR-Ausdrucke Lösungsmittelsignale, Signale von Stereoisomeren der Zielverbindungen, die ebenfalls Gegenstand der Erfindung sind, und/oder Peaks von Verunreinigungen zeigen.

Bei der Angabe von Verbindungssignalen im Delta-Bereich von Lösungsmitteln und/oder Wasser sind in unseren Listen von 1H-NMR-Peaks die gewöhnlichen Lösungsmittelpeaks, zum Beispiel Peaks von DMSO in DMSO-D6 und der Peak von Wasser, gezeigt, die gewöhnlich im Durchschnitt eine hohe Intensität aufweisen.

Die Peaks von Stereoisomeren der Targetverbindungen und/oder Peaks von Verunreinigungen haben gewöhnlich im Durchschnitt eine geringere Intensität als die Peaks der Zielverbindungen (zum Beispiel mit einer Reinheit von >90%).

Solche Stereoisomere und/oder Verunreinigungen können typisch für das jeweilige Herstellungsverfahren sein. Ihre Peaks können somit dabei helfen, die Reproduktion unseres Herstellungsverfahrens anhand von "Nebenprodukt-Fingerabdrücken" zu erkennen.

Einem Experten, der die Peaks der Zielverbindungen mit bekannten Verfahren (MestreC, ACD-Simulation, aber auch mit empirisch ausgewerteten Erwartungswerten) berechnet, kann je nach Bedarf die Peaks der Zielverbindungen isolieren, wobei gegebenenfalls zusätzliche Intensitätsfilter eingesetzt werden. Diese Isolierung wäre ähnlich dem betreffenden Peak-Picking bei der klassischen 1H-NMR-Interpretation.

Weitere Details zu 1H-NMR-Peaklisten können der Research Disclosure Database Number 564025 entnommen werden.

| |
|---|
| Beispiel I-T2-1: ¹H-NMR (400,0 MHz, CD3CN): δ= 8,297 (5,2); 8,291 (5,1); 7,846 (9,6); 7,825 (3,3); 7,818 (2,0); 7,545 (3,5); 7,525 (2,9); 7,523 (2,8); 7,443 (9,1); 6,977 (1,2); 6,544 (5,1); 6,538 (4,9); 4,085 (0,4); 4,068 (1,3); 4,050 (1,4); 4,032 (0,5); 3,440 (0,4); 3,374 (0,4); 2,862 (0,8); 2,853 (1,2); 2,844 (1,9); 2,834 (1,8); 2,826 (1,3); 2,816 (0,9); 2,240 (41,5); 2,150 (23,6); 2,086 (3,2); 1,972 (6,1); 1,965 (1,2); 1,958 (2,9); 1,953 (13,9); 1,947 (24,8); 1,941 (32,9); 1,934 (22,7); 1,928 (11,7); 1,436 (16,0); 1,269 (0,5); 1,221 (1,6); 1,204 (3,1); 1,186 (1,5); 0,790 (1,0); 0,778 (3,2); 0,773 (4,1); 0,760 (4,2); 0,755 (3,2); 0,743 (1,4); 0,614 (1,3); 0,604 (3,7); 0,597 (3,9); 0,593 (3,4); 0,588 (3,3); 0,575 (1,0); 0,000 (3,0) |
| Beispiel I-T2-2: ¹H-NMR (400,0 MHz, CD3CN): δ= 8,311 (4,9); 8,304 (5,0); 7,898 (10,0); 7,878 (3,3); 7,872 (2,0); 7,624 (2,0); 7,583 (3,2); 7,561 (2,8); 7,444 (9,7); 6,555 (4,9); 6,549 (4,9); 5,447 (0,7); 4,086 (0,5); 4,068 (1,6); 4,050 (1,6); 4,032 (0,6); 2,240 (45,7); 2,146 (80,1); 2,114 (0,7); 2,108 (0,7); 2,102 (0,5); 1,972 (7,1); 1,964 (3,1); 1,958 (8,0); 1,953 (36,5); 1,946 (65,9); 1,940 (87,5); 1,934 (62,1); 1,928 (33,1); 1,775 (0,4); 1,769 (0,6); 1,763 (0,4); 1,591 (2,0); 1,576 (5,7); 1,569 (5,7); 1,556 (2,8); 1,516 (0,4); 1,437 (16,0); 1,410 (0,4); 1,369 (2,8); 1,356 (5,7); 1,349 (6,0); 1,334 (2,1); 1,296 (0,3); 1,269 (1,9); 1,222 (1,9); 1,204 (3,6); 1,186 (1,8); 0,000 (4,5) |
| Beispiel I-T3-1: ¹H-NMR (400,0 MHz, CD3CN): δ= 8,118 (11,7); 8,061 (12,6); 7,689 (7,9); 7,683 (8,6); 7,667 (0,7); 7,652 (4,9); 7,646 (3,5); 7,631 (5,5); 7,625 (4,3); 7,535 (16,0); 7,463 (7,8); 7,442 (6,2); 6,895 (2,5); 4,068 (0,8); 4,050 (0,8); 3,912 (0,7); 2,881 (0,5); 2,871 (1,5); 2,862 (2,1); 2,853 (3,2); 2,844 (3,2); 2,835 (2,2); 2,826 (1,5); 2,816 (0,5); 2,270 (0,5); 2,261 (0,3); 2,143 (107,9); 2,138 (145,5); 2,111 (71,2); 1,972 (5,1); 1,964 (10,3); 1,958 (27,0); 1,952 (94,0); 1,946 (160,4); 1,940 (199,1); 1,934 (136,5); 1,928 (68,3); 1,780 (0,5); 1,774 (0,9); 1,768 (1,1); 1,762 (0,8); 1,756 (0,4); 1,437 (13,0); 1,271 (1,0); 1,222 (1,0); 1,204 (1,9); 1,186 (0,9); 0,794 (1,7); 0,782 (6,4); 0,777 (7,2); 0,764 (7,9); 0,759 (5,7); 0,747 (2,4); 0,725 (0,3); 0,610 (2,4); 0,600 (7,1); 0,592 (7,5); 0,588 (6,7); 0,584 (5,9); 0,571 (1,7); 0,146 (0,4); 0,000 (86,0); -0,008 (5,2); -0,150 (0,4) |
| Beispiel I-T3-2: ¹H-NMR (400,0 MHz, CD3CN): δ= 8,131 (12,6); 8,084 (13,3); 7,735 (7,6); 7,729 (9,9); 7,710 (5,2); 7,704 (3,5); 7,689 (5,8); 7,683 (4,6); 7,537 (16,0); 7,514 (8,9); 7,493 (7,4); 7,458 (1,9); 4,140 (1,8); 4,124 (2,1); 4,117 (5,8); 4,100 (5,9); 4,093 (6,2); 4,077 (5,9); 4,069 (2,6); 4,053 (2,1); 3,914 (0,6); 2,891 (0,7); 2,773 (0,6); 2,480 (0,7); 2,475 (1,3); 2,470 (1,8); 2,466 (1,3); 2,461 (0,7); 2,325 (0,4); 2,273 (1,9); 2,221 (979,0); 2,115 (79,7); 2,097 (1,4); 1,973 (3,4); 1,966 (10,4); 1,960 (22,6); 1,954 (102,7); 1,948 (182,7); 1,942 (241,3); 1,936 (167,3); 1,930 (87,0); 1,783 (0,7); 1,777 (1,1); 1,770 (1,5); 1,764 (1,1); 1,758 (0,6); 1,437 (15,2); 1,296 (0,5); 1,270 (1,7); 1,222 (0,7); 1,204 (1,3); 1,186 (0,6); 0,146 (0,3); 0,008 (2,7); 0,000 (69,9); -0,008 (3,3) |
| Beispiel I-T3-3: ¹H-NMR (400,0 MHz, CD3CN): δ= 8,129 (2,6); 8,075 (2,6); 7,738 (1,6); 7,732 (2,0); 7,701 (1,1); 7,696 (0,8); 7,681 (1,2); 7,675 (1,1); 7,572 (0,5); 7,536 (3,2); 7,496 (1,8); 7,475 (1,5); 2,146 (33,6); 2,113 (16,5); 1,972 (0,4); 1,964 (2,4); 1,958 (5,6); 1,953 (29,5); 1,946 (53,8); 1,940 (72,5); 1,934 (50,1); 1,928 (25,8); 1,769 (0,4); 1,599 (0,8); 1,585 (1,9); 1,578 (2,0); 1,564 (1,1); 1,437 (16,0); 1,359 (1,1); 1,345 (2,0); 1,338 (2,0); 1,324 (0,8); 1,269 (0,6); 0,008 (1,9); 0,000 (51,3); -0,009 (2,0) |
| Beispiel I-T3-4: ¹H-NMR (400,0 MHz, CD3CN): δ= 8,132 (5,7); 8,077 (6,1); 7,719 (3,4); 7,714 (4,3); 7,678 (2,2); 7,673 (1,8); 7,657 (2,6); 7,652 (2,3); 7,537 (7,8); 7,515 (1,0); 7,495 (1,0); 7,486 (4,3); 7,465 (3,3); 5,338 (0,9); 5,316 (1,8); 5,296 (1,8); 5,274 (1,0); 3,544 (2,3); 3,521 (4,7); 3,499 (3,1); 3,370 (3,0); 3,349 (4,6); 3,346 (4,3); 3,325 (2,4); 2,469 (0,4); 2,274 (0,4); 2,206 (300,4); 2,154 (0,5); 2,115 (36,2); 1,973 (1,3); 1,966 (3,0); 1,960 (6,3); 1,954 (30,3); 1,948 (55,1); 1,942 (73,7); 1,935 (52,5); 1,929 (28,5); 1,776 (0,4); 1,770 (0,5); 1,764 (0,4); 1,437 (16,0); 1,269 (1,6); 1,222 (0,3); 1,204 (0,6); 0,008 (0,7); 0,000 (20,8) |
| Beispiel I-T3-5: ¹H-NMR (601,6 MHz, CD3CN): δ= 8,125 (8,3); 8,124 (8,5); 8,072 (9,3); 8,071 (9,2); 7,680 (16,0); 7,677 (5,7); 7,668 (5,3); 7,664 (3,0); 7,537 (9,4); 7,485 (4,7); 7,482 (2,1); 7,473 (1,9); 7,470 (4,3); 7,461 (2,3); 2,146 (139,0); 2,113 (53,4); 2,060 (0,5); 2,056 (0,9); 2,052 (1,3); 2,048 (0,9); 2,044 (0,4); 1,966 (5,4); 1,958 (14,4); 1,953 (16,2); 1,950 (93,5); 1,945 (166,6); 1,941 (244,5); 1,937 (162,8); 1,933 (81,9); 1,924 (1,3); 1,835 (0,5); 1,831 (0,9); 1,827 (1,4); 1,822 (0,9); 1,818 (0,5); 1,393 (2,4); 1,383 (5,5); 1,380 (5,9); 1,370 (3,2); 1,343 (0,4); 1,269 (0,6); 1,251 (1,3); 1,248 (1,4); 1,238 (4,5); 1,228 (1,0); 1,225 (1,0); 0,096 (0,4); 0,005 (3,0); 0,000 (105,8); -0,006 (3,3); -0,100 (0,4) |
| Beispiel I-T3-6: ¹H-NMR (601,6 MHz, CD3CN): δ= 8,130 (11,9); 8,129 (12,8); 8,082 (13,1); 8,081 (13,4); 7,809 (8,6); 7,805 (9,0); 7,697 (5,1); 7,693 (4,8); 7,683 (5,9); 7,679 (5,7); 7,538 (13,6); 7,508 (9,3); 7,494 (8,0); 7,304 (1,1); 7,295 (1,9); 7,286 (1,1); 7,069 (1,2); 4,045 (16,0); 4,036 (15,9); 3,973 (1,6); 3,962 (1,8); 3,957 (5,2); 3,946 (5,2); 3,941 (5,5); 3,930 (5,3); 3,926 (2,0); 3,915 (1,8); 2,220 (0,4); 2,153 (19,3); 2,115 (73,8); 2,104 (1,0); 2,052 (0,4); 2,006 (0,4); 1,966 (1,4); 1,958 (3,8); 1,953 (4,5); 1,950 (25,7); 1,945 (45,5); 1,941 (67,9); 1,937 (46,1); 1,933 (22,7); 1,827 (0,4); 1,268 (1,1); 0,005 (0,8); 0,000 (28,7); -0,006 (0,9) |
| Beispiel I-T3-7: ¹H-NMR (400,0 MHz, d₆-DMSO): δ= 9,043 (1,2); 9,038 (1,2); 8,803 (1,3); 8,798 (1,3); 8,672 (3,2); 8,648 (0,9); 8,638 (0,9); 8,405 (3,2); 8,390 (1,1); 8,384 (1,9); 8,379 (1,0); 8,316 (0,3); 7,602 (3,5); 3,902 (9,7); 3,330 (83,7); 3,243 (0,6); 3,169 (0,4); 2,903 (0,3); 2,893 (0,5); 2,885 (0,7); 2,875 (0,7); 2,867 (0,4); |
| 2,857 (0,3); 2,676 (0,4); 2,672 (0,5); 2,667 (0,4); 2,525 (1,4); 2,512 (30,7); 2,507 (61,4); 2,503 (80,2); 2,498 (57,8); 2,494 (27,6); 2,334 (0,3); 2,329 (0,5); 2,325 (0,3); 2,131 (16,0); 1,909 (0,3); 0,763 (0,4); 0,750 (1,1); 0,745 (1,6); 0,733 (1,5); 0,727 (1,3); 0,716 (0,6); 0,619 (0,6); 0,608 (1,7); 0,602 (1,4); 0,598 (1,4); 0,593 (1,2); 0,580 (0,4); 0,000 (9,2) |
| Beispiel I-T3-8: ¹H-NMR (400,0 MHz, d₆-DMSO): δ= 9,538 (2,0); 9,102 (1,4); 9,097 (1,4); 8,827 (1,4); 8,823 (1,5); 8,695 (3,2); 8,444 (1,1); 8,439 (1,9); 8,434 (1,2); 8,424 (3,3); 7,605 (3,8); 3,902 (6,0); 3,374 (0,4); 3,330 (90,4); 3,243 (0,4); 3,169 (2,1); 2,676 (0,4); 2,672 (0,6); 2,667 (0,5); 2,542 (0,5); 2,507 (75,8); 2,503 (97,5); 2,498 (74,7); 2,334 (0,4); 2,329 (0,6); 2,325 (0,5); 2,132 (16,0); 1,628 (0,7); 1,614 (2,0); 1,607 (2,1); 1,594 (0,9); 1,347 (0,9); 1,334 (2,1); 1,327 (2,1); 1,313 (0,7); 0,000 (8,2) |
| Beispiel I-T3-9: ¹H-NMR (400,0 MHz, d₆-DMSO): δ= 8,508 (2,0); 8,506 (2,0); 8,473 (1,0); 8,463 (1,0); 8,276 (2,2); 7,895 (0,5); 7,876 (1,0); 7,861 (0,5); 7,592 (4,1); 7,399 (0,5); 7,383 (1,0); 7,368 (0,7); 7,305 (1,1); 7,286 (1,7); 7,266 (0,7); 3,902 (5,7); 3,330 (72,5); 3,243 (0,4); 3,175 (0,4); 3,162 (0,3); 2,875 (0,4); 2,865 (0,5); 2,857 (0,7); 2,847 (0,7); 2,838 (0,5); 2,828 (0,3); 2,672 (0,5); 2,507 (63,9); 2,503 (80,2); 2,329 (0,5); 2,114 (16,0); 0,725 (0,4); 0,707 (1,8); 0,695 (1,7); 0,689 (1,5); 0,678 (0,6); 0,556 (0,6); 0,545 (1,8); 0,539 (1,8); 0,530 (1,6); 0,518 (0,5); 0,000 (6,1) |
| Beispiel I-T3-10: ¹H-NMR (400,0 MHz, d₆-DMSO): δ= 9,369 (2,0); 8,535 (1,8); 8,532 (1,9); 8,290 (2,0); 7,968 (0,4); 7,965 (0,5); 7,949 (0,9); 7,946 (0,9); 7,931 (0,5); 7,927 (0,5); 7,595 (3,9); 7,479 (0,4); 7,475 (0,4); 7,459 (0,9); 7,443 (0,6); 7,440 (0,5); 7,352 (1,0); 7,333 (1,7); 7,314 (0,8); 3,903 (8,2); 3,372 (0,4); 3,329 (104,6); 3,243 (0,6); 3,175 (0,3); 2,675 (0,4); 2,671 (0,5); 2,667 (0,4); 2,541 (0,5); 2,507 (70,8); 2,502 (89,6); 2,498 (66,7); 2,333 (0,4); 2,329 (0,5); 2,324 (0,4); 2,115 (16,0); 1,598 (0,8); 1,584 (2,0); 1,577 (2,1); 1,564 (0,9); 1,292 (0,9); 1,278 (2,1); 1,272 (2,2); 1,257 (0,8); 1,169 (0,3); 1,068 (0,4); 0,007 (0,4); 0,000 (7,5); -0,008 (0,4) |
| Beispiel I-T3-11: ¹H-NMR (400,0 MHz, d₆-DMSO): δ= 8,522 (0,9); 8,512 (0,9); 8,500 (3,3); 8,211 (3,2); 7,818 (1,8); 7,799 (1,8); 7,646 (1,8); 7,621 (1,8); 7,590 (3,5); 3,902 (3,1); 3,330 (117,7); 3,304 (0,3); 2,861 (0,3); 2,851 (0,4); 2,842 (0,7); 2,832 (0,7); 2,824 (0,4); 2,676 (0,4); 2,671 (0,5); 2,667 (0,3); 2,525 (1,5); 2,511 (29,4); 2,507 (58,1); 2,502 (75,7); 2,498 (54,9); 2,493 (26,6); 2,334 (0,3); 2,329 (0,5); 2,324 (0,3); 2,127 (16,0); 0,733 (0,4); 0,720 (1,2); 0,715 (1,7); 0,703 (1,6); 0,697 (1,3); 0,685 (0,6); 0,575 (0,6); 0,564 (1,7); 0,558 (1,5); 0,554 (1,4); 0,548 (1,3); 0,536 (0,4); 0,000 (7,9) |
| Beispiel I-T3-12: ¹H-NMR (400,0 MHz, d₆-DMSO): δ= 9,396 (1,8); 8,522 (3,2); 8,223 (3,2); 7,906 (1,6); 7,887 (1,6); 7,712 (1,6); 7,687 (1,6); 7,593 (3,7); 3,903 (2,7); 3,332 (98,2); 2,672 (0,5); 2,542 (0,4); 2,507 (67,0); 2,503 (83,7); 2,498 (62,0); 2,334 (0,4); 2,329 (0,5); 2,325 (0,4); 2,129 (16,0); 1,604 (0,8); 1,590 (2,0); 1,583 (2,1); 1,570 (0,9); 1,312 (0,9); 1,299 (2,0); 1,292 (2,0); 1,278 (0,8); 0,000 (7,1) |
| Beispiel I-T3-13: ¹H-NMR (400,0 MHz, d₆-DMSO): δ= 8,514 (1,8); 8,510 (1,9); 8,480 (0,9); 8,470 (0,9); 8,285 (2,1); 8,226 (0,8); 8,220 (0,8); 8,207 (0,8); 8,202 (0,8); 7,767 (0,4); 7,762 (0,5); 7,755 (0,5); 7,749 (0,6); 7,746 (0,6); 7,740 (0,5); 7,734 (0,5); 7,728 (0,4); 7,595 (3,7); 7,402 (0,9); 7,380 (0,9); 7,375 (1,0); 7,354 (0,8); 3,902 (1,5); 3,444 (0,4); 3,425 (0,6); 3,405 (1,1); 3,353 (438,8); 3,292 (0,5); 3,273 (0,3); 2,864 (0,5); 2,855 (0,7); 2,846 (0,7); 2,837 (0,5); 2,827 (0,3); 2,678 (0,4); 2,673 (0,5); 2,669 (0,4); 2,509 (61,5); 2,504 (78,8); 2,500 (57,5); 2,335 (0,4); 2,331 (0,5); 2,327 (0,4); 2,121 (16,0); 0,746 (0,4); 0,733 (1,2); 0,728 (1,7); 0,716 (1,6); 0,710 (1,3); 0,699 (0,6); 0,602 (0,6); 0,592 (1,7); 0,585 (1,6); 0,576 (1,3); 0,564 (0,4) |
| Beispiel I-T3-14: ¹H-NMR (400,0 MHz, d₆-DMSO): δ= 8,600 (1,8); 8,596 (1,8); 8,536 (1,1); 8,525 (1,1); 8,352 (1,9); 7,977 (1,6); 7,960 (1,6); 7,594 (3,7); 7,437 (1,7); 7,411 (1,7); 3,902 (4,8); 3,332 (129,0); 2,826 (0,4); 2,817 (0,7); 2,807 (0,7); 2,799 (0,4); 2,789 (0,3); 2,676 (0,4); 2,672 (0,5); 2,667 (0,4); 2,511 (32,0); 2,507 (61,8); 2,503 (79,3); 2,498 (57,6); 2,494 (28,2); 2,334 (0,3); 2,329 (0,5); 2,325 (0,4); 2,115 (16,0); 0,733 (0,4); 0,720 (1,2); 0,715 (1,6); 0,703 (1,5); 0,697 (1,3); 0,685 (0,5); 0,567 (0,5); 0,557 (1,7); 0,551 (1,5); 0,547 (1,4); 0,541 (1,3); 0,529 (0,4); 0,000 (6,1) |
| Beispiel I-T3-15: ¹H-NMR (400,0 MHz, d₆-DMSO): δ= 8,711 (3,3); 8,694 (1,1); 8,684 (1,0); 8,450 (3,3); 8,369 (1,8); 8,174 (1,8); 7,971 (1,8); 7,601 (3,9); 3,903 (1,0); 3,331 (150,2); 2,910 (0,3); 2,901 (0,5); 2,892 (0,7); 2,882 (0,7); 2,874 (0,5); 2,865 (0,4); 2,672 (0,5); 2,507 (59,3); 2,503 (74,7); 2,499 (55,2); 2,334 (0,4); 2,330 (0,5); 2,133 (16,0); 0,769 (0,4); 0,755 (1,3); 0,751 (1,7); 0,738 (1,6); 0,733 (1,4); 0,721 (0,6); 0,630 (0,6); 0,620 (1,8); 0,613 (1,7); 0,604 (1,4); 0,592 (0,4) |
| Beispiel I-T3-16: ¹H-NMR (601,6 MHz, d₆-DMSO): δ= 8,439 (0,8); 8,433 (0,9); 8,344 (3,1); 8,343 (3,2); 8,082 (3,2); 8,081 (3,3); 7,912 (1,7); 7,909 (1,7); 7,670 (0,9); 7,667 (0,9); 7,657 (1,0); 7,654 (1,0); 7,600 (3,4); 7,368 (1,3); 7,354 (1,2); 3,376 (0,5); 3,367 (1,1); 3,351 (401,9); 3,328 (0,5); 3,324 (0,5); 2,997 (3,2); 2,856 (0,4); 2,850 (0,7); 2,844 (0,7); 2,838 (0,4); 2,831 (0,3); 2,618 (0,4); 2,615 (0,6); 2,612 (0,4); 2,543 (5,5); 2,524 (1,0); 2,521 (1,3); 2,518 (1,2); 2,509 (30,4); 2,506 (67,4); 2,503 (93,3); 2,500 (68,5); 2,497 (31,5); 2,438 (7,5); 2,390 (0,4); 2,387 (0,6); 2,384 (0,4); 2,146 (16,0); 0,715 (0,4); 0,707 (1,2); 0,703 (1,6); 0,695 (1,5); 0,692 (1,3); 0,684 (0,5); 0,590 (0,6); 0,583 (1,6); 0,579 (1,4); 0,576 (1,3); 0,572 (1,3); 0,564 (0,4); 0,000 (2,6) |
| Beispiel I-T3-17: ¹H-NMR (400,0 MHz, d₆-DMSO): δ= 9,576 (0,4); 9,437 (2,3); 8,736 (0,7); 8,625 (1,7); 8,621 (1,8); 8,472 (0,7); 8,411 (0,4); 8,375 (1,8); 8,372 (1,9); 8,316 (0,5); 8,243 (0,4); 8,030 (1,7); 8,013 (1,6); 7,993 (0,3); 7,597 (3,8); 7,547 (1,7); 7,521 (1,9); 4,036 (1,1); 3,903 (6,7); 3,630 (0,4); 3,623 (0,4); 3,614 (0,4); 3,608 (0,3); 3,597 (0,3); 3,392 (0,7); 3,332 (259,6); 3,287 (0,3); 3,175 (0,4); 3,162 (0,5); 3,155 (0,4); 3,145 (0,5); 3,138 (0,4); 3,127 (0,5); 3,056 (0,4); 3,022 (4,3); 2,751 (0,4); 2,690 (1,7); 2,676 (0,8); 2,672 (1,1); 2,667 (0,9); 2,525 (3,7); 2,511 (72,5); 2,507 (143,5); 2,503 (187,7); 2,498 (136,4); 2,494 (66,3); 2,338 (0,4); 2,334 (0,8); 2,329 (1,1); 2,325 (0,8); 2,134 (3,5); 2,116 (16,0); 1,614 (0,5); 1,607 (1,1); 1,593 (1,9); 1,586 (2,0); 1,573 (0,8); 1,344 (0,5); 1,337 (0,5); 1,310 (0,9); 1,296 (1,9); 1,289 (2,1); 1,274 (3,8); 1,259 (6,5); 1,244 (6,0); 1,225 (1,2); 0,008 (0,5); 0,000 (16,5); -0,009 (0,5) |
| Beispiel I-T3-18: ¹H-NMR (400,0 MHz, d₆-DMSO): δ= 9,576 (2,0); 8,736 (3,2); 8,472 (3,3); 8,411 (1,7); 8,316 (0,3); 8,243 (1,6); 7,994 (1,6); 7,605 (3,6); 3,903 (10,2); 3,372 (0,7); 3,333 (152,4); 3,243 (1,3); 3,175 (0,4); 3,162 (0,4); 2,690 (0,4); 2,676 (0,4); 2,672 (0,6); 2,667 (0,4); 2,542 (0,6); 2,525 (1,9); 2,512 (39,2); 2,507 (77,8); 2,503 (101,6); 2,498 (73,7); 2,494 (35,7); 2,334 (0,4); 2,330 (0,6); 2,325 (0,4); 2,134 (16,0); 2,116 (0,5); 1,629 (0,7); 1,614 (1,8); 1,607 (2,0); 1,594 (0,9); 1,358 (0,9); 1,345 (1,9); 1,338 (2,0); 1,323 (0,7); 1,259 (0,5); 1,244 (0,4); 1,017 (0,6); 1,001 (0,6); 0,000 (9,1) |
| Beispiel I-T3-19: ¹H-NMR (400,0 MHz, d₆-DMSO): δ= 9,367 (1,9); 8,537 (1,8); 8,534 (1,9); 8,300 (2,2); 8,277 (0,8); 8,271 (0,9); 8,259 (0,9); 8,253 (0,8); 7,796 (0,4); 7,791 (0,5); 7,784 (0,5); |
| 7,778 (0,6); 7,775 (0,6); 7,769 (0,6); 7,763 (0,5); 7,757 (0,5); 7,598 (3,8); 7,457 (0,8); 7,435 (0,8); 7,430 (1,0); 7,409 (0,7); 3,903 (4,8); 3,335 (104,3); 2,672 (0,4); 2,542 (0,3); 2,507 (54,0); 2,503 (69,3); 2,499 (52,7); 2,330 (0,4); 2,122 (16,0); 1,609 (0,8); 1,595 (2,0); 1,588 (2,1); 1,575 (0,9); 1,323 (0,9); 1,309 (2,0); 1,303 (2,1); 1,288 (0,8); 0,000 (5,6) |
| Beispiel I-T3-20: ¹H-NMR (400,0 MHz, d₆-DMSO): δ= 8,816 (2,3); 8,810 (2,3); 8,695 (3,4); 8,681 (1,1); 8,670 (1,1); 8,451 (3,4); 8,184 (2,3); 8,178 (2,2); 7,601 (3,8); 3,903 (7,0); 3,333 (173,9); 3,289 (0,4); 3,242 (0,9); 3,175 (0,6); 3,162 (0,5); 2,859 (0,4); 2,850 (0,7); 2,840 (0,7); 2,832 (0,4); 2,822 (0,3); 2,676 (0,4); 2,671 (0,5); 2,667 (0,4); 2,542 (0,4); 2,511 (34,1); 2,507 (66,3); 2,502 (85,9); 2,498 (63,0); 2,494 (31,0); 2,333 (0,4); 2,329 (0,5); 2,325 (0,4); 2,132 (0,7); 2,117 (16,0); 1,016 (0,4); 1,001 (0,4); 0,755 (0,5); 0,742 (1,3); 0,737 (1,7); 0,725 (1,6); 0,719 (1,4); 0,707 (0,5); 0,564 (0,6); 0,553 (1,7); 0,547 (1,6); 0,543 (1,5); 0,538 (1,4); 0,526 (0,4); 0,000 (7,1) |
| Beispiel I-T3-21: ¹H-NMR (600,1 MHz, d₆-DMSO): δ= 9,593 (1,8); 8,868 (2,3); 8,864 (2,3); 8,709 (3,3); 8,456 (3,4); 8,262 (2,3); 8,258 (2,2); 7,603 (3,7); 3,388 (0,4); 3,383 (0,4); 3,381 (0,5); 3,369 (0,8); 3,340 (1164,0); 2,994 (0,7); 2,617 (0,6); 2,615 (0,8); 2,612 (0,6); 2,542 (39,9); 2,523 (1,4); 2,520 (1,7); 2,517 (1,8); 2,508 (48,7); 2,505 (103,1); 2,502 (140,8); 2,499 (100,9); 2,497 (46,2); 2,389 (0,6); 2,386 (0,8); 2,383 (0,6); 2,117 (16,0); 1,636 (0,8); 1,627 (2,0); 1,622 (2,1); 1,613 (0,8); 1,288 (0,9); 1,278 (1,9); 1,274 (2,1); 1,264 (0,8); 0,005 (0,8); 0,000 (21,9); -0,006 (0,7) |
| Beispiel I-T3-22: ¹H-NMR (400,1 MHz, d₆-DMSO): δ= 9,397 (6,5); 8,682 (5,3); 8,677 (5,1); 8,405 (7,9); 8,366 (0,3); 8,087 (16,0); 8,060 (0,3); 8,041 (0,7); 7,981 (1,7); 7,966 (2,6); 7,963 (2,7); 7,948 (1,5); 7,944 (1,4); 7,763 (0,3); 7,505 (1,2); 7,501 (1,3); 7,486 (2,7); 7,470 (1,8); 7,466 (1,6); 7,384 (0,6); 7,374 (3,1); 7,365 (1,1); 7,355 (5,1); 7,345 (0,8); 7,336 (2,3); 5,761 (0,8); 3,348 (68,6); 3,028 (1,2); 2,875 (1,0); 2,712 (0,4); 2,671 (0,3); 2,542 (99,6); 2,507 (38,4); 2,502 (50,3); 2,498 (38,2); 2,368 (0,4); 2,087 (0,3); 1,601 (2,4); 1,587 (6,3); 1,580 (6,5); 1,567 (2,8); 1,288 (3,1); 1,275 (6,2); 1,268 (6,6); 1,254 (2,5); 1,234 (0,8); 1,169 (0,9); 0,146 (0,3); 0,000 (72,5); -0,008 (4,9); -0,150 (0,4) |
| Beispiel I-T3-23: ¹H-NMR (400,0 MHz, CD3CN): δ= 8,202 (5,5); 8,187 (0,5); 8,161 (5,9); 8,146 (0,5); 7,931 (0,4); 7,900 (9,0); 7,886 (0,8); 7,739 (3,5); 7,734 (4,4); 7,720 (0,5); 7,707 (2,6); 7,701 (1,9); 7,686 (3,0); 7,680 (2,5); 7,648 (1,5); 7,507 (4,1); 7,486 (3,4); 4,360 (0,5); 4,342 (0,5); 4,086 (1,0); 4,068 (2,9); 4,050 (3,0); 4,032 (1,1); 2,162 (61,1); 2,149 (10,3); 2,120 (0,4); 2,114 (0,4); 2,108 (0,5); 2,102 (0,4); 1,972 (13,4); 1,965 (4,8); 1,959 (8,2); 1,953 (30,5); 1,947 (52,3); 1,940 (68,1); 1,934 (48,2); 1,928 (27,3); 1,769 (0,4); 1,596 (1,8); 1,581 (4,5); 1,574 (4,4); 1,561 (2,8); 1,437 (16,0); 1,422 (1,2); 1,401 (0,5); 1,371 (0,6); 1,361 (2,6); 1,353 (1,9); 1,347 (4,6); 1,341 (4,6); 1,336 (1,5); 1,326 (2,3); 1,268 (1,6); 1,222 (3,7); 1,204 (7,2); 1,186 (3,8); 0,000 (2,2) |
| Beispiel I-T3-24: ¹H-NMR (600,1 MHz, CD3CN): δ= 8,189 (6,2); 8,144 (6,7); 7,897 (10,1); 7,690 (3,8); 7,687 (4,5); 7,652 (2,6); 7,648 (2,1); 7,638 (2,8); 7,634 (2,4); 7,471 (4,4); 7,457 (3,7); 6,891 (1,0); 5,446 (2,0); 4,077 (2,0); 4,065 (6,2); 4,053 (6,2); 4,041 (2,1); 2,864 (0,8); 2,857 (1,1); 2,852 (1,7); 2,845 (1,7); 2,839 (1,1); 2,833 (0,8); 2,129 (55,4); 2,054 (0,5); 2,050 (0,7); 2,046 (0,5); 1,971 (27,5); 1,963 (6,3); 1,955 (8,8); 1,951 (10,1); 1,947 (46,9); 1,943 (78,2); 1,939 (115,2); 1,935 (77,9); 1,931 (39,3); 1,922 (0,5); 1,828 (0,4); 1,824 (0,6); 1,820 (0,4); 1,437 (16,0); 1,270 (0,4); 1,216 (7,4); 1,204 (14,6); 1,192 (7,3); 0,786 (1,0); 0,777 (2,8); 0,774 (3,6); 0,765 (3,6); 0,762 (2,7); 0,754 (1,2); 0,606 (1,1); 0,599 (2,9); 0,598 (2,9); 0,595 (3,0); 0,591 (2,8); 0,588 (2,8); 0,580 (0,9); 0,005 (2,1); 0,000 (69,9); -0,006 (2,2) |
| Beispiel I-T3-25: ¹H-NMR (601,6 MHz, CD3CN): δ= 8,201 (9,9); 8,163 (10,8); 7,899 (16,0); 7,738 (5,9); 7,734 (7,4); 7,710 (4,2); 7,706 (3,3); 7,696 (4,6); 7,693 (4,0); 7,522 (6,8); 7,508 (5,8); 7,328 (1,4); 7,265 (0,6); 7,251 (1,5); 7,239 (1,1); 7,195 (1,4); 7,183 (1,0); 7,162 (0,5); 7,150 (0,7); 5,446 (1,0); 4,127 (1,3); 4,116 (1,5); 4,111 (4,2); 4,100 (4,3); 4,095 (4,5); 4,085 (4,3); 4,080 (1,7); 4,069 (1,4); 2,328 (5,9); 2,134 (32,5); 2,132 (53,6); 2,058 (0,4); 2,054 (0,6); 2,050 (1,0); 2,046 (0,7); 1,971 (1,2); 1,964 (7,9); 1,955 (12,2); 1,951 (13,8); 1,947 (67,1); 1,943 (113,1); 1,939 (165,4); 1,935 (111,4); 1,931 (56,0); 1,833 (0,5); 1,829 (0,7); 1,825 (0,9); 1,821 (0,7); 1,437 (5,7); 1,269 (0,8); 1,204 (0,6); 1,192 (0,3); 0,000 (1,4) |
| Beispiel I-T3-26: ¹H-NMR (601,6 MHz, CD3CN): δ= 8,222 (8,5); 8,184 (9,2); 8,183 (8,4); 7,933 (13,8); 7,743 (5,6); 7,739 (6,7); 7,712 (3,8); 7,709 (3,0); 7,699 (4,2); 7,695 (3,6); 7,530 (6,3); 7,516 (5,4); 7,228 (0,3); 7,216 (0,4); 7,172 (1,1); 5,481 (0,5); 4,022 (0,9); 3,653 (2,8); 3,641 (6,5); 3,631 (6,6); 3,620 (2,9); 2,605 (0,6); 2,594 (1,2); 2,586 (2,0); 2,582 (0,9); 2,575 (3,7); 2,568 (2,2); 2,564 (2,1); 2,556 (3,9); 2,549 (0,9); 2,545 (2,0); 2,538 (1,3); 2,527 (0,6); 2,505 (0,6); 2,502 (1,0); 2,499 (1,4); 2,496 (1,0); 2,361 (1,2); 2,216 (233,1); 2,214 (233,8); 2,213 (216,8); 2,211 (239,7); 2,210 (216,4); 2,206 (358,9); 2,092 (0,7); 2,088 (1,2); 2,084 (1,6); 2,080 (1,2); 2,076 (0,6); 2,005 (1,3); 1,998 (14,1); 1,990 (21,9); 1,985 (26,7); 1,982 (121,7); 1,978 (205,2); 1,973 (304,1); 1,969 (210,4); 1,965 (107,9); 1,867 (0,7); 1,863 (1,2); 1,859 (1,7); 1,855 (1,2); 1,850 (0,6); 1,470 (16,0); 1,303 (0,4); 1,237 (0,6); 0,033 (1,8) |
| Beispiel I-T3-27: ¹H-NMR (400,0 MHz, d₆-DMSO): δ= 9,439 (6,6); 8,693 (9,5); 8,441 (9,4); 8,317 (0,9); 8,274 (16,0); 7,807 (2,2); 7,802 (3,8); 7,792 (5,8); 7,786 (9,9); 7,569 (5,1); 7,553 (1,2); 7,546 (4,2); 4,020 (0,3); 3,568 (10,9); 3,328 (401,1); 2,675 (2,2); 2,671 (2,9); 2,666 (2,2); 2,506 (372,0); 2,502 (465,0); 2,497 (338,4); 2,333 (2,3); 2,328 (2,9); 2,324 (2,1); 1,989 (1,3); 1,615 (2,4); 1,601 (6,2); 1,594 (6,2); 1,581 (2,5); 1,398 (5,4); 1,287 (2,8); 1,274 (6,1); 1,267 (6,2); 1,253 (2,2); 1,235 (0,4); 1,192 (0,4); 1,175 (0,7); 1,157 (0,4); 0,146 (0,7); 0,000 (164,4); -0,008 (7,8); -0,150 (0,8) |
| Beispiel I-T3-28: ¹H-NMR (400,0 MHz, d₆-DMSO): δ= 8,684 (0,4); 8,672 (9,5); 8,535 (3,2); 8,524 (3,2); 8,453 (0,3); 8,427 (9,5); 8,271 (16,0); 7,749 (2,3); 7,744 (3,3); 7,723 (12,2); 7,519 (4,6); 7,498 (3,9); 4,056 (0,5); 4,038 (1,4); 4,020 (1,4); 4,002 (0,5); 3,568 (7,8); 3,329 (74,2); 2,857 (0,9); 2,848 (1,3); 2,839 (1,9); 2,829 (2,0); 2,820 (1,3); 2,811 (0,9); 2,801 (0,3); 2,676 (0,4); 2,671 (0,6); 2,667 (0,4); 2,524 (1,5); 2,511 (34,6); 2,507 (68,9); 2,502 (89,7); 2,498 (64,3); 2,493 (30,8); 2,333 (0,4); 2,329 (0,6); 2,324 (0,4); 1,989 (6,1); 1,397 (15,9); 1,193 (1,6); 1,175 (3,1); 1,157 (1,6); 0,728 (1,3); 0,716 (3,7); 0,711 (4,9); 0,698 (4,7); 0,693 (3,9); 0,681 (1,6); 0,561 (1,7); 0,551 (5,1); 0,544 (4,7); 0,535 (4,1); 0,523 (1,2); 0,008 (1,4); 0,000 (37,7); -0,008 (1,4) |
| Beispiel I-T3-29: ¹H-NMR (400,1 MHz, d₆-DMSO): δ= 9,042 (6,2); 9,036 (6,0); 8,962 (0,4); 8,831 (6,4); 8,826 (6,3); 8,807 (9,8); 8,678 (3,3); 8,668 (3,2); 8,507 (9,6); 8,478 (0,5); 8,385 (3,9); 8,379 (6,4); 8,374 (3,5); 8,111 (16,0); 3,368 (0,3); 3,367 (0,3); 3,365 (0,4); 3,362 (0,5); 3,361 (0,6); 3,360 (0,6); 3,357 (0,7); 3,356 (0,8); 3,350 (1,8); 3,330 (278,8); 3,313 (3,5); 3,309 (2,7); 3,308 (2,6); 3,306 (2,5); 3,297 (1,0); 3,295 (1,0); 3,294 (0,9); 3,287 (0,6); 3,284 (0,5); 3,281 (0,4); 3,279 (0,4); 3,277 (0,4); 2,915 (0,4); 2,905 (1,0); 2,896 (1,5); 2,887 (2,2); 2,877 (2,3); 2,869 (1,5); 2,859 (1,1); 2,849 (0,4); 2,711 (0,4); 2,671 (0,3); 2,565 (0,4); 2,564 (0,5); 2,563 (0,5); 2,562 (0,6); 2,560 (0,7); 2,559 (0,8); 2,558 (0,9); 2,557 (1,1); 2,555 (1,4); 2,542 (109,1); 2,533 (2,6); 2,532 (2,3); 2,530 (2,0); 2,529 (1,9); 2,528 (1,8); 2,527 (1,8); 2,525 (1,9); 2,524 (2,0); 2,523 (2,0); 2,511 (15,9); |
| 2,507 (30,0); 2,502 (38,9); 2,498 (28,5); 2,494 (14,2); 2,368 (0,4); 2,130 (0,7); 1,234 (0,5); 0,765 (1,4); 0,752 (4,2); 0,747 (5,5); 0,735 (5,4); 0,729 (4,4); 0,717 (2,0); 0,696 (0,3); 0,618 (2,1); 0,608 (5,9); 0,602 (5,4); 0,592 (4,5); 0,580 (1,5); 0,146 (0,5); 0,022 (0,4); 0,021 (0,5); 0,020 (0,6); 0,019 (0,7); 0,017 (0,7); 0,016 (0,9); 0,008 (6,5); 0,000 (110,7); -0,009 (5,4); -0,013 (2,0); -0,014 (1,7); -0,015 (1,6); -0,016 (1,4); -0,018 (1,3); -0,019 (1,2); -0,020 (1,1); -0,021 (1,0); -0,023 (1,0); -0,024 (0,9); -0,025 (0,9); -0,026 (0,8); -0,027 (0,7); -0,029 (0,7); - 0,031 (0,5); -0,034 (0,5); -0,035 (0,4); -0,036 (0,4); -0,150 (0,5) |
| Beispiel I-T3-30: ¹H-NMR (400,1 MHz, d₆-DMSO): δ= 9,562 (6,5); 9,101 (6,1); 9,095 (6,1); 8,989 (0,4); 8,855 (6,3); 8,850 (6,4); 8,829 (9,9); 8,5253 (9,7); 8,5245 (9,7); 8,496 (0,5); 8,438 (3,7); 8,432 (6,4); 8,427 (3,6); 8,115 (16,0); 5,759 (0,5); 3,361 (0,8); 3,329 (283,9); 2,712 (0,5); 2,671 (0,4); 2,568 (0,3); 2,567 (0,4); 2,565 (0,4); 2,564 (0,5); 2,563 (0,5); 2,562 (0,6); 2,560 (0,7); 2,559 (0,7); 2,558 (0,9); 2,557 (1,0); 2,555 (1,2); 2,554 (1,5); 2,542 (137,6); 2,533 (2,7); 2,532 (2,2); 2,530 (2,0); 2,529 (1,9); 2,528 (1,8); 2,527 (1,7); 2,525 (1,8); 2,524 (1,8); 2,523 (1,9); 2,511 (16,2); 2,507 (31,6); 2,502 (41,8); 2,498 (30,6); 2,493 (15,2); 2,368 (0,5); 1,631 (2,6); 1,617 (6,5); 1,610 (6,6); 1,597 (3,0); 1,348 (3,1); 1,334 (6,6); 1,328 (6,6); 1,313 (2,5); 1,234 (0,4); 0,146 (0,5); 0,026 (0,3); 0,025 (0,4); 0,024 (0,4); 0,022 (0,5); 0,021 (0,6); 0,020 (0,6); 0,019 (0,7); 0,016 (0,9); 0,008 (5,8); 0,000 (109,7); -0,008 (4,9); -0,014 (1,3); -0,015 (1,2); -0,016 (1,1); -0,018 (1,0); -0,019 (0,9); -0,020 (0,9); -0,023 (0,7); -0,024 (0,6); - 0,025 (0,6); -0,027 (0,5); -0,029 (0,4); -0,030 (0,4); -0,031 (0,4); -0,150 (0,5) |
| Beispiel I-T3-31: ¹H-NMR (601,6 MHz, CD3CN): δ= 19,953 (0,4); 8,476 (0,6); 8,461 (16,0); 8,193 (0,3); 8,170 (0,6); 8,156 (14,9); 8,148 (0,5); 8,052 (0,3); 7,934 (7,1); 7,931 (7,2); 7,901 (0,6); 7,866 (5,7); 7,852 (8,0); 7,779 (4,3); 7,765 (3,1); 7,707 (9,8); 7,703 (11,5); 7,689 (0,7); 7,669 (6,5); 7,665 (5,3); 7,655 (7,3); 7,651 (6,3); 7,609 (0,4); 7,477 (11,0); 7,463 (9,5); 6,905 (2,4); 3,912 (2,1); 2,873 (0,6); 2,866 (1,9); 2,860 (2,7); 2,854 (4,1); 2,848 (4,3); 2,842 (2,7); 2,836 (2,0); 2,830 (0,7); 2,145 (513,7); 2,068 (0,6); 2,064 (0,6); 2,060 (3,3); 2,056 (5,5); 2,052 (8,1); 2,048 (5,6); 2,044 (2,9); 1,966 (31,6); 1,958 (83,8); 1,953 (98,4); 1,950 (560,5); 1,945 (964,6); 1,941 (1429,4); 1,937 (989,8); 1,933 (503,8); 1,925 (8,1); 1,843 (0,3); 1,835 (3,0); 1,831 (5,4); 1,827 (7,9); 1,823 (5,4); 1,818 (2,7); 1,340 (0,3); 1,285 (0,7); 1,269 (2,9); 1,123 (0,4); 0,882 (0,7); 0,790 (2,5); 0,782 (6,8); 0,779 (9,4); 0,770 (9,0); 0,767 (7,4); 0,759 (3,0); 0,744 (0,4); 0,732 (0,4); 0,636 (0,4); 0,609 (2,9); 0,601 (7,4); 0,598 (7,8); 0,595 (7,3); 0,592 (7,5); 0,583 (2,4); 0,097 (2,5); 0,005 (17,5); 0,000 (598,4); -0,006 (20,1); -0,100 (2,5) |
| Beispiel I-T3-32: ¹H-NMR (601,6 MHz, CD3CN): δ= 19,978 (0,8); 8,505 (16,0); 8,234 (0,7); 8,197 (15,1); 7,962 (7,3); 7,933 (1,0); 7,901 (6,0); 7,887 (8,2); 7,812 (4,3); 7,798 (3,2); 7,785 (9,1); 7,781 (11,3); 7,755 (6,0); 7,752 (4,8); 7,742 (6,5); 7,738 (5,8); 7,557 (10,5); 7,543 (9,3); 7,451 (1,9); 7,284 (1,2); 7,272 (0,9); 7,228 (1,1); 7,216 (0,9); 7,183 (0,6); 5,481 (0,6); 4,162 (2,0); 4,151 (2,3); 4,147 (6,6); 4,136 (6,5); 4,131 (7,2); 4,120 (6,5); 4,115 (3,0); 4,104 (2,2); 3,946 (0,8); 2,497 (1,3); 2,361 (4,7); 2,211 (114,1); 2,208 (135,0); 2,203 (158,6); 2,200 (140,9); 2,198 (164,0); 2,092 (0,8); 2,088 (1,1); 2,084 (1,6); 2,080 (1,1); 1,997 (12,7); 1,989 (19,6); 1,985 (22,3); 1,981 (108,3); 1,977 (181,4); 1,973 (268,0); 1,969 (184,4); 1,965 (94,6); 1,862 (1,0); 1,858 (1,5); 1,854 (1,1); 1,303 (1,1); 0,033 (2,1) |
| Beispiel I-T3-33: ¹H-NMR (601,6 MHz, CD3CN): δ= 8,489 (10,8); 8,221 (0,7); 8,184 (10,0); 7,966 (4,8); 7,932 (1,1); 7,902 (4,0); 7,888 (5,4); 7,813 (3,0); 7,799 (2,1); 7,759 (6,4); 7,755 (7,3); 7,725 (4,1); 7,722 (3,2); 7,712 (4,4); 7,708 (3,5); 7,533 (7,0); 7,519 (5,9); 7,111 (1,4); 3,659 (3,4); 3,648 (7,7); 3,637 (7,6); 3,626 (3,3); 2,609 (0,8); 2,598 (1,4); 2,591 (2,3); 2,579 (4,4); 2,572 (2,5); 2,568 (2,5); 2,561 (4,4); 2,549 (2,4); 2,542 (1,5); 2,531 (0,8); 2,184 (375,5); 2,182 (324,4); 2,181 (324,2); 2,177 (394,7); 2,173 (444,9); 2,092 (1,2); 2,088 (2,1); 2,084 (3,3); 2,080 (2,2); 2,076 (1,1); 1,998 (27,5); 1,990 (42,2); 1,985 (47,1); 1,981 (234,7); 1,977 (393,2); 1,973 (581,0); 1,969 (400,8); 1,965 (207,8); 1,867 (1,2); 1,863 (2,2); 1,859 (3,3); 1,855 (2,2); 1,850 (1,2); 1,471 (16,0); 1,303 (1,3); 0,033 (3,4) |
| Beispiel I-T3-34: ¹H-NMR (400,0 MHz, CD3CN): δ= 8,493 (16,0); 8,193 (15,4); 8,099 (0,4); 8,087 (6,7); 8,065 (8,0); 7,850 (4,4); 7,827 (4,3); 7,815 (7,2); 7,752 (9,3); 7,747 (11,6); 7,720 (6,3); 7,715 (4,4); 7,699 (7,2); 7,694 (5,8); 7,569 (4,2); 7,514 (10,8); 7,493 (8,8); 4,012 (0,8); 3,891 (0,5); 3,458 (0,5); 3,452 (0,5); 3,236 (1,8); 3,067 (0,5); 3,056 (0,5); 2,848 (0,4); 2,140 (115,1); 2,120 (1,0); 2,114 (1,4); 2,108 (1,6); 2,102 (1,2); 2,095 (0,6); 1,972 (1,7); 1,965 (8,4); 1,958 (21,0); 1,953 (103,8); 1,947 (185,9); 1,940 (245,9); 1,934 (166,9); 1,928 (84,5); 1,781 (0,6); 1,775 (1,1); 1,769 (1,5); 1,763 (1,0); 1,756 (0,6); 1,605 (4,6); 1,591 (11,9); 1,584 (11,8); 1,570 (6,0); 1,530 (0,8); 1,437 (6,2); 1,407 (0,7); 1,367 (6,3); 1,353 (11,8); 1,346 (12,0); 1,332 (4,7); 1,294 (0,6); 1,269 (5,1); 1,204 (0,6); 0,882 (0,6); 0,146 (1,2); 0,008 (11,5); 0,000 (282,0); -0,009 (9,7); -0,150 (1,3) |
| Beispiel I-T3-35: ¹H-NMR (400,0 MHz, CD3CN): δ= 8,496 (0,7); 8,480 (16,0); 8,182 (15,3); 8,181 (14,8); 8,087 (6,6); 8,065 (7,9); 7,848 (4,7); 7,825 (4,8); 7,812 (7,7); 7,702 (9,9); 7,697 (11,9); 7,670 (6,9); 7,664 (4,9); 7,649 (7,8); 7,643 (6,3); 7,481 (10,8); 7,460 (8,6); 6,929 (2,9); 2,887 (0,7); 2,877 (2,1); 2,868 (2,9); 2,859 (4,5); 2,849 (4,5); 2,841 (2,9); 2,831 (2,1); 2,822 (0,7); 2,467 (0,4); 2,463 (0,5); 2,458 (0,4); 2,153 (188,6); 2,120 (0,8); 2,114 (1,1); 2,108 (1,3); 2,102 (0,9); 2,096 (0,5); 1,972 (2,2); 1,965 (8,7); 1,959 (23,3); 1,953 (95,7); 1,947 (167,7); 1,941 (215,6); 1,934 (146,4); 1,928 (73,1); 1,781 (0,5); 1,775 (0,9); 1,769 (1,2); 1,763 (0,8); 1,757 (0,4); 1,437 (6,9); 1,269 (0,6); 1,204 (0,5); 0,800 (2,4); 0,788 (7,7); 0,783 (9,7); 0,770 (10,2); 0,765 (7,3); 0,753 (3,1); 0,731 (0,4); 0,713 (0,4); 0,656 (0,4); 0,646 (0,4); 0,617 (3,3); 0,605 (8,7); 0,599 (9,2); 0,595 (8,3); 0,590 (7,6); 0,577 (2,2); 0,522 (0,3); 0,146 (1,0); 0,000 (233,4); -0,009 (9,6); -0,150 (1,0) |
| Beispiel I-T3-36: ¹H-NMR (400,0 MHz, CD3CN): δ= 8,496 (16,0); 8,192 (15,3); 8,093 (6,4); 8,071 (7,7); 7,850 (4,3); 7,827 (4,3); 7,814 (7,1); 7,749 (9,1); 7,743 (11,5); 7,726 (6,8); 7,721 (4,1); 7,706 (7,6); 7,700 (5,6); 7,532 (10,3); 7,511 (8,3); 7,340 (2,4); 4,149 (2,3); 4,132 (2,8); 4,125 (7,1); 4,109 (7,4); 4,102 (7,4); 4,085 (7,2); 4,078 (2,6); 4,061 (2,3); 2,137 (51,6); 2,120 (0,6); 2,114 (0,9); 2,108 (1,0); 2,102 (0,7); 2,095 (0,4); 1,965 (6,7); 1,958 (18,2); 1,953 (74,7); 1,946 (129,6); 1,940 (165,9); 1,934 (111,8); 1,928 (55,4); 1,781 (0,4); 1,775 (0,7); 1,769 (1,0); 1,763 (0,6); 1,437 (3,7); 1,270 (0,3); 0,146 (0,8); 0,008 (11,2); 0,000 (188,1); -0,009 (6,3); -0,150 (0,8) |
| Beispiel I-T3-37: ¹H-NMR (400,0 MHz, CD3CN): δ= 8,518 (0,5); 8,490 (16,0); 8,238 (0,5); 8,193 (14,9); 8,090 (6,7); 8,069 (7,7); 7,850 (4,2); 7,827 (4,0); 7,814 (6,7); 7,735 (9,2); 7,729 (11,3); 7,717 (0,4); 7,696 (6,1); 7,690 (4,5); 7,675 (7,0); 7,669 (5,9); 7,587 (0,4); 7,536 (0,3); 7,514 (0,6); 7,504 (11,0); 7,494 (0,6); 7,483 (9,1); 7,459 (1,7); 7,445 (1,6); 6,694 (0,4); 6,666 (0,3); 5,364 (0,6); 5,343 (2,4); 5,322 (4,7); 5,301 (4,6); 5,280 (2,4); 5,259 (0,7); 4,006 (0,5); 3,589 (0,4); 3,567 (0,4); 3,549 (6,1); 3,545 (3,8); 3,525 (11,4); 3,507 (4,3); 3,503 (7,9); 3,379 (8,1); 3,375 (5,1); 3,358 (11,5); 3,355 (10,8); 3,338 (3,6); 3,334 (6,2); 3,067 (0,5); 2,848 (0,5); 2,472 (0,5); 2,468 (1,0); 2,463 (1,3); 2,458 (1,0); 2,453 (0,5); 2,264 (0,3); 2,245 (0,4); 2,151 (305,9); 2,120 (1,6); 2,114 (2,3); 2,107 (2,8); 2,101 (2,0); 2,095 (1,0); 2,022 (1,9); 2,003 (0,5); 1,964 (14,6); 1,958 (33,8); 1,952 (185,3); 1,946 (333,6); 1,940 (449,3); 1,934 (307,4); 1,928 (157,3); 1,915 (1,9); 1,781 (1,0); 1,775 (1,8); 1,768 (2,5); 1,762 (1,7); 1,756 (0,8); 1,269 (2,1); 0,146 (3,1); 0,025 (0,7); 0,008 (22,9); 0,000 (696,9); -0,009 (23,3); -0,150 (3,1) |
| Beispiel I-T3-38: ¹H-NMR (400,0 MHz, CD3CN): δ= 8,238 (7,9); 8,119 (7,5); 7,748 (4,6); 7,742 (5,7); 7,729 (4,0); 7,711 (3,1); 7,706 (2,3); 7,690 (3,5); 7,685 (2,8); 7,666 (1,4); 7,644 (2,6); 7,595 (5,4); 7,574 (4,0); 7,500 (5,1); 7,479 (4,2); 4,068 (1,0); 4,050 (1,0); 4,032 (0,3); 2,800 (1,9); 2,781 (5,9); 2,762 (6,0); 2,744 (2,0); 2,139 (27,7); 2,120 (0,5); 2,113 (0,5); 2,107 (0,6); 2,101 (0,4); 1,972 (4,6); 1,964 (2,9); 1,958 (7,5); 1,952 (33,5); 1,946 (58,9); 1,940 (77,3); 1,933 (53,2); 1,927 (27,2); 1,774 (0,4); 1,768 (0,5); 1,762 (0,3); 1,601 (2,2); 1,587 (6,0); 1,580 (6,0); 1,566 (3,0); 1,526 (0,4); 1,402 (0,3); 1,362 (3,0); 1,348 (6,0); 1,342 (6,1); 1,327 (2,3); 1,270 (1,6); 1,221 (1,2); 1,204 (2,3); 1,186 (1,1); 1,113 (7,7); 1,095 (16,0); 1,076 (7,4); 0,146 (1,1); 0,008 (13,7); 0,000 (231,5); -0,009 (10,7); -0,150 (1,1) |
| Beispiel I-T3-39: ¹H-NMR (400,0 MHz, CD3CN): δ= 8,226 (8,7); 8,120 (0,4); 8,108 (8,2); 7,726 (4,1); 7,700 (4,7); 7,694 (5,9); 7,680 (0,5); 7,663 (4,5); 7,657 (3,5); 7,642 (6,1); 7,637 (5,3); 7,593 (5,2); 7,572 (2,8); 7,467 (5,6); 7,447 (4,6); 6,927 (1,4); 3,874 (0,7); 3,051 (0,4); 2,938 (0,4); 2,875 (0,9); 2,865 (1,4); 2,857 (2,1); 2,847 (2,1); 2,838 (1,4); 2,829 (1,0); 2,819 (0,3); 2,798 (2,0); 2,780 (6,2); 2,761 (6,4); 2,742 (2,2); 2,463 (0,4); 2,160 (108,1); 2,120 (0,8); 2,114 (0,9); 2,108 (1,0); 2,101 (0,7); 2,095 (0,4); 1,972 (0,6); 1,964 (3,4); 1,958 (8,7); 1,952 (47,6); 1,946 (86,2); 1,940 (115,8); 1,934 (80,2); 1,928 (41,7); 1,781 (0,4); 1,775 (0,5); 1,768 (0,7); 1,762 (0,5); 1,437 (6,6); 1,270 (1,4); 1,112 (7,8); 1,102 (1,0); 1,093 (16,0); 1,074 (7,6); 0,797 (1,2); 0,784 (3,7); 0,779 (4,7); 0,766 (4,9); 0,761 (3,7); 0,749 (1,7); 0,614 (1,6); 0,602 (4,5); 0,596 (4,5); 0,592 (4,0); 0,587 (4,0); 0,574 (1,2); 0,146 (1,3); 0,008 (10,2); 0,007 (10,2); 0,000 (266,8); -0,008 (11,5); -0,150 (1,3) |
| Beispiel I-T3-40: ¹H-NMR (400,0 MHz, CD3CN): δ= 8,241 (8,2); 8,240 (8,8); 8,117 (8,2); 7,746 (4,6); 7,741 (6,2); 7,727 (4,2); 7,718 (3,5); 7,712 (2,4); 7,697 (3,6); 7,691 (3,1); 7,664 (1,4); 7,642 (2,6); 7,597 (5,2); 7,576 (2,7); 7,516 (5,5); 7,495 (4,5); 7,352 (0,9); 4,144 (1,2); 4,128 (1,3); 4,121 (3,6); 4,104 (3,6); 4,097 (3,8); 4,081 (3,6); 4,074 (1,4); 4,057 (1,2); 2,800 (2,0); 2,781 (6,2); 2,763 (6,3); 2,744 (2,1); 2,153 (11,7); 2,149 (14,3); 1,971 (0,5); 1,964 (1,3); 1,958 (3,1); 1,952 (16,8); 1,946 (30,8); 1,940 (41,6); 1,934 (28,7); 1,927 (14,8); 1,436 (10,4); 1,268 (0,4); 1,114 (7,8); 1,095 (16,0); 1,076 (7,6); 0,146 (0,6); 0,008 (4,4); 0,000 (116,7); -0,008 (5,1); -0,150 (0,6) |
| Beispiel I-T3-41: ¹H-NMR (400,0 MHz, CD3CN): δ= 8,239 (7,9); 8,122 (7,5); 7,732 (8,1); 7,727 (9,3); 7,689 (3,0); 7,684 (2,4); 7,668 (4,6); 7,663 (4,2); 7,644 (2,6); 7,598 (4,8); 7,577 (2,6); 7,491 (5,3); 7,470 (4,9); 5,342 (1,2); 5,320 (2,4); 5,300 (2,4); 5,279 (1,2); 3,548 (3,0); 3,524 (6,0); 3,502 (4,1); 3,374 (4,1); 3,371 (2,6); 3,354 (6,0); 3,351 (5,6); 3,330 (3,2); 2,803 (1,9); 2,784 (5,9); 2,765 (6,0); 2,746 (2,1); 2,468 (0,8); 2,464 (0,9); 2,459 (0,7); 2,156 (336,8); 2,120 (1,6); 2,114 (2,0); 2,107 (2,3); 2,101 (1,6); 2,095 (1,0); 1,964 (10,5); 1,958 (27,4); 1,952 (132,9); 1,946 (239,9); 1,940 (318,6); 1,934 (221,8); 1,928 (114,5); 1,781 (0,8); 1,775 (1,4); 1,769 (1,9); 1,762 (1,3); 1,756 (0,7); 1,437 (0,8); 1,269 (2,2); 1,115 (7,6); 1,096 (16,0); 1,078 (7,4); 0,146 (3,8); 0,008 (39,2); 0,000 (832,6); -0,008 (44,8); -0,150 (4,0) |
| Beispiel I-T3-42: ¹H-NMR (400,0 MHz, d₆-DMSO): δ= 8,830 (5,8); 8,821 (4,0); 8,815 (4,0); 8,694 (1,8); 8,683 (1,8); 8,548 (5,3); 8,547 (5,5); 8,315 (0,6); 8,192 (4,1); 8,186 (3,9); 8,107 (8,4); 3,902 (16,0); 3,333 (334,0); 3,243 (1,4); 3,175 (0,9); 3,162 (0,9); 2,870 (0,5); 2,861 (0,7); 2,852 (1,1); 2,842 (1,1); 2,833 (0,7); 2,824 (0,5); 2,680 (0,3); 2,676 (0,7); 2,672 (0,9); 2,667 (0,7); 2,662 (0,3); 2,542 (0,6); 2,525 (2,7); 2,511 (58,6); 2,507 (116,6); 2,502 (152,6); 2,498 (110,8); 2,493 (53,8); 2,338 (0,3); 2,334 (0,7); 2,329 (0,9); 2,325 (0,7); 1,909 (0,5); 1,016 (0,6); 1,001 (0,6); 0,757 (0,7); 0,744 (2,0); 0,739 (2,8); 0,727 (2,6); 0,721 (2,2); 0,709 (0,9); 0,566 (0,9); 0,555 (2,6); 0,549 (2,4); 0,545 (2,3); 0,540 (2,2); 0,528 (0,7); 0,008 (0,5); 0,000 (16,2); -0,009 (0,5) |
| Beispiel I-T3-43: ¹H-NMR (400,0 MHz, d₆-DMSO): δ= 9,603 (7,1); 8,876 (6,9); 8,870 (6,9); 8,843 (10,4); 8,833 (0,4); 8,554 (10,3); 8,315 (0,8); 8,280 (7,0); 8,274 (6,8); 8,110 (16,0); 3,903 (14,5); 3,434 (0,4); 3,333 (565,4); 3,045 (0,5); 2,869 (0,5); 2,676 (1,3); 2,671 (1,7); 2,667 (1,3); 2,662 (0,7); 2,542 (1,5); 2,524 (5,6); 2,511 (106,6); 2,507 (206,6); 2,502 (266,2); 2,498 (191,5); 2,493 (91,5); 2,338 (0,5); 2,334 (1,1); 2,329 (1,5); 2,325 (1,1); 1,643 (2,4); 1,629 (5,7); 1,622 (6,0); 1,609 (2,6); 1,298 (2,9); 1,284 (5,7); 1,277 (6,1); 1,263 (2,3); 1,249 (0,4); 1,236 (0,4); 0,008 (0,8); 0,000 (22,4); -0,009 (0,7) |
| Beispiel I-T3-44: ¹H-NMR (400,0 MHz, CD3CN): δ= 8,115 (9,4); 8,071 (10,0); 7,690 (6,4); 7,685 (6,3); 7,668 (1,1); 7,653 (3,9); 7,632 (4,2); 7,586 (0,3); 7,549 (8,1); 7,509 (0,4); 7,463 (5,6); 7,442 (4,5); 6,896 (2,5); 4,068 (0,5); 4,051 (0,5); 2,871 (1,3); 2,862 (1,9); 2,853 (2,6); 2,844 (2,5); 2,835 (1,9); 2,826 (1,2); 2,816 (0,5); 2,452 (2,7); 2,434 (7,4); 2,415 (7,5); 2,396 (2,8); 2,251 (0,5); 2,143 (127,4); 2,113 (3,7); 2,092 (29,0); 1,971 (7,1); 1,952 (73,0); 1,946 (111,8); 1,943 (112,2); 1,940 (130,1); 1,937 (90,9); 1,934 (90,5); 1,928 (48,4); 1,774 (0,6); 1,768 (0,7); 1,437 (3,1); 1,221 (0,7); 1,204 (1,2); 1,186 (0,6); 1,082 (8,3); 1,063 (16,0); 1,044 (7,9); 0,794 (1,7); 0,780 (6,1); 0,777 (6,2); 0,764 (6,6); 0,747 (2,1); 0,726 (0,4); 0,610 (2,4); 0,600 (6,7); 0,592 (7,0); 0,572 (1,7); 0,535 (0,4); 0,528 (0,3); 0,524 (0,3); 0,147 (1,4); 0,000 (240,4); -0,149 (1,3) |
| Beispiel I-T3-45: ¹H-NMR (400,0 MHz, CD3CN): δ= 8,126 (9,0); 8,092 (9,4); 7,735 (4,9); 7,730 (6,5); 7,710 (3,2); 7,705 (2,4); 7,689 (3,6); 7,684 (3,1); 7,549 (5,9); 7,542 (5,8); 7,513 (5,6); 7,492 (4,6); 7,422 (1,5); 4,140 (1,2); 4,123 (1,5); 4,116 (3,7); 4,100 (3,9); 4,093 (4,1); 4,076 (3,7); 4,069 (1,6); 4,052 (1,2); 3,545 (1,6); 2,464 (1,4); 2,455 (2,7); 2,436 (6,7); 2,417 (6,9); 2,398 (2,4); 2,378 (0,9); 2,253 (0,5); 2,221 (1,4); 2,176 (369,0); 2,126 (0,6); 2,120 (0,7); 2,114 (1,0); 2,108 (1,5); 2,094 (27,0); 1,953 (71,9); 1,947 (129,9); 1,941 (173,4); 1,935 (125,8); 1,928 (67,7); 1,781 (0,4); 1,775 (0,7); 1,769 (1,1); 1,763 (0,7); 1,757 (0,4); 1,436 (9,9); 1,269 (0,4); 1,102 (0,5); 1,084 (8,0); 1,065 (16,0); 1,046 (7,7); 1,025 (0,5); 0,146 (2,0); 0,000 (393,0); -0,150 (2,0) |
| Beispiel I-T3-46: ¹H-NMR (400,0 MHz, CD3CN): δ= 8,127 (4,1); 8,085 (4,4); 7,738 (2,5); 7,732 (3,1); 7,702 (1,6); 7,697 (1,2); 7,681 (1,8); 7,676 (1,5); 7,625 (0,9); 7,551 (2,4); 7,543 (2,3); 7,495 (2,9); 7,474 (2,3); 2,453 (1,1); 2,435 (3,1); 2,416 (3,2); 2,397 (1,1); 2,158 (61,0); 2,114 (0,4); 2,108 (0,5); 2,092 (12,6); 1,964 (2,0); 1,958 (4,9); 1,953 (25,4); 1,946 (45,6); 1,940 (61,1); 1,934 (42,3); 1,928 (21,8); 1,769 (0,4); 1,598 (1,2); 1,583 (3,1); 1,576 (3,1); 1,563 (1,6); 1,437 (16,0); 1,358 (1,6); 1,345 (3,1); 1,338 (3,2); 1,323 (1,3); 1,269 (1,8); 1,083 (4,2); 1,064 (8,8); 1,045 (4,0); 0,146 (0,7); 0,017 (0,4); 0,008 (5,9); 0,000 (150,8); -0,009 (6,1); -0,150 (0,7) |
| Beispiel I-T3-47: ¹H-NMR (400,0 MHz, CD3CN): δ= 8,128 (3,6); 8,084 (3,8); 7,721 (2,0); 7,715 (2,5); 7,678 (1,2); 7,673 (1,0); 7,657 (1,4); 7,652 (1,2); 7,551 (2,1); 7,544 (2,1); 7,485 (2,4); 7,464 (2,2); 5,338 (0,6); 5,316 (1,1); 5,296 (1,1); 5,275 (0,6); 3,544 (1,4); 3,520 (2,8); 3,498 (1,8); 3,370 (1,7); 3,349 (2,6); 3,346 (2,4); 3,325 (1,4); 2,456 (0,9); 2,437 (2,6); 2,418 (2,7); 2,399 (0,9); 2,166 (9,4); 2,153 (19,8); 2,107 (0,4); 2,095 (10,7); 1,964 (1,2); 1,958 (3,1); 1,952 (15,2); 1,946 (27,1); 1,940 (36,2); 1,934 (25,2); 1,928 (13,1); 1,437 (16,0); 1,085 (3,2); 1,066 (6,7); 1,047 (3,1); 0,146 (0,4); 0,008 (3,9); 0,000 (86,8); -0,008 (4,3); -0,150 (0,5) |
| Beispiel I-T3-48: ¹H-NMR (400,0 MHz, CD3CN): δ= 8,261 (10,8); 8,206 (0,8); 8,193 (16,0); 7,692 (9,7); 7,686 (12,3); 7,675 (1,0); 7,657 (6,5); 7,652 (4,9); 7,637 (9,5); 7,630 (13,3); 7,608 (11,0); 7,475 (11,5); 7,455 (9,1); 6,940 (2,5); 3,911 (0,6); 2,882 (0,7); 2,872 (2,0); 2,863 (2,8); 2,854 (4,4); 2,845 (4,3); 2,836 (2,9); 2,827 (2,0); 2,817 (0,7); 2,467 (0,3); 2,463 (0,4); 2,163 (117,4); 2,120 (0,3); 2,114 (0,5); 2,108 (0,7); 2,102 (0,5); 1,972 (1,7); 1,965 (3,3); 1,959 (8,5); 1,953 (43,3); 1,947 (77,6); 1,941 (103,6); 1,934 (71,4); 1,928 (36,7); 1,775 (0,4); 1,769 (0,6); 1,763 (0,4); 1,437 (2,0); 1,269 (0,7); 1,221 (0,4); 1,204 (0,7); 1,186 (0,3); 0,795 (2,4); 0,783 (7,5); 0,778 (9,7); 0,765 (10,2); 0,760 (7,3); 0,748 (3,3); 0,726 (0,4); 0,708 (0,4); 0,654 (0,4); 0,644 (0,4); 0,614 (3,3); 0,604 (8,6); 0,597 (9,0); 0,593 (7,8); 0,588 (7,7); 0,575 (2,3); 0,514 (0,4); 0,146 (1,3); 0,026 (0,4); 0,008 (10,5); 0,000 (259,4); -0,009 (10,3); -0,150 (1,2) |
| Beispiel I-T3-49: ¹H-NMR (400,0 MHz, CD3CN): δ= 8,277 (10,6); 8,206 (16,0); 7,740 (9,1); 7,735 (12,1); 7,709 (6,2); 7,704 (4,7); 7,688 (7,6); 7,683 (7,1); 7,669 (3,6); 7,645 (1,5); 7,631 (10,9); 7,611 (10,7); 7,574 (0,5); 7,510 (11,0); 7,500 (0,6); 7,489 (9,0); 7,475 (0,4); 7,221 (0,4); 5,448 (8,3); 4,034 (0,9); 3,914 (1,0); 3,906 (0,7); 3,897 (0,5); 2,469 (1,1); 2,464 (1,5); 2,460 (1,1); 2,243 (0,4); 2,175 (509,2); 2,120 (1,2); 2,114 (1,7); 2,108 (2,0); 2,102 (1,5); 2,096 (0,9); 1,965 (8,1); 1,959 (21,5); 1,953 (116,7); 1,947 (211,5); 1,941 (284,4); 1,934 (197,6); 1,928 (103,4); 1,781 (0,8); 1,775 (1,3); 1,769 (1,8); 1,763 (1,3); 1,757 (0,7); 1,635 (0,4); 1,598 (4,6); 1,584 (12,3); 1,577 (12,3); 1,563 (6,3); 1,523 (0,8); 1,437 (0,7); 1,403 (0,8); 1,363 (6,4); 1,349 (12,1); 1,342 (12,8); 1,328 (4,9); 1,290 (0,6); 1,270 (2,7); 1,206 (1,3); 1,190 (1,2); 0,882 (0,3); 0,146 (3,5); 0,008 (26,4); 0,000 (693,0); -0,008 (31,4); -0,048 (0,4); -0,150 (3,5) |
| Beispiel I-T3-50: ¹H-NMR (400,0 MHz, CD3CN): δ= 8,131 (3,8); 8,120 (0,3); 8,108 (3,8); 7,670 (2,4); 7,664 (2,9); 7,636 (1,7); 7,630 (1,2); 7,615 (1,9); 7,609 (1,6); 7,462 (2,8); 7,441 (2,1); 7,320 (3,0); 7,300 (1,2); 6,909 (0,6); 4,085 (0,5); 4,068 (1,4); 4,050 (1,4); 4,032 (0,5); 3,901 (16,0); 2,870 (0,5); 2,861 (0,7); 2,852 (1,1); 2,843 (1,1); 2,834 (0,7); 2,825 (0,5); 2,147 (64,0); 2,114 (0,3); 2,107 (0,4); 1,972 (6,8); 1,964 (3,1); 1,958 (6,8); 1,952 (27,1); 1,946 (46,5); 1,940 (60,6); 1,934 (41,2); 1,928 (20,8); 1,768 (0,3); 1,437 (1,1); 1,221 (1,7); 1,204 (3,2); 1,186 (1,6); 0,793 (0,6); 0,781 (1,8); 0,776 (2,3); 0,763 (2,4); 0,758 (1,7); 0,746 (0,8); 0,610 (0,9); 0,599 (2,1); 0,593 (2,1); 0,589 (1,9); 0,584 (1,8); 0,571 (0,5); 0,146 (0,7); 0,008 (9,0); 0,000 (144,6); -0,009 (5,4); -0,150 (0,7) |
| Beispiel I-T3-51: ¹H-NMR (400,0 MHz, CD3CN): δ= 8,206 (0,4); 8,147 (3,5); 8,120 (3,6); 7,717 (2,2); 7,712 (2,8); 7,686 (1,6); 7,680 (1,3); 7,675 (0,4); 7,665 (2,0); 7,659 (1,9); 7,654 (0,6); 7,644 (0,5); 7,630 (0,5); 7,610 (0,3); 7,503 (0,4); 7,495 (2,6); 7,482 (0,3); 7,474 (2,1); 7,321 (2,8); 7,302 (1,1); 4,068 (0,9); 4,050 (0,9); 3,902 (16,0); 2,170 (60,8); 2,114 (0,4); 2,108 (0,5); 2,102 (0,3); 1,972 (4,1); 1,965 (2,3); 1,959 (5,7); 1,953 (31,1); 1,947 (56,0); 1,940 (74,9); 1,934 (51,0); 1,928 (25,9); 1,775 (0,3); 1,769 (0,4); 1,595 (1,1); 1,581 (2,7); 1,574 (2,7); 1,560 (1,5); 1,437 (1,0); 1,359 (1,5); 1,346 (2,7); 1,339 (2,8); 1,324 (1,1); 1,222 (1,1); 1,204 (2,1); 1,186 (1,0); 1,140 (0,5); 1,132 (0,6); 0,928 (0,6); 0,921 (0,6); 0,146 (0,8); 0,008 (7,0); 0,000 (187,7); -0,009 (6,3); -0,150 (0,8) |
| Beispiel I-T3-52: ¹H-NMR (400,0 MHz, CD3CN): δ= 8,276 (6,4); 8,163 (0,3); 8,151 (6,6); 8,149 (7,0); 8,128 (3,5); 8,104 (1,6); 8,082 (1,8); 7,863 (2,9); 7,842 (2,4); 7,694 (4,0); 7,689 (5,1); 7,680 (0,5); 7,660 (3,0); 7,654 (2,2); 7,639 (3,3); 7,633 (2,8); 7,474 (4,9); 7,454 (3,9); 6,935 (1,1); 4,086 (0,7); 4,068 (2,0); 4,050 (2,1); 4,032 (0,7); 2,873 (0,8); 2,863 (1,2); 2,855 (1,8); 2,845 (1,9); 2,836 (1,2); 2,827 (0,9); 2,165 (66,0); 2,163 (75,8); 1,972 (9,5); 1,965 (1,3); 1,959 (3,0); 1,953 (16,9); 1,947 (30,5); 1,941 (41,0); 1,935 (28,3); 1,928 (14,5); 1,436 (16,0); 1,269 (0,5); 1,221 (2,5); 1,204 (4,8); 1,186 (2,4); 0,796 (1,0); 0,784 (2,9); 0,778 (3,9); 0,766 (4,1); 0,761 (3,0); 0,748 (1,4); 0,613 (1,4); 0,602 (3,5); 0,596 (3,5); 0,592 (3,2); 0,587 (3,2); 0,574 (1,0); 0,008 (2,1); 0,000 (62,9); -0,009 (2,3) |
| Beispiel I-T3-53: ¹H-NMR (400,0 MHz, CD3CN): δ= 8,291 (3,8); 8,162 (4,2); 8,130 (2,0); 8,108 (0,9); 8,086 (1,0); 7,866 (1,8); 7,844 (1,5); 7,742 (2,5); 7,736 (3,2); 7,709 (2,0); 7,703 (1,5); 7,688 (2,7); 7,682 (2,4); 7,506 (3,0); 7,485 (2,4); 2,196 (8,5); 2,183 (24,4); 1,972 (1,1); 1,965 (0,6); 1,959 (1,4); 1,954 (7,8); 1,947 (14,2); 1,941 (19,1); 1,935 (13,1); 1,929 (6,7); 1,599 (1,3); 1,585 (3,1); 1,578 (3,1); 1,564 (1,7); 1,436 (16,0); 1,362 (1,7); 1,349 (3,0); 1,342 (3,1); 1,327 (1,3); 1,204 (0,6); 0,008 (1,6); 0,000 (44,8); -0,009 (1,6) |
| Beispiel I-T3-54: ¹H-NMR (400,0 MHz, CD3CN): δ= 8,162 (0,9); 8,153 (15,7); 8,108 (12,4); 7,996 (6,8); 7,952 (7,0); 7,683 (9,7); 7,678 (12,2); 7,666 (0,8); 7,650 (6,7); 7,644 (4,9); 7,629 (7,9); 7,624 (6,4); 7,513 (0,4); 7,469 (11,7); 7,448 (9,3); 6,931 (2,4); 5,448 (0,6); 4,235 (0,4); 4,218 (0,4); 4,086 (0,6); 4,068 (1,8); 4,057 (0,4); 4,050 (1,8); 4,032 (0,6); 2,879 (0,8); 2,870 (2,2); 2,860 (2,8); 2,852 (4,7); 2,842 (4,7); 2,834 (2,9); 2,824 (2,2); 2,814 (0,7); 2,473 (0,6); 2,468 (1,0); 2,463 (1,4); 2,459 (1,0); 2,454 (0,5); 2,276 (0,4); 2,264 (0,4); 2,245 (0,6); 2,226 (0,8); 2,159 (388,9); 2,116 (47,5); 2,108 (3,9); 2,101 (1,9); 2,095 (1,0); 2,050 (0,8); 2,035 (0,7); 2,017 (1,1); 1,998 (1,0); 1,972 (9,4); 1,964 (12,6); 1,958 (30,5); 1,953 (165,9); 1,946 (298,2); 1,940 (398,5); 1,934 (272,4); 1,928 (139,0); 1,915 (1,9); 1,781 (0,9); 1,775 (1,6); 1,769 (2,3); 1,762 (1,6); 1,756 (0,8); 1,509 (0,3); 1,437 (13,5); 1,341 (0,4); 1,307 (1,0); 1,289 (1,9); 1,269 (16,0); 1,222 (2,4); 1,204 (4,5); 1,186 (2,2); 0,898 (0,7); 0,881 (2,2); 0,864 (1,0); 0,793 (2,4); 0,780 (7,3); 0,775 (9,5); 0,762 (10,1); 0,757 (6,9); 0,745 (3,2); 0,723 (0,5); 0,705 (0,5); 0,650 (0,4); 0,640 (0,5); 0,631 (0,5); 0,626 (0,6); 0,610 (3,5); 0,600 (7,9); 0,598 (7,8); 0,593 (8,2); 0,588 (7,1); 0,583 (7,2); 0,571 (2,4); 0,523 (0,3); 0,393 (0,5); 0,385 (0,5); 0,381 (0,5); 0,376 (0,5); 0,146 (3,4); 0,008 (28,7); 0,000 (825,5); -0,009 (28,8); -0,030 (0,5); -0,150 (3,4) |
| Beispiel I-T3-55: ¹H-NMR (601,6 MHz, CD3CN): δ= 8,166 (3,1); 8,165 (3,2); 8,124 (2,4); 8,000 (1,2); 7,954 (1,2); 7,732 (2,0); 7,729 (2,3); 7,697 (1,3); 7,693 (1,1); 7,683 (1,5); 7,679 (1,3); 7,499 (2,3); 7,485 (2,0); 2,180 (8,0); 2,177 (8,1); 2,175 (8,7); 2,172 (9,0); 2,169 (10,1); 2,167 (8,6); 2,163 (12,2); 2,117 (8,6); 1,973 (0,6); 1,966 (0,7); 1,958 (1,9); 1,954 (2,1); 1,950 (12,7); 1,946 (22,2); 1,942 (32,1); 1,938 (21,1); 1,934 (10,5); 1,591 (1,0); 1,581 (2,2); 1,577 (2,2); 1,568 (1,1); 1,436 (16,0); 1,354 (1,2); 1,345 (2,2); 1,341 (2,3); 1,331 (1,0); 1,204 (0,4); 0,005 (1,3); 0,000 (42,8); -0,006 (1,2) |
| Beispiel I-T3-56: ¹H-NMR (400,0 MHz, CD3CN): δ= 8,210 (7,6); 8,194 (0,9); 8,186 (15,9); 8,185 (16,0); 8,161 (0,7); 8,149 (14,1); 8,059 (7,6); 7,686 (9,6); 7,681 (12,3); 7,653 (6,8); 7,647 (5,0); 7,632 (7,9); 7,626 (6,5); 7,517 (0,3); 7,473 (11,6); 7,452 (9,1); 6,900 (2,7); 2,878 (0,7); 2,869 (2,1); 2,859 (2,9); 2,851 (4,6); 2,841 (4,6); 2,832 (2,9); 2,823 (2,2); 2,813 (0,7); 2,136 (41,9); 2,120 (0,5); 2,113 (0,6); 2,107 (0,8); 2,101 (0,5); 2,086 (0,4); 1,964 (15,3); 1,958 (9,2); 1,952 (49,0); 1,946 (88,5); 1,940 (118,3); 1,934 (80,8); 1,927 (41,2); 1,915 (0,5); 1,774 (0,5); 1,768 (0,7); 1,762 (0,5); 1,270 (0,4); 0,792 (2,4); 0,780 (7,2); 0,775 (9,6); 0,762 (10,1); 0,757 (7,0); 0,745 (3,3); 0,723 (0,4); 0,705 (0,4); 0,650 (0,4); 0,640 (0,4); 0,610 (3,3); 0,600 (8,0); 0,599 (8,0); 0,593 (8,4); 0,589 (7,4); 0,584 (7,5); 0,571 (2,4); 0,520 (0,4); 0,146 (0,9); 0,008 (7,4); 0,000 (218,3); -0,009 (7,6); - 0,150 (0,9) |
| Beispiel I-T3-57: ¹H-NMR (400,0 MHz, CD3CN): |
| δ= 8,211 (2,2); 8,197 (4,2); 8,163 (3,5); 8,060 (2,1); 7,735 (2,4); 7,730 (3,0); 7,704 (1,6); 7,699 (1,1); 7,683 (1,8); 7,678 (1,5); 7,553 (1,2); 7,508 (2,8); 7,487 (2,3); 2,133 (61,2); 2,113 (0,8); 2,107 (0,9); 2,101 (0,7); 2,095 (0,4); 1,964 (4,3); 1,958 (11,3); 1,952 (55,5); 1,946 (99,8); 1,940 (134,0); 1,934 (93,4); 1,927 (48,7); 1,774 (0,6); 1,768 (0,8); 1,762 (0,5); 1,596 (1,1); 1,582 (3,0); 1,575 (3,1); 1,561 (1,6); 1,437 (16,0); 1,361 (1,6); 1,348 (3,0); 1,341 (3,1); 1,326 (1,2); 1,269 (0,3); 0,146 (1,1); 0,008 (9,0); 0,000 (233,2); -0,009 (12,0); -0,150 (1,0) |
| Beispiel I-T3-58: ¹H-NMR (400,0 MHz, CD3CN): δ= 8,212 (1,1); 8,196 (2,4); 8,168 (2,0); 8,062 (1,1); 7,732 (1,4); 7,727 (2,0); 7,710 (1,1); 7,704 (0,7); 7,689 (1,2); 7,683 (1,0); 7,523 (1,8); 7,502 (1,4); 4,139 (0,4); 4,123 (0,4); 4,116 (1,2); 4,099 (1,2); 4,092 (1,3); 4,076 (1,2); 4,068 (0,5); 4,052 (0,4); 2,154 (2,8); 2,152 (3,0); 1,958 (0,6); 1,952 (3,2); 1,946 (5,8); 1,940 (7,8); 1,934 (5,3); 1,928 (2,7); 1,436 (16,0); 0,008 (0,5); 0,000 (14,4); -0,009 (0,5) |
| Beispiel I-T3-59: ¹H-NMR (601,6 MHz, d₆-DMSO): δ= 19,976 (2,1); 9,451 (11,5); 9,045 (16,0); 8,978 (7,7); 8,792 (7,9); 8,789 (7,8); 8,502 (16,0); 8,320 (2,2); 7,918 (8,4); 7,914 (11,9); 7,904 (7,1); 7,900 (4,5); 7,890 (6,8); 7,886 (5,6); 7,573 (10,3); 7,560 (9,7); 4,034 (1,6); 4,022 (1,5); 3,338 (576,9); 2,615 (4,0); 2,524 (5,6); 2,521 (7,1); 2,518 (8,3); 2,509 (220,0); 2,506 (474,2); 2,503 (654,0); 2,500 (473,2); 2,497 (216,3); 2,387 (3,5); 1,990 (4,8); 1,615 (4,4); 1,606 (10,1); 1,602 (10,7); 1,593 (4,6); 1,398 (2,2); 1,300 (4,9); 1,291 (9,5); 1,286 (10,1); 1,277 (4,3); 1,175 (3,1); 0,096 (2,5); 0,005 (23,7); 0,000 (635,1); -0,006 (20,4); -0,100 (2,7) |
| Beispiel I-T3-60: ¹H-NMR (400,0 MHz, CDCl₃): δ= 8,687 (8,6); 8,685 (8,5); 8,596 (15,9); 8,577 (0,6); 8,173 (8,6); 8,168 (8,5); 8,114 (16,0); 7,901 (10,7); 7,896 (11,0); 7,567 (5,3); 7,561 (5,3); 7,555 (1,2); 7,546 (7,0); 7,540 (6,9); 7,483 (0,4); 7,426 (11,9); 7,406 (8,9); 7,264 (25,7); 6,415 (3,4); 5,301 (12,8); 2,991 (0,6); 2,982 (1,7); 2,973 (3,0); 2,964 (4,1); 2,955 (4,1); 2,946 (3,1); 2,937 (1,8); 2,928 (0,7); 1,601 (5,6); 1,378 (1,1); 1,333 (0,6); 1,327 (0,4); 1,285 (1,1); 1,255 (5,4); 0,938 (2,5); 0,921 (10,0); 0,907 (9,8); 0,903 (8,2); 0,890 (3,5); 0,880 (1,2); 0,868 (0,9); 0,862 (0,7); 0,850 (0,9); 0,836 (0,6); 0,742 (0,4); 0,733 (0,4); 0,703 (3,0); 0,689 (8,1); 0,685 (8,5); 0,680 (8,2); 0,676 (7,9); 0,662 (2,4); 0,557 (0,5); 0,551 (0,5); 0,008 (0,6); 0,000 (19,7); -0,008 (1,0) |
| Beispiel I-T3-61: ¹H-NMR (400,0 MHz, CD3CN): δ= 8,266 (6,2); 8,208 (0,5); 8,195 (8,9); 7,692 (5,7); 7,687 (6,8); 7,678 (0,7); 7,658 (3,7); 7,652 (2,8); 7,637 (4,3); 7,632 (3,5); 7,593 (5,8); 7,572 (5,8); 7,475 (6,2); 7,454 (4,9); 6,962 (1,7); 5,449 (0,9); 4,086 (0,3); 4,068 (1,1); 4,050 (1,1); 4,032 (0,4); 2,882 (0,4); 2,873 (1,2); 2,863 (1,6); 2,855 (2,6); 2,845 (2,6); 2,837 (1,7); 2,827 (1,2); 2,817 (0,4); 2,181 (57,5); 1,972 (4,8); 1,965 (1,5); 1,959 (3,8); 1,953 (16,3); 1,947 (29,0); 1,941 (37,9); 1,935 (26,4); 1,929 (13,7); 1,436 (16,0); 1,268 (0,8); 1,221 (1,3); 1,204 (2,5); 1,186 (1,2); 0,795 (1,3); 0,783 (4,5); 0,777 (5,6); 0,765 (5,9); 0,760 (4,4); 0,747 (1,8); 0,615 (1,8); 0,605 (5,0); 0,603 (5,0); 0,598 (5,5); 0,593 (4,9); 0,588 (4,6); 0,576 (1,4); 0,000 (58,7); -0,009 (3,0) |
| Beispiel I-T3-62: ¹H-NMR (601,6 MHz, CD3CN): δ= 8,282 (5,8); 8,209 (9,7); 7,742 (5,2); 7,738 (6,1); 7,705 (3,5); 7,702 (2,9); 7,692 (4,0); 7,688 (3,5); 7,644 (0,8); 7,594 (5,2); 7,580 (5,1); 7,506 (6,4); 7,493 (5,6); 2,197 (13,4); 2,194 (14,8); 2,191 (16,6); 2,188 (16,2); 2,186 (16,6); 2,184 (16,2); 2,181 (15,5); 2,179 (16,7); 1,973 (1,0); 1,967 (1,1); 1,959 (2,7); 1,954 (3,0); 1,951 (18,5); 1,947 (31,7); 1,942 (46,4); 1,938 (30,9); 1,934 (15,5); 1,594 (2,6); 1,584 (6,6); 1,580 (6,4); 1,571 (3,2); 1,544 (0,3); 1,436 (16,0); 1,359 (3,2); 1,350 (6,2); 1,345 (6,8); 1,336 (2,7); 1,266 (0,4); 1,204 (0,5); 0,005 (1,2); 0,000 (39,8); -0,006 (1,3) |
| Beispiel I-T3-63: ¹H-NMR (400,0 MHz, d₆-DMSO): δ= 8,666 (5,1); 8,523 (1,9); 8,512 (2,0); 8,389 (5,2); 7,850 (2,9); 7,814 (2,7); 7,736 (1,2); 7,730 (1,9); 7,710 (7,1); 7,511 (2,5); 7,490 (2,1); 3,327 (42,2); 2,856 (0,5); 2,846 (0,8); 2,838 (1,2); 2,828 (1,2); 2,819 (0,8); 2,810 (0,5); 2,671 (0,4); 2,506 (46,0); 2,502 (59,6); 2,498 (46,8); 2,438 (0,6); 2,329 (0,4); 2,203 (0,6); 2,188 (12,9); 1,398 (16,0); 0,727 (0,7); 0,714 (2,3); 0,709 (3,0); 0,697 (2,8); 0,691 (2,5); 0,680 (0,9); 0,560 (0,9); 0,549 (3,0); 0,543 (3,1); 0,534 (2,7); 0,522 (0,7); 0,000 (42,2) |
| Beispiel I-T3-64: ¹H-NMR (400,0 MHz, d₆-DMSO): δ= 9,432 (2,1); 8,689 (2,8); 8,404 (2,9); 7,853 (1,5); 7,816 (1,5); 7,795 (0,6); 7,789 (1,2); 7,780 (1,7); 7,775 (2,6); 7,560 (1,6); 7,552 (0,4); 7,538 (1,4); 3,327 (22,2); 2,507 (17,7); 2,502 (23,3); 2,498 (17,9); 2,436 (0,3); 2,189 (6,9); 1,989 (0,4); 1,615 (0,7); 1,600 (1,8); 1,594 (1,9); 1,581 (0,8); 1,398 (16,0); 1,284 (0,8); 1,270 (1,8); 1,264 (2,0); 1,249 (0,7); 0,008 (0,7); 0,000 (19,4) |
| Beispiel I-T3-65: ¹H-NMR (500,1 MHz, d₆-DMSO): δ= 9,726 (2,4); 8,559 (1,7); 8,556 (1,8); 8,306 (2,2); 7,930 (0,7); 7,914 (1,5); 7,897 (0,8); 7,588 (3,9); 7,467 (1,5); 7,450 (1,4); 3,305 (13,5); 2,508 (2,9); 2,504 (6,0); 2,501 (8,2); 2,497 (6,1); 2,494 (3,0); 2,106 (16,0); 1,645 (0,8); 1,634 (2,0); 1,628 (2,1); 1,617 (0,8); 1,239 (1,1); 1,228 (2,0); 1,222 (2,1); 1,211 (0,8); 0,000 (5,4) |
| Beispiel I-T3-66: ¹H-NMR (400,2 MHz, d₆-DMSO): δ= 9,726 (4,5); 8,587 (3,6); 8,583 (3,6); 8,305 (4,5); 7,934 (1,5); 7,913 (3,1); 7,892 (1,7); 7,597 (3,1); 7,562 (3,1); 7,468 (3,1); 7,447 (2,9); 5,753 (0,4); 3,427 (0,5); 3,307 (130,4); 3,283 (0,9); 3,236 (0,8); 2,669 (0,5); 2,504 (61,2); 2,500 (82,9); 2,496 (61,6); 2,431 (1,1); 2,412 (3,4); 2,394 (3,5); 2,375 (1,2); 2,327 (0,5); 2,322 (0,4); 2,087 (16,0); 1,987 (0,6); 1,648 (1,6); 1,634 (4,0); 1,628 (4,2); 1,614 (1,7); 1,463 (0,4); 1,240 (2,1); 1,227 (4,0); 1,220 (4,2); 1,206 (1,5); 1,174 (0,4); 1,031 (5,0); 1,012 (10,6); 0,993 (4,8); 0,146 (0,6); 0,008 (5,8); 0,000 (128,9); -0,150 (0,6) |
| Beispiel I-T3-67: ¹H-NMR (400,0 MHz, d₆-DMSO): δ= 8,778 (1,2); 8,767 (1,2); 8,544 (1,7); 8,539 (1,8); 8,297 (2,1); 7,876 (0,8); 7,855 (1,5); 7,834 (0,8); 7,593 (3,9); 7,418 (1,5); 7,397 (1,4); 3,903 (3,9); 3,331 (120,3); 2,855 (0,5); 2,846 (0,7); 2,836 (0,7); 2,827 (0,5); 2,818 (0,3); 2,676 (0,5); 2,671 (0,7); 2,667 (0,6); 2,524 (2,0); 2,511 (45,1); 2,507 (87,8); 2,502 (113,1); 2,498 (83,2); 2,333 (0,5); 2,329 (0,7); 2,324 (0,5); 2,120 (0,9); 2,105 (16,0); 0,752 (0,4); 0,739 (1,3); 0,734 (1,7); 0,721 (1,7); 0,716 (1,4); 0,704 (0,5); 0,522 (0,6); 0,511 (1,6); 0,505 (1,6); 0,501 (1,5); 0,496 (1,5); 0,483 (0,5); 0,000 (6,3) |
| Beispiel I-T3-68: ¹H-NMR (500,1 MHz, d₆-DMSO): δ= 8,752 (2,4); 8,743 (2,4); 8,561 (3,4); 8,558 (3,7); 8,289 (4,4); 7,868 (1,5); 7,851 (3,0); 7,834 (1,6); 7,597 (3,0); 7,563 (3,0); 7,411 (3,0); 7,394 (2,9); 3,304 (38,0); 2,861 (0,6); 2,854 (0,9); 2,847 (1,4); 2,839 (1,4); 2,831 (0,9); 2,824 (0,7); 2,507 (6,9); 2,504 (14,1); 2,500 (19,4); 2,497 (14,5); 2,493 (7,2); 2,426 (1,2); 2,411 (3,5); 2,396 (3,6); 2,381 (1,2); 2,101 (1,1); 2,089 (16,0); 1,029 (5,5); 1,013 (11,1); 0,998 (5,0); 0,746 (0,9); 0,736 (2,7); 0,732 (3,5); 0,722 (3,4); 0,718 (2,8); 0,708 (1,0); 0,521 (1,1); 0,512 (3,3); 0,508 (3,3); 0,505 (3,1); 0,500 (3,1); 0,490 (0,9); 0,006 (0,7); 0,000 (14,7); -0,007 (0,6) |
| Beispiel I-T3-69: ¹H-NMR (400,2 MHz, d₆-DMSO): |
| δ= 9,352 (4,3); 8,553 (4,2); 8,286 (4,4); 7,965 (1,0); 7,946 (2,0); 7,927 (1,1); 7,601 (3,3); 7,567 (3,5); 7,478 (0,9); 7,460 (2,0); 7,444 (1,3); 7,350 (2,0); 7,331 (3,4); 7,312 (1,5); 3,342 (0,4); 3,308 (118,1); 3,290 (0,6); 2,669 (0,3); 2,504 (47,2); 2,500 (55,9); 2,496 (39,1); 2,443 (1,2); 2,425 (3,6); 2,406 (3,6); 2,387 (1,2); 2,327 (0,3); 2,097 (16,0); 1,595 (1,7); 1,581 (4,8); 1,574 (4,3); 1,561 (1,8); 1,293 (2,0); 1,279 (4,8); 1,273 (4,3); 1,258 (1,6); 1,236 (0,8); 1,041 (4,9); 1,022 (10,0); 1,003 (4,6); 0,000 (32,0); -0,008 (1,3) |
| Beispiel I-T3-70: ¹H-NMR (400,2 MHz, d₆-DMSO): δ= 8,528 (3,4); 8,524 (3,6); 8,456 (1,7); 8,445 (1,7); 8,270 (4,0); 7,896 (0,9); 7,891 (0,9); 7,877 (1,7); 7,873 (1,8); 7,858 (1,0); 7,854 (0,9); 7,598 (2,8); 7,565 (2,8); 7,403 (0,8); 7,399 (0,8); 7,384 (1,7); 7,368 (1,2); 7,364 (1,1); 7,303 (2,5); 7,284 (3,9); 7,265 (1,7); 3,309 (77,4); 2,875 (0,6); 2,866 (0,8); 2,857 (1,3); 2,847 (1,4); 2,839 (0,8); 2,829 (0,6); 2,509 (11,1); 2,505 (23,7); 2,500 (33,3); 2,496 (24,3); 2,491 (11,5); 2,442 (1,1); 2,423 (3,4); 2,404 (3,5); 2,386 (1,2); 2,096 (16,0); 1,236 (0,6); 1,040 (5,1); 1,021 (11,2); 1,002 (5,0); 0,725 (0,9); 0,712 (2,5); 0,707 (3,5); 0,695 (3,3); 0,689 (2,7); 0,677 (1,2); 0,557 (1,2); 0,546 (3,4); 0,540 (3,0); 0,536 (2,8); 0,530 (2,8); 0,518 (0,9); 0,008 (0,7); 0,000 (21,2); -0,009 (0,8) |
| Beispiel I-T3-71: ¹H-NMR (400,0 MHz, CD3CN): δ= 8,160 (3,6); 8,139 (3,7); 7,764 (2,0); 7,739 (2,0); 7,733 (2,7); 7,704 (1,3); 7,699 (1,2); 7,683 (3,4); 7,678 (2,3); 7,553 (1,2); 7,502 (2,4); 7,481 (1,9); 3,060 (0,5); 2,851 (0,5); 2,520 (0,8); 2,501 (2,5); 2,482 (2,5); 2,463 (0,9); 2,134 (30,8); 2,114 (0,4); 2,107 (0,5); 2,101 (0,4); 1,964 (2,0); 1,958 (4,9); 1,952 (28,1); 1,946 (52,7); 1,940 (72,6); 1,934 (52,6); 1,928 (28,4); 1,768 (0,4); 1,762 (0,3); 1,598 (1,0); 1,583 (2,5); 1,577 (2,7); 1,563 (1,4); 1,437 (16,0); 1,361 (1,3); 1,347 (2,7); 1,340 (2,9); 1,326 (1,1); 1,102 (3,5); 1,083 (7,4); 1,064 (3,4); 0,146 (0,4); 0,008 (3,1); 0,000 (97,3); -0,150 (0,4) |
| Beispiel I-T3-72: ¹H-NMR (400,0 MHz, CD3CN): δ= 8,150 (5,4); 8,130 (5,6); 7,764 (2,7); 7,689 (4,2); 7,684 (6,5); 7,655 (1,9); 7,650 (1,5); 7,634 (2,2); 7,629 (1,9); 7,469 (3,6); 7,448 (2,9); 6,939 (0,9); 2,871 (0,6); 2,862 (0,9); 2,853 (1,4); 2,843 (1,4); 2,834 (1,0); 2,825 (0,6); 2,518 (1,2); 2,499 (3,7); 2,480 (3,8); 2,462 (1,4); 2,168 (77,5); 2,114 (0,3); 2,108 (0,4); 1,965 (1,9); 1,959 (4,8); 1,953 (25,8); 1,947 (47,0); 1,941 (63,4); 1,935 (44,6); 1,929 (23,7); 1,769 (0,4); 1,437 (16,0); 1,100 (5,0); 1,081 (10,2); 1,062 (4,8); 0,794 (0,8); 0,781 (2,3); 0,776 (3,1); 0,763 (3,2); 0,758 (2,4); 0,746 (1,1); 0,611 (1,0); 0,600 (2,8); 0,594 (2,9); 0,590 (2,6); 0,585 (2,6); 0,572 (0,8); 0,146 (0,3); 0,008 (2,6); 0,000 (69,9); -0,008 (4,3); -0,149 (0,3) |
| Beispiel I-T3-73: ¹H-NMR (400,1 MHz, d₆-DMSO): 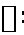 = 8,77 (0,0325); 8,76 (0,0329); 8,67 (0,0447); 8,40 (0,0688); 8,07 (0,1396); 7,89 (0,0039); 7,88 (0,0202); 7,87 (0,0405); 7,43 (0,0406); 7,42 (0,0383); 3,78 (0,0029); 3,59 (0,0071); 3,30 (1,0000); 3,17 (0,0044); 3,16 (0,0042); 2,85 (0,0123); 2,84 (0,0187); 2,83 (0,0088); 2,64 (0,0025); 2,50 (0,4120); 2,37 (0,0016); 1,24 (0,0054); 0,73 (0,0482); 0,72 (0,0467); 0,71 (0,0149); 0,52 (0,0154); 0,50 (0,0420); 0,49 (0,0128); 0,12 (0,0012); 0,00 (0,2886); -0,12 (0,0012) |
| Beispiel I-T3-74: ¹H-NMR (400,0 MHz, d₆-DMSO): δ= 9,381 (5,5); 8,653 (5,5); 8,317 (0,7); 8,293 (5,6); 7,988 (1,2); 7,984 (1,3); 7,969 (2,4); 7,965 (2,4); 7,950 (1,4); 7,946 (1,3); 7,747 (4,4); 7,734 (1,5); 7,713 (7,2); 7,706 (4,3); 7,683 (0,7); 7,493 (1,1); 7,489 (1,2); 7,474 (2,5); 7,458 (1,6); 7,454 (1,5); 7,362 (3,0); 7,343 (5,1); 7,324 (2,2); 3,903 (11,7); 3,333 (264,8); 3,267 (0,3); 3,174 (0,6); 3,162 (0,6); 2,802 (1,7); 2,783 (5,4); 2,765 (5,5); 2,746 (1,8); 2,676 (1,4); 2,672 (1,9); 2,667 (1,4); 2,542 (1,1); 2,525 (5,6); 2,511 (121,2); 2,507 (238,9); 2,503 (309,3); 2,498 (228,5); 2,494 (115,5); 2,334 (1,3); 2,329 (1,8); 2,325 (1,4); 1,603 (2,2); 1,589 (5,6); 1,582 (6,0); 1,569 (2,6); 1,298 (2,8); 1,285 (5,7); 1,278 (6,1); 1,264 (2,3); 1,237 (0,5); 1,060 (7,3); 1,042 (16,0); 1,023 (7,2); 0,008 (0,6); 0,000 (17,6); -0,008 (0,7) |
| Beispiel I-T3-75: ¹H-NMR (400,2 MHz, d₆-DMSO): δ= 8,621 (4,9); 8,618 (5,3); 8,463 (2,4); 8,452 (2,4); 8,277 (5,0); 8,274 (5,4); 7,913 (1,3); 7,909 (1,4); 7,894 (2,4); 7,890 (2,5); 7,875 (1,4); 7,871 (1,3); 7,742 (4,3); 7,730 (1,5); 7,709 (7,2); 7,702 (4,2); 7,679 (0,7); 7,416 (1,1); 7,412 (1,2); 7,397 (2,4); 7,381 (1,7); 7,377 (1,6); 7,313 (3,4); 7,294 (5,4); 7,275 (2,3); 3,333 (0,6); 3,324 (0,5); 3,307 (125,1); 3,285 (0,5); 2,879 (0,8); 2,870 (1,2); 2,861 (1,9); 2,851 (1,9); 2,842 (1,2); 2,833 (0,9); 2,822 (0,4); 2,798 (1,7); 2,780 (5,3); 2,761 (5,5); 2,742 (1,8); 2,509 (17,8); 2,505 (38,4); 2,500 (54,1); 2,496 (39,8); 2,491 (19,1); 2,327 (0,3); 1,235 (0,5); 1,061 (7,3); 1,042 (16,0); 1,023 (7,1); 0,729 (1,2); 0,716 (3,4); 0,711 (4,8); 0,699 (4,5); 0,693 (3,7); 0,682 (1,6); 0,562 (1,6); 0,552 (4,7); 0,546 (4,2); 0,542 (4,0); 0,536 (3,9); 0,524 (1,2); 0,008 (1,4); 0,000 (45,5); -0,009 (1,9) |
| Beispiel I-T3-76: ¹H-NMR (400,1 MHz, d₆-DMSO): δ= 9,531 (3,2); 9,102 (3,2); 9,096 (3,3); 8,822 (3,2); 8,817 (3,3); 8,727 (5,1); 8,442 (1,9); 8,436 (3,4); 8,428 (5,4); 7,617 (2,1); 7,582 (2,1); 5,759 (16,0); 3,568 (2,8); 3,437 (0,3); 3,424 (0,4); 3,326 (355,3); 3,303 (1,2); 2,711 (0,5); 2,675 (0,6); 2,670 (0,7); 2,667 (0,5); 2,557 (0,4); 2,554 (0,7); 2,552 (0,9); 2,551 (1,1); 2,541 (159,1); 2,530 (1,2); 2,528 (1,0); 2,527 (1,0); 2,524 (1,3); 2,510 (33,8); 2,506 (67,9); 2,502 (90,5); 2,497 (63,3); 2,493 (29,5); 2,458 (0,9); 2,440 (2,5); 2,421 (2,5); 2,402 (0,9); 2,368 (0,6); 2,333 (0,6); 2,329 (0,7); 2,324 (0,6); 2,111 (12,0); 2,086 (1,1); 1,629 (1,2); 1,615 (2,9); 1,608 (3,2); 1,595 (1,5); 1,346 (1,4); 1,332 (2,9); 1,325 (3,2); 1,311 (1,1); 1,072 (0,6); 1,055 (1,3); 1,048 (4,1); 1,037 (0,9); 1,029 (8,8); 1,010 (3,8); 0,008 (2,0); 0,000 (66,6); -0,008 (2,4); -0,014 (0,4) |
| Beispiel I-T3-77: ¹H-NMR (400,1 MHz, d₆-DMSO): δ= 9,043 (4,2); 9,038 (4,3); 8,798 (4,2); 8,793 (4,4); 8,705 (6,6); 8,645 (1,9); 8,635 (2,0); 8,409 (6,8); 8,389 (2,6); 8,384 (4,6); 8,379 (2,6); 7,613 (2,8); 7,580 (2,9); 5,759 (4,7); 3,327 (158,6); 2,902 (0,6); 2,892 (1,0); 2,884 (1,5); 2,874 (1,5); 2,866 (1,0); 2,856 (0,7); 2,671 (0,4); 2,541 (65,9); 2,511 (20,9); 2,506 (42,9); 2,502 (59,0); 2,498 (43,4); 2,493 (22,4); 2,458 (2,1); 2,438 (3,5); 2,419 (3,4); 2,401 (1,3); 2,367 (0,3); 2,329 (0,4); 2,110 (16,0); 2,086 (1,2); 1,989 (0,4); 1,072 (0,4); 1,055 (1,0); 1,048 (5,3); 1,029 (11,6); 1,010 (5,3); 0,763 (0,9); 0,750 (2,3); 0,745 (3,4); 0,733 (3,3); 0,727 (2,8); 0,716 (1,4); 0,617 (1,2); 0,607 (3,5); 0,600 (3,1); 0,591 (2,8); 0,579 (1,0); 0,008 (1,3); 0,000 (40,7); -0,008 (2,2) |
| Beispiel I-T3-78: ¹H-NMR (400,1 MHz, d₆-DMSO): δ= 9,542 (5,5); 9,127 (5,7); 9,122 (5,9); 8,857 (8,8); 8,834 (5,6); 8,829 (5,9); 8,472 (3,3); 8,467 (5,7); 8,461 (3,2); 8,434 (8,9); 7,757 (4,0); 7,724 (7,7); 7,699 (0,6); 5,759 (5,9); 4,020 (0,4); 3,611 (0,6); 3,568 (1,5); 3,426 (0,9); 3,326 (364,4); 3,303 (1,4); 3,235 (1,3); 2,821 (1,6); 2,802 (5,1); 2,783 (5,1); 2,765 (1,7); 2,711 (0,6); 2,670 (0,9); 2,666 (0,7); 2,541 (164,7); 2,510 (48,8); 2,506 (100,5); 2,502 (137,3); 2,497 (98,8); 2,493 (47,9); 2,367 (0,6); 2,329 (0,8); 1,989 (1,5); 1,633 (2,1); 1,619 (5,2); 1,612 (5,6); 1,599 (2,4); 1,350 (2,6); 1,337 (5,3); 1,330 (5,5); 1,316 (2,0); 1,234 (0,4); 1,192 (0,4); 1,174 (0,8); 1,156 (0,5); 1,146 (0,5); 1,069 (7,2); 1,050 (16,0); 1,032 (7,1); 0,146 (0,4); 0,008 (2,9); 0,000 (90,5); -0,008 (3,5) |
| Beispiel I-T3-79: ¹H-NMR (500,1 MHz, d₆-DMSO): δ= 9,064 (5,0); 9,059 (5,1); 8,821 (8,9); 8,811 (5,2); 8,807 (5,3); 8,630 (2,5); 8,622 (2,5); 8,413 (3,8); 8,408 (13,3); 7,748 (4,3); 7,738 (1,8); 7,721 (6,6); 7,712 (3,8); 7,695 (0,9); 5,752 (1,0); 3,305 (76,3); 3,281 (0,4); 2,910 (0,4); 2,902 (0,9); 2,895 (1,3); 2,888 (2,0); 2,880 (2,0); 2,873 (1,3); 2,865 (1,0); 2,858 (0,4); 2,813 (1,7); 2,798 (5,5); 2,783 (5,6); 2,768 (1,9); 2,508 (13,7); 2,504 (28,5); 2,501 (39,3); 2,497 (29,3); |
| 2,494 (14,5); 1,908 (2,7); 1,236 (0,5); 1,068 (7,4); 1,053 (16,0); 1,038 (7,3); 0,761 (1,2); 0,751 (3,5); 0,747 (4,8); 0,737 (4,6); 0,733 (3,8); 0,723 (1,6); 0,620 (1,6); 0,612 (4,7); 0,607 (4,4); 0,604 (4,2); 0,599 (4,0); 0,589 (1,2); 0,006 (1,3); 0,000 (30,6); -0,007 (1,4) |
| Beispiel I-T3-80: ¹H-NMR (400,0 MHz, CD3CN): δ= 8,128 (2,6); 8,082 (2,8); 7,736 (1,6); 7,731 (2,1); 7,701 (1,1); 7,696 (0,8); 7,680 (1,2); 7,675 (1,1); 7,608 (0,4); 7,562 (1,4); 7,549 (1,4); 7,494 (1,9); 7,473 (1,5); 2,448 (0,6); 2,429 (1,9); 2,410 (2,0); 2,391 (0,7); 2,164 (9,8); 2,155 (20,2); 2,088 (7,6); 1,965 (1,0); 1,959 (2,5); 1,953 (13,9); 1,946 (25,4); 1,940 (34,2); 1,934 (24,1); 1,928 (12,6); 1,598 (0,8); 1,583 (2,0); 1,577 (2,0); 1,563 (1,1); 1,437 (16,0); 1,358 (1,0); 1,345 (2,0); 1,338 (2,1); 1,323 (0,8); 1,268 (0,7); 1,092 (2,7); 1,073 (5,7); 1,054 (2,6); 0,008 (1,1); 0,000 (34,6); -0,009 (1,5) |
| Beispiel I-T3-81: ¹H-NMR (400,0 MHz, CD3CN): δ= 8,115 (7,2); 8,067 (7,4); 7,688 (4,4); 7,683 (5,6); 7,667 (0,5); 7,652 (3,0); 7,646 (2,4); 7,631 (3,5); 7,625 (3,0); 7,560 (4,1); 7,546 (4,1); 7,462 (5,2); 7,441 (4,2); 6,903 (1,2); 2,880 (0,3); 2,871 (0,9); 2,861 (1,3); 2,853 (2,1); 2,843 (2,1); 2,835 (1,3); 2,825 (1,0); 2,815 (0,4); 2,447 (1,8); 2,428 (5,5); 2,409 (5,7); 2,390 (1,9); 2,141 (55,3); 2,120 (1,0); 2,113 (0,9); 2,107 (1,0); 2,101 (0,9); 2,086 (21,5); 1,964 (4,2); 1,958 (10,5); 1,952 (54,5); 1,946 (98,5); 1,940 (132,1); 1,934 (92,1); 1,927 (48,4); 1,774 (0,5); 1,768 (0,8); 1,762 (0,5); 1,437 (1,3); 1,270 (1,0); 1,090 (7,7); 1,071 (16,0); 1,052 (7,4); 0,794 (1,1); 0,781 (3,3); 0,776 (4,4); 0,763 (4,7); 0,758 (3,4); 0,746 (1,6); 0,610 (1,5); 0,598 (3,8); 0,592 (4,0); 0,588 (3,6); 0,583 (3,6); 0,571 (1,1); 0,146 (1,8); 0,031 (0,4); 0,030 (0,4); 0,0272 (0,4); 0,0265 (0,4); 0,026 (0,4); 0,022 (0,6); 0,008 (16,0); 0,000 (381,4); -0,009 (18,6); -0,150 (1,8) |
| Beispiel I-T3-82: ¹H-NMR (400,0 MHz, CD3CN): δ= 8,127 (7,5); 8,085 (7,7); 7,734 (4,3); 7,729 (5,7); 7,708 (3,2); 7,703 (2,2); 7,688 (3,6); 7,682 (2,9); 7,561 (3,9); 7,548 (3,8); 7,512 (5,3); 7,491 (4,4); 7,365 (0,9); 4,141 (1,1); 4,124 (1,2); 4,117 (3,5); 4,100 (3,5); 4,093 (3,7); 4,077 (3,5); 4,070 (1,3); 4,053 (1,2); 2,462 (0,4); 2,457 (0,4); 2,450 (1,8); 2,431 (5,5); 2,412 (5,7); 2,393 (1,9); 2,150 (76,9); 2,120 (0,5); 2,113 (0,7); 2,107 (0,8); 2,101 (0,8); 2,090 (21,3); 1,964 (3,5); 1,958 (8,4); 1,952 (45,7); 1,946 (82,9); 1,940 (111,8); 1,934 (77,5); 1,927 (40,4); 1,774 (0,4); 1,768 (0,6); 1,762 (0,4); 1,437 (1,0); 1,269 (1,0); 1,093 (7,7); 1,074 (16,0); 1,055 (7,4); 0,146 (1,4); 0,008 (11,1); 0,000 (291,0); -0,009 (12,7); -0,150 (1,4) |
| Beispiel I-T3-83: ¹H-NMR (400,0 MHz, CD3CN): δ= 8,128 (7,9); 8,079 (8,4); 7,719 (4,9); 7,713 (6,1); 7,677 (3,1); 7,671 (2,5); 7,656 (3,5); 7,650 (3,1); 7,562 (4,8); 7,548 (4,7); 7,484 (5,6); 7,463 (5,1); 7,436 (1,2); 5,447 (1,2); 5,337 (1,3); 5,316 (2,5); 5,295 (2,5); 5,274 (1,3); 4,068 (0,4); 4,050 (0,4); 3,543 (3,2); 3,540 (2,1); 3,520 (6,4); 3,498 (4,2); 3,370 (4,2); 3,367 (2,8); 3,350 (6,2); 3,346 (5,9); 3,326 (3,3); 2,462 (0,3); 2,451 (2,0); 2,432 (6,0); 2,413 (6,2); 2,394 (2,1); 2,150 (115,6); 2,120 (0,5); 2,114 (0,7); 2,107 (0,9); 2,091 (23,5); 1,972 (1,9); 1,964 (3,0); 1,958 (7,9); 1,952 (39,4); 1,946 (71,5); 1,940 (96,0); 1,934 (68,4); 1,928 (36,7); 1,774 (0,4); 1,768 (0,6); 1,762 (0,4); 1,437 (2,7); 1,268 (1,0); 1,221 (0,4); 1,204 (0,8); 1,186 (0,4); 1,094 (7,8); 1,075 (16,0); 1,056 (7,5); 0,146 (1,1); 0,008 (9,8); 0,000 (220,9); -0,150 (1,1) |
| Beispiel I-T3-84: ¹H-NMR (400,0 MHz, CD3CN): δ= 8,162 (9,4); 8,125 (7,5); 7,997 (4,3); 7,952 (4,4); 7,729 (4,8); 7,724 (6,8); 7,707 (3,5); 7,701 (2,3); 7,686 (3,9); 7,680 (3,2); 7,519 (6,1); 7,498 (4,9); 7,348 (1,3); 4,140 (1,3); 4,123 (1,4); 4,116 (3,9); 4,100 (3,9); 4,092 (4,2); 4,076 (3,9); 4,069 (1,7); 4,052 (1,4); 2,146 (92,5); 2,120 (27,2); 2,108 (1,6); 2,101 (0,9); 2,095 (0,5); 1,971 (0,9); 1,964 (3,5); 1,958 (9,1); 1,952 (47,9); 1,946 (87,4); 1,940 (118,0); 1,934 (83,5); 1,928 (44,7); 1,774 (0,5); 1,768 (0,7); 1,762 (0,5); 1,437 (16,0); 1,270 (0,6); 0,146 (0,9); 0,008 (6,8); 0,000 (179,6); -0,008 (10,9); - 0,150 (0,9) |
| Beispiel I-T3-85: ¹H-NMR (400,0 MHz, CD3CN): δ= 8,163 (5,2); 8,118 (4,2); 7,998 (2,5); 7,953 (2,5); 7,716 (3,0); 7,710 (3,7); 7,675 (1,9); 7,669 (1,5); 7,654 (2,2); 7,648 (1,9); 7,490 (3,5); 7,469 (2,8); 7,418 (0,8); 7,400 (0,8); 5,335 (0,8); 5,314 (1,6); 5,293 (1,6); 5,272 (0,8); 3,542 (2,0); 3,538 (1,3); 3,518 (4,0); 3,496 (2,6); 3,370 (2,6); 3,367 (1,7); 3,349 (3,8); 3,346 (3,6); 3,326 (2,0); 2,133 (15,0); 2,120 (15,4); 2,101 (0,5); 1,971 (1,1); 1,964 (1,7); 1,958 (4,3); 1,952 (19,7); 1,946 (35,3); 1,940 (47,2); 1,934 (33,6); 1,927 (18,1); 1,437 (16,0); 1,204 (0,4); 0,146 (0,4); 0,008 (3,9); 0,000 (78,7); -0,150 (0,4) |
| Beispiel I-T3-86: ¹H-NMR (400,0 MHz, CD3CN): δ= 8,252 (0,3); 8,146 (2,7); 8,110 (0,4); 8,102 (2,9); 7,899 (1,3); 7,708 (1,4); 7,690 (1,7); 7,685 (2,2); 7,655 (1,2); 7,649 (0,9); 7,634 (1,5); 7,628 (1,4); 7,468 (2,2); 7,447 (1,8); 6,891 (0,5); 2,871 (0,4); 2,862 (0,5); 2,853 (0,8); 2,843 (0,8); 2,835 (0,5); 2,825 (0,4); 2,415 (1,0); 2,171 (8,3); 2,132 (10,6); 1,971 (0,5); 1,964 (1,1); 1,958 (2,6); 1,952 (13,9); 1,946 (25,2); 1,940 (34,0); 1,933 (23,7); 1,927 (12,5); 1,437 (16,0); 1,269 (0,4); 0,794 (0,4); 0,781 (1,3); 0,776 (1,7); 0,764 (1,8); 0,758 (1,3); 0,746 (0,6); 0,611 (0,6); 0,599 (1,5); 0,593 (1,6); 0,589 (1,4); 0,584 (1,4); 0,571 (0,5); 0,008 (2,3); 0,000 (63,9); -0,009 (3,1) |
| Beispiel I-T3-87: ¹H-NMR (400,0 MHz, CD3CN): δ= 8,171 (11,0); 8,170 (11,0); 8,122 (12,2); 8,120 (11,4); 7,729 (6,8); 7,724 (8,7); 7,697 (4,9); 7,691 (3,7); 7,676 (5,6); 7,670 (4,6); 7,606 (16,0); 7,604 (15,8); 7,551 (3,2); 7,500 (8,3); 7,479 (6,7); 5,446 (0,7); 4,085 (0,6); 4,068 (2,0); 4,050 (2,0); 4,032 (0,7); 3,240 (0,6); 2,132 (42,8); 2,119 (0,5); 2,113 (0,7); 2,107 (0,9); 2,101 (0,6); 2,095 (0,3); 1,971 (9,0); 1,964 (4,0); 1,958 (10,1); 1,952 (56,8); 1,946 (103,2); 1,940 (138,6); 1,933 (95,2); 1,927 (49,0); 1,914 (0,7); 1,780 (0,3); 1,774 (0,6); 1,768 (0,8); 1,762 (0,6); 1,595 (3,6); 1,581 (8,5); 1,574 (8,5); 1,560 (4,6); 1,520 (0,5); 1,437 (11,1); 1,400 (0,6); 1,360 (4,7); 1,346 (8,6); 1,340 (8,8); 1,325 (3,6); 1,317 (0,8); 1,287 (0,4); 1,269 (1,4); 1,221 (2,5); 1,204 (4,7); 1,186 (2,3); 0,146 (1,8); 0,008 (14,1); 0,000 (400,9); -0,009 (15,1); -0,150 (1,8) |
| Beispiel I-T3-88: ¹H-NMR (400,0 MHz, CD3CN): δ= 8,160 (10,6); 8,111 (11,3); 7,681 (6,2); 7,675 (7,9); 7,647 (3,9); 7,642 (3,1); 7,627 (4,5); 7,621 (4,0); 7,605 (16,0); 7,467 (7,2); 7,447 (5,7); 6,936 (2,0); 5,448 (0,4); 4,067 (0,5); 4,049 (0,5); 2,879 (0,4); 2,869 (1,3); 2,860 (2,0); 2,851 (2,8); 2,842 (2,9); 2,833 (2,0); 2,824 (1,4); 2,814 (0,5); 2,467 (1,2); 2,462 (1,7); 2,458 (1,3); 2,253 (0,6); 2,226 (0,4); 2,158 (239,6); 2,120 (1,0); 2,113 (1,3); 2,107 (1,6); 2,101 (1,2); 2,095 (0,7); 1,971 (3,4); 1,964 (8,0); 1,958 (20,3); 1,952 (98,7); 1,946 (178,8); 1,940 (240,6); 1,934 (170,9); 1,928 (91,2); 1,781 (0,5); 1,774 (1,0); 1,768 (1,3); 1,762 (1,0); 1,756 (0,5); 1,437 (3,0); 1,269 (0,9); 1,221 (0,5); 1,203 (1,0); 1,185 (0,5); 0,792 (1,7); 0,779 (4,8); 0,774 (6,6); 0,761 (6,5); 0,756 (5,1); 0,744 (2,2); 0,610 (2,2); 0,599 (6,0); 0,592 (6,3); 0,583 (5,4); 0,571 (1,6); 0,146 (3,3); 0,007 (29,1); 0,000 (640,2); -0,150 (3,3) |
| Beispiel I-T3-89: ¹H-NMR (400,0 MHz, d₆-DMSO): δ= 9,437 (11,1); 8,788 (15,9); 8,367 (16,0); 8,317 (0,8); 7,986 (0,4); 7,903 (6,6); 7,897 (6,7); 7,824 (9,1); 7,810 (13,0); 7,805 (11,4); 7,802 (13,2); 7,797 (8,0); 7,791 (3,7); 7,614 (3,7); 7,610 (3,5); 7,591 (2,9); 7,588 (2,9); 7,560 (9,0); 7,551 (1,7); 7,546 (1,5); 7,537 (7,7); 3,903 (8,5); 3,332 (418,5); 3,174 (0,7); 3,162 (0,6); 2,676 (2,0); 2,671 (2,7); 2,667 (2,1); 2,541 (1,6); 2,507 (359,7); 2,502 (464,1); 2,498 (346,7); 2,333 (2,1); 2,329 (2,9); 2,325 (2,2); 1,618 (3,7); 1,604 (9,3); 1,597 (9,9); 1,584 (4,2); 1,543 (0,4); 1,327 (0,4); 1,287 (4,3); 1,274 (9,4); 1,267 (9,9); 1,253 (3,8); 1,234 (1,5); 1,215 (0,5); 1,181 (0,4); 1,177 (0,4); 0,861 (0,4); 0,853 (0,4); 0,843 (0,4); 0,834 (0,4); 0,824 (0,4); |
| 0,813 (0,3); 0,008 (0,9); 0,000 (24,6); -0,008 (1,1) |
| Beispiel I-T3-90: ¹H-NMR (400,0 MHz, CD3CN): δ= 8,565 (0,4); 8,349 (0,6); 8,333 (14,0); 8,123 (0,6); 8,109 (13,1); 8,096 (0,6); 7,904 (0,4); 7,881 (0,4); 7,712 (8,7); 7,689 (16,0); 7,683 (10,7); 7,672 (1,0); 7,654 (5,5); 7,648 (4,2); 7,633 (6,1); 7,627 (5,3); 7,617 (5,0); 7,611 (5,5); 7,475 (0,5); 7,467 (9,7); 7,454 (0,7); 7,446 (9,4); 7,438 (3,3); 7,422 (2,4); 7,419 (2,6); 7,416 (2,5); 6,891 (2,1); 5,446 (0,4); 3,899 (0,6); 2,881 (0,6); 2,872 (1,7); 2,862 (2,4); 2,854 (3,8); 2,844 (3,9); 2,835 (2,5); 2,826 (1,8); 2,816 (0,6); 2,132 (62,2); 2,113 (1,1); 2,107 (1,3); 2,101 (0,9); 2,095 (0,5); 1,996 (0,3); 1,971 (0,9); 1,964 (5,7); 1,958 (14,2); 1,952 (81,0); 1,946 (147,9); 1,940 (200,1); 1,933 (139,4); 1,927 (72,6); 1,780 (0,5); 1,774 (0,8); 1,768 (1,1); 1,762 (0,8); 1,756 (0,4); 1,268 (2,3); 0,881 (0,3); 0,796 (2,0); 0,783 (5,8); 0,778 (7,9); 0,765 (8,2); 0,760 (6,0); 0,748 (2,8); 0,726 (0,4); 0,709 (0,4); 0,653 (0,3); 0,643 (0,3); 0,613 (2,8); 0,601 (6,7); 0,595 (7,1); 0,591 (6,3); 0,586 (6,3); 0,574 (2,0); 0,146 (2,4); 0,008 (17,6); 0,000 (508,1); -0,009 (24,9); -0,150 (2,4) |
| Beispiel I-T3-91: ¹H-NMR (400,0 MHz, d₆-DMSO): δ= 8,837 (3,4); 8,831 (3,6); 8,747 (6,3); 8,532 (1,9); 8,521 (2,0); 8,474 (6,2); 8,100 (9,7); 7,954 (3,3); 7,949 (3,5); 4,109 (0,4); 4,095 (0,4); 3,904 (16,0); 3,335 (287,0); 3,267 (0,5); 3,243 (0,4); 3,174 (2,4); 3,162 (2,5); 2,877 (0,6); 2,868 (0,9); 2,859 (1,3); 2,849 (1,3); 2,840 (0,9); 2,831 (0,6); 2,676 (1,0); 2,671 (1,3); 2,667 (1,0); 2,507 (156,9); 2,502 (206,2); 2,498 (158,4); 2,334 (0,9); 2,329 (1,2); 2,325 (0,9); 1,258 (0,4); 1,002 (1,3); 0,986 (1,2); 0,740 (0,8); 0,727 (2,3); 0,722 (3,1); 0,710 (2,9); 0,704 (2,4); 0,693 (1,0); 0,568 (1,0); 0,558 (3,1); 0,552 (2,9); 0,548 (2,7); 0,542 (2,5); 0,530 (0,7); 0,000 (1,8) |
| Beispiel I-T3-92: ¹H-NMR (400,0 MHz, d₆-DMSO): δ= 9,415 (4,9); 8,892 (4,2); 8,886 (4,4); 8,765 (7,6); 8,488 (7,3); 8,104 (11,7); 8,044 (4,2); 8,039 (4,3); 3,904 (16,0); 3,593 (0,4); 3,336 (427,9); 3,173 (1,6); 3,163 (1,6); 2,676 (1,4); 2,672 (1,7); 2,667 (1,3); 2,518 (32,9); 2,511 (114,6); 2,507 (211,5); 2,503 (266,6); 2,498 (196,7); 2,334 (1,1); 2,329 (1,5); 2,325 (1,1); 1,613 (1,8); 1,599 (4,5); 1,592 (4,8); 1,579 (2,0); 1,315 (2,1); 1,301 (4,6); 1,295 (4,7); 1,280 (1,7); 1,235 (0,3); 0,000 (2,1) |
| Beispiel I-T3-93: ¹H-NMR (400,0 MHz, CD3CN): δ= 8,187 (3,1); 8,146 (3,3); 7,899 (4,8); 7,653 (1,3); 7,648 (2,7); 7,639 (0,5); 7,624 (1,5); 7,618 (1,0); 7,462 (1,9); 7,441 (1,5); 7,115 (0,7); 5,449 (1,2); 4,068 (0,5); 4,050 (0,5); 2,174 (33,4); 1,972 (2,4); 1,965 (0,8); 1,959 (1,9); 1,953 (10,3); 1,947 (18,6); 1,941 (24,9); 1,934 (16,9); 1,928 (8,7); 1,448 (9,5); 1,437 (16,0); 1,270 (0,5); 1,221 (0,6); 1,204 (1,2); 1,186 (0,6); 0,837 (0,7); 0,824 (2,1); 0,820 (2,2); 0,808 (0,9); 0,673 (1,1); 0,661 (2,4); 0,656 (2,5); 0,644 (0,8); 0,008 (0,4); 0,000 (10,3); -0,009 (0,3) |
| Beispiel I-T3-94: ¹H-NMR (400,0 MHz, CD3CN): δ= 8,149 (5,3); 8,106 (4,3); 7,996 (2,3); 7,951 (2,4); 7,645 (2,3); 7,639 (5,5); 7,632 (0,8); 7,618 (2,7); 7,612 (1,8); 7,457 (3,0); 7,436 (2,4); 7,098 (1,2); 2,468 (0,4); 2,464 (0,5); 2,459 (0,4); 2,165 (184,2); 2,116 (15,3); 2,102 (0,5); 1,972 (1,1); 1,965 (3,0); 1,959 (7,6); 1,953 (41,5); 1,947 (74,8); 1,940 (100,0); 1,934 (68,4); 1,928 (35,0); 1,775 (0,4); 1,769 (0,6); 1,763 (0,4); 1,447 (16,0); 1,437 (3,0); 1,270 (2,2); 1,204 (0,3); 0,835 (1,1); 0,822 (3,7); 0,818 (3,8); 0,807 (1,5); 0,673 (1,8); 0,662 (4,3); 0,656 (4,3); 0,644 (1,3); 0,008 (1,2); 0,000 (38,1); -0,009 (1,4) |
| Beispiel I-T3-95: ¹H-NMR (400,0 MHz, d₆-DMSO): δ= 9,596 (2,3); 8,893 (2,0); 8,887 (2,0); 8,791 (3,3); 8,545 (3,3); 8,315 (0,5); 8,300 (2,2); 8,294 (2,3); 8,283 (5,7); 4,038 (0,4); 4,020 (0,4); 3,322 (38,3); 2,671 (0,6); 2,502 (83,6); 2,328 (0,6); 1,989 (1,8); 1,643 (0,8); 1,628 (2,2); 1,621 (2,3); 1,609 (0,9); 1,398 (16,0); 1,298 (1,0); 1,285 (2,2); 1,278 (2,3); 1,264 (0,8); 1,193 (0,5); 1,175 (0,9); 1,157 (0,5); 0,146 (0,4); 0,000 (75,9); -0,150 (0,4) |
| Beispiel I-T3-96: ¹H-NMR (400,0 MHz, d₆-DMSO): δ= 8,838 (2,0); 8,833 (2,0); 8,618 (3,2); 8,513 (1,0); 8,502 (1,0); 8,377 (3,3); 7,954 (1,9); 7,949 (1,9); 7,596 (3,6); 4,104 (0,3); 3,903 (10,1); 3,409 (0,5); 3,350 (346,6); 3,302 (0,5); 3,175 (1,8); 3,162 (1,8); 2,865 (0,4); 2,857 (0,7); 2,847 (0,7); 2,838 (0,4); 2,677 (0,4); 2,672 (0,6); 2,668 (0,4); 2,526 (1,8); 2,512 (37,4); 2,508 (74,7); 2,503 (97,3); 2,499 (71,2); 2,494 (35,6); 2,489 (12,6); 2,335 (0,4); 2,330 (0,6); 2,325 (0,5); 2,117 (16,0); 1,003 (0,6); 0,987 (0,6); 0,739 (0,5); 0,726 (1,2); 0,721 (1,7); 0,709 (1,6); 0,703 (1,3); 0,692 (0,6); 0,568 (0,6); 0,557 (1,7); 0,551 (1,5); 0,547 (1,4); 0,542 (1,4); 0,529 (0,4); 0,000 (0,7) |
| Beispiel I-T3-97: ¹H-NMR (400,0 MHz, d₆-DMSO): δ= 9,395 (2,2); 8,892 (2,0); 8,886 (2,0); 8,640 (3,3); 8,391 (3,4); 8,040 (1,9); 8,035 (1,9); 7,599 (3,7); 4,108 (0,5); 4,095 (0,5); 3,904 (10,2); 3,333 (130,8); 3,174 (2,4); 3,161 (2,4); 2,676 (0,4); 2,671 (0,6); 2,667 (0,5); 2,541 (0,4); 2,525 (1,8); 2,511 (39,6); 2,507 (81,0); 2,502 (95,3); 2,498 (69,0); 2,493 (34,2); 2,334 (0,4); 2,329 (0,6); 2,324 (0,4); 2,118 (16,0); 1,613 (0,8); 1,599 (1,9); 1,592 (2,1); 1,579 (0,9); 1,311 (0,9); 1,297 (2,0); 1,291 (2,1); 1,276 (0,8); 1,002 (0,5); 0,987 (0,5); 0,000 (1,2) |
| Beispiel I-T3-98: ¹H-NMR (400,0 MHz, d₆-DMSO): δ= 8,839 (6,0); 8,833 (6,2); 8,791 (0,5); 8,781 (9,8); 8,692 (3,1); 8,681 (3,2); 8,549 (0,5); 8,540 (9,8); 8,282 (16,0); 8,217 (6,2); 8,210 (6,1); 5,756 (1,1); 3,326 (37,2); 2,871 (0,8); 2,861 (1,2); 2,852 (1,8); 2,842 (1,9); 2,833 (1,2); 2,824 (0,9); 2,814 (0,3); 2,671 (0,4); 2,525 (0,9); 2,511 (20,9); 2,507 (43,3); 2,502 (58,6); 2,498 (44,1); 2,494 (22,5); 2,329 (0,4); 1,989 (0,4); 1,397 (0,4); 0,757 (1,2); 0,744 (3,4); 0,739 (4,7); 0,726 (4,5); 0,721 (3,8); 0,709 (1,5); 0,568 (1,5); 0,557 (4,5); 0,551 (4,2); 0,548 (4,1); 0,542 (3,9); 0,530 (1,2); 0,146 (0,5); 0,008 (3,4); 0,000 (97,0); -0,008 (4,2); -0,150 (0,5) |
| Beispiel I-T3-99: ¹H-NMR (400,0 MHz, d₆-DMSO): δ= 8,851 (0,6); 8,845 (0,8); 8,840 (3,5); 8,834 (3,6); 8,770 (6,0); 8,525 (5,8); 8,337 (3,6); 8,331 (3,5); 8,283 (11,0); 8,226 (0,6); 8,220 (0,6); 3,328 (25,7); 3,030 (16,0); 2,798 (0,7); 2,790 (0,8); 2,781 (1,3); 2,771 (0,9); 2,758 (2,9); 2,543 (55,4); 2,525 (0,6); 2,508 (28,1); 2,503 (37,2); 2,499 (27,6); 0,814 (0,3); 0,773 (0,4); 0,604 (0,5); 0,585 (2,1); 0,576 (2,8); 0,566 (1,0); 0,543 (1,2); 0,532 (2,2); 0,514 (2,0); 0,501 (0,4); 0,495 (0,4); 0,008 (1,0); 0,000 (28,2); -0,008 (1,0) |
| Beispiel I-T3-100: ¹H-NMR (400,0 MHz, d₆-DMSO): δ= 8,826 (5,3); 8,817 (2,9); 8,811 (2,8); 8,531 (4,2); 8,320 (2,7); 8,314 (2,7); 8,206 (0,4); 8,200 (0,4); 8,111 (7,7); 3,904 (16,0); 3,395 (0,6); 3,337 (288,8); 3,257 (0,4); 3,243 (0,4); 3,175 (0,9); 3,162 (1,0); 3,029 (12,3); 2,795 (0,5); 2,788 (0,6); 2,778 (1,0); 2,768 (0,8); 2,757 (2,2); 2,676 (0,8); 2,672 (1,1); 2,668 (0,8); 2,512 (73,1); 2,507 (138,1); 2,503 (175,3); 2,499 (129,8); 2,334 (0,7); 2,330 (1,0); 2,325 (0,7); 1,002 (1,0); 0,987 (1,0); 0,833 (0,4); 0,815 (0,3); 0,603 (0,4); 0,584 (1,7); 0,574 (2,2); 0,565 (0,8); 0,542 (0,9); 0,531 (1,7); 0,513 (1,5); 0,000 (1,8) |
| Beispiel I-T3-101: ¹H-NMR (400,0 MHz, d₆-DMSO): δ= 8,828 (0,4); 8,823 (0,4); 8,815 (2,1); 8,809 (2,2); 8,690 (3,8); 8,442 (0,7); 8,435 (3,3); 8,293 (2,2); 8,287 (2,1); 8,190 (0,4); 8,184 (0,3); |
| 7,603 (4,3); 3,904 (8,8); 3,339 (233,4); 3,175 (0,6); 3,162 (0,6); 3,027 (9,9); 2,795 (0,4); 2,787 (0,5); 2,778 (0,8); 2,768 (0,6); 2,756 (1,8); 2,676 (0,6); 2,672 (0,8); 2,667 (0,6); 2,512 (51,7); 2,507 (99,0); 2,503 (126,8); 2,498 (93,4); 2,494 (47,1); 2,334 (0,6); 2,330 (0,8); 2,325 (0,6); 2,130 (16,0); 2,122 (3,6); 1,002 (0,4); 0,987 (0,4); 0,583 (1,3); 0,574 (1,7); 0,565 (0,6); 0,543 (0,7); 0,531 (1,3); 0,514 (1,1); 0,000 (0,9) |
| Beispiel I-T3-102: ¹H-NMR (400,0 MHz, d₆-DMSO): δ= 8,908 (0,7); 8,879 (2,4); 8,874 (2,4); 8,833 (4,2); 8,820 (1,2); 8,538 (3,8); 8,496 (1,0); 8,431 (0,7); 8,427 (0,7); 8,288 (2,4); 8,283 (2,4); 8,111 (9,5); 3,904 (16,0); 3,591 (0,4); 3,341 (514,4); 3,175 (1,0); 3,162 (1,0); 3,136 (2,9); 2,914 (11,4); 2,676 (1,1); 2,672 (1,5); 2,668 (1,1); 2,507 (189,0); 2,503 (242,9); 2,499 (186,3); 2,334 (1,1); 2,330 (1,5); 2,326 (1,1); 1,713 (2,7); 1,489 (2,0); 0,000 (2,0) |
| Beispiel I-T3-103: ¹H-NMR (400,0 MHz, d₆-DMSO): δ= 8,907 (0,4); 8,878 (1,3); 8,872 (1,3); 8,702 (2,2); 8,685 (0,6); 8,443 (2,1); 8,406 (0,9); 8,270 (1,3); 8,264 (1,3); 7,604 (4,3); 3,904 (12,6); 3,395 (0,4); 3,338 (210,6); 3,270 (0,4); 3,256 (0,3); 3,175 (0,8); 3,162 (0,9); 3,133 (1,6); 2,915 (6,3); 2,676 (0,6); 2,672 (0,8); 2,668 (0,6); 2,525 (2,1); 2,507 (102,9); 2,503 (131,5); 2,498 (97,6); 2,334 (0,7); 2,330 (0,9); 2,325 (0,6); 2,122 (16,0); 1,718 (1,3); 1,713 (1,4); 1,484 (1,0); 1,002 (0,8); 0,987 (0,8); 0,000 (1,9) |
| Beispiel I-T3-104: ¹H-NMR (400,0 MHz, d₆-DMSO): δ= 8,865 (9,2); 8,857 (0,5); 8,845 (6,3); 8,839 (6,4); 8,694 (3,1); 8,683 (3,1); 8,470 (0,5); 8,459 (9,4); 8,229 (6,5); 8,223 (6,4); 7,751 (4,6); 7,728 (0,3); 7,710 (11,3); 4,457 (0,4); 4,403 (0,4); 4,392 (0,4); 4,121 (0,3); 4,108 (1,0); 4,095 (1,0); 4,082 (0,4); 3,904 (15,4); 3,395 (0,4); 3,334 (355,6); 3,243 (0,4); 3,175 (4,8); 3,161 (4,8); 2,883 (0,3); 2,873 (0,9); 2,864 (1,2); 2,855 (1,9); 2,845 (1,9); 2,836 (1,2); 2,827 (0,9); 2,816 (0,5); 2,807 (1,7); 2,788 (5,3); 2,769 (5,4); 2,751 (1,8); 2,680 (0,6); 2,676 (1,1); 2,671 (1,4); 2,667 (1,1); 2,542 (1,0); 2,525 (4,5); 2,511 (90,6); 2,507 (178,4); 2,502 (231,0); 2,498 (168,2); 2,494 (82,9); 2,334 (1,0); 2,329 (1,4); 2,325 (1,0); 1,056 (7,2); 1,038 (16,0); 1,019 (7,1); 1,002 (1,6); 0,987 (1,5); 0,759 (1,2); 0,746 (3,5); 0,741 (4,8); 0,729 (4,5); 0,723 (3,7); 0,711 (1,5); 0,568 (1,5); 0,557 (4,5); 0,551 (4,3); 0,547 (4,0); 0,542 (3,9); 0,529 (1,2); 0,000 (3,5) |
| Beispiel I-T3-105: ¹H-NMR (400,0 MHz, d₆-DMSO): δ= 9,606 (5,9); 8,899 (6,1); 8,893 (6,2); 8,882 (8,7); 8,466 (9,0); 8,308 (6,0); 8,302 (5,8); 7,753 (4,6); 7,730 (0,3); 7,712 (11,2); 3,904 (12,3); 3,332 (328,2); 3,175 (1,4); 3,161 (1,4); 3,047 (0,5); 2,866 (0,5); 2,807 (1,7); 2,789 (5,3); 2,770 (5,4); 2,751 (1,8); 2,676 (1,1); 2,671 (1,5); 2,667 (1,1); 2,524 (4,8); 2,511 (97,8); 2,507 (190,1); 2,502 (244,3); 2,498 (178,1); 2,493 (87,7); 2,333 (1,1); 2,329 (1,5); 2,324 (1,1); 1,650 (2,1); 1,636 (5,3); 1,629 (5,6); 1,616 (2,3); 1,295 (2,5); 1,281 (5,3); 1,275 (5,7); 1,260 (2,1); 1,055 (7,3); 1,037 (16,0); 1,018 (7,1); 0,000 (3,2) |
| Beispiel I-T3-106: ¹H-NMR (400,0 MHz, d₆-DMSO): δ= 8,824 (4,0); 8,818 (4,1); 8,781 (4,7); 8,694 (1,9); 8,683 (1,9); 8,515 (6,0); 8,223 (2,4); 8,196 (4,1); 8,190 (4,0); 7,937 (2,5); 4,456 (0,3); 4,402 (0,4); 4,391 (0,4); 4,107 (0,7); 4,094 (0,8); 3,904 (16,0); 3,332 (223,1); 3,243 (0,4); 3,174 (4,3); 3,161 (4,4); 2,868 (0,5); 2,859 (0,8); 2,850 (1,2); 2,840 (1,2); 2,831 (0,8); 2,822 (0,6); 2,676 (0,9); 2,671 (1,2); 2,667 (0,9); 2,541 (0,7); 2,525 (3,7); 2,511 (78,4); 2,507 (154,9); 2,502 (201,4); 2,498 (148,3); 2,493 (74,4); 2,334 (0,9); 2,329 (1,2); 2,325 (0,9); 2,147 (13,7); 1,002 (1,6); 0,987 (1,6); 0,755 (0,8); 0,742 (2,2); 0,737 (3,1); 0,725 (2,9); 0,719 (2,4); 0,708 (1,0); 0,563 (1,0); 0,552 (2,9); 0,546 (2,7); 0,542 (2,6); 0,536 (2,5); 0,524 (0,8); 0,000 (2,7) |
| Beispiel I-T3-107: ¹H-NMR (400,0 MHz, d₆-DMSO): δ= 8,833 (0,5); 8,826 (0,6); 8,819 (2,8); 8,813 (2,8); 8,770 (3,5); 8,503 (0,9); 8,495 (4,3); 8,312 (2,8); 8,306 (2,6); 8,223 (2,0); 8,207 (0,5); 8,201 (0,4); 7,939 (2,1); 3,904 (16,0); 3,332 (177,8); 3,175 (1,5); 3,162 (1,6); 3,027 (12,7); 2,794 (0,5); 2,787 (0,6); 2,778 (1,0); 2,767 (0,8); 2,760 (2,8); 2,751 (0,4); 2,676 (0,8); 2,672 (1,0); 2,667 (0,7); 2,525 (3,3); 2,511 (67,3); 2,507 (128,5); 2,503 (163,4); 2,498 (118,9); 2,494 (59,0); 2,334 (0,7); 2,329 (1,0); 2,325 (0,7); 2,158 (9,9); 1,002 (1,1); 0,987 (1,1); 0,830 (0,3); 0,813 (0,3); 0,606 (0,4); 0,586 (1,6); 0,577 (2,2); 0,568 (0,8); 0,545 (1,0); 0,534 (1,7); 0,516 (1,5); 0,000 (2,1) |
| Beispiel I-T3-108: ¹H-NMR (400,0 MHz, d₆-DMSO): δ= 9,604 (5,0); 8,878 (4,1); 8,872 (4,2); 8,793 (5,7); 8,519 (7,0); 8,277 (4,3); 8,271 (4,2); 8,225 (3,2); 7,938 (3,2); 4,458 (0,3); 4,404 (0,4); 4,393 (0,4); 4,123 (0,4); 4,110 (1,1); 4,097 (1,1); 4,083 (0,4); 3,904 (16,0); 3,433 (0,4); 3,337 (510,1); 3,270 (0,6); 3,256 (0,4); 3,242 (0,4); 3,175 (4,2); 3,162 (4,3); 3,043 (0,4); 2,872 (0,4); 2,672 (1,4); 2,506 (184,9); 2,503 (232,7); 2,499 (181,4); 2,329 (1,4); 2,148 (15,6); 1,643 (1,6); 1,629 (4,1); 1,622 (4,4); 1,609 (1,8); 1,293 (1,8); 1,280 (4,1); 1,273 (4,3); 1,259 (1,6); 1,002 (1,2); 0,987 (1,2); 0,000 (0,9) |
| Beispiel I-T3-109: ¹H-NMR (400,0 MHz, d₆-DMSO): δ= 8,821 (4,5); 8,815 (4,5); 8,727 (6,7); 8,683 (2,3); 8,672 (2,3); 8,456 (6,9); 8,191 (4,6); 8,185 (4,4); 7,611 (3,0); 7,577 (3,0); 4,112 (0,4); 4,099 (0,5); 3,904 (16,0); 3,433 (0,3); 3,422 (0,4); 3,341 (478,8); 3,283 (0,5); 3,272 (0,4); 3,269 (0,4); 3,257 (0,4); 3,243 (0,3); 3,175 (2,2); 3,162 (2,3); 2,868 (0,6); 2,858 (0,9); 2,850 (1,4); 2,840 (1,4); 2,831 (0,9); 2,821 (0,7); 2,676 (0,9); 2,672 (1,2); 2,667 (0,9); 2,542 (0,7); 2,525 (3,9); 2,511 (83,9); 2,507 (159,5); 2,503 (202,9); 2,498 (149,6); 2,443 (1,3); 2,424 (3,5); 2,405 (3,5); 2,387 (1,2); 2,334 (0,9); 2,330 (1,2); 2,325 (0,9); 2,096 (16,0); 1,169 (1,4); 1,035 (5,1); 1,016 (10,9); 1,002 (2,2); 0,997 (5,0); 0,987 (1,4); 0,755 (0,9); 0,742 (2,7); 0,737 (3,5); 0,725 (3,4); 0,719 (2,8); 0,708 (1,1); 0,563 (1,1); 0,553 (3,4); 0,547 (3,3); 0,543 (3,1); 0,537 (3,0); 0,525 (0,9); 0,000 (2,0) |
| Beispiel I-T3-110: ¹H-NMR (400,0 MHz, d₆-DMSO): δ= 9,594 (4,6); 8,875 (3,8); 8,869 (3,9); 8,742 (6,3); 8,462 (6,5); 8,272 (3,9); 8,266 (3,9); 7,613 (3,3); 7,578 (3,3); 4,108 (0,5); 4,095 (0,5); 3,904 (12,4); 3,405 (0,3); 3,334 (307,0); 3,269 (0,5); 3,256 (0,4); 3,242 (0,4); 3,175 (2,2); 3,161 (2,3); 3,043 (0,4); 2,871 (0,4); 2,671 (1,3); 2,502 (210,6); 2,445 (1,6); 2,426 (3,6); 2,407 (3,7); 2,388 (1,4); 2,329 (1,3); 2,097 (16,0); 1,645 (1,5); 1,630 (4,1); 1,624 (4,4); 1,610 (1,8); 1,291 (1,8); 1,277 (4,2); 1,271 (4,6); 1,256 (1,7); 1,235 (0,5); 1,169 (1,0); 1,036 (4,8); 1,018 (10,0); 0,999 (5,0); 0,987 (1,2); 0,000 (2,4) |
| Beispiel I-T3-111: ¹H-NMR (400,0 MHz, d₆-DMSO): δ= 8,917 (0,5); 8,913 (0,5); 8,882 (1,9); 8,876 (1,9); 8,782 (2,6); 8,753 (0,7); 8,503 (3,1); 8,461 (0,8); 8,419 (0,5); 8,414 (0,5); 8,284 (1,9); 8,279 (1,8); 8,223 (2,2); 7,938 (2,3); 3,904 (16,0); 3,334 (271,2); 3,175 (0,9); 3,162 (1,0); 3,131 (2,1); 2,919 (9,2); 2,676 (0,8); 2,672 (1,0); 2,667 (0,8); 2,525 (3,1); 2,511 (66,1); 2,507 (129,1); 2,503 (166,9); 2,498 (121,9); 2,494 (60,4); 2,334 (0,8); 2,329 (1,0); 2,325 (0,8); 2,149 (11,9); 1,718 (1,7); 1,712 (1,9); 1,487 (1,3); 0,000 (2,6) |
| Beispiel I-T3-112: ¹H-NMR (400,0 MHz, d₆-DMSO): δ= 8,843 (5,5); 8,825 (4,0); 8,819 (4,0); 8,701 (2,1); 8,690 (2,0); 8,561 (6,3); 8,513 (3,0); 8,198 (3,9); 8,192 (3,8); 8,094 (2,9); 4,109 (0,4); 4,096 (0,5); 3,904 (16,0); 3,333 (218,4); 3,267 (0,4); 3,174 (2,5); 3,162 (2,6); 2,870 (0,6); 2,860 (0,9); 2,851 (1,3); 2,841 (1,3); 2,833 (0,9); 2,823 (0,6); 2,676 (0,9); 2,671 (1,2); 2,667 (0,9); 2,524 (3,8); 2,507 (149,9); 2,502 (194,0); 2,498 (144,0); 2,333 (0,9); 2,329 (1,2); 2,325 |
| (0,9); 1,002 (1,2); 0,987 (1,2); 0,756 (0,8); 0,743 (2,5); 0,738 (3,3); 0,726 (3,1); 0,720 (2,6); 0,709 (1,0); 0,564 (1,0); 0,553 (3,2); 0,547 (3,1); 0,544 (2,9); 0,538 (2,8); 0,526 (0,8); 0,000 (2,3) |
| Beispiel I-T3-113: ¹H-NMR (400,0 MHz, d₆-DMSO): δ= 8,831 (3,5); 8,818 (2,5); 8,812 (2,5); 8,547 (0,8); 8,540 (3,7); 8,512 (1,9); 8,325 (2,4); 8,319 (2,4); 8,213 (0,4); 8,207 (0,4); 8,096 (1,8); 3,904 (16,0); 3,381 (0,4); 3,332 (195,5); 3,175 (1,3); 3,161 (1,4); 3,028 (11,1); 2,794 (0,5); 2,787 (0,5); 2,777 (0,9); 2,767 (0,7); 2,760 (2,4); 2,676 (0,7); 2,672 (0,9); 2,667 (0,7); 2,525 (2,8); 2,511 (58,6); 2,507 (114,3); 2,502 (147,1); 2,498 (107,3); 2,494 (52,8); 2,334 (0,6); 2,329 (0,9); 2,325 (0,6); 1,002 (1,0); 0,987 (0,9); 0,585 (1,4); 0,577 (1,9); 0,568 (0,7); 0,545 (0,8); 0,534 (1,4); 0,516 (1,3); 0,000 (2,0) |
| Beispiel I-T3-114: ¹H-NMR (400,0 MHz, d₆-DMSO): δ= 9,607 (2,4); 8,880 (2,4); 8,874 (2,4); 8,854 (3,1); 8,566 (3,7); 8,515 (1,6); 8,282 (2,4); 8,276 (2,4); 8,095 (1,6); 4,108 (0,4); 4,095 (0,4); 3,904 (16,0); 3,334 (175,5); 3,175 (2,1); 3,161 (2,1); 3,044 (0,5); 2,872 (0,5); 2,676 (0,6); 2,672 (0,8); 2,667 (0,6); 2,541 (0,5); 2,525 (2,4); 2,511 (50,8); 2,507 (99,8); 2,502 (129,1); 2,498 (94,9); 2,494 (47,4); 2,334 (0,6); 2,329 (0,7); 2,325 (0,6); 1,643 (0,8); 1,629 (2,0); 1,622 (2,2); 1,608 (0,9); 1,298 (1,0); 1,284 (2,0); 1,278 (2,2); 1,263 (0,8); 1,002 (0,9); 0,987 (0,8); 0,000 (1,0) |
| Beispiel I-T3-115: ¹H-NMR (400,0 MHz, d₆-DMSO): δ= 8,898 (1,1); 8,630 (1,1); 8,376 (0,7); 7,972 (0,4); 7,595 (4,0); 3,903 (4,3); 3,328 (177,7); 2,876 (1,2); 2,675 (0,9); 2,671 (1,2); 2,667 (0,9); 2,541 (0,9); 2,506 (159,0); 2,502 (205,8); 2,498 (157,5); 2,385 (2,8); 2,333 (1,0); 2,329 (1,3); 2,324 (1,0); 2,122 (16,0); 1,686 (0,6); 1,487 (0,8); 0,000 (1,2) |
| Beispiel I-T3-116: ¹H-NMR (400,0 MHz, d₆-DMSO): δ= 8,911 (1,0); 8,880 (3,4); 8,874 (3,4); 8,842 (5,1); 8,810 (1,3); 8,548 (5,6); 8,512 (4,5); 8,423 (0,9); 8,292 (3,4); 8,286 (3,4); 8,093 (4,3); 3,904 (4,9); 3,327 (229,2); 3,133 (3,9); 2,918 (16,0); 2,675 (1,2); 2,671 (1,6); 2,667 (1,3); 2,541 (1,5); 2,506 (202,4); 2,502 (261,5); 2,498 (200,6); 2,333 (1,1); 2,329 (1,5); 2,325 (1,2); 1,711 (3,8); 1,488 (2,6); -0,001 (1,3) |
| Beispiel I-T3-117: ¹H-NMR (400,0 MHz, d₆-DMSO): δ= 8,837 (3,9); 8,832 (3,9); 8,696 (5,5); 8,528 (2,3); 8,517 (2,3); 8,440 (6,7); 8,213 (3,3); 7,955 (3,9); 7,949 (3,9); 7,929 (3,4); 3,904 (3,8); 3,328 (223,0); 2,875 (0,7); 2,865 (1,0); 2,857 (1,5); 2,847 (1,5); 2,838 (1,1); 2,828 (0,7); 2,671 (1,5); 2,502 (230,7); 2,329 (1,4); 2,145 (16,0); 0,738 (0,9); 0,720 (3,6); 0,708 (3,4); 0,702 (3,0); 0,691 (1,1); 0,566 (1,2); 0,555 (3,7); 0,549 (3,6); 0,540 (3,2); 0,527 (0,9); 0,000 (1,2) |
| Beispiel I-T3-118: ¹H-NMR (400,0 MHz, d₆-DMSO): δ= 8,825 (3,2); 8,820 (3,1); 8,700 (5,9); 8,438 (7,3); 8,213 (3,4); 8,037 (3,0); 8,031 (3,0); 7,930 (3,5); 7,908 (0,5); 3,904 (11,1); 3,330 (239,1); 3,022 (14,7); 2,779 (0,4); 2,764 (0,9); 2,753 (1,3); 2,743 (1,0); 2,737 (1,0); 2,723 (2,0); 2,676 (1,0); 2,671 (1,3); 2,667 (1,0); 2,542 (1,0); 2,524 (4,1); 2,511 (78,7); 2,507 (153,6); 2,502 (199,9); 2,498 (148,4); 2,392 (13,3); 2,364 (1,8); 2,333 (0,9); 2,329 (1,2); 2,325 (0,9); 2,154 (16,0); 0,817 (0,5); 0,802 (0,5); 0,755 (0,6); 0,484 (4,8); 0,466 (2,5); 0,000 (1,4) |
| Beispiel I-T3-119: ¹H-NMR (400,0 MHz, d₆-DMSO): δ= 9,408 (4,7); 8,892 (3,9); 8,886 (3,9); 8,716 (5,6); 8,452 (7,3); 8,217 (2,9); 8,041 (3,8); 8,035 (3,8); 7,932 (3,0); 3,904 (9,3); 3,330 (168,1); 3,175 (1,1); 3,162 (1,1); 2,676 (0,7); 2,671 (1,0); 2,667 (0,8); 2,541 (0,9); 2,511 (82,5); 2,507 (129,7); 2,502 (165,2); 2,498 (122,9); 2,333 (0,7); 2,329 (1,0); 2,325 (0,7); 2,147 (16,0); 1,612 (1,5); 1,598 (4,0); 1,591 (4,3); 1,578 (1,8); 1,312 (1,8); 1,299 (4,1); 1,292 (4,2); 1,278 (1,5); 0,000 (1,1) |
| Beispiel I-T3-120: ¹H-NMR (400,0 MHz, d₆-DMSO): δ= 8,826 (1,6); 8,821 (1,6); 8,621 (4,1); 8,375 (3,7); 8,025 (1,5); 8,020 (1,6); 7,593 (4,8); 3,903 (7,6); 3,331 (235,6); 3,022 (7,6); 2,756 (0,7); 2,743 (0,5); 2,720 (1,0); 2,671 (1,1); 2,541 (0,9); 2,507 (130,2); 2,502 (168,8); 2,498 (127,0); 2,386 (6,8); 2,359 (0,9); 2,329 (1,0); 2,129 (16,0); 0,482 (2,7); 0,465 (1,4); 0,000 (1,0) |
| Beispiel I-T3-121: ¹H-NMR (400,0 MHz, CD3CN): δ= 8,836 (16,0); 8,460 (13,4); 8,441 (12,5); 7,871 (1,5); 7,554 (14,8); 2,909 (0,5); 2,899 (1,4); 2,890 (2,1); 2,881 (3,1); 2,871 (3,2); 2,862 (2,1); 2,853 (1,6); 2,843 (0,5); 2,469 (0,5); 2,464 (0,6); 2,460 (0,5); 2,287 (0,5); 2,263 (0,4); 2,245 (0,8); 2,226 (0,6); 2,164 (134,4); 2,128 (74,2); 2,108 (1,2); 2,102 (0,8); 2,096 (0,5); 1,976 (0,8); 1,965 (34,9); 1,959 (10,4); 1,953 (53,7); 1,947 (97,8); 1,941 (132,8); 1,935 (92,8); 1,928 (48,6); 1,829 (0,7); 1,781 (0,4); 1,775 (0,6); 1,769 (0,8); 1,763 (0,5); 1,540 (0,4); 1,470 (0,3); 1,429 (0,3); 1,320 (1,0); 1,269 (9,8); 1,135 (0,4); 0,897 (0,4); 0,881 (1,1); 0,864 (0,5); 0,834 (1,8); 0,821 (4,8); 0,816 (6,9); 0,803 (6,8); 0,798 (5,3); 0,786 (2,4); 0,764 (0,4); 0,746 (0,4); 0,721 (0,4); 0,710 (0,4); 0,681 (2,4); 0,669 (6,3); 0,663 (6,5); 0,659 (5,7); 0,653 (5,3); 0,641 (1,6); 0,000 (1,3) |
| Beispiel I-T3-122: ¹H-NMR (400,0 MHz, CD3CN): δ= 8,795 (7,1); 8,792 (7,1); 8,712 (16,0); 8,398 (7,3); 8,393 (7,2); 8,249 (14,8); 8,230 (0,4); 8,035 (5,4); 8,031 (10,0); 8,027 (5,8); 7,832 (3,6); 7,830 (4,9); 7,826 (3,7); 7,821 (2,0); 7,814 (5,0); 7,810 (5,4); 7,716 (5,3); 7,700 (3,9); 7,697 (5,2); 7,585 (0,4); 7,519 (5,8); 7,500 (9,9); 7,480 (4,3); 7,146 (1,9); 4,086 (0,4); 4,068 (1,3); 4,050 (1,3); 4,033 (0,5); 2,907 (0,6); 2,897 (1,9); 2,888 (2,8); 2,879 (4,2); 2,869 (4,1); 2,861 (2,8); 2,851 (2,0); 2,842 (0,7); 2,468 (0,7); 2,463 (0,9); 2,459 (0,7); 2,266 (0,3); 2,250 (0,4); 2,144 (668,5); 2,120 (3,2); 2,114 (4,0); 2,108 (5,1); 2,101 (3,3); 2,095 (1,7); 2,016 (0,5); 2,014 (0,5); 1,972 (7,8); 1,964 (22,3); 1,958 (55,1); 1,952 (312,9); 1,946 (568,6); 1,940 (766,2); 1,934 (526,3); 1,928 (270,3); 1,787 (0,4); 1,781 (1,7); 1,775 (3,2); 1,769 (4,4); 1,762 (3,0); 1,756 (1,5); 1,437 (5,8); 1,356 (0,3); 1,338 (0,6); 1,319 (0,4); 1,285 (0,5); 1,270 (2,2); 1,222 (1,6); 1,204 (3,0); 1,186 (1,6); 1,089 (0,9); 0,881 (0,4); 0,793 (2,2); 0,781 (6,2); 0,775 (8,7); 0,763 (8,9); 0,757 (6,5); 0,746 (3,2); 0,724 (0,5); 0,707 (0,5); 0,687 (0,5); 0,677 (0,4); 0,647 (3,3); 0,637 (8,3); 0,630 (8,0); 0,626 (7,0); 0,620 (6,8); 0,608 (2,2); 0,008 (1,7); 0,000 (60,4); -0,009 (2,1) |
| Beispiel I-T3-123: ¹H-NMR (400,0 MHz, d₆-DMSO): δ= 8,894 (2,1); 8,708 (1,4); 8,439 (1,2); 8,213 (2,9); 7,984 (0,8); 7,930 (3,0); 3,904 (15,2); 3,331 (445,1); 3,174 (0,5); 3,161 (0,5); 3,121 (0,5); 2,877 (2,5); 2,675 (1,3); 2,671 (1,8); 2,667 (1,4); 2,542 (1,7); 2,506 (224,4); 2,502 (292,1); 2,498 (219,8); 2,389 (5,1); 2,333 (1,3); 2,329 (1,8); 2,325 (1,4); 2,148 (16,0); 1,687 (1,2); 1,492 (1,5); 1,416 (0,6); 1,249 (0,4); 1,235 (0,4); 0,000 (1,8) |
| Beispiel I-T3-124: ¹H-NMR (400,0 MHz, d₆-DMSO): δ= 8,836 (5,2); 8,831 (5,2); 8,756 (7,5); 8,544 (2,8); 8,534 (2,9); 8,502 (4,3); 8,488 (8,8); 8,087 (4,0); 7,952 (5,0); 7,946 (5,0); 4,467 (0,8); 4,454 (2,0); 4,440 (0,8); 4,113 (0,5); 4,100 (0,5); 3,904 (16,0); 3,507 (0,4); 3,482 (0,5); 3,468 (0,5); 3,456 (0,5); 3,395 (5,7); 3,388 (4,9); 3,381 (6,3); 3,340 (838,4); 3,174 (2,4); 3,161 (2,3); 2,886 (0,3); 2,876 (0,9); 2,867 (1,2); 2,858 (1,9); 2,848 (1,9); 2,840 (1,3); 2,830 (0,9); 2,820 (0,4); 2,676 (1,6); 2,672 (2,1); 2,667 (1,7); 2,507 (264,8); 2,503 (344,8); 2,498 (274,4); 2,334 (1,5); 2,329 (2,0); 2,325 (1,5); 1,273 (0,4); 1,258 (0,8); 1,242 (0,8); 0,873 (0,4); 0,739 (1,2); 0,726 (3,4); 0,721 (4,6); 0,709 (4,3); 0,703 (3,7); 0,692 (1,5); 0,567 (1,5); 0,556 |
| (4,6); 0,550 (4,3); 0,546 (4,1); 0,540 (3,8); 0,528 (1,1); 0,008 (2,2); 0,000 (60,2); -0,008 (2,5) |
| Beispiel I-T3-125: ¹H-NMR (400,1 MHz, d₆-DMSO): δ= 9,41 (0,0144); 8,89 (0,0123); 8,83 (0,0014); 8,77 (0,0184); 8,49 (0,0301); 8,23 (0,0012); 8,09 (0,0103); 8,03 (0,0119); 3,31 (1,0000); 2,54 (0,6709); 2,50 (0,2387); 1,59 (0,0138); 1,30 (0,0136); 0,15 (0,0007); 0,00 (0,1822); -0,16 (0,0004) |
| Beispiel I-T3-126: ¹H-NMR (400,0 MHz, d₆-DMSO): δ= 9,406 (0,9); 8,069 (2,0); 7,667 (0,4); 7,646 (1,1); 7,621 (0,6); 7,616 (0,6); 7,538 (0,8); 7,533 (0,7); 6,479 (0,9); 3,322 (20,1); 2,524 (0,4); 2,519 (0,7); 2,511 (11,8); 2,506 (24,6); 2,502 (33,4); 2,497 (24,8); 2,493 (12,4); 1,989 (0,8); 1,608 (0,4); 1,594 (0,8); 1,587 (0,9); 1,574 (0,4); 1,398 (16,0); 1,291 (0,4); 1,278 (0,7); 1,271 (0,8); 1,257 (0,3); 1,175 (0,5); 0,008 (0,6); 0,000 (18,6); -0,009 (0,7) |
| Beispiel I-T3-127: ¹H-NMR (400,0 MHz, d₆-DMSO): δ= 8,850 (5,1); 8,845 (5,4); 8,692 (9,2); 8,530 (3,1); 8,520 (3,2); 8,464 (9,2); 8,273 (16,0); 7,973 (5,1); 7,967 (5,3); 4,105 (0,5); 4,091 (0,5); 3,903 (5,0); 3,329 (87,9); 3,175 (1,9); 3,162 (1,8); 2,877 (0,8); 2,868 (1,2); 2,859 (1,9); 2,849 (1,9); 2,840 (1,3); 2,831 (0,9); 2,820 (0,3); 2,672 (0,6); 2,667 (0,5); 2,507 (79,2); 2,502 (111,9); 2,463 (0,7); 2,334 (0,5); 2,329 (0,6); 0,740 (1,1); 0,727 (3,5); 0,722 (4,8); 0,710 (4,4); 0,704 (4,0); 0,693 (1,5); 0,571 (1,5); 0,561 (4,6); 0,555 (4,7); 0,551 (4,6); 0,545 (4,2); 0,533 (1,1); 0,000 (0,4) |
| Beispiel I-T3-128: ¹H-NMR (400,0 MHz, d₆-DMSO): δ= 8,840 (3,6); 8,835 (3,5); 8,698 (8,9); 8,465 (8,7); 8,272 (15,4); 8,058 (3,3); 8,053 (3,4); 7,922 (0,5); 3,903 (5,1); 3,328 (66,8); 3,175 (1,0); 3,162 (1,0); 3,026 (16,0); 2,891 (0,3); 2,784 (0,4); 2,767 (0,9); 2,756 (1,4); 2,746 (1,0); 2,721 (1,9); 2,676 (0,5); 2,672 (0,6); 2,667 (0,5); 2,542 (0,7); 2,511 (37,1); 2,507 (71,3); 2,503 (93,3); 2,498 (71,7); 2,395 (14,5); 2,369 (1,9); 2,334 (0,4); 2,329 (0,6); 2,325 (0,4); 0,819 (0,5); 0,803 (0,5); 0,758 (0,6); 0,481 (5,4); 0,463 (2,8); 0,000 (0,4) |
| Beispiel I-T3-129: ¹H-NMR (400,0 MHz, d₆-DMSO): δ= 9,412 (6,2); 8,906 (4,7); 8,900 (5,0); 8,710 (9,0); 8,478 (8,5); 8,276 (16,0); 8,063 (4,7); 8,058 (4,9); 3,903 (5,3); 3,434 (0,4); 3,334 (71,6); 3,169 (3,4); 2,672 (0,8); 2,520 (27,6); 2,507 (94,6); 2,503 (122,2); 2,499 (100,6); 2,329 (0,7); 1,612 (1,9); 1,598 (5,2); 1,591 (5,7); 1,578 (2,3); 1,317 (2,3); 1,304 (5,3); 1,297 (5,6); 1,283 (1,9); 0,000 (0,4) |
| Beispiel I-T3-130: ¹H-NMR (400,0 MHz, d₆-DMSO): δ= 8,821 (3,6); 8,815 (3,5); 8,760 (6,9); 8,499 (4,1); 8,484 (8,5); 8,085 (3,9); 8,046 (3,3); 8,040 (3,3); 7,911 (0,5); 3,904 (10,2); 3,327 (87,7); 3,176 (0,7); 3,163 (0,7); 3,025 (16,0); 2,779 (0,4); 2,768 (0,8); 2,762 (0,9); 2,753 (1,5); 2,742 (1,1); 2,736 (1,1); 2,724 (2,2); 2,676 (0,5); 2,672 (0,7); 2,667 (0,5); 2,525 (2,4); 2,512 (41,2); 2,507 (81,2); 2,503 (106,8); 2,498 (80,2); 2,494 (41,1); 2,398 (14,3); 2,369 (1,8); 2,334 (0,5); 2,330 (0,7); 2,325 (0,5); 0,820 (0,5); 0,803 (0,5); 0,757 (0,6); 0,485 (4,9); 0,467 (2,7); 0,000 (0,5) |
| Beispiel I-T3-131: ¹H-NMR (400,0 MHz, d₆-DMSO): δ= 8,891 (3,3); 8,768 (2,3); 8,499 (5,4); 8,485 (2,0); 8,085 (4,9); 7,990 (1,3); 3,903 (16,0); 3,327 (119,4); 3,175 (0,8); 3,162 (0,9); 3,122 (0,7); 2,879 (3,8); 2,676 (0,7); 2,672 (1,0); 2,667 (0,8); 2,542 (0,7); 2,525 (3,3); 2,511 (61,8); 2,507 (121,8); 2,503 (160,9); 2,498 (122,6); 2,494 (64,2); 2,396 (8,3); 2,334 (0,8); 2,329 (1,0); 2,325 (0,8); 1,686 (1,8); 1,497 (2,2); 1,420 (0,8); 0,000 (0,7) |
| Beispiel I-T3-132: ¹H-NMR (400,1 MHz, d₆-DMSO): δ= 8,907 (3,3); 8,699 (3,2); 8,461 (1,9); 8,269 (16,0); 8,002 (1,1); 4,088 (0,5); 4,075 (0,5); 3,311 (245,5); 3,269 (0,3); 3,175 (2,1); 3,162 (2,1); 3,123 (0,6); 2,875 (3,1); 2,710 (0,5); 2,674 (0,4); 2,670 (0,5); 2,540 (120,2); 2,505 (48,9); 2,501 (64,0); 2,497 (45,6); 2,464 (0,3); 2,396 (8,6); 2,367 (0,8); 2,328 (0,5); 2,323 (0,4); 1,686 (1,6); 1,495 (2,0); 1,431 (0,8); 1,423 (0,8); 0,146 (0,4); 0,008 (3,2); 0,000 (89,8); - 0,008 (4,5); -0,150 (0,4) |
| Beispiel I-T3-133: ¹H-NMR (400,1 MHz, d₆-DMSO): δ= 8,822 (3,7); 8,817 (3,4); 8,750 (9,7); 8,470 (9,1); 8,094 (15,1); 8,040 (3,3); 8,035 (3,3); 7,903 (0,4); 3,311 (86,3); 3,287 (0,3); 3,026 (16,0); 2,781 (0,4); 2,765 (1,0); 2,754 (1,5); 2,743 (1,1); 2,738 (1,1); 2,721 (1,6); 2,711 (0,7); 2,555 (0,4); 2,554 (0,5); 2,553 (0,6); 2,552 (0,7); 2,550 (0,8); 2,549 (1,0); 2,540 (89,5); 2,529 (0,7); 2,528 (0,6); 2,527 (0,6); 2,525 (0,6); 2,524 (0,6); 2,523 (0,6); 2,522 (0,6); 2,510 (12,1); 2,505 (23,8); 2,501 (31,3); 2,497 (21,3); 2,492 (9,9); 2,395 (14,5); 2,371 (1,7); 0,819 (0,4); 0,804 (0,5); 0,755 (0,6); 0,482 (5,8); 0,464 (3,0); 0,013 (0,3); 0,011 (0,4); 0,008 (2,1); 0,007 (1,4); 0,000 (61,4); -0,006 (1,7); -0,009 (2,2); -0,013 (0,4); -0,014 (0,3) |
| Beispiel I-T3-134: ¹H-NMR (400,0 MHz, d₆-DMSO): δ= 8,823 (4,4); 8,427 (4,3); 8,403 (0,4); 8,263 (1,3); 8,242 (1,6); 8,112 (2,9); 8,071 (2,1); 8,050 (1,8); 7,818 (2,7); 7,813 (2,7); 7,760 (1,6); 7,755 (1,5); 7,747 (1,0); 7,739 (1,8); 7,734 (1,5); 7,724 (0,9); 7,560 (1,0); 7,553 (2,6); 7,540 (1,0); 7,532 (2,1); 3,327 (28,9); 3,321 (6,8); 3,015 (11,5); 2,766 (0,4); 2,756 (0,8); 2,748 (1,0); 2,740 (1,3); 2,730 (1,2); 2,716 (2,7); 2,676 (0,4); 2,673 (0,4); 2,507 (45,7); 2,503 (53,0); 2,499 (41,0); 2,330 (0,4); 2,076 (16,0); 2,068 (2,0); 1,170 (0,4); 0,820 (0,4); 0,812 (0,4); 0,804 (0,4); 0,757 (0,5); 0,748 (0,5); 0,559 (2,6); 0,551 (2,5); 0,503 (0,4); 0,472 (2,1); 0,455 (2,2); 0,000 (51,7); -0,009 (7,9) |
| Beispiel I-T3-135: ¹H-NMR (400,1 MHz, d₆-DMSO): δ= 8,547 (0,5); 8,505 (10,0); 8,454 (9,8); 8,448 (8,1); 8,442 (4,1); 8,281 (10,0); 8,068 (16,0); 7,686 (6,9); 7,680 (6,8); 3,568 (0,4); 3,410 (4,1); 3,394 (11,2); 3,378 (4,3); 3,309 (165,8); 3,286 (0,7); 2,832 (0,3); 2,822 (0,9); 2,812 (1,3); 2,804 (2,0); 2,794 (2,0); 2,785 (1,3); 2,776 (0,9); 2,765 (0,4); 2,710 (0,9); 2,674 (0,5); 2,669 (0,6); 2,665 (0,5); 2,560 (0,6); 2,540 (226,8); 2,523 (1,6); 2,509 (31,4); 2,505 (60,7); 2,500 (78,7); 2,496 (53,0); 2,492 (24,2); 2,366 (0,8); 2,332 (0,4); 2,327 (0,6); 2,323 (0,4); 1,887 (4,2); 1,871 (11,1); 1,854 (3,9); 1,235 (0,4); 0,704 (1,4); 0,691 (3,7); 0,686 (5,2); 0,674 (4,8); 0,668 (4,0); 0,657 (1,7); 0,547 (1,8); 0,536 (5,3); 0,530 (4,6); 0,526 (4,3); 0,520 (4,2); 0,508 (1,3); 0,146 (0,5); 0,008 (4,2); 0,000 (120,8); -0,008 (4,5); -0,150 (0,5) |
| Beispiel I-T3-136: ¹H-NMR (400,0 MHz, d₆-DMSO): δ= 8,510 (2,6); 8,502 (7,1); 8,497 (6,4); 8,441 (8,5); 8,259 (8,4); 8,129 (2,2); 8,117 (2,5); 8,106 (1,0); 8,088 (5,7); 8,080 (16,0); 4,467 (1,4); 4,453 (4,0); 4,439 (1,5); 4,111 (0,4); 4,099 (0,4); 3,904 (15,8); 3,804 (0,4); 3,483 (0,4); 3,470 (0,5); 3,455 (0,4); 3,395 (8,9); 3,388 (7,6); 3,381 (9,5); 3,338 (810,3); 3,174 (1,7); 3,161 (1,6); 2,953 (0,4); 2,932 (11,5); 2,920 (11,5); 2,847 (0,4); 2,837 (0,9); 2,828 (1,3); 2,819 (1,9); 2,810 (1,9); 2,801 (1,3); 2,792 (0,9); 2,783 (0,4); 2,671 (2,2); 2,616 (0,3); 2,506 (280,8); 2,502 (354,5); 2,498 (275,8); 2,329 (2,2); 1,234 (0,6); 0,873 (0,6); 0,854 (0,5); 0,742 (1,0); 0,724 (4,4); 0,711 (4,1); 0,706 (3,6); 0,694 (1,4); 0,587 (1,5); 0,576 (4,6); 0,570 (4,4); 0,561 (3,8); 0,548 (1,1); 0,000 (48,0) |
| Beispiel I-T3-137: ¹H-NMR (400,0 MHz, d₆-DMSO): δ= 8,543 (6,9); 8,537 (7,0); 8,455 (8,0); 8,364 (2,9); 8,356 (3,0); 8,268 (8,1); 8,118 (4,5); 8,113 (4,5); 8,083 (13,0); 4,467 (0,5); 4,453 (1,5); 4,439 (0,6); 4,112 (0,4); 4,099 (0,4); 3,904 (16,0); 3,483 (0,3); 3,469 (0,4); 3,433 (0,5); 3,407 (0,8); 3,395 (4,1); 3,388 (3,4); 3,381 (4,6); 3,338 (731,2); 3,174 (1,7); 3,161 (1,6); 2,955 (0,8); 2,852 (0,8); 2,844 (1,2); 2,835 (1,8); 2,826 (2,0); 2,818 (2,0); 2,809 (2,0); 2,800 (2,0); |
| 2,791 (1,8); 2,782 (1,2); 2,772 (0,8); 2,676 (1,4); 2,672 (1,9); 2,667 (1,5); 2,507 (235,5); 2,502 (302,0); 2,498 (229,6); 2,333 (1,4); 2,329 (1,8); 2,325 (1,4); 1,237 (0,4); 0,873 (0,4); 0,854 (0,3); 0,765 (1,1); 0,753 (3,5); 0,748 (4,4); 0,736 (5,1); 0,731 (4,0); 0,718 (5,1); 0,706 (3,9); 0,700 (3,5); 0,689 (1,4); 0,582 (1,4); 0,571 (4,3); 0,565 (4,0); 0,556 (3,5); 0,544 (1,0); 0,466 (1,3); 0,455 (4,0); 0,450 (4,1); 0,445 (4,0); 0,440 (3,9); 0,428 (1,1); 0,000 (39,5) |
| Beispiel I-T3-138: ¹H-NMR (400,0 MHz, d₆-DMSO): δ= 8,524 (1,8); 8,515 (1,9); 8,487 (3,8); 8,482 (3,9); 8,443 (6,4); 8,432 (1,1); 8,419 (1,9); 8,405 (1,0); 8,274 (0,4); 8,262 (6,3); 8,120 (4,0); 8,114 (3,6); 8,081 (10,3); 4,453 (0,7); 3,904 (16,0); 3,606 (1,0); 3,592 (3,0); 3,579 (3,7); 3,566 (1,8); 3,523 (3,7); 3,511 (4,9); 3,498 (1,9); 3,473 (0,5); 3,449 (0,3); 3,395 (2,5); 3,387 (2,3); 3,381 (2,9); 3,338 (556,7); 3,299 (28,1); 3,286 (1,6); 3,262 (0,8); 3,256 (0,7); 3,174 (0,9); 3,161 (0,9); 2,840 (0,6); 2,831 (0,9); 2,822 (1,3); 2,812 (1,4); 2,804 (0,9); 2,795 (0,6); 2,676 (1,4); 2,672 (2,0); 2,667 (1,4); 2,507 (219,1); 2,503 (282,2); 2,498 (210,9); 2,334 (1,2); 2,329 (1,7); 2,325 (1,3); 1,235 (0,5); 0,747 (0,8); 0,734 (2,3); 0,729 (3,2); 0,717 (2,9); 0,711 (2,6); 0,700 (1,0); 0,589 (1,1); 0,579 (3,3); 0,573 (3,0); 0,569 (3,0); 0,563 (2,7); 0,551 (0,9); 0,008 (1,7); 0,000 (45,2); -0,008 (1,8) |
| Beispiel I-T3-139: ¹H-NMR (400,0 MHz, d₆-DMSO): δ= 9,474 (2,4); 8,837 (1,9); 8,831 (2,1); 8,602 (3,2); 8,355 (3,4); 7,970 (2,0); 7,965 (2,2); 7,596 (4,3); 4,467 (0,4); 4,453 (1,0); 4,440 (0,4); 3,904 (6,3); 3,423 (0,3); 3,395 (2,3); 3,387 (2,3); 3,381 (2,8); 3,340 (340,7); 3,174 (0,4); 3,161 (0,4); 3,063 (0,6); 2,880 (0,6); 2,672 (0,9); 2,668 (0,7); 2,507 (107,5); 2,503 (142,8); 2,498 (119,4); 2,329 (1,2); 2,325 (1,2); 2,312 (0,8); 2,299 (0,6); 2,292 (0,5); 2,110 (16,0); 1,615 (0,8); 1,601 (2,0); 1,594 (2,3); 1,581 (1,0); 1,324 (0,9); 1,310 (2,1); 1,304 (2,3); 1,289 (0,8); 1,257 (0,4); 1,243 (0,4); 1,168 (0,4); 0,993 (2,1); 0,982 (3,3); 0,962 (1,9); 0,000 (10,4) |
| Beispiel I-T3-140: ¹H-NMR (400,0 MHz, d₆-DMSO): δ= 8,785 (2,0); 8,780 (1,9); 8,595 (1,3); 8,584 (4,5); 8,345 (3,4); 7,890 (2,0); 7,885 (1,9); 7,594 (4,2); 4,455 (0,3); 3,904 (3,2); 3,408 (0,5); 3,394 (1,3); 3,382 (1,7); 3,342 (322,7); 3,174 (0,4); 3,162 (0,4); 2,892 (0,5); 2,883 (0,7); 2,873 (0,7); 2,865 (0,5); 2,855 (0,4); 2,672 (0,7); 2,503 (110,1); 2,352 (0,4); 2,345 (0,5); 2,334 (1,3); 2,314 (0,5); 2,111 (16,0); 0,973 (1,9); 0,961 (1,8); 0,951 (1,9); 0,931 (1,6); 0,740 (0,4); 0,723 (1,7); 0,710 (1,7); 0,705 (1,4); 0,694 (0,6); 0,582 (0,6); 0,571 (1,8); 0,564 (1,8); 0,555 (1,5); 0,543 (0,4); 0,000 (8,3) |
| Beispiel I-T3-141: ¹H-NMR (400,0 MHz, d₆-DMSO): δ= 9,093 (2,2); 8,668 (2,1); 8,662 (2,2); 8,577 (3,2); 8,327 (3,3); 8,314 (2,3); 8,308 (2,2); 7,593 (3,8); 3,969 (10,4); 3,904 (3,5); 3,409 (0,5); 3,343 (332,3); 3,285 (0,3); 2,676 (0,5); 2,672 (0,7); 2,668 (0,6); 2,525 (2,4); 2,512 (45,6); 2,507 (89,1); 2,503 (116,0); 2,498 (87,1); 2,334 (0,5); 2,330 (0,7); 2,325 (0,5); 2,121 (16,0); 1,602 (0,8); 1,587 (2,0); 1,581 (2,2); 1,567 (0,9); 1,305 (1,0); 1,292 (2,1); 1,285 (2,2); 1,271 (0,8); 0,008 (0,4); 0,000 (11,6); -0,008 (0,5) |
| Beispiel I-T3-142: ¹H-NMR (400,0 MHz, d₆-DMSO): δ= 8,767 (2,0); 8,582 (3,2); 8,343 (3,2); 7,948 (2,0); 7,851 (0,3); 7,591 (5,1); 4,454 (0,8); 3,904 (4,5); 3,381 (3,9); 3,341 (346,8); 3,218 (0,4); 3,175 (0,5); 3,162 (0,4); 3,043 (7,9); 2,791 (1,0); 2,781 (0,9); 2,766 (1,5); 2,672 (1,0); 2,503 (159,1); 2,330 (1,0); 2,122 (16,0); 1,926 (0,6); 1,915 (0,9); 1,901 (0,7); 1,882 (0,4); 0,992 (1,5); 0,961 (2,3); 0,943 (2,0); 0,824 (0,4); 0,809 (0,4); 0,764 (0,5); 0,540 (2,1); 0,482 (1,7); 0,467 (1,6); 0,000 (14,1) |
| Beispiel I-T3-143: ¹H-NMR (400,0 MHz, d₆-DMSO): δ= 8,608 (2,1); 8,602 (2,1); 8,556 (3,2); 8,308 (3,3); 8,266 (1,0); 8,256 (1,0); 8,227 (2,2); 8,221 (2,1); 7,590 (3,9); 3,948 (10,3); 3,904 (3,5); 3,395 (0,9); 3,343 (295,8); 3,175 (0,3); 2,873 (0,3); 2,863 (0,5); 2,855 (0,7); 2,845 (0,7); 2,837 (0,5); 2,827 (0,3); 2,676 (0,5); 2,672 (0,7); 2,668 (0,5); 2,507 (88,5); 2,503 (112,5); 2,498 (84,8); 2,334 (0,5); 2,329 (0,7); 2,325 (0,5); 2,120 (16,0); 0,740 (0,4); 0,727 (1,3); 0,722 (1,8); 0,709 (1,6); 0,704 (1,4); 0,692 (0,6); 0,580 (0,6); 0,570 (1,8); 0,564 (1,7); 0,554 (1,4); 0,542 (0,4); 0,008 (0,7); 0,000 (14,6) |
| Beispiel I-T3-144: ¹H-NMR (400,0 MHz, d₆-DMSO): δ= 8,563 (2,0); 8,558 (2,0); 8,526 (3,6); 8,302 (3,3); 8,005 (1,9); 7,999 (2,0); 7,590 (4,6); 4,468 (0,3); 4,454 (0,8); 4,440 (0,3); 3,915 (9,8); 3,904 (7,4); 3,425 (0,4); 3,395 (2,6); 3,388 (2,3); 3,381 (3,0); 3,341 (345,9); 3,282 (0,4); 2,981 (8,7); 2,748 (0,5); 2,738 (0,8); 2,724 (0,6); 2,711 (0,3); 2,700 (0,9); 2,676 (0,7); 2,672 (0,9); 2,668 (0,8); 2,507 (111,1); 2,503 (144,6); 2,499 (113,9); 2,334 (0,6); 2,329 (0,8); 2,127 (16,0); 0,466 (3,1); 0,449 (1,7); 0,000 (11,6) |
| Beispiel I-T3-145: ¹H-NMR (400,0 MHz, d₆-DMSO): δ= 8,848 (0,6); 8,841 (0,8); 8,835 (4,1); 8,829 (4,2); 8,781 (6,9); 8,537 (7,1); 8,316 (0,4); 8,283 (13,4); 8,274 (4,6); 8,257 (0,6); 8,251 (0,5); 3,424 (0,3); 3,409 (0,3); 3,398 (0,4); 3,387 (0,3); 3,371 (0,4); 3,324 (185,3); 2,776 (0,4); 2,764 (0,8); 2,759 (0,9); 2,750 (1,6); 2,739 (1,0); 2,733 (0,9); 2,722 (0,5); 2,675 (1,1); 2,671 (1,5); 2,667 (1,1); 2,524 (4,5); 2,506 (173,0); 2,502 (225,3); 2,497 (167,2); 2,333 (1,1); 2,329 (1,5); 2,324 (1,1); 1,398 (16,0); 1,238 (4,1); 1,220 (8,5); 1,202 (3,9); 1,120 (0,5); 1,102 (1,0); 1,085 (0,5); 0,951 (0,4); 0,935 (0,5); 0,577 (2,8); 0,549 (2,5); 0,532 (2,1); 0,146 (0,9); 0,008 (7,2); 0,000 (188,0); -0,008 (8,4); -0,150 (0,9) |
| Beispiel I-T3-146: ¹H-NMR (400,0 MHz, d₆-DMSO): δ= 10,752 (3,5); 8,900 (3,6); 8,894 (3,7); 8,806 (5,3); 8,571 (5,2); 8,570 (5,2); 8,393 (3,7); 8,387 (3,6); 8,284 (8,3); 8,283 (8,3); 8,028 (4,8); 7,502 (4,8); 7,501 (5,0); 5,756 (5,9); 4,056 (0,5); 4,038 (1,6); 4,020 (1,6); 4,002 (0,5); 3,837 (16,0); 3,324 (33,1); 2,671 (0,4); 2,524 (1,0); 2,520 (1,4); 2,511 (20,1); 2,507 (41,3); 2,502 (54,9); 2,497 (40,1); 2,493 (19,7); 2,329 (0,4); 1,989 (6,9); 1,193 (1,9); 1,175 (3,8); 1,157 (1,9); 0,008 (1,9); 0,000 (58,8); -0,009 (2,2) |
| Beispiel I-T3-147: ¹H-NMR (400,0 MHz, d₆-DMSO): δ= 8,999 (3,5); 8,993 (3,6); 8,778 (6,5); 8,499 (6,5); 8,384 (3,5); 8,378 (3,5); 8,279 (10,7); 5,757 (1,1); 4,384 (1,6); 4,366 (5,0); 4,348 (5,1); 4,331 (1,7); 3,894 (0,5); 3,324 (128,6); 2,714 (16,0); 2,675 (0,9); 2,671 (1,3); 2,666 (1,0); 2,524 (3,3); 2,511 (69,0); 2,506 (138,7); 2,502 (184,5); 2,498 (139,2); 2,333 (0,8); 2,329 (1,1); 2,325 (0,9); 1,989 (0,7); 1,377 (5,4); 1,360 (11,3); 1,342 (5,4); 1,234 (0,5); 1,175 (0,4); 0,146 (0,9); 0,008 (6,9); 0,000 (189,8); -0,150 (0,9) |
| Beispiel I-T3-148: ¹H-NMR (400,0 MHz, CD3CN): δ= 8,344 (1,0); 8,180 (2,1); 8,179 (2,0); 8,138 (1,8); 8,094 (1,0); 7,688 (1,2); 7,683 (1,6); 7,655 (0,9); 7,650 (0,6); 7,634 (1,0); 7,629 (0,8); 7,474 (1,5); 7,454 (1,2); 6,895 (0,4); 6,892 (0,4); 2,860 (0,4); 2,851 (0,6); 2,842 (0,6); 2,833 (0,4); 2,132 (17,4); 2,107 (0,3); 1,964 (1,5); 1,958 (3,8); 1,952 (20,1); 1,946 (36,2); 1,940 (48,4); 1,933 (33,3); 1,927 (17,3); 1,437 (16,0); 0,781 (1,0); 0,776 (1,2); 0,763 (1,3); 0,758 (0,9); 0,746 (0,4); 0,611 (0,4); 0,601 (1,1); 0,594 (1,2); 0,590 (1,0); 0,584 (1,0); 0,000 (2,5) |
| Beispiel I-T3-149: ¹H-NMR (400,0 MHz, d₆-DMSO): δ= 18,228 (0,3); 18,070 (0,4); 11,873 (0,3); 9,464 (0,4); 9,435 (13,6); 9,407 (0,4); 9,265 (9,2); 9,168 (16,0); 8,869 (9,9); 8,544 (15,8); 8,514 (0,4); 8,316 (1,1); 8,148 (0,4); 8,012 (0,4); 7,945 (12,0); 7,931 (6,4); 7,910 (6,2); 7,905 (5,4); 7,850 (0,4); 7,843 (0,4); 7,839 (0,4); 7,702 |
| (0,4); 7,639 (0,4); 7,582 (9,8); 7,561 (9,0); 7,543 (0,4); 3,637 (0,4); 3,591 (0,4); 3,572 (0,4); 3,547 (0,4); 3,535 (0,6); 3,512 (0,5); 3,469 (0,6); 3,434 (0,9); 3,392 (2,8); 3,344 (1316,4); 3,339 (765,9); 3,331 (992,7); 3,218 (1,0); 3,211 (0,9); 3,178 (0,5); 3,121 (0,3); 3,058 (0,3); 2,731 (0,4); 2,671 (5,7); 2,638 (0,4); 2,584 (0,4); 2,506 (689,3); 2,502 (847,8); 2,417 (1,1); 2,381 (0,8); 2,333 (4,7); 2,328 (5,7); 2,288 (0,4); 2,283 (0,4); 1,658 (0,3); 1,620 (4,0); 1,606 (11,5); 1,599 (12,3); 1,586 (5,4); 1,546 (0,6); 1,489 (0,4); 1,370 (0,3); 1,350 (0,7); 1,310 (5,0); 1,296 (11,9); 1,290 (12,3); 1,275 (4,4); 1,237 (0,5); 0,146 (2,0); 0,000 (404,8); -0,150 (2,2); -3,146 (0,3) |
| Beispiel I-T3-150: ¹H-NMR (400,0 MHz, d₆-DMSO): δ= 9,265 (5,5); 9,179 (0,4); 9,144 (12,1); 9,086 (0,6); 8,868 (5,9); 8,863 (6,0); 8,554 (0,6); 8,536 (16,0); 8,525 (4,5); 8,504 (0,7); 8,316 (0,8); 8,292 (0,4); 7,872 (14,1); 7,866 (7,0); 7,855 (6,0); 7,850 (3,3); 7,569 (0,3); 7,534 (7,4); 7,527 (1,7); 7,518 (1,6); 7,511 (6,7); 3,568 (0,6); 3,468 (0,4); 3,455 (0,4); 3,444 (0,5); 3,341 (576,1); 3,339 (590,5); 3,331 (552,2); 2,875 (0,5); 2,864 (1,2); 2,855 (1,6); 2,846 (2,6); 2,836 (2,6); 2,828 (1,7); 2,818 (1,3); 2,807 (0,5); 2,676 (2,6); 2,672 (3,7); 2,667 (2,8); 2,662 (1,4); 2,580 (0,4); 2,525 (9,0); 2,520 (13,1); 2,511 (197,7); 2,507 (411,4); 2,502 (559,2); 2,498 (423,2); 2,493 (212,6); 2,458 (0,5); 2,334 (2,7); 2,329 (3,7); 2,325 (2,7); 0,736 (1,7); 0,724 (4,5); 0,718 (6,6); 0,706 (6,1); 0,700 (5,1); 0,689 (2,3); 0,650 (0,3); 0,608 (0,4); 0,578 (2,2); 0,568 (6,4); 0,562 (5,7); 0,558 (5,5); 0,552 (5,2); 0,540 (1,7); 0,146 (1,7); 0,030 (0,4); 0,024 (0,4); 0,017 (0,6); 0,008 (12,6); 0,000 (401,4); -0,009 (15,0); -0,150 (1,7) |
| Beispiel I-T3-151: ¹H-NMR (400,0 MHz, d₆-DMSO): δ= 9,444 (12,0); 9,048 (16,0); 8,890 (7,0); 8,886 (7,3); 8,610 (8,1); 8,605 (7,8); 8,506 (15,9); 8,495 (0,3); 8,317 (4,4); 7,899 (6,5); 7,894 (13,4); 7,885 (2,6); 7,870 (6,7); 7,865 (4,8); 7,579 (9,1); 7,559 (8,2); 3,410 (0,4); 3,383 (0,7); 3,364 (1,2); 3,327 (1576,2); 3,293 (1,2); 2,694 (0,5); 2,676 (8,0); 2,671 (11,2); 2,667 (8,3); 2,643 (0,4); 2,630 (0,4); 2,623 (0,5); 2,599 (0,7); 2,524 (28,8); 2,520 (43,4); 2,511 (594,8); 2,507 (1217,9); 2,502 (1623,1); 2,498 (1195,4); 2,493 (589,7); 2,419 (0,6); 2,338 (3,7); 2,333 (7,9); 2,329 (11,1); 2,324 (8,1); 2,320 (4,0); 1,620 (3,9); 1,606 (9,5); 1,599 (10,3); 1,586 (4,4); 1,546 (0,4); 1,342 (0,4); 1,303 (4,6); 1,289 (9,5); 1,282 (10,2); 1,268 (3,8); 1,234 (0,6); 1,148 (0,9); 0,146 (8,7); 0,049 (0,4); 0,039 (0,7); 0,008 (63,6); 0,000 (1893,8); -0,009 (67,7); -0,035 (1,3); -0,045 (0,8); -0,088 (0,3); -0,150 (8,7) |
| Beispiel I-T3-152: ¹H-NMR (400,0 MHz, d₆-DMSO): δ= 9,046 (0,6); 9,022 (16,0); 8,893 (8,5); 8,608 (9,0); 8,604 (8,5); 8,543 (6,0); 8,532 (6,0); 8,515 (0,9); 8,496 (15,9); 8,453 (0,3); 8,317 (2,0); 7,901 (0,4); 7,896 (0,4); 7,828 (8,6); 7,823 (11,4); 7,818 (11,0); 7,812 (8,2); 7,682 (0,3); 7,532 (8,5); 7,521 (2,4); 7,510 (7,5); 3,508 (0,4); 3,327 (1034,9); 3,230 (0,5); 3,210 (0,4); 2,874 (0,7); 2,864 (1,6); 2,854 (2,4); 2,846 (3,5); 2,836 (3,5); 2,827 (2,5); 2,817 (1,7); 2,807 (0,8); 2,671 (8,3); 2,622 (0,7); 2,608 (0,8); 2,506 (980,0); 2,502 (1179,0); 2,329 (8,1); 2,297 (0,4); 2,281 (0,3); 1,236 (0,7); 1,149 (0,6); 0,735 (2,2); 0,717 (8,7); 0,705 (8,5); 0,699 (7,0); 0,688 (2,9); 0,666 (0,5); 0,648 (0,4); 0,615 (0,3); 0,603 (0,4); 0,574 (3,0); 0,563 (9,1); 0,556 (9,0); 0,548 (7,5); 0,535 (2,1); 0,525 (0,5); 0,488 (0,3); 0,146 (5,2); 0,000 (1050,7); -0,150 (5,5) |
| Beispiel I-T3-153: ¹H-NMR (400,0 MHz, d₆-DMSO): δ= 9,441 (7,1); 9,380 (9,1); 8,960 (4,8); 8,954 (5,0); 8,641 (9,2); 8,581 (3,9); 8,577 (3,9); 8,317 (0,9); 8,166 (1,1); 8,002 (5,1); 7,997 (6,5); 7,969 (3,3); 7,963 (2,5); 7,948 (3,5); 7,942 (3,0); 7,589 (6,2); 7,568 (5,7); 3,430 (0,6); 3,411 (1,9); 3,393 (2,3); 3,378 (2,6); 3,359 (2,8); 3,328 (240,7); 2,950 (0,6); 2,932 (2,1); 2,913 (2,4); 2,898 (2,1); 2,880 (1,9); 2,862 (0,5); 2,676 (1,9); 2,671 (2,7); 2,667 (2,0); 2,542 (1,0); 2,525 (6,6); 2,520 (10,2); 2,511 (145,4); 2,507 (298,6); 2,502 (396,9); 2,498 (292,6); 2,493 (144,6); 2,334 (1,9); 2,329 (2,6); 2,325 (1,9); 2,320 (1,0); 2,075 (0,6); 1,908 (0,5); 1,627 (2,3); 1,613 (5,6); 1,606 (6,0); 1,593 (2,7); 1,314 (2,6); 1,301 (5,6); 1,294 (5,9); 1,279 (2,3); 1,106 (7,4); 1,088 (16,0); 1,069 (7,2); 0,146 (2,3); 0,008 (17,1); 0,000 (509,3); -0,009 (19,8); -0,031 (0,4); -0,034 (0,4); -0,150 (2,3) |
| Beispiel I-T3-154: ¹H-NMR (400,0 MHz, d₆-DMSO): δ= 9,448 (6,4); 9,231 (4,3); 9,226 (4,3); 9,186 (8,2); 8,634 (4,2); 8,629 (4,2); 8,596 (8,2); 8,317 (3,6); 7,954 (4,3); 7,949 (6,0); 7,934 (3,1); 7,929 (2,0); 7,913 (3,2); 7,908 (2,5); 7,595 (5,2); 7,574 (4,7); 4,152 (1,7); 4,133 (5,6); 4,115 (5,6); 4,096 (1,8); 3,459 (0,4); 3,445 (0,3); 3,436 (0,3); 3,328 (1346,5); 2,694 (0,4); 2,676 (6,7); 2,671 (9,0); 2,667 (6,9); 2,629 (0,5); 2,620 (0,5); 2,524 (24,4); 2,506 (1009,7); 2,502 (1305,6); 2,498 (988,2); 2,405 (0,6); 2,389 (0,6); 2,333 (6,4); 2,329 (8,7); 2,325 (6,5); 1,623 (2,1); 1,608 (5,2); 1,602 (5,6); 1,589 (2,4); 1,575 (0,5); 1,326 (6,4); 1,308 (16,0); 1,289 (11,7); 1,274 (2,1); 1,258 (0,6); 1,247 (0,5); 1,236 (0,7); 1,158 (0,5); 1,147 (0,4); 1,068 (0,8); 0,146 (6,9); 0,008 (62,7); 0,000 (1428,7); -0,059 (0,5); -0,080 (0,4); -0,101 (0,4); -0,150 (7,0) |
| Beispiel I-T3-155: ¹H-NMR (400,0 MHz, CD3CN): δ= 8,345 (0,9); 8,192 (1,8); 8,153 (1,5); 8,094 (0,9); 7,738 (1,0); 7,732 (1,3); 7,706 (0,6); 7,700 (0,5); 7,685 (0,7); 7,680 (0,6); 7,563 (0,4); 7,507 (1,1); 7,486 (0,9); 2,144 (6,2); 2,114 (0,5); 2,108 (0,4); 1,972 (1,1); 1,964 (1,1); 1,958 (2,8); 1,952 (14,4); 1,946 (26,2); 1,940 (35,3); 1,934 (25,1); 1,928 (13,6); 1,596 (0,5); 1,582 (1,3); 1,575 (1,3); 1,561 (0,7); 1,437 (16,0); 1,362 (0,6); 1,349 (1,3); 1,342 (1,4); 1,327 (0,5); 1,204 (0,5); 0,146 (0,8); 0,008 (7,0); 0,000 (147,1); -0,008 (10,2); -0,150 (0,7) |
| Beispiel I-T3-156: ¹H-NMR (400,0 MHz, d₆-DMSO): δ= 9,077 (8,4); 9,076 (8,4); 8,593 (4,1); 8,589 (4,2); 8,540 (3,1); 8,529 (3,2); 8,471 (8,5); 8,469 (8,4); 8,317 (0,7); 8,019 (4,1); 8,015 (4,0); 7,848 (1,5); 7,843 (5,5); 7,839 (7,4); 7,834 (5,9); 7,826 (4,6); 7,820 (2,2); 7,526 (5,7); 7,517 (1,0); 7,513 (0,9); 7,504 (5,1); 3,328 (238,4); 3,109 (1,9); 3,091 (6,4); 3,072 (6,5); 3,054 (2,0); 2,874 (0,4); 2,864 (0,9); 2,854 (1,2); 2,846 (1,9); 2,836 (2,0); 2,827 (1,2); 2,818 (1,0); 2,808 (0,4); 2,676 (1,5); 2,671 (2,2); 2,667 (1,6); 2,662 (0,8); 2,525 (5,6); 2,520 (8,4); 2,511 (110,7); 2,507 (227,2); 2,502 (302,7); 2,498 (222,3); 2,493 (109,6); 2,338 (0,6); 2,334 (1,4); 2,329 (1,9); 2,324 (1,4); 2,320 (0,7); 1,398 (1,0); 1,235 (8,0); 1,217 (16,0); 1,198 (7,0); 0,736 (1,2); 0,723 (3,4); 0,718 (4,9); 0,706 (4,5); 0,700 (3,8); 0,688 (1,6); 0,577 (1,6); 0,566 (4,8); 0,560 (4,3); 0,556 (4,1); 0,550 (3,9); 0,538 (1,2); 0,146 (0,6); 0,008 (4,5); 0,000 (140,4); -0,009 (5,1); -0,150 (0,6) |
| Beispiel I-T3-157: ¹H-NMR (400,0 MHz, d₆-DMSO): δ= 9,389 (0,4); 9,355 (9,8); 8,964 (5,4); 8,959 (5,4); 8,642 (0,5); 8,627 (9,7); 8,577 (4,7); 8,540 (3,8); 8,529 (3,8); 8,317 (1,2); 7,913 (12,3); 7,893 (4,1); 7,887 (2,8); 7,541 (4,9); 7,520 (4,4); 4,038 (0,9); 4,020 (0,8); 4,002 (0,4); 3,454 (0,4); 3,425 (0,9); 3,406 (2,4); 3,387 (2,9); 3,372 (3,7); 3,353 (5,7); 3,329 (694,0); 2,952 (0,7); 2,934 (2,2); 2,915 (2,5); 2,900 (2,2); 2,882 (2,0); 2,864 (1,6); 2,855 (1,6); 2,846 (2,3); 2,836 (2,3); 2,827 (1,6); 2,818 (1,1); 2,807 (0,5); 2,676 (3,2); 2,671 (4,1); 2,667 (3,2); 2,507 (462,3); 2,502 (589,8); 2,498 (442,8); 2,333 (2,8); 2,329 (3,8); 2,325 (2,8); 1,989 (3,4); 1,398 (1,9); 1,234 (0,7); 1,193 (1,0); 1,175 (1,8); 1,157 (0,9); 1,099 (7,6); 1,081 (16,0); 1,063 (7,3); 0,741 (1,3); 0,728 (4,0); 0,723 (5,4); 0,711 (5,1); 0,705 (4,3); 0,694 (1,7); 0,583 (1,8); 0,573 (5,5); 0,566 (5,3); 0,557 (4,5); 0,545 (1,3); 0,146 (0,3); 0,008 (3,2); 0,000 (63,6) |
| Beispiel I-T3-158: ¹H-NMR (400,0 MHz, d₆-DMSO): δ= 9,234 (1,6); 9,229 (1,6); 9,165 (3,1); 8,632 (1,6); 8,627 (1,6); 8,584 (3,2); 8,547 (1,2); 8,536 (1,2); 7,875 (4,5); 7,871 (1,9); 7,857 (1,5); 7,852 (0,9); 7,548 (1,5); 7,543 (0,7); 7,529 (0,6); 7,525 (1,4); 4,151 (0,7); 4,132 (2,2); 4,114 (2,2); 4,095 (0,7); 3,329 (71,4); 2,867 (0,3); 2,857 (0,4); 2,849 (0,7); 2,839 (0,7); 2,830 (0,5); 2,821 (0,3); 2,676 (0,4); 2,672 (0,6); 2,667 (0,4); 2,525 (1,6); 2,511 (33,5); 2,507 (66,9); |
| 2,502 (87,7); 2,498 (64,8); 2,494 (32,5); 2,334 (0,4); 2,329 (0,6); 2,325 (0,4); 1,398 (16,0); 1,324 (2,6); 1,306 (5,7); 1,287 (2,6); 1,236 (0,3); 0,738 (0,4); 0,725 (1,3); 0,720 (1,8); 0,708 (1,6); 0,702 (1,4); 0,691 (0,6); 0,580 (0,6); 0,569 (1,8); 0,563 (1,6); 0,554 (1,4); 0,541 (0,4); 0,008 (0,4); 0,000 (10,6); -0,008 (0,4) |
| Beispiel I-T3-159: ¹H-NMR (400,0 MHz, d₆-DMSO): δ= 9,812 (2,8); 9,173 (7,1); 8,904 (2,9); 8,880 (0,5); 8,874 (0,4); 8,852 (1,7); 8,845 (2,6); 8,822 (6,9); 8,816 (7,2); 8,765 (9,2); 8,566 (0,7); 8,554 (1,9); 8,512 (5,8); 8,484 (11,2); 8,455 (6,7); 8,449 (6,6); 8,318 (0,7); 8,263 (1,1); 8,257 (1,1); 8,237 (0,4); 8,093 (5,7); 3,903 (16,0); 3,680 (2,8); 3,593 (0,6); 3,582 (0,9); 3,570 (0,7); 3,388 (0,9); 3,333 (306,9); 3,276 (1,0); 3,267 (1,3); 3,168 (13,0); 3,044 (0,4); 2,980 (0,9); 2,891 (2,0); 2,732 (1,7); 2,676 (1,9); 2,672 (2,6); 2,667 (1,9); 2,542 (0,9); 2,525 (6,2); 2,511 (158,8); 2,507 (322,0); 2,503 (423,2); 2,498 (311,3); 2,494 (156,1); 2,334 (2,0); 2,329 (2,7); 2,325 (2,1); 2,083 (0,4); 2,065 (0,3); 1,877 (2,4); 1,867 (5,7); 1,857 (6,2); 1,848 (2,6); 1,718 (0,4); 1,709 (0,4); 1,435 (0,4); 1,355 (0,6); 1,298 (0,6); 1,284 (0,5); 1,276 (0,6); 1,259 (3,3); 1,249 (6,9); 1,239 (9,7); 1,236 (9,6); 1,001 (0,6); 0,991 (0,5); 0,986 (0,5); 0,871 (0,5); 0,862 (0,6); 0,854 (1,3); 0,843 (0,5); 0,837 (0,7); 0,827 (0,3); 0,008 (0,7); 0,000 (25,7); -0,008 (1,0) |
| Beispiel I-T3-160: ¹H-NMR (400,0 MHz, CD3CN): δ= 8,728 (0,3); 8,722 (0,5); 8,704 (9,5); 8,698 (9,6); 8,524 (0,3); 8,353 (7,7); 8,234 (15,1); 8,219 (12,8); 8,100 (7,8); 8,052 (10,3); 8,046 (10,1); 7,603 (0,4); 7,592 (0,6); 7,560 (0,5); 7,537 (0,5); 7,495 (0,5); 7,490 (0,5); 7,342 (0,5); 7,067 (2,6); 5,449 (0,7); 4,054 (1,4); 3,893 (0,3); 3,441 (0,7); 3,375 (0,6); 3,241 (1,1); 3,154 (3,0); 3,070 (0,6); 2,886 (0,9); 2,876 (1,9); 2,867 (2,7); 2,858 (3,9); 2,849 (3,9); 2,840 (2,7); 2,831 (1,9); 2,821 (0,7); 2,600 (0,3); 2,590 (0,4); 2,531 (0,4); 2,470 (3,6); 2,465 (5,0); 2,460 (3,7); 2,432 (0,3); 2,425 (0,4); 2,394 (0,4); 2,368 (0,5); 2,359 (0,5); 2,329 (0,6); 2,316 (0,6); 2,289 (0,8); 2,261 (1,3); 2,257 (1,3); 2,255 (1,3); 2,243 (1,9); 2,178 (1072,5); 2,127 (0,9); 2,121 (1,9); 2,114 (3,1); 2,108 (4,0); 2,102 (2,8); 2,096 (1,6); 2,087 (0,7); 2,057 (0,4); 2,036 (0,7); 2,017 (1,1); 1,998 (1,2); 1,965 (20,6); 1,959 (52,5); 1,953 (270,7); 1,947 (487,5); 1,941 (651,6); 1,935 (450,7); 1,929 (234,0); 1,782 (1,4); 1,775 (2,7); 1,769 (3,6); 1,763 (2,5); 1,757 (1,3); 1,711 (2,4); 1,384 (0,4); 1,380 (0,8); 1,269 (16,0); 0,897 (0,7); 0,881 (1,8); 0,864 (0,9); 0,808 (2,2); 0,795 (6,8); 0,790 (8,9); 0,778 (9,1); 0,772 (6,9); 0,760 (3,0); 0,738 (0,4); 0,721 (0,4); 0,661 (0,4); 0,651 (0,4); 0,621 (3,0); 0,609 (8,1); 0,603 (8,3); 0,599 (7,4); 0,594 (7,1); 0,581 (2,1); 0,543 (0,5); 0,390 (0,4); 0,385 (0,5); 0,146 (8,5); 0,085 (0,4); 0,078 (0,5); 0,065 (0,5); 0,008 (68,5); 0,000 (1708,3); -0,009 (75,1); -0,049 (0,5); -0,058 (0,4); -0,150 (8,4) |
| Beispiel I-T3-161: ¹H-NMR (400,0 MHz, CD3CN): δ= 8,626 (2,2); 8,153 (1,1); 8,140 (16,0); 8,100 (13,1); 7,994 (7,6); 7,949 (7,7); 7,629 (9,3); 7,624 (11,5); 7,596 (6,2); 7,591 (4,8); 7,585 (2,3); 7,575 (7,4); 7,570 (6,0); 7,488 (0,5); 7,467 (0,4); 7,436 (11,0); 7,415 (8,4); 4,085 (1,8); 4,068 (5,5); 4,050 (5,6); 4,032 (1,9); 3,435 (0,7); 3,425 (1,5); 3,416 (1,9); 3,407 (3,0); 3,394 (2,9); 3,383 (1,8); 3,376 (1,3); 3,365 (0,6); 3,033 (0,5); 2,905 (0,4); 2,683 (0,6); 2,665 (0,6); 2,467 (0,8); 2,143 (2313,8); 2,117 (49,9); 2,108 (11,0); 2,101 (6,8); 2,095 (3,7); 1,972 (32,2); 1,964 (49,2); 1,958 (118,9); 1,953 (611,9); 1,946 (1099,9); 1,940 (1474,5); 1,934 (1019,6); 1,928 (525,2); 1,781 (2,8); 1,775 (5,7); 1,769 (7,9); 1,762 (5,4); 1,756 (2,4); 1,437 (7,6); 1,270 (1,1); 1,222 (6,5); 1,204 (13,0); 1,186 (6,4); 0,951 (2,0); 0,939 (6,4); 0,934 (8,6); 0,921 (8,9); 0,915 (6,5); 0,902 (2,8); 0,881 (0,8); 0,863 (0,6); 0,821 (0,5); 0,811 (0,4); 0,782 (2,8); 0,770 (7,8); 0,764 (8,1); 0,760 (6,9); 0,754 (6,8); 0,741 (1,9); 0,192 (0,4); 0,146 (19,2); 0,087 (1,0); 0,063 (1,5); 0,008 (155,4); 0,000 (3971,4); -0,009 (173,7); -0,068 (0,4); -0,150 (18,8) |
| Beispiel I-T3-162: ¹H-NMR (400,0 MHz, CD3CN): δ= 8,194 (6,9); 8,179 (6,7); 7,972 (3,4); 7,759 (3,0); 7,688 (3,5); 7,682 (4,6); 7,655 (2,4); 7,650 (1,8); 7,635 (2,7); 7,629 (2,4); 7,477 (4,3); 7,456 (3,4); 6,951 (1,1); 2,872 (0,7); 2,862 (1,1); 2,854 (1,6); 2,844 (1,6); 2,835 (1,1); 2,826 (0,8); 2,471 (0,3); 2,466 (0,5); 2,461 (0,3); 2,180 (199,2); 2,134 (0,5); 2,115 (0,5); 2,109 (0,6); 2,102 (0,4); 1,965 (2,7); 1,959 (6,7); 1,953 (37,6); 1,947 (69,0); 1,941 (93,6); 1,935 (65,6); 1,929 (34,4); 1,776 (0,4); 1,770 (0,5); 1,763 (0,4); 1,437 (16,0); 1,269 (0,4); 0,795 (0,9); 0,782 (2,6); 0,777 (3,6); 0,765 (3,7); 0,759 (2,8); 0,747 (1,2); 0,612 (1,2); 0,601 (3,3); 0,595 (3,4); 0,591 (3,1); 0,586 (3,0); 0,573 (0,9); 0,146 (1,5); 0,008 (10,7); 0,000 (294,5); -0,150 (1,5) |
| Beispiel I-T3-163: ¹H-NMR (400,0 MHz, CD3CN): δ= 8,205 (4,0); 8,190 (4,1); 7,973 (2,2); 7,760 (2,0); 7,736 (2,0); 7,731 (2,6); 7,704 (1,2); 7,700 (1,0); 7,684 (1,4); 7,679 (1,2); 7,600 (0,8); 7,509 (2,3); 7,488 (1,9); 2,161 (116,4); 2,121 (0,5); 2,114 (0,5); 2,108 (0,6); 2,102 (0,4); 1,963 (2,5); 1,952 (29,4); 1,946 (52,9); 1,941 (70,8); 1,934 (50,0); 1,928 (26,6); 1,769 (0,4); 1,598 (1,0); 1,583 (2,9); 1,576 (2,8); 1,563 (1,3); 1,436 (16,0); 1,362 (1,3); 1,348 (2,9); 1,341 (3,0); 1,327 (1,0); 1,269 (0,5); 0,145 (1,2); 0,000 (226,5); -0,150 (1,2) |
| Beispiel I-T3-164: ¹H-NMR (400,0 MHz, d₆-DMSO): δ= 9,460 (5,0); 8,854 (7,4); 8,671 (4,0); 8,651 (4,0); 8,535 (7,4); 7,846 (1,3); 7,840 (3,0); 7,833 (4,3); 7,827 (5,4); 7,824 (4,4); 7,818 (1,5); 7,594 (4,1); 7,584 (0,8); 7,582 (0,8); 7,572 (3,6); 4,056 (1,2); 4,038 (3,7); 4,020 (3,7); 4,002 (1,3); 3,934 (1,6); 3,329 (39,1); 2,671 (0,4); 2,525 (1,1); 2,507 (42,6); 2,502 (56,3); 2,498 (42,6); 2,329 (0,4); 1,989 (16,0); 1,619 (1,7); 1,605 (4,3); 1,598 (4,6); 1,585 (1,9); 1,397 (5,6); 1,295 (2,0); 1,281 (4,2); 1,275 (4,5); 1,260 (1,6); 1,193 (4,2); 1,175 (8,4); 1,157 (4,1); 1,069 (10,8); 0,008 (1,8); 0,000 (48,4); -0,008 (2,3) |
| Beispiel I-T3-165: ¹H-NMR (400,0 MHz, CD3CN): δ= 8,748 (1,7); 8,742 (1,8); 8,353 (1,5); 8,243 (2,8); 8,228 (2,4); 8,116 (1,9); 8,110 (2,1); 8,101 (1,5); 7,706 (0,5); 3,236 (0,9); 3,070 (0,4); 2,883 (0,4); 2,284 (0,3); 2,154 (118,3); 2,120 (0,8); 2,114 (0,9); 2,108 (0,9); 2,102 (0,7); 2,095 (0,4); 1,972 (0,7); 1,965 (3,4); 1,958 (8,8); 1,953 (47,0); 1,946 (85,3); 1,940 (114,8); 1,934 (80,1); 1,928 (41,8); 1,775 (0,5); 1,769 (0,7); 1,763 (0,5); 1,612 (0,8); 1,597 (2,0); 1,591 (2,0); 1,577 (1,0); 1,437 (16,0); 1,370 (1,0); 1,356 (2,0); 1,349 (2,1); 1,334 (0,8); 1,269 (0,9); 0,146 (1,9); 0,008 (15,7); 0,000 (390,9); - 0,009 (19,9); -0,150 (2,0) |
| Beispiel I-T3-166: ¹H-NMR (400,0 MHz, d₆-DMSO): δ= 9,440 (11,8); 9,127 (15,9); 8,569 (7,7); 8,491 (16,0); 8,318 (2,5); 7,965 (7,6); 7,961 (7,7); 7,926 (7,5); 7,921 (12,0); 7,912 (6,7); 7,906 (3,4); 7,891 (6,2); 7,885 (4,8); 7,573 (10,1); 7,553 (9,2); 3,459 (0,4); 3,399 (0,7); 3,365 (1,8); 3,331 (1516,7); 3,298 (1,6); 2,701 (0,4); 2,694 (0,3); 2,676 (6,5); 2,671 (9,1); 2,667 (6,9); 2,638 (0,4); 2,576 (1,1); 2,529 (53,9); 2,520 (35,9); 2,511 (509,9); 2,507 (1041,8); 2,502 (1375,8); 2,498 (1016,8); 2,417 (0,5); 2,351 (0,6); 2,334 (6,6); 2,329 (9,1); 2,325 (6,8); 2,302 (0,3); 1,621 (3,9); 1,607 (9,8); 1,600 (10,6); 1,587 (4,4); 1,547 (0,4); 1,348 (0,4); 1,307 (4,5); 1,294 (9,7); 1,287 (10,6); 1,273 (3,7); 1,237 (0,4); 0,146 (3,4); 0,024 (0,3); 0,008 (24,7); 0,000 (766,8); -0,008 (29,5); -0,032 (0,8); -0,150 (3,5) |
| Beispiel I-T3-167: ¹H-NMR (400,0 MHz, d₆-DMSO): δ= 9,440 (6,2); 9,103 (8,3); 9,094 (0,8); 8,587 (4,2); 8,479 (8,5); 8,317 (1,1); 8,021 (4,5); 7,917 (3,9); 7,911 (6,2); 7,903 (3,6); 7,898 (1,8); 7,882 (3,2); 7,877 (2,5); 7,597 (0,4); 7,574 (5,4); 7,554 (4,7); 3,329 (494,7); 3,110 (1,9); 3,092 (6,3); 3,074 (6,4); 3,056 (2,0); 2,871 (0,3); 2,676 (3,0); 2,671 (4,2); 2,667 (3,1); 2,524 (9,6); 2,507 (478,6); 2,502 (637,0); 2,498 (477,3); 2,408 (0,8); 2,333 (2,9); 2,329 (4,1); 2,325 |
| (3,1); 1,621 (2,0); 1,607 (5,2); 1,600 (5,6); 1,587 (2,3); 1,306 (2,4); 1,293 (5,3); 1,286 (5,7); 1,272 (2,1); 1,261 (0,6); 1,235 (7,6); 1,217 (16,0); 1,199 (7,1); 0,146 (1,6); 0,008 (11,5); 0,000 (362,6); -0,008 (14,5); -0,026 (0,6); -0,150 (1,6) |
| Beispiel I-T3-168: ¹H-NMR (400,0 MHz, d₆-DMSO): δ= 8,917 (4,0); 8,676 (2,2); 8,612 (2,2); 8,554 (1,4); 8,543 (1,4); 8,439 (4,1); 7,783 (0,8); 7,777 (1,6); 7,770 (2,3); 7,764 (3,0); 7,539 (2,1); 7,527 (0,4); 7,517 (1,8); 4,038 (0,4); 4,020 (0,4); 3,936 (2,3); 3,333 (40,9); 2,864 (0,4); 2,854 (0,6); 2,846 (0,8); 2,836 (0,8); 2,827 (0,6); 2,818 (0,4); 2,507 (26,6); 2,503 (34,6); 2,498 (26,4); 1,989 (1,7); 1,296 (0,7); 1,193 (0,5); 1,175 (0,9); 1,157 (0,5); 1,069 (16,0); 0,733 (0,5); 0,720 (1,6); 0,716 (2,1); 0,703 (2,0); 0,698 (1,8); 0,686 (0,7); 0,566 (0,7); 0,555 (2,1); 0,549 (2,0); 0,540 (1,8); 0,527 (0,6); 0,000 (11,0) |
| Beispiel I-T3-169: ¹H-NMR (400,0 MHz, d₆-DMSO): δ= 9,461 (11,1); 9,416 (0,4); 8,936 (15,7); 8,681 (8,8); 8,616 (8,7); 8,450 (16,0); 7,833 (11,0); 7,827 (9,7); 7,820 (7,2); 7,652 (0,3); 7,589 (8,1); 7,578 (1,9); 7,567 (7,0); 7,556 (0,5); 4,038 (0,8); 4,020 (0,8); 3,937 (0,6); 3,333 (133,2); 2,672 (0,7); 2,503 (110,4); 2,330 (0,7); 1,989 (3,2); 1,622 (3,6); 1,607 (9,7); 1,601 (10,5); 1,587 (4,3); 1,563 (0,4); 1,556 (0,4); 1,547 (0,5); 1,334 (0,4); 1,314 (0,5); 1,300 (4,8); 1,294 (5,2); 1,281 (9,9); 1,274 (10,4); 1,259 (3,7); 1,235 (0,6); 1,193 (0,9); 1,175 (1,7); 1,157 (0,9); 1,069 (3,7); 0,000 (35,7) |
| Beispiel I-T3-170: ¹H-NMR (400,0 MHz, d₆-DMSO): δ= 9,441 (1,5); 9,436 (2,4); 8,694 (1,7); 8,619 (3,2); 8,443 (1,7); 8,398 (3,2); 8,274 (2,8); 7,960 (1,6); 7,956 (1,6); 7,810 (0,9); 7,804 (2,1); 7,797 (2,3); 7,792 (2,9); 7,787 (3,1); 7,782 (0,9); 7,742 (1,6); 7,739 (1,6); 7,569 (1,0); 7,557 (2,1); 7,546 (1,3); 7,535 (1,8); 4,056 (1,2); 4,038 (3,6); 4,020 (3,7); 4,002 (1,2); 3,332 (35,6); 3,116 (0,7); 3,097 (2,3); 3,079 (2,4); 3,061 (0,7); 2,525 (0,5); 2,512 (10,5); 2,507 (21,4); 2,503 (28,1); 2,498 (20,5); 2,494 (9,9); 1,990 (16,0); 1,615 (1,1); 1,601 (2,8); 1,594 (2,9); 1,581 (1,3); 1,289 (1,3); 1,276 (2,8); 1,269 (3,0); 1,255 (1,1); 1,208 (2,7); 1,193 (5,6); 1,190 (6,2); 1,175 (9,2); 1,157 (4,2); 0,000 (3,5) |
| Beispiel I-T3-171: ¹H-NMR (400,0 MHz, d₆-DMSO): δ= 9,443 (12,6); 9,162 (16,0); 8,676 (8,2); 8,530 (16,0); 8,412 (7,8); 8,317 (4,0); 7,937 (8,0); 7,932 (12,0); 7,921 (6,8); 7,915 (3,8); 7,900 (6,4); 7,895 (5,0); 7,716 (0,4); 7,584 (10,3); 7,563 (9,1); 4,358 (2,5); 4,332 (7,5); 4,306 (7,8); 4,280 (2,7); 4,104 (0,5); 4,079 (0,4); 3,496 (0,5); 3,480 (0,4); 3,466 (0,5); 3,452 (0,4); 3,396 (0,8); 3,329 (1554,1); 3,287 (1,0); 2,676 (8,0); 2,671 (11,1); 2,667 (8,6); 2,645 (0,6); 2,525 (28,7); 2,511 (614,7); 2,507 (1266,3); 2,502 (1687,1); 2,498 (1264,4); 2,389 (0,6); 2,380 (0,6); 2,333 (7,8); 2,329 (11,0); 2,325 (8,3); 2,256 (0,4); 2,075 (1,4); 1,623 (4,0); 1,608 (10,0); 1,601 (10,8); 1,588 (4,6); 1,548 (0,5); 1,347 (0,4); 1,306 (4,7); 1,293 (10,0); 1,286 (10,8); 1,272 (3,9); 1,234 (0,7); 0,146 (0,5); 0,017 (0,4); 0,008 (3,6); 0,000 (115,1); -0,008 (5,1); -0,150 (0,6) |
| Beispiel I-T3-172: ¹H-NMR (400,0 MHz, d₆-DMSO): δ= 8,613 (0,3); 8,596 (8,4); 8,529 (2,9); 8,518 (3,0); 8,492 (0,7); 8,381 (8,5); 8,333 (0,6); 7,956 (4,4); 7,953 (4,4); 7,749 (2,3); 7,743 (4,8); 7,739 (5,0); 7,725 (13,2); 7,507 (4,3); 7,488 (2,3); 7,485 (3,1); 4,055 (1,2); 4,038 (3,6); 4,020 (3,7); 4,002 (1,2); 3,329 (51,5); 3,112 (1,9); 3,094 (6,1); 3,076 (6,1); 3,057 (2,0); 3,048 (0,4); 3,029 (0,9); 3,011 (0,9); 2,856 (0,8); 2,847 (1,1); 2,838 (1,8); 2,828 (1,8); 2,819 (1,1); 2,809 (0,9); 2,676 (0,4); 2,671 (0,5); 2,667 (0,4); 2,524 (1,3); 2,511 (30,4); 2,507 (60,9); 2,502 (79,7); 2,498 (58,7); 2,494 (29,4); 2,333 (0,4); 2,329 (0,5); 2,324 (0,4); 1,989 (16,0); 1,235 (0,4); 1,207 (6,8); 1,192 (7,1); 1,189 (14,7); 1,175 (10,4); 1,170 (7,2); 1,157 (4,7); 1,068 (0,4); 0,727 (1,1); 0,714 (3,3); 0,709 (4,6); 0,697 (4,3); 0,691 (3,7); 0,680 (1,5); 0,563 (1,5); 0,552 (4,6); 0,546 (4,3); 0,536 (3,8); 0,524 (1,1); 0,008 (2,2); 0,000 (61,3); -0,008 (2,4) |
| Beispiel I-T3-173: ¹H-NMR (400,0 MHz, d₆-DMSO): δ= 8,653 (6,0); 8,567 (3,1); 8,561 (3,2); 8,401 (5,7); 8,091 (10,1); 8,013 (3,1); 8,007 (3,2); 7,957 (0,4); 6,579 (0,7); 5,409 (0,3); 3,923 (16,0); 3,592 (0,3); 3,367 (923,7); 2,985 (14,3); 2,767 (0,4); 2,740 (1,3); 2,725 (0,9); 2,704 (1,4); 2,674 (0,9); 2,509 (96,0); 2,505 (123,6); 2,501 (91,6); 2,332 (0,8); 2,074 (1,6); 1,271 (0,8); 1,169 (5,1); 0,467 (4,6); 0,450 (2,4); 0,008 (1,4); 0,000 (28,9) |
| Beispiel I-T3-174: ¹H-NMR (400,0 MHz, d₆-DMSO): δ= 8,835 (7,5); 8,672 (3,0); 8,669 (4,0); 8,650 (3,9); 8,649 (3,9); 8,645 (2,9); 8,556 (2,5); 8,545 (2,5); 8,525 (7,6); 7,787 (1,7); 7,781 (2,9); 7,766 (4,0); 7,763 (11,3); 7,546 (3,2); 7,542 (1,7); 7,527 (1,5); 7,524 (2,8); 4,056 (1,2); 4,038 (3,6); 4,020 (3,7); 4,002 (1,2); 3,329 (59,6); 2,865 (0,7); 2,856 (0,9); 2,847 (1,4); 2,836 (1,5); 2,828 (0,9); 2,818 (0,7); 2,671 (0,4); 2,525 (1,0); 2,520 (1,6); 2,511 (21,0); 2,507 (42,8); 2,502 (56,7); 2,498 (41,2); 2,493 (19,8); 2,329 (0,4); 1,989 (16,0); 1,193 (4,3); 1,175 (8,7); 1,157 (4,2); 0,733 (1,0); 0,720 (2,7); 0,715 (3,8); 0,703 (3,5); 0,697 (2,9); 0,686 (1,3); 0,566 (1,3); 0,555 (3,7); 0,549 (3,3); 0,545 (3,1); 0,540 (3,0); 0,527 (0,9); 0,008 (1,4); 0,000 (40,4); - 0,009 (1,3) |
| Beispiel I-T3-175: ¹H-NMR (400,0 MHz, CD3CN): δ= 8,146 (3,1); 8,082 (4,8); 7,714 (1,6); 7,697 (2,0); 7,692 (2,5); 7,663 (1,3); 7,658 (1,0); 7,642 (1,5); 7,637 (1,3); 7,472 (2,3); 7,451 (1,8); 6,931 (0,6); 2,873 (0,4); 2,864 (0,6); 2,855 (0,9); 2,845 (0,9); 2,837 (0,6); 2,827 (0,4); 2,165 (79,0); 2,115 (0,4); 2,108 (0,5); 2,102 (0,3); 1,965 (1,5); 1,959 (4,0); 1,953 (28,1); 1,947 (52,6); 1,941 (72,5); 1,935 (50,5); 1,929 (26,2); 1,769 (0,4); 1,437 (16,0); 0,796 (0,5); 0,783 (1,5); 0,778 (2,0); 0,765 (2,0); 0,760 (1,5); 0,748 (0,7); 0,613 (0,6); 0,601 (1,7); 0,595 (1,8); 0,591 (1,6); 0,586 (1,6); 0,574 (0,5); 0,000 (0,6) |
| Beispiel I-T3-176: ¹H-NMR (400,0 MHz, CD3CN): δ= 19,983 (0,4); 8,920 (0,7); 8,270 (0,3); 8,158 (11,0); 8,124 (0,4); 8,095 (11,8); 8,083 (6,1); 8,035 (0,7); 7,746 (6,2); 7,741 (7,8); 7,713 (9,2); 7,692 (4,8); 7,686 (4,4); 7,669 (3,3); 7,590 (0,6); 7,505 (6,9); 7,484 (5,7); 3,901 (3,1); 2,470 (2,5); 2,466 (3,4); 2,461 (2,6); 2,417 (1,0); 2,179 (1701,9); 2,153 (39,9); 2,121 (2,8); 2,115 (3,6); 2,109 (4,3); 2,103 (3,2); 2,096 (1,9); 2,034 (0,5); 1,965 (12,0); 1,954 (216,5); 1,947 (401,4); 1,941 (546,5); 1,935 (384,1); 1,929 (201,2); 1,782 (1,6); 1,776 (2,6); 1,770 (3,4); 1,764 (2,5); 1,599 (3,1); 1,584 (8,5); 1,577 (8,4); 1,564 (4,1); 1,524 (0,7); 1,437 (16,0); 1,401 (0,7); 1,362 (3,9); 1,349 (8,6); 1,342 (8,8); 1,327 (3,1); 1,268 (2,7); 0,882 (0,4); 0,000 (4,2) |
| Beispiel I-T3-177: ¹H-NMR (400,0 MHz, d₆-DMSO): δ= 9,462 (7,5); 8,855 (1,9); 8,790 (8,8); 8,673 (1,1); 8,653 (1,1); 8,536 (1,9); 8,483 (8,9); 8,391 (4,5); 8,388 (4,6); 8,105 (4,5); 8,102 (4,5); 7,846 (0,5); 7,840 (1,0); 7,833 (2,8); 7,827 (5,2); 7,820 (5,9); 7,814 (7,0); 7,810 (5,4); 7,804 (1,9); 7,595 (1,3); 7,586 (5,5); 7,573 (1,8); 7,564 (4,6); 4,055 (0,5); 4,038 (1,4); 4,020 (1,4); 4,002 (0,5); 3,331 (91,4); 3,168 (2,0); 3,150 (6,5); 3,131 (6,6); 3,113 (2,0); 2,676 (0,5); 2,672 (0,7); 2,667 (0,5); 2,525 (1,8); 2,511 (39,2); 2,507 (78,6); 2,502 (103,0); 2,498 (76,1); 2,494 (38,1); 2,334 (0,5); 2,329 (0,7); 2,325 (0,5); 1,989 (6,1); 1,619 (2,5); 1,604 (6,3); 1,598 (6,8); 1,585 (2,8); 1,397 (5,9); 1,293 (2,9); 1,279 (6,3); 1,273 (6,7); 1,258 (2,4); 1,203 (7,5); 1,193 (2,5); 1,184 (16,0); 1,175 (4,1); 1,166 (7,2); 1,157 (2,0); 1,069 (0,5); 0,008 (2,2); 0,000 (62,2); -0,008 (2,5) |
| Beispiel I-T3-178: ¹H-NMR (400,0 MHz, d₆-DMSO): δ= 8,730 (5,4); 8,726 (5,4); 8,701 (10,4); 8,556 (3,7); 8,545 (3,7); 8,483 (0,5); 8,463 (10,3); 8,318 (0,6); 8,268 (5,5); 8,264 (5,4); 7,760 |
| (2,4); 7,754 (3,8); 7,736 (14,8); 7,524 (4,5); 7,505 (2,3); 7,502 (3,8); 4,055 (1,2); 4,037 (3,6); 4,020 (3,7); 4,002 (1,2); 3,460 (1,7); 3,442 (5,0); 3,424 (5,0); 3,405 (1,7); 3,329 (124,3); 2,868 (0,4); 2,858 (1,0); 2,848 (1,4); 2,840 (2,2); 2,830 (2,2); 2,821 (1,4); 2,811 (1,0); 2,802 (0,4); 2,675 (1,1); 2,671 (1,5); 2,667 (1,2); 2,541 (6,3); 2,506 (175,4); 2,502 (223,8); 2,498 (167,1); 2,333 (1,1); 2,329 (1,4); 2,324 (1,1); 1,989 (15,7); 1,235 (0,4); 1,193 (4,2); 1,175 (8,3); 1,157 (5,1); 1,150 (7,5); 1,132 (16,0); 1,113 (7,2); 0,728 (1,4); 0,715 (4,2); 0,710 (5,5); 0,698 (5,2); 0,692 (4,4); 0,681 (1,7); 0,561 (1,8); 0,551 (5,6); 0,545 (5,4); 0,535 (4,6); 0,523 (1,3); 0,146 (0,4); 0,000 (94,7); -0,008 (4,3); - 0,150 (0,4) |
| Beispiel I-T3-179: ¹H-NMR (400,0 MHz, d₆-DMSO): δ= 8,862 (9,5); 8,701 (0,4); 8,548 (3,2); 8,537 (3,2); 8,504 (0,4); 8,487 (10,0); 8,482 (5,1); 8,477 (4,7); 8,463 (0,4); 8,317 (1,4); 8,095 (4,8); 8,091 (4,8); 7,769 (2,1); 7,764 (3,9); 7,753 (5,3); 7,748 (11,5); 7,736 (0,7); 7,540 (5,3); 7,533 (1,3); 7,524 (1,3); 7,517 (4,5); 4,055 (1,0); 4,037 (3,2); 4,020 (3,2); 4,002 (1,1); 3,507 (0,3); 3,443 (0,4); 3,424 (0,4); 3,396 (0,5); 3,373 (0,8); 3,332 (779,0); 3,293 (0,7); 3,061 (0,5); 3,042 (1,8); 3,024 (2,1); 3,008 (2,2); 2,995 (0,5); 2,990 (2,0); 2,971 (0,6); 2,870 (0,4); 2,860 (0,9); 2,850 (1,3); 2,842 (2,0); 2,831 (2,1); 2,822 (1,3); 2,813 (1,0); 2,803 (0,4); 2,680 (1,3); 2,676 (2,7); 2,671 (3,7); 2,667 (2,8); 2,584 (1,0); 2,565 (2,6); 2,547 (3,3); 2,542 (5,6); 2,525 (10,0); 2,520 (15,4); 2,511 (205,0); 2,507 (417,5); 2,502 (550,6); 2,498 (403,5); 2,493 (198,2); 2,333 (2,6); 2,329 (3,6); 2,325 (2,7); 1,989 (13,8); 1,298 (0,3); 1,259 (0,5); 1,235 (0,9); 1,193 (3,9); 1,175 (7,6); 1,157 (3,8); 1,132 (0,6); 1,047 (7,2); 1,029 (16,0); 1,010 (6,9); 0,733 (1,3); 0,720 (3,5); 0,715 (5,2); 0,703 (4,7); 0,697 (4,1); 0,685 (1,7); 0,563 (1,7); 0,552 (5,0); 0,546 (4,7); 0,542 (4,4); 0,536 (4,2); 0,524 (1,3); 0,146 (1,1); 0,008 (8,8); 0,000 (281,7); -0,009 (10,6); -0,150 (1,1) |
| Beispiel I-T3-180: ¹H-NMR (400,0 MHz, d₆-DMSO): δ= 9,105 (5,5); 9,100 (4,5); 8,889 (0,7); 8,871 (9,2); 8,581 (4,6); 8,570 (6,0); 8,564 (6,8); 8,559 (5,5); 8,545 (9,1); 8,317 (0,9); 7,817 (0,3); 7,812 (0,3); 7,774 (3,3); 7,768 (4,2); 7,755 (7,5); 7,751 (13,2); 7,553 (4,7); 7,548 (2,1); 7,535 (2,4); 7,530 (3,5); 4,055 (0,8); 4,037 (2,1); 4,020 (2,1); 4,002 (0,7); 3,559 (2,3); 3,541 (6,5); 3,523 (6,4); 3,504 (2,1); 3,334 (39,2); 3,328 (131,0); 2,866 (1,3); 2,856 (1,8); 2,847 (2,5); 2,837 (2,3); 2,829 (1,5); 2,819 (1,0); 2,809 (0,4); 2,676 (2,3); 2,671 (2,5); 2,667 (1,8); 2,621 (0,4); 2,507 (343,0); 2,502 (380,1); 2,498 (256,9); 2,333 (2,1); 2,329 (2,4); 2,324 (1,6); 1,995 (2,3); 1,989 (8,7); 1,298 (0,4); 1,258 (0,6); 1,249 (0,7); 1,236 (1,2); 1,193 (3,8); 1,181 (8,8); 1,175 (7,3); 1,163 (16,0); 1,157 (4,7); 1,144 (6,8); 1,114 (0,6); 0,731 (1,9); 0,719 (5,2); 0,714 (5,7); 0,702 (5,8); 0,696 (4,2); 0,684 (1,6); 0,593 (0,3); 0,563 (2,7); 0,553 (6,7); 0,547 (6,1); 0,538 (4,5); 0,525 (1,3); 0,006 (15,8); 0,000 (61,8); -0,008 (2,7) |
| Beispiel I-T3-181: ¹H-NMR (400,0 MHz, d₆-DMSO): δ= 9,108 (0,4); 9,087 (10,3); 8,892 (5,1); 8,888 (5,2); 8,613 (0,4); 8,591 (10,7); 8,579 (3,8); 8,568 (3,7); 8,407 (5,3); 8,403 (5,3); 7,787 (2,0); 7,781 (4,5); 7,775 (6,6); 7,769 (7,7); 7,765 (6,0); 7,759 (2,3); 7,575 (6,0); 7,564 (1,5); 7,552 (5,0); 4,056 (1,1); 4,038 (3,4); 4,020 (3,4); 4,002 (1,2); 3,330 (39,4); 3,101 (0,5); 3,083 (1,8); 3,065 (2,2); 3,049 (2,3); 3,031 (2,1); 3,012 (0,6); 2,879 (0,4); 2,869 (1,0); 2,860 (1,4); 2,851 (2,1); 2,841 (2,1); 2,833 (1,4); 2,823 (1,0); 2,813 (0,4); 2,676 (0,5); 2,672 (0,6); 2,667 (0,5); 2,601 (0,7); 2,583 (2,1); 2,564 (2,5); 2,549 (2,3); 2,530 (2,5); 2,525 (2,1); 2,507 (69,4); 2,503 (90,4); 2,498 (68,1); 2,334 (0,4); 2,329 (0,6); 2,325 (0,4); 1,989 (14,7); 1,193 (3,9); 1,175 (7,7); 1,158 (3,8); 1,081 (0,4); 1,067 (7,5); 1,048 (16,0); 1,030 (7,2); 0,738 (1,3); 0,725 (4,0); 0,720 (5,5); 0,708 (5,1); 0,702 (4,4); 0,691 (1,8); 0,566 (1,8); 0,555 (5,4); 0,549 (5,2); 0,539 (4,6); 0,527 (1,3); 0,008 (0,6); 0,000 (16,1) |
| Beispiel I-T3-182: ¹H-NMR (400,0 MHz, d₆-DMSO): δ= 9,454 (7,2); 8,884 (10,2); 8,500 (10,2); 8,482 (4,9); 8,478 (5,0); 8,317 (0,4); 8,098 (5,0); 8,093 (5,0); 7,829 (1,5); 7,823 (5,9); 7,821 (7,8); 7,815 (6,3); 7,807 (4,8); 7,801 (2,4); 7,588 (5,9); 7,579 (1,0); 7,574 (1,0); 7,566 (5,1); 4,056 (1,2); 4,038 (3,6); 4,020 (3,6); 4,002 (1,2); 3,329 (62,1); 3,069 (0,5); 3,051 (1,8); 3,032 (2,1); 3,017 (2,3); 2,998 (2,1); 2,980 (0,6); 2,676 (0,6); 2,672 (0,8); 2,667 (0,6); 2,594 (0,6); 2,575 (2,1); 2,557 (2,5); 2,541 (2,4); 2,523 (3,8); 2,520 (3,9); 2,511 (46,1); 2,507 (93,1); 2,503 (122,1); 2,498 (89,1); 2,494 (44,0); 2,334 (0,6); 2,329 (0,8); 2,325 (0,6); 1,989 (15,8); 1,622 (2,4); 1,607 (5,9); 1,601 (6,4); 1,588 (2,7); 1,290 (2,8); 1,276 (5,9); 1,270 (6,4); 1,255 (2,4); 1,235 (0,5); 1,193 (4,2); 1,175 (8,3); 1,157 (4,1); 1,134 (0,3); 1,051 (7,3); 1,033 (16,0); 1,014 (7,1); 0,008 (0,8); 0,000 (23,6); - 0,008 (0,9) |
| Beispiel I-T3-183: ¹H-NMR (400,0 MHz, d₆-DMSO): δ= 9,464 (4,6); 8,733 (3,3); 8,729 (3,6); 8,723 (6,9); 8,477 (6,6); 8,270 (3,3); 8,266 (3,3); 7,820 (1,3); 7,814 (3,0); 7,808 (4,3); 7,803 (4,8); 7,798 (3,7); 7,792 (1,4); 7,573 (4,0); 7,562 (0,9); 7,551 (3,4); 4,055 (1,2); 4,038 (3,6); 4,020 (3,7); 4,002 (1,2); 3,465 (0,9); 3,447 (2,9); 3,429 (3,0); 3,410 (1,0); 3,329 (48,5); 2,676 (0,4); 2,671 (0,6); 2,667 (0,5); 2,525 (1,6); 2,511 (35,4); 2,507 (71,4); 2,502 (93,1); 2,498 (68,1); 2,494 (33,4); 2,333 (0,4); 2,329 (0,6); 2,325 (0,4); 1,989 (16,0); 1,614 (1,6); 1,600 (3,9); 1,593 (4,1); 1,580 (1,7); 1,290 (1,9); 1,277 (4,0); 1,270 (4,3); 1,256 (1,6); 1,235 (0,5); 1,193 (4,4); 1,175 (8,8); 1,157 (4,9); 1,152 (5,1); 1,134 (10,7); 1,115 (4,8); 0,008 (0,6); 0,000 (19,6); -0,009 (0,7) |
| Beispiel I-T3-184: ¹H-NMR (400,0 MHz, CD3CN): δ= 8,222 (1,1); 8,212 (14,6); 8,193 (0,9); 8,163 (0,7); 8,151 (14,2); 8,150 (13,6); 7,984 (5,6); 7,968 (5,6); 7,937 (0,6); 7,860 (0,5); 7,837 (0,5); 7,699 (8,7); 7,694 (11,4); 7,666 (5,9); 7,660 (4,9); 7,645 (6,7); 7,639 (6,1); 7,588 (0,8); 7,523 (0,3); 7,480 (10,7); 7,459 (8,5); 6,928 (2,6); 5,448 (5,4); 2,881 (0,7); 2,871 (1,9); 2,862 (2,7); 2,853 (4,3); 2,844 (4,3); 2,835 (2,8); 2,825 (2,1); 2,816 (0,7); 2,474 (0,9); 2,469 (1,8); 2,464 (2,4); 2,460 (1,9); 2,455 (1,0); 2,293 (0,4); 2,270 (0,7); 2,266 (0,7); 2,246 (1,0); 2,227 (1,0); 2,160 (875,2); 2,121 (3,1); 2,114 (4,2); 2,108 (5,3); 2,102 (3,8); 2,096 (2,1); 2,036 (0,6); 2,018 (0,8); 1,998 (0,9); 1,965 (19,7); 1,959 (49,7); 1,953 (311,9); 1,947 (575,9); 1,941 (786,4); 1,935 (548,9); 1,929 (287,9); 1,883 (0,6); 1,782 (1,8); 1,775 (3,4); 1,769 (4,7); 1,763 (3,2); 1,757 (1,8); 1,525 (0,4); 1,385 (0,3); 1,372 (0,5); 1,359 (0,4); 1,340 (1,4); 1,335 (0,8); 1,285 (2,9); 1,270 (16,0); 1,204 (0,3); 0,918 (0,4); 0,899 (0,9); 0,882 (2,1); 0,864 (1,1); 0,832 (0,4); 0,795 (2,4); 0,783 (7,1); 0,777 (9,7); 0,765 (9,8); 0,760 (7,5); 0,747 (3,3); 0,726 (0,5); 0,708 (0,5); 0,652 (0,5); 0,643 (0,5); 0,627 (0,4); 0,613 (3,3); 0,601 (8,2); 0,595 (8,8); 0,591 (7,8); 0,586 (7,8); 0,573 (2,3); 0,536 (0,3); 0,520 (0,4); 0,478 (0,3); 0,392 (0,4); 0,387 (0,4); 0,008 (0,7); 0,000 (20,9); -0,009 (1,0) |
| Beispiel I-T3-185: ¹H-NMR (400,0 MHz, CD3CN): δ= 17,517 (0,4); 15,219 (0,3); 14,973 (0,3); 13,920 (0,3); 8,404 (0,4); 8,394 (0,4); 8,224 (16,0); 8,208 (0,8); 8,165 (12,9); 7,986 (5,2); 7,970 (5,1); 7,938 (1,2); 7,864 (0,5); 7,840 (0,4); 7,747 (7,8); 7,741 (10,5); 7,715 (5,2); 7,710 (4,3); 7,694 (6,0); 7,689 (5,7); 7,654 (3,5); 7,624 (1,2); 7,601 (2,1); 7,594 (1,3); 7,590 (2,0); 7,564 (1,9); 7,541 (1,1); 7,513 (9,5); 7,492 (7,5); 7,292 (0,7); 7,282 (0,4); 7,270 (0,7); 7,201 (0,4); 7,176 (1,6); 7,168 (0,4); 7,151 (0,4); 7,064 (0,5); 7,045 (0,4); 6,914 (0,4); 6,892 (0,4); 6,881 (0,8); 6,859 (0,6); 6,178 (0,3); 6,160 (0,4); 6,111 (0,4); 6,099 (0,4); 6,067 (0,4); 6,042 (0,4); 6,038 (0,4); 6,017 (0,4); 5,640 (0,4); 5,594 (0,3); 5,540 (0,3); 5,516 (0,4); 5,485 (0,4); 5,427 (0,3); 5,373 (0,3); 4,507 (0,6); 4,491 (0,6); 4,068 (1,3); 4,050 (1,1); 4,032 (0,5); 3,789 (0,5); 3,776 (1,0); 3,758 (2,9); 3,656 (0,4); 3,149 (0,4); 3,128 (0,4); 2,720 (13,7); 2,656 (0,5); 2,492 (0,9); 2,475 (2,8); 2,470 (6,1); 2,465 (9,0); 2,461 (6,7); 2,456 (3,4); 2,285 (0,4); 2,264 (0,7); 2,247 (1,5); 2,237 (1,1); 2,171 (2483,4); 2,121 (7,1); 2,114 (9,5); 2,108 (11,9); 2,102 (8,8); 2,096 (5,2); 2,075 (1,8); 2,032 |
| (0,9); 2,020 (0,7); 2,011 (0,6); 1,972 (8,4); 1,965 (39,5); 1,959 (102,1); 1,953 (642,3); 1,947 (1199,8); 1,941 (1645,9); 1,935 (1167,9); 1,929 (619,1); 1,818 (1,3); 1,782 (4,2); 1,775 (7,5); 1,769 (10,3); 1,763 (7,4); 1,757 (4,5); 1,722 (0,9); 1,708 (0,9); 1,696 (0,9); 1,688 (0,9); 1,674 (0,7); 1,638 (0,8); 1,597 (4,8); 1,583 (11,6); 1,576 (12,0); 1,562 (6,3); 1,543 (0,8); 1,522 (1,2); 1,501 (0,6); 1,472 (0,6); 1,437 (9,0); 1,402 (1,2); 1,361 (6,2); 1,348 (11,9); 1,341 (12,8); 1,327 (7,6); 1,311 (3,5); 1,269 (8,7); 1,222 (1,9); 1,204 (3,2); 1,186 (1,7); 1,164 (0,6); 1,154 (0,6); 1,145 (0,5); 1,131 (0,6); 1,109 (0,6); 1,095 (0,6); 1,091 (0,6); 1,047 (0,5); 1,040 (0,4); 1,031 (0,4); 1,009 (0,4); 0,987 (0,4); 0,976 (0,4); 0,952 (0,4); 0,945 (0,5); 0,897 (0,8); 0,881 (1,6); 0,855 (1,3); 0,838 (0,9); 0,824 (0,5); 0,806 (0,5); 0,797 (0,4); 0,776 (0,4); 0,766 (0,5); 0,739 (0,4); 0,636 (0,3); 0,526 (0,3); 0,147 (0,4); 0,008 (2,5); 0,000 (80,2); -0,020 (0,5); -0,121 (0,3); -0,149 (0,4); -0,213 (0,4); -2,478 (0,3); -3,017 (0,3) |
| Beispiel I-T3-186: ¹H-NMR (400,0 MHz, CD3CN): δ= 8,202 (8,0); 8,190 (8,4); 7,927 (0,7); 7,858 (4,8); 7,731 (2,7); 7,708 (2,9); 7,690 (4,8); 7,685 (6,1); 7,657 (2,8); 7,652 (2,5); 7,637 (3,4); 7,632 (3,2); 7,590 (0,8); 7,477 (5,0); 7,456 (4,0); 6,984 (0,3); 6,951 (1,9); 6,041 (0,3); 5,521 (0,4); 5,491 (0,4); 5,466 (0,3); 3,874 (0,9); 3,056 (0,3); 2,890 (4,6); 2,872 (1,0); 2,863 (1,5); 2,853 (2,1); 2,844 (2,2); 2,835 (1,7); 2,825 (1,1); 2,799 (0,4); 2,772 (4,2); 2,711 (0,4); 2,684 (0,4); 2,671 (0,4); 2,662 (0,4); 2,619 (0,4); 2,601 (1,1); 2,583 (0,4); 2,543 (0,5); 2,522 (0,5); 2,505 (0,6); 2,466 (6,3); 2,351 (0,9); 2,310 (1,3); 2,298 (1,3); 2,179 (2377,2); 2,121 (5,6); 2,115 (6,3); 2,108 (7,1); 2,102 (5,6); 2,043 (0,8); 2,018 (1,0); 1,953 (344,9); 1,947 (623,4); 1,941 (840,7); 1,935 (641,3); 1,929 (374,2); 1,827 (0,6); 1,781 (2,1); 1,776 (3,6); 1,770 (4,8); 1,763 (3,6); 1,758 (2,2); 1,437 (16,0); 1,311 (0,4); 1,283 (0,5); 1,268 (0,9); 0,794 (1,2); 0,777 (5,1); 0,764 (5,1); 0,747 (1,8); 0,738 (0,4); 0,612 (1,5); 0,600 (4,9); 0,594 (5,3); 0,586 (4,9); 0,573 (1,5); 0,000 (28,5) |
| Beispiel I-T3-187: ¹H-NMR (400,0 MHz, CD3CN): δ= 19,987 (0,6); 8,214 (12,6); 8,200 (13,9); 7,859 (6,8); 7,738 (9,0); 7,733 (14,7); 7,707 (9,2); 7,701 (5,6); 7,686 (6,6); 7,680 (5,9); 7,610 (3,2); 7,587 (1,7); 7,510 (9,9); 7,489 (8,2); 7,448 (0,5); 2,469 (1,8); 2,464 (2,6); 2,459 (2,0); 2,157 (1156,2); 2,120 (5,2); 2,114 (6,3); 2,108 (7,5); 2,102 (5,6); 2,096 (3,3); 1,965 (26,5); 1,959 (71,5); 1,953 (401,8); 1,947 (750,3); 1,941 (1019,6); 1,935 (723,9); 1,928 (383,2); 1,781 (2,7); 1,775 (4,6); 1,769 (6,2); 1,763 (4,5); 1,757 (2,6); 1,634 (0,6); 1,597 (4,4); 1,583 (11,2); 1,576 (11,2); 1,563 (6,0); 1,523 (1,0); 1,437 (16,0); 1,401 (1,1); 1,361 (6,0); 1,347 (11,3); 1,341 (12,0); 1,326 (4,9); 1,270 (8,5); 0,882 (2,0); 0,857 (2,2); 0,000 (34,6) |
| Beispiel I-T3-188: ¹H-NMR (400,0 MHz, CD3CN): δ= 8,340 (9,9); 8,272 (16,0); 8,192 (0,3); 7,761 (9,7); 7,755 (12,3); 7,742 (0,4); 7,737 (0,3); 7,727 (6,7); 7,721 (5,0); 7,706 (7,8); 7,700 (6,5); 7,569 (4,5); 7,524 (11,5); 7,503 (9,3); 5,447 (0,8); 2,576 (0,8); 2,572 (0,8); 2,250 (0,4); 2,139 (89,4); 2,120 (0,7); 2,114 (0,7); 2,108 (0,9); 2,102 (0,6); 2,095 (0,3); 1,965 (3,2); 1,959 (8,4); 1,953 (52,4); 1,947 (96,8); 1,940 (132,2); 1,934 (91,5); 1,928 (47,5); 1,781 (0,3); 1,775 (0,6); 1,769 (0,8); 1,763 (0,6); 1,604 (5,0); 1,590 (12,7); 1,583 (12,8); 1,569 (6,7); 1,529 (0,9); 1,406 (0,8); 1,366 (6,8); 1,352 (12,5); 1,346 (13,2); 1,331 (5,3); 1,309 (0,5); 1,293 (0,9); 1,285 (1,5); 1,269 (7,2); 0,898 (0,3); 0,881 (0,9); 0,864 (0,4); 0,000 (3,9) |
| Beispiel I-T3-189: ¹H-NMR (400,0 MHz, CD3CN): δ= 8,726 (0,3); 8,720 (0,4); 8,701 (10,1); 8,694 (10,3); 8,265 (0,8); 8,253 (16,0); 8,246 (15,9); 8,062 (0,6); 8,051 (11,3); 8,045 (11,1); 7,978 (7,4); 7,766 (6,5); 7,051 (2,2); 5,449 (0,6); 4,068 (0,4); 4,050 (0,4); 3,024 (0,4); 2,888 (0,6); 2,878 (1,7); 2,869 (2,5); 2,860 (3,8); 2,851 (3,8); 2,842 (2,5); 2,833 (1,8); 2,823 (0,6); 2,729 (0,6); 2,473 (0,5); 2,468 (0,9); 2,464 (1,2); 2,459 (0,9); 2,454 (0,5); 2,166 (234,9); 2,121 (0,8); 2,114 (1,2); 2,108 (1,5); 2,102 (1,1); 2,096 (0,6); 2,087 (0,5); 2,035 (0,4); 2,017 (0,7); 1,998 (0,6); 1,972 (2,6); 1,965 (5,7); 1,959 (13,8); 1,953 (85,4); 1,947 (157,6); 1,941 (215,5); 1,935 (150,1); 1,928 (78,1); 1,782 (0,5); 1,775 (0,9); 1,769 (1,3); 1,763 (0,9); 1,757 (0,5); 1,437 (3,4); 1,308 (0,3); 1,268 (8,7); 1,222 (0,6); 1,204 (1,0); 1,186 (0,5); 0,898 (0,4); 0,881 (1,1); 0,864 (0,5); 0,810 (2,1); 0,797 (6,2); 0,792 (8,4); 0,779 (8,6); 0,774 (6,4); 0,762 (2,8); 0,740 (0,4); 0,722 (0,4); 0,662 (0,3); 0,652 (0,4); 0,622 (2,8); 0,610 (7,3); 0,604 (7,7); 0,600 (6,9); 0,595 (6,8); 0,582 (2,1); 0,543 (0,4); 0,391 (0,3); 0,386 (0,3); 0,008 (1,2); 0,000 (38,3); -0,009 (1,6) |
| Beispiel I-T3-190: ¹H-NMR (400,0 MHz, d₆-DMSO): δ= 9,487 (7,1); 9,105 (5,1); 9,102 (5,1); 8,887 (9,5); 8,557 (11,6); 8,317 (0,4); 7,826 (4,4); 7,820 (6,3); 7,815 (6,9); 7,810 (5,3); 7,602 (5,1); 7,591 (1,5); 7,580 (4,2); 4,055 (1,3); 4,038 (3,8); 4,020 (3,8); 4,002 (1,3); 3,560 (1,9); 3,542 (6,2); 3,523 (6,3); 3,505 (2,1); 3,328 (157,1); 2,671 (1,6); 2,506 (190,5); 2,502 (243,8); 2,329 (1,7); 1,989 (16,0); 1,617 (2,3); 1,602 (6,1); 1,596 (6,6); 1,583 (2,7); 1,296 (2,7); 1,282 (6,2); 1,276 (6,6); 1,261 (2,3); 1,193 (4,5); 1,181 (7,3); 1,175 (10,1); 1,163 (14,9); 1,158 (7,2); 1,145 (6,8); 0,000 (25,1) |
| Beispiel I-T3-191: ¹H-NMR (400,0 MHz, d₆-DMSO): δ= 9,426 (3,3); 8,594 (4,6); 8,359 (4,8); 8,317 (0,5); 7,785 (4,0); 7,779 (2,8); 7,770 (2,1); 7,765 (1,1); 7,545 (2,7); 7,537 (0,6); 7,530 (0,5); 7,522 (2,3); 7,351 (6,5); 3,331 (366,6); 3,015 (2,0); 2,997 (6,6); 2,979 (6,7); 2,960 (2,1); 2,676 (1,1); 2,671 (1,6); 2,667 (1,2); 2,524 (4,4); 2,511 (86,9); 2,507 (177,3); 2,502 (236,3); 2,498 (176,3); 2,493 (90,0); 2,333 (1,1); 2,329 (1,5); 2,324 (1,2); 1,611 (1,1); 1,597 (2,7); 1,590 (2,9); 1,577 (1,2); 1,398 (15,1); 1,285 (1,3); 1,271 (2,7); 1,264 (2,9); 1,250 (1,1); 1,195 (7,5); 1,177 (16,0); 1,158 (7,3); 0,146 (1,7); 0,008 (13,5); 0,000 (371,8); -0,008 (16,5); -0,150 (1,7) |
| Beispiel I-T3-192: ¹H-NMR (400,0 MHz, d₆-DMSO): δ= 9,435 (6,5); 8,687 (9,2); 8,408 (9,3); 8,317 (1,5); 7,796 (4,7); 7,787 (4,3); 7,780 (6,0); 7,775 (6,7); 7,770 (5,2); 7,764 (2,0); 7,559 (5,8); 7,548 (5,4); 7,537 (5,3); 3,330 (735,7); 3,123 (0,7); 3,082 (1,9); 3,063 (6,4); 3,045 (6,6); 3,027 (2,0); 2,838 (0,6); 2,676 (2,8); 2,671 (4,1); 2,667 (3,1); 2,525 (10,0); 2,520 (15,7); 2,511 (220,9); 2,507 (459,4); 2,502 (612,6); 2,498 (450,6); 2,493 (223,7); 2,333 (2,9); 2,329 (4,0); 2,324 (3,0); 1,614 (2,1); 1,600 (5,3); 1,593 (5,7); 1,580 (2,4); 1,284 (2,5); 1,271 (5,3); 1,264 (5,7); 1,250 (2,1); 1,205 (7,5); 1,187 (16,0); 1,168 (7,5); 0,146 (1,8); 0,008 (13,1); 0,000 (404,0); -0,009 (15,5); -0,150 (1,8) |
| Beispiel I-T3-193: ¹H-NMR (400,0 MHz, d₆-DMSO): δ= 9,458 (0,3); 8,777 (8,3); 8,542 (3,2); 8,531 (3,0); 8,504 (0,3); 8,462 (8,6); 8,337 (3,7); 8,317 (1,8); 7,962 (3,6); 7,750 (2,0); 7,745 (3,2); 7,738 (1,0); 7,726 (12,9); 7,717 (1,1); 7,710 (1,7); 7,692 (1,1); 7,659 (1,1); 7,653 (0,9); 7,569 (1,2); 7,548 (1,0); 7,529 (3,7); 7,526 (2,4); 7,510 (2,0); 7,507 (3,2); 7,484 (1,3); 7,465 (0,8); 7,420 (0,5); 7,402 (0,6); 7,058 (1,6); 6,924 (3,7); 6,789 (1,9); 4,055 (1,2); 4,037 (3,6); 4,020 (3,6); 4,002 (1,2); 3,328 (98,6); 3,305 (0,8); 2,858 (0,9); 2,849 (1,2); 2,840 (1,9); 2,830 (1,9); 2,821 (1,3); 2,812 (0,9); 2,801 (0,4); 2,676 (1,1); 2,671 (1,6); 2,667 (1,2); 2,662 (0,6); 2,524 (3,6); 2,511 (83,9); 2,507 (175,0); 2,502 (233,7); 2,498 (171,2); 2,493 (84,1); 2,338 (0,5); 2,333 (1,1); 2,329 (1,5); 2,324 (1,1); 1,989 (16,0); 1,234 (0,8); 1,193 (4,2); 1,175 (8,4); 1,157 (4,2); 0,729 (1,2); 0,716 (3,5); 0,711 (4,9); 0,699 (4,5); 0,693 (4,0); 0,682 (1,6); 0,568 (0,4); 0,559 (1,8); 0,549 (5,1); 0,543 (4,5); 0,534 (3,7); 0,522 (1,2); 0,146 (0,4); 0,008 (2,5); 0,000 (83,8); -0,009 (3,2); -0,150 (0,4) |
| Beispiel I-T3-194: ¹H-NMR (400,0 MHz, CD3CN): δ= 8,349 (9,1); 8,304 (0,4); 8,286 (14,8); 8,241 (0,3); 7,801 (9,7); 7,795 (11,3); 7,718 (6,0); 7,712 (5,6); 7,697 (7,3); 7,692 (6,9); 7,665 (0,5); 7,647 (0,7); 7,643 (0,7); 7,635 (0,5); 7,617 (0,6); 7,614 (0,7); 7,584 (1,1); 7,541 (0,6); 7,522 (11,4); 7,501 (9,2); 7,484 (0,5); 7,453 |
| (4,1); 7,422 (0,4); 7,236 (0,4); 7,215 (0,3); 6,837 (0,5); 6,673 (0,4); 6,617 (1,3); 6,575 (0,4); 5,973 (0,3); 5,954 (0,3); 5,896 (1,4); 5,447 (5,8); 3,817 (0,8); 3,769 (0,4); 3,550 (1,0); 3,545 (0,9); 2,579 (0,4); 2,575 (0,5); 2,269 (0,4); 2,253 (0,5); 2,140 (512,8); 2,120 (7,2); 2,114 (6,9); 2,108 (7,9); 2,102 (5,5); 2,095 (3,1); 1,965 (25,3); 1,959 (63,5); 1,953 (411,4); 1,947 (768,8); 1,940 (1065,1); 1,934 (761,0); 1,928 (406,5); 1,849 (1,2); 1,799 (0,7); 1,781 (2,8); 1,775 (4,8); 1,769 (6,7); 1,763 (4,7); 1,756 (2,7); 1,728 (0,5); 1,714 (0,5); 1,699 (0,5); 1,677 (0,4); 1,666 (0,5); 1,649 (0,4); 1,628 (0,4); 1,580 (0,4); 1,570 (0,4); 1,556 (0,4); 1,515 (0,6); 1,477 (5,4); 1,466 (14,4); 1,457 (16,0); 1,447 (6,5); 1,407 (0,7); 1,398 (0,5); 1,386 (0,8); 1,366 (0,5); 1,340 (6,0); 1,305 (1,0); 1,285 (8,1); 1,270 (5,0); 1,247 (0,7); 1,230 (0,5); 1,217 (0,6); 1,199 (0,5); 1,190 (0,6); 1,185 (0,7); 1,145 (6,1); 1,135 (15,7); 1,126 (14,8); 1,115 (5,4); 1,076 (0,6); 1,063 (0,4); 0,994 (0,4); 0,976 (0,8); 0,958 (0,5); 0,951 (0,4); 0,930 (0,4); 0,923 (0,4); 0,882 (1,1); 0,856 (0,9); 0,842 (0,7); 0,783 (0,4); 0,771 (0,4); 0,764 (0,5); 0,735 (0,4); 0,597 (0,3); 0,564 (0,4); 0,008 (1,0); 0,000 (32,8) |
| Beispiel I-T3-195: ¹H-NMR (400,0 MHz, d₆-DMSO): δ= 9,457 (8,1); 8,920 (11,3); 8,511 (11,3); 8,325 (5,6); 8,319 (6,1); 8,030 (5,3); 7,802 (8,5); 7,797 (7,4); 7,789 (5,4); 7,784 (3,0); 7,591 (6,0); 7,582 (1,3); 7,569 (5,0); 4,037 (0,6); 4,019 (0,6); 3,329 (278,3); 3,090 (0,5); 3,071 (1,9); 3,053 (2,4); 3,037 (2,5); 3,019 (2,1); 3,000 (0,7); 2,675 (3,1); 2,671 (4,3); 2,667 (3,5); 2,597 (0,4); 2,506 (494,1); 2,502 (653,4); 2,498 (511,0); 2,470 (6,8); 2,452 (3,7); 2,434 (2,4); 2,416 (1,0); 2,333 (3,0); 2,329 (4,1); 2,325 (3,3); 1,989 (2,4); 1,621 (2,6); 1,607 (6,7); 1,600 (7,6); 1,587 (3,1); 1,397 (1,3); 1,335 (0,4); 1,327 (0,3); 1,297 (0,8); 1,286 (3,2); 1,273 (7,0); 1,266 (7,6); 1,252 (3,2); 1,235 (5,1); 1,193 (0,7); 1,175 (1,3); 1,157 (0,7); 1,107 (0,4); 0,982 (7,4); 0,964 (16,0); 0,945 (7,3); 0,854 (0,5); 0,835 (0,3); 0,000 (20,8) |
| Beispiel I-T3-196: ¹H-NMR (601,6 MHz, CD3CN): δ= 8,223 (0,5); 8,218 (0,5); 8,045 (0,3); 8,041 (0,3); 2,621 (0,7); 2,150 (4,8); 1,948 (1,3); 1,944 (2,2); 1,940 (3,2); 1,936 (2,2); 1,932 (1,1); 1,135 (16,0); 0,000 (0,9) |
| Beispiel I-T3-197: ¹H-NMR (601,6 MHz, CD3CN): δ= 8,765 (7,5); 8,761 (7,5); 8,2493 (9,5); 8,2486 (9,9); 8,230 (8,0); 8,115 (8,0); 8,111 (8,0); 8,038 (4,4); 7,992 (4,5); 7,394 (1,5); 3,844 (16,0); 3,552 (11,2); 3,542 (11,1); 3,312 (0,7); 3,303 (0,7); 2,172 (78,0); 2,155 (28,3); 2,088 (0,6); 2,084 (0,8); 2,080 (0,6); 1,998 (2,0); 1,989 (5,4); 1,985 (7,5); 1,982 (52,9); 1,977 (98,7); 1,973 (145,4); 1,969 (98,4); 1,965 (48,4); 1,956 (0,7); 1,863 (0,5); 1,859 (0,8); 1,854 (0,6); 1,312 (2,7); 1,303 (8,1); 1,299 (8,0); 1,291 (3,5); 1,266 (0,4); 1,192 (0,4); 1,166 (3,5); 1,158 (7,9); 1,154 (7,9); 1,146 (2,6) |
| Beispiel I-T3-198: ¹H-NMR (400,0 MHz, d₆-DMSO): δ= 9,484 (3,8); 9,467 (0,6); 8,776 (0,9); 8,753 (5,3); 8,574 (0,6); 8,561 (8,6); 8,509 (5,3); 8,491 (0,8); 8,317 (1,8); 8,178 (0,4); 7,782 (7,1); 7,778 (3,2); 7,765 (2,2); 7,759 (1,3); 7,579 (2,8); 7,557 (2,4); 4,049 (0,4); 3,497 (0,3); 3,479 (0,7); 3,461 (0,7); 3,413 (1,0); 3,395 (1,5); 3,377 (1,6); 3,329 (582,9); 3,287 (1,0); 2,676 (4,0); 2,671 (5,5); 2,667 (4,1); 2,524 (14,3); 2,507 (629,0); 2,502 (826,7); 2,498 (606,0); 2,333 (3,8); 2,329 (5,2); 2,324 (3,9); 1,614 (1,3); 1,600 (3,4); 1,593 (3,7); 1,580 (1,5); 1,289 (1,5); 1,276 (3,3); 1,269 (3,6); 1,255 (1,3); 1,237 (0,4); 1,150 (0,4); 1,126 (0,7); 1,108 (8,3); 1,089 (16,0); 1,071 (7,0); 0,008 (0,5); 0,000 (18,9); -0,008 (0,7) |
| Beispiel I-T3-199: ¹H-NMR (400,1 MHz, d₆-DMSO): d= 8,86 (0,0328); 8,85 (0,0664); 8,56 (0,0471);8,51 (0,0250);8,18 (0,0290);8,17 (0,0298);8,09 (0,0245);3,47 (0,0401);3,45 (0,0407);3,31 (0,7767);2,54 (0,3233);2,50 (0,3250);2,50 (0,4400);2,50 (0,3578);1,25 (0,0306);1,25 (0,0369);1,15 (0,0210);1,14 (0,0347);1,13 (0,0321);0,00 (1,0000) |
| Beispiel I-T3-200: ¹H-NMR (400,0 MHz, CD3CN): δ= 8,745 (8,9); 8,739 (8,9); 8,566 (1,6); 8,560 (1,6); 8,264 (13,9); 8,256 (14,3); 8,244 (0,7); 8,115 (9,6); 8,109 (9,4); 8,056 (1,8); 8,050 (1,7); 7,998 (0,4); 7,983 (7,2); 7,980 (7,2); 7,767 (6,3); 7,690 (2,3); 7,666 (0,6); 7,587 (0,3); 5,448 (3,1); 3,724 (0,8); 3,071 (0,5); 2,882 (0,5); 2,626 (0,7); 2,603 (0,5); 2,468 (0,4); 2,463 (0,5); 2,458 (0,4); 2,152 (189,2); 2,120 (1,4); 2,114 (1,8); 2,108 (2,1); 2,102 (1,5); 2,096 (0,8); 1,965 (8,9); 1,959 (23,8); 1,953 (126,4); 1,947 (227,5); 1,941 (305,0); 1,935 (212,6); 1,928 (110,8); 1,868 (0,3); 1,781 (0,8); 1,775 (1,4); 1,769 (1,9); 1,763 (1,3); 1,757 (0,7); 1,615 (3,9); 1,600 (10,0); 1,593 (10,8); 1,580 (6,7); 1,571 (2,5); 1,557 (1,3); 1,540 (0,7); 1,410 (0,6); 1,386 (0,4); 1,370 (5,3); 1,356 (10,0); 1,350 (10,4); 1,335 (5,3); 1,325 (2,3); 1,310 (1,1); 1,297 (0,5); 1,285 (0,7); 1,270 (2,4); 1,202 (0,6); 1,134 (16,0); 0,882 (0,4); 0,008 (0,5); 0,000 (15,0); -0,008 (0,8) |
| Beispiel I-T3-201: ¹H-NMR (400,0 MHz, d₆-DMSO): δ= 9,467 (2,2); 8,775 (3,1); 8,578 (1,5); 8,573 (1,5); 8,490 (3,1); 8,177 (1,4); 7,800 (0,6); 7,795 (1,2); 7,787 (1,8); 7,782 (2,4); 7,778 (1,9); 7,578 (1,7); 7,555 (1,5); 3,498 (0,6); 3,479 (1,9); 3,461 (1,9); 3,443 (0,6); 3,330 (198,5); 2,676 (0,7); 2,671 (1,0); 2,667 (0,7); 2,524 (2,5); 2,507 (107,2); 2,502 (141,8); 2,498 (106,1); 2,333 (0,7); 2,329 (0,9); 2,325 (0,7); 1,989 (0,4); 1,614 (0,7); 1,600 (1,8); 1,593 (1,9); 1,580 (0,8); 1,398 (16,0); 1,287 (0,8); 1,274 (1,8); 1,267 (2,0); 1,253 (0,7); 1,235 (0,3); 1,126 (2,1); 1,108 (4,7); 1,089 (2,1); 0,146 (0,9); 0,008 (7,4); 0,000 (188,9); -0,008 (8,7); -0,150 (0,9) |
| Beispiel I-T3-202: ¹H-NMR (400,0 MHz, d₆-DMSO): δ= 9,428 (4,8); 8,626 (0,4); 8,603 (6,6); 8,357 (6,9); 8,316 (0,6); 7,790 (1,3); 7,785 (2,8); 7,777 (4,1); 7,772 (5,1); 7,768 (4,0); 7,562 (3,3); 7,548 (3,9); 7,537 (0,8); 7,525 (3,2); 7,428 (3,2); 3,343 (390,5); 2,991 (1,5); 2,973 (4,8); 2,955 (5,1); 2,937 (1,8); 2,676 (1,0); 2,672 (1,3); 2,668 (1,0); 2,507 (163,2); 2,503 (208,0); 2,499 (152,2); 2,334 (1,0); 2,330 (1,3); 2,325 (0,9); 2,188 (1,0); 2,100 (16,0); 2,075 (0,5); 1,613 (1,6); 1,598 (4,1); 1,592 (4,5); 1,579 (2,0); 1,284 (2,0); 1,270 (4,3); 1,263 (4,5); 1,249 (1,6); 1,232 (0,4); 1,214 (0,7); 1,192 (5,5); 1,173 (11,4); 1,155 (5,2); 0,146 (0,5); 0,008 (4,9); 0,000 (117,1); -0,008 (4,9); -0,150 (0,5) |
| Beispiel I-T3-203: ¹H-NMR (400,0 MHz, d₆-DMSO): δ= 8,601 (0,6); 8,581 (6,8); 8,520 (2,4); 8,509 (2,4); 8,343 (7,1); 8,137 (0,8); 7,732 (1,5); 7,726 (2,4); 7,707 (9,8); 7,561 (3,2); 7,499 (2,9); 7,477 (2,5); 7,427 (3,2); 3,329 (31,4); 2,989 (1,5); 2,971 (5,0); 2,952 (5,1); 2,940 (0,7); 2,934 (1,6); 2,922 (0,4); 2,854 (0,6); 2,844 (0,9); 2,836 (1,4); 2,826 (1,4); 2,818 (1,0); 2,808 (0,7); 2,676 (0,5); 2,671 (0,7); 2,667 (0,5); 2,524 (1,7); 2,511 (39,4); 2,507 (80,4); 2,502 (106,1); 2,498 (78,2); 2,333 (0,5); 2,329 (0,7); 2,324 (0,5); 2,187 (1,2); 2,116 (0,8); 2,101 (16,0); 2,075 (1,0); 1,909 (0,5); 1,230 (0,4); 1,212 (0,9); 1,190 (5,7); 1,172 (11,9); 1,154 (5,4); 0,726 (0,9); 0,713 (2,6); 0,708 (3,6); 0,696 (3,5); 0,690 (3,0); 0,679 (1,3); 0,560 (1,2); 0,550 (3,7); 0,544 (3,5); 0,534 (3,0); 0,522 (0,9); 0,008 (2,2); 0,000 (67,6); -0,008 (2,8) |
| Beispiel I-T3-204: ¹H-NMR (400,0 MHz, d₆-DMSO): δ= 8,968 (6,3); 8,871 (0,8); 8,864 (4,0); 8,858 (3,7); 8,617 (5,8); 8,558 (2,8); 8,554 (2,9); 8,469 (3,0); 8,464 (2,8); 8,379 (3,8); 8,373 (3,6); 8,318 (1,3); 8,261 (0,5); 8,255 (0,6); 4,155 (1,4); 3,332 (210,2); 3,309 (0,8); 3,036 (16,0); 3,014 (1,0); 2,886 (0,9); 2,809 (0,3); 2,798 (0,7); 2,791 (0,8); 2,782 (1,3); 2,772 (1,0); 2,762 (3,0); 2,727 (0,4); 2,676 (0,8); 2,672 (1,1); 2,667 (0,9); 2,541 (0,4); 2,525 (2,7); 2,511 (61,5); 2,507 (127,7); 2,503 (170,1); 2,498 (126,6); 2,494 (64,4); 2,334 (0,8); 2,329 (1,1); 2,325 (0,9); 2,075 (2,2); 1,169 (0,5); 0,836 (0,3); 0,817 (0,4); 0,775 (0,4); 0,608 (0,4); 0,587 (2,1); 0,579 (2,6); 0,570 (1,1); 0,562 (0,6); 0,544 (1,0); 0,532 (2,1); 0,515 (2,1); 0,496 (0,5); 0,146 |
| (0,4); 0,008 (2,9); 0,000 (95,4); -0,008 (4,6); -0,150 (0,4) |
| Beispiel I-T3-205: ¹H-NMR (400,0 MHz, d₆-DMSO): δ= 8,991 (0,5); 8,972 (14,8); 8,876 (0,4); 8,861 (9,9); 8,855 (10,1); 8,716 (4,8); 8,706 (5,3); 8,672 (0,4); 8,661 (0,4); 8,642 (0,8); 8,631 (14,8); 8,562 (7,0); 8,557 (7,2); 8,471 (7,3); 8,467 (6,8); 8,318 (8,7); 8,264 (0,7); 8,258 (0,8); 8,247 (9,6); 8,240 (9,4); 7,948 (0,4); 7,942 (0,4); 7,795 (0,4); 4,156 (4,3); 3,329 (163,2); 3,306 (4,7); 2,887 (0,6); 2,877 (1,4); 2,867 (2,0); 2,859 (3,0); 2,849 (3,2); 2,840 (2,2); 2,830 (1,7); 2,821 (0,8); 2,676 (1,6); 2,671 (2,1); 2,667 (1,6); 2,525 (5,4); 2,507 (239,7); 2,502 (316,6); 2,498 (235,7); 2,333 (1,4); 2,329 (2,0); 2,325 (1,5); 2,076 (16,0); 0,760 (1,9); 0,747 (5,6); 0,743 (7,6); 0,730 (7,4); 0,725 (6,3); 0,713 (2,8); 0,570 (2,3); 0,559 (7,1); 0,553 (7,0); 0,550 (6,7); 0,544 (6,4); 0,532 (2,3); 0,495 (0,4); 0,146 (0,8); 0,008 (5,8); 0,000 (177,1); -0,008 (8,0); -0,150 (0,8) |
| Beispiel I-T3-206: ¹H-NMR (400,0 MHz, d₆-DMSO): δ= 9,449 (4,9); 8,859 (6,6); 8,459 (6,7); 8,318 (4,2); 8,038 (3,0); 7,936 (3,0); 7,803 (3,4); 7,799 (4,7); 7,793 (4,8); 7,787 (3,7); 7,781 (1,6); 7,581 (4,2); 7,570 (0,9); 7,558 (3,6); 3,733 (0,3); 3,690 (0,4); 3,329 (896,7); 3,282 (0,7); 2,981 (1,3); 2,963 (1,5); 2,947 (1,5); 2,929 (1,4); 2,910 (0,5); 2,676 (7,6); 2,671 (10,3); 2,667 (7,7); 2,525 (28,4); 2,511 (589,6); 2,507 (1188,5); 2,502 (1555,2); 2,498 (1146,1); 2,493 (578,6); 2,389 (1,6); 2,370 (1,8); 2,354 (1,8); 2,333 (8,0); 2,329 (10,4); 2,324 (7,7); 2,296 (16,0); 1,909 (0,6); 1,621 (1,6); 1,607 (4,0); 1,600 (4,3); 1,587 (2,0); 1,282 (2,1); 1,269 (3,9); 1,262 (4,2); 1,248 (1,6); 1,147 (0,8); 0,945 (4,8); 0,927 (10,5); 0,908 (4,6); 0,146 (3,6); 0,008 (30,4); 0,000 (880,4); -0,008 (42,5); -0,150 (3,7) |
| Beispiel I-T3-207: ¹H-NMR (400,0 MHz, d₆-DMSO): δ= 9,624 (6,5); 9,586 (0,7); 8,984 (9,3); 8,916 (5,7); 8,910 (5,5); 8,728 (0,7); 8,636 (9,3); 8,571 (1,5); 8,560 (4,6); 8,473 (4,6); 8,469 (4,3); 8,330 (6,1); 8,324 (6,0); 7,943 (0,4); 7,798 (0,4); 7,793 (0,4); 4,156 (3,5); 3,332 (174,7); 3,051 (0,6); 2,875 (0,6); 2,672 (1,1); 2,667 (0,9); 2,507 (117,6); 2,503 (153,3); 2,499 (115,3); 2,330 (1,0); 2,325 (0,7); 2,076 (16,0); 1,648 (2,1); 1,634 (5,4); 1,627 (6,2); 1,614 (2,5); 1,304 (2,4); 1,291 (5,2); 1,284 (5,7); 1,269 (2,1); 1,262 (0,8); 1,254 (0,7); 1,240 (0,3); 0,146 (0,4); 0,008 (2,8); 0,000 (78,1); -0,150 (0,4) |
| Beispiel I-T3-208: ¹H-NMR (400,0 MHz, CD3CN): δ= 8,696 (3,0); 8,690 (3,1); 8,249 (4,2); 8,240 (4,6); 8,003 (3,2); 7,997 (3,1); 7,982 (2,3); 7,978 (2,3); 7,946 (0,5); 7,940 (0,5); 7,766 (2,1); 3,068 (16,0); 2,800 (2,7); 2,783 (0,6); 2,776 (0,6); 2,767 (1,1); 2,756 (0,7); 2,748 (0,6); 2,465 (0,4); 2,170 (94,3); 2,115 (0,5); 2,109 (0,6); 2,102 (0,4); 1,965 (2,7); 1,959 (7,1); 1,954 (39,1); 1,947 (71,0); 1,941 (94,9); 1,935 (64,8); 1,929 (33,1); 1,776 (0,4); 1,770 (0,5); 1,763 (0,4); 0,855 (0,3); 0,789 (0,4); 0,579 (1,4); 0,535 (1,7); 0,525 (1,1); 0,518 (1,6); 0,000 (0,5) |
| Beispiel I-T3-209: ¹H-NMR (400,0 MHz, d₆-DMSO): δ= 9,461 (4,8); 8,713 (6,6); 8,448 (6,8); 8,317 (1,4); 8,281 (3,0); 8,094 (3,1); 7,800 (1,3); 7,794 (2,8); 7,787 (4,1); 7,782 (5,1); 7,778 (4,1); 7,569 (3,8); 7,557 (0,8); 7,546 (3,2); 3,393 (0,4); 3,331 (347,0); 2,676 (2,0); 2,672 (2,7); 2,667 (2,1); 2,524 (7,4); 2,507 (323,5); 2,503 (427,2); 2,498 (324,8); 2,426 (0,4); 2,334 (1,9); 2,329 (2,7); 2,325 (2,1); 2,197 (0,8); 2,160 (16,0); 1,614 (1,5); 1,600 (4,0); 1,593 (4,4); 1,580 (1,8); 1,284 (1,8); 1,270 (4,0); 1,264 (4,4); 1,249 (1,5); 1,147 (0,6); 1,079 (4,6); 1,061 (10,1); 1,042 (4,5); 0,146 (0,9); 0,008 (6,6); 0,000 (197,0); -0,150 (1,0) |
| Beispiel I-T3-210: ¹H-NMR (400,0 MHz, d₆-DMSO): δ= 8,835 (0,4); 8,692 (6,8); 8,552 (2,5); 8,540 (2,5); 8,513 (0,5); 8,435 (7,0); 8,318 (0,5); 8,278 (3,0); 8,094 (3,0); 7,873 (0,4); 7,742 (1,5); 7,736 (2,5); 7,718 (10,2); 7,520 (3,0); 7,502 (1,5); 7,498 (2,5); 3,357 (1,0); 3,329 (76,1); 3,304 (1,0); 2,857 (0,6); 2,847 (0,9); 2,839 (1,4); 2,829 (1,4); 2,820 (0,9); 2,811 (0,7); 2,676 (0,8); 2,671 (1,1); 2,667 (0,9); 2,524 (3,0); 2,511 (66,6); 2,507 (135,2); 2,502 (178,3); 2,498 (130,4); 2,493 (64,6); 2,333 (0,9); 2,329 (1,2); 2,324 (0,9); 2,193 (1,1); 2,159 (16,0); 2,075 (0,6); 1,153 (0,3); 1,135 (0,7); 1,078 (4,8); 1,060 (10,5); 1,041 (4,6); 0,727 (0,9); 0,714 (2,6); 0,709 (3,6); 0,697 (3,5); 0,691 (3,0); 0,680 (1,3); 0,559 (1,2); 0,548 (3,6); 0,542 (3,3); 0,539 (3,1); 0,533 (3,0); 0,521 (0,9); 0,146 (0,4); 0,008 (3,0); 0,000 (92,3); -0,008 (3,7); -0,150 (0,4) |
| Beispiel I-T3-211: ¹H-NMR (400,0 MHz, d₆-DMSO): δ= 8,641 (0,5); 8,541 (0,8); 8,530 (3,2); 8,524 (3,1); 8,504 (5,0); 8,495 (0,7); 8,385 (0,5); 8,318 (1,5); 8,288 (5,2); 8,280 (0,8); 7,971 (3,1); 7,965 (3,0); 7,943 (0,5); 7,803 (0,8); 7,532 (2,9); 7,375 (2,7); 7,322 (0,3); 7,209 (0,3); 4,421 (0,8); 4,404 (2,1); 4,386 (2,3); 4,369 (1,0); 4,240 (1,1); 4,224 (3,6); 4,206 (3,6); 4,189 (1,2); 4,179 (0,5); 4,162 (0,4); 3,741 (0,4); 3,727 (0,4); 3,328 (176,8); 3,027 (0,8); 2,985 (16,0); 2,886 (0,8); 2,775 (0,5); 2,762 (1,0); 2,748 (1,4); 2,734 (1,2); 2,717 (2,0); 2,676 (2,7); 2,671 (3,8); 2,667 (2,8); 2,524 (10,0); 2,510 (212,4); 2,507 (426,0); 2,502 (559,5); 2,498 (412,6); 2,456 (0,7); 2,333 (2,6); 2,329 (3,5); 2,324 (2,7); 2,075 (1,5); 1,361 (0,8); 1,344 (1,7); 1,329 (5,1); 1,312 (10,0); 1,294 (5,0); 1,282 (0,6); 1,229 (0,6); 1,213 (4,5); 1,196 (8,4); 1,178 (4,2); 1,160 (0,4); 1,147 (0,4); 0,788 (0,3); 0,779 (0,4); 0,704 (0,4); 0,469 (4,1); 0,454 (2,9); 0,146 (1,0); 0,008 (7,5); 0,000 (219,5); -0,008 (9,3); -0,150 (1,0) |
| Beispiel I-T3-212: ¹H-NMR (400,0 MHz, d₆-DMSO): δ= 9,450 (2,0); 8,714 (2,7); 8,433 (2,7); 8,220 (1,3); 8,083 (1,3); 7,802 (0,5); 7,796 (1,2); 7,790 (1,7); 7,785 (1,8); 7,780 (1,4); 7,774 (0,6); 7,567 (1,5); 7,556 (0,4); 7,545 (1,3); 3,329 (62,0); 2,675 (0,5); 2,671 (0,7); 2,667 (0,5); 2,506 (78,1); 2,502 (103,1); 2,498 (77,2); 2,329 (0,7); 1,615 (0,6); 1,601 (1,6); 1,594 (1,7); 1,581 (0,7); 1,398 (16,0); 1,287 (0,7); 1,274 (1,6); 1,267 (1,7); 1,253 (0,6); 0,008 (1,3); 0,000 (41,2); -0,008 (1,8) |
| Beispiel I-T3-213: ¹H-NMR (400,0 MHz, d₆-DMSO): δ= 8,692 (2,3); 8,542 (0,8); 8,531 (0,8); 8,417 (2,3); 8,220 (1,0); 8,216 (1,1); 8,079 (1,1); 8,075 (1,0); 7,742 (0,5); 7,737 (0,8); 7,717 (3,3); 7,517 (1,0); 7,496 (0,9); 3,348 (0,4); 3,330 (73,9); 2,839 (0,5); 2,829 (0,5); 2,676 (0,3); 2,671 (0,5); 2,667 (0,4); 2,525 (1,2); 2,520 (1,9); 2,511 (26,1); 2,507 (54,6); 2,502 (72,8); 2,498 (52,8); 2,493 (25,6); 2,333 (0,3); 2,329 (0,5); 2,324 (0,3); 1,398 (16,0); 0,716 (0,8); 0,710 (1,2); 0,698 (1,1); 0,692 (0,9); 0,681 (0,4); 0,562 (0,4); 0,552 (1,2); 0,545 (1,1); 0,536 (0,9); 0,008 (1,1); 0,000 (33,4); -0,009 (1,1) |
| Beispiel I-T3-214: ¹H-NMR (400,0 MHz, CD3CN): δ= 20,020 (0,4); 8,203 (15,1); 8,187 (0,8); 8,173 (15,3); 7,865 (8,4); 7,716 (8,3); 7,711 (10,7); 7,683 (5,4); 7,677 (4,2); 7,662 (6,4); 7,656 (5,5); 7,608 (8,3); 7,528 (0,3); 7,504 (10,0); 7,484 (7,9); 7,016 (4,8); 6,931 (3,0); 6,835 (9,8); 6,653 (5,0); 2,910 (0,6); 2,901 (1,7); 2,891 (2,6); 2,883 (3,9); 2,873 (4,0); 2,865 (2,6); 2,855 (1,8); 2,846 (0,6); 2,174 (590,6); 2,150 (3,3); 2,144 (3,9); 2,138 (4,9); 2,132 (3,3); 2,125 (2,0); 1,995 (20,3); 1,988 (51,7); 1,983 (284,0); 1,977 (520,7); 1,970 (703,8); 1,964 (488,9); 1,958 (254,0); 1,811 (1,6); 1,805 (2,8); 1,799 (4,0); 1,793 (2,8); 1,786 (1,4); 1,467 (16,0); 1,299 (0,7); 0,824 (2,1); 0,811 (6,5); 0,807 (8,8); 0,794 (8,9); 0,789 (6,8); 0,777 (2,9); 0,755 (0,4); 0,737 (0,4); 0,670 (0,4); 0,641 (2,8); 0,629 (8,1); 0,623 (8,3); 0,614 (7,3); 0,602 (2,1); 0,030 (2,9) |
| Beispiel I-T3-215: ¹H-NMR (400,0 MHz, CD3CN): δ= 8,215 (15,4); 8,189 (16,0); 7,866 (9,4); 7,763 (8,8); 7,758 (11,3); 7,732 (5,7); 7,726 (4,7); 7,711 (6,6); 7,705 (5,9); 7,672 (4,3); 7,609 (9,4); 7,536 (9,6); 7,515 (7,8); 7,022 (4,7); 6,840 (9,6); 6,659 (4,7); 4,096 (0,8); 4,079 (0,8); 2,495 (1,5); 2,491 (1,3); 2,206 (651,9); 2,150 |
| (2,6); 2,144 (2,8); 2,138 (3,0); 2,131 (2,4); 2,001 (6,5); 1,994 (14,1); 1,983 (141,8); 1,976 (255,1); 1,970 (340,8); 1,964 (252,4); 1,958 (142,1); 1,811 (1,0); 1,805 (1,6); 1,799 (2,1); 1,793 (1,6); 1,787 (1,0); 1,664 (0,3); 1,626 (4,3); 1,611 (12,2); 1,605 (12,9); 1,591 (6,2); 1,551 (0,7); 1,466 (4,4); 1,431 (0,8); 1,390 (5,7); 1,376 (12,2); 1,370 (13,2); 1,355 (4,7); 1,318 (0,5); 1,297 (1,2); 1,251 (1,0); 1,233 (1,9); 1,215 (1,0); 0,029 (1,1) |
| Beispiel I-T3-216: ¹H-NMR (400,0 MHz, CD3CN): δ= 8,197 (4,6); 8,169 (4,8); 7,865 (3,0); 7,669 (1,3); 7,664 (1,9); 7,644 (6,9); 7,609 (3,0); 7,584 (0,6); 7,579 (0,6); 7,516 (2,2); 7,495 (1,7); 7,023 (1,3); 7,016 (0,4); 6,841 (2,7); 6,835 (0,7); 6,660 (1,3); 3,086 (16,0); 2,794 (3,5); 2,778 (0,8); 2,769 (1,2); 2,759 (0,9); 2,751 (0,7); 2,741 (0,3); 2,184 (18,2); 2,175 (42,1); 2,144 (0,3); 2,138 (0,4); 2,002 (1,1); 1,994 (1,5); 1,988 (3,9); 1,983 (19,8); 1,976 (36,2); 1,970 (48,7); 1,964 (34,4); 1,958 (18,2); 1,466 (8,2); 1,233 (0,5); 0,869 (0,5); 0,852 (0,5); 0,806 (0,6); 0,795 (0,5); 0,598 (1,8); 0,512 (1,7); 0,504 (1,6); 0,495 (1,8) |
| Beispiel I-T3-217: ¹H-NMR (400,0 MHz, CD3CN): δ= 8,724 (2,9); 8,717 (3,2); 8,247 (7,4); 8,240 (1,1); 8,028 (3,1); 8,022 (3,1); 7,971 (0,5); 7,965 (0,5); 7,869 (2,3); 7,615 (2,4); 7,034 (1,5); 6,852 (2,9); 6,846 (0,6); 6,671 (1,5); 3,097 (16,0); 2,828 (2,6); 2,812 (0,6); 2,805 (0,6); 2,797 (1,1); 2,786 (0,7); 2,778 (0,6); 2,179 (56,3); 2,150 (0,4); 2,144 (0,5); 2,138 (0,6); 2,131 (0,4); 1,994 (4,3); 1,988 (6,2); 1,982 (37,6); 1,976 (69,1); 1,970 (93,5); 1,964 (64,7); 1,958 (33,4); 1,805 (0,4); 1,799 (0,6); 1,792 (0,4); 0,817 (0,4); 0,608 (1,4); 0,573 (1,0); 0,565 (1,7); 0,556 (1,1); 0,548 (1,5) |
| Beispiel I-T3-218: ¹H-NMR (400,0 MHz, CD3CN): δ= 20,011 (0,4); 8,773 (8,6); 8,766 (9,0); 8,741 (1,2); 8,735 (1,2); 8,265 (14,9); 8,261 (16,0); 8,253 (3,2); 8,250 (2,9); 8,143 (9,4); 8,137 (9,5); 8,009 (1,3); 8,002 (1,3); 7,870 (7,3); 7,689 (3,7); 7,614 (8,2); 7,029 (4,5); 6,847 (9,1); 6,666 (4,5); 5,477 (0,5); 3,753 (3,4); 3,653 (0,4); 3,636 (0,6); 3,628 (0,6); 3,611 (0,4); 3,327 (0,4); 3,315 (0,8); 3,304 (0,7); 3,098 (0,7); 3,087 (0,6); 2,911 (0,4); 2,168 (284,6); 2,150 (2,8); 2,144 (3,6); 2,138 (4,5); 2,131 (3,1); 2,125 (1,7); 1,994 (21,0); 1,988 (48,8); 1,982 (280,0); 1,976 (513,9); 1,970 (694,8); 1,964 (480,4); 1,958 (247,2); 1,811 (1,5); 1,805 (2,9); 1,799 (4,1); 1,792 (2,9); 1,786 (1,4); 1,644 (3,6); 1,629 (9,1); 1,623 (9,3); 1,609 (4,8); 1,568 (0,6); 1,440 (0,5); 1,399 (4,9); 1,386 (9,0); 1,379 (9,4); 1,364 (3,7); 1,327 (0,4); 1,299 (0,5); 1,164 (0,7); 0,029 (2,4) |
| Beispiel I-T3-219: ¹H-NMR (400,0 MHz, CD3CN): δ= 8,186 (4,3); 8,170 (4,3); 7,971 (2,5); 7,758 (2,2); 7,641 (1,2); 7,636 (1,8); 7,616 (6,7); 7,558 (0,6); 7,552 (0,5); 7,490 (2,0); 7,488 (1,7); 7,483 (0,7); 7,470 (1,3); 7,468 (1,5); 7,462 (0,6); 3,057 (16,0); 2,765 (3,5); 2,755 (0,7); 2,747 (0,7); 2,738 (1,1); 2,728 (0,7); 2,720 (0,6); 2,139 (32,4); 2,114 (0,4); 2,108 (0,5); 2,102 (0,4); 1,965 (2,1); 1,959 (5,3); 1,953 (31,9); 1,947 (58,9); 1,941 (79,9); 1,934 (55,2); 1,928 (28,6); 1,775 (0,3); 1,769 (0,5); 1,437 (6,1); 0,840 (0,4); 0,822 (0,4); 0,777 (0,5); 0,766 (0,4); 0,568 (1,4); 0,482 (1,4); 0,474 (1,3); 0,464 (1,5) |
| Beispiel I-T3-220: ¹H-NMR (400,0 MHz, CD3CN): δ= 8,695 (3,0); 8,689 (3,2); 8,219 (7,0); 8,000 (3,0); 7,994 (3,0); 7,937 (8,6); 7,897 (0,6); 5,449 (12,1); 3,067 (16,0); 2,891 (0,7); 2,869 (0,3); 2,798 (2,8); 2,785 (0,6); 2,778 (0,7); 2,769 (1,2); 2,758 (0,8); 2,750 (0,7); 2,741 (0,4); 2,474 (0,3); 2,469 (0,5); 2,464 (0,4); 2,189 (56,5); 2,121 (0,4); 2,115 (0,4); 2,109 (0,5); 2,103 (0,4); 2,087 (3,4); 1,965 (1,9); 1,959 (4,3); 1,954 (23,2); 1,947 (42,6); 1,941 (57,3); 1,935 (40,0); 1,929 (20,9); 1,770 (0,3); 1,316 (1,1); 1,300 (0,9); 1,285 (0,3); 1,269 (1,0); 0,853 (0,4); 0,834 (0,4); 0,787 (0,4); 0,776 (0,4); 0,578 (1,6); 0,535 (1,9); 0,525 (1,3); 0,518 (1,7); 0,146 (0,6); 0,008 (4,9); 0,000 (131,9); -0,008 (6,9); -0,150 (0,6) |
| Beispiel I-T3-221: ¹H-NMR (400,0 MHz, CD3CN): δ= 8,693 (6,2); 8,687 (6,2); 8,234 (0,7); 8,225 (9,9); 8,212 (10,1); 8,053 (0,4); 8,042 (6,8); 8,036 (6,6); 7,932 (16,0); 7,011 (1,9); 5,447 (15,9); 2,886 (0,5); 2,876 (1,3); 2,867 (1,8); 2,858 (2,8); 2,848 (2,8); 2,840 (1,8); 2,830 (1,3); 2,821 (0,4); 2,149 (16,6); 2,121 (1,4); 2,114 (1,2); 2,108 (1,2); 2,102 (0,9); 2,096 (0,6); 1,965 (6,2); 1,959 (9,3); 1,953 (46,1); 1,947 (83,1); 1,941 (110,8); 1,934 (76,5); 1,928 (39,4); 1,775 (0,5); 1,769 (0,7); 1,763 (0,4); 1,269 (1,0); 1,259 (0,5); 0,809 (1,5); 0,796 (4,5); 0,791 (5,9); 0,778 (6,1); 0,773 (4,5); 0,761 (2,0); 0,620 (2,0); 0,608 (5,1); 0,603 (5,5); 0,599 (4,9); 0,593 (4,7); 0,581 (1,4); 0,146 (1,3); 0,008 (11,5); 0,000 (286,0); -0,009 (12,3); -0,150 (1,3) |
| Beispiel I-T3-222: ¹H-NMR (400,0 MHz, CD3CN): δ= 8,742 (7,6); 8,736 (7,9); 8,237 (11,7); 8,230 (10,9); 8,112 (8,3); 8,106 (8,3); 7,935 (16,0); 7,717 (1,6); 6,777 (0,5); 5,448 (3,1); 2,170 (68,6); 2,121 (0,4); 2,115 (0,6); 2,108 (0,7); 2,102 (0,5); 1,965 (4,0); 1,959 (7,7); 1,953 (42,0); 1,947 (76,9); 1,941 (104,1); 1,935 (72,7); 1,929 (37,9); 1,776 (0,5); 1,769 (0,6); 1,763 (0,5); 1,697 (0,5); 1,612 (3,1); 1,598 (7,6); 1,591 (7,8); 1,577 (4,2); 1,537 (0,5); 1,523 (1,3); 1,505 (1,2); 1,408 (0,5); 1,368 (4,3); 1,355 (7,7); 1,348 (8,0); 1,333 (3,2); 1,277 (0,4); 1,269 (0,9); 1,259 (1,1); 1,193 (0,9); 1,187 (0,6); 1,183 (0,4); 1,177 (1,0); 1,171 (0,6); 1,166 (0,4); 0,146 (1,1); 0,008 (9,5); 0,000 (259,4); -0,009 (11,5); -0,150 (1,1) |
| Beispiel I-T3-223: ¹H-NMR (600,1 MHz, CD3CN): δ= 8,772 (0,8); 8,732 (3,1); 8,728 (3,0); 8,222 (7,7); 8,215 (5,8); 8,200 (0,8); 8,021 (3,2); 8,017 (3,1); 7,943 (13,2); 6,642 (0,5); 4,077 (1,3); 4,065 (3,9); 4,053 (3,9); 4,042 (1,3); 3,752 (0,8); 3,165 (3,1); 3,069 (0,4); 2,934 (16,0); 2,880 (0,4); 2,245 (0,5); 2,240 (0,6); 2,222 (0,8); 2,146 (25,4); 2,078 (1,0); 2,059 (0,8); 2,055 (1,1); 2,050 (1,3); 2,046 (1,0); 2,042 (0,7); 1,972 (17,2); 1,964 (2,0); 1,956 (5,4); 1,952 (7,4); 1,948 (55,6); 1,944 (100,9); 1,940 (146,1); 1,936 (99,1); 1,931 (49,9); 1,833 (0,4); 1,829 (0,7); 1,825 (0,9); 1,821 (0,7); 1,816 (0,4); 1,664 (3,8); 1,661 (4,0); 1,505 (0,4); 1,473 (3,0); 1,443 (0,6); 1,425 (0,6); 1,422 (0,6); 1,409 (0,8); 1,406 (0,8); 1,390 (2,1); 1,388 (1,6); 1,372 (1,4); 1,363 (0,5); 1,358 (0,7); 1,341 (1,7); 1,316 (0,9); 1,303 (0,9); 1,285 (3,5); 1,277 (3,9); 1,271 (6,9); 1,221 (1,2); 1,216 (6,4); 1,214 (5,7); 1,204 (9,5); 1,201 (2,5); 1,192 (4,8); 1,180 (0,6); 1,175 (0,3); 0,948 (0,4); 0,893 (0,9); 0,882 (1,8); 0,870 (1,5); 0,863 (0,9); 0,860 (0,9); 0,856 (0,9); 0,846 (0,9); 0,000 (7,9) |
| Beispiel I-T3-224: ¹H-NMR (400,0 MHz, CD3CN): δ= 8,271 (7,9); 8,267 (7,8); 8,184 (15,8); 8,183 (15,9); 8,152 (0,7); 8,141 (16,0); 7,791 (6,9); 7,693 (9,4); 7,688 (12,0); 7,678 (0,8); 7,662 (6,6); 7,656 (4,7); 7,641 (7,7); 7,635 (6,2); 7,591 (0,7); 7,479 (11,5); 7,458 (9,1); 6,959 (2,7); 4,086 (0,9); 4,068 (2,7); 4,050 (2,8); 4,032 (0,9); 2,986 (0,4); 2,883 (0,8); 2,873 (2,2); 2,864 (3,0); 2,855 (4,6); 2,846 (4,6); 2,837 (2,9); 2,828 (2,2); 2,818 (0,9); 2,567 (0,7); 2,536 (0,3); 2,503 (0,4); 2,477 (1,2); 2,472 (1,9); 2,467 (2,6); 2,462 (1,9); 2,458 (1,1); 2,411 (0,5); 2,398 (0,5); 2,373 (0,6); 2,310 (0,9); 2,281 (1,2); 2,187 (1104,6); 2,121 (0,9); 2,115 (2,0); 2,109 (2,7); 2,103 (1,9); 2,096 (0,9); 1,993 (0,6); 1,973 (14,2); 1,966 (15,1); 1,960 (36,7); 1,954 (209,3); 1,948 (380,7); 1,941 (511,0); 1,935 (347,0); 1,929 (175,8); 1,916 (1,4); 1,782 (1,0); 1,776 (1,9); 1,770 (2,8); 1,764 (1,8); 1,757 (0,8); 1,437 (14,7); 1,340 (0,4); 1,285 (0,8); 1,270 (2,8); 1,222 (3,4); 1,204 (6,6); 1,186 (3,2); 0,882 (0,5); 0,857 (0,5); 0,841 (0,4); 0,796 (2,4); 0,784 (7,0); 0,779 (9,4); 0,766 (9,7); 0,761 (6,9); 0,749 (3,1); 0,727 (0,5); 0,709 (0,4); 0,654 (0,4); 0,644 (0,4); 0,614 (3,2); 0,602 (7,9); 0,597 (8,2); 0,593 (7,3); 0,587 (7,3); 0,575 (2,2); 0,526 (0,3); 0,146 (7,1); 0,138 (0,4); 0,079 (0,4); 0,069 (0,4); 0,066 (0,3); 0,058 (0,5); 0,054 (0,5); 0,049 (0,5); 0,045 (0,6); 0,037 (0,8); 0,023 (2,0); 0,008 (59,7); 0,000 (1582,5); -0,009 (57,5); -0,033 (0,5); -0,036 |
| (0,5); -0,150 (7,0) |
| Beispiel I-T3-225: ¹H-NMR (400,0 MHz, CD3CN): δ= 8,269 (2,4); 8,266 (2,4); 8,170 (4,4); 8,126 (4,2); 7,787 (2,2); 7,644 (1,2); 7,639 (1,7); 7,624 (1,0); 7,618 (5,4); 7,613 (1,7); 7,561 (0,6); 7,556 (0,5); 7,490 (2,1); 7,489 (2,0); 7,482 (0,7); 7,470 (1,6); 7,468 (1,6); 7,462 (0,6); 3,058 (14,9); 3,006 (0,4); 2,778 (0,6); 2,771 (3,2); 2,761 (0,6); 2,753 (0,7); 2,744 (1,1); 2,734 (0,7); 2,726 (0,8); 2,127 (33,1); 2,113 (0,8); 2,106 (0,7); 2,100 (0,5); 1,971 (0,8); 1,963 (2,6); 1,957 (6,7); 1,951 (37,7); 1,945 (68,8); 1,939 (92,9); 1,933 (64,3); 1,927 (33,3); 1,774 (0,4); 1,767 (0,5); 1,761 (0,4); 1,437 (16,0); 1,270 (0,8); 1,204 (0,4); 0,841 (0,5); 0,823 (0,4); 0,780 (0,5); 0,769 (0,4); 0,581 (1,4); 0,575 (1,4); 0,487 (1,5); 0,479 (1,3); 0,470 (1,5); 0,146 (1,1); 0,008 (9,5); 0,000 (262,4); -0,009 (11,4); -0,150 (1,2) |
| Beispiel I-T3-226: ¹H-NMR (400,0 MHz, CD3CN): δ= 8,269 (7,2); 8,266 (7,5); 8,191 (16,0); 8,147 (15,3); 8,056 (0,9); 7,789 (6,5); 7,741 (8,2); 7,735 (10,5); 7,708 (5,2); 7,703 (4,0); 7,687 (6,0); 7,682 (5,1); 7,582 (0,8); 7,554 (4,5); 7,509 (10,0); 7,488 (8,2); 4,084 (0,6); 4,067 (1,9); 4,049 (1,9); 4,032 (0,6); 3,063 (0,7); 3,040 (5,6); 2,902 (4,9); 2,854 (0,6); 2,568 (0,4); 2,126 (170,0); 2,112 (2,8); 2,106 (2,7); 2,100 (1,9); 2,093 (1,0); 2,035 (0,4); 1,970 (10,0); 1,962 (11,5); 1,956 (26,7); 1,951 (152,2); 1,944 (278,1); 1,938 (376,5); 1,932 (259,9); 1,926 (134,2); 1,913 (1,4); 1,779 (0,8); 1,773 (1,5); 1,767 (2,1); 1,761 (1,4); 1,754 (0,7); 1,597 (4,0); 1,583 (10,7); 1,576 (10,4); 1,563 (5,3); 1,523 (0,7); 1,436 (8,2); 1,404 (0,7); 1,364 (5,4); 1,350 (10,4); 1,344 (11,2); 1,329 (4,1); 1,318 (0,3); 1,292 (0,5); 1,269 (2,7); 1,221 (2,2); 1,203 (4,2); 1,185 (2,1); 0,881 (0,4); 0,858 (0,3); 0,145 (5,2); 0,031 (0,9); 0,0071 (35,1); 0,0066 (35,1); -0,001 (985,7); -0,009 (44,0); -0,026 (0,9); -0,040 (0,4); -0,151 (5,1) |
| Beispiel I-T3-227: ¹H-NMR (400,0 MHz, CD3CN): δ= 8,511 (7,8); 8,508 (7,8); 8,174 (16,0); 8,118 (13,4); 8,099 (7,8); 7,694 (9,8); 7,688 (12,4); 7,662 (6,6); 7,656 (5,0); 7,641 (7,7); 7,635 (6,4); 7,499 (0,3); 7,476 (11,6); 7,455 (9,2); 6,938 (2,9); 3,062 (0,6); 2,880 (0,7); 2,870 (2,1); 2,861 (3,0); 2,852 (4,7); 2,843 (4,7); 2,834 (3,0); 2,825 (2,2); 2,815 (0,7); 2,543 (0,4); 2,468 (0,4); 2,463 (0,6); 2,459 (0,4); 2,163 (162,8); 2,120 (1,0); 2,114 (1,0); 2,108 (1,1); 2,102 (0,9); 2,087 (20,5); 1,972 (1,6); 1,965 (5,0); 1,959 (13,4); 1,953 (69,2); 1,947 (125,1); 1,941 (166,4); 1,935 (115,7); 1,928 (59,8); 1,781 (0,4); 1,775 (0,7); 1,769 (1,0); 1,763 (0,6); 1,757 (0,3); 1,285 (0,4); 1,269 (1,7); 1,204 (0,6); 1,186 (0,3); 1,179 (0,8); 0,794 (2,5); 0,781 (7,3); 0,776 (9,8); 0,763 (10,2); 0,758 (7,4); 0,746 (3,4); 0,724 (0,4); 0,707 (0,4); 0,652 (0,4); 0,642 (0,4); 0,612 (3,3); 0,601 (8,5); 0,595 (8,9); 0,591 (8,1); 0,586 (7,9); 0,573 (2,5); 0,536 (0,4); 0,528 (0,3); 0,146 (2,7); 0,029 (0,4); 0,008 (25,1); 0,000 (580,3); -0,009 (29,4); - 0,028 (0,7); -0,150 (2,7) |
| Beispiel I-T3-228: ¹H-NMR (400,0 MHz, CD3CN): δ= 8,508 (2,3); 8,184 (4,7); 8,130 (3,9); 8,099 (2,3); 7,741 (2,6); 7,735 (3,4); 7,711 (1,8); 7,705 (1,3); 7,690 (2,1); 7,684 (1,7); 7,644 (1,3); 7,509 (3,2); 7,488 (2,6); 2,545 (0,6); 2,468 (0,5); 2,463 (0,7); 2,459 (0,5); 2,159 (239,3); 2,119 (0,6); 2,113 (0,8); 2,107 (0,9); 2,101 (0,7); 2,095 (0,4); 1,971 (1,0); 1,964 (3,7); 1,958 (9,3); 1,952 (53,0); 1,946 (96,2); 1,940 (130,0); 1,933 (90,1); 1,927 (46,8); 1,780 (0,3); 1,774 (0,6); 1,768 (0,8); 1,762 (0,6); 1,595 (1,3); 1,581 (3,4); 1,574 (3,5); 1,560 (1,8); 1,437 (16,0); 1,363 (1,8); 1,349 (3,4); 1,343 (3,5); 1,328 (1,4); 1,270 (0,7); 1,204 (0,4); 0,146 (1,3); 0,008 (10,5); 0,000 (282,8); -0,009 (11,9); -0,150 (1,3) |
| Beispiel I-T3-229: ¹H-NMR (400,0 MHz, CD3CN): δ= 8,507 (1,6); 8,161 (3,4); 8,104 (3,1); 7,644 (0,8); 7,639 (1,1); 7,619 (3,9); 7,562 (0,4); 7,557 (0,4); 7,488 (1,4); 7,481 (0,5); 7,466 (1,1); 7,460 (0,4); 3,056 (10,5); 2,855 (0,4); 2,771 (2,3); 2,763 (0,5); 2,755 (0,5); 2,745 (0,8); 2,736 (0,5); 2,728 (0,4); 2,544 (0,4); 2,131 (16,7); 1,971 (0,5); 1,963 (1,1); 1,957 (2,7); 1,951 (15,4); 1,945 (28,4); 1,939 (38,5); 1,933 (26,5); 1,927 (13,7); 1,437 (16,0); 0,779 (0,4); 0,576 (1,0); 0,488 (1,0); 0,480 (0,9); 0,471 (1,0); 0,146 (0,4); 0,008 (3,0); 0,000 (85,2); -0,009 (3,4); -0,150 (0,4) |
| Beispiel I-T4-1: ¹H-NMR (400,0 MHz, CD3CN): δ= 8,170 (4,8); 7,860 (5,7); 7,840 (2,0); 7,833 (1,3); 7,687 (4,2); 7,561 (1,9); 7,539 (1,6); 7,436 (5,5); 6,972 (0,9); 2,871 (0,5); 2,862 (0,8); 2,853 (1,1); 2,843 (1,1); 2,835 (0,8); 2,825 (0,5); 2,251 (24,5); 2,140 (6,0); 1,971 (0,5); 1,964 (0,6); 1,958 (1,4); 1,952 (6,2); 1,946 (11,2); 1,940 (15,0); 1,934 (10,9); 1,928 (5,9); 1,436 (16,0); 0,796 (0,6); 0,784 (1,9); 0,779 (2,5); 0,766 (2,6); 0,761 (2,0); 0,749 (0,9); 0,619 (0,8); 0,608 (2,3); 0,601 (2,5); 0,592 (2,1); 0,580 (0,6); 0,000 (15,7) |
| Beispiel I-T4-2: ¹H-NMR (400,0 MHz, CD3CN): δ= 8,184 (6,7); 8,183 (6,5); 7,920 (2,5); 7,915 (8,6); 7,894 (3,0); 7,887 (1,9); 7,699 (6,2); 7,680 (1,6); 7,602 (3,0); 7,600 (2,8); 7,582 (2,4); 7,580 (2,6); 7,438 (7,8); 2,463 (0,3); 2,253 (39,2); 2,151 (134,8); 2,120 (0,5); 2,114 (0,7); 2,108 (0,9); 2,102 (0,6); 2,095 (0,4); 1,972 (1,2); 1,965 (4,2); 1,959 (10,5); 1,953 (54,2); 1,947 (98,7); 1,940 (132,9); 1,934 (93,2); 1,928 (48,4); 1,775 (0,6); 1,769 (0,8); 1,763 (0,6); 1,599 (1,8); 1,585 (4,6); 1,578 (4,7); 1,564 (2,4); 1,437 (16,0); 1,415 (0,4); 1,375 (2,4); 1,361 (4,7); 1,354 (4,9); 1,340 (1,8); 1,269 (1,5); 1,204 (0,4); 0,146 (0,7); 0,008 (5,3); 0,000 (150,3); -0,008 (7,3); -0,149 (0,7) |
| Beispiel I-T4-3: ¹H-NMR (400,0 MHz, CD3CN): δ= 8,929 (2,4); 8,922 (2,4); 8,231 (2,7); 8,224 (2,6); 8,205 (4,3); 7,754 (3,8); 7,444 (5,0); 7,072 (0,7); 2,881 (0,4); 2,872 (0,7); 2,863 (1,0); 2,853 (1,0); 2,845 (0,7); 2,835 (0,4); 2,252 (22,9); 2,140 (16,7); 1,964 (1,1); 1,952 (13,4); 1,946 (23,9); 1,940 (31,3); 1,934 (21,8); 1,928 (11,3); 1,436 (16,0); 0,814 (0,5); 0,800 (1,8); 0,796 (2,2); 0,783 (2,3); 0,778 (1,7); 0,766 (0,7); 0,633 (0,7); 0,622 (2,1); 0,616 (2,2); 0,612 (2,0); 0,607 (1,8); 0,594 (0,5); 0,146 (0,6); 0,000 (113,1); -0,150 (0,6) |
| Beispiel I-T4-4: ¹H-NMR (400,0 MHz, CD3CN): δ= 8,974 (3,9); 8,967 (3,9); 8,300 (4,1); 8,294 (4,0); 8,216 (6,7); 7,764 (6,3); 7,753 (0,6); 7,735 (1,8); 7,446 (7,8); 4,067 (0,9); 4,050 (0,9); 3,076 (1,0); 2,898 (1,0); 2,254 (38,8); 2,144 (39,1); 2,114 (0,6); 2,107 (0,6); 2,101 (0,4); 2,095 (0,4); 2,086 (0,4); 2,063 (0,4); 1,972 (4,1); 1,964 (2,3); 1,958 (6,0); 1,952 (30,2); 1,946 (53,9); 1,940 (71,6); 1,934 (48,9); 1,928 (25,1); 1,768 (0,4); 1,617 (1,7); 1,602 (4,5); 1,595 (4,4); 1,581 (2,2); 1,437 (16,0); 1,388 (2,3); 1,375 (4,5); 1,368 (4,5); 1,353 (1,7); 1,269 (0,9); 1,221 (1,0); 1,204 (2,0); 1,186 (1,0); 0,146 (1,1); 0,008 (8,7); 0,000 (211,3); -0,009 (8,7); -0,150 (1,1) |
| Beispiel I-T22-1: ¹H-NMR (400,0 MHz, CD3CN): δ= 7,934 (2,4); 7,928 (3,4); 7,908 (1,9); 7,902 (1,3); 7,887 (2,0); 7,882 (1,6); 7,615 (3,0); 7,594 (2,6); 7,486 (5,3); 7,037 (0,7); 6,864 (0,4); 6,858 (6,5); 2,877 (0,5); 2,867 (0,8); 2,858 (1,2); 2,849 (1,2); 2,840 (0,8); 2,831 (0,6); 2,258 (25,0); 2,168 (12,6); 1,965 (0,4); 1,959 (1,0); 1,953 (5,8); 1,947 (10,6); 1,941 (14,3); 1,935 (10,0); 1,928 (5,3); 1,436 (16,0); 0,800 (0,6); 0,788 (1,8); 0,783 (2,5); 0,770 (2,6); 0,765 (1,9); 0,753 (0,9); 0,624 (0,9); 0,613 (2,2); 0,606 (2,3); 0,602 (2,0); 0,597 (2,0); 0,585 (0,7); 0,008 (0,7); 0,000 (20,3); -0,009 (0,9) |
| Beispiel I-T22-2: ¹H-NMR (400,0 MHz, CD3CN): δ= 7,992 (6,1); 7,986 (8,0); 7,960 (4,3); 7,954 (3,3); 7,939 (4,6); 7,933 (4,0); 7,709 (2,2); 7,654 (7,3); 7,633 (6,4); 7,487 (13,6); 6,877 (16,0); 5,448 (2,4); 2,418 (0,4); 2,260 (66,3); 2,153 (49,7); 2,120 (0,4); 2,114 (0,4); 2,108 (0,5); 2,098 (0,5); 2,086 (1,9); 1,972 (0,6); 1,964 |
| (1,9); 1,958 (4,9); 1,953 (28,0); 1,946 (51,5); 1,940 (70,2); 1,934 (49,9); 1,928 (27,0); 1,775 (0,3); 1,769 (0,4); 1,603 (3,1); 1,588 (8,0); 1,581 (8,3); 1,568 (4,3); 1,528 (0,5); 1,436 (0,9); 1,419 (0,5); 1,379 (4,2); 1,365 (8,0); 1,359 (8,7); 1,344 (3,3); 1,268 (0,4); 0,146 (0,5); 0,008 (3,8); 0,000 (117,9); -0,008 (7,0); -0,150 (0,6) |
| Beispiel I-T22-3: ¹H-NMR (400,0 MHz, CD3CN): δ= 7,982 (3,5); 7,977 (5,3); 7,966 (3,0); 7,960 (1,9); 7,945 (2,9); 7,939 (2,5); 7,669 (4,3); 7,648 (3,7); 7,487 (9,9); 6,883 (8,4); 4,152 (1,0); 4,136 (1,1); 4,129 (3,0); 4,112 (3,1); 4,105 (3,3); 4,089 (3,1); 4,082 (1,4); 4,065 (1,1); 2,262 (40,4); 2,156 (20,4); 2,101 (0,4); 1,972 (0,5); 1,964 (1,0); 1,958 (2,4); 1,953 (12,4); 1,946 (23,1); 1,940 (31,5); 1,934 (23,1); 1,928 (12,8); 1,436 (16,0); 0,008 (2,0); 0,000 (53,5) |
| Beispiel I-T22-4: ¹H-NMR (400,0 MHz, CD3CN): δ= 8,983 (1,2); 8,977 (1,3); 8,360 (1,3); 8,355 (1,3); 7,953 (1,3); 7,944 (1,4); 7,036 (2,1); 2,306 (6,0); 2,160 (8,1); 1,953 (4,2); 1,947 (7,8); 1,941 (10,6); 1,935 (7,9); 1,929 (4,3); 1,619 (0,6); 1,604 (1,6); 1,597 (1,6); 1,584 (0,8); 1,437 (16,0); 1,390 (0,8); 1,376 (1,6); 1,369 (1,7); 1,354 (0,6) |
| Beispiel I-T22-5: ¹H-NMR (400,0 MHz, CD3CN): δ= 7,942 (4,2); 7,936 (5,2); 7,918 (1,6); 7,912 (1,1); 7,897 (1,6); 7,892 (1,3); 7,619 (2,4); 7,598 (2,1); 7,045 (0,8); 6,922 (4,2); 2,874 (0,4); 2,865 (0,7); 2,856 (1,0); 2,847 (1,0); 2,838 (0,7); 2,828 (0,4); 2,305 (10,8); 2,183 (27,7); 1,960 (0,8); 1,954 (3,8); 1,948 (6,9); 1,942 (9,3); 1,936 (6,5); 1,930 (3,4); 1,436 (16,0); 0,799 (0,5); 0,786 (1,7); 0,781 (2,2); 0,769 (2,2); 0,764 (1,7); 0,751 (0,7); 0,624 (0,7); 0,613 (2,1); 0,607 (2,1); 0,603 (2,0); 0,597 (1,8); 0,585 (0,5); 0,000 (23,8) |
| Beispiel I-T22-6: ¹H-NMR (400,0 MHz, CD3CN): δ= 8,939 (1,4); 8,933 (1,4); 8,292 (1,5); 8,286 (1,4); 7,952 (1,1); 7,942 (1,1); 7,150 (0,3); 7,022 (2,3); 2,874 (0,4); 2,865 (0,5); 2,855 (0,5); 2,847 (0,4); 2,305 (5,7); 2,187 (7,7); 1,973 (0,7); 1,960 (0,4); 1,954 (2,8); 1,948 (5,1); 1,942 (7,0); 1,936 (4,8); 1,930 (2,5); 1,436 (16,0); 1,204 (0,3); 0,803 (0,9); 0,798 (1,1); 0,785 (1,2); 0,780 (0,9); 0,767 (0,4); 0,636 (0,4); 0,624 (1,0); 0,619 (1,1); 0,615 (0,9); 0,609 (0,9); 0,000 (5,3) |
| Beispiel I-T22-7: ¹H-NMR (400,0 MHz, CD3CN): δ= 7,999 (1,6); 7,994 (2,1); 7,970 (1,1); 7,965 (0,8); 7,949 (2,6); 7,944 (2,6); 7,937 (1,8); 7,659 (2,2); 7,638 (1,6); 6,939 (3,3); 2,306 (8,4); 2,155 (9,0); 1,972 (0,6); 1,965 (0,3); 1,953 (5,6); 1,947 (10,3); 1,941 (14,0); 1,935 (9,7); 1,929 (5,0); 1,602 (0,8); 1,587 (2,1); 1,581 (2,1); 1,567 (1,1); 1,436 (16,0); 1,380 (1,1); 1,366 (2,1); 1,360 (2,2); 1,345 (0,8) |
| Beispiel I-T23-1: ¹H-NMR (400,0 MHz, CD3CN): δ= 7,961 (5,3); 7,958 (2,0); 7,942 (1,7); 7,937 (1,0); 7,864 (5,1); 7,609 (1,6); 7,606 (1,0); 7,590 (1,0); 7,587 (1,4); 7,148 (5,1); 6,968 (0,5); 2,876 (0,4); 2,867 (0,6); 2,858 (0,9); 2,849 (0,9); 2,840 (0,6); 2,831 (0,4); 2,134 (6,5); 1,964 (1,6); 1,958 (2,5); 1,952 (11,3); 1,946 (20,0); 1,940 (26,1); 1,934 (17,8); 1,928 (9,0); 1,437 (16,0); 0,800 (0,5); 0,787 (1,5); 0,782 (1,9); 0,770 (2,0); 0,764 (1,4); 0,752 (0,7); 0,624 (0,7); 0,614 (1,6); 0,606 (1,7); 0,602 (1,5); 0,597 (1,5); 0,584 (0,5); 0,000 (0,7) |
| Beispiel I-T23-2: ¹H-NMR (400,0 MHz, CD3CN): δ= 8,014 (3,3); 8,010 (1,3); 7,997 (1,1); 7,991 (0,6); 7,866 (3,6); 7,648 (1,5); 7,642 (0,8); 7,630 (0,6); 7,625 (1,2); 7,162 (3,0); 5,447 (16,0); 2,140 (12,0); 1,972 (0,4); 1,964 (2,4); 1,958 (4,0); 1,952 (17,2); 1,946 (30,2); 1,940 (39,3); 1,934 (26,8); 1,928 (13,7); 1,600 (0,6); 1,586 (1,7); 1,579 (1,7); 1,565 (0,9); 1,437 (0,9); 1,380 (0,9); 1,367 (1,6); 1,360 (1,7); 1,345 (0,7); 0,000 (0,9) |
| Beispiel I-T46-1: ¹H-NMR (400,0 MHz, CD3CN): δ= 7,669 (6,3); 7,664 (9,6); 7,654 (5,5); 7,648 (2,9); 7,633 (5,5); 7,627 (4,3); 7,582 (3,1); 7,509 (16,0); 7,426 (8,2); 7,405 (6,8); 7,166 (4,7); 7,161 (8,3); 7,156 (5,0); 6,767 (3,7); 6,760 (7,3); 6,754 (5,5); 6,735 (5,6); 6,730 (6,3); 6,723 (3,8); 2,468 (0,7); 2,463 (1,0); 2,459 (0,7); 2,298 (0,5); 2,161 (388,0); 2,139 (78,8); 2,121 (1,2); 2,114 (1,5); 2,108 (1,7); 2,102 (1,2); 2,096 (0,7); 1,993 (0,7); 1,977 (0,9); 1,965 (8,8); 1,959 (16,3); 1,953 (98,1); 1,947 (179,6); 1,941 (243,9); 1,935 (167,4); 1,928 (86,0); 1,856 (0,9); 1,842 (0,6); 1,782 (0,6); 1,775 (1,1); 1,769 (1,5); 1,763 (1,0); 1,757 (0,5); 1,585 (3,5); 1,570 (9,4); 1,563 (9,3); 1,550 (4,6); 1,510 (0,5); 1,394 (0,5); 1,354 (4,7); 1,340 (9,4); 1,333 (9,8); 1,319 (3,5); 0,146 (0,4); 0,008 (2,9); 0,000 (87,8); -0,008 (3,4); -0,150 (0,4) |
| Beispiel I-T46-2: ¹H-NMR (400,0 MHz, CD3CN): δ= 7,682 (3,3); 7,677 (6,3); 7,676 (6,2); 7,673 (6,2); 7,668 (4,7); 7,665 (5,9); 7,653 (5,1); 7,647 (7,8); 7,628 (1,3); 7,623 (1,7); 7,606 (1,1); 7,600 (0,7); 7,585 (1,1); 7,579 (1,0); 7,518 (4,3); 7,517 (4,5); 7,507 (3,5); 7,496 (6,1); 7,478 (6,1); 7,475 (3,5); 7,459 (3,7); 7,422 (2,5); 7,419 (1,8); 7,409 (1,0); 7,403 (2,8); 7,394 (1,8); 7,385 (1,0); 7,373 (1,2); 7,153 (0,9); 7,148 (1,6); 7,143 (1,1); 6,961 (0,8); 6,900 (0,5); 6,760 (0,8); 6,753 (1,5); 6,747 (1,1); 6,724 (1,1); 6,720 (1,3); 6,717 (1,2); 6,713 (0,9); 3,855 (0,7); 3,051 (1,3); 2,881 (0,4); 2,871 (1,1); 2,862 (1,6); 2,853 (2,4); 2,843 (3,0); 2,834 (1,8); 2,826 (1,3); 2,816 (0,6); 2,476 (0,8); 2,472 (1,3); 2,467 (1,8); 2,462 (1,4); 2,427 (0,5); 2,391 (0,4); 2,383 (0,3); 2,359 (0,4); 2,327 (0,5); 2,182 (852,2); 2,138 (15,6); 2,121 (1,5); 2,115 (2,2); 2,109 (2,8); 2,102 (2,4); 2,096 (1,2); 1,992 (0,9); 1,966 (9,9); 1,959 (26,3); 1,954 (166,7); 1,947 (309,9); 1,941 (423,2); 1,935 (291,5); 1,929 (148,5); 1,782 (0,9); 1,776 (1,7); 1,770 (2,4); 1,764 (1,7); 1,757 (0,9); 1,437 (16,0); 1,270 (1,6); 0,790 (1,1); 0,778 (3,3); 0,773 (4,8); 0,766 (2,0); 0,760 (4,8); 0,755 (4,0); 0,743 (1,7); 0,736 (0,5); 0,614 (1,5); 0,602 (4,2); 0,593 (4,9); 0,587 (4,7); 0,575 (1,8); 0,000 (1,7) |
| Beispiel I-T46-3: ¹H-NMR (400,0 MHz, CD3CN): δ= 8,649 (2,9); 8,643 (3,2); 7,935 (2,9); 7,929 (2,9); 7,873 (7,9); 7,322 (1,5); 7,318 (2,7); 7,313 (1,9); 6,923 (1,5); 6,917 (2,0); 6,916 (2,3); 6,910 (1,9); 6,802 (1,7); 6,798 (2,2); 6,795 (2,0); 6,790 (1,9); 3,058 (16,0); 2,790 (2,7); 2,783 (0,4); 2,772 (0,6); 2,765 (0,6); 2,757 (1,2); 2,745 (0,7); 2,738 (0,6); 2,170 (18,1); 1,966 (1,0); 1,960 (2,0); 1,954 (11,8); 1,948 (21,8); 1,941 (29,7); 1,935 (20,7); 1,929 (10,7); 1,269 (0,6); 0,844 (0,3); 0,826 (0,4); 0,783 (0,4); 0,573 (1,4); 0,528 (1,7); 0,518 (1,1); 0,511 (1,6); 0,000 (7,0); -0,008 (0,3) |
| Beispiel I-T46-4: ¹H-NMR (400,0 MHz, CD3CN): δ= 8,649 (6,8); 8,643 (7,0); 7,990 (0,3); 7,976 (7,4); 7,970 (7,3); 7,871 (16,0); 7,320 (4,1); 7,315 (6,6); 7,310 (4,1); 6,976 (1,8); 6,920 (3,8); 6,914 (4,9); 6,912 (5,2); 6,907 (4,0); 6,805 (4,5); 6,801 (4,8); 6,798 (4,5); 6,794 (3,8); 5,448 (0,6); 2,876 (0,4); 2,867 (1,2); 2,857 (1,7); 2,849 (2,7); 2,839 (2,8); 2,831 (1,8); 2,821 (1,3); 2,811 (0,4); 2,143 (54,7); 2,114 (0,4); 2,108 (0,4); 1,965 (2,0); 1,959 (5,3); 1,953 (26,5); 1,947 (48,2); 1,941 (64,2); 1,934 (44,7); 1,928 (23,1); 1,769 (0,4); 1,269 (0,9); 1,200 (0,4); 0,799 (1,4); 0,786 (4,5); 0,781 (5,9); 0,769 (6,1); 0,763 (4,5); 0,751 (1,9); 0,614 (1,9); 0,602 (5,1); 0,597 (5,6); 0,593 (5,0); 0,587 (4,8); 0,575 (1,4); 0,008 (0,5); 0,000 (15,3) |
| Beispiel I-T46-5: ¹H-NMR (400,0 MHz, CD3CN): δ= 8,693 (7,0); 8,687 (7,3); 8,034 (7,5); 8,028 (7,5); 7,873 (16,0); 7,693 (1,3); 7,335 (3,9); 7,330 (6,8); 7,325 (4,4); 6,927 (3,7); 6,922 (4,7); 6,920 (5,0); 6,914 (4,3); 6,816 (4,3); 6,812 (4,8); 6,808 (4,4); 6,804 (3,9); 5,449 (14,1); 2,173 (58,5); 2,115 (0,4); 2,109 (0,5); 2,103 (0,4); 1,966 (2,9); 1,960 (5,1); 1,954 (29,3); 1,948 (53,8); 1,941 (72,9); 1,935 (50,8); 1,929 (26,4); 1,776 (0,3); 1,770 (0,4); 1,764 (0,3); 1,602 |
| (2,9); 1,588 (7,3); 1,581 (7,4); 1,567 (3,9); 1,551 (0,4); 1,523 (0,7); 1,505 (0,6); 1,405 (0,5); 1,365 (4,1); 1,351 (7,2); 1,345 (7,6); 1,330 (3,0); 1,269 (0,9); 1,259 (0,5); 1,200 (0,6); 1,193 (0,4); 1,187 (0,4); 1,177 (0,5); 1,171 (0,4); 0,008 (0,4); 0,000 (13,0) |
| Beispiel I-T46-6: ¹H-NMR (400,0 MHz, CD3CN): δ= 8,729 (1,0); 8,692 (3,0); 8,687 (3,0); 8,145 (1,0); 7,954 (3,0); 7,949 (3,0); 7,871 (14,1); 7,325 (3,5); 7,071 (0,6); 6,926 (2,7); 6,920 (3,2); 6,914 (2,1); 6,808 (2,6); 6,803 (2,8); 6,643 (1,1); 6,496 (1,1); 3,751 (1,8); 3,659 (0,8); 3,649 (0,9); 3,643 (2,1); 3,624 (1,8); 3,159 (4,2); 3,076 (1,9); 3,062 (0,3); 2,927 (16,0); 2,905 (0,3); 2,887 (0,3); 2,874 (0,3); 2,240 (1,1); 2,176 (137,5); 2,121 (0,6); 2,115 (0,7); 2,109 (0,7); 2,103 (0,6); 2,097 (0,4); 1,966 (2,5); 1,960 (6,5); 1,954 (35,6); 1,948 (65,4); 1,942 (88,3); 1,935 (62,6); 1,929 (33,5); 1,819 (1,0); 1,811 (1,1); 1,803 (2,6); 1,794 (1,2); 1,786 (1,1); 1,776 (0,6); 1,770 (0,8); 1,764 (0,6); 1,758 (0,4); 1,698 (0,4); 1,653 (4,1); 1,599 (0,4); 1,586 (0,3); 1,548 (0,4); 1,541 (0,4); 1,523 (0,9); 1,505 (1,0); 1,468 (3,4); 1,453 (1,6); 1,415 (1,4); 1,405 (1,4); 1,389 (9,5); 1,358 (1,0); 1,340 (1,1); 1,315 (1,4); 1,303 (3,2); 1,285 (4,6); 1,270 (11,8); 1,221 (12,0); 1,214 (12,0); 1,200 (3,3); 1,193 (1,3); 1,190 (1,5); 1,177 (1,0); 1,172 (1,1); 1,161 (0,7); 1,121 (0,6); 1,107 (0,6); 1,093 (0,9); 1,057 (0,7); 0,974 (0,4); 0,957 (0,5); 0,947 (0,6); 0,934 (0,5); 0,923 (0,7); 0,898 (1,3); 0,882 (3,1); 0,876 (2,5); 0,858 (2,8); 0,840 (1,7); 0,815 (0,6); 0,000 (3,4) |

| |
|---|
| ¹⁾ Bei der angegebenen Masse handelt es sich um den Peak des Isotopenmusters des [M+H]⁺ Ions mit der höchsten Intensität; falls das [M-H]⁻ Ion detektiert wurde, ist die Massenangabe mit ² gekennzeichnet. ²⁾ Bei der angegebenen Masse handelt es sich um den Peak des Isotopenmusters des [M-H]⁻ Ions mit der höchsten Intensität. ^{a)} Anmerkung zur Bestimmung der logP-Werte und Massendetektion: Die Bestimmung der angegebenen logP-Werte erfolgte gemäß EEC-Direktive 79/831 Annex V.A8 durch HPLC (High Performance Liquid Chromatography) an einer Phasenumkehrsäule (C18).Agilent 1100 LC-System; 50*4,6 Zorbax Eclipse Plus C18 1,8 microm; Eluent A: Acetonitril (0,1 % Ameisensäure); Eluent B: Wasser (0,09 % Ameisensäure); linearer Gradient von 10 % Acetonitril bis 95 % Acetonitril in 4,25 min, dann 95% Acetonitril für weitere 1,25 min; Ofentemperatur 55°C; Fluß:2,0 mL/min. Die Massendetektion erfolgt über ein Agilent MSD-System. |

### Biologische Beispiele

### Rhipicephalus sanguineus - in-vitro Kontakttests mit Adulten der braunen Hundezecke

Für die Beschichtung der Teströhrchen werden zunächst 9 mg Wirkstoff in 1 ml Aceton p.a. gelöst und anschließend mit Aceton p.a. auf die gewünschte Konzentration verdünnt. 250 µl der Lösung werden durch Drehen und Kippen auf einem Rotationsschüttler (2 h Schaukelrotation bei 30 rpm) homogen auf den Innenwänden und dem Boden eines 25ml Glasröhrchens verteilt. Bei 900 ppm Wirkstofflösung und 44,7 cm² Innenoberfläche wird bei homogener Verteilung eine Flächendosis von 5 µg/cm² erreicht.

Nach Abdampfen des Lösungsmittels werden die Gläschen mit 5-10 adulten Hundezecken (*Rhipicephalus sanguineus*) besetzt, mit einem gelochten Kunststoffdeckel verschlossen und liegend im Dunkeln bei Raumtemperatur und Umgebungsfeuchte inkubiert. Nach 48 h wird die Wirksamkeit bestimmt. Hierzu werden die Zecken auf den Boden des Gläschens geklopft und auf einer Wärmeplatte bei 45-50°C maximal 5 min. inkubiert. Zecken, die unbeweglich auf dem Boden verbleiben oder sich so unkoordiniert bewegen, dass sie nicht gezielt der Wärme durch nach oben klettern ausweichen können, gelten als tot bzw. angeschlagen.

Eine Substanz zeigt gute Wirkung gegen *Rhipicephalus sanguineus,* wenn in diesem Test bei einer Aufwandmenge von 5 µg/cm² mindestens 80% Wirkung erzielt wurde. Dabei bedeutet 100% Wirkung, dass alle Zecken angeschlagen oder tot waren. 0% Wirkung bedeutet, dass keine Zecken geschädigt wurden.

Bei diesem Test zeigen z.B. die folgenden Verbindungen der Herstellungsbeispiele eine Wirkung von 100% bei einer Aufwandmenge von 5 µg/cm²: I-T3-1, I-T3-3, I-T3-20, I-T3-21, I-T3-23, I-T3-24, I-T3-42, I-T3-44, I-T3-46, I-T3-47, I-T3-52, I-T3-53, I-T3-54, I-T3-55, I-T3-56, I-T3-61, I-T3-63, I-T3-71, I-T3-72, I-T3-81, I-T3-90, I-T3-91, I-T3-96, I-T3-97, I-T3-98, I-T3-104, I-T3-106, I-T3-109, I-T3-110, I-T3-112, I-T3-117, I-T3-119, I-T3-148, I-T3-155, I-T3-160, I-T3-161, I-T3-162, I-T3-163, I-T3-165, I-T3-175, I-T3-176, I-T3-189, I-T3-196, I-T4-1, I-T4-2, I-T4-3, I-T4-4, I-T22-2, I-T22-1, I-T22-4, I-T22-5, I-T22-6, I-T22-7

Bei diesem Test zeigen z.B. die folgenden Verbindungen der Herstellungsbeispiele eine Wirkung von 80% bei einer Aufwandmenge von 5 µg/cm²: I-T3-38, I-T3-43, I-T3-80, I-T3-88, I-T3-92, I-T3-143

Bei diesem Test zeigen z.B. die folgenden Verbindungen der Herstellungsbeispiele eine Wirkung von 100% bei einer Aufwandmenge von 1 µg/cm²: I-T3-108, I-T3-114, I-T3-141

Bei diesem Test zeigen z.B. die folgenden Verbindungen der Herstellungsbeispiele eine Wirkung von 80% bei einer Aufwandmenge von 1 µg/cm²: I-T3-94, I-T3-123

Bei diesem Test zeigen z.B. die folgenden Verbindungen der Herstellungsbeispiele eine Wirkung von 100% bei einer Aufwandmenge von 0,2 µg/cm²: I-T3-105

Bei diesem Test zeigen z.B. die folgenden Verbindungen der Herstellungsbeispiele eine Wirkung von 80% bei einer Aufwandmenge von 0,2 µg/cm²: I-T3-64

### Ctenocephalides felis - in-vitro Kontakttests mit adulten Katzenflöhen

Für die Beschichtung der Teströhrchen werden zunächst 9 mg Wirkstoff in 1 ml Aceton p.a. gelöst und anschließend mit Aceton p.a. auf die gewünschte Konzentration verdünnt. 250 µl der Lösung werden durch Drehen und Kippen auf einem Rotationsschüttler (2 h Schaukelrotation bei 30 rpm) homogen auf den Innenwänden und dem Boden eines 25ml Glasröhrchens verteilt. Bei 900 ppm Wirkstofflösung und 44,7 cm² Innenoberfläche wird bei homogener Verteilung eine Flächendosis von 5 µg/cm² erreicht.

Nach Abdampfen des Lösungsmittels werden die Gläschen mit 5-10 adulten Katzenflöhen (*Ctenocephalides felis*) besetzt, mit einem gelochten Kunststoffdeckel verschlossen und liegend bei Raumtemperatur und Umgebungsfeuchte inkubiert. Nach 48 h wird die Wirksamkeit bestimmt. Hierzu werden die Gläschen aufrecht gestellt und die Flöhe auf den Boden des Gläschens geklopft. Flöhe, die unbeweglich auf dem Boden verbleiben oder sich unkoordiniert bewegen, gelten als tot bzw. angeschlagen.

Eine Substanz zeigt gute Wirkung gegen *Ctenocephalides felis,* wenn in diesem Test bei einer Aufwandmenge von 5 µg/cm² mindestens 80% Wirkung erzielt wurde. Dabei bedeutet 100% Wirkung, dass alle Flöhe angeschlagen oder tot waren. 0% Wirkung bedeutet, dass keine Flöhe geschädigt wurden.

Bei diesem Test zeigen z.B. die folgenden Verbindungen der Herstellungsbeispiele eine Wirkung von 100% bei einer Aufwandmenge von 5 µg/cm² (= 500g/ha): I-T3-1, I-T3-3, I-T3-7, I-T3-9, I-T3-17, I-T3-20, I-T3-21, I-T3-23, I-T3-24, I-T3-25, I-T3-27, I-T3-28, I-T3-29, I-T3-30, I-T3-31, I-T3-42, I-T3-43, I-T3-44, I-T3-46, I-T3-54, I-T3-55, I-T3-56, I-T3-57, I-T3-61, I-T3-63, I-T3-64, I-T3-71, I-T3-72, I-T3-80, I-T3-81, I-T3-84, I-T3-85, I-T3-86, I-T3-87, I-T3-88, I-T3-91, I-T3-92, I-T3-93, I-T3-94, I-T3-95, I-T3-96, I-T3-97, I-T3-98, I-T3-99, I-T3-100, I-T3-101, I-T3-102, I-T3-103, I-T3-106, I-T3-107, I-T3-108, I-T3-109, I-T3-110, I-T3-111, I-T3-112, I-T3-113, I-T3-114, I-T3-115, I-T3-116, I-T3-117, I-T3-118, I-T3-119, I-T3-120, I-T3-123, I-T3-124, I-T3-125, I-T3-127, I-T3-128, I-T3-129, I-T3-130, I-T3-131, I-T3-132, I-T3-133, I-T3-136, I-T3-137, I-T3-138, I-T3-143, I-T3-145, I-T3-147, I-T3-148, I-T3-155, I-T3-160, I-T3-162, I-T3-163, I-T3-165, I-T3-175, I-T3-176, I-T3-189, I-T3-196, I-T3-199, I-T4-2, I-T4-3, I-T4-4, I-T22-1, I-T22-2, I-T22-3, I-T22-5, I-T22-7, I-T23-1, I-T23-2, I-T46-2

### Amblyomma hebaraeum -Test

### Lösungsmittel: Dimethylsulfoxid

Zur Herstellung einer zweckmäßigen Wirkstoffzubereitung vermischt man 10 mg Wirkstoff mit 0,5 ml Dimethylsulfoxid und verdünnt das Konzentrat mit Wasser auf die gewünschte Konzentration.

Zeckennymphen (*Amblyomma hebraeum*) werden in perforierte Plastikbecher gesetzt und in der gewünschten Konzentration eine Minute getaucht. Die Zecken werden auf Filterpapier in eine Petrischale überführt und in einem Klimaschrank gelagert.

Nach 42 Tagen wird die Abtötung in % bestimmt. Dabei bedeutet 100 %, dass alle Zecken abgetötet wurden; 0 % bedeutet, dass keine der Zecken abgetötet wurde.

Bei diesem Test zeigen z. B. die folgenden Verbindungen der Herstellungsbeispiele eine Wirkung von 100% bei einer Aufwandmenge von 100ppm: I-T3-1, I-T3-3, I-T3-20, I-T3-21, I-T3-24, I-T3-28, I-T3-42, I-T3-43, I-T3-44, I-T3-54, I-T3-55, I-T3-56, I-T3-57, I-T3-63, I-T3-64, I-T3-71, I-T3-72, I-T3-81, I-T3-86, I-T3-91, I-T3-92, I-T3-95, I-T3-96, I-T3-97, I-T3-98, I-T3-100, I-T3-104, I-T3-106, I-T3-107, I-T3-108, I-T3-109, I-T3-110, I-T3-112, I-T3-114, I-T3-116, I-T3-117, I-T3-119, I-T3-124, I-T3-125, I-T3-131, I-T3-148, I-T3-155, I-T3-162, I-T3-163, I-T22-1, I-T22-2, I-T23-1, I-T4-3, I-T4-4

Bei diesem Test zeigen z. B. die folgenden Verbindungen der Herstellungsbeispiele eine Wirkung von 95% bei einer Aufwandmenge von 100ppm: I-T3-101

Bei diesem Test zeigen z. B. die folgenden Verbindungen der Herstellungsbeispiele eine Wirkung von 90% bei einer Aufwandmenge von 100ppm: I-T3-102, I-T3-103

Bei diesem Test zeigen z. B. die folgenden Verbindungen der Herstellungsbeispiele eine Wirkung von 85% bei einer Aufwandmenge von 100ppm: I-T3-105

Bei diesem Test zeigen z. B. die folgenden Verbindungen der Herstellungsbeispiele eine Wirkung von 80% bei einer Aufwandmenge von 100ppm: I-T3-53, I-T3-61, I-T3-111, I-T3-123

### Boophilus microplus -Injektionstest

### Lösungsmittel: Dimethylsulfoxid

Zur Herstellung einer zweckmäßigen Wirkstoffzubereitung vermischt man 10 mg Wirkstoff mit 0,5 ml Lösungsmittel und verdünnt das Konzentrat mit Lösungsmittel auf die gewünschte Konzentration.

1µl der Wirkstofflösung wird in das Abdomen von 5 vollgesogenen, adulten, weiblichen Rinderzecken *(Boophilus microplus)* injiziert. Die Tiere werden in Schalen überführt und in einem klimatisierten Raum aufbewahrt.

Die Wirkungskontrolle erfolgt nach 7 Tagen auf Ablage fertiler Eier. Eier, deren Fertilität nicht äußerlich sichtbar ist, werden bis zum Larvenschlupf nach etwa 42 Tagen im Klimaschrank aufbewahrt. Eine Wirkung von 100 % bedeutet, dass keine der Zecken fertile Eier gelegt hat, 0% bedeutet, dass alle Eier fertil sind.

Bei diesem Test zeigen z.B. die folgenden Verbindungen der Herstellungsbeispiele eine Wirkung von 100% bei einer Aufwandmenge von 20µg/Tier: I-T2-1, I-T2-2, I-T3-1, I-T3-2, I-T3-3, I-T3-4, I-T3-5, I-T3-6, I-T3-7, I-T3-8, I-T3-9, I-T3-10, I-T3-11, I-T3-12, I-T3-13, I-T3-15, I-T3-17, I-T3-18, I-T3-19, I-T3-20, I-T3-21, I-T3-23, I-T3-24, I-T3-25, I-T3-26, I-T3-27, I-T3-28, I-T3-29, I-T3-30, I-T3-31, I-T3-32, I-T3-33, I-T3-34, I-T3-35, I-T3-36, I-T3-37, I-T3-38, I-T3-39, I-T3-40, I-T3-41, I-T3-42, I-T3-43, I-T3-44, I-T3-45, I-T3-46, I-T3-47, I-T3-48, I-T3-49, I-T3-50, I-T3-51, I-T3-52, I-T3-53, I-T3-54, I-T3-55, I-T3-56, I-T3-57, I-T3-58, I-T3-59, I-T3-60, I-T3-61, I-T3-62, I-T3-63, I-T3-64, I-T3-65, I-T3-66, I-T3-67, I-T3-68, I-T3-69, I-T3-70, I-T3-71, I-T3-72, I-T3-73, I-T3-74, I-T3-76, I-T3-77, I-T3-78, I-T3-79, I-T3-80, I-T3-81, I-T3-82, I-T3-83, I-T3-84, I-T3-85, I-T3-86, I-T3-87, I-T3-88, I-T3-89, I-T3-90, I-T3-91, I-T3-92, I-T3-93, I-T3-94, I-T3-95, I-T3-96, I-T3-97, I-T3-98, I-T3-99, I-T3-100, I-T3-101, I-T3-102, I-T3-103, I-T3-104, I-T3-105, I-T3-106, I-T3-107, I-T3-108, I-T3-109, I-T3-110, I-T3-111, I-T3-112, I-T3-113, I-T3-114, I-T3-115, I-T3-116, I-T3-117, I-T3-118, I-T3-119, I-T3-120, I-T3-123, I-T3-124, I-T3-125, I-T3-126, I-T3-127, I-T3-128, I-T3-129, I-T3-130, I-T3-131, I-T3-132, I-T3-133, I-T3-136, I-T3-137, I-T3-145, I-T3-139, I-T3-140, I-T3-141, I-T3-142, I-T3-143, I-T3-144, I-T3-146, I-T3-148, I-T3-149, I-T3-150, I-T3-151, I-T3-155, I-T3-160, I-T3-161, I-T3-162, I-T3-163, I-T3-165, I-T3-168, I-T3-175, I-T3-176, I-T3-189, I-T4-1, I-T4-2, I-T4-3, I-T4-4, I-T22-1, I-T22-2, I-T22-3, I-T22-4, I-T22-5, I-T22-6, I-T22-7, I-T23-1, I-T23-2, I-T46-2

Bei diesem Test zeigen z.B. die folgenden Verbindungen der Herstellungsbeispiele eine Wirkung von 90% bei einer Aufwandmenge von 20µg/Tier: I-T3-75

Bei diesem Test zeigen z.B. die folgenden Verbindungen der Herstellungsbeispiele eine Wirkung von 80% bei einer Aufwandmenge von 20µg/Tier: I-T3-121

### Boophilus microplus - Diptest

### Testtiere: Rinderzecken (Boophilus microplus) Stamm Parkhurst,SP-resistent

### Lösungsmittel: Dimethylsulfoxid

10 mg Wirkstoff werden in 0,5 ml Dimethylsulfoxid gelöst. Zwecks Herstellung einer geeigneten Formulierung verdünnt man die Wirkstofflösung mit Wasser auf die jeweils gewünschte Konzentration.

Diese Wirkstoffzubereitung wird in Röhrchen pipettiert. 8-10 gesogene, adulte, weibliche Rinderzecken (*Boophilus microplus*) werden in ein weiteres Röhrchen mit Löchern überführt. Das Röhrchen wird in die Wirkstoffzubereitung getaucht wobei alle Zecken vollständig benetzt werden. Nach Ablaufen der Flüssigkeit werden die Zecken auf Filterscheiben in Kunststoffschalen überführt und in einem klimatisierten Raum aufbewahrt.

Die Wirkungskontrolle erfolgt nach 7 Tagen auf Ablage fertiler Eier. Eier, deren Fertilität nicht äußerlich sichtbar ist, werden bis zum Larvenschlupf nach etwa 42 Tagen im Klimaschrank aufbewahrt. Eine Wirkung von 100 % bedeutet, dass keine der Zecken fertile Eier gelegt hat, 0% bedeutet, dass alle Eier fertil sind.

Bei diesem Test zeigen z.B. die folgenden Verbindungen der Herstellungsbeispiele eine Wirkung von 100% bei einer Aufwandmenge von 100ppm: I-T3-1, I-T3-3, I-T3-20, I-T3-21, I-T3-24, I-T3-28, I-T3-39, I-T3-42, I-T3-43, I-T3-44, I-T3-48, I-T3-53, I-T3-54, I-T3-55, I-T3-56, I-T3-57, I-T3-61, I-T3-63, I-T3-64, I-T3-71, I-T3-72, I-T3-81, I-T3-86, I-T3-91, I-T3-92, I-T3-95, I-T3-96, I-T3-97, I-T3-98, I-T3-100, I-T3-101, I-T3-102, I-T3-103, I-T3-104, I-T3-106, I-T3-107, I-T3-108, I-T3-109, I-T3-110, I-T3-112, I-T3-113, I-T3-114, I-T3-115, I-T3-116, I-T3-117, I-T3-118, I-T3-119, I-T3-120, I-T3-123, I-T3-124, I-T3-125, I-T3-130, I-T3-131, I-T3-133, I-T3-148, I-T3-155, I-T3-160, I-T3-162, I-T3-163, I-T3-165, I-T3-175, I-T3-176, I-T4-3, I-T4-4, I-T22-1, I-T22-2, I-T22-4, I-T22-5, I-T22-6, I-T22-7, I-T23-1

Bei diesem Test zeigen z.B. die folgenden Verbindungen der Herstellungsbeispiele eine Wirkung von 98% bei einer Aufwandmenge von 100ppm: I-T3-111

Bei diesem Test zeigen z.B. die folgenden Verbindungen der Herstellungsbeispiele eine Wirkung von 90% bei einer Aufwandmenge von 100ppm: I-T3-99

Bei diesem Test zeigen z.B. die folgenden Verbindungen der Herstellungsbeispiele eine Wirkung von 80% bei einer Aufwandmenge von 100ppm: I-T3-27, I-T3-80

### Ctenocephalides felis - Oraltest

### Lösungsmittel: Dimethylsulfoxid

Zwecks Herstellung einer zweckmäßigen Wirkstoffzubereitung vermischt man 10 mg Wirkstoff mit 0,5 ml Dimethylsulfoxid. Durch Verdünnen mit citriertem Rinderblut erhält man die gewünschte Konzentration.

Ca. 20 nüchterne adulte Katzenflöhe (*Ctenocephalides felis*) werden in eine Kammer eingesetzt, die oben und unten mit Gaze verschlossen ist. Auf die Kammer wird ein Metallzylinder gestellt, dessen Unterseite mit Parafilm verschlossen ist. Der Zylinder enthält die Blut-Wirkstoffzubereitung, die von den Flöhen durch die Parafilmmembran aufgenommen werden kann.

Nach 2 Tagen wird die Abtötung in % bestimmt. Dabei bedeutet 100 %, dass alle Flöhe abgetötet wurden; 0 % bedeutet, dass keiner der Flöhe abgetötet wurde.

Bei diesem Test zeigen z.B. die folgenden Verbindungen der Herstellungsbeispiele eine Wirkung von 100% bei einer Aufwandmenge von 100ppm: I-T3-1, I-T3-2, I-T3-3, I-T3-4, I-T3-5, I-T3-7, I-T3-8, I-T3-9, I-T3-10, I-T3-12, I-T3-18, I-T3-20, I-T3-21, I-T3-23, I-T3-24, I-T3-25, I-T3-26, I-T3-27, I-T3-28, I-T3-29, I-T3-30, I-T3-31, I-T3-32, I-T3-33, I-T3-34, I-T3-35, I-T3-38, I-T3-39, I-T3-40, I-T3-42, I-T3-43, I-T3-44, I-T3-46, I-T3-47, I-T3-48, I-T3-49, I-T3-50, I-T3-51, I-T3-52, I-T3-53, I-T3-54, I-T3-55, I-T3-56, I-T3-57, I-T3-58, I-T3-59, I-T3-61, I-T3-62, I-T3-63, I-T3-64, I-T3-65, I-T3-66, I-T3-67, I-T3-68, I-T3-69, I-T3-71, I-T3-72, I-T3-73, I-T3-76, I-T3-77, I-T3-78, I-T3-80, I-T3-81, I-T3-84, I-T3-85, I-T3-86, I-T3-87, I-T3-88, I-T3-89, I-T3-90, I-T3-91, I-T3-92, I-T3-93, I-T3-94, I-T3-95, I-T3-96, I-T3-97, I-T3-98, I-T3-99, I-T3-100, I-T3-101, I-T3-102, I-T3-103, I-T3-104, I-T3-105, I-T3-106, I-T3-107, I-T3-108, I-T3-109, I-T3-110, I-T3-111, I-T3-112, I-T3-113, I-T3-114, I-T3-115, I-T3-116, I-T3-117, I-T3-118, I-T3-119, I-T3-120, I-T3-123, I-T3-124, I-T3-125, I-T3-127, I-T3-128, I-T3-129, I-T3-130, I-T3-131, I-T3-132, I-T3-133, I-T3-135, I-T3-136, I-T3-137, I-T3-139, I-T3-140, I-T3-141, I-T3-143, I-T3-145, I-T3-146, I-T3-148, I-T3-149, I-T3-150, I-T3-151, I-T3-155, I-T3-160, I-T3-161, I-T3-162, I-T3-163, I-T3-165, I-T3-168, I-T3-175, I-T3-176, I-T3-189, I-T4-1, I-T4-2, I-T4-3, I-T4-4, I-T22-1, I-T22-2, I-T22-3, I-T22-4, I-T22-5, I-T22-7, I-T23-1, I-T23-2, I-T46-2

Bei diesem Test zeigen z.B. die folgenden Verbindungen der Herstellungsbeispiele eine Wirkung von 95% bei einer Aufwandmenge von 100ppm: I-T3-11, I-T3-17, I-T3-19, I-T3-41, I-T3-45, I-T3-70, I-T3-79, I-T3-82, I-T3-83, I-T22-6

Bei diesem Test zeigen z.B. die folgenden Verbindungen der Herstellungsbeispiele eine Wirkung von 90% bei einer Aufwandmenge von 100ppm: I-T3-15, I-T3-37, I-T3-60, I-T3-126, I-T3-144

Bei diesem Test zeigen z.B. die folgenden Verbindungen der Herstellungsbeispiele eine Wirkung von 80% bei einer Aufwandmenge von 100ppm: I-T3-13, I-T3-16, I-T3-36

### Lucilia cuprina - Test

### Lösungsmittel: Dimethylsulfoxid

Zur Herstellung einer zweckmäßigen Wirkstoffzubereitung vermischt man 10 mg Wirkstoff mit 0,5 ml Dimethylsulfoxid und verdünnt das Konzentrat mit Wasser auf die gewünschte Konzentration.

Ca. 20 L1-Larven der Australischen Schafgoldfliege (*Lucilia cuprina*) werden in ein Testgefäß überführt, welches gehacktes Pferdefleisch und die Wirkstoffzubereitung der gewünschten Konzentration enthält.

Nach 2 Tagen wird die Abtötung in % bestimmt. Dabei bedeutet 100 %, dass alle Larven abgetötet wurden; 0 % bedeutet, dass keine Larven abgetötet wurden.

Bei diesem Test zeigen z.B. die folgenden Verbindungen der Herstellungsbeispiele eine Wirkung von 100% bei einer Aufwandmenge von 100ppm: I-T3-1, I-T3-2, I-T3-3, I-T3-4, I-T3-5, I-T3-6, I-T3-7, I-T3-8, I-T3-9, I-T3-10, I-T3-15, I-T3-17, I-T3-18, I-T3-20, I-T3-21, I-T3-23, I-T3-24, I-T3-25, I-T3-26, I-T3-27, I-T3-28, I-T3-29, I-T3-30, I-T3-31, I-T3-32, I-T3-33, I-T3-34, I-T3-35, I-T3-36, I-T3-37, I-T3-38, I-T3-39, I-T3-40, I-T3-42, I-T3-43, I-T3-44, I-T3-45, I-T3-46, I-T3-47, I-T3-48, I-T3-49, I-T3-50, I-T3-51, I-T3-52, I-T3-53, I-T3-54, I-T3-55, I-T3-56, I-T3-57, I-T3-58, I-T3-59, I-T3-61, I-T3-62, I-T3-63, I-T3-64, I-T3-65, I-T3-66, I-T3-67, I-T3-68, I-T3-70, I-T3-71, I-T3-72, I-T3-73, I-T3-77, I-T3-78, I-T3-80, I-T3-81, I-T3-82, I-T3-83, I-T3-84, I-T3-85, I-T3-86, I-T3-87, I-T3-88, I-T3-89, I-T3-90, I-T3-91, I-T3-92, I-T3-93, I-T3-94, I-T3-96, I-T3-97, I-T3-98, I-T3-99, I-T3-100, I-T3-101, I-T3-102, I-T3-103, I-T3-104, I-T3-105, I-T3-106, I-T3-107, I-T3-108, I-T3-109, I-T3-110, I-T3-111, I-T3-112, I-T3-113, I-T3-114, I-T3-115, I-T3-116, I-T3-117, I-T3-118, I-T3-119, I-T3-120, I-T3-123, I-T3-124, I-T3-125, I-T3-130, I-T3-131, I-T3-133, I-T3-136, I-T3-139, I-T3-140, I-T3-141, I-T3-143, I-T3-144, I-T3-145, I-T3-148, I-T3-149, I-T3-150, I-T3-151, I-T3-155, I-T3-160, I-T3-161, I-T3-162, I-T3-163, I-T3-165, I-T3-168, I-T3-175, I-T3-176, I-T3-189, I-T4-1, I-T4-2, I-T4-3, I-T4-4, I-T22-1, I-T22-2, I-T22-3, I-T22-5, I-T22-6, I-T22-7, I-T23-1, I-T23-2, I-T46-2

Bei diesem Test zeigen z.B. die folgenden Verbindungen der Herstellungsbeispiele eine Wirkung von 95% bei einer Aufwandmenge von 100ppm: I-T3-69

Bei diesem Test zeigen z.B. die folgenden Verbindungen der Herstellungsbeispiele eine Wirkung von 90% bei einer Aufwandmenge von 100ppm: I-T3-41, I-T3-60, I-T3-74, I-T3-76, I-T3-127, I-T3-146

Bei diesem Test zeigen z.B. die folgenden Verbindungen der Herstellungsbeispiele eine Wirkung von 80% bei einer Aufwandmenge von 100ppm: I-T3-12, I-T3-75, I-T3-79, I-T3-121, I-T3-137

### Musca domestica-Test

### Lösungsmittel: Dimethylsulfoxid

Zur Herstellung einer zweckmäßigen Wirkstoffzubereitung vermischt man 10 mg Wirkstoff mit 0,5 ml Dimethylsulfoxid und verdünnt das Konzentrat mit Wasser auf die gewünschte Konzentration.

Gefäße, die einen Schwamm enthalten, der mit Zuckerlösung und der Wirkstoffzubereitung der gewünschten Konzentration behandelt wurde, werden mit 10 adulten Stubenfliegen *(Musca domestica)* besetzt.

Nach 2 Tagen wird die Abtötung in % bestimmt. Dabei bedeutet 100 %, dass alle Fliegen abgetötet wurden; 0 % bedeutet, dass keine der Fliegen abgetötet wurde.

Bei diesem Test zeigen z.B. die folgenden Verbindungen der Herstellungsbeispiele eine Wirkung von 100% bei einer Aufwandmenge von 100ppm: I-T3-1, I-T3-2, I-T3-3, I-T3-4, I-T3-5, I-T3-8, I-T3-20, I-T3-21, I-T3-23, I-T3-24, I-T3-25, I-T3-26, I-T3-27, I-T3-29, I-T3-31, I-T3-34, I-T3-38, I-T3-42, I-T3-43, I-T3-46, I-T3-48, I-T3-52, I-T3-53, I-T3-54, I-T3-55, I-T3-56, I-T3-57, I-T3-58, I-T3-61, I-T3-62, I-T3-63, I-T3-64, I-T3-65, I-T3-71, I-T3-72, I-T3-73, I-T3-77, I-T3-80, I-T3-84, I-T3-85, I-T3-86, I-T3-87, I-T3-89, I-T3-91, I-T3-92, I-T3-93, I-T3-94, I-T3-96, I-T3-97, I-T3-100, I-T3-101, I-T3-102, I-T3-103, I-T3-104, I-T3-106, I-T3-107, I-T3-108, I-T3-109, I-T3-110, I-T3-111, I-T3-112, I-T3-113, I-T3-114, I-T3-115, I-T3-116, I-T3-117, I-T3-118, I-T3-119, I-T3-120, I-T3-123, I-T3-124, I-T3-125, I-T3-130, I-T3-131, I-T3-133, I-T3-136, I-T3-137, I-T3-141, I-T3-143, I-T3-144, I-T3-148, I-T3-149, I-T3-150, I-T3-151, I-T3-155, I-T3-160, I-T3-161, I-T3-162, I-T3-163, I-T3-165, I-T3-175, I-T3-176, I-T3-189, I-T4-2, I-T22-1, I-T22-2, I-T22-3, I-T22-5, I-T22-7, I-T23-1, I-T23-2

Bei diesem Test zeigen z.B. die folgenden Verbindungen der Herstellungsbeispiele eine Wirkung von 95% bei einer Aufwandmenge von 100ppm: I-T3-51

Bei diesem Test zeigen z.B. die folgenden Verbindungen der Herstellungsbeispiele eine Wirkung von 90% bei einer Aufwandmenge von 100ppm: I-T3-30, I-T3-67, I-T3-76, I-T3-81, I-T3-90, I-T3-98, I-T3-99, I-T3-139, I-T3-145, I-T22-6

Bei diesem Test zeigen z.B. die folgenden Verbindungen der Herstellungsbeispiele eine Wirkung von 80% bei einer Aufwandmenge von 100ppm: I-T3-7, I-T3-66, I-T3-68, I-T3-79, I-T3-88, I-T3-105, I-T3-121, I-T3-129

Bei diesem Test zeigen z.B. die folgenden Verbindungen der Herstellungsbeispiele eine Wirkung von 80% bei einer Aufwandmenge von 20ppm: I-T3-28

Bei diesem Test zeigen z.B. die folgenden Verbindungen der Herstellungsbeispiele eine Wirkung von 80% bei einer Aufwandmenge von 4ppm: I-T3-35

### Meloidogyne incognita- Test

### Lösungsmittel: 125,0 Gewichtsteile Aceton

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser auf die gewünschte Konzentration.

Gefäße werden mit Sand, Wirkstofflösung, einer Ei-Larven-Suspension des südlichen Wurzelgallenälchens (*Meloidogyne incognita*) und Salatsamen gefüllt. Die Salatsamen keimen und die Pflänzchen entwickeln sich. An den Wurzeln entwickeln sich die Gallen.

Nach 14 Tagen wird die nematizide Wirkung anhand der Gallenbildung in % bestimmt. Dabei bedeutet 100 %, dass keine Gallen gefunden wurden; 0 % bedeutet, dass die Zahl der Gallen an den behandelten Pflanzen der unbehandelten Kontrolle entspricht.

Bei diesem Test zeigen z. B. die folgenden Verbindungen der Herstellungsbeispiele Wirkung von 90% bei einer Aufwandmenge von 20ppm: I-T3-27, I-T3-28, I-T3-184, I-T3-185

### Myzus persicae - Sprühtest

### Lösungsmittel: 78 Gewichtsteile Aceton und 1,5 Gewichtsteile Dimethylformamid

### Emulgator: Alkylarylpolyglykolether

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Chinakohlblattscheiben (*Brassica pekinensis*), die von allen Stadien der Grünen Pfirsichblattlaus (*Myzus persicae*) befallen sind, werden mit einer Wirkstoffzubereitung der gewünschten Konzentration gespritzt.

Nach 6 Tagen wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgenden Verbindungen der Herstellungsbeispiele Wirkung von 100% bei einer Aufwandmenge von 500g/ha: I-T3-7, I-T3-20, I-T3-43, I-T3-44, I-T3-46, I-T3-92, I-T3-100, I-T3-106, I-T3-107, I-T3-108, I-T3-110, I-T3-122, I-T3-185, I-T3-187

Bei diesem Test zeigen z. B. die folgenden Verbindungen der Herstellungsbeispiele Wirkung von 90% bei einer Aufwandmenge von 500g/ha: I-T3-8, I-T3-21, I-T3-29, I-T3-30, I-T3-42, I-T3-91, I-T3-97, I-T3-103, I-T3-105, I-T3-109, I-T3-114, I-T3-117, I-T3-119, I-T3-120, I-T3-186

Bei diesem Test zeigen z. B. die folgenden Verbindungen der Herstellungsbeispiele Wirkung von 100% bei einer Aufwandmenge von 100g/ha: I-T3-1, I-T3-3, I-T3-27, I-T3-54, I-T3-55, I-T3-77, I-T3-88, I-T3-99, I-T3-101, I-T3-112, I-T3-113, I-T3-115, I-T3-116, I-T3-118, I-T3-123, I-T3-124, I-T3-125, I-T3-127, I-T3-128, I-T3-129, I-T3-130, I-T3-162, I-T3-165, I-T3-170, I-T3-174, I-T3-175, I-T3-176, I-T3-179, I-T3-184, I-T3-189, I-T22-1, I-T22-2, I-T22-5, I-T22-7

Bei diesem Test zeigen z. B. die folgenden Verbindungen der Herstellungsbeispiele Wirkung von 90% bei einer Aufwandmenge von 100g/ha: I-T3-28, I-T3-38, I-T3-39, I-T3-53, I-T3-64, I-T3-72, I-T3-76, I-T3-80, I-T3-81, I-T3-85, I-T3-87, I-T3-95, I-T3-96, I-T3-98, I-T3-131, I-T3-132, I-T3-145, I-T3-160, I-T3-164, I-T3-163, I-T4-3

Bei diesem Test zeigen z. B. die folgenden Verbindungen der Herstellungsbeispiele Wirkung von 90% bei einer Aufwandmenge von 20g/ha: I-T3-182, I-T4-2

### Phaedon cochleariae - Sprühtest

### Lösungsmittel: 78,0 Gewichtsteile Aceton und 1,5 Gewichtsteile Dimethylformamid

### Emulgator: Alkylarylpolyglykolether

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Chinakohlblattscheiben *(Brassica pekinensis)* werden mit einer Wirkstoffzubereitung der gewünschten Konzentration gespritzt und nach dem Abtrocknen mit Larven des Meerrettichblattkäfers *(Phaedon cochleariae)* besetzt.

Nach 7 Tagen wird die Wirkung in % bestimmt. Dabei bedeutet 100 %, dass alle Käferlarven abgetötet wurden; 0 % bedeutet, dass keine Käferlarven abgetötet wurden.

Bei diesem Test zeigen z. B. die folgenden Verbindungen der Herstellungsbeispiele Wirkung von 100% bei einer Aufwandmenge von 500g/ha: I-T3-7, I-T3-8, I-T3-9, I-T3-10, I-T3-12, I-T3-15, I-T3-17, I-T3-18, I-T3-19, I-T3-20, I-T3-21, I-T3-22, I-T3-23, I-T3-24, I-T3-25, I-T3-26, I-T3-29, I-T3-30, I-T3-31, I-T3-34, I-T3-35, I-T3-36, I-T3-37, I-T3-42, I-T3-43, I-T3-44, I-T3-45, I-T3-46, I-T3-47, I-T3-65, I-T3-66, I-T3-67, I-T3-68, I-T3-69, I-T3-70, I-T3-73, I-T3-74, I-T3-75, I-T3-76, I-T3-77, I-T3-78, I-T3-79, I-T3-89, I-T3-90, I-T3-91, I-T3-92, I-T3-96, I-T3-97, I-T3-98, I-T3-100, I-T3-101, I-T3-102, I-T3-103, I-T3-104, I-T3-105, I-T3-106, I-T3-107, I-T3-108, I-T3-109, I-T3-110, I-T3-111, I-T3-112, I-T3-113, I-T3-114, I-T3-115, I-T3-116, I-T3-117, I-T3-118, I-T3-119, I-T3-120, I-T3-126, I-T3-184, I-T3-185, I-T3-186, I-T3-187, I-T3-188, I-T23-1, I-T23-2

Bei diesem Test zeigen z. B. die folgenden Verbindungen der Herstellungsbeispiele Wirkung von 100% bei einer Aufwandmenge von 100g/ha: I-T2-1, I-T2-2, I-T3-1, I-T3-2, I-T3-3, I-T3-4, I-T3-5, I-T3-6, I-T3-27, I-T3-28, I-T3-38, I-T3-39, I-T3-40, I-T3-41, I-T3-48, I-T3-49, I-T3-50, I-T3-51, I-T3-52, I-T3-53, I-T3-54, I-T3-55, I-T3-56, I-T3-57, I-T3-58, I-T3-59, I-T3-61, I-T3-62, I-T3-63, I-T3-64, I-T3-71, I-T3-72, I-T3-80, I-T3-81, I-T3-82, I-T3-83, I-T3-84, I-T3-85, I-T3-86, I-T3-87, I-T3-88, I-T3-93, I-T3-94, I-T3-95, I-T3-99, I-T3-123, I-T3-124, I-T3-125, I-T3-127, I-T3-128, I-T3-129, I-T3-130, I-T3-131, I-T3-132, I-T3-133, I-T3-136, I-T3-137, I-T3-139, I-T3-140, I-T3-141, I-T3-143, I-T3-144, I-T3-145, I-T3-148, I-T3-149, I-T3-151, I-T3-152, I-T3-153, I-T3-155, I-T3-160, I-T3-161, I-T3-162, I-T3-163, I-T3-164, I-T3-165, I-T3-168, I-T3-169, I-T3-170, I-T3-171, I-T3-172, I-T3-174, I-T3-175, I-T3-176, I-T3-177, I-T3-178, I-T3-179, I-T3-180, I-T3-181, I-T3-182, I-T3-183, I-T3-189, I-T3-190, I-T3-191, I-T3-192, I-T3-195, I-T3-197, I-T3-198, I-T3-220, I-T3-221, I-T3-222, I-T3-223, I-T4-1, I-T4-2, I-T4-3, I-T4-4, I-T22-1, I-T22-2, I-T22-3, I-T22-4, I-T22-5, I-T22-6, I-T22-7, I-T46-2, I-T46-3, I-T46-4, I-T46-5, I-T46-6

Bei diesem Test zeigen z. B. die folgenden Verbindungen der Herstellungsbeispiele Wirkung von 83% bei einer Aufwandmenge von 100g/ha: I-T3-138

### Spodoptera frugiperda - Sprühtest

### Lösungsmittel: 78,0 Gewichtsteile Aceton und 1,5 Gewichtsteile Dimethylformamid

### Emulgator: Alkylarylpolyglykolether

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Maisblattscheiben (*Zea mays*) werden mit einer Wirkstoffzubereitung der gewünschten Konzentration gespritzt und nach dem Abtrocknen mit Raupen des Heerwurms (*Spodoptera frugiperda*) besetzt.

Nach 7 Tagen wird die Wirkung in % bestimmt. Dabei bedeutet 100 %, dass alle Raupen abgetötet wurden; 0 % bedeutet, dass keine Raupe abgetötet wurde.

Bei diesem Test zeigen z. B. die folgenden Verbindungen der Herstellungsbeispiele Wirkung von 100% bei einer Aufwandmenge von 500g/ha: I-T3-7, I-T3-8, I-T3-9, I-T3-10, I-T3-12, I-T3-17, I-T3-18, I-T3-19, I-T3-20, I-T3-21, I-T3-22, I-T3-23, I-T3-24, I-T3-25, I-T3-26, I-T3-29, I-T3-30, I-T3-31, I-T3-34, I-T3-42, I-T3-43, I-T3-44, I-T3-45, I-T3-46, I-T3-47, I-T3-65, I-T3-66, I-T3-67, I-T3-68, I-T3-69, I-T3-70, I-T3-73, I-T3-74, I-T3-75, I-T3-76, I-T3-77, I-T3-78, I-T3-79, I-T3-89, I-T3-90, I-T3-91, I-T3-92, I-T3-96, I-T3-97, I-T3-98, I-T3-100, I-T3-102, I-T3-103, I-T3-104, I-T3-105, I-T3-106, I-T3-107, I-T3-108, I-T3-109, I-T3-110, I-T3-111, I-T3-112, I-T3-113, I-T3-114, I-T3-115, I-T3-116, I-T3-117, I-T3-118, I-T3-119, I-T3-120, I-T3-126, I-T3-184, I-T3-185, I-T3-186, I-T3-187, I-T23-1, I-T23-2

Bei diesem Test zeigen z. B. die folgenden Verbindungen der Herstellungsbeispiele Wirkung von 83% bei einer Aufwandmenge von 500g/ha: I-T3-101

Bei diesem Test zeigen z. B. die folgenden Verbindungen der Herstellungsbeispiele Wirkung von 100% bei einer Aufwandmenge von 100g/ha: I-T2-2, I-T3-1, I-T3-2, I-T3-3, I-T3-4, I-T3-27, I-T3-28, I-T3-38, I-T3-39, I-T3-40, I-T3-41, I-T3-48, I-T3-52, I-T3-53, I-T3-54, I-T3-55, I-T3-56, I-T3-57, I-T3-58, I-T3-61, I-T3-62, I-T3-63, I-T3-64, I-T3-71, I-T3-72, I-T3-80, I-T3-81, I-T3-82, I-T3-83, I-T3-84, I-T3-85, I-T3-86, I-T3-87, I-T3-88, I-T3-93, I-T3-94, I-T3-95, I-T3-99, I-T3-123, I-T3-124, I-T3-125, I-T3-130, I-T3-131, I-T3-133, I-T3-136, I-T3-137, I-T3-138, I-T3-139, I-T3-140, I-T3-141, I-T3-143, I-T3-145, I-T3-148, I-T3-151, I-T3-152, I-T3-155, I-T3-160, I-T3-161, I-T3-162, I-T3-163, I-T3-164, I-T3-165, I-T3-170, I-T3-174, I-T3-175, I-T3-176, I-T3-189, I-T3-191, I-T3-192, I-T3-197, I-T3-198, I-T4-1, I-T4-2, I-T4-3, I-T4-4, I-T22-1, I-T22-2, I-T22-3, I-T22-5, I-T22-7, I-T46-2, I-T46-3, I-T46-4, I-T46-5, I-T46-6

Bei diesem Test zeigen z. B. die folgenden Verbindungen der Herstellungsbeispiele Wirkung von 83% bei einer Aufwandmenge von 100g/ha: I-T3-35, I-T3-50, I-T3-169, I-T3-177

### Tetranychus urticae - Sprühtest, OP-resistent

### Lösungsmittel: 78,0 Gewichtsteile Aceton und 1,5 Gewichtsteile Dimethylformamid

### Emulgator: Alkylarylpolyglykolether

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Bohnenblattscheiben *(Phaseolus vulgaris),* die von allen Stadien der Gemeinen Spinnmilbe *(Tetranychus urticae)* befallen sind, werden mit einer Wirkstoffzubereitung der gewünschten Konzentration gespritzt.

Nach 6 Tagen wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigen z. B. die folgenden Verbindungen der Herstellungsbeispiele eine Wirkung von 100% bei einer Aufwandmenge von 500g/ha: I-T3-7, I-T3-8, I-T3-9, I-T3-10, I-T3-20, I-T3-21, I-T3-22, I-T3-23, I-T3-24, I-T3-26, I-T3-29, I-T3-30, I-T3-31, I-T3-34, I-T3-42, I-T3-43, I-T3-44, I-T3-45, I-T3-46, I-T3-47, I-T3-69, I-T3-75, I-T3-76, I-T3-77, I-T3-78, I-T3-91, I-T3-92, I-T3-96, I-T3-97, I-T3-98, I-T3-100, I-T3-101, I-T3-103, I-T3-106, I-T3-107, I-T3-108, I-T3-109, I-T3-110, I-T3-112, I-T3-113, I-T3-114, I-T3-115, I-T3-119, I-T3-120, I-T3-184, I-T3-185, I-T3-186, I-T3-187, I-T23-1, I-T23-2

Bei diesem Test zeigen z. B. die folgenden Verbindungen der Herstellungsbeispiele eine Wirkung von 90% bei einer Aufwandmenge von 500g/ha: I-T3-25, I-T3-65, I-T3-70, I-T3-89, I-T3-90, I-T3-102, I-T3-104, I-T3-105, I-T3-116, I-T3-117, I-T3-118

Bei diesem Test zeigen z. B. die folgenden Verbindungen der Herstellungsbeispiele eine Wirkung von 100% bei einer Aufwandmenge von 100g/ha: I-T3-1, I-T3-2, I-T3-3, I-T3-4, I-T3-27, I-T3-28, I-T3-38, I-T3-39, I-T3-41, I-T3-51, I-T3-53, I-T3-54, I-T3-55, I-T3-56, I-T3-57, I-T3-58, I-T3-61, I-T3-62, I-T3-63, I-T3-64, I-T3-72, I-T3-73, I-T3-80, I-T3-81, I-T3-82, I-T3-83, I-T3-84, I-T3-85, I-T3-86, I-T3-87, I-T3-88, I-T3-93, I-T3-94, I-T3-95, I-T3-99, I-T3-124, I-T3-125, I-T3-127, I-T3-129, I-T3-130, I-T3-131, I-T3-132, I-T3-133, I-T3-139, I-T3-145, I-T3-146, I-T3-155, I-T3-160, I-T3-161, I-T3-162, I-T3-163, I-T3-164, I-T3-165, I-T3-168, I-T3-169, I-T3-170, I-T3-174, I-T3-175, I-T3-176, I-T3-177, I-T3-178, I-T3-179, I-T3-180, I-T3-181, I-T3-182, I-T3-183, I-T3-189, I-T3-190, I-T3-192, I-T3-197, I-T3-221, I-T3-222, I-T3-223, I-T4-1, I-T4-2, I-T4-3, I-T4-4, I-T22-4, I-T22-5, I-T22-7, I-T46-4, I-T46-5, I-T46-6

Bei diesem Test zeigen z. B. die folgenden Verbindungen der Herstellungsbeispiele eine Wirkung von 90% bei einer Aufwandmenge von 100g/ha: I-T3-50, I-T3-52, I-T3-71, I-T3-74, I-T3-111, I-T3-123, I-T3-137, I-T3-138, I-T3-147, I-T3-148, I-T3-151, I-T3-172, I-T3-195, I-T22-1, I-T22-2, I-T22-3

Bei diesem Test zeigen z. B. die folgenden Verbindungen der Herstellungsbeispiele eine Wirkung von 100% bei einer Aufwandmenge von 20g/ha: I-T3-49

### Anopheles Test (ANPHGB Oberflächenbehandlung)

### Lösungsmittel: Aceton + 2000ppm Rapsölmethylester (RME)

Zur Herstellung einer zweckmäßigen Wirkstoffzubereitung löst man den Wirkstoff im Lösungsmittel (2mg/ml). Die Wirkstoffzubereitung wird auf eine lasierte Kachel pipettiert und nach Abtrocknen werden adulte Mücken der Spezies Anopheles gambiae Stamm RSPH (homozygot kdr) auf die behandelte Kachel gesetzt. Die Expositionszeit beträgt 30 Minuten.

24 Stunden nach Kontakt zur behandelten Oberfläche wird die Mortalität in % bestimmt. Dabei bedeutet 100%, dass alle Mücken abgetötet wurden; 0% bedeutet, dass keine Mücken abgetötet wurden.

Bei diesem Test zeigen z.B. die folgenden Verbindungen der Herstellungsbeispiele Wirkung von 90-100% bei einer Aufwandmenge von 100mg/m²: I-T3-20, I-T3-24, I-T3-27, I-T3-28, I-T3-43, I-T3-52, I-T3-53, I-T3-54, I-T3-56, I-T3-57, I-T3-61, I-T3-100, I-T3-102, I-T3-112, I-T3-123, I-T3-130, I-T3-133, I-T3-134, I-T3-136, I-T3-145, I-T3-148, I-T3-155, I-T3-160, I-T3-162, I-T3-173, I-T3-189, I-T22-1

Bei diesem Test zeigen z.B. die folgenden Verbindungen der Herstellungsbeispiele Wirkung von 90-100% bei einer Aufwandmenge von 20mg/m²: I-T3-23, I-T3-24, I-T3-26, I-T3-27, I-T3-28, I-T3-43, I-T3-52, I-T3-53, I-T3-54, I-T3-57, I-T3-58, I-T3-61, I-T3-87, I-T3-91, I-T3-92, I-T3-100, I-T3-102, I-T3-106, I-T3-112, I-T3-116, I-T3-130; I-T3-133, I-T3-134, I-T3-136, I-T3-137, I-T3-145, I-T3-148, I-T3-155, I-T3-159, I-T3-160, I-T3-162, I-T3-189, I-T22-2

### Anopheles Test (ANPHFU Oberflächenbehandlung)

### Lösungsmittel: Aceton + 2000ppm Rapsölmethylester (RME)

Zur Herstellung einer zweckmäßigen Wirkstoffzubereitung löst man den Wirkstoff im Lösungsmittel (2mg/ml). Die Wirkstoffzubereitung wird auf eine lasierte Kachel pipettiert und nach Abtrocknen werden adulte Mücken der Spezies Anopheles funestus Stamm FUMOZ-R (Hunt et al., Med Vet Entomol. 2005 Sep; 19(3):271-5) auf die behandelte Kachel gesetzt. Die Expositionszeit beträgt 30 Minuten.

24 Stunden nach Kontakt zur behandelten Oberfläche wird die Mortalität in % bestimmt. Dabei bedeutet 100%, dass alle Mücken abgetötet wurden; 0% bedeutet, dass keine Mücken abgetötet wurden.

Bei diesem Test zeigen z.B. die folgenden Verbindungen der Herstellungsbeispiele Wirkung von 90-100% bei einer Aufwandmenge von 100mg/m²: I-T3-24, I-T3-25, I-T3-38, I-T3-43, I-T3-46, I-T3-54, I-T3-56, I-T3-58, I-T3-63, I-T3-86, I-T3-92, I-T3-99, I-T3-100, I-T3-102, I-T3-107, I-T3-112, I-T3-113, I-T3-115, I-T3-123, I-T3-133, I-T3-134, I-T3-136, I-T3-145, I-T3-148, I-T3-155, I-T3-160, I-T3-162, I-T3-189, I-T22-1, I-T22-2

Bei diesem Test zeigen z.B. die folgenden Verbindungen der Herstellungsbeispiele Wirkung von 90-100% bei einer Aufwandmenge von 20mg/m²: I-T3-3, I-T3-24, I-T3-25, I-T3-26, I-T3-38, I-T3-42, I-T3-43, I-T3-46, I-T3-52, I-T3-53, I-T3-54, I-T3-55, I-T3-57, I-T3-61, I-T3-63, I-T3-92, I-T3-93, I-T3-99, I-T3-100, I-T3-102, I-T3-107, I-T3-112, I-T3-113, I-T3-116, I-T3-123, I-T3-134, I-T3-136, I-T3-145, I-T3-148, I-T3-155, I-T3-159, I-T3-160, I-T3-162, I-T3-189, I-T22-1, I-T22-2, I-T23-1, I-T23-2

### Aedes Test (AEDSAE Oberflächenbehandlung)

### Lösungsmittel: Aceton + 2000ppm Rapsölmethylester (RME)

Zur Herstellung einer zweckmäßigen Wirkstoffzubereitung löst man den Wirkstoff im Lösungsmittel (2mg/ml). Die Wirkstoffzubereitung wird auf eine lasierte Kachel pipettiert und nach Abtrocknen werden adulte Mücken der Spezies Aedes aegypti Stamm MONHEIM auf die behandelte Kachel gesetzt. Die Expositionszeit beträgt 30 Minuten.

24 Stunden nach Kontakt zur behandelten Oberfläche wird die Mortalität in % bestimmt. Dabei bedeutet 100%, dass alle Mücken abgetötet wurden; 0% bedeutet, dass keine Mücken abgetötet wurden.

Bei diesem Test zeigen z.B. die folgenden Verbindungen der Herstellungsbeispiele Wirkung von 90-100% bei einer Aufwandmenge von 100mg/m²: I-T3-1, I-T3-3, I-T3-8, I-T3-20, I-T3-21, I-T3-23, I-T3-24, I-T3-25, I-T3-27, I-T3-28, I-T3-38, I-T3-42, I-T3-43, I-T3-46, I-T3-52, I-T3-53, I-T3-54, I-T3-55, I-T3-56, I-T3-57, I-T3-58, I-T3-61, I-T3-63, I-T3-64, I-T3-86, I-T3-87, I-T3-91, I-T3-92, I-T3-93, I-T3-96, I-T3-98, I-T3-99, I-T3-100, I-T3-101, I-T3-102, I-T3-103, I-T3-106, I-T3-107, I-T3-108, I-T3-112, I-T3-113, I-T3-115, I-T3-117, I-T3-118, I-T3-120, I-T3-123, I-T3-130, I-T3-133, I-T3-134, I-T3-136, I-T3-145, I-T3-148, I-T3-155, I-T3-160, I-T3-162, I-T3-163, I-T3-173, I-T3-189, I-T22-1, I-T22-2, I-T23-1, I-T23-2

Bei diesem Test zeigen z.B. die folgenden Verbindungen der Herstellungsbeispiele Wirkung von 90-100% bei einer Aufwandmenge von 20mg/m²: I-T3-1, I-T3-3, I-T3-8, I-T3-20, I-T3-21, I-T3-23, I-T3-24, I-T3-25, I-T3-27, I-T3-28, I-T3-38, I-T3-42, I-T3-43, I-T3-46, I-T3-52, I-T3-53, I-T3-54, I-T3-55, I-T3-56, I-T3-57, I-T3-58, I-T3-61, I-T3-63, I-T3-64, I-T3-86, I-T3-87, I-T3-91, I-T3-92, I-T3-93, I-T3-95, I-T3-96, I-T3-98, I-T3-99, I-T3-100, I-T3-101, I-T3-102, I-T3-103, I-T3-106, I-T3-107, I-T3-108, I-T3-112, I-T3-113, I-T3-115, I-T3-116, I-T3-117, I-T3-118, I-T3-123, I-T3-130, I-T3-133, I-T3-134, I-T3-136, I-T3-145, I-T3-148, I-T3-155, I-T3-159, I-T3-160, I-T3-162, I-T3-163, I-T3-173, I-T3-189, I-T22-1, I-T22-2, I-T23-1, I-T23-2

## Patentansprüche

1. Verbindungen der Formel (Ia'') 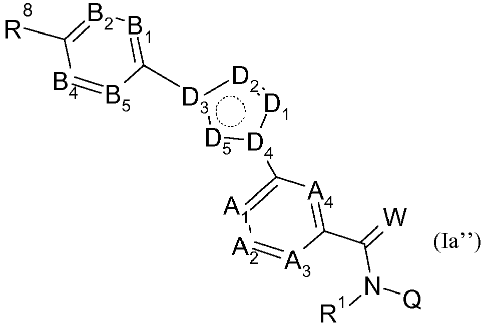 worin
D₁ für C-R¹¹ oder ein Heteroatom ausgewählt aus N oder O steht;
D₂ für C-R¹¹ oder ein Heteroatom ausgewählt aus N oder O steht;
D₃ für C oder N steht;
D₄ für C, oder N steht;
D₅ für C-R¹¹ oder N steht;
wobei maximal ein (1) oder zwei Gruppierungen ausgewählt aus D₁, D₂, D₃, D₄ und D₅ für ein Heteroatom stehen; 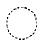 für ein aromatisches System steht; und
R¹ für H, jeweils gegebenenfalls substituiertes C₂-C₆-Alkenyl, C₂-C₆-Alkinyl, C₃-C₇-Cycloalkyl, C₁-C₆-Alkylcarbonyl, C₁-C₆-Alkoxycarbonyl, Aryl(C₁-C₃)-alkyl, oder Heteroaryl(C₁-C₃)-alkyl steht;
die Gruppierungen
A₁ C-H,
A₂ CR³ oder N,
A₃ CR⁴,
A₄ C-H,
B₁ CR⁶ oder N,
B₂ C-H,
B₄ C-H und
B₅ CR¹⁰ oder N stehen;
R³, R⁴, R⁶ und R¹⁰ unabhängig voneinander für H, Halogen, Cyano, Nitro, jeweils gegebenenfalls substituiertes C₁-C₆-Alkyl, C₃-C₆-Cycloalkyl, C₁-C₆-Alkoxy, N-C₁-C₆-Alkoxy-imino-C₁-C₃-alkyl, C₁-C₆-Alkylsulfanyl, C₁-C₆-Alkylsulfinyl, C₁-C₆-Alkylsulfonyl, *N*-C₁-C₆-Alkylamino, *N,N*-Di-C₁-C₆-alkylamino, oder *N*-C₁-C₃-Alkoxy-C₁-C₄-Alkylamino oder 1-Pyrrolidinyl stehen;
wenn keine der Gruppierungen A₂ und A₃ für N steht, können R³ und R⁴ gemeinsam mit dem Kohlenstoffatom, an das sie gebunden sind, einen 5- oder 6-gliedrigen Ring bilden, der 0, 1 oder 2 N-Atome und/oder 0 oder 1 O-Atom und/oder 0 oder 1 S-Atom enthält, oder
wenn keine der Gruppierungen A₁ und A₂ für N steht, können R² und R³ gemeinsam mit dem Kohlenstoffatom, an das sie gebunden sind, einen 6-gliedrigen Ring bilden, der 0, 1 oder 2 N-Atome enthält;
R⁸ für mit Fluor substituiertes C₁-C₄-Alkoxy oder mit Fluor substituiertes C₁-C₄-Alkyl steht;
R¹¹ unabhängig voneinander für H, Halogen, Cyano, Nitro, Amino oder ein gegebenenfalls substituiertes C₁-C₆-Alkyl, C₁-C₆-Alkyloxy, C₁-C₆-Alkylcarbonyl, C₁-C₆-Alkylsulfanyl, C₁-C₆-Alkylsulfinyl, C₁-C₆-Alkylsulfonyl, bevorzugt für H steht;
W für O oder S steht;
Q für H, Formyl, Hydroxy, Amino oder jeweils gegebenenfalls substituiertes C₁-C₆-Alkyl, C₂-C₆-Alkenyl, C₂-C₆-Alkinyl, C₃-C₆-Cycloalkyl, C₁-C₅-Heterocycloalkyl, C₁-C₄-Alkoxy, C₁-C₆-Alkyl-C₃-C₆-cycloalkyl, C₃-C₆-Cycloalkyl-C₁-C₆-alkyl, C₆-,C₁₀-,C₁₄-Aryl, C₁-C₅-Heteroaryl, C₆-,C₁₀-,C₁₄-Aryl-(C₁-C₃)-alkyl, C₁-C₅-Heteroaryl-(C₁-C₃)-alkyl, *N*-C₁-C₄-Alkylamino, *N*-C₁-C₄-Alkylcarbonylamino, oder *N,N*-Di-C₁-C₄-alkylamino steht; oder
für einen gegebenenfalls mehrfach mit V substituierten, ungesättigten 6-gliedrigen Carbozyklus steht; oder
für einen gegebenenfalls mehrfach mit V substituierten, ungesättigten 4-, 5- bzw. 6-gliedrigen, heterozyklischen Ring steht, wobei
V unabhängig voneinander für Halogen, Cyano, Nitro, jeweils gegebenenfalls substituiertes C₁-C₆-Alkyl, C₁-C₄-Alkenyl, C₁-C₄-Alkinyl, C₃-C₆-Cycloalkyl, C₁-C₆-Alkoxy, *N*-C₁-C₆-Alkoxy-imino-C₁-C₃-alkyl, C₁-C₆-Alkylsulfanyl, C₁-C₆-Alkylsulfinyl, C₁-C₆-Alkylsulfonyl, oder *N,N*-Di-(C₁-C₆-alkyl)amino steht,
wobei für den Fall, dass die Gruppen R³, R⁴, R⁶, R¹⁰, R¹¹, Q und V jeweils unabhängig voneinander substituiert sind, die Substituenten ein (1) Substituent oder mehrere Substituenten ausgewählt sind aus einer Gruppe bestehend aus Amino, Hydroxy, Halogen, Nitro, Cyano, Isocyano, Mercapto, Isothiocyanato, C₁-C₄-Carboxy, Carbonamid, SF₅, Aminosulfonyl, C₁-C₄-Alkyl, C₃-C₄-Cycloalkyl, C₂-C₄-Alkenyl, C₃-C₄-Cycloalkenyl, C₂-C₄-Alkinyl, *N*-Mono-C₁-C₄-alkyl-amino, *N,N*-Di-C₁-C₄-alkylamino, *N*-C₁-C₄-Alkanoylamino, C₁-C₄-Alkoxy, C₂-C₄-Alkenyloxy, C₂-C₄-Alkinyloxy, C₃-C₄-Cycloalkoxy, C₃-C₄-Cycloalkenyloxy, C₁-C₄-Alkoxycarbonyl, C₂-C₄-Alkenyloxycarbonyl, C₂-C₄-Alkinyloxycarbonyl, C₆-,C₁₀-,C₁₄-Aryloxycarbonyl, C₁-C₄-Alkanoyl, C₂-C₄-Alkenylcarbonyl, C₂-C₄-Alkinylcarbonyl, C₆-,C₁₀-,C₁₄-Arylcarbonyl, C₁-C₄-Alkylsulfanyl, C₃-C₄-Cycloalkylsulfanyl, C₁-C₄-Alkylthio, C₂-C₄-Alkenylthio, C₃-C₄-Cycloalkenylthio, C₂-C₄-Alkinylthio, C₁-C₄-Alkylsulfenyl und C₁-C₄-Alkylsulfinyl, wobei beide Enantiomere der C₁-C₄-Alkylsulfinylgruppe umfasst sind, C₁-C₄-Alkylsulfonyl, *N*-Mono-C₁-C₄-alkyl-aminosulfonyl, *N,N*-Di-C₁-C₄-alkyl-aminosulfonyl, C₁-C₄-Alkylphosphinyl, C₁-C₄-Alkylphosphonyl, wobei für C₁-C₄-Alkylphosphinyl bzw. C₁-C₄-Alkylphosphonyl beide Enantiomere umfasst sind, *N*-C₁-C₄-Alkyl-aminocarbonyl, *N,N-*Di-C₁-C₄-alkyl-amino-carbonyl, N-C₁-C₄-Alkanoyl-amino-carbonyl, N-C₁-C₄-Alkanoyl-N-C₁-C₄-alkyl-aminocarbonyl, C₆-,C₁₀-,C₁₄-Aryl, C₆-,C₁₀-,C₁₄-Aryloxy, Benzyl, Benzyloxy, Benzylthio, C₆-,C₁₀-,C₁₄-Arylthio, C₆-,C₁₀-,C₁₄-Arylamino, Benzylamino, Heterocyclyl und Trialkylsilyl, mit einer Doppelbindung verbundene Substituenten wie C₁-C₄-Alkyliden (z. B. Methyliden oder Ethyliden), eine Oxogruppe, eine Thioxogruppe, eine Iminogruppe sowie einer substituierten Iminogruppe,
sowie Salze, N-Oxide und tautomere Formen der Verbindungen der Formel (Ia").

2. Verbindungen gemäß Anspruch 1, wobei die Verbindungen der Formel (Ia") Verbindungen der Formel (I-T3) darstellen 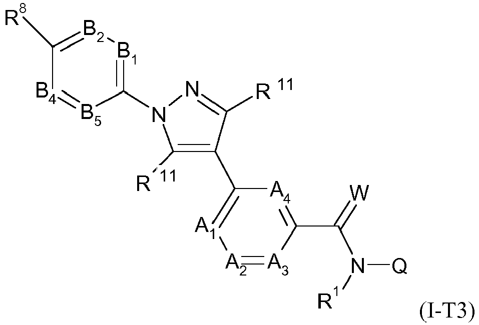 worin
R¹, A₁, A₂, A₃, A₄, R¹¹, B₁, B₂, B₄, B₅, R⁸, Q und W wie in Anspruch 1 beschrieben definiert sind.

3. Verbindungen gemäß Anspruch 1, wobei die Verbindungen der Formel (Ia") Verbindungen der Formel (I-T2) darstellen 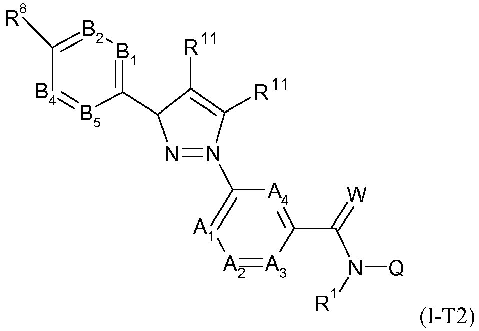 worin
R¹, A₁, A₂, A₃, A₄, R¹¹, B₁, B₂, B₄, B₅, R⁸, Q und W wie in Anspruch 1 beschrieben definiert sind.

4. Verbindungen gemäß Anspruch 1, wobei die Verbindungen der Formel (Ia") Verbindungen der Formel (I-T4) darstellen 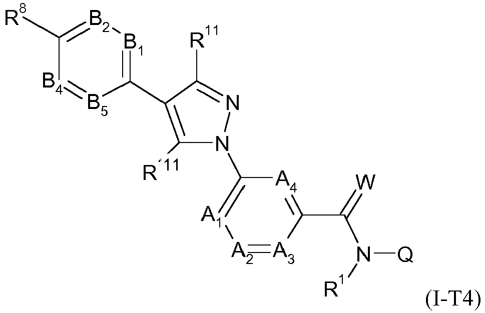 worin
R¹, A₁, A₂, A₃, A₄, R¹¹, B₁, B₂, B₄, B₅, R⁸, Q und W wie in Anspruch 1 beschrieben definiert sind.

5. Verbindungen gemäß Anspruch 1, wobei die Verbindungen der Formel (Ia") Verbindungen der Formel (I-T22) darstellen 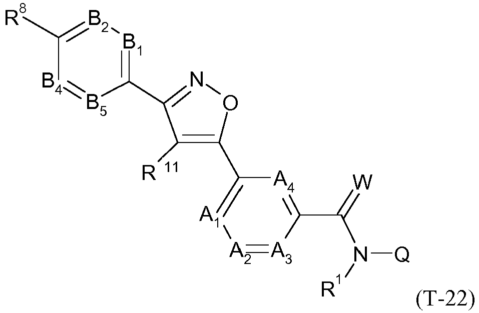 worin
R¹, A₁, A₂, A₃, A₄, R¹¹, B₁, B₂, B₄, B₅, R⁸, Q und W wie in Anspruch 1 beschrieben definiert sind.

6. Verbindungen gemäß Anspruch 1, wobei die Verbindungen der Formel (Ia") Verbindungen der Formel (I-T23) darstellen 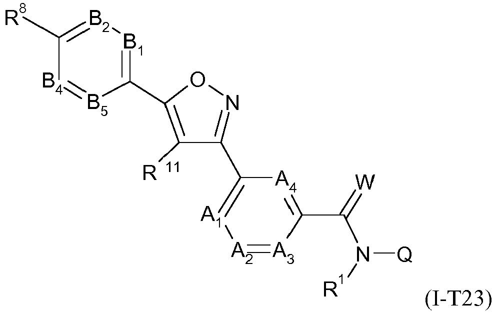 worin
R¹, A₁, A₂, A₃, A₄, R¹¹, B₁, B₂, B₄, B₅, R⁸, Q und W wie in Anspruch 1 beschrieben definiert sind.

7. Verbindungen gemäß Anspruch 1, wobei die Verbindungen der Formel (Ia") Verbindungen der Formel (I-T46) darstellen 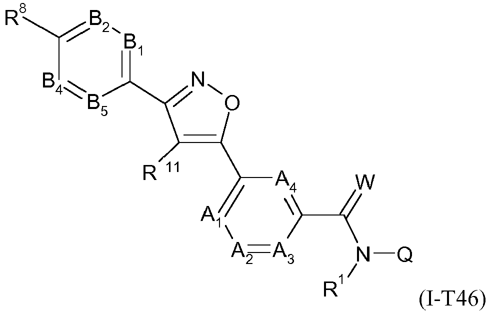 worin
R¹, A₁, A₂, A₃, A₄, R¹¹, B₁, B₂, B₄, B₅, R⁸, Q und W wie in Anspruch 1 beschrieben definiert sind.

8. Verbindungen gemäß einem der Ansprüche 1 bis 7, wobei R¹¹ unabhängig voneinander für H steht und W für O steht.

9. Verbindungen gemäß einem der Ansprüche 1 bis 8, wobei R¹ für H steht.

10. Verbindungen gemäß einem der Ansprüche 1 bis 9, wobei Q für C₁-C₃-Alkyl, Cyclopropyl, 1-(Cyano)-cyclopropyl, 1-(perfluoriertes C₁-C₃-Alkyl)-cyclopropyl, 1-(C₁-C₄-Alkyl)-cyclopropyl, 1-(Thiocarbamoyl)-cyclopropyl, mit Halogen substituiertes C₁-C₃-Alkyl, Thietan-3-yl, N-Methyl-Pyrazol-3-yl oder 2-oxo-2(2,2,2-trifluorethylamino)ethyl steht.

11. Insektizides Mittel, **gekennzeichnet durch** einen Gehalt von mindestens einer Verbindung der Formel (Ia") gemäß einem der Ansprüch 1 bis 10 und einem Streckmittel und/oder einer oberflächenaktiven Substanz.

## Claims

1. Compounds of the formula (Ia") 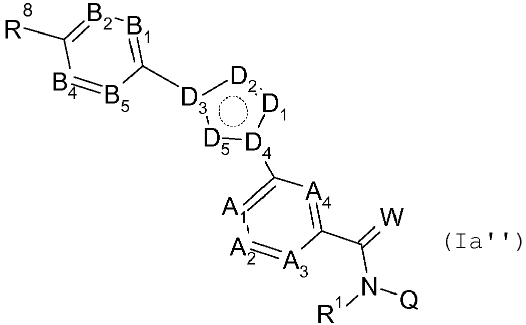 where
D₁ is C-R¹¹ or a heteroatom selected from N and O;
D₂ is C-R¹¹ or a heteroatom selected from N and O;
D₃ is C or N;
D₄ is C or N;
D₅ is C-R¹¹ or N;
where not more than one (1) or two moieties selected from D₁, D₂, D₃, D₄ and D₅ are a heteroatom; 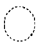 is an aromatic system; and
R¹ is H, in each case optionally substituted C₂-C₆-alkenyl, C₂-C₆-alkynyl, C₃-C₇-cycloalkyl, C₁-C₆-alkylcarbonyl, C₁-C₆-alkoxycarbonyl, aryl (C₁-C₃)-alkyl, or heteroaryl(C₁-C₃)-alkyl; the following moieties are as follows:
A₁ is C-H,
A₂ is CR³ or N,
A₃ is CR⁴,
A₄ is C-H,
B₁ is CR⁶ or N,
B₂ is C-H,
B₄ is C-H and
B₅ is CR¹⁰ or N;
R³, R⁴, R⁶ and R¹⁰ are each independently H, halogen, cyano, nitro, in each case optionally substituted C₁-C₆-alkyl, C₃-C₆-cycloalkyl, C₁-C₆-alkoxy, *N*-C₁-C₆-alkoxyimino-C₁-C₃-alkyl, C₁-C₆-alkylsulphanyl, C₁-C₆-alkylsulphinyl, C₁-C₆-alkylsulphonyl, *N*-C₁-C₆-alkylamino, *N,N*-di-C₁-C₆-alkylamino or *N*-C₁-C₃-alkoxy-C₁-C₄-alkylamino or 1-pyrrolidinyl; if neither of the A₂ and A₃ moieties is N, R³ and R⁴ together with the carbon atom to which they are bonded may form a 5- or 6-membered ring containing 0, 1 or 2 nitrogen atoms and/or 0 or 1 oxygen atom and/or 0 or 1 sulphur atom, or if neither of the A₁ and A₂ moieties is N, R² and R³ together with the carbon atom to which they are bonded may form a 6-membered ring containing 0, 1 or 2 nitrogen atoms;
R⁸ is fluorine-substituted C₁-C₄-alkoxy or fluorine-substituted C₁-C₄-alkyl;
R¹¹ is independently H, halogen, cyano, nitro, amino or an optionally substituted C₁-C₆-alkyl, C₁-C₆-alkyloxy, C₁-C₆-alkylcarbonyl, C₁-C₆-alkylsulphanyl, C₁-C₆-alkylsulphinyl, C₁-C₆-alkylsulphonyl, preferably H;
W is O or S;
Q is H, formyl, hydroxyl, amino or in each case optionally substituted C₁-C₆-alkyl, C₂-C₆-alkenyl, C₂-C₆-alkynyl, C₃-C₆-cycloalkyl, C₁-C₅-heterocycloalkyl, C₁-C₄-alkoxy, C₁-C₆-alkyl-C₃-C₆-cycloalkyl, C₃-C₆-cycloalkyl-C₁-C₆-alkyl, C₆-, C₁₀-C₁₄-aryl, C₁-C₅-heteroaryl, C₆-, C₁₀-, C₁₄-aryl-(C₁-C₃)-alkyl, C₁-C₅-heteroaryl-(C₁-C₃) - alkyl, *N*-C₁-C₄-alkylamino, *N*-C₁-C₄-alkylcarbonylamino, or *N,N*-di-C₁-C₄-alkylamino; or
is an optionally poly-V-substituted unsaturated 6-membered carbocycle; or
is an optionally poly-V-substituted unsaturated 4-, 5- or 6-membered heterocyclic ring, where
V is independently halogen, cyano, nitro, in each case optionally substituted C₁-C₆-alkyl, C₁-C₄-alkenyl, C₁-C₄-alkynyl, C₃-C₆-cycloalkyl, C₁-C₆-alkoxy, *N*-C₁-C₆-alkoxyimino-C₁-C₃-alkyl, C₁-C₆-alkylsulphanyl, C₁-C₆-alkylsulphinyl, C₁-C₆-alkylsulphonyl, or *N,N*-di-(C₁-C₆-alkyl)amino;
where, if the groups R³, R⁴, R⁶, R¹⁰, R¹¹, Q and V are each substituted independently of one another, the substituents are one (1) substituent or a plurality of substituents selected from a group consisting of amino, hydroxyl, halogen, nitro, cyano, isocyano, mercapto, isothiocyanato, C₁-C₄-carboxyl, carbonamide, SF₅, aminosulphonyl, C₁-C₄-alkyl, C₃-C₄-cycloalkyl, C₂-C₄-alkenyl, C₃-C₄₋cycloalkenyl, C₂-C₄-alkynyl, *N*-mono-C₁-C₄-alkylamino, *N,N*-di-C₁-C₄-alkylamino, *N*-C₁-C₄-alkanoylamino, C₁-C₄-alkoxy, C₂-C₄-alkenyloxy, C₂-C₄-alkynyloxy, C₃-C₄₋cycloalkoxy, C₃-C₄₋cycloalkenyloxy, C₁-C₄-alkoxycarbonyl, C₂-C₄-alkenyloxycarbonyl, C₂-C₄-alkynyloxycarbonyl, C₆-,C₁₀-,C₁₄-aryloxycarbonyl, C₁-C₄-alkanoyl, C₂-C₄-alkenylcarbonyl, C₂-C₄-alkynylcarbonyl, C₆-,C₁₀-,C₁₄-arylcarbonyl, C₁-C₄-alkylsulphanyl, C₃-C₄-cycloalkylsulphanyl, C₁-C₄-alkylthio, C₂-C₄-alkenylthio, C₃-C₄₋cycloalkenylthio, C₂-C₄-alkynylthio, C₁-C₄-alkylsulphenyl and C₁-C₄-alkylsulphinyl, including both enantiomers of the C₁-C₄-alkylsulphinyl group, C₁-C₄-alkylsulphonyl, N-mono-C₁-C₄-alkylaminosulphonyl, *N,N*-di-C₁-C₄-alkylaminosulphonyl, C₁-C₄-alkylphosphinyl, C₁-C₄-alkylphosphonyl, including both enantiomers of C₁-C₄-alkylphosphinyl and C₁-C₄-alkylphosphonyl, *N*-C₁-C₄-alkylaminocarbonyl, *N,N*-di-C₁-C₄-alkylaminocarbonyl, N-C₁-C₄-alkanoylaminocarbonyl, N-C₁-C₄-alkanoyl-N-C₁-C₄-alkylaminocarbonyl, C₆,C₁₀,C₁₄-aryl, C₆-,C₁₀-,C₁₄-aryloxy, benzyl, benzyloxy, benzylthio, C₆-,C₁₀-,C₁₄-arylthio, C₆-,C₁₀-,C₁₄-arylamino, benzylamino, heterocyclyl and trialkylsilyl, substituents bonded via a double bond, such as C₁-C₄-alkylidene (e.g. methylidene or ethylidene), an oxo group, a thioxo group, an imino group and a substituted imino group,
and salts, N-oxides and tautomeric forms of the compounds of the formula (Ia").

2. Compounds according to Claim 1, wherein the compounds of the formula (Ia") are compounds of the formula (I-T3) 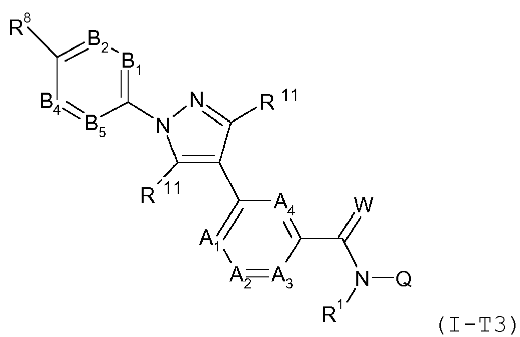 where
R¹, A₁, A₂, A₃, A₄, R¹¹, B₁, B₂, B₄, B₅, R⁸, Q and W are each as defined in Claim 1.

3. Compounds according to Claim 1, wherein the compounds of the formula (Ia") are compounds of the formula (I-T2) 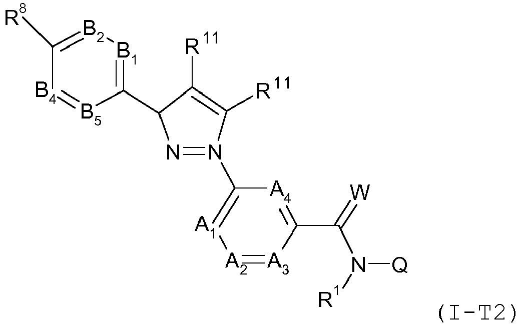 where
R¹, A₁, A₂, A₃, A₄, R¹¹, B₁, B₂, B₄, B₅, R⁸, Q and W are each as defined in Claim 1.

4. Compounds according to Claim 1, wherein the compounds of the formula (Ia") are compounds of the formula (I-T4) 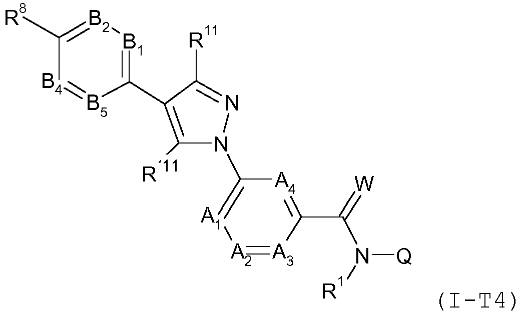 where
R¹, A₁, A₂, A₃, A₄, R¹¹, B₁, B₂, B₄, B₅, R⁸, Q and W are each as defined in Claim 1.

5. Compounds according to Claim 1, wherein the compounds of the formula (Ia") are compounds of the formula (I-T22) 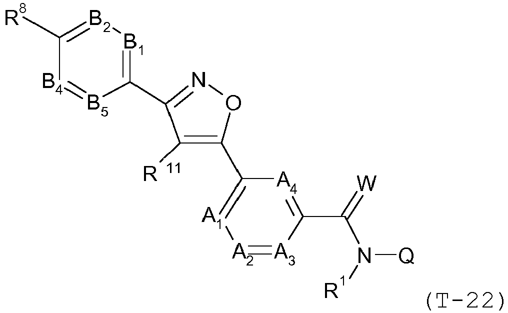 where
R¹, A₁, A₂, A₃, A₄, R¹¹, B₁, B₂, B₄, B₅, R⁸, Q and W are each as defined in Claim 1.

6. Compounds according to Claim 1, wherein the compounds of the formula (Ia") are compounds of the formula (I-T23) 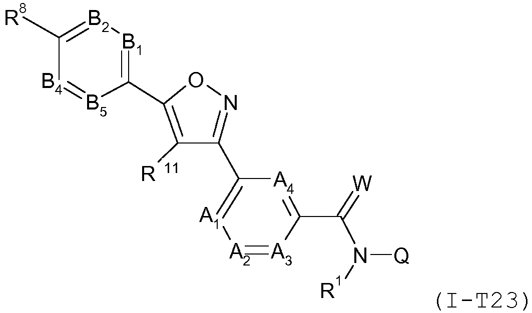 where
R¹, A₁, A₂, A₃, A₄, R¹¹, B₁, B₂, B₄, B₅, R⁸, Q and W are each as defined in Claim 1.

7. Compounds according to Claim 1, wherein the compounds of the formula (Ia") are compounds of the formula (I-T46) 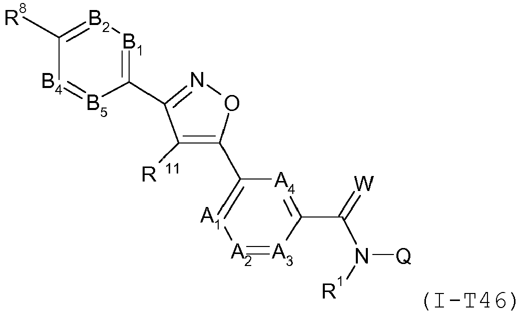 where
R¹, A₁, A₂, A₃, A₄, R¹¹, B₁, B₂, B₄, B₅, R⁸, Q and W are each as defined in Claim 1.

8. Compounds according to any of Claims 1 to 7, wherein R¹¹ is independently H and W is O.

9. Compounds according to any of Claims 1 to 8, wherein R¹ is H.

10. Compounds according to any of Claims 1 to 9, wherein Q is C₁-C₃-alkyl, cyclopropyl, 1-(cyano)cyclopropyl, 1-(perfluorinated C₁-C₃-alkyl)cyclopropyl, 1-(C₁-C₄-alkyl)cyclopropyl, 1-(thiocarbamoyl)cyclopropyl, halogen-substituted C₁-C₃-alkyl, thietan-3-yl, N-methylpyrazol-3-yl or 2-oxo-2(2,2,2-trifluoroethylamino)ethyl.

11. Insecticidal composition, **characterized by** a content of at least one compound of the formula (Ia") according to any of Claims 1 to 10 and an extender and/or a surface-active substance.

## Revendications

1. Composés de formule (Ia") 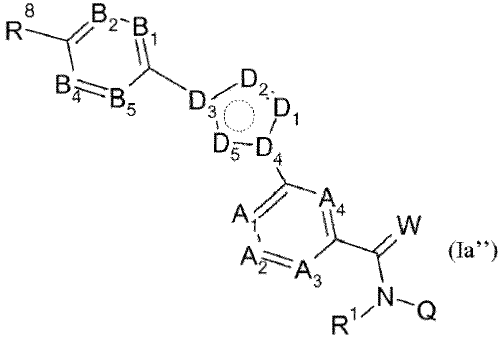 dans laquelle
D₁ représente C-R¹¹ ou un hétéroatome choisi parmi N ou O ;
D₂ représente C-R¹¹ ou un hétéroatome choisi parmi N ou O ;
D₃ représente C ou N ;
D₄ représente C ou N ;
D₅ représente C-R¹¹ ou N ;
au plus un (1) ou deux groupes choisis parmi D₁, D₂, D₃, D₄ et D₅ représentant un hétéroatome ; 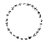
représente un système aromatique ; et
R¹ représente H, alcényle en C₂-C₆, alcynyle en C₂-C₆, cycloalkyle en C₃-C₇, alkylcarbonyle en C₁-C₆, alcoxycarbonyle en C₁-C₆, aryl-alkyle en (C₁-C₃) ou hétéroaryl-alkyle en (C₁-C₃), chacun éventuellement substitués ;
les groupes suivants représentent
A₁ C-H,
A₂ CR³ ou N,
A₃ CR⁴,
A₄ C-H,
B₁ CR⁶ ou N,
B₂ C-H,
B₄ C-H et
B₅ CR¹⁰ ou N ;
R³, R⁴, R⁶ et R¹⁰ représentent indépendamment les uns des autres H, halogène, cyano, nitro, alkyle en C₁-C₆, cycloalkyle en C₃-C₆, alcoxy en C₁-C₆, N-alcoxy en C₁-C₆-imino-alkyle en C₁-C₃, alkylsulfanyle en C₁-C₆, alkylsulfinyle en C₁-C₆, alkylsulfonyle en C₁-C₆, N-alkylamino en C₁-C₆, N,N-di-alkylamino en C₁-C₆ ou N-alcoxy en C₁-C₃-alkylamino en C₁-C₄ ou 1-pyrrolidinyle, chacun éventuellement substitués ;
lorsqu'aucun des groupes A₂ et A₃ ne représente N, R³ et R⁴ peuvent former ensemble avec l'atome de carbone auquel ils sont reliés un cycle à 5 ou 6 chaînons, qui contient 0, 1 ou 2 atomes N et/ou 0 ou 1 atome O et/ou 0 ou 1 atome S,
ou
lorsqu'aucun des groupes A₁ et A₂ ne représente N, R² et R³ peuvent former ensemble avec l'atome de carbone auquel ils sont reliés un cycle à 6 chaînons, qui contient 0, 1 ou 2 atomes N ;
R⁸ représente alcoxy en C₁-C₄ substitué avec fluor ou alkyle en C₁-C₄ substitué avec fluor ;
les R¹¹ représentent indépendamment les uns des autres H, halogène, cyano, nitro, amino ou alkyle en C₁-C₆, alkyloxy en C₁-C₆, alkylcarbonyle en C₁-C₆, alkylsulfanyle en C₁-C₆, alkylsulfinyle en C₁-C₆, alkylsulfonyle en C₁-C₆, éventuellement substitués, de préférence H ;
W représente O ou S ;
Q représente H, formyle, hydroxy, amino ou alkyle en C₁-C₆, alcényle en C₂-C₆, alcynyle en C₂-C₆, cycloalkyle en C₃-C₆, hétérocycloalkyle en C₁-C₅, alcoxy en C₁-C₄, alkyle en C₁-C₆-cycloalkyle en C₃-C₆, cycloalkyle en C₃-C₆-alkyle en C₁-C₆, aryle en C₆, C₁₀, C₁₄, hétéroaryle en C₁-C₅, aryle en C₆, C₁₀, C₁₄-alkyle en C₁-C₃, hétéroaryle en C₁-C₅-alkyle en C₁-C₃, N-alkylamino en C₁-C₄, N-alkylcarbonylamino en C₁-C₄ ou N,N-di-alkylamino en C₁-C₄, chacun éventuellement substitués ; ou
représente un carbocycle insaturé à 6 chaînons, éventuellement substitué à plusieurs reprises avec V ; ou
représente un cycle hétérocyclique insaturé à 4, 5 ou 6 chaînons, éventuellement substitué à plusieurs reprises avec V,
les V représentant indépendamment les uns des autres halogène, cyano, nitro, alkyle en C₁-C₆, alcényle en C₁-C₄, alcynyle en C₁-C₄, cycloalkyle en C₃-C₆, alcoxy en C₁-C₆, N-alcoxy en C₁-C₆-imino-alkyle en C₁-C₃, alkylsulfanyle en C₁-C₆, alkylsulfinyle en C₁-C₆, alkylsulfonyle en C₁-C₆ ou N,N-di-(alkyle en C₁-C₆)amino, lorsque les groupes R³, R⁴, R⁶, R¹⁰, R¹¹, Q et V sont chacun substitués indépendamment les uns des autres, les substituants étant un (1) ou plusieurs substituants choisis dans le groupe constitué par amino, hydroxy, halogène, nitro, cyano, isocyano, mercapto, isothiocyanato, carboxy en C₁-C₄, carbonamide, SF₅, aminosulfonyle, alkyle en C₁-C₄, cycloalkyle en C₃-C₄, alcényle en C₂-C₄, cycloalcényle en C₃-C₄, alcynyle en C₂-C₄, N-mono-alkylamino en C₁-C₄, N,N-di-alkylamino en C₁-C₄, N-alcanoylamino en C₁-C₄, alcoxy en C₁-C₄, alcényloxy en C₂-C₄, alcynyloxy en C₂-C₄, cycloalcoxy en C₃-C₄, cycloalcényloxy en C₃-C₄, alcoxycarbonyle en C₁-C₄, alcényloxycarbonyle en C₂-C₄, alcynyloxycarbonyle en C₂-C₄, aryloxycarbonyle en C₆, C₁₀, C₁₄, alcanoyle en C₁-C₄, alcénylcarbonyle en C₂-C₄, alcynylcarbonyle en C₂-C₄, arylcarbonyle en C₆, C₁₀, C₁₄, alkylsulfanyle en C₁-C₄, cycloalkylsulfanyle en C₃-C₄, alkylthio en C₁-C₄, alcénylthio en C₂-C₄, cycloalcénylthio en C₃-C₄, alcynylthio en C₂-C₄, alkylsulfényle en C₁-C₄ et alkylsulfinyle en C₁-C₄, les deux énantiomères du groupe alkylsulfinyle en C₁-C₄ étant compris, alkylsulfonyle en C₁-C₄, N-mono-alkyle en C₁-C₄-aminosulfonyle, N,N-di-alkyle en C₁-C₄-aminosulfonyle, alkylphosphinyle en C₁-C₄, alkylphosphonyle en C₁-C₄, les deux énantiomères étant compris pour alkylphosphinyle en C₁-C₄ et alkylphosphonyle en C₁-C₄, N-alkyle en C₁-C₄-aminocarbonyle, N,N-di-alkyle en C₁-C₄-aminocarbonyle, N-alcanoyle en C₁-C₄-aminocarbonyle, N-alcanoyle en C₁-C₄-N-alkyle en C₁-C₄-aminocarbonyle, aryle en C₆, C₁₀, C₁₄, aryloxy en C₆, C₁₀, C₁₄, benzyle, benzyloxy, benzylthio, arylthio en C₆, C₁₀, C₁₄, arylamino en C₆, C₁₀, C₁₄, benzylamino, hétérocyclyle et trialkylsilyle, les substituants reliés avec une double liaison tels qu'alkylidène en C₁-C₄ (p. ex. méthylidène ou éthylidène), un groupe oxo, un groupe thioxo, un groupe imino et un groupe imino substitué,
ainsi que les sels, les N-oxydes et les formes tautomères des composés de formule (Ia").

2. Composés selon la revendication 1, dans lesquels les composés de formule (Ia") sont des composés de formule (I-T3) 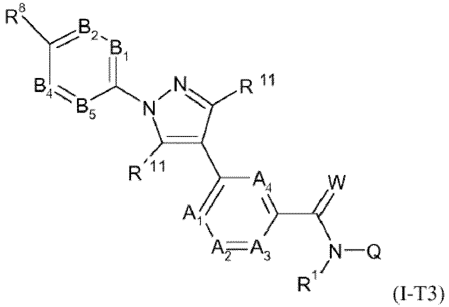 dans laquelle
R¹, A₁, A₂, A₃, A₄, R¹¹, B₁, B₂, B₄, B₅, R⁸, Q et W sont tels définis dans la revendication 1.

3. Composés selon la revendication 1, dans lesquels les composés de formule (Ia") sont des composés de formule (I-T2) 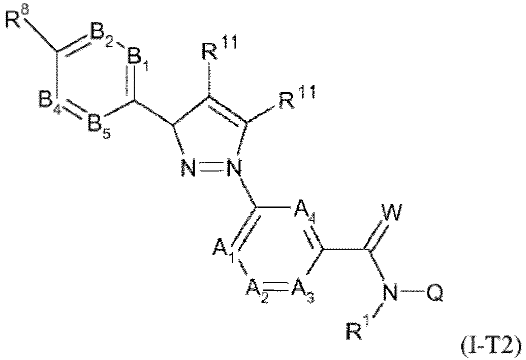 dans laquelle
R¹, A₁, A₂, A₃, A₄, R¹¹, B₁, B₂, B₄, B₅, R⁸, Q et W sont tels définis dans la revendication 1.

4. Composés selon la revendication 1, dans lesquels les composés de formule (Ia") sont des composés de formule (I-T4) 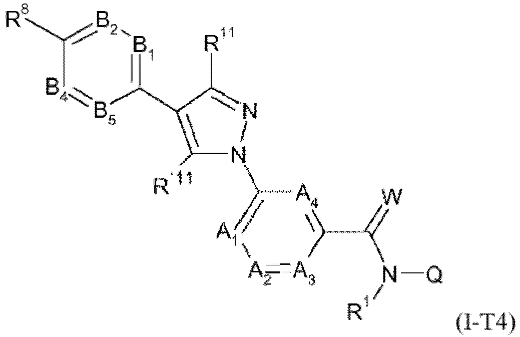 dans laquelle
R¹, A₁, A₂, A₃, A₄, R¹¹, B₁, B₂, B₄, B₅, R⁸, Q et W sont tels définis dans la revendication 1.

5. Composés selon la revendication 1, dans lesquels les composés de formule (Ia") sont des composés de formule (I-T22) 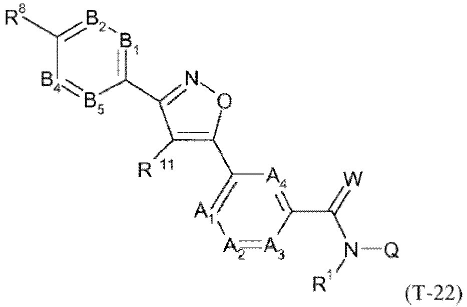 dans laquelle
R¹, A₁, A₂, A₃, A₄, R¹¹, B₁, B₂, B₄, B₅, R⁸, Q et W sont tels définis dans la revendication 1.

6. Composés selon la revendication 1, dans lesquels les composés de formule (Ia") sont des composés de formule (I-T23) 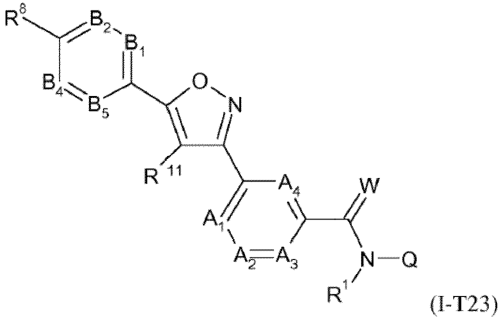 dans laquelle
R¹, A₁, A₂, A₃, A₄, R¹¹, B₁, B₂, B₄, B₅, R⁸, Q et W sont tels définis dans la revendication 1.

7. Composés selon la revendication 1, dans lesquels les composés de formule (Ia") sont des composés de formule (I-T46) 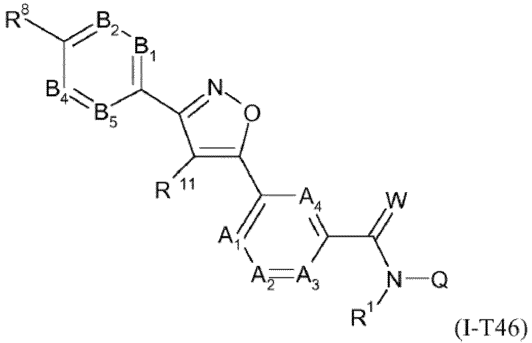 dans laquelle
R¹, A₁, A₂, A₃, A₄, R¹¹, B₁, B₂, B₄, B₅, R⁸, Q et W sont tels définis dans la revendication 1.

8. Composés selon l'une quelconque des revendications 1 à 7, dans lesquels les R¹¹ représentent indépendamment les uns des autres H et W représente O.

9. Composés selon l'une quelconque des revendications 1 à 8, dans lesquels R¹ représente H.

10. Composés selon l'une quelconque des revendications 1 à 9, dans lesquels Q représente alkyle en C₁-C₃, cyclopropyle, 1-(cyano)-cyclopropyle, 1-(alkyle en C₁-C₃ perfluoré)-cyclopropyle, 1-(alkyle en C₁-C₄)-cyclopropyle, 1-(thiocarbamoyl)-cyclopropyle, alkyle en C₁-C₃ substitué avec halogène, thiétan-3-yle, N-méthyl-pyrazol-3-yle ou 2-oxo-2(2,2,2-trifluoroéthylamino) éthyle.

11. Agent insecticide, **caractérisé par** une teneur en au moins un composé de formule (Ia") selon l'une quelconque des revendications 1 à 10 et un extendeur et/ou une substance tensioactive.
